# EUROPEAN PATENT APPLICATION

(11) **EP 4 851 162 A1**
(43) Date of publication of application: **09.12.2026**
(21) Application number: 25747637.4
(22) Date of filing: 21.01.2025
(51) Int. Cl.: C07D 471/04, C07D 471/18, C07D 519/00, A61K 31/4353, A61K 31/437, A61K 31/439

(54) **SMALL-MOLECULE COMPOUND HAVING GLP-1 RECEPTOR AGONIST ACTIVITY AND USE THEREOF**

(30) Priority: 30.01.2024 CN 202410128363; 24.05.2024 CN 202410656836; 15.07.2024 CN 202410945775; 30.09.2024 CN 202411388298
(71) Applicant: Hinova Pharmaceuticals Inc., Chengdu, Sichuan 610041 (CN)
(72) Inventor: FAN, Lei, Chengdu, Sichuan 610041 (CN); YU, Hua, Chengdu, Sichuan 610041 (CN); WANG, Fei, Chengdu, Sichuan 610041 (CN); AI, Chaowu, Chengdu, Sichuan 610041 (CN); XU, Kexin, Chengdu, Sichuan 610041 (CN); LIU, Xingtai, Chengdu, Sichuan 610041 (CN); DU, Jing, Chengdu, Sichuan 610041 (CN); PENG, Ying, Chengdu, Sichuan 610041 (CN); LUO, Tongchuan, Chengdu, Sichuan 610041 (CN); TAN, Bin, Chengdu, Sichuan 610041 (CN); WANG, Wenzhong, Chengdu, Sichuan 610041 (CN); YAN, Dejun, Chengdu, Sichuan 610041 (CN); WANG, Xuzhao, Chengdu, Sichuan 610041 (CN); LI, Liang, Chengdu, Sichuan 610041 (CN); LIU, Chengcheng, Chengdu, Sichuan 610041 (CN); LUO, Yao, Chengdu, Sichuan 610041 (CN); CHU, Xiaohui, Chengdu, Sichuan 610041 (CN); YUAN, Hongwei, Chengdu, Sichuan 610041 (CN); LI, Jing, Chengdu, Sichuan 610041 (CN); HE, Bei, Chengdu, Sichuan 610041 (CN); LI, Xinghai, Chengdu, Sichuan 610041 (CN); CHEN, Yuanwei, Chengdu, Sichuan 610041 (CN)
(74) Representative: Altmann Stößel Dick Patentanwälte PartG mbB
(86) International application number: PCT/CN2025/073617
(87) International publication number: WO 2025/162065

(57) **Abstract**

The present invention provides a small molecule compound with GLP-1 receptor agonist effect and its uses, belonging to the field of chemical drug technology. The small molecule compound of the present invention is a compound represented by formula I, a pharmaceutically acceptable salt thereof, or a stereoisomer thereof. The small molecule compound of the present invention can be used as a GLP-1 receptor agonist for the manufacture of medicaments for preventing and/or treating diseases such as diabetes, obesity, fatty liver, and polycystic ovary syndrome. Compared with LY3502970, the compound of the present invention has superior pharmaceutical properties, specifically reflected in better pharmacokinetic (PK) properties, better oral bioavailability, lower toxicity, better permeability, and better efficacy. Therefore, the compound of the present invention has broad application prospects.

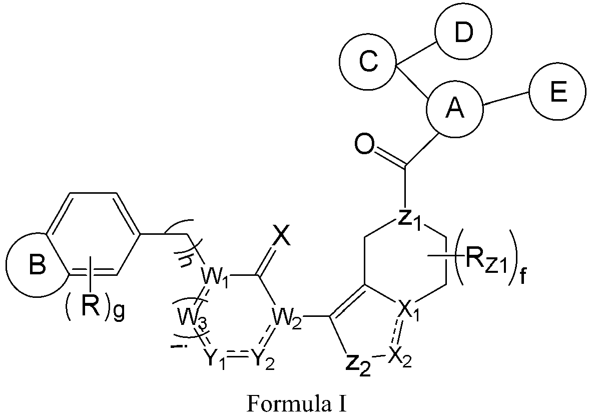

## Description

### Field of the invention

The present invention belongs to the field of chemical drug technology, and specifically relates to a small molecule compound with GLP-1 receptor agonist activity and uses thereof.

### Background of the invention

Glucagon-like peptide-1 (GLP-1) is an incretin hormone synthesized by L cells in the distal small intestine and colon, which exerts its effects by binding to the GLP-1 receptor (GLP-1R) on the surface of target cell membranes. Its therapeutic effects on type 2 diabetes include glucose-dependent insulin synthesis and secretion, inhibition of postprandial glucagon secretion, protection of pancreatic β cells, appetite suppression, delay of gastric emptying, reduction of hepatic glucose output, increase in insulin sensitivity, body weight reduction, and the same. GLP-1R agonists (GLP-1RA) have the advantage of avoiding rapid degradation by dipeptidyl-peptidase IV (DPP-IV) in the body, making them a hotspot in the research and treatment of diabetes. Currently, GLP-1RAs occupy an important position in the "Guidelines for Prevention and Treatment of Type 2 Diabetes in China (2013 Edition)" published by the Diabetes Branch of the Chinese Medical Association, as well as other guidelines.

It has been found that GLP-1 receptors are not only expressed in the pancreas, but also widely expressed in extra-pancreatic tissues, including the gastrointestinal tract, cardiovascular system, central nervous system, lungs, kidneys, bones, skin, etc. GLP-1 receptor agonists can not only be used to treat type 2 diabetes, but also hold promise as medicaments for treating obesity, fatty liver, polycystic ovary syndrome, and other diseases.

LY3502970 is a known GLP-1 receptor agonist that can be used for the treatment of type 2 diabetes, obesity, and other related diseases. However, the pharmaceutical properties of LY3502970 need to be further improved.

### Summary of the invention

The objective of the present invention is to provide a small molecule compound with GLP-1 receptor agonist activity and uses thereof.

The present invention provides a compound represented by formula I, a pharmaceutically acceptable salt thereof, or a stereoisomer thereof: 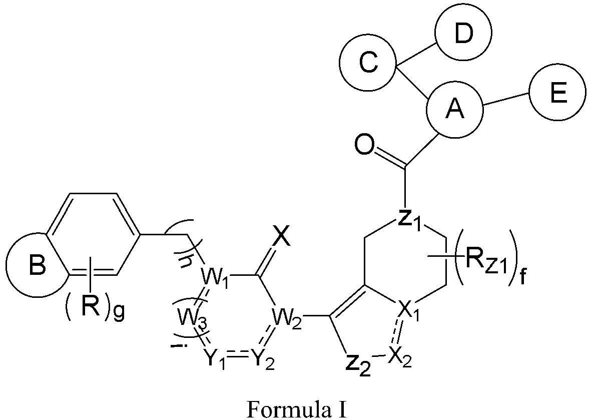 wherein,
ring A is selected from the group consisting of aryl, heteroaryl, fused cycloalkyl, and fused heterocyclyl;
ring E is selected from the group consisting of substituted or unsubstituted (3-10)-membered cycloalkyl, and substituted or unsubstituted (3-10)-membered heterocycloalkyl;
ring C is selected from the group consisting of substituted or unsubstituted (3-10)-membered cycloalkyl, and substituted or unsubstituted (3-10)-membered heterocycloalkyl;
ring D is selected from the group consisting of substituted or unsubstituted aryl, and substituted or unsubstituted heteroaryl;
Z₁ is selected from N and CH;
f represents the number of the substituent group R_{Z1}, and is selected from integers ranging from 0 to 6;
each R_{Z1} is independently selected from the group consisting of H, substituted or unsubstituted C₁-C₈ alkyl, substituted or unsubstituted C₁-C₈ alkoxy, and halogen; or two adjacent R_{Z1} are joined to form a (3-10)-membered cycloalkyl, and (3-10)-membered heterocycloalkyl; or two non-adjacent R_{Z1} are joined to form 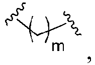 wherein m is 1, 2, or 3;
X₁ is selected from N or C; X₂ is selected from N or CH₂; and there is exactly one N in X₁ and X₂; the dashed line between X₁ and X₂ is absent or a bond according to the selection of X₁ and X₂;
Z₂ is selected from NR_{z2} and CHR_{z2};
R_{z2} is selected from substituted or unsubstituted aryl, and substituted or unsubstituted heteroaryl;
X is selected from O and S;
i is selected from 0 or 1;
W₁ and W₂ are each independently selected from N or C; W₃ is selected from CH or N; and at least one of W₁, W₂, and W₃ is N;
Y₁ and Y₂ are each independently selected from C=O, C=S, N, or CR₁₄; R₁₄ is each independently selected from H, substituted or unsubstituted C₁-C₈ alkyl, substituted or unsubstituted C₁-C₈ alkoxy, halogen, hydroxyl, carboxyl, substituted or unsubstituted (3-10)-membered cycloalkyl;
the dashed lines between W₁, W₂, W₃, Y₁, and Y₂ are either absent or a bond, depending on the selection of W₁, W₂, W₃, Y₁, and Y₂;
h is selected from integers between 0 and 3;
g represents the number of the substituent R, and is selected from integers ranging from 0 to 3;
each R is independently selected from H, substituted or unsubstituted C₁-C₈ alkyl, substituted or unsubstituted C₁-C₈ alkoxy, and halogen;
ring B is selected from the group consisting of absence, substituted or unsubstituted aryl, and substituted or unsubstituted heteroaryl;
the substituents of the alkyl, alkenyl, alkynyl, alkoxy, aldehyde, amine group, ester group, and amide are selected from the following groups: deuterium, halogen, hydroxyl, cyano, C₁-C₈ alkyl, halogenated C₁-C₈ alkyl, C₁-C₈ alkoxy, halogenated C₁-C₈ alkoxy, substituted or unsubstituted (3-10)-membered cycloalkyl, substituted or unsubstituted (3-10)-membered heterocycloalkyl, substituted or unsubstituted aryl, substituted or unsubstituted heteroaryl, - C(O)R₁', and -NR₂'R₃';
the substituents of the cycloalkyl, heterocycloalkyl, aryl, and heteroaryl are selected from the following groups: substituted or unsubstituted C₁-C₈ alkyl, halogen, cyano, hydroxyl, substituted or unsubstituted C₁-C₈ alkoxy, substituted or unsubstituted C₂-C₈ alkenyl, substituted or unsubstituted C₂-C₈ alkynyl, substituted or unsubstituted (3-10)-membered cycloalkyl, substituted or unsubstituted (3-10)-membered heterocycloalkyl, substituted or unsubstituted C₁-C₈ ester group, substituted or unsubstituted C₁-C₈ amide, amino, carboxyl, substituted or unsubstituted C₁-C₈ aldehyde, substituted or unsubstituted C₁-C₈ amine group, =O; or the substituents on the same carbon atom in the cycloalkyl, heterocycloalkyl, aryl, and heteroaryl form =O or =S;
R₁', R₂', and R₃' are each independently selected from H and C₁-C₈ alkyl;
the compound is not:

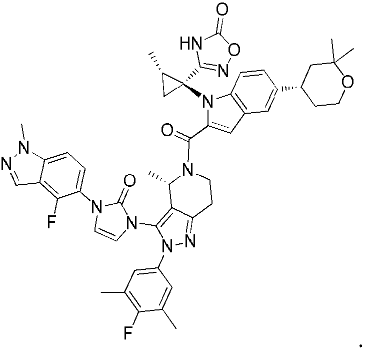

Further, the compound is represented by formula I-1: 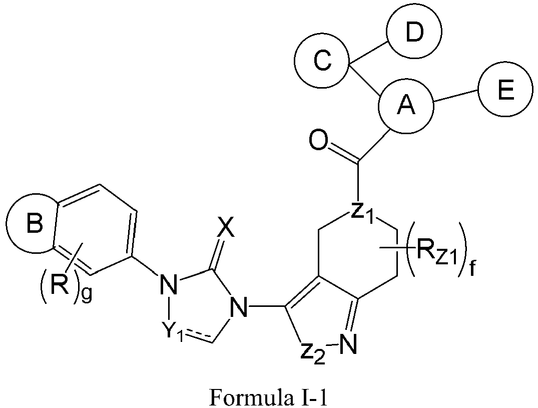 wherein,
ring A is selected from the group consisting of aryl, heteroaryl, fused cycloalkyl, and fused heterocyclyl;
ring E is selected from the group consisting of substituted or unsubstituted (3-8)-membered cycloalkyl, and substituted or unsubstituted (3-8)-membered heterocycloalkyl; the substituents are each independently selected from the group consisting of C₁-C₈ alkyl, C₁-C₈ alkoxy, halogenated C₁-C₈ alkyl, halogenated C₁-C₈ alkoxy, and halogen;
ring C is selected from the group consisting of substituted or unsubstituted (3-8)-membered saturated cycloalkyl, and substituted or unsubstituted (3-8)-membered saturated heterocycloalkyl; the substituents are each independently selected from the group consisting of C₁-C₈ alkyl, C₁-C₈ alkoxy, halogenated C₁-C₈ alkyl, halogenated C₁-C₈ alkoxy, and halogen;
ring D is selected from the group consisting of aryl and heteroaryl;
Z₁ is selected from N and CH;
f is selected from integers ranging from 0 to 6;
each R_{Z1} is independently selected from the group consisting of H, C₁-C₈ alkyl, C₁-C₈ alkoxy, halogenated C₁-C₈ alkyl, halogenated C₁-C₈ alkoxy, and halogen; or two adjacent R_{Z1} are joined to form a (3-8)-membered cycloalkyl, and (3-8)-membered heterocycloalkyl; or two non-adjacent R_{Z1} are joined to form 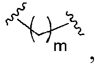 wherein m is 1, 2, or 3;
Z₂ is selected from NR_{z2} and CHR_{z2};
R_{Z2} is selected from substituted or unsubstituted aryl, and substituted or unsubstituted heteroaryl; the substituents are each independently selected from the group consisting of C₁-C₈ alkyl, C₁-C₈ alkoxy, halogenated C₁-C₈ alkyl, halogenated C₁-C₈ alkoxy, halogen, (3-8)-membered saturated cycloalkyl, and (3-8)-membered saturated heterocycloalkyl;
X is selected from O and S;
- - - - - is selected from - or =;
provided that - - - - - is -, Y₁ represents C=O or C=S;
provided that - - - - - is =, Y₁ represents N or CR₁₄; R₁₄ is selected from the group consisting of H, C₁-C₈ alkyl, C₁-C₈ alkoxy, halogenated C₁-C₈ alkyl, halogenated C₁-C₈ alkoxy, halogen, and hydroxyl;
g is selected from integers ranging from 0 to 3;
each R is independently selected from the group consisting of H, C₁-C₈ alkyl, C₁-C₈ alkoxy, halogenated C₁-C₈ alkyl, halogenated C₁-C₈ alkoxy, and halogen;
ring B is selected from the group consisting of substituted or unsubstituted aryl, and substituted or unsubstituted heteroaryl; the substituents are each independently selected from the group consisting of C₁-C₈ alkyl, C₁-C₈ alkoxy, halogenated C₁-C₈ alkyl, halogenated C₁-C₈ alkoxy, and halogen;
the compound is not:

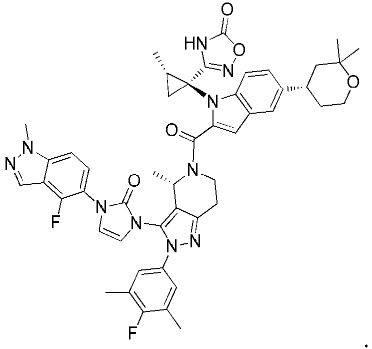

Further, the structure of the compound is as represented by formula II-1: 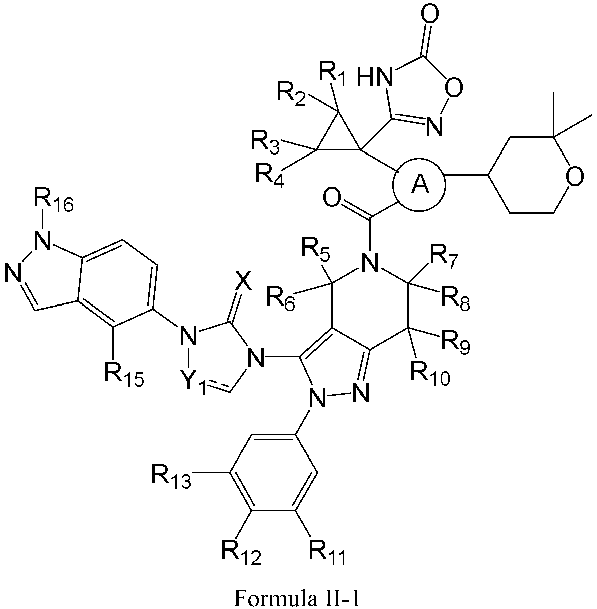 wherein,
A 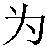 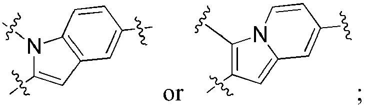
R₁ is selected from the group consisting of H, C₁-C₅ alkyl, and halogen;
R₂ is selected from the group consisting of H, C₁-C₅ alkyl, and halogen;
R₃ is selected from the group consisting of H, C₁-C₅ alkyl, and halogen;
R₄ is selected from the group consisting of H, C₁-C₅ alkyl, and halogen;
R₅ is selected from the group consisting of H, C₁-C₅ alkyl, and halogen;
R₆ is selected from the group consisting of H, C₁-C₅ alkyl, and halogen;
R₇ is selected from the group consisting of H, C₁-C₅ alkyl, and halogen;
R₈ is selected from the group consisting of H, C₁-C₅ alkyl, and halogen;
R₉ is selected from the group consisting of H, C₁-C₅ alkyl, and halogen;
R₁₀ is selected from the group consisting of H, C₁-C₅ alkyl, and halogen;
alternatively, R₅ and R₆ are linked to form a (3-6)-membered saturated cycloalkyl;
alternatively, R₅ and R₉ are linked to form 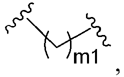 with m1 being 1, 2, or 3;
alternatively, R₅ and R₇ are linked to form 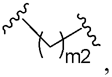 with m2 being 1, 2, or 3;
R₁₁ is selected from the group consisting of H, C₁-C₅ alkyl, halogen, and (3-6)-membered saturated cycloalkyl;
R₁₂ is selected from the group consisting of H, C₁-C₅ alkyl, halogen, and (3-6)-membered saturated cycloalkyl;
R₁₃ is selected from the group consisting of H, C₁-C₅ alkyl, halogen, and (3-6)-membered saturated cycloalkyl;
X is selected from O and S;
- - - - - is selected from - or 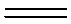 ;
provided that - - - - - is -, Y₁ represents C=O or C=S;
provided that - - - - - is 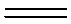 , Y₁ represents N or CR₁₄; R₁₄ is selected from the group consisting of H, C₁-C₅ alkyl, halogen, and hydroxyl;
R₁₅ is selected from the group consisting of H, C₁-C₅ alkyl, and halogen;
R₁₆ is selected from the group consisting of C₁-C₅ alkyl, and deuterated C₁-C₅ alkyl.

Further, the structure of the compound is as represented by formula III-1: 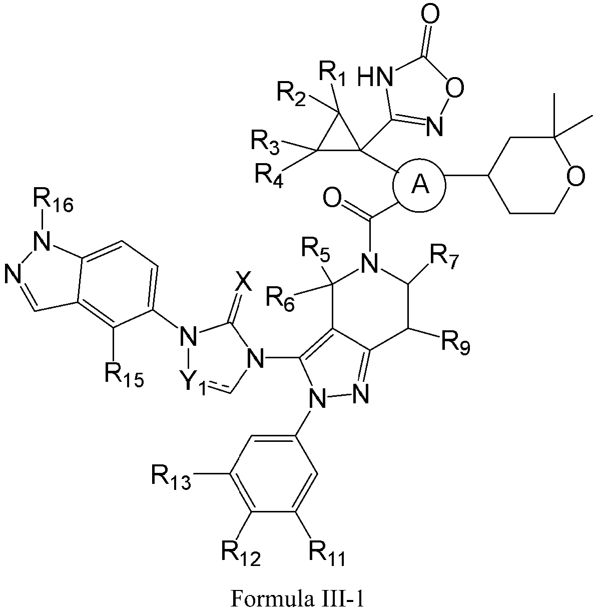 wherein,
A is 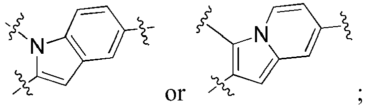
R₁ is selected from the group consisting of H, C₁-C₃ alkyl, and halogen;
R₂ is selected from the group consisting of H, C₁-C₃ alkyl, and halogen;
R₃ is selected from the group consisting of H, C₁-C₃ alkyl, and halogen;
R₄ is selected from the group consisting of H, C₁-C₃ alkyl, and halogen;
R₅ is selected from the group consisting of H, C₁-C₃ alkyl, and halogen;
R₆ is selected from the group consisting of H, C₁-C₃ alkyl, and halogen;
R₇ is selected from the group consisting of H, C₁-C₃ alkyl, and halogen;
R₉ is selected from the group consisting of H, C₁-C₃ alkyl, and halogen;
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
- - - - - is selected from - or 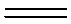 ;
provided that - - - - - is -, Y₁ represents C=O or C=S;
provided that - - - - - is 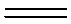 , Y₁ represents N or CR₁₄; R₁₄ is selected from the group consisting of H, C₁-C₃ alkyl, halogen, and hydroxyl;
R₁₅ is selected from the group consisting of H, C₁-C₃ alkyl, and halogen;
R₁₆ is selected from the group consisting of C₁-C₃ alkyl, and deuterated C₁-C₃ alkyl.

Further, the structure of the compound is as represented by formula III-1-1, formula III-2-1, formula III-3-1, formula III-4-1, formula III-5-1, formula III-6-1, formula III-7-1, formula III-8-1, formula III-9-1, or formula III-10-1: 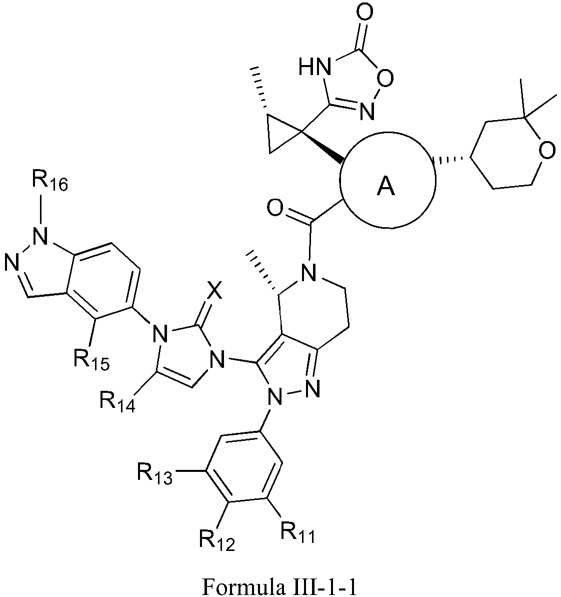 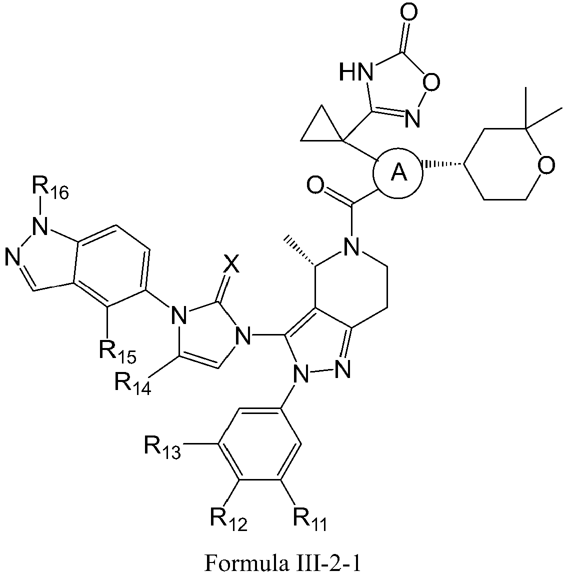 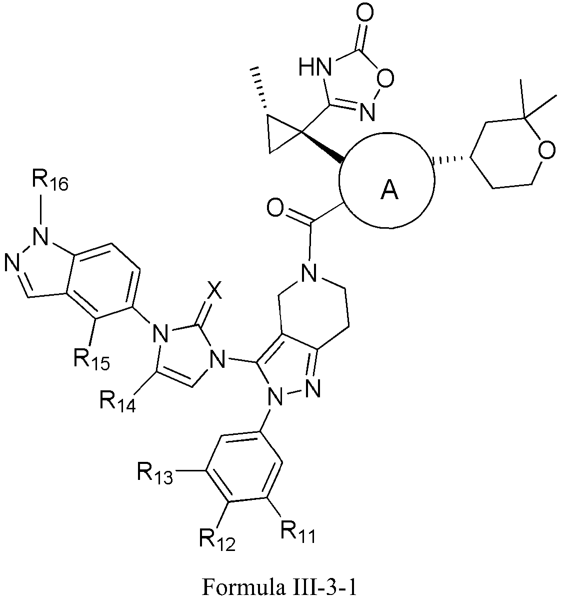 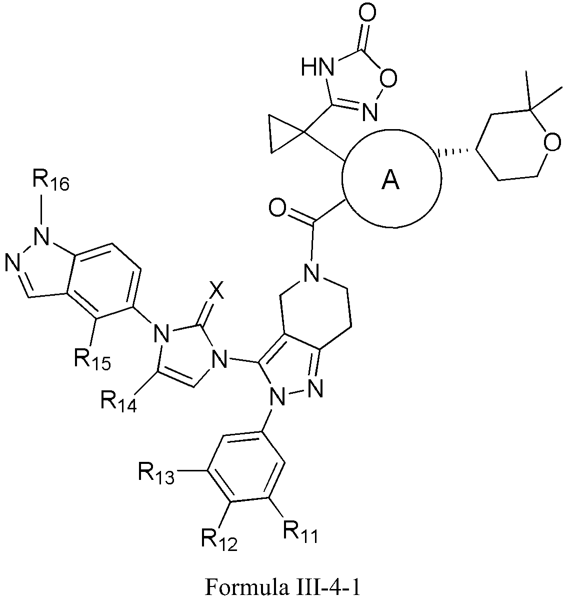 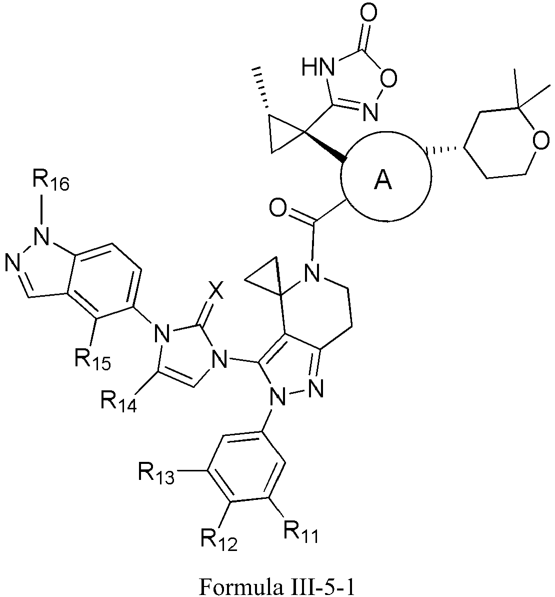 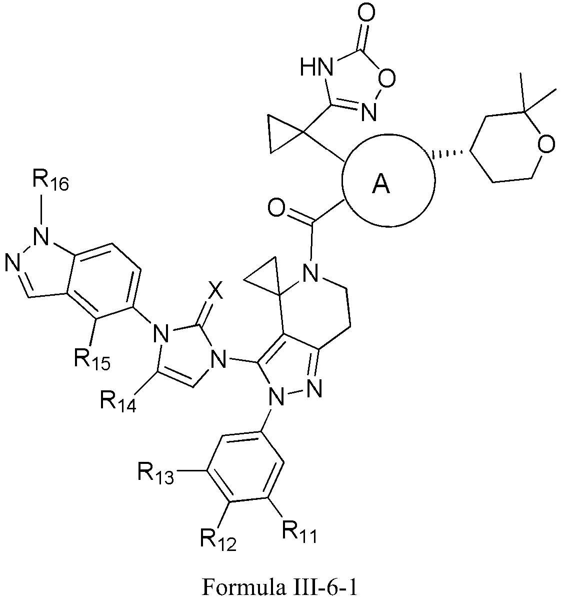 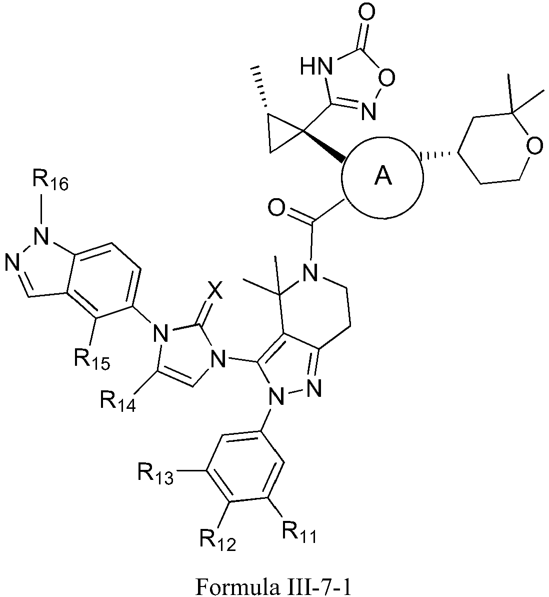 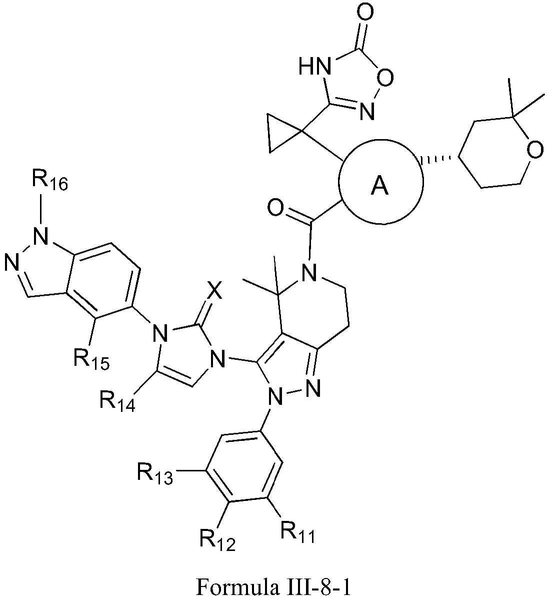 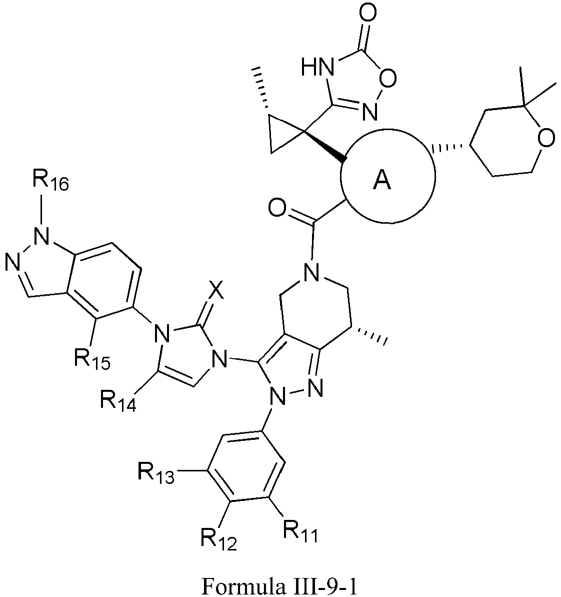 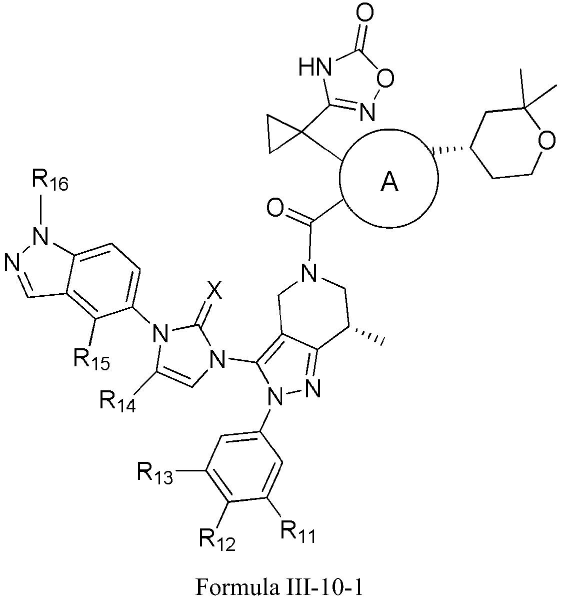 wherein,
A is 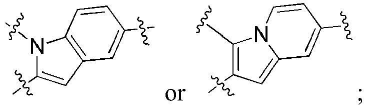
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
R₁₄ is selected from the group consisting of H, C₁-C₃ alkyl, halogen, and hydroxyl;
R₁₅ is selected from the group consisting of H, C₁-C₃ alkyl, and halogen;
R₁₆ is selected from the group consisting of C₁-C₃ alkyl, and deuterated C₁-C₃ alkyl.

Further, the structure of the compound is as represented by formula III-11-1, formula III-12-1, and formula III-13-1: 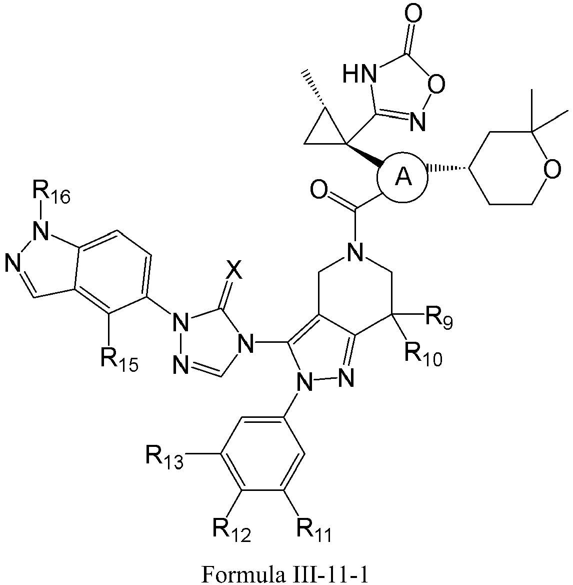 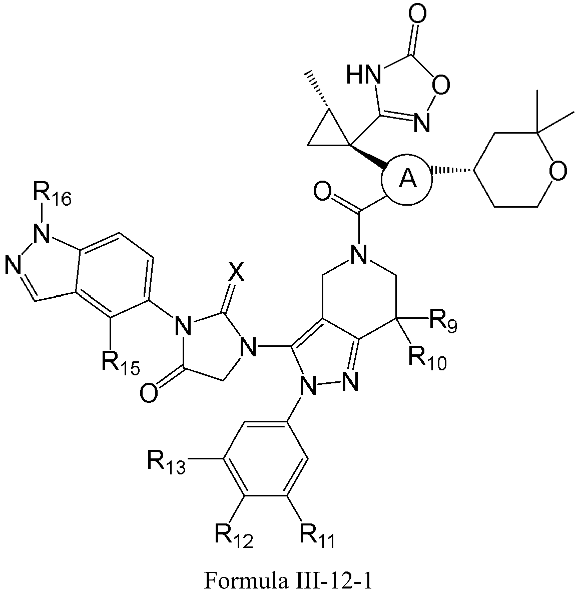 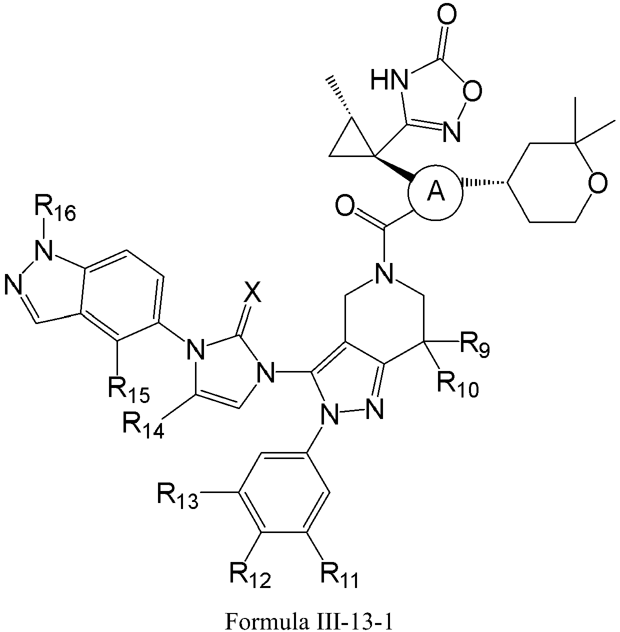 wherein,
A is 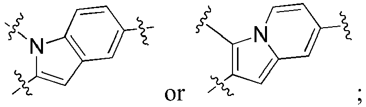
R₉ is selected from the group consisting of H, C₁-C₃ alkyl, and halogen;
R₁₀ is selected from the group consisting of H, C₁-C₃ alkyl, and halogen;
alternatively, R₉ and R₁₀ are linked to form a (3-6)-membered saturated cycloalkyl;
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
R₁₄ is selected from the group consisting of H, C₁-C₃ alkyl, halogen, and hydroxyl;
R₁₅ is selected from the group consisting of H, C₁-C₅ alkyl, and halogen;
R₁₆ is selected from the group consisting of C₁-C₅ alkyl, and deuterated C₁-C₅ alkyl.

Further, the structure of the compound is as represented by formula IV-1 or formula IV'-1: 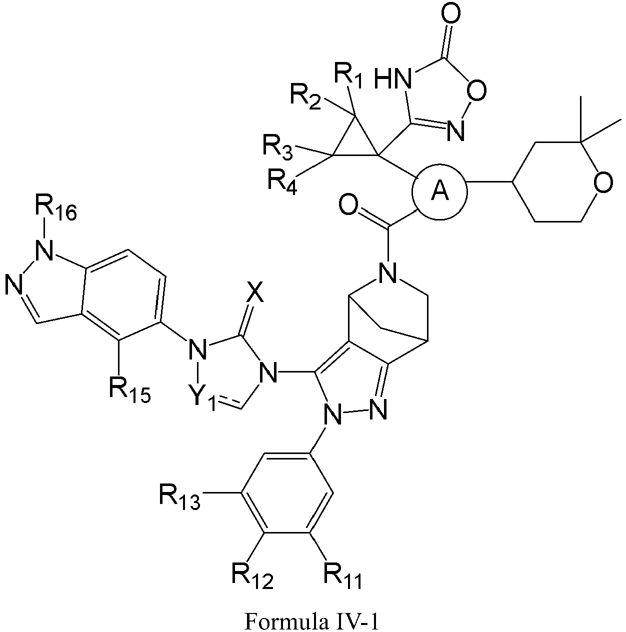 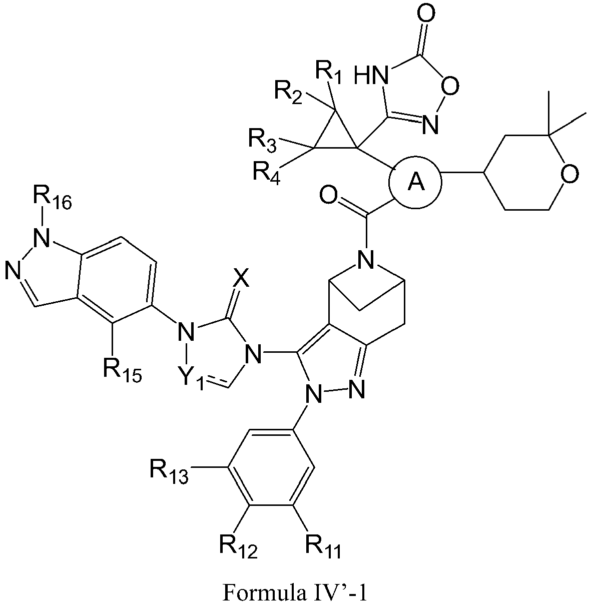 wherein,
A is 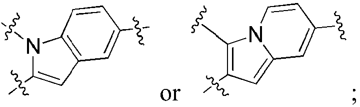
R₁ is selected from the group consisting of H, C₁-C₃ alkyl, and halogen;
R₂ is selected from the group consisting of H, C₁-C₃ alkyl, and halogen;
R₃ is selected from the group consisting of H, C₁-C₃ alkyl, and halogen;
R₄ is selected from the group consisting of H, C₁-C₃ alkyl, and halogen;
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
- - - - - is selected from - or 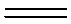 ;
provided that - - - - - is -, Y₁ represents C=O or C=S;
provided that - - - - - is 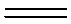 , Y₁ represents N or CR₁₄; R₁₄ is selected from the group consisting of H, C₁-C₃ alkyl, halogen, and hydroxyl;
R₁₅ is selected from the group consisting of H, C₁-C₃ alkyl, and halogen;
R₁₆ is selected from the group consisting of C₁-C₃ alkyl, and deuterated C₁-C₃ alkyl.

Further, the structure of the compound is as represented by formula IV-1-1, formula IV-2-1, formula IV-3-1, formula IV-4-1, formula IV-5-1, formula IV-6-1, formula IV-7-1, or formula IV-8-1: 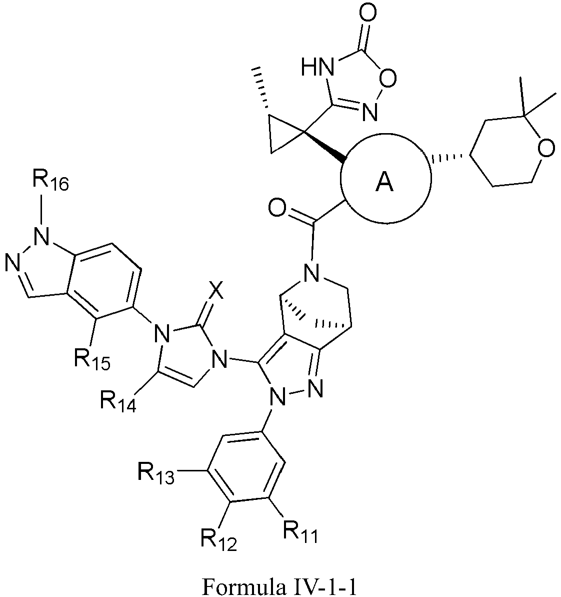 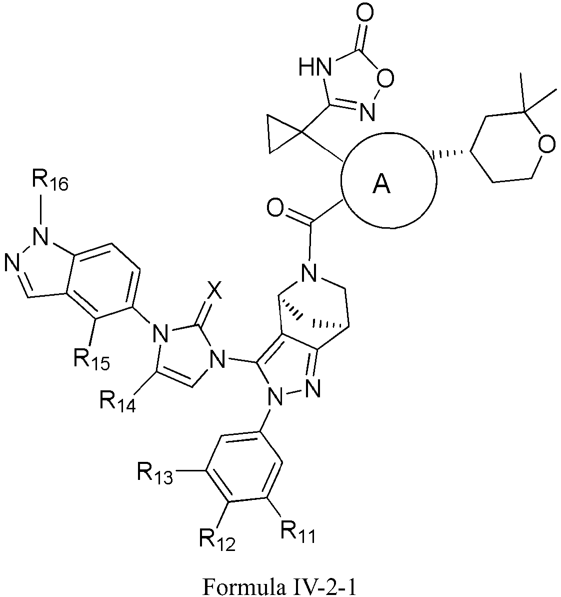 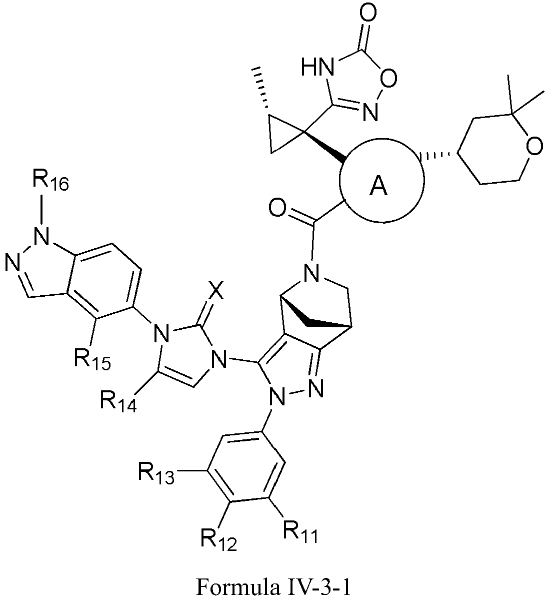 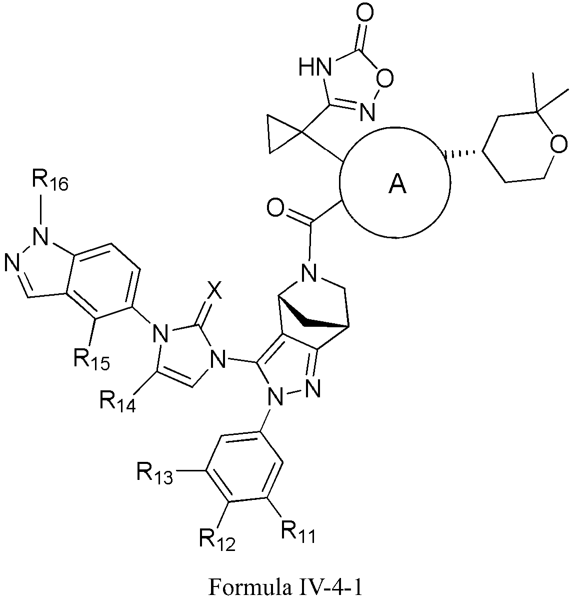 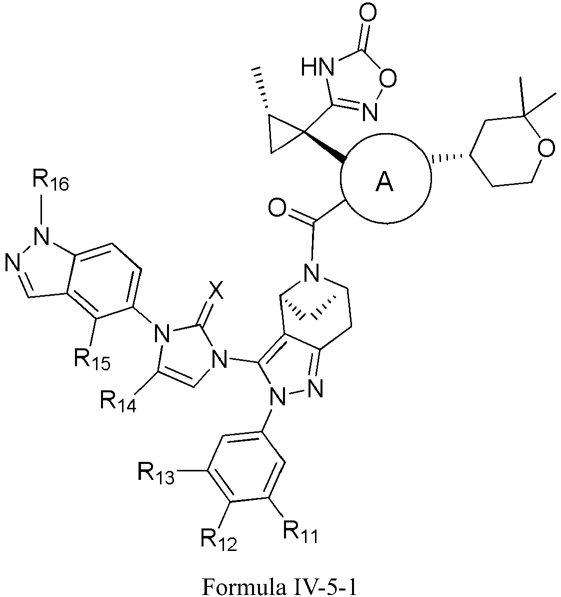 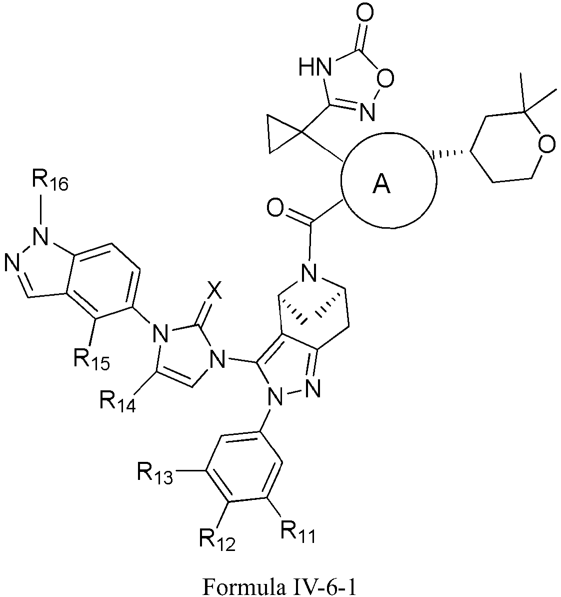 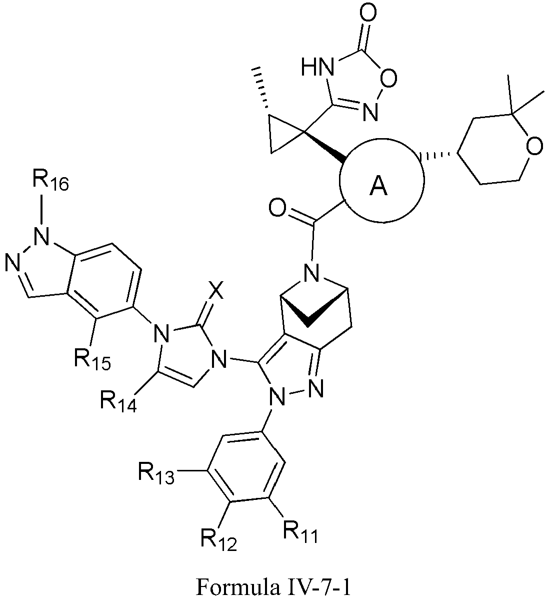 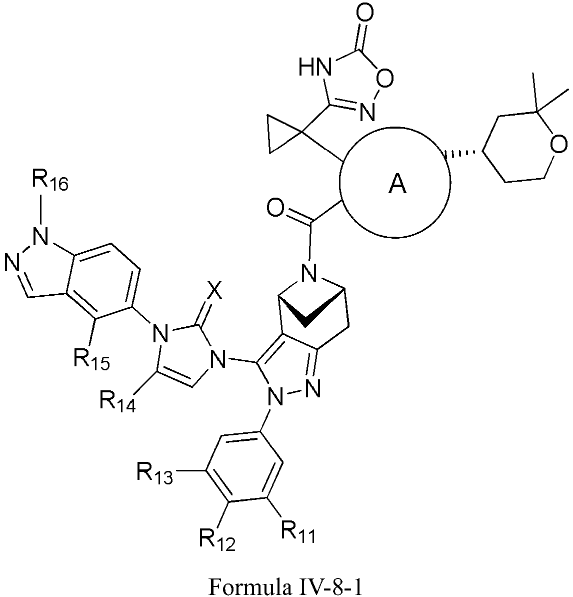 wherein,
A is 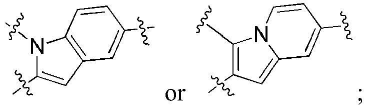
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
R₁₄ is selected from the group consisting of H, C₁-C₃ alkyl, halogen, and hydroxyl;
R₁₅ is selected from the group consisting of H, C₁-C₃ alkyl, and halogen;
R₁₆ is selected from the group consisting of C₁-C₃ alkyl, and deuterated C₁-C₃ alkyl.

Furthermore, the structure of the compound is as represented by formula V-1: 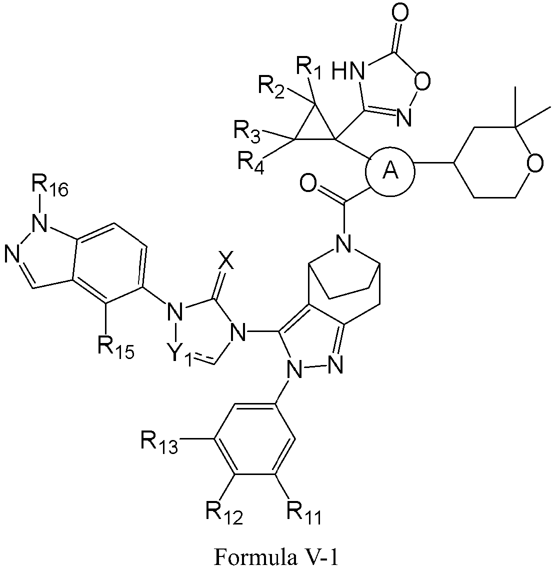 wherein,
A is 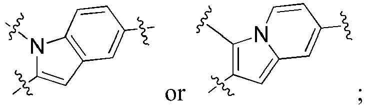
R₁ is selected from the group consisting of H, C₁-C₃ alkyl, and halogen;
R₂ is selected from the group consisting of H, C₁-C₃ alkyl, and halogen;
R₃ is selected from the group consisting of H, C₁-C₃ alkyl, and halogen;
R₄ is selected from the group consisting of H, C₁-C₃ alkyl, and halogen;
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
- - - - - is selected from - or 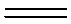 ;
provided that - - - - - is -, Y₁ represents C=O or C=S;
provided that - - - - - is 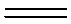 , Y₁ represents N or CR₁₄; R₁₄ is selected from the group consisting of H, C₁-C₃ alkyl, halogen, and hydroxyl;
R₁₅ is selected from the group consisting of H, C₁-C₃ alkyl, and halogen;
R₁₆ is selected from the group consisting of C₁-C₃ alkyl, and deuterated C₁-C₃ alkyl.

Further, the structure of the compound is as represented by formula V-1-1, formula V-2-1, formula V-3-1, or formula V-4-1: 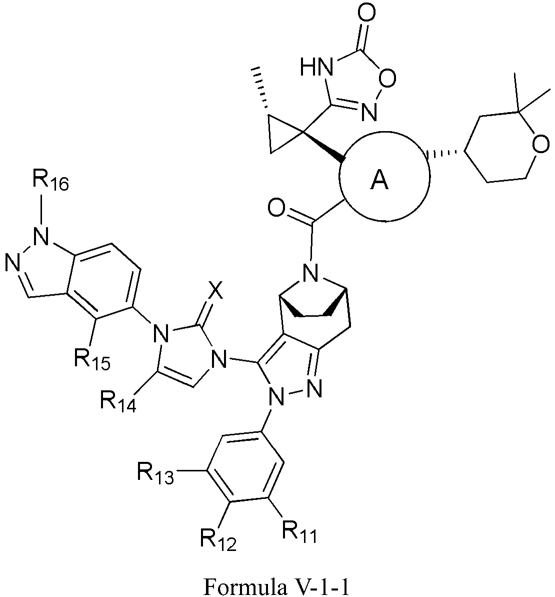 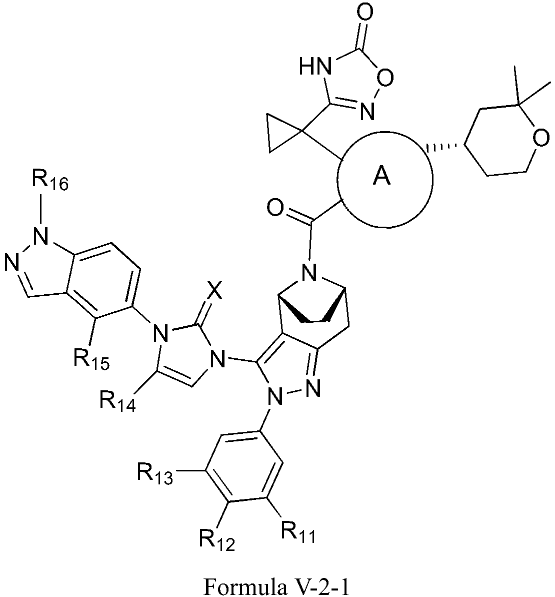 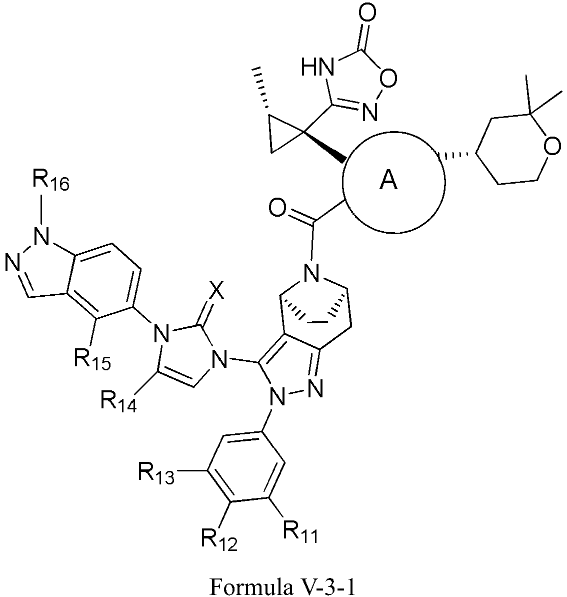 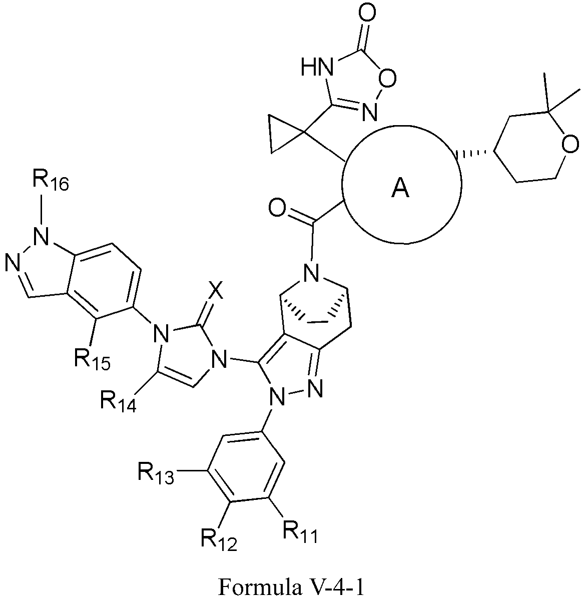 wherein,
A is 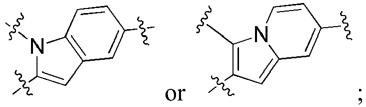
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
R₁₄ is selected from the group consisting of H, C₁-C₃ alkyl, halogen, and hydroxyl;
R₁₅ is selected from the group consisting of H, C₁-C₃ alkyl, and halogen;
R₁₆ is selected from the group consisting of C₁-C₃ alkyl, and deuterated C₁-C₃ alkyl.

Further, the structure of the compound is as represented by formula I-2, formula I-3, formula I-4, or formula I-5: 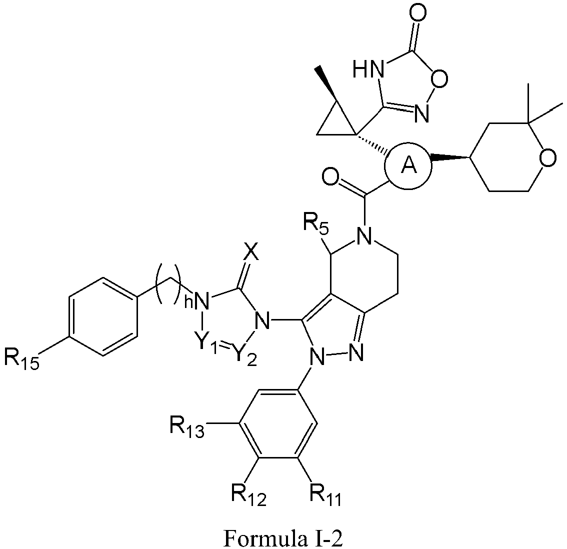 wherein,
A is 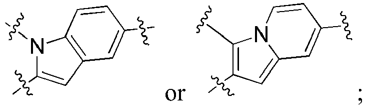
R₅ is selected from the group consisting of H, C₁-C₃ alkyl, and halogen;
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
Y₁ and Y₂ are each independently selected from N or CR₁₄; R₁₄ is selected from H, C₁-C₃ alkyl, halogenated C₁-C₃ alkyl, hydroxyl-substituted C₁-C₃ alkyl, carboxyl, halogen, and hydroxyl;
h is 0 or 1;
R₁₅ is selected from H, C₁-C₃ alkyl, C₁-C₃ alkoxy, and halogen; 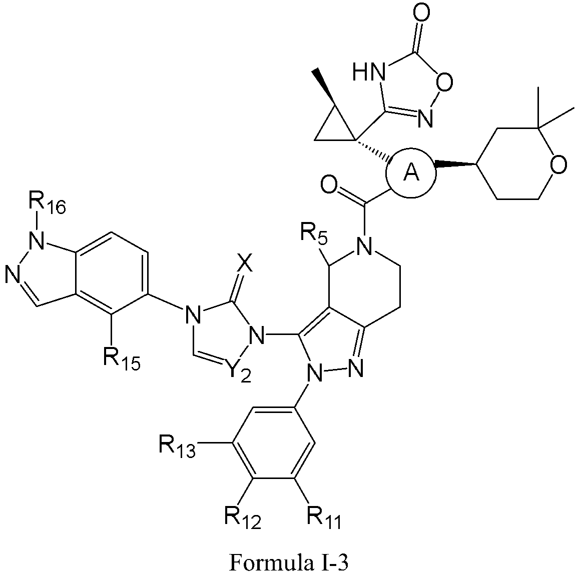 wherein,
A is 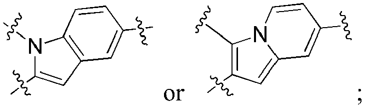
R₅ is selected from the group consisting of H, C₁-C₃ alkyl, and halogen;
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
Y₂ is independently selected from N or CR₁₄; R₁₄ is selected from H, C₁-C₃ alkyl, halogenated C₁-C₃ alkyl, hydroxyl-substituted C₁-C₃ alkyl, carboxyl, halogen, hydroxyl, and (3-6)-membered saturated cycloalkyl;
R₁₅ is selected from the group consisting of H, C₁-C₃ alkyl, and halogen;
R₁₆ is selected from the group consisting of C₁-C₃ alkyl, and deuterated C₁-C₃ alkyl; 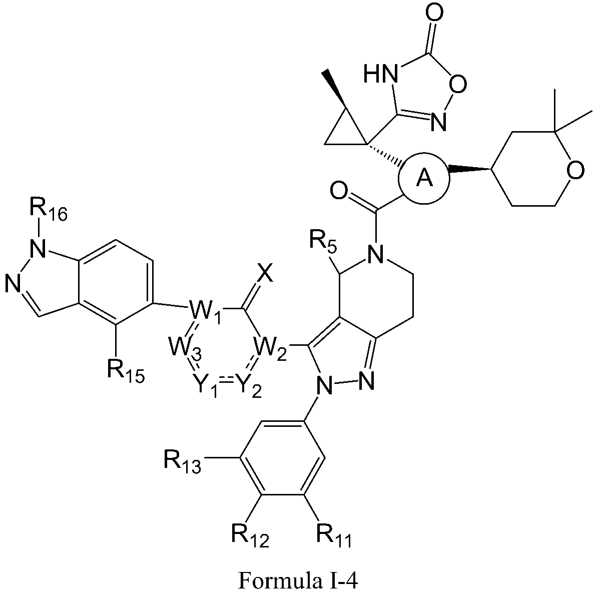 wherein,
A is 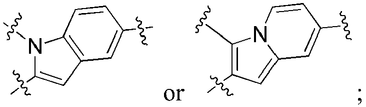
R₅ is selected from the group consisting of H, C₁-C₃ alkyl, and halogen;
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
W₁ and W₂ are each independently selected from N or C; W₃ is selected from CH or N; and at least one of W₁, W₂, and W₃ is N;
Y₁ and Y₂ are each independently selected from N or CR₁₄; R₁₄ is selected from the group consisting of H, C₁-C₃ alkyl, halogenated C₁-C₃ alkyl, hydroxyl-substituted C₁-C₃ alkyl, carboxyl, halogen, hydroxyl, and 3-6 membered saturated cycloalkyl;
The dashed lines between W₁, W₂, W₃, Y₁, and Y₂ are either absent or a bond, depending on the selection of W₁, W₂, W₃, Y₁, and Y₂;
R₁₅ is selected from the group consisting of H, C₁-C₃ alkyl, and halogen;
R₁₆ is selected from the group consisting of C₁-C₃ alkyl, and deuterated C₁-C₃ alkyl; 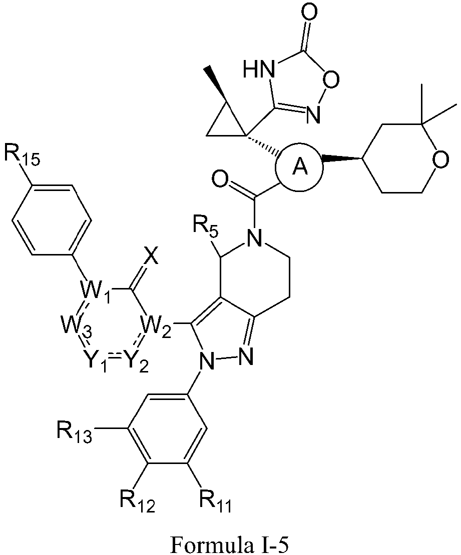 wherein,
A is 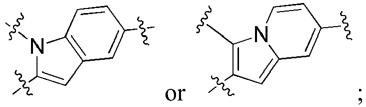
R₅ is selected from the group consisting of H, C₁-C₃ alkyl, and halogen;
R₁₁ is selected from the group consisting of H, C₁-C₃ alkyl, halogen, and (3-6)-membered saturated cycloalkyl;
R₁₂ is selected from the group consisting of H, C₁-C₃ alkyl, halogen, and (3-6)-membered saturated cycloalkyl;
R₁₃ is selected from the group consisting of H, C₁-C₃ alkyl, halogen, and (3-6)-membered saturated cycloalkyl;
X is selected from O and S;
W₁ and W₂ are each independently selected from N or C; W₃ is selected from CH or N; and at least one of W₁, W₂, and W₃ is N;
Y₁ and Y₂ are each independently selected from N or CR₁₄; R₁₄ is selected from the group consisting of H, C₁-C₃ alkyl, halogenated C₁-C₃ alkyl, hydroxyl-substituted C₁-C₃ alkyl, carboxyl, halogen, hydroxyl, and 3-6 membered saturated cycloalkyl;
The dashed lines between W₁, W₂, W₃, Y₁, and Y₂ are either absent or a bond, depending on the selection of W₁, W₂, W₃, Y₁, and Y₂;
R₁₅ is selected from the group consisting of H, C₁-C₃ alkyl, and halogen.

Further, the compound is as represented by formula I-6: 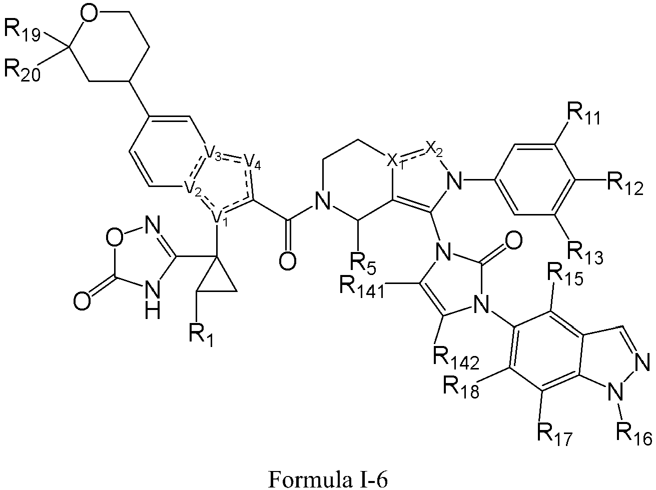 wherein,
R₁₂ is selected from the group consisting of H, substituted or unsubstituted C₁-C₆ alkyl, and halogen;
R₁₁ and R₁₃ are each independently selected from the group consisting of H, substituted or unsubstituted C₁-C₆ alkyl, substituted or unsubstituted C₂-C₆ alkenyl, substituted or unsubstituted C₂-C₆ alkynyl, and halogen; and at least one of R₁₁ and R₁₃ is selected from substituted or unsubstituted C₂-C₆ alkenyl, or substituted or unsubstituted C₂-C₆ alkynyl;
R₁₄₁ and R₁₄₂ are each independently selected from the group consisting of H, substituted or unsubstituted C₁-C₆ alkyl, substituted or unsubstituted C₁-C₆ alkoxy, and halogen;
R₁, R₅, R₁₅, R₁₆, R₁₇, R₁₈, R₁₉, and R₂₀ are each independently selected from the group consisting of H, substituted or unsubstituted C₁-C₆ alkyl, and halogen;
X₁ is selected from N or C; X₂ is selected from N or CH₂; and there is exactly one N in X₁ and X₂; the dashed line between X₁ and X₂ is absent or a bond according to the selection of X₁ and X₂;
V₁, V₂, and V₃ are each independently selected from N or C; V₄ is selected from N or CH; the dashed line connecting V₁, V₂, V₃, and V₄ is absent or a bond depending on the selection of V₁, V₂, V₃, and V₄;
the substituents of the alkyl, alkenyl, and alkynyl are selected from the following groups: halogen, hydroxyl, C₁-C₆ alkyl, C₁-C₆ alkoxy, substituted or unsubstituted (3-10)-membered cycloalkyl, substituted or unsubstituted (3-10)-membered heterocycloalkyl, substituted or unsubstituted (6-10)-membered aryl, substituted or unsubstituted (5-10)-membered heteroaryl, - C(O)R₁', and -NR₂'R₃';
the substituent of the alkoxy is selected from the following groups: C₁-C₆ alkyl, halogenated C₁-C₆ alkyl, halogen, and -C(O)R₁';
the substituents of the cycloalkyl, heterocycloalkyl, aryl, and heteroaryl are selected from the following groups: C₁-C₆ alkyl, halogenated C₁-C₆ alkyl, halogen, and hydroxyl;
R₁', R₂', and R₃' are each independently selected from the group consisting of H and C₁-C₆ alkyl.

Further, the structure of the compound is as represented by formula II-6: 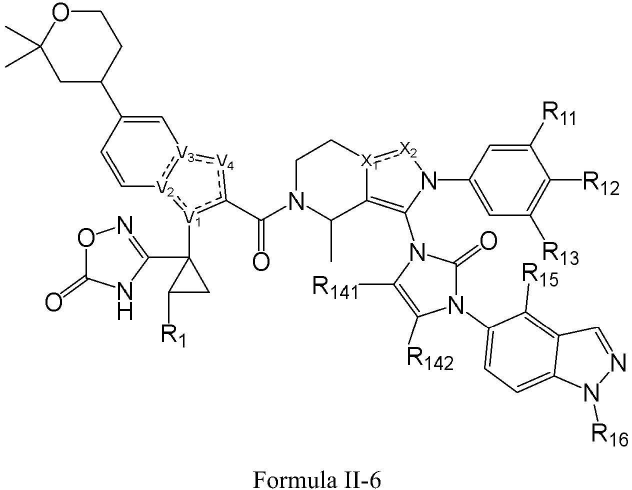 wherein,
R₁₂ is selected from the group consisting of H, substituted or unsubstituted C₁-C₆ alkyl, and halogen;
R₁₁ and R₁₃ are each independently selected from the group consisting of H, substituted or unsubstituted C₁-C₆ alkyl, substituted or unsubstituted C₂-C₆ alkenyl, substituted or unsubstituted C₂-C₆ alkynyl, and halogen; and at least one of R₁₁ and R₁₃ is selected from substituted or unsubstituted C₂-C₆ alkenyl, or substituted or unsubstituted C₂-C₆ alkynyl;
R₁₄₁ and R₁₄₂ are each independently selected from the group consisting of H, substituted or unsubstituted C₁-C₆ alkyl, substituted or unsubstituted C₁-C₆ alkoxy, and halogen;
R₁, R₁₅, and R₁₆ are each independently selected from the group consisting of H, substituted or unsubstituted C₁-C₆ alkyl, and halogen;
X₁ is selected from N or C; X₂ is selected from N or CH₂; and there is exactly one N in X₁ and X₂; the dashed line between X₁ and X₂ is absent or a bond according to the selection of X₁ and X₂;
V₁, V₂, and V₃ are each independently selected from N or C; V₄ is selected from N or CH; the dashed line connecting V₁, V₂, V₃, and V₄ is absent or a bond depending on the selection of V₁, V₂, V₃, and V₄;
the substituents of the alkyl, alkenyl, and alkynyl are selected from the following groups: halogen, hydroxyl, C₁-C₆ alkyl, C₁-C₆ alkoxy, substituted or unsubstituted (3-10)-membered cycloalkyl, substituted or unsubstituted (3-10)-membered heterocycloalkyl, substituted or unsubstituted (6-10)-membered aryl, substituted or unsubstituted (5-10)-membered heteroaryl, - C(O)R₁', and -NR₂'R₃';
the substituent of the alkoxy is selected from the following groups: C₁-C₆ alkyl, halogenated C₁-C₆ alkyl, halogen, and -C(O)R₁';
the substituents of the cycloalkyl, heterocycloalkyl, aryl, and heteroaryl are selected from the following groups: C₁-C₆ alkyl, halogenated C₁-C₆ alkyl, halogen, and hydroxyl;
R₁', R₂', and R₃' are each independently selected from the group consisting of H and C₁-C₆ alkyl.

Further, the structure of the compound is as represented by formula III-6: 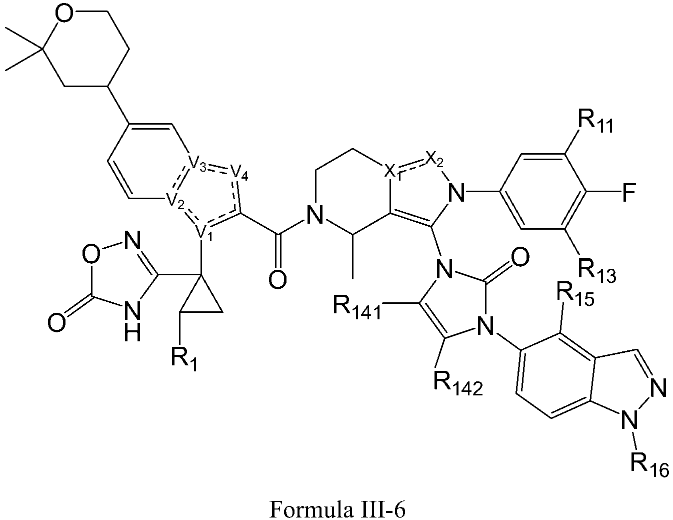 wherein,
R₁₁ and R₁₃ are each independently selected from the group consisting of H, substituted or unsubstituted C₁-C₆ alkyl, substituted or unsubstituted C₂-C₆ alkenyl, substituted or unsubstituted C₂-C₆ alkynyl, and halogen; and at least one of R₁₁ and R₁₃ is selected from substituted or unsubstituted C₂-C₆ alkenyl, or substituted or unsubstituted C₂-C₆ alkynyl;
R₁₄₁ and R₁₄₂ are each independently selected from the group consisting of H, substituted or unsubstituted C₁-C₆ alkyl, substituted or unsubstituted C₁-C₆ alkoxy, and halogen;
R₁, R₁₅, and R₁₆ are each independently selected from the group consisting of H, substituted or unsubstituted C₁-C₆ alkyl, and halogen;
X₁ is selected from N or C; X₂ is selected from N or CH₂; and there is exactly one N in X₁ and X₂; the dashed line between X₁ and X₂ is absent or a bond according to the selection of X₁ and X₂;
V₁, V₂, and V₃ are each independently selected from N or C; V₄ is selected from N or CH; the dashed line connecting V₁, V₂, V₃, and V₄ is absent or a bond depending on the selection of V₁, V₂, V₃, and V₄;
the substituents of the alkyl, alkenyl, and alkynyl are selected from the following groups: halogen, hydroxyl, C₁-C₆ alkyl, C₁-C₆ alkoxy, substituted or unsubstituted (3-10)-membered cycloalkyl, substituted or unsubstituted (3-10)-membered heterocycloalkyl, substituted or unsubstituted (6-10)-membered aryl, substituted or unsubstituted (5-10)-membered heteroaryl, - C(O)R₁', and -NR₂'R₃';
the substituent of the alkoxy is selected from the following groups: C₁-C₆ alkyl, halogenated C₁-C₆ alkyl, halogen, and -C(O)R₁';
the substituents of the cycloalkyl, heterocycloalkyl, aryl, and heteroaryl are selected from the following groups: C₁-C₆ alkyl, halogenated C₁-C₆ alkyl, halogen, and hydroxyl;
R₁', R₂', and R₃' are each independently selected from the group consisting of H and C₁-C₆ alkyl.

Further, the structure of the compound is as represented by formula IV-6: 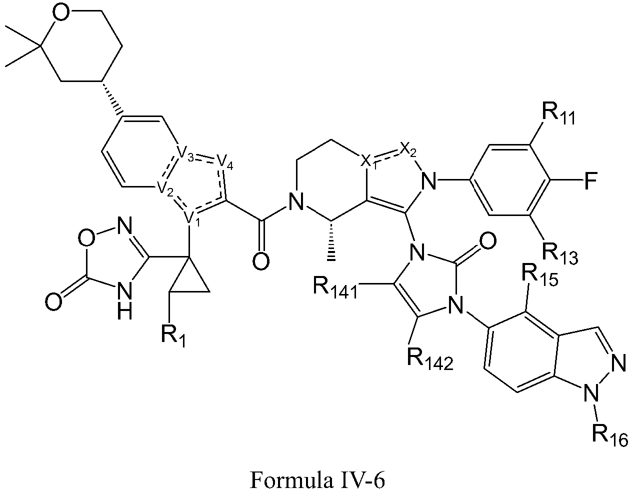 wherein,
R₁₁ and R₁₃ are each independently selected from the group consisting of H, substituted or unsubstituted C₁-C₆ alkyl, substituted or unsubstituted C₂-C₆ alkenyl, substituted or unsubstituted C₂-C₆ alkynyl, and halogen; and at least one of R₁₁ and R₁₃ is selected from substituted or unsubstituted C₂-C₆ alkenyl, or substituted or unsubstituted C₂-C₆ alkynyl;
R₁₄₁ and R₁₄₂ are each independently selected from the group consisting of H, substituted or unsubstituted C₁-C₆ alkyl, substituted or unsubstituted C₁-C₆ alkoxy, and halogen;
R₁, R₁₅, and R₁₆ are each independently selected from the group consisting of H, substituted or unsubstituted C₁-C₆ alkyl, and halogen;
X₁ is selected from N or C; X₂ is selected from N or CH₂; and there is exactly one N in X₁ and X₂; the dashed line between X₁ and X₂ is absent or a bond according to the selection of X₁ and X₂;
V₁, V₂, and V₃ are each independently selected from N or C; V₄ is selected from N or CH; the dashed line connecting V₁, V₂, V₃, and V₄ is absent or a bond depending on the selection of V₁, V₂, V₃, and V₄;
the substituents of the alkyl, alkenyl, and alkynyl are selected from the following groups: halogen, hydroxyl, C₁-C₆ alkyl, C₁-C₆ alkoxy, substituted or unsubstituted (3-10)-membered cycloalkyl, substituted or unsubstituted (3-10)-membered heterocycloalkyl, substituted or unsubstituted (6-10)-membered aryl, substituted or unsubstituted (5-10)-membered heteroaryl, - C(O)R₁', and -NR₂'R₃';
the substituent of the alkoxy is selected from the following groups: C₁-C₆ alkyl, halogenated C₁-C₆ alkyl, halogen, and -C(O)R₁';
the substituents of the cycloalkyl, heterocycloalkyl, aryl, and heteroaryl are selected from the following groups: C₁-C₆ alkyl, halogenated C₁-C₆ alkyl, halogen, and hydroxyl;
R₁', R₂', and R₃' are each independently selected from the group consisting of H and C₁-C₆ alkyl.

Further, the structure of the compound is as represented by formula Va-6: 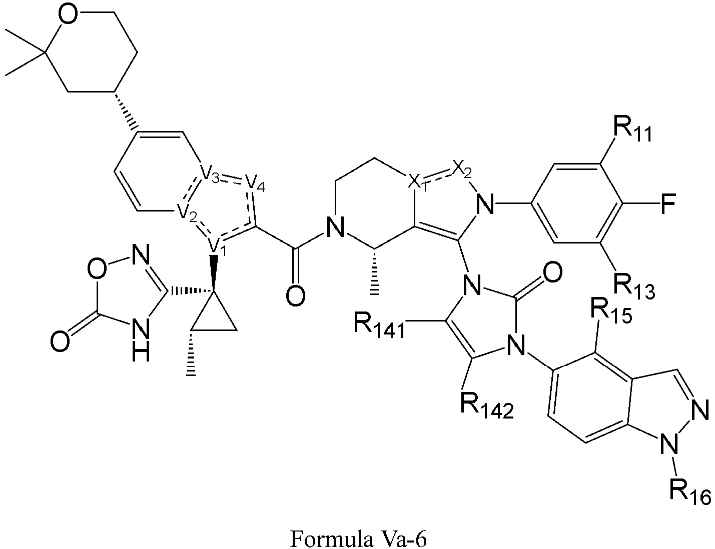 wherein,
R₁₁ and R₁₃ are each independently selected from the group consisting of H, substituted or unsubstituted C₁-C₆ alkyl, substituted or unsubstituted C₂-C₆ alkenyl, substituted or unsubstituted C₂-C₆ alkynyl, and halogen; and at least one of R₁₁ and R₁₃ is selected from substituted or unsubstituted C₂-C₆ alkenyl, or substituted or unsubstituted C₂-C₆ alkynyl;
R₁₄₁ and R₁₄₂ are each independently selected from the group consisting of H, substituted or unsubstituted C₁-C₆ alkyl, substituted or unsubstituted C₁-C₆ alkoxy, and halogen;
R₁₅ and R₁₆ are each independently selected from the group consisting of H, substituted or unsubstituted C₁-C₆ alkyl, and halogen;
X₁ is selected from N or C; X₂ is selected from N or CH₂; and there is exactly one N in X₁ and X₂; the dashed line between X₁ and X₂ is absent or a bond according to the selection of X₁ and X₂;
V₁, V₂, and V₃ are each independently selected from N or C; V₄ is selected from N or CH; the dashed line connecting V₁, V₂, V₃, and V₄ is absent or a bond depending on the selection of V₁, V₂, V₃, and V₄;
the substituents of the alkyl, alkenyl, and alkynyl are selected from the following groups: halogen, hydroxyl, C₁-C₆ alkyl, C₁-C₆ alkoxy, substituted or unsubstituted (3-10)-membered cycloalkyl, substituted or unsubstituted (3-10)-membered heterocycloalkyl, substituted or unsubstituted (6-10)-membered aryl, substituted or unsubstituted (5-10)-membered heteroaryl, - C(O)R₁', and -NR₂'R₃';
the substituent of the alkoxy is selected from the following groups: C₁-C₆ alkyl, halogenated C₁-C₆ alkyl, halogen, and -C(O)R₁';
the substituents of the cycloalkyl, heterocycloalkyl, aryl, and heteroaryl are selected from the following groups: C₁-C₆ alkyl, halogenated C₁-C₆ alkyl, halogen, and hydroxyl;
R₁', R₂', and R₃' are each independently selected from the group consisting of H and C₁-C₆ alkyl.

Further, the structure of the compound is as represented by formula Vb-6: 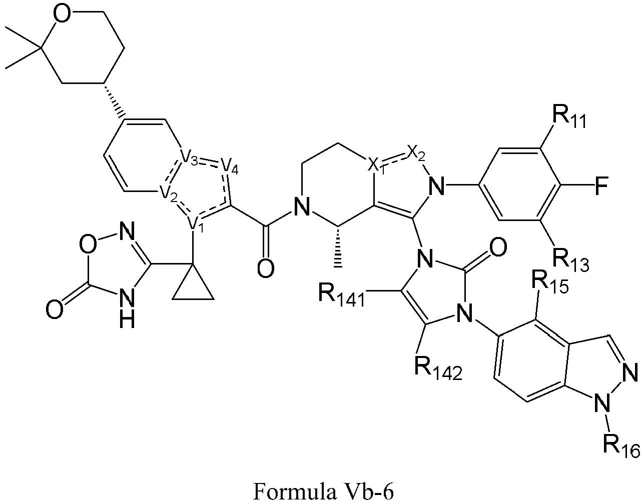 wherein,
R₁₁ and R₁₃ are each independently selected from the group consisting of H, substituted or unsubstituted C₁-C₆ alkyl, substituted or unsubstituted C₂-C₆ alkenyl, substituted or unsubstituted C₂-C₆ alkynyl, and halogen; and at least one of R₁₁ and R₁₃ is selected from substituted or unsubstituted C₂-C₆ alkenyl, or substituted or unsubstituted C₂-C₆ alkynyl;
R₁₄₁ and R₁₄₂ are each independently selected from the group consisting of H, substituted or unsubstituted C₁-C₆ alkyl, substituted or unsubstituted C₁-C₆ alkoxy, and halogen;
R₁₅ and R₁₆ are each independently selected from the group consisting of H, substituted or unsubstituted C₁-C₆ alkyl, and halogen;
X₁ is selected from N or C; X₂ is selected from N or CH₂; and there is exactly one N in X₁ and X₂; the dashed line between X₁ and X₂ is absent or a bond according to the selection of X₁ and X₂;
V₁, V₂, and V₃ are each independently selected from N or C; V₄ is selected from N or CH; the dashed line connecting V₁, V₂, V₃, and V₄ is absent or a bond depending on the selection of V₁, V₂, V₃, and V₄;
the substituents of the alkyl, alkenyl, and alkynyl are selected from the following groups: halogen, hydroxyl, C₁-C₆ alkyl, C₁-C₆ alkoxy, substituted or unsubstituted (3-10)-membered cycloalkyl, substituted or unsubstituted (3-10)-membered heterocycloalkyl, substituted or unsubstituted (6-10)-membered aryl, substituted or unsubstituted (5-10)-membered heteroaryl, - C(O)R₁', and -NR₂'R₃';
the substituent of the alkoxy is selected from the following groups: C₁-C₆ alkyl, halogenated C₁-C₆ alkyl, halogen, and -C(O)R₁';
the substituents of the cycloalkyl, heterocycloalkyl, aryl, and heteroaryl are selected from the following groups: C₁-C₆ alkyl, halogenated C₁-C₆ alkyl, halogen, and hydroxyl;
R₁', R₂', and R₃' are each independently selected from the group consisting of H and C₁-C₆ alkyl.

Further,
R₁₁ and R₁₃ are each independently selected from the group consisting of H, substituted or unsubstituted C₁-C₆ alkyl, substituted or unsubstituted C₂-C₆ alkenyl, substituted or unsubstituted C₂-C₆ alkynyl, and halogen; and at least one of R₁₁ and R₁₃ is selected from substituted or unsubstituted C₂-C₆ alkenyl, and substituted or unsubstituted C₂-C₆ alkynyl;
the substituents of the alkenyl and alkynyl are selected from the following groups: halogen, hydroxyl, C₁-C₆ alkyl, C₁-C₆ alkoxy, substituted or unsubstituted (3-10)-membered cycloalkyl, substituted or unsubstituted tetrahydropyranyl, substituted or unsubstituted piperidinyl, substituted or unsubstituted pyridinyl, substituted or unsubstituted phenyl, substituted or unsubstituted pyrazolyl, substituted or unsubstituted pyrrolyl, substituted or unsubstituted indazolyl, substituted or unsubstituted indolyl, -C(O)R₁', and -NR₂'R₃';
the substituent of the alkyl is selected from the following groups: halogen, hydroxyl, C₁-C₆ alkyl, and C₁-C₆ alkoxy;
R₁', R₂', and R₃' are each independently selected from the group consisting of H and C₁-C₆ alkyl;
the substituents of the cycloalkyl, tetrahydropyranyl, piperidinyl, pyridinyl, phenyl, pyrazolyl, pyrrolyl, indazolyl, and indolyl are selected from the following groups: C₁-C₆ alkyl, halogenated C₁-C₆ alkyl, halogen, and hydroxyl.

Further, 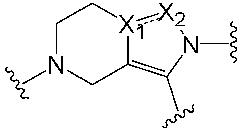 is

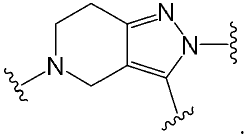

Further, 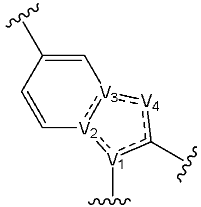 is

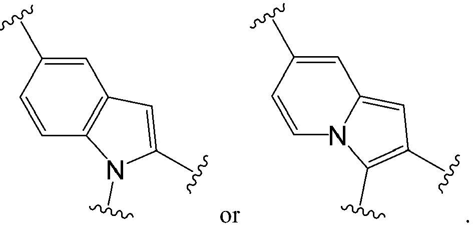

Further, the structure of the compound is as represented by formula VIa-6: 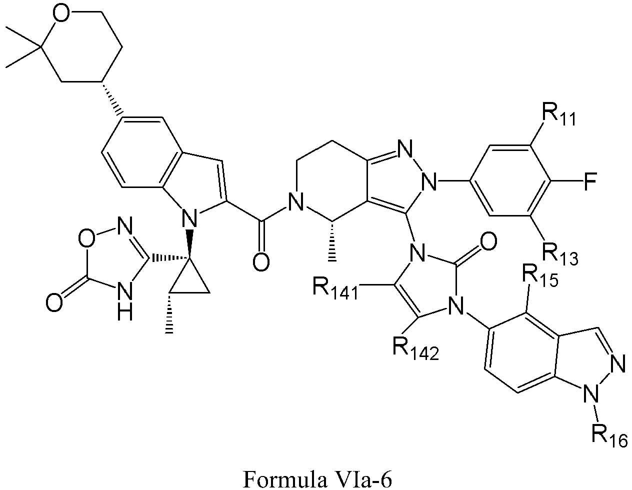 wherein,
R₁₁ and R₁₃ are each independently selected from the group consisting of **H,** substituted or unsubstituted C₁-C₆ alkyl, substituted or unsubstituted C₂-C₆ alkenyl, substituted or unsubstituted C₂-C₆ alkynyl, and halogen; and at least one of R₁₁ and R₁₃ is selected from substituted or unsubstituted C₂-C₆ alkenyl, and substituted or unsubstituted C₂-C₆ alkynyl;
the substituents of the alkenyl and alkynyl are selected from the following groups: halogen, hydroxyl, C₁-C₆ alkyl, C₁-C₆ alkoxy, substituted or unsubstituted (3-10)-membered cycloalkyl, substituted or unsubstituted tetrahydropyranyl, substituted or unsubstituted piperidinyl, substituted or unsubstituted pyridinyl, substituted or unsubstituted phenyl, substituted or unsubstituted pyrazolyl, substituted or unsubstituted pyrrolyl, substituted or unsubstituted indazolyl, substituted or unsubstituted indolyl, -C(O)R₁', and -NR₂'R₃';
R₁₄₁ and R₁₄₂ are each independently selected from the group consisting of H, substituted or unsubstituted C₁-C₆ alkyl, substituted or unsubstituted C₁-C₆ alkoxy, and halogen;
R₁₅ and R₁₆ are each independently selected from the group consisting of **H,** substituted or unsubstituted C₁-C₆ alkyl, and halogen;
the substituents of the cycloalkyl, tetrahydropyranyl, piperidinyl, pyridinyl, phenyl, pyrazolyl, pyrrolyl, indazolyl, and indolyl are selected from the following groups: C₁-C₆ alkyl, halogenated C₁-C₆ alkyl, halogen, and hydroxyl;
the substituent of the alkoxy is selected from the following groups: C₁-C₆ alkyl, halogenated C₁-C₆ alkyl, halogen, and -C(O)R₁';
the substituent of the alkyl is selected from the following groups: halogen, hydroxyl, C₁-C₆ alkyl, and C₁-C₆ alkoxy;
R₁', R₂', and R₃' are each independently selected from the group consisting of H and C₁-C₆ alkyl;

Further, the structure of the compound is as represented by formula VIa-6-1: 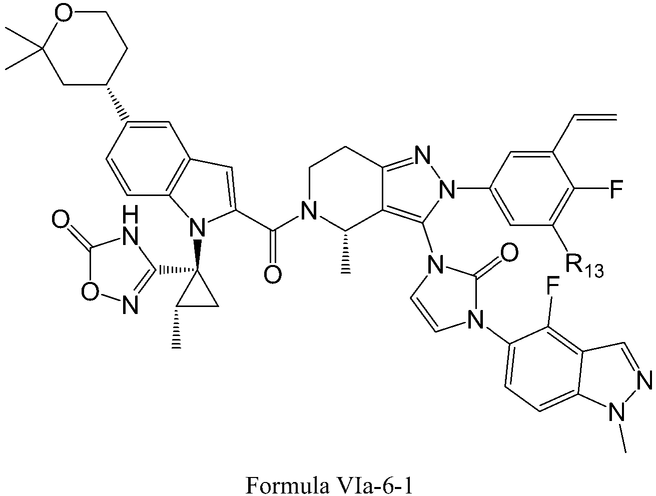 wherein,
each R₁₃ is independently selected from the group consisting of H, C₁-C₆ alkyl, C₂-C₆ alkenyl, C₂-C₆ alkynyl, and halogen.

Further, the structure of the compound is as represented by formula VIb-6: 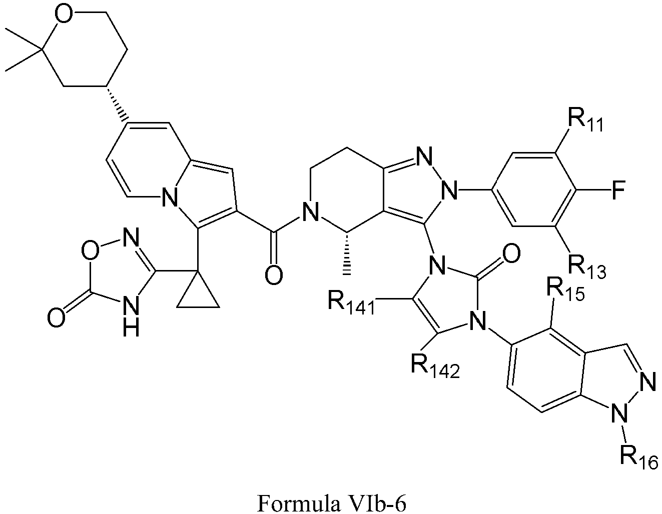 wherein,
R₁₁ and R₁₃ are each independently selected from the group consisting of H, substituted or unsubstituted C₁-C₆ alkyl, substituted or unsubstituted C₂-C₆ alkenyl, substituted or unsubstituted C₂-C₆ alkynyl, and halogen; and at least one of R₁₁ and R₁₃ is selected from substituted or unsubstituted C₂-C₆ alkenyl, and substituted or unsubstituted C₂-C₆ alkynyl;
the substituents of the alkenyl and alkynyl are selected from the following groups: halogen, hydroxyl, C₁-C₆ alkyl, C₁-C₆ alkoxy, substituted or unsubstituted (3-10)-membered cycloalkyl, substituted or unsubstituted tetrahydropyranyl, substituted or unsubstituted piperidinyl, substituted or unsubstituted pyridinyl, substituted or unsubstituted phenyl, substituted or unsubstituted pyrazolyl, substituted or unsubstituted pyrrolyl, substituted or unsubstituted indazolyl, substituted or unsubstituted indolyl, -C(O)R₁', and -NR₂'R₃';
R₁₄₁ and R₁₄₂ are each independently selected from the group consisting of H, substituted or unsubstituted C₁-C₆ alkyl, substituted or unsubstituted C₁-C₆ alkoxy, and halogen;
R₁₅ and R₁₆ are each independently selected from the group consisting of H, substituted or unsubstituted C₁-C₆ alkyl, and halogen;
the substituents of the cycloalkyl, tetrahydropyranyl, piperidinyl, pyridinyl, phenyl, pyrazolyl, pyrrolyl, indazolyl, and indolyl are selected from the following groups: C₁-C₆ alkyl, halogenated C₁-C₆ alkyl, halogen, and hydroxyl;
the substituent of the alkoxy is selected from the following groups: C₁-C₆ alkyl, halogenated C₁-C₆ alkyl, halogen, and -C(O)R₁';
the substituent of the alkyl is selected from the following groups: halogen, hydroxyl, C₁-C₆ alkyl, and C₁-C₆ alkoxy;
R₁', R₂', and R₃' are each independently selected from the group consisting of H and C₁-C₆ alkyl;

Further, the compound is as represented by formula I-7: 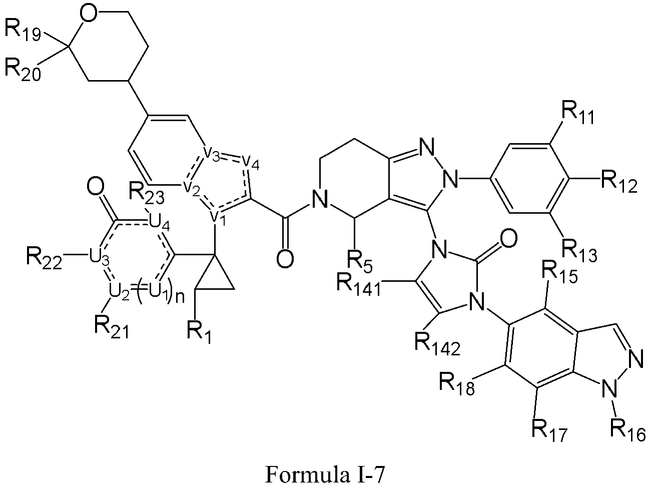 wherein,
R₁₁, R₁₂, and R₁₃ are each independently selected from the group consisting of H, substituted or unsubstituted C₁-C₆ alkyl, substituted or unsubstituted C₁-C₆ alkoxy, substituted or unsubstituted C₂-C₆ alkenyl, substituted or unsubstituted C₂-C₆ alkynyl, halogen, cyano, substituted or unsubstituted (3-10)-membered cycloalkyl, substituted or unsubstituted C₁-C₆ ester group, substituted or unsubstituted C₁-C₆ amide group;
R₁₄₁ and R₁₄₂ are each independently selected from the group consisting of H, substituted or unsubstituted C₁-C₆ alkyl, substituted or unsubstituted C₁-C₆ alkoxy, and halogen;
R₁₅, R₁₆, R₁₇, and R₁₈ are each independently selected from the group consisting of H, substituted or unsubstituted C₁-C₆ alkyl, and halogen;
R₁, R₅, R₁₉, and R₂₀ are each independently selected from the group consisting of H, substituted or unsubstituted C₁-C₆ alkyl, and halogen;
V₁, V₂, and V₃ are each independently selected from the group consisting of N or C; V₄ is selected from N or CH; the dashed line connecting V₁, V₂, V₃, and V₄ is absent or a bond according to the selection of V₁, V₂, V₃, and V₄;
n is 0 or 1;
U₁ is selected from N or CH; U₂, U₃, and U₄ are each independently selected from N, C, and O; R₂₁, R₂₂, and R₂₃ are each independently selected from the group consisting of absence, H, hydroxyl, substituted or unsubstituted C₁-C₆ alkyl, substituted or unsubstituted C₁-C₆ alkoxy, halogen, substituted or unsubstituted (3-10)-membered cycloalkyl, cyano, amino, carboxyl, substituted or unsubstituted C₁-C₆ ester group, substituted or unsubstituted C₁-C₆ amide group, substituted or unsubstituted C₁-C₆ aldehyde group, substituted or unsubstituted C₁-C₆ amine group, O=; the dashed line connecting U₁, U₂, U₃, and U₄ is either absent or a bond, depending on the selection of U₁, U₂, U₃, U₄, R₂₁, R₂₂, and R₂₃;
the substituents of the alkyl, alkenyl, alkynyl, alkoxy, aldehyde group, amine group, ester group, and amide group are selected from the following groups: halogen, hydroxyl, cyano, C₁-C₆ alkyl, halogenated C₁-C₆ alkyl, C₁-C₆ alkoxy, substituted or unsubstituted (3-10)-membered cycloalkyl, substituted or unsubstituted (3-10)-membered heterocycloalkyl, substituted or unsubstituted (6-10)-membered aryl, substituted or unsubstituted (5-10)-membered heteroaryl, - C(O)R₁', and -NR₂'R₃';
the substituent of the cycloalkyl, heterocycloalkyl, aryl, and heteroaryl is selected from the following groups: C₁-C₆ alkyl, halogenated C₁-C₆ alkyl, halogen, and hydroxyl;
R₁', R₂', and R₃' are each independently selected from H and C₁-C₆ alkyl.

Further, the structure of the compound is as represented by formula II-A-7 or formula II-B-7: 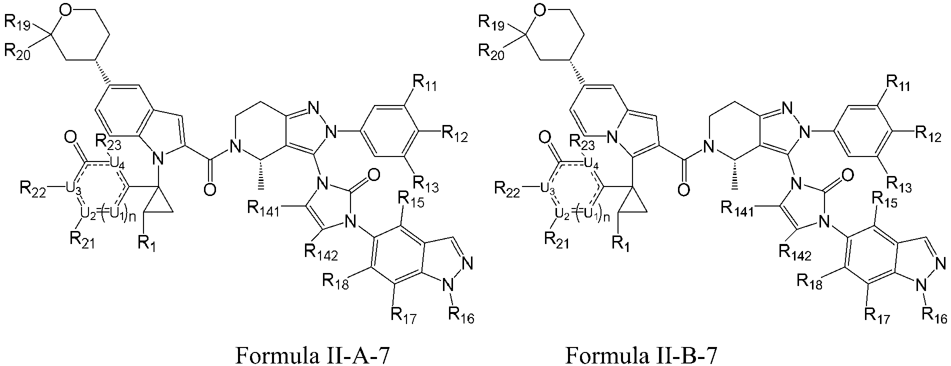 wherein,
R₁₁, R₁₂, and R₁₃ are each independently selected from the group consisting of H, substituted or unsubstituted C₁-C₆ alkyl, substituted or unsubstituted C₁-C₆ alkoxy, substituted or unsubstituted C₂-C₆ alkenyl, substituted or unsubstituted C₂-C₆ alkynyl, halogen, cyano, substituted or unsubstituted (3-10)-membered cycloalkyl, substituted or unsubstituted C₁-C₆ ester group, substituted or unsubstituted C₁-C₆ amide group;
R₁₄₁ and R₁₄₂ are each independently selected from the group consisting of H, substituted or unsubstituted C₁-C₆ alkyl, substituted or unsubstituted C₁-C₆ alkoxy, and halogen;
R₁₅, R₁₆, R₁₇, and R₁₈ are each independently selected from the group consisting of H, substituted or unsubstituted C₁-C₆ alkyl, and halogen;
R₁, R₁₉, and R₂₀ are each independently selected from the group consisting of H, substituted or unsubstituted C₁-C₆ alkyl, and halogen;
n is 0 or 1;
U₁ is selected from N or CH; U₂, U₃, and U₄ are each independently selected from N, C, and O; R₂₁, R₂₂, and R₂₃ are each independently selected from the group consisting of absence, H, hydroxyl, substituted or unsubstituted C₁-C₆ alkyl, substituted or unsubstituted C₁-C₆ alkoxy, halogen, substituted or unsubstituted (3-10)-membered cycloalkyl, cyano, amino, carboxyl, substituted or unsubstituted C₁-C₆ ester group, substituted or unsubstituted C₁-C₆ amide group, substituted or unsubstituted C₁-C₆ aldehyde group, substituted or unsubstituted C₁-C₆ amine group, O=; the dashed line connecting U₁, U₂, U₃, and U₄ is either absent or a bond, depending on the selection of U₁, U₂, U₃, U₄, R₂₁, R₂₂, and R₂₃;
the substituents of the alkyl, alkenyl, alkynyl, alkoxy, aldehyde group, amine group, ester group, and amide group are selected from the following groups: halogen, hydroxyl, cyano, C₁-C₆ alkyl, halogenated C₁-C₆ alkyl, C₁-C₆ alkoxy, substituted or unsubstituted (3-10)-membered cycloalkyl, substituted or unsubstituted (3-10)-membered heterocycloalkyl, substituted or unsubstituted (6-10)-membered aryl, substituted or unsubstituted (5-10)-membered heteroaryl, - C(O)R₁', and -NR₂'R₃';
the substituent of the cycloalkyl, heterocycloalkyl, aryl, and heteroaryl is selected from the following groups: C₁-C₆ alkyl, halogenated C₁-C₆ alkyl, halogen, and hydroxyl;
R₁', R₂', and R₃' are each independently selected from H and C₁-C₆ alkyl.

Further, the structure of the compound is as represented by formula III-A-7 or formula III-B-7: 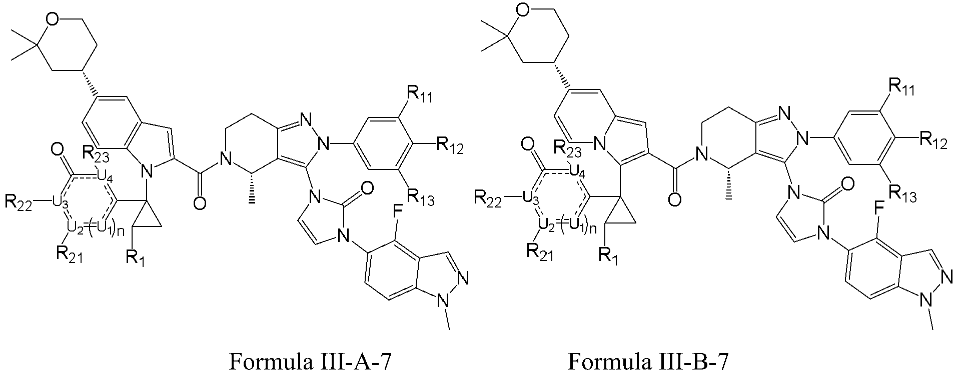 wherein,
R₁₁, R₁₂, and R₁₃ are each independently selected from the group consisting of H, substituted or unsubstituted C₁-C₆ alkyl, substituted or unsubstituted C₁-C₆ alkoxy, substituted or unsubstituted C₂-C₆ alkenyl, substituted or unsubstituted C₂-C₆ alkynyl, halogen, cyano, substituted or unsubstituted (3-10)-membered cycloalkyl, substituted or unsubstituted C₁-C₆ ester group, and substituted or unsubstituted C₁-C₆ amide group;
R₁ is selected from the group consisting of H, substituted or unsubstituted C₁-C₆ alkyl, and halogen;
n is 0 or 1;
U₁ is selected from N or CH; U₂, U₃, and U₄ are each independently selected from N, C, and O; R₂₁, R₂₂, and R₂₃ are each independently selected from the group consisting of absence, H, hydroxyl, substituted or unsubstituted C₁-C₆ alkyl, substituted or unsubstituted C₁-C₆ alkoxy, halogen, substituted or unsubstituted (3-10)-membered cycloalkyl, cyano, amino, carboxyl, substituted or unsubstituted C₁-C₆ ester group, substituted or unsubstituted C₁-C₆ amide group, substituted or unsubstituted C₁-C₆ aldehyde group, substituted or unsubstituted C₁-C₆ amine group, and O=; the dashed line connecting U₁, U₂, U₃, and U₄ is either absent or a bond, depending on the selection of U₁, U₂, U₃, U₄, R₂₁, R₂₂, and R₂₃;
the substituent of the alkyl, alkenyl, alkynyl, alkoxy, aldehyde group, amine group, ester group, and amide group is selected from the following groups: halogen, hydroxyl, cyano, C₁-C₆ alkyl, halogenated C₁-C₆ alkyl, C₁-C₆ alkoxy, substituted or unsubstituted (3-10)-membered cycloalkyl, substituted or unsubstituted (3-10)-membered heterocycloalkyl, substituted or unsubstituted (6-10)-membered aryl, substituted or unsubstituted (5-10)-membered heteroaryl, - C(O)R₁', and -NR₂'R₃';
the substituent of the cycloalkyl, heterocycloalkyl, aryl, and heteroaryl is selected from the following groups: C₁-C₆ alkyl, halogenated C₁-C₆ alkyl, halogen, and hydroxyl;
R₁', R₂', and R₃' are each independently selected from H and C₁-C₆ alkyl.

Further, 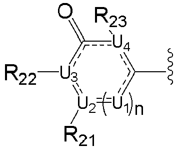 is selected from the group consisting of 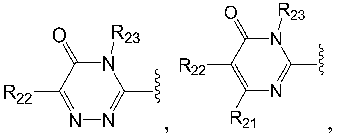 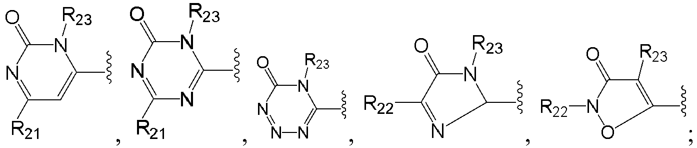
R₂₁, R₂₂, and R₂₃ are each independently selected from the group consisting of H, hydroxyl, substituted or unsubstituted C₁-C₆ alkyl, substituted or unsubstituted C₁-C₆ alkoxy, halogen, substituted or unsubstituted (3-10)-membered cycloalkyl, cyano, amino, carboxyl, substituted or unsubstituted C₁-C₆ ester group, substituted or unsubstituted C₁-C₆ amide group, substituted or unsubstituted C₁-C₆ aldehyde group, substituted or unsubstituted C₁-C₆ amine group, and O=;
the substituent of the alkyl, alkoxy, aldehyde group, amine group, ester group, and amide group is selected from the following groups: halogen, hydroxyl, cyano, C₁-C₆ alkyl, halogenated C₁-C₆ alkyl, and C₁-C₆ alkoxy;
the substituent of the cycloalkyl is selected from the following groups: C₁-C₆ alkyl, halogenated C₁-C₆ alkyl, halogen, and hydroxyl;

Further, 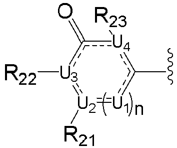 is selected from the group consisting of 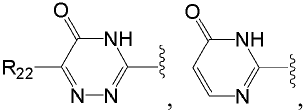 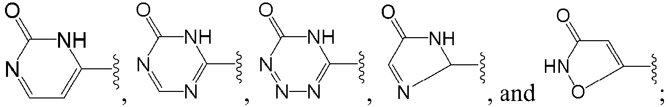 R₂₂ is selected from the group consisting of H, hydroxyl, C₁-C₃ alkyl, C₁-C₃ alkoxy, methyl substituted with 1-3 fluorines, halogen, (3-6)-membered cycloalkyl, cyano, amino, carboxyl, C₃ ester group, C₁ amide group, formaldehyde group, C₃ amine group, and O=.

Further, the structure of the compound is as represented by formula IV-7: 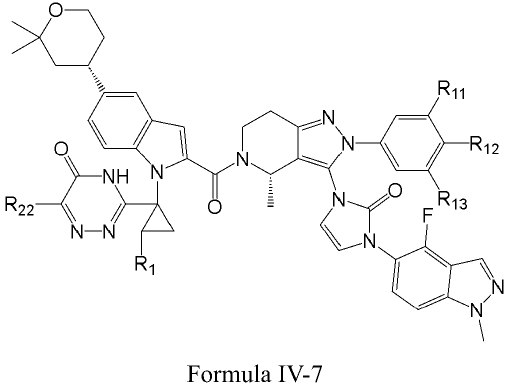 wherein,
R₁₁, R₁₂, and R₁₃ are each independently selected from the group consisting of H, substituted or unsubstituted C₁-C₆ alkyl, substituted or unsubstituted C₁-C₆ alkoxy, substituted or unsubstituted C₂-C₆ alkenyl, substituted or unsubstituted C₂-C₆ alkynyl, halogen, cyano, substituted or unsubstituted (3-10)-membered cycloalkyl, substituted or unsubstituted C₁-C₆ ester group, and substituted or unsubstituted C₁-C₆ amide group;
R₁ is selected from the group consisting of H, substituted or unsubstituted C₁-C₆ alkyl, and halogen;
R₂₂ is selected from the group consisting of H, hydroxyl, substituted or unsubstituted C₁-C₆ alkyl, substituted or unsubstituted C₁-C₆ alkoxy, halogen, substituted or unsubstituted (3-10)-membered cycloalkyl, cayano, amino, carboxyl, substituted or unsubstituted C₁-C₆ ester group, substituted or unsubstituted C₁-C₆ amide group, substituted or unsubstituted C₁-C₆ aldehyde group, substituted or unsubstituted C₁-C₆ amine group, and O=;
the substituent of the alkyl, alkenyl, alkynyl, alkoxy, aldehyde group, amine group, ester group, and amide group is selected from the following groups: halogen, hydroxyl, cyano, C₁-C₆ alkyl, halogenated C₁-C₆ alkyl, and C₁-C₆ alkoxy;
the substituent of the cycloalkyl is selected from the following groups: C₁-C₆ alkyl, halogenated C₁-C₆ alkyl, halogen, and hydroxyl;
preferably, R₂₂ is selected from the group consisting of H, hydroxyl, C₁-C₃ alkyl, C₁-C₃ alkoxy, methyl substituted with 1-3 fluorines, halogen, (3-6)-membered cycloalkyl, cyano, amino, carboxyl, C₃ ester group, C₁ amide group, formaldehyde group, C₃ amine group, and O=.

Further, the structure of the compound is as represented by formula V-7: 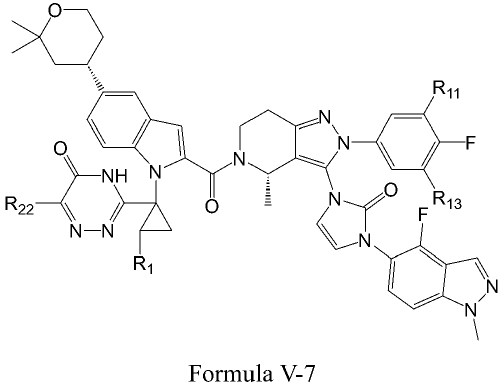 wherein,
R₁₁ and R₁₃ are each independently selected from the group consisting of H, substituted or unsubstituted C₁-C₆ alkyl, substituted or unsubstituted C₁-C₆ alkoxy, substituted or unsubstituted C₂-C₆ alkenyl, substituted or unsubstituted C₂-C₆ alkynyl, halogen, cyano, substituted or unsubstituted (3-10)-membered cycloalkyl, substituted or unsubstituted C₁-C₆ ester group, and substituted or unsubstituted C₁-C₆ amide group;
R₁ is selected from the group consisting of H, substituted or unsubstituted C₁-C₆ alkyl, and halogen;
R₂₂ is selected from the group consisting of H, hydroxyl, substituted or unsubstituted C₁-C₆ alkyl, substituted or unsubstituted C₁-C₆ alkoxy, halogen, substituted or unsubstituted (3-10)-membered cycloalkyl, cayano, amino, carboxyl, substituted or unsubstituted C₁-C₆ ester group, substituted or unsubstituted C₁-C₆ amide group, substituted or unsubstituted C₁-C₆ aldehyde group, substituted or unsubstituted C₁-C₆ amine group, and O=;
the substituent of the alkyl, alkenyl, alkynyl, alkoxy, aldehyde group, amine group, ester group, and amide group is selected from the following groups: halogen, hydroxyl, cyano, C₁-C₆ alkyl, halogenated C₁-C₆ alkyl, and C₁-C₆ alkoxy;
the substituent of the cycloalkyl is selected from the following groups: C₁-C₆ alkyl, halogenated C₁-C₆ alkyl, halogen, and hydroxyl;
preferably, R₂₂ is selected from the group consisting of H, hydroxyl, C₁-C₃ alkyl, C₁-C₃ alkoxy, methyl substituted with 1-3 fluorines, halogen, (3-6)-membered cycloalkyl, cyano, amino, carboxyl, C₃ ester group, C₁ amide group, formaldehyde group, C₃ amine group, and O=.

Further, the structure of the compound is as represented by formula V-7-1: 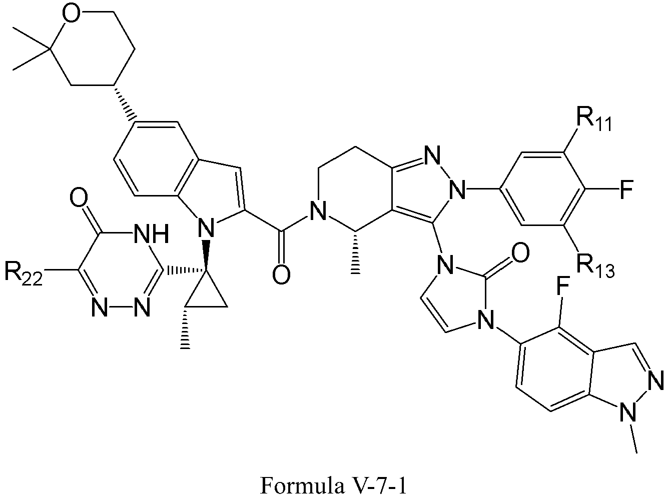 wherein,
R₁₁ and R₁₃ are each independently selected from the group consisting of C₁-C₆ alkyl;
R₂₂ is selected from the group consisting of 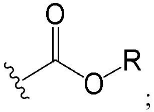 R is selected from the group consisting of C₁ alkyl, C₂ alkyl, C₃ alkyl, C₄ alkyl, C₅ alkyl, and C₆ alkyl.

Further, the compound is one of the following structures:

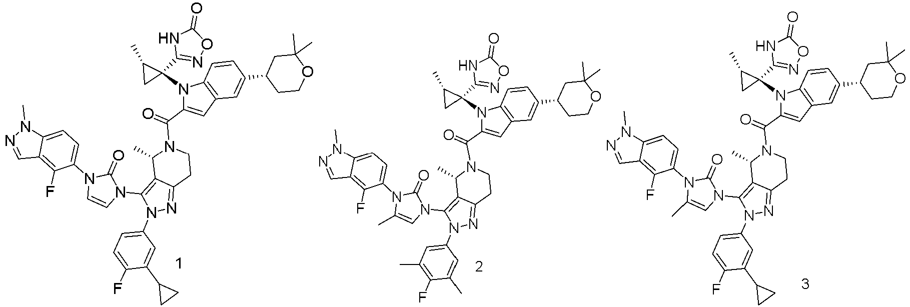

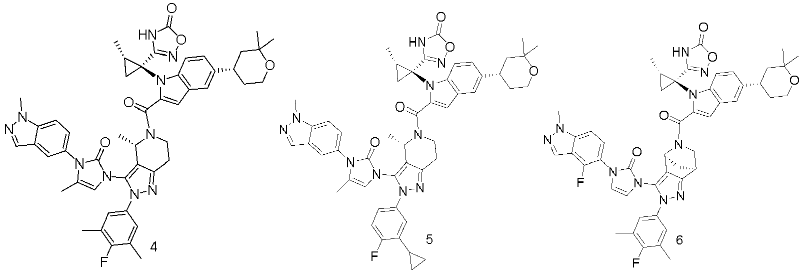

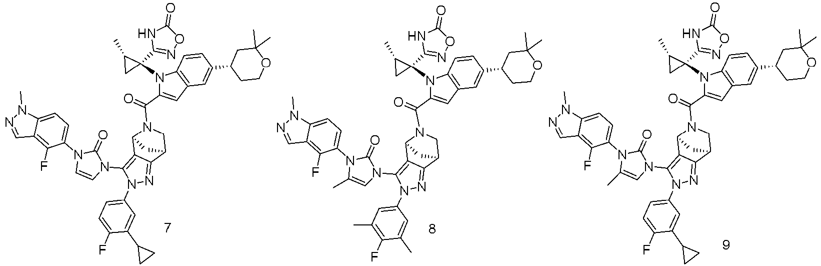

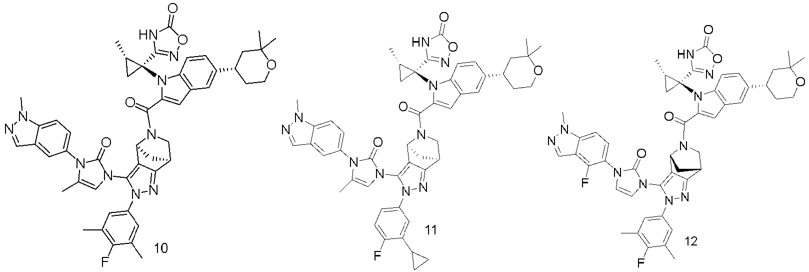

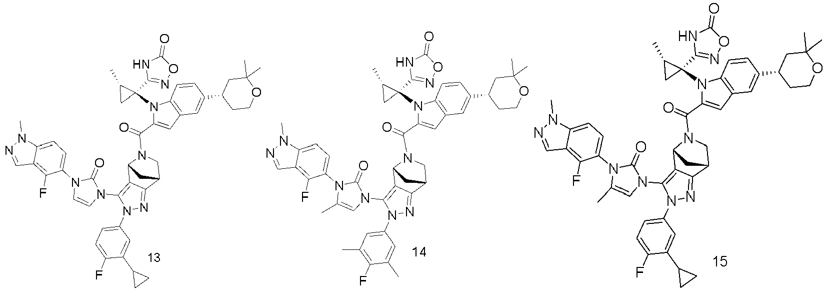

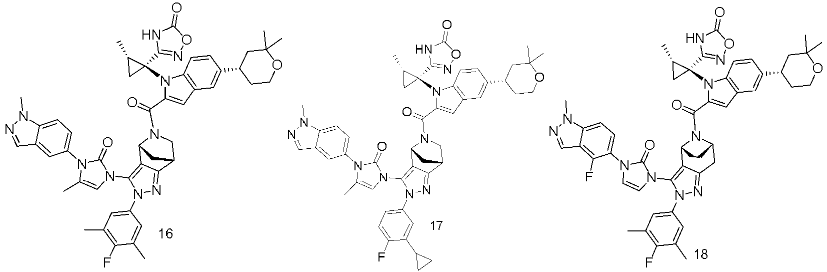

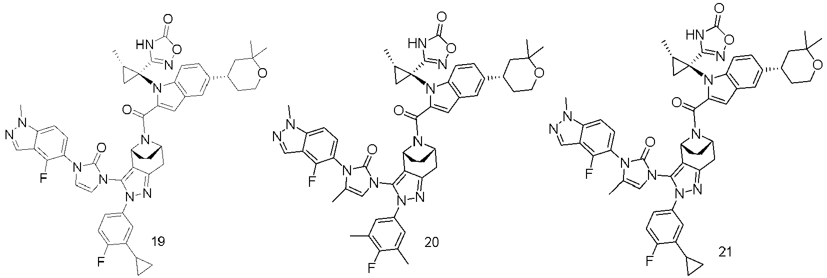

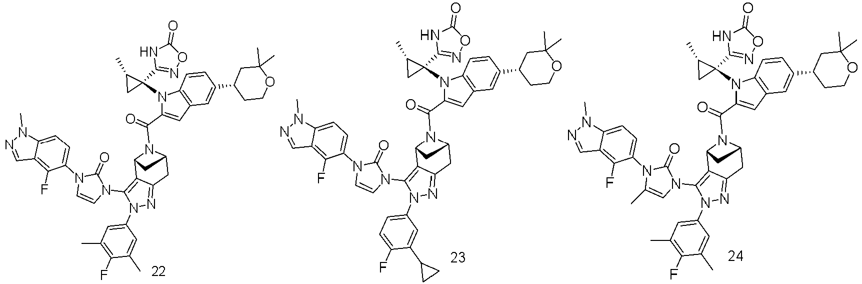

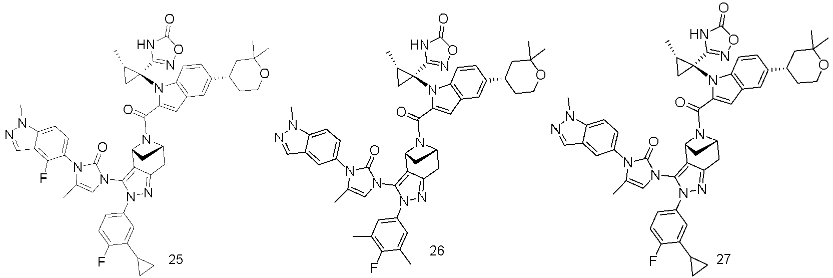

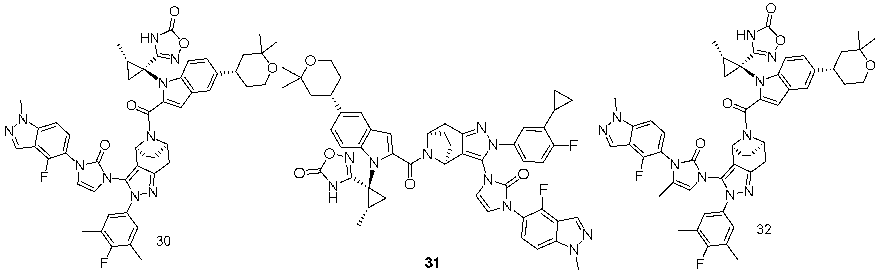

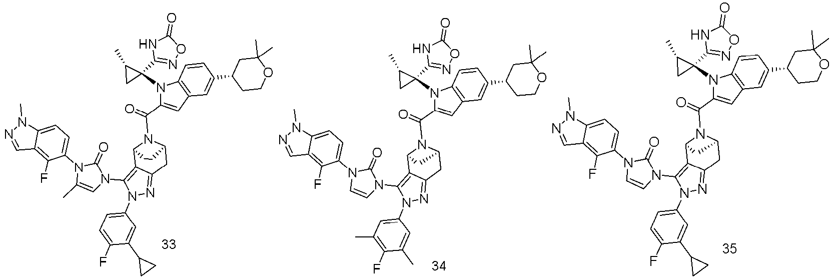

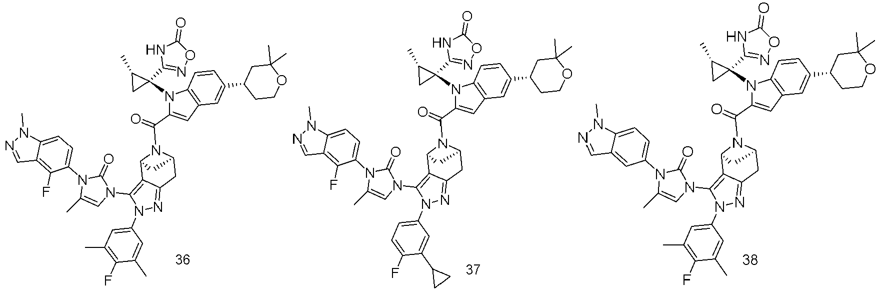

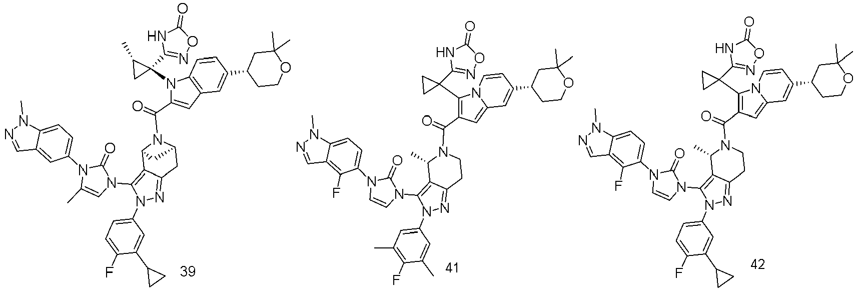

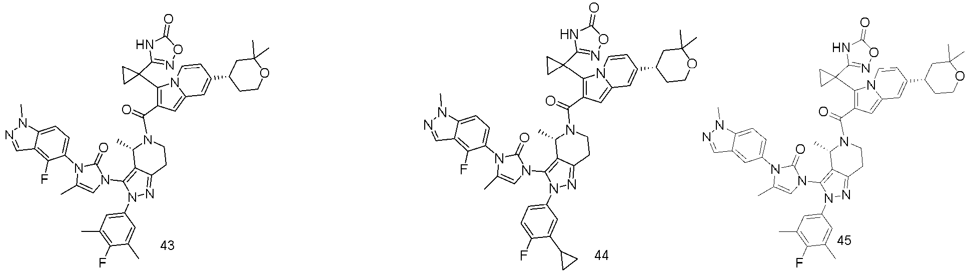

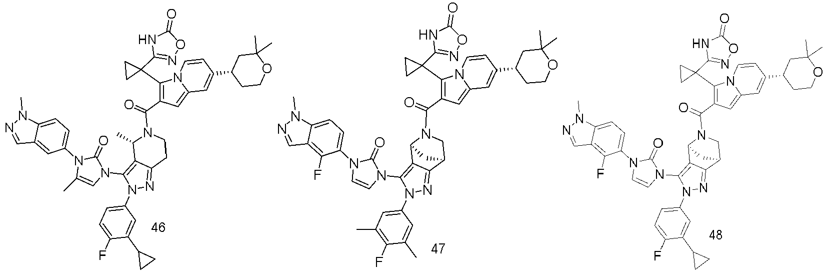

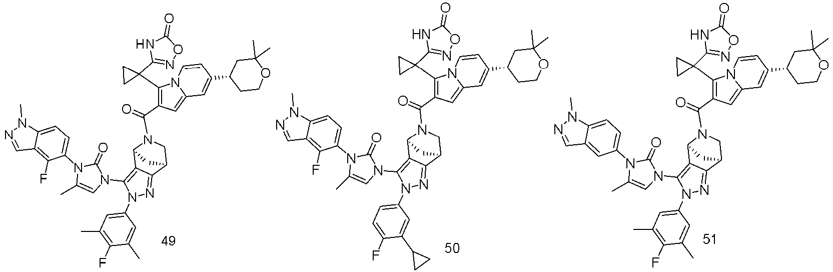

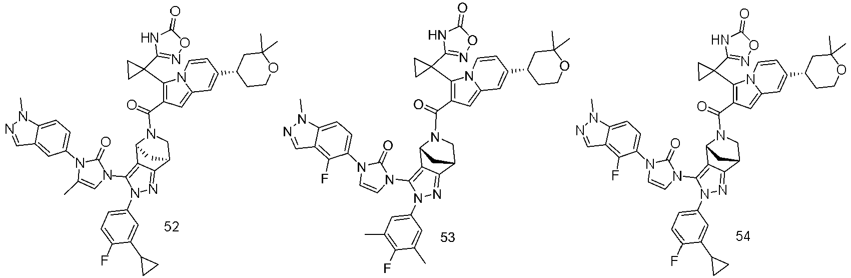

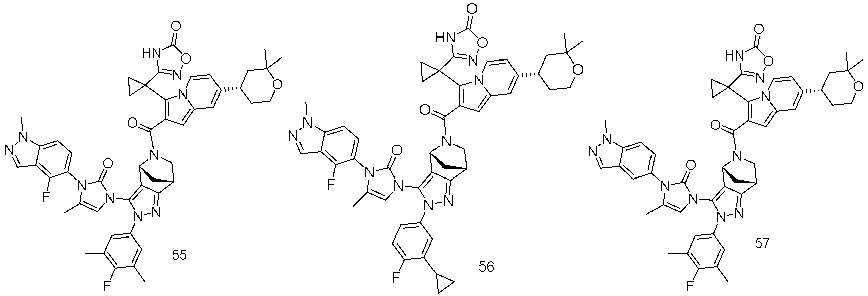

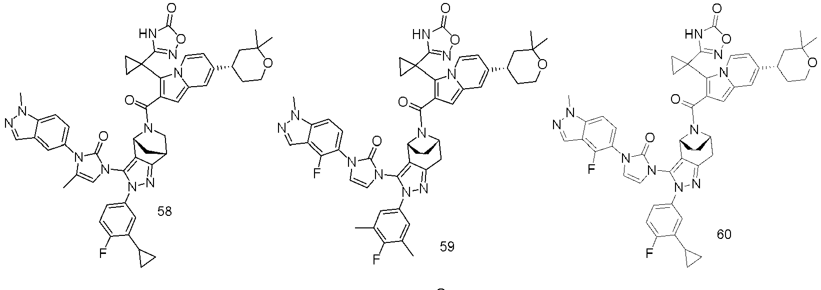

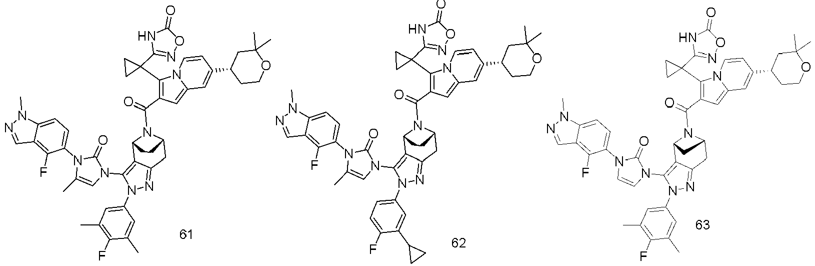

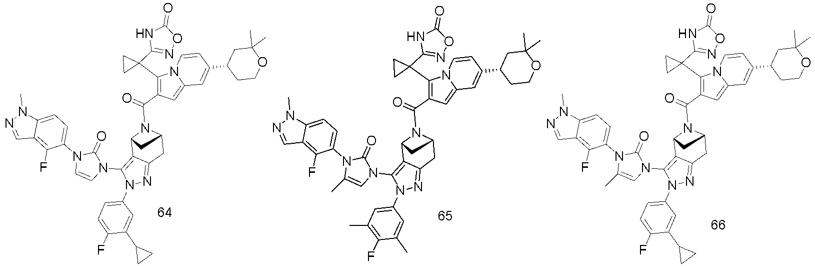

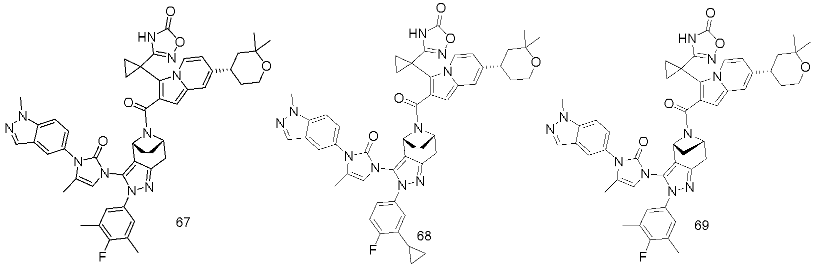

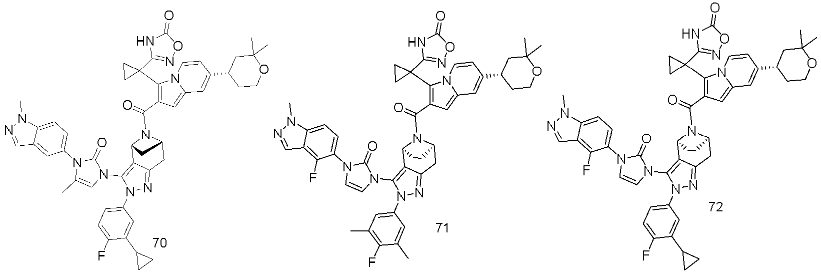

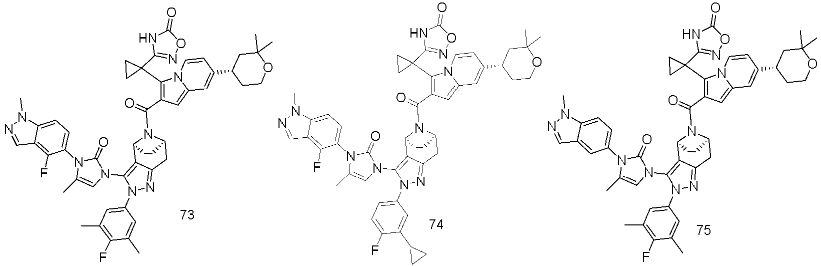

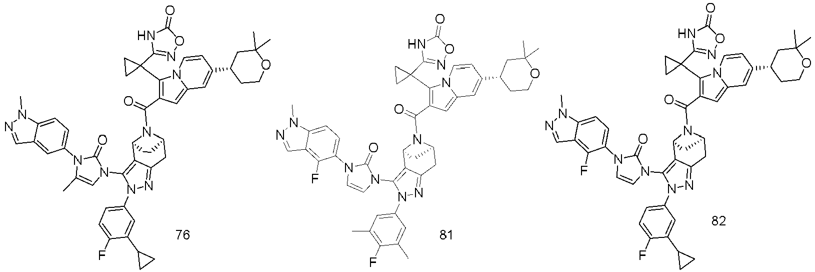

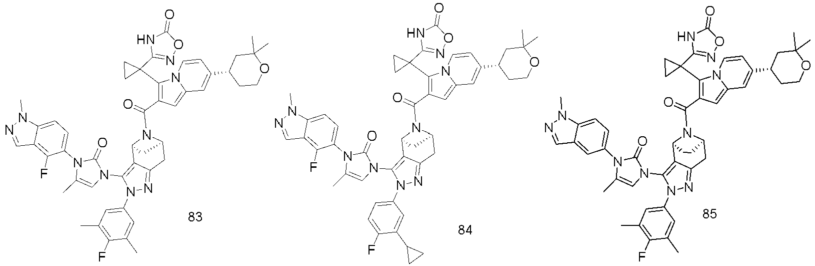

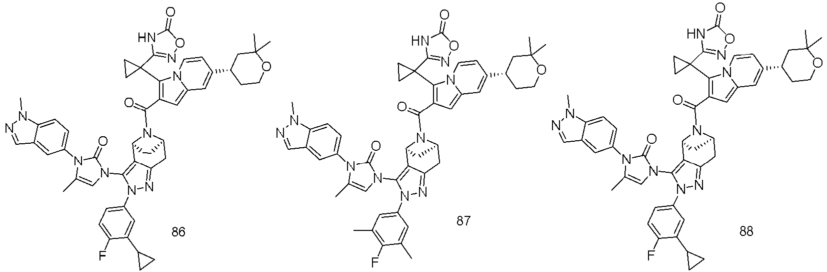

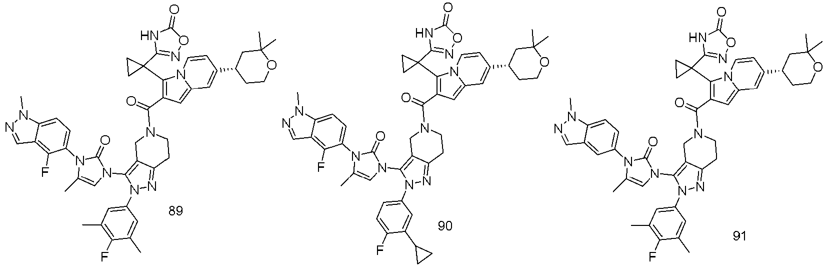

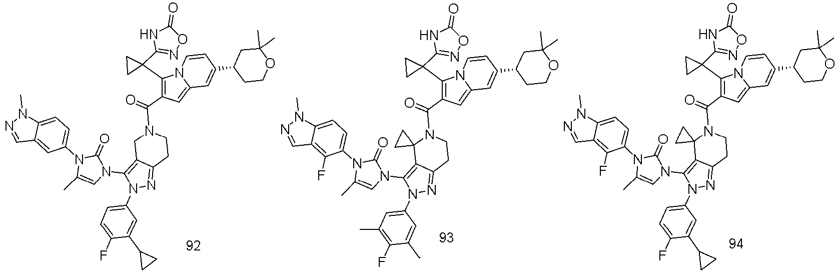

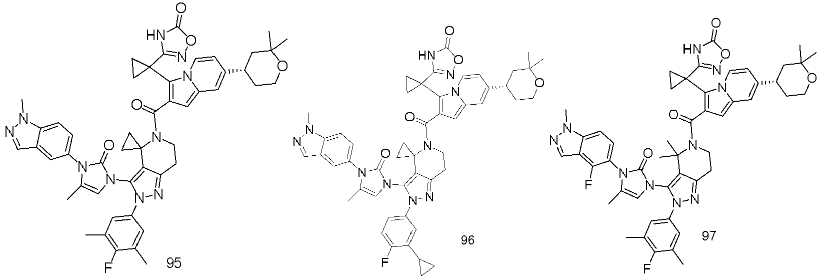

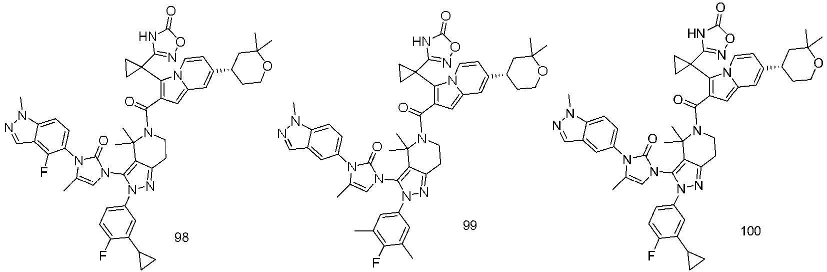

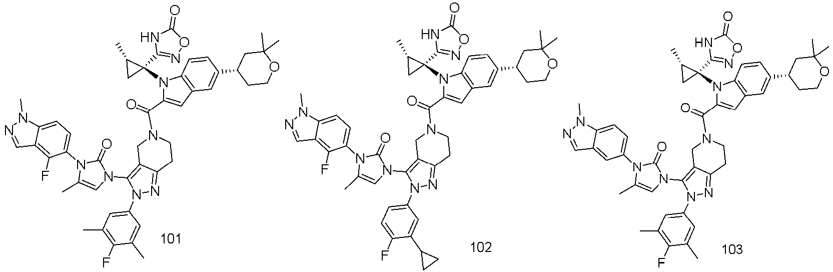

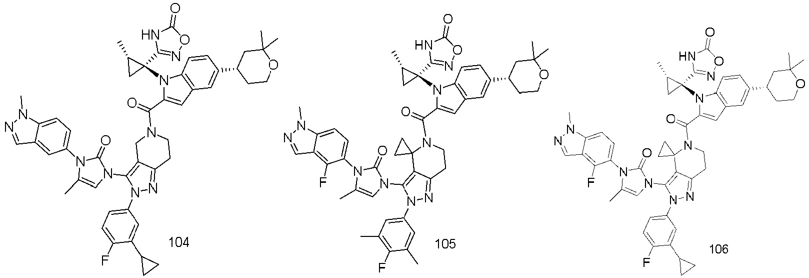

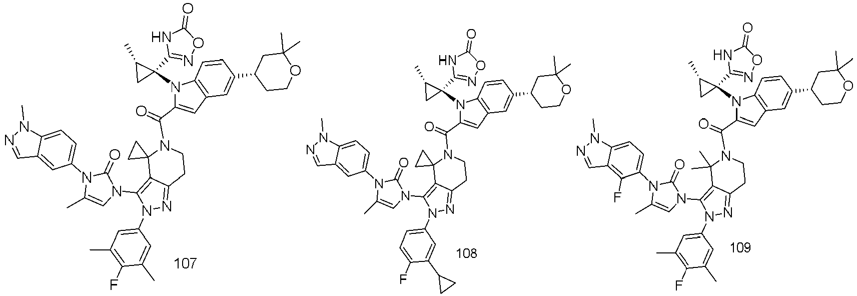

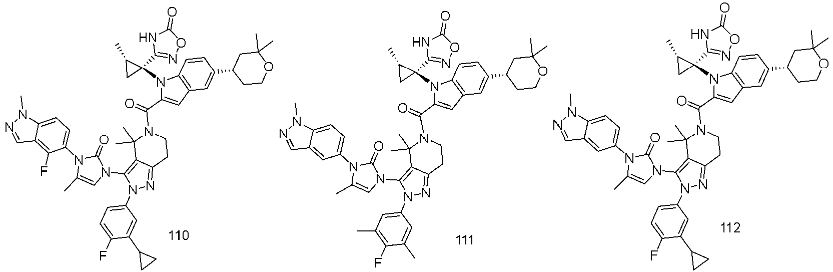

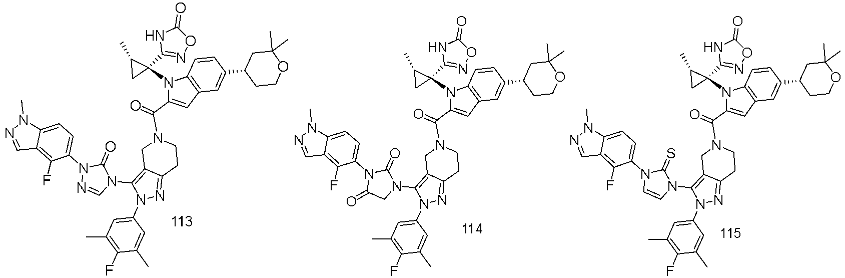

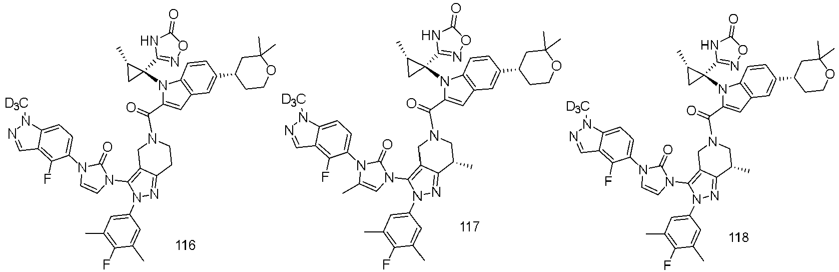

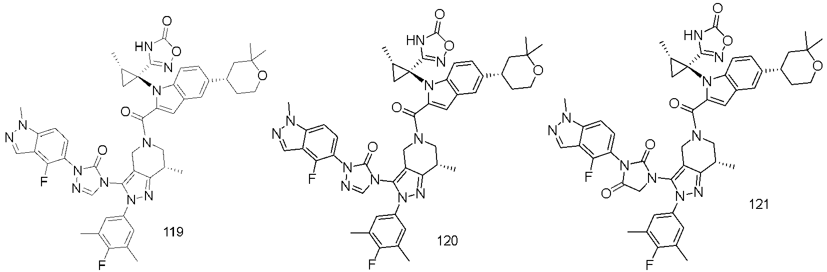

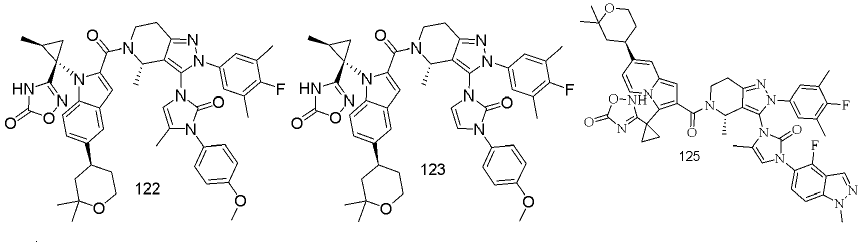

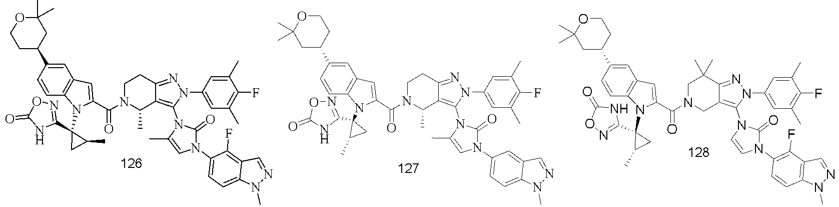

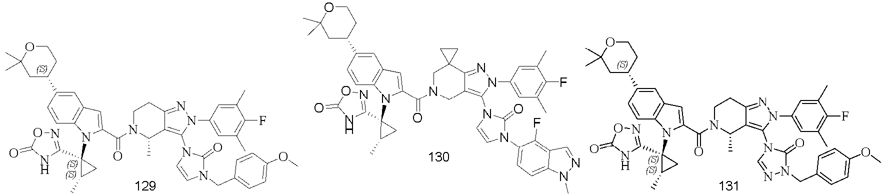

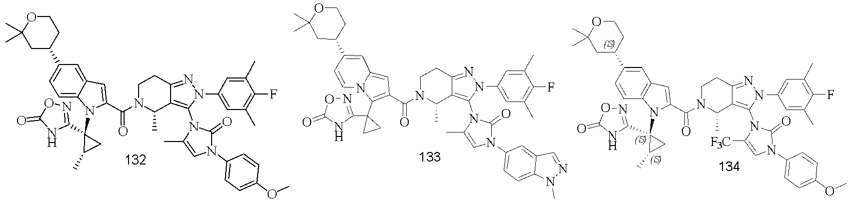

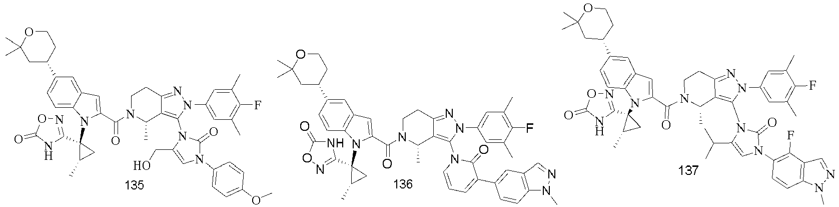

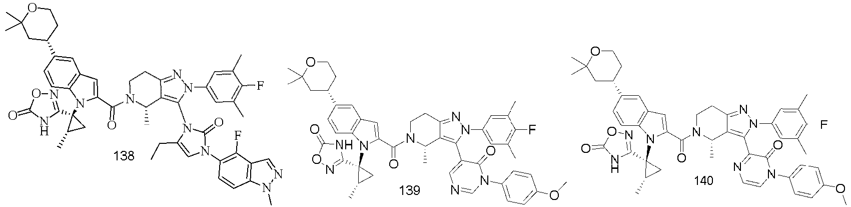

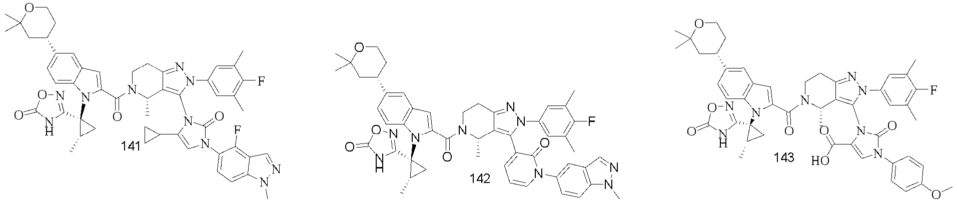

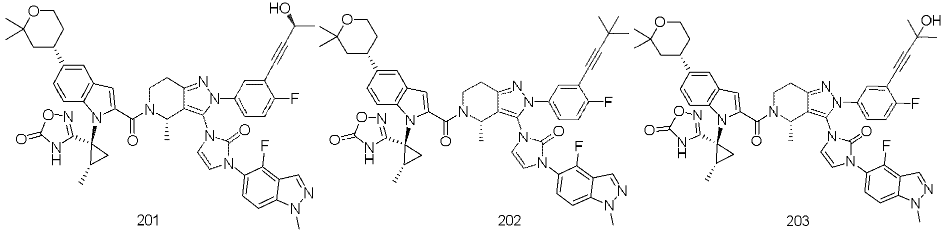

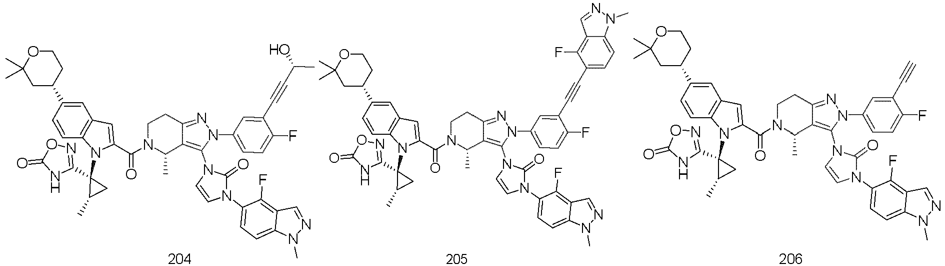

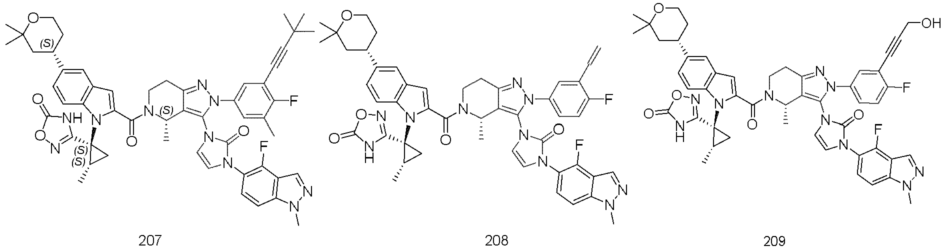

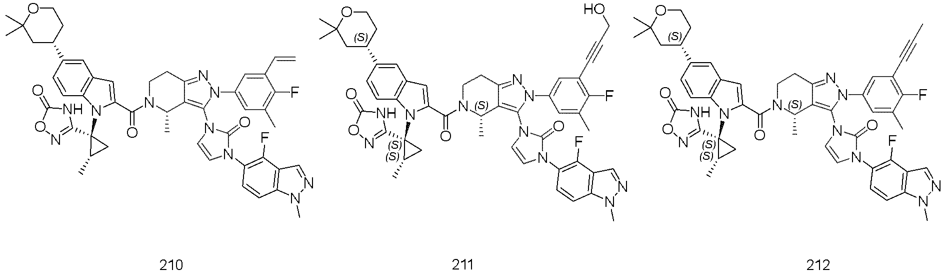

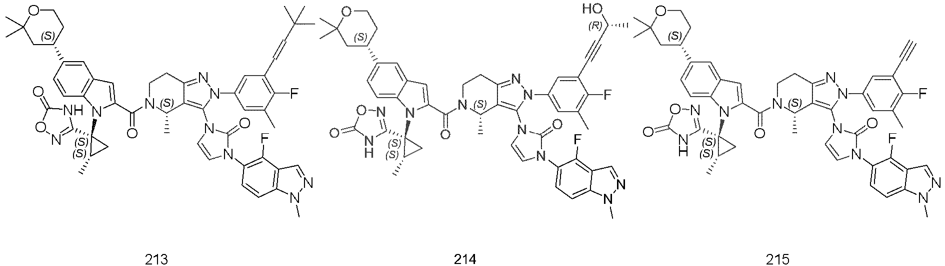

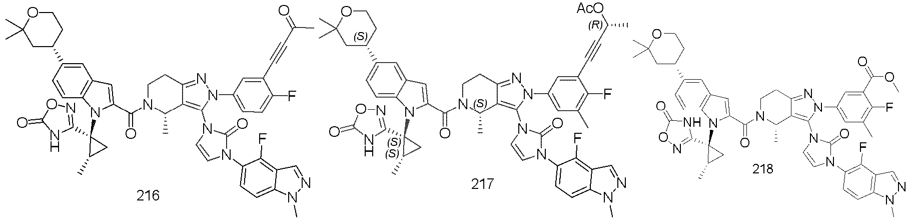

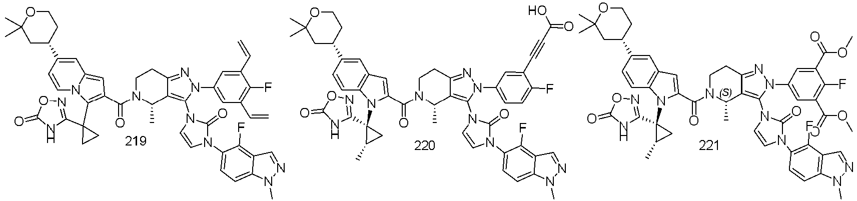

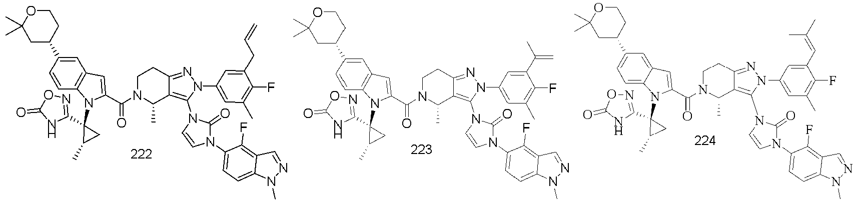

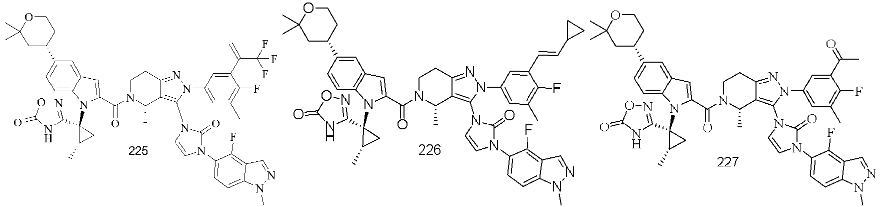

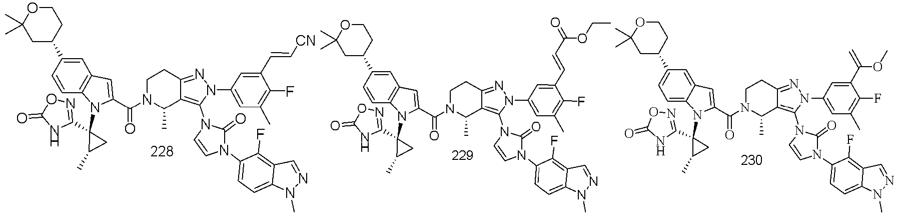

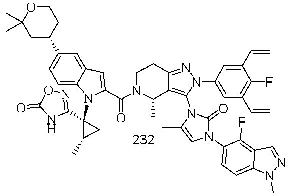

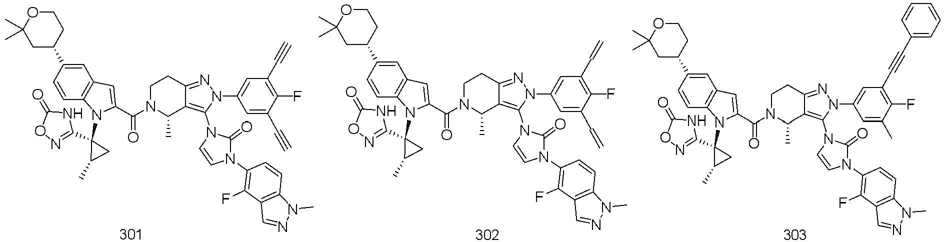

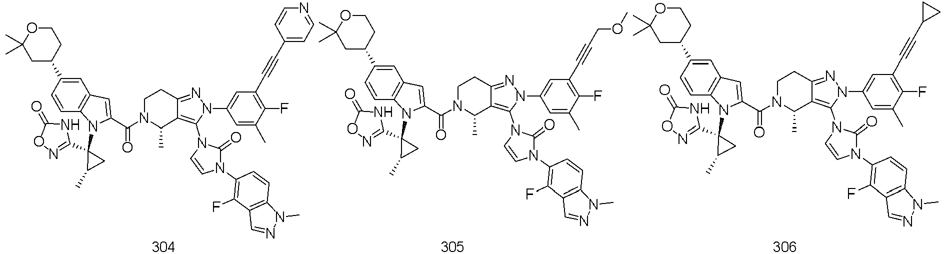

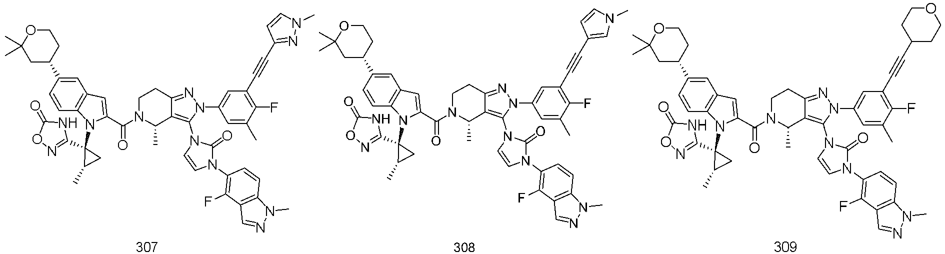

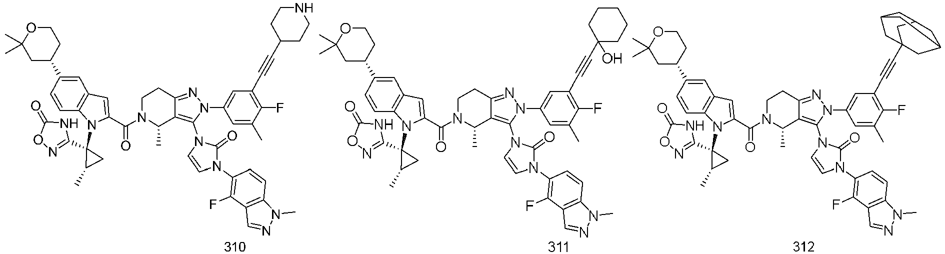

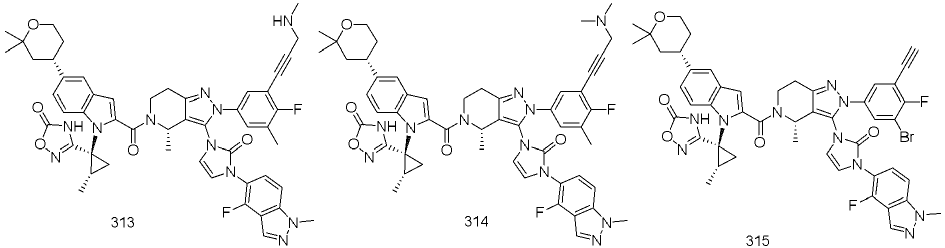

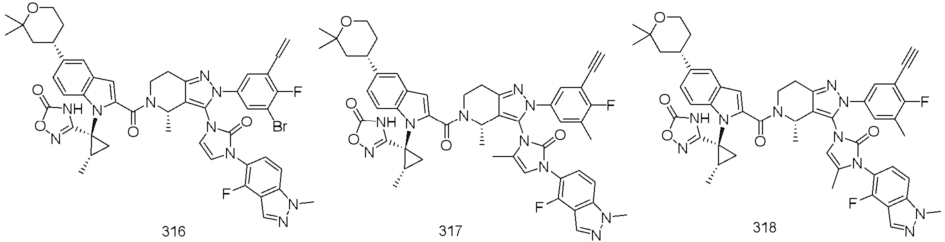

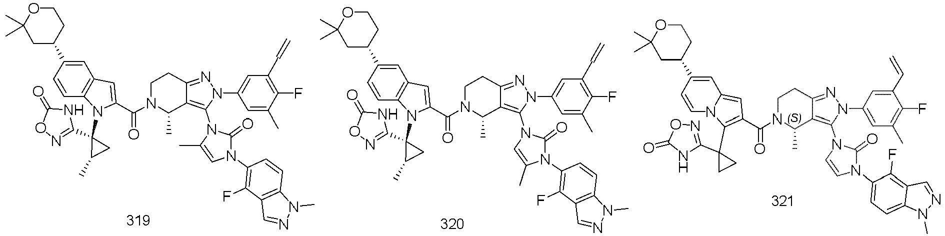

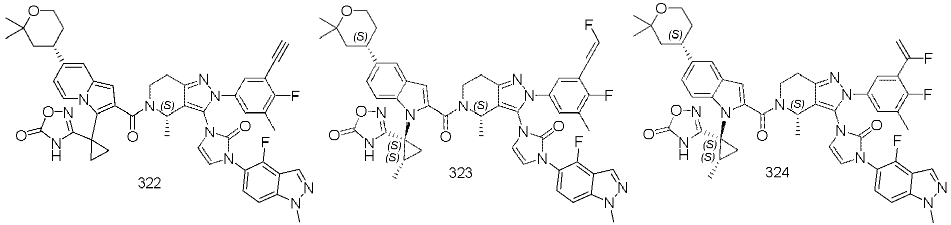

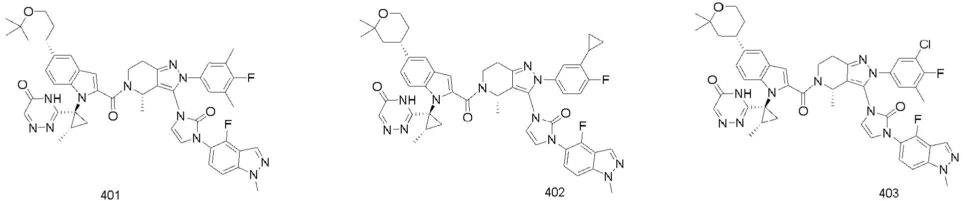

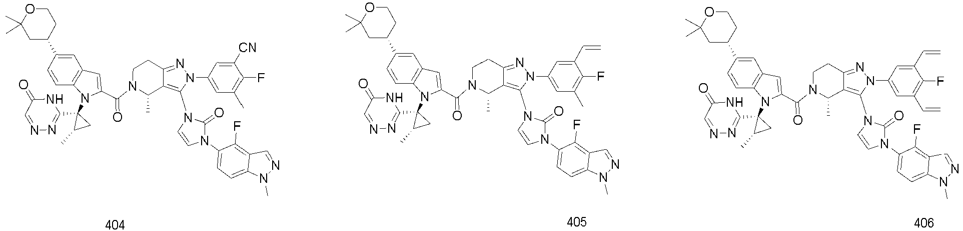

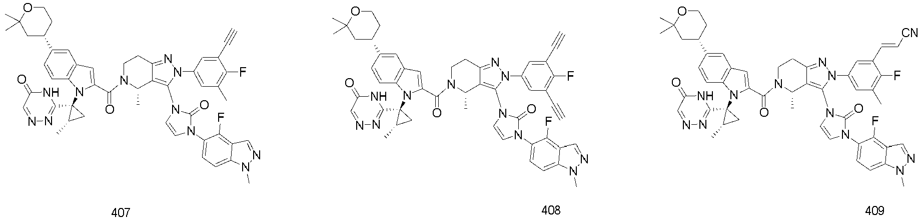

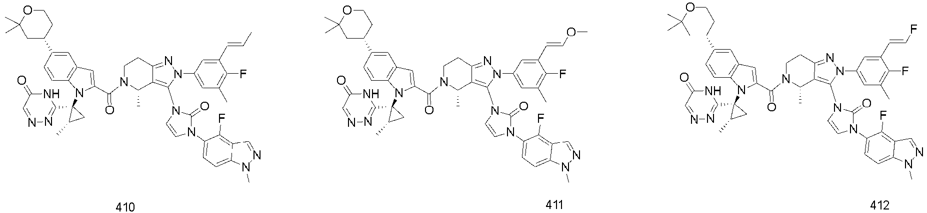

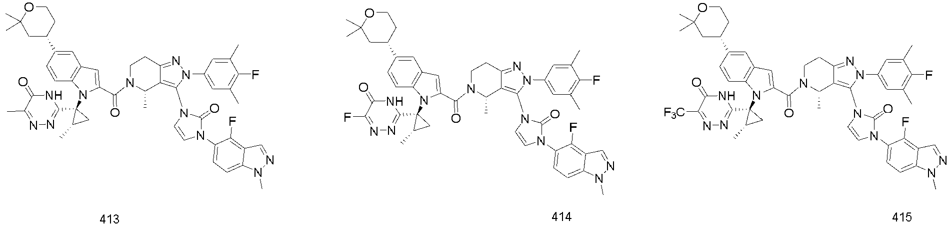

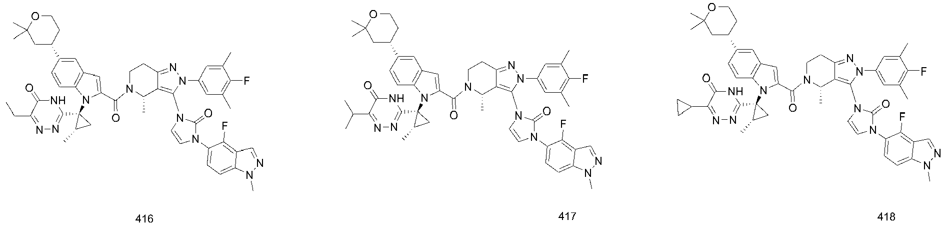

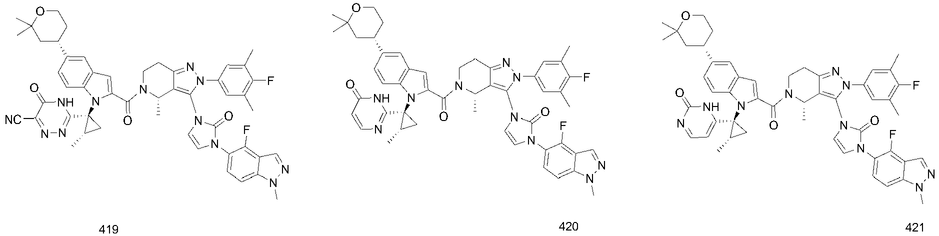

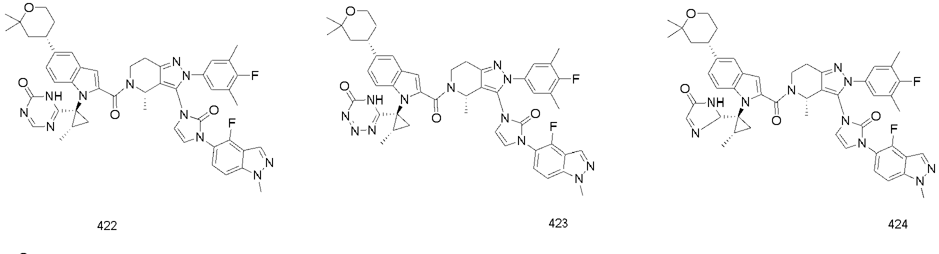

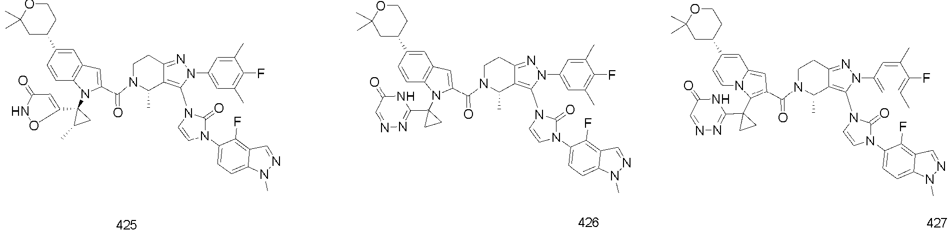

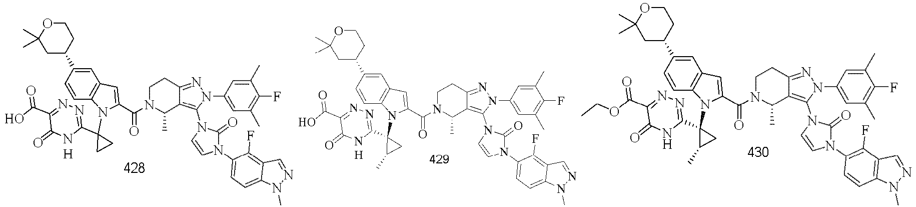

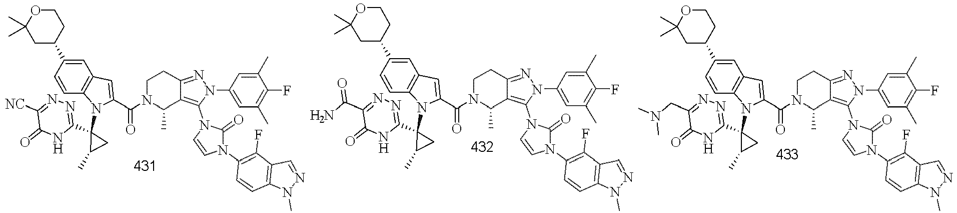

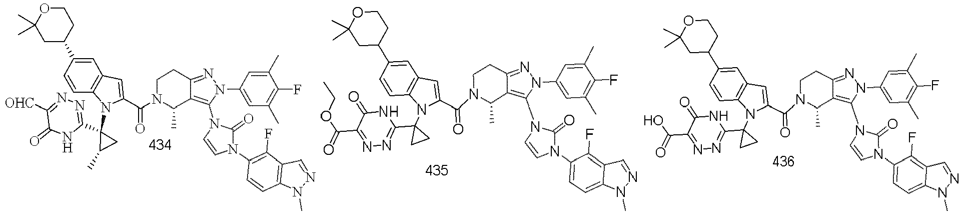

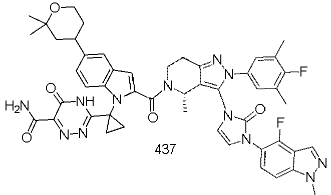

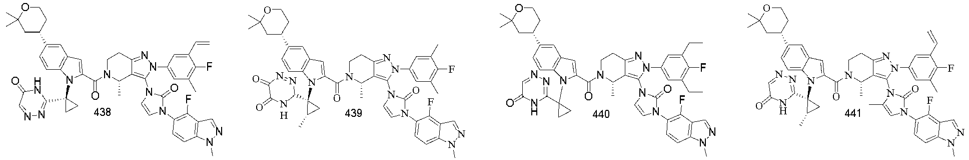

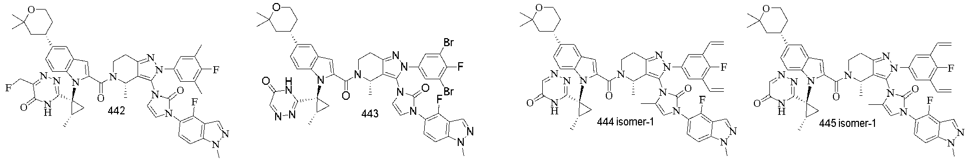

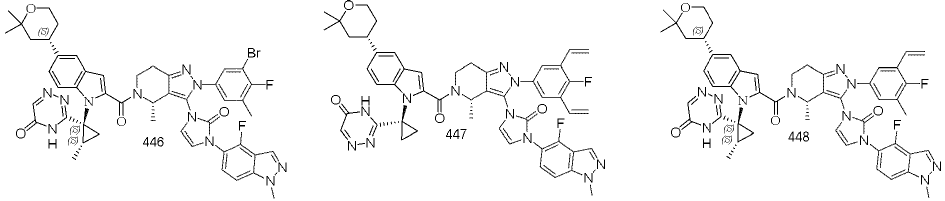

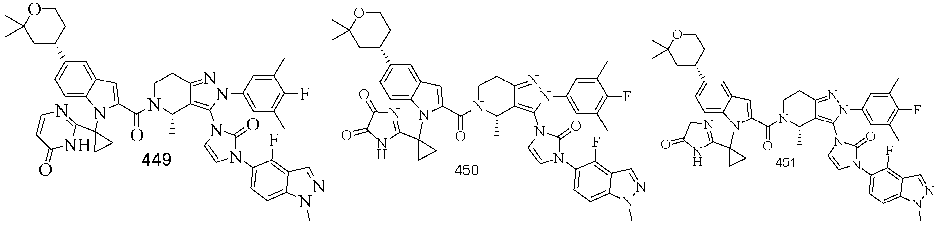

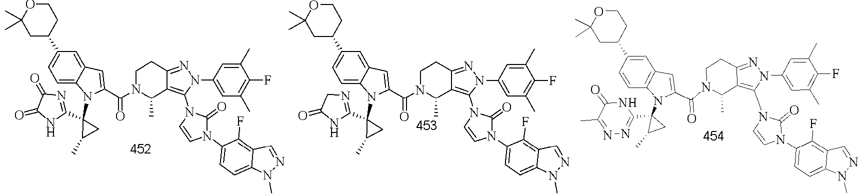

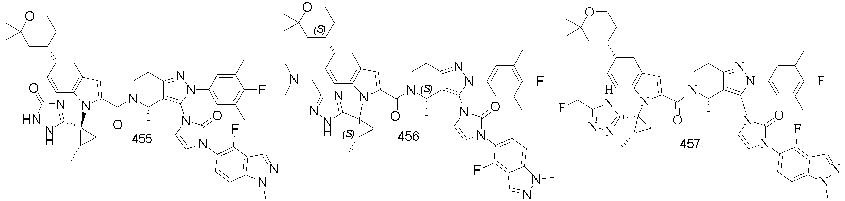

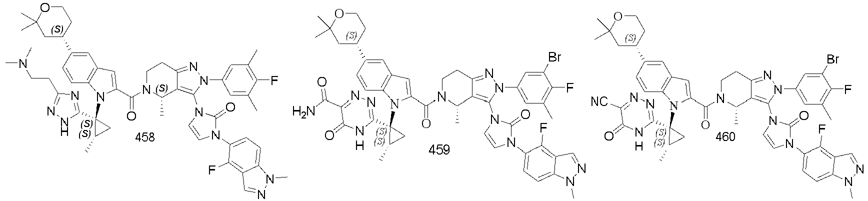

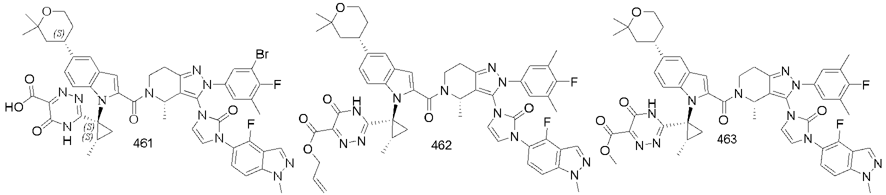

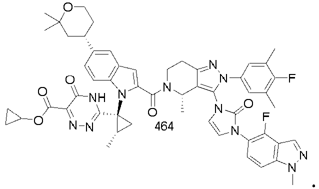

The present invention also provides the use of the above compound, a pharmaceutically acceptable salt thereof, or a stereoisomer thereof in the manufacture of a GLP-1 receptor agonist;

The present invention also provides the use of the above compound, a pharmaceutically acceptable salt thereof, or a stereoisomer thereof in the manufacture of a medicament for preventing and/or treating diseases associated with the GLP-1 receptor;
further, the diseases associated with the GLP-1 receptor are diabetes, obesity, fatty liver, and polycystic ovary syndrome;
preferably, the diabetes is type 2 diabetes.

The present invention also provides a pharmaceutical preparation, which is prepared using the above compound, a pharmaceutically acceptable salt thereof, or a stereoisomer thereof as the active ingredient, in combination with a pharmaceutically acceptable adjuvant or auxiliary ingredient.

The compounds and derivatives provided in the present invention can be named according to IUPAC (International Union of Pure and Applied Chemistry) or CAS (Chemical Abstracting Service, Columbus, OH) naming system.

For the definition of terms used in the present invention: unless defined otherwise, the initial definition provided for the group or term herein applies to the group or term of the whole specification; for the terms that are not specifically defined herein, they should have the same meaning as commonly understood by one of ordinary skill in the art to which the disclosure belongs.

"Substitution" means that the hydrogen in a molecule is substituted with other different atoms or molecules.

The minimum and maximum carbon atom content in a hydrocarbon group is indicated by a prefix. For example, the prefix Cₐ-C_{b} alkyl indicates any alkyl containing "a" to "b" carbon atoms. Therefore, for example, "C₁-C₆ alkyl" refers to alkyls containing 1-6 carbon atoms, specifically C₁, C₂, C₃, C₄, C₅, and C₆ alkyls; "C₁-C₆ alkoxy" refers to alkoxys containing 1-6 carbon atoms, specifically C₁, C₂, C₃, C₄, C₅, and C₆ alkoxys.

"Alkyl" refers to a saturated hydrocarbon chain with a specified number of carbon atoms. For example, C₁-C₆ alkyl refers to alkyls with 1-6 carbon atoms, namely 1, 2, 3, 4, 5, or 6 carbon atoms. Alkyl groups can be straight-chain or branched. Representative branched alkyl groups have one, two, or three branches. Alkyl includes methyl, ethyl, propyl (n-propyl and isopropyl), butyl (n-butyl, isobutyl, and tert-butyl), pentyl (n-pentyl, isopentyl, and neopentyl), hexyl, and so on.

"Alkenyl" refers to an aliphatic hydrocarbon group containing at least one carbon-carbon double bond. Alkenyl groups can be either straight-chain or branched.

"Alkynyl " refers to an aliphatic hydrocarbon group containing at least one carbon-carbon triple bond. Alkynyl can be either straight-chain or branched.

"Halogen" refers to fluorine, chlorine, bromine, or iodine.

"Cycloalkyl" refers to a saturated or unsaturated all-carbon monocyclic or polycyclic ring (including fused, spiro, or bridged rings) without a conjugated π-electron system, including but not limited to: 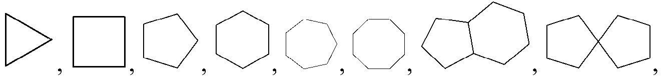 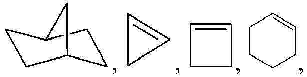 and the same.

"Heterocycloalkyl" refers to a cycloalkyl in which at least one carbon atom in the ring is replaced by a heteroatom, such as O, N, or S. It is a saturated or unsaturated monocyclic or polycyclic ring system (including fused, spiro, or bridged rings) without conjugated π-electron systems. Examples include, but are not limited to: 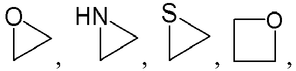 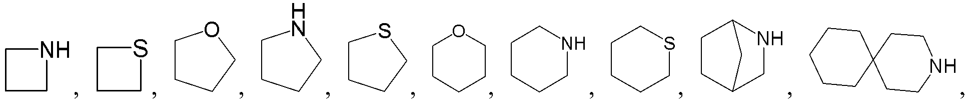 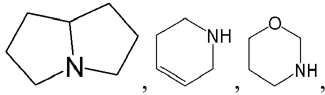 and so on.

"Aryl" refers to a fully carbon-based monocyclic or polycyclic ring (including fused, spiro, or bridged rings) with a conjugated π-electron system, such as but not limited to: phenyl, naphthyl, phenanthryl, anthryl, fluorenyl, and indenyl. The aromatic ring can be fused with other cyclic groups (including saturated and unsaturated rings), but it must not contain heteroatoms such as O, N, or S. Additionally, the point of attachment to the parent structure must be on a carbon atom in the ring with a conjugated π-electron system, such as but not limited to 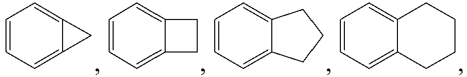 and so on.

"Heteroaryl" refers to an aryl with at least one carbon atom in the conjugated π-electron system replaced by a heteroatom, such as O, N, or S. Examples include, but are not limited to, thiophenyl, furanyl, and isothiazolyl.

"Ester group" refers to a group linked to an attachment site via a carbon chain, and the carbon chain contains at least one ester bond (i.e., -COO-). Its structure is 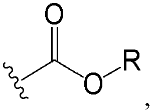 wherein R represents C₁-C₆ alkyl.

"Amide group" refers to a group that is linked to an attachment site via a carbon chain, and the carbon chain contains at least one amide bond (i.e., -CONH-). The structure is 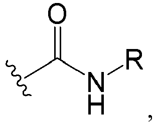 wherein R represents C₁-C₆ alkyl.

"Amino" refers to a group that is linked to an attachment site via a carbon chain or N, and the carbon chain contains at least one N.

"Aldehyde group" refers to a group that is linked to an attachment site via a carbon chain, and the carbon chain contains at least one -CHO.

The substituent is selected from "O=", indicating that the oxygen atom is linked to the attachment site via a double bond.

In the rings containing X₁, X₂, X₃, and X₄, as well as the rings containing Y₁, Y₂, Y₃, and Y₄, provided that a dashed line represents absence, it indicates a single bond; provided that a dashed line represents a bond, it indicates a double bond.

Provided that R₁₂, R₁₃, and R₁₄ are absent, it is indicated that no group is attached.

In the present invention, the pharmaceutically acceptable salts include acetate, adipate, aspartate, benzoate, benzenesulfonate, bicarbonate, carbonate, bisulfate, sulfate, borate, camphorsulfonate, citrate, cyclohexanesulfonate, ethanedisulfonate, ethanesulfonate, formate, fumarate, glucoheptonate, gluconate, glucuronate, hexafluorophosphate, hydrochloride, hydrobromide, hydroiodide, hydroxyethylsulfonate, lactate, malate, maleate, malonate, methanesulfonate, methylsulfate, naphthoate, naphthalenesulfonate, nicotinate, nitrate, orotate, oxalate, palmitate, pamoate, phosphate, hydrophosphate, dihydrogen phosphate, pyroglutamate, saccharate, stearate, succinate, tannate, tartrate, toluenesulfonate, trifluoroacetate, xinafoate, methanesulfonate, p-toluenesulfonate, quaternary ammonium salt, or succinate, and so on.

Compared with the prior art, the beneficial effects of the present invention are as follows:
The present invention provides a novel class of small molecule compounds. The small molecule compounds of the present invention can serve as GLP-1 receptor agonists for the manufacture of medicaments for preventing and/or treating diseases such as diabetes, obesity, fatty liver, and polycystic ovary syndrome. Compared to LY3502970, the compounds of the present invention exhibit superior pharmaceutical properties, specifically manifested in better pharmacokinetic (PK) properties, improved oral bioavailability, reduced toxicity, enhanced permeability, and improved efficacy. Therefore, the compounds of the present invention hold broad application prospects.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Examples

The starting materials and equipment used in the specific examples of the present invention are all known products obtained by synthesizing via literature method or purchasing those commercially available.

The synthesis method of some general intermediates of the present invention was as follows:
1. Synthesis of compound tert-butyl (S)-2-(3-bromo-4-fluoro-5-methylphenyl)-3-(3-(4-fluoro-1-methyl-1H-indazole-5-yl)-2-oxo-2,3-dihydro-1H-imidazole-1-yl)-4-methyl-2,4,6,7-tetrahydro-5H-pyrazolo[4,3-c]pyridine-5-carboxylate (**Int 1**)

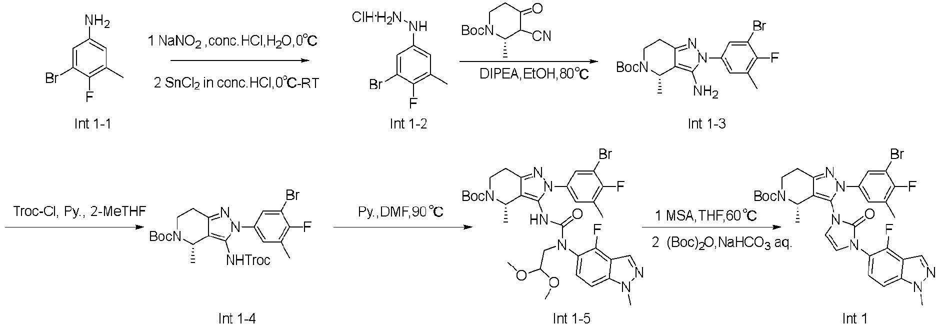

### Synthesis of intermediate (3-bromo-4-fluoro-5-methylphenyl)hydrazine hydrochloride (Int 1-2)

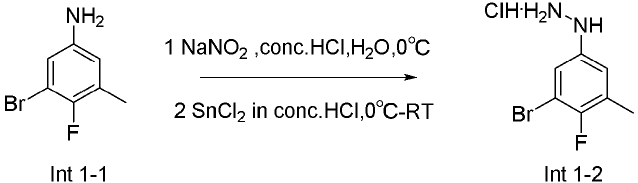

To a reaction flask, were added 3-bromo-4-fluoro-5-methylaniline (**Int 1-1,** 7.8 g, 38 mmol), water (78 mL) and concentrated hydrochloric acid (40 mL), and then NaNO₂ (3.97 g, 57 mmol) aqueous solution (8 mL) was added dropwise at 0 °C. After that, the reaction was stirred at 0 °C for 1 hour. Then, at the same temperature, a concentrated hydrochloric acid solution (40 mL) of SnCl₂ (21 g, 110 mmol) was added dropwise. Subsequently, the reaction was stirred at room temperature for 2 h, and filtered. The filter cake was dried, and then triturated in ethyl acetate (20 mL). After filtration, the crude solid compound **Int 1-2** (9.9 g, 39 mmol) was obtained, with a yield of 103%. Ms: 219, 221 (M+H⁺)

### Synthesis of intermediate tert-butyl (S)-3-amino-2-(3-bromo-4-fluoro-5-methylphenyl)-4-methyl-2,4,6,7-tetrahydro-5H-pyrazolo[4,3-c]pyridine-5-carboxylate (Int 1-3)

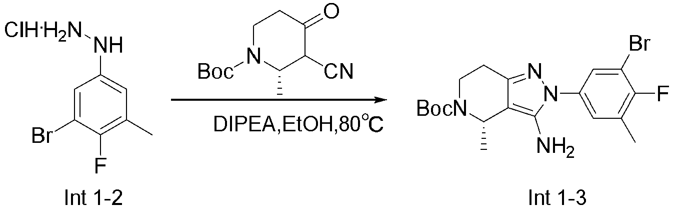

To a reaction flask, were added (3-bromo-4-fluoro-5-methylphenyl)hydrazine hydrochloride **Int 1-2** (9.9 g, 39 mmol), tert-butyl (2S)-3-cyano-2-methyl-4-oxopiperidine-1-carboxylate (8.31 g, 35 mmol), N,N-diisopropylethylamine (9 g, 69 mmol), and ethanol (100 mL). The solution was heated to 80 °C and stirred overnight. After the reaction was complete, the solution was cooled to room temperature, and then water (100 mL) was added. The resultant solution was extracted with ethyl acetate (100 mL x 3). The organic layers were combined, and dried over anhydrous sodium sulfate. The solvent was rotary evaporated. The residue was purified via column chromatography to obtain the solid compound **Int 1-3** (7 g, 16 mmol), with a yield of 46.6%. Ms: 439, 441 (M+H⁺)

### Synthesis of intermediate tert-butyl (S)-2-(3-bromo-4-fluoro-5-methylphenyl)-4-methyl-3-(((2,2,2-trichloroethoxy)carbonyl)amino)-2,4,6,7-tetrahydro-5H-pyrazolo[4,3-c]pyridine-5-carboxylate (Int 1-4)

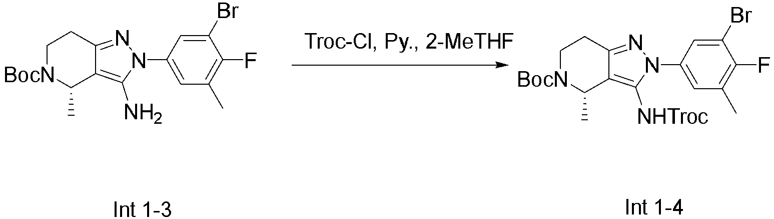

To a reaction flask, were added tert-butyl (S)-3-amino-2-(3-bromo-4-fluoro-5-methylphenyl)-4-methyl-2,4,6,7-tetrahydro-5H-pyrazolo[4,3-c]pyridine-5-carboxylate **Int 1-3** (4.9 g, 11.2 mmol), pyridine (2.7 g, 33.6 mmol), and 2-methyltetrahydrofuran (50 mL), and then 2,2,2-trichloroethyl chloroformate (3.6 g, 16.8 mmol) were added dropwise in an ice bath. After that, the reaction was continued. Upon completion of the reaction, water (50 mL) was added, followed by extraction with ethyl acetate (50 mL x 3). The organic layers were combined, and dried over anhydrous sodium sulfate. The solvent was rotary evaporated. The residue was purified via column chromatography to obtain the solid compound **Int1-4** (6 g, 9.8 mmol), with a yield of 88%. Ms: 613, 615, 617 (M+H⁺)

### Synthesis of intermediate tert-butyl (S)-2-(3-bromo-4-fluoro-5-methylphenyl)-3-(3-(2,2-dimethoxyethyl)-3-(4-fluoro-1-methyl-1H-indazole-5-yl)ureido)-4-methyl-2,4,6,7-tetrahydro-5H-pyrazolo[4,3-c]pyridine-5-carboxylate (Int 1-5)

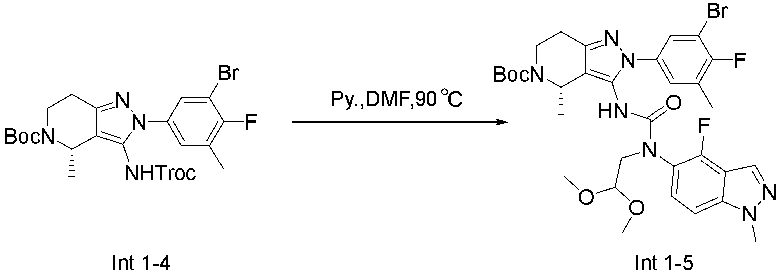

To a reaction flask, were added tert-butyl (S)-2-(3-bromo-4-fluoro-5-methylphenyl)-4-methyl-3-(((2,2,2-trichloroethoxy)carbonyl)amino)-2,4,6,7-tetrahydro-5H-pyrazolo[4,3-c]pyridine-5-carboxylate **Int 1-4** (6 g, 9.8 mmol), N-(2,2-dimethoxyethyl)-4-fluoro-1-methyl-1H-indazole-5-amine (5 g, 19.8 mmol), pyridine (23.2 g, 294 mmol), and N,N-dimethylformamide (120 mL). The solution was heated to 90 °C for reaction. After completion of the reaction, the solution was cooled to room temperature, and then ethyl acetate (200 mL) was added. The resultant solution was extracted with water (120 mL x 5), and the organic layer was dried over anhydrous sodium sulfate. After the solvent was removed by rotary evaporation, the residue was purified via column chromatography to obtain solid compound **Int 1-5** (5.4 g, 7.5 mmol), with a yield of 77%. Ms: 718, 720 (M+H⁺)

### Synthesis of compound tert-butyl (S)-2-(3-bromo-4-fluoro-5-methylphenyl)-3-(3-(4-fluoro-1-methyl-1H-indazole-5-yl)-2-oxo-2,3-dihydro-1H-imidazole-1-yl)-4-methyl-2,4,6,7-tetrahydro-5H-pyrazolo[4,3-c]pyridine-5-carboxylate (Int 1)

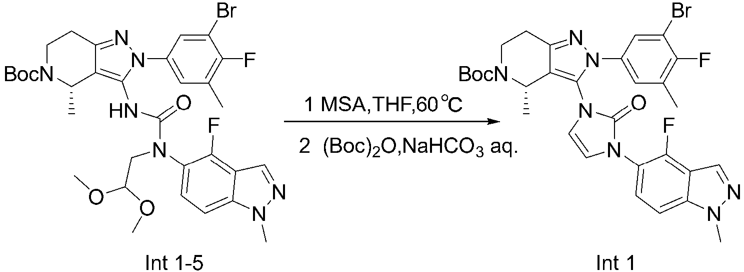

To a reaction flask, were added tert-butyl (S)-2-(3-bromo-4-fluoro-5-methylphenyl)-3-(3-(2,2-dimethoxyethyl)-3-(4-fluoro-1-methyl-1H-indazole-5-yl)ureido)-4-methyl-2,4,6,7-tetrahydro-5H-pyrazolo[4,3-c]pyridine-5-carboxylate **Int 1-5** (5.4 g, 7.5 mmol) and tetrahydrofuran (60 mL), and then at room temperature, methanesulfonic acid (7.4 g, 75 mmol) was added. The solution was heated to 60 °C for reaction. After the reaction was complete, the solution was cooled to room temperature. NaHCO₃ saturated aqueous solution was added to adjust the pH to 7-8. After the adjustment, di-tert-butyl dicarbonate (6.5 g, 30 mmol) was added. After completion of the reaction, water (50 mL) was added. The resultant solution was extracted with ethyl acetate (50 mL x 3). The organic layers were combined, and dried over anhydrous sodium sulfate. The solvent was rotary evaporated. The residue was then purified by column chromatography to obtain the solid compound **Int 1** (4.3 g, 6.6 mmol), with a yield of 88%. Ms: 654, 656 (M+H⁺)

### 2. Synthesis of intermediate (S)-1-(-2-(3,5-diethynyl-4-fluorophenyl)-4-methyl-4,5,6,7-tetrahydro-2H-pyrazolo[4,3-c]pyridine-3-yl)-3-(4-fluoro-1-methyl-1H-indazole-5-yl)-1,3-dihydro-2H-imidazole-2-one (Int 2)

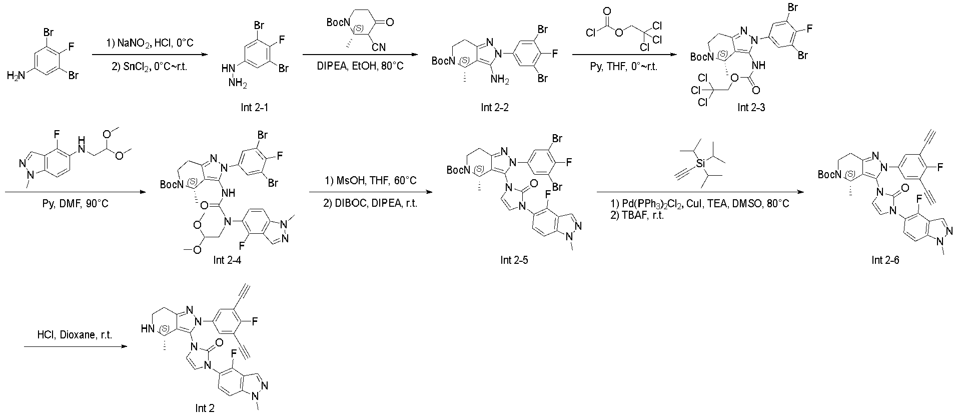

### Step 1: Synthesis of intermediate (3,5-dibromo-4-fluorophenyl)hydrazine (Int 2-1)

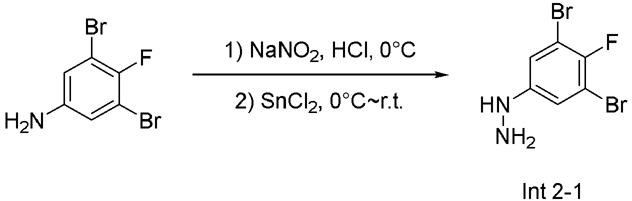

Procedures: 3,5-dibromo-4-fluoroaniline (5.0 g, 18.6 mmol) was dissolved in 6N hydrochloric acid (50 mL), and then cooled to 0 °C, to which was added a solution of sodium nitrite (1.9 g, 27.9 mmol) in water (4 mL) dropwise. The mixture was allowed to react at 0 °C for 0.5 h, and then a solution of SnCl₂ (7.1 g, 37.2 mmol) in concentrated hydrochloric acid (7 mL) was added dropwise. The reaction was naturally warmed to room temperature and reacted for 2 h, followed by filtering. The filter cake was triturated in methyl tert-butyl ether (15 mL), and filtered. The filter cake was dried to obtain 3.5 g of product (**Int 2-1**) hydrochloride salt, with a yield of 58.7%. Ms: 284.9/282.9/286.9 (M+H⁺).

### Step 2: Synthesis of intermediate tert-butyl (S)-3-amino-2-(3,5-dibromo-4-fluorophenyl)-4-methyl-2,4,6,7-tetrahydro-5H-pyrazolo[4,3-c]pyridine-5-carboxylate (Int 2-2)

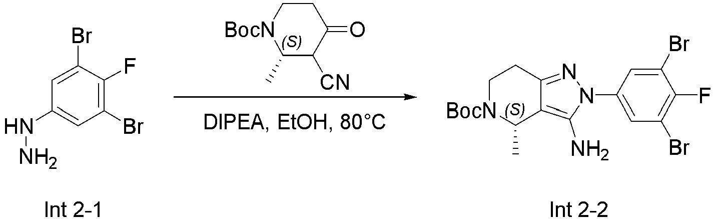

Procedures: Intermediate **Int 2-1** hydrochloride (3.5 g, 10.9 mmol), tert-butyl (2S)-3-cyano-2-methyl-4-oxopiperidine-1-carboxylate (2.6 g, 10.9 mmol), and N,N-diisopropylethylamine (2.8 g, 21.8 mmol) were successively added to ethanol (35 mL), reacted at 80°C overnight, and concentrated under reduced pressure. The residue was purified by column chromatography to obtain 2.5 g of product (**Int 2-2**), with a yield of 45.4%. Ms: 505.0/503.0/507.0 (M+H⁺).

### Step 3: Synthesis of intermediate tert-butyl (S)-2-(3,5-dibromo-4-fluorophenyl)-4-methyl-3-(((2,2,2-trichloroethoxy)carbonyl)amino)-2,4,6,7-tetrahydro-5H-pyrazolo[4,3-c]pyridine-5-carboxylate (Int 2-3)

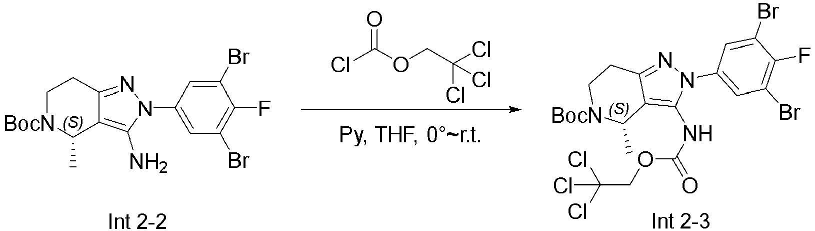

Procedures: Intermediate **Int 2-2** (2.5 g, 5.0 mmol) and pyridine (0.8 g, 9.9 mmol) were added to tetrahydrofuran (50 mL) in sequence, and then cooled to 0°C, to which was added a solution of chloroformic acid-2,2,2-trichloroethyl ester (1.6 g, 7.4 mmol) in tetrahydrofuran (16 mL) dropwise. The reaction was naturally warmed to room temperature and reacted overnight. Subsequently, K₂CO₃ saturated aqueous solution (25 mL) was added and stirred for 4 h. The resultant solution was extracted with ethyl acetate (50 mL). The organic layer was washed once with saturated saline (25 mL), dried over anhydrous sodium sulfate, and concentrated under reduced pressure. The residue was purified by column chromatography to obtain 2.9 g of product (**Int 2-3**). Yield: 86.1%. Ms: 678.8/680.8/682.8 (M+H⁺).

### Step 4: Synthesis of intermediate tert-butyl (S)-2-(3,5-dibromo-4-fluorophenyl)-3-(3-(2,2-dimethoxyethyl)-3-(4-fluoro-1-methyl-1H-indazole-5-yl)ureido)-4-methyl-2,4,6,7-tetrahydro-5H-pyrazolo[4,3-c]pyridine-5-carboxylate (Int 2-4)

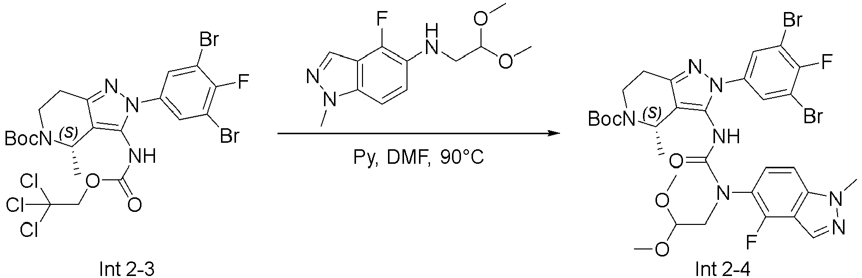

Procedures: Intermediate **Int 2-3** (2.9 g, 4.3 mmol), N-(2,2-dimethoxyethyl)-4-fluoro-1-methyl-1H-indazole-5-amine (2.2 g, 8.5 mmol), and pyridine (10.1 g, 128 mmol) were successively added to N,N-dimethylformamide (30 mL), and reacted at 90°C for 3 h, followed by adding ethyl acetate (50 mL) for dilution. The reaction was washed once with water (60 mL), and then washed once with saturated saline (60 mL). The resultant solution was dried over anhydrous sodium sulfate, and concentrated under reduced pressure. The residue was purified by column chromatography to obtain 2.5 g of product (**Int 2-4**). Yield: 74.8%. Ms: 784.0/782.0/786.0 (M+H⁺).

### Step 5: Synthesis of intermediate tert-butyl (S)-2-(3,5-dibromo-4-fluorophenyl)-3-(3-(4-fluoro-1-methyl-1H-indazole-5-yl)-2-oxo-2,3-dihydro-1H-imidazole-1-yl)-4-methyl-2,4,6,7-tetrahydro-5H-pyrazolo[4,3-c]pyridine-5-carboxylate (Int 2-5)

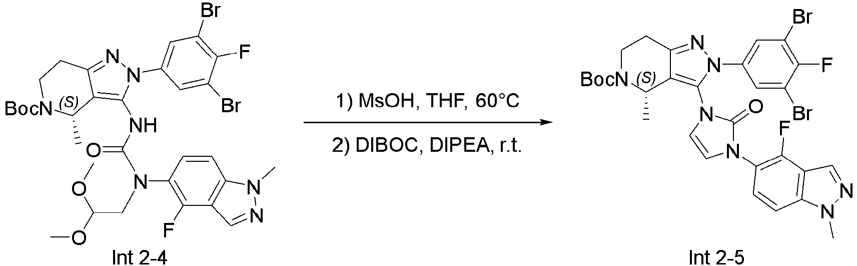

Procedures: Intermediate **Int 2-4** (2.5 g, 3.2 mmol) and methanesulfonic acid (3.1 g, 31.9 mmol) were added to tetrahydrofuran (30 mL) in sequence, reacted at 60°C for 1 h, and cooled to room temperature, to which were successively added N,N-diisopropylethylamine (5.4 g, 41.5 mmol) and di-tert-butyl dicarbonate (1.4 g, 6.4 mmol). The mixture was allowed to react at room temperature overnight, diluted with ethyl acetate (60 mL), and then washed once with water (50 mL) and saturated saline solution (50 mL), respectively. The resultant solution was dried over anhydrous sodium sulfate, and concentrated under reduced pressure. The residue was purified by column chromatography to obtain 2.1 g of product (**Int 2-5**), with a yield of 91.5% Ms: 720.0/717.9/722.0 (M+H⁺).

### Step 6: Synthesis of intermediate tert-butyl (S)-2-(3,5-diethynyl-4-fluorophenyl)-3-(3-(4-fluoro-1-methyl-1H-indazole-5-yl)-2-oxo-2,3-dihydro-1H-imidazole-1-yl)-4-methyl-2,4,6,7-tetrahydro-5H-pyrazolo[4,3-c]pyridine-5-carboxylate (Int 2-6)

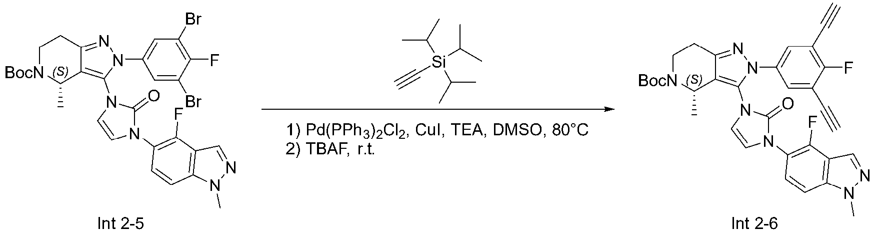

Procedures: Intermediate **Int 2-5** (320 mg, 0.44 mmol), triisopropylsilylacetylene (811 mg, 4.4 mmol), bis(triphenylphosphine)palladium(II) dichloride (62 mg, 0.09 mmol), CuI (17 mg, 0.09 mmol), and triethylamine (450 mg, 4.4 mmol) were successively added into dimethylsulfoxide (6 mL). The system was purged with nitrogen three times, and then reacted at 80 °C for 8 h, followed by cooling to room temperature, to which was added a solution of tetrabutylammonium fluoride in tetrahydrofuran (1 M, 5 mL). The resultant solution was stirred at room temperature for 1 h, and filtered. The filter cake was triturated in ethyl acetate (20 mL × 2). The filtrates were combined, washed with water (20 mL×3) and then washed once with saturated saline (20 mL), dried over anhydrous sodium sulfate, and concentrated under reduced pressure. The residue was purified by column chromatography to obtain 140 mg of product (**Int** 2-6). Yield: 51.6%. Ms: 610.2 (M+H⁺).

### Step 7: Synthesis of intermediate (S)-1-(-2-(3,5-diethynyl-4-fluorophenyl)-4-methyl-4,5,6,7-tetrahydro-2H-pyrazolo[4,3-c]pyridine-3-yl)-3-(4-fluoro-1-methyl-1H-indazole-5-yl)-1,3-dihydro-2H-imidazole-2-one (Int 2)

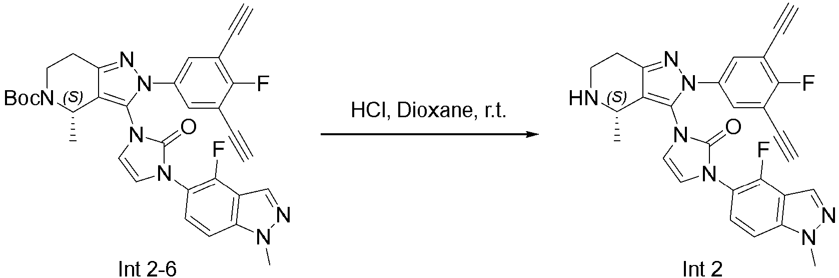

Procedures: Intermediate **Int 2-6** (140 mg, 0.25 mmol) was added to a solution of HCl in 1,4-dioxane (4 M, 3 mL), and reacted at room temperature for 2 h. The reaction solution was adjusted to pH >7 with NaHCO₃ saturated aqueous solution. The resultant solution was extracted with ethyl acetate (20 mL × 2). The organic layers were combined, washed with saturated saline solution (20 mL), dried over anhydrous sodium sulfate, and concentrated under reduced pressure, to obtain 100 mg of product (**Int 2**), with a yield of 85.5%. Ms: 510.1 (M+H⁺).

### 3. Synthesis of intermediate (S)-1-(-2-(3,5-divinyl-4-fluorophenyl)-4-methyl-4,5,6,7-tetrahydro-2H-pyrazolo[4,3-c]pyridine-3-yl)-3-(4-fluoro-1-methyl-1H-indazole-5-yl)-1,3-dihydro-2H-imidazole-2-one (Int 3)

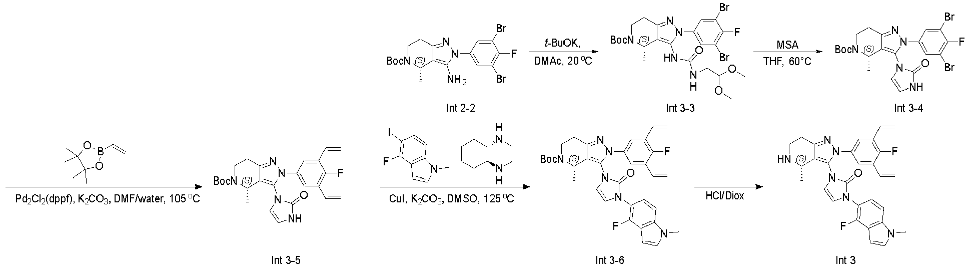

### Step 1: Synthesis of intermediate tert-butyl (S)-2-(3,5-dibromo-4-fluorophenyl)-3-(3-(2,2-dimethoxyethyl)ureido)-4-methyl-2,4,6,7-tetrahydro-5H-pyrazolo[4,3-c]pyridine-5-carboxylate (Int 3-3)

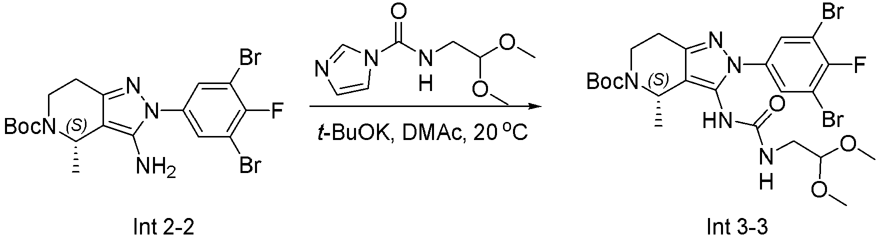

Procedures: Intermediate **Int 2-2** (8.0 g, 15.9 mmol), N-(2,2-dimethoxyethyl)-1H-imidazole-1-carboxamide, and potassium tert-butoxide (5.35 g, 47.7 mmol) were sequentially added to N,N-dimethylacetamide (80 mL) and reacted at 20 °C for 2 h. The system was then added with 200 mL of water, and extracted with ethyl acetate (50*3 mL). The organic layer was washed once with saturated saline (50 mL), dried over anhydrous sodium sulfate, and concentrated under reduced pressure. The residue was purified by column chromatography to obtain 9.1 g of product (**Int 3-3**). Yield: 91.1%. Ms: 636/634/638 (M+H⁺).

### Step 2: Synthesis of intermediate tert-butyl (S)-2-(3,5-dibromo-4-fluorophenyl)-4-methyl-3-(2-oxo-2,3-dihydro-1H-imidazole-1-yl)-2,4,6,7-tetrahydro-5H-pyrazolo[4,3-c]pyridine-5-carboxylate (Int 3-4)

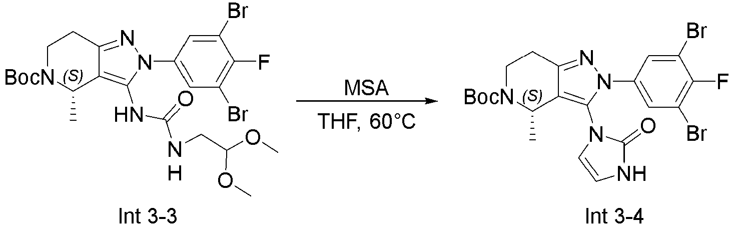

Procedures: Intermediate **Int 3-3** (9.1 g, 14.3 mmol) and methanesulfonic acid (2.8 g, 27.6 mmol) were sequentially added to tetrahydrofuran (300 mL) and reacted at 60 °C for 2 h. The system was diluted with water (150 mL), extracted with ethyl acetate (50 mL*3), washed once with saturated sodium bicarbonate (50 mL) and once with saturated saline (50 mL), dried over anhydrous sodium sulfate, and concentrated under reduced pressure. The residue was purified by column chromatography to obtain 5.0 g of product (**Int 3-4**). Yield: 61.1%. Ms: 572/570/573 (M+H⁺).

### Step 3: Synthesis of intermediate tert-butyl (S)-2-(4-fluoro-3,5-divinylphenyl)-4-methyl-3-(2-oxo-2,3-dihydro-1H-imidazole-1-yl)-2,4,6,7-tetrahydro-5H-pyrazolo[4,3-c]pyridine-5-carboxylate (Int 3-5)

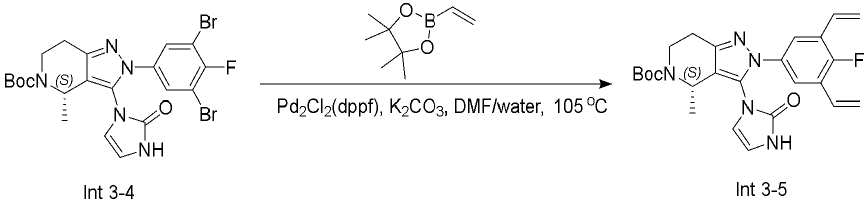

Procedures: Intermediate **Int 3-4** (5.0 g, 8.75 mmol), allylboronic acid pinacol ester (6.76 g, 43.8 mmol), 1,1'-bis(diphenylphosphino)ferrocene-palladium dichloride (0.64 g, 0.87 mmol), and K₂CO₃ (24.1 g, 17.5 mmol) were sequentially added to N,N-dimethylformamide/water (100 mL, 10:1) and reacted at 105 °C for 2 h. The reaction solution was cooled to room temperature, diluted with ethyl acetate (300 mL), washed three times with water (50 mL) and once with saturated saline (50 mL), dried over anhydrous sodium sulfate, and concentrated under reduced pressure. The residue was purified by column chromatography to obtain 2.8 g of product (**Int 3-5**). Yield: 70.0%. Ms: 466 (M+H⁺).

### Step 4: Synthesis of intermediate tert-butyl (S)-3-(3-(4-fluoro-1-methyl-1H-indole-5-yl)-2-oxo-2,3-dihydro-1H-imidazole-1-yl)-2-(4-fluoro-3,5-divinylphenyl)-4-methyl-2,4,6,7-tetrahydro-5H-pyrazolo[4,3-c]pyridine-5-carboxylate (Int 3-6)

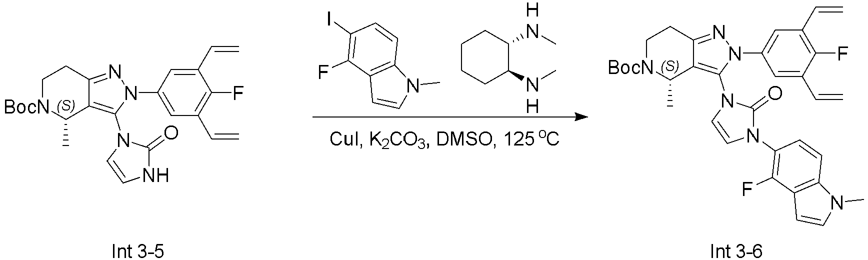

Procedures: Intermediate **Int 3-5** (1.8 g, 3.87 mmol), 4-fluoro-5-iodo-1-methyl-1H-indole (1.50 g, 5.41 mmol), CuI (0.29 g, 1.93 mmol), (1S,2S)-N¹,N²-dimethylcyclohexane-1,2-diamine (550 mg, 3.87 mmol), and K₂CO₃ (1.1 g, 7.74 mmol) were sequentially added to dimethylsulfoxide (40 mL). The system was purged with nitrogen three times, reacted at 125 °C for 28 h, cooled to room temperature, and filtered. The filtrate was diluted with water (100 mL), and then extracted with ethyl acetate (40 mL × 2). The combined organic phases were washed with water (20 mL × 3) and then once with saturated saline (20 mL), dried over anhydrous sodium sulfate, concentrated under reduced pressure, and purified by column chromatography, to obtain 1.0 g of product (**Int 3-6**). Yield: 42.2%. Ms: 613 (M+H⁺).

### Step 5: Synthesis of intermediate (S)-1-(-2-(3,5-divinyl-4-fluorophenyl)-4-methyl-4,5,6,7-tetrahydro-2H-pyrazolo[4,3-c]pyridine-3-yl)-3-(4-fluoro-1-methyl-1H-indazole-5-yl)-1,3-dihydro-2H-imidazole-2-one (Int 3)

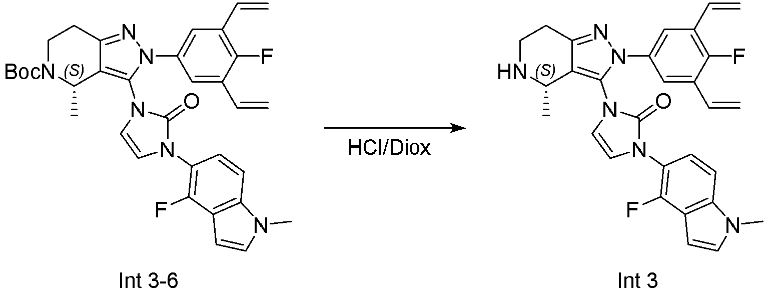

Procedures: Intermediate **Int 3-6** (1.0 g, 1.63 mmol) was added to a solution of HCl in 1,4-dioxane (4 M, 5 mL), and allowed to react at room temperature for 0.5 h. The pH was adjusted to >7 using an aqueous NaHCO₃ solution. The resultant solution was extracted with ethyl acetate (40 mL × 2). The organic layers were combined, washed with saturated saline solution (20 mL), dried over anhydrous sodium sulfate, and concentrated under reduced pressure. The residue was purified by column chromatography to obtain 600 mg of product (**Int 3**), with a yield of 71.1 %. Ms: 513 (M+H⁺).

### 4. Synthesis of intermediate (S)-1-(4-fluoro-1-methyl-1H-indazole-5-yl)-3-(2-(4-fluoro-3,5-dimethylphenyl)-4-methyl-4,5,6,7-tetrahydro-2H-pyrazolo[4,3-c]pyridine-3-yl)-1,3-dihydro-2H-imidazole-2-one (Int 4)

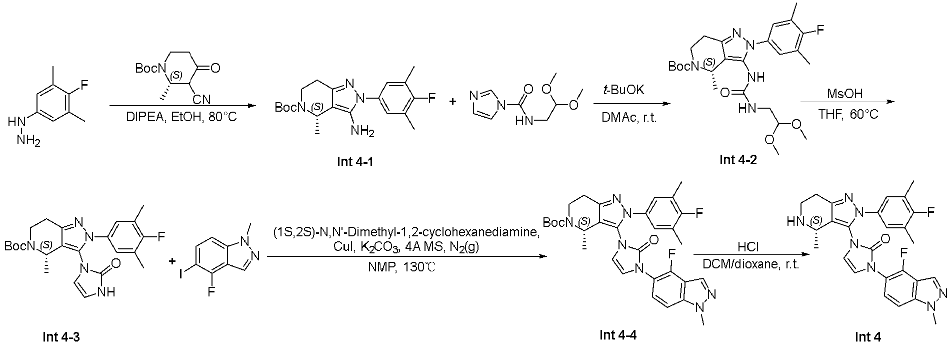

### Step 1: Synthesis of intermediate tert-butyl (S)-3-amino-2-(4-fluoro-3,5-dimethylphenyl)-4-methyl-2,4,6,7-tetrahydro-5H-pyrazolo[4,3-c]pyridine-5-carboxylate (Int 4-1)

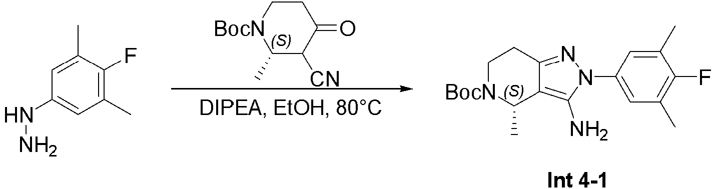

Procedures: Intermediate tert-butyl (2S)-3-cyano-2-methyl-4-oxopiperidine-1-carboxylate (20 g, 84.0 mmol) was dissolved in ethanol (200 mL), to which were sequentially added (4-fluoro-3,5-dimethylphenyl)hydrazine hydrochloride (17.5 g, 92.4 mmol) and *N,N-*diisopropylethylamine (21.7 g, 168.0 mmol). The mixture was heated to 80°C and reacted overnight. The reaction solution was concentrated under reduced pressure. The residue was purified by column chromatography to obtain 23 g of product (**Int 4-1**). Yield: 73.2%. Ms: 375.2 (M+H⁺).

### Step 2: Synthesis of intermediate tert-butyl (S)-3-(3-(2,2-dimethoxyethyl)urea)-2-(4-fluoro-3,5-dimethylphenyl)-4-methyl-2,4,6,7-tetrahydro-5H-pyrazolo[4,3-c]pyridine-5-carboxylate (Int 4-2)

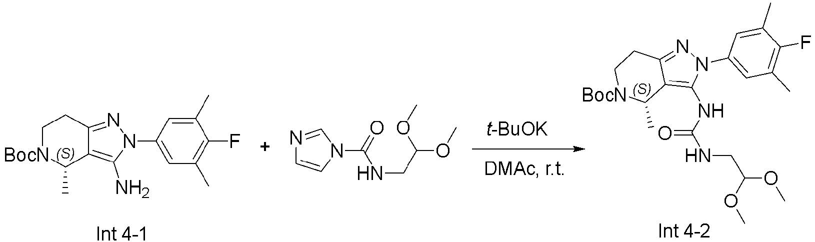

Procedures: Intermediate **Int 4-1** (2 g, 5.35 mmol), *N*-(2,2-dimethoxyethyl)-1*H*-imidazole-1-carboxamide (1.38 g, 6.42 mmol), and potassium tert-butoxide (0.9 g, 8.03 mmol) were sequentially added to DMAc (10 mL) and stirred at room temperature. The reaction was monitored by TLC. After the reaction was complete, the reaction mixture was cooled to room temperature, dropped into water, and filtered. The resultant solid was purified by column chromatography to obtain 1.1 g of product (**Int 4-2**). Yield: 40.7%. Ms: 506.1 (M+H⁺).

### Step 3: Synthesis of intermediate tert-butyl (S)-2-(4-fluoro-3,5-dimethylphenyl)-4-methyl-3-(2-oxo-2,3-dihydro-1H-imidazole-1-yl)-2,4,6,7-tetrahydro-5H-pyrazolo[4,3-c]pyridine-5-carboxylate (Int 4-3)

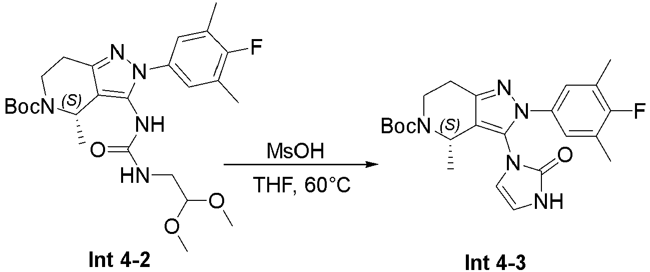

Procedures: Intermediate **Int 4-2** (1.1 g, 2.17 mmol) was dissolved in tetrahydrofuran (22 mL), to which was added methanesulfonic acid (0.21 g, 2.17 mmol) dropwise, and then the mixture was stirred at 60°C. The reaction was monitored by TLC. Water (110 mL) was added, and the resultant solution was extracted with ethyl acetate (50 mL × 3). The organic layer was washed once with saturated saline (50 mL), dried over anhydrous sodium sulfate, and concentrated under reduced pressure. The residue was purified by column chromatography to obtain 0.92 g of product (**Int 4-3**), with a yield of 96.1%. Ms: 442.1 (M+H⁺).

### Step 4: Synthesis of intermediate tert-butyl (S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-2,4,6,7-tetrahydro-5H-pyrazolo[4,3-c]pyridine-5-carboxylate (Int 4-4)

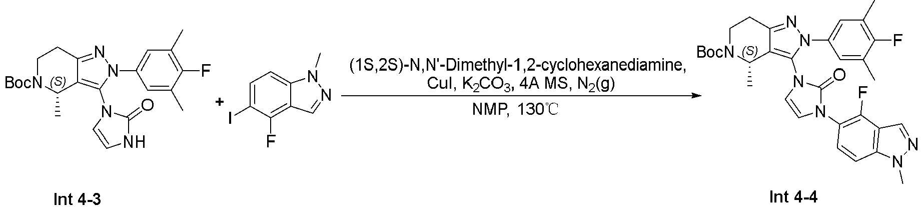

Procedures: Intermediate **Int 4-3** (0.92 g, 2 mmol), 4-fluoro-5-iodo-1-methyl-1*H*-indazole (1.1 g, 4 mmol), (1S,2S)-N,N'-dimethyl-1,2-cyclohexanediamine (0.28 g, 2 mmol), CuI (0.38 g, 2 mmol), K₂CO₃ (0.82 g, 6 mmol), and molecular sieves (0.92 g) were sequentially added to NMP (10 mL). Under N₂ (g) protection, the mixture was reacted at 130 °C for 12 h. Ethyl acetate (50 mL) was added for dilution, followed by washing once with water (10 mL) and once with saturated saline (60 mL). The reaction solution was then dried over anhydrous sodium sulfate, concentrated under reduced pressure, and purified via column chromatography to obtain 680 mg of product (**Int 4-4**). Yield: 57.7%. Ms: 590.1 (M+H⁺).

### Step 5: Synthesis of (S)-1-(4-fluoro-1-methyl-1H-indazole-5-yl)-3-(2-(4-fluoro-3,5-dimethylphenyl)-4-methyl-4,5,6,7-tetrahydro-2H-pyrazolo[4,3-c]pyridine-3-yl)-1,3-dihydro-2H-imidazole-2-one (Int 4)

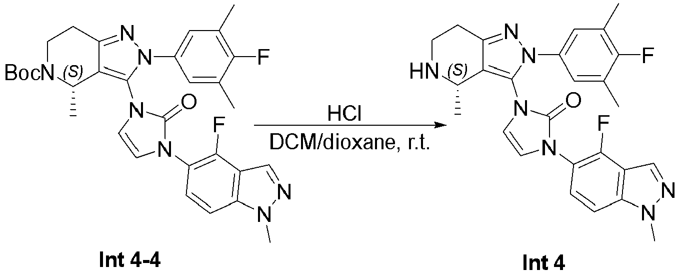

Procedures: Intermediate **Int 4-4** (680 mg, 1.15 mmol) was added to dichloromethane (7 ml) and a solution of HCl in 1,4-dioxane (4 M, 3 mL), and then reacted at room temperature for 2 h. The pH was adjusted to >7 with NaHCO₃ saturated aqueous solution. The resultant solution was extracted with ethyl acetate (20 mL × 2). The organic layers were combined, washed with saturated saline (20 mL), dried over anhydrous sodium sulfate, and concentrated under reduced pressure to obtain 511 mg of product (**Int 4**). Yield: 91%. Ms: 490.1 (M+H⁺).

### 5. Synthesis of intermediate (S)-1-(1-cyanocyclopropyl)-5-(2,2-dimethyltetrahydro-2H-pyran-4-yl)-1H-indole-2-carboxylic acid (Int 5)

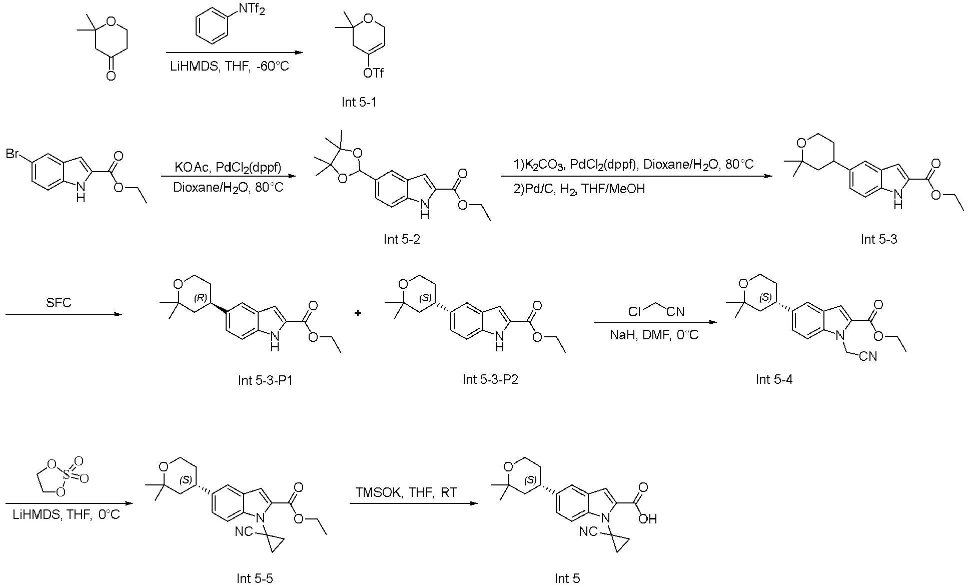

### Step 1: Synthesis of intermediate 2,2-dimethyl-3,6-dihydro-2H-pyran-4-yltrifluoromethanesulfonate (Int 5-1)

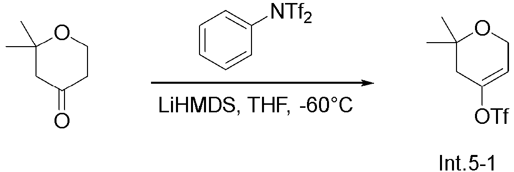

Procedures: Tetrahydro-2,2-dimethyl-4*H*-pyran-4-one (25.6 g, 200.0 mmol) was dissolved in tetrahydrofuran (250 mL), and cooled to -70 °C in a dry ice bath, to which was slowly added a solution of LiHMDS (1 M, 240 mL) in tetrahydrofuran dropwise. The mixture was allowed to react at -70 °C for 0.5 h. Bis(trifluoromethylsulfonyl)aniline (77 g, 220 mmol) was dissolved in tetrahydrofuran (250 mL), and then slowly added to the reaction solution, during which the temperature was kept no higher than -60 °C. After that, the reaction solution was naturally warmed and reacted overnight. A saturated aqueous solution of ammonium chloride (500 mL) was added to the reaction solution. The resultant solution was extracted with ethyl acetate (500 mL × 2). The organic layers were combined, and dried over anhydrous sodium sulfate, to obtain 100.0 g of crude product (**Int 5-1**), which was directly used in the next step.

### Step 2: Synthesis of intermediate ethyl 5-(4,4,5,5-tetramethyl-1,3-dioxolane-2-yl)-1H-indole-2-carboxylate (Int 5-2)

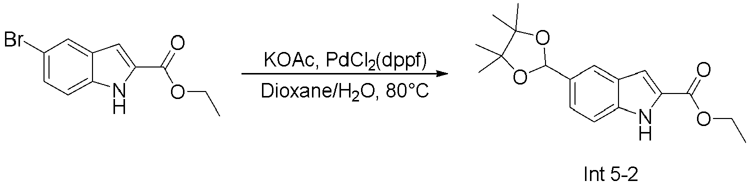

Procedures: Ethyl 5-bromoindole-2-carboxylate (26.8 g, 100.0 mmol), bis(pinacolato)diboron (50.8 g, 200.0 mmol), potassium acetate (19.6 g, 200.0 mmol), and PdCl₂(dppf) (7.3 g, 10.0 mmol) were successively added to dioxane/water (v/v = 10:1, 500 mL). The system was purged with nitrogen three times, and reacted at 80 °C overnight. The reaction solution was cooled to room temperature, and filtered through diatomaceous earth. The filter cake was rinsed with ethyl acetate (100 mL). Water (500 mL) was added to the filtrate, followed by extracting with ethyl acetate (300 mL × 2). The organic layers were combined, dried over anhydrous sodium sulfate, and then concentrated under reduced pressure. The residue was purified by column chromatography to obtain 20.0 g of product (**Int 5-2**). Yield: 63.0%. Ms: 318.4 (M+H⁺).

### Step 3: Synthesis of intermediate ethyl 5-(2,2-dimethyltetrahydro-2H-pyran-4-yl)-1H-indole-2-carboxylate (Int 5-3)

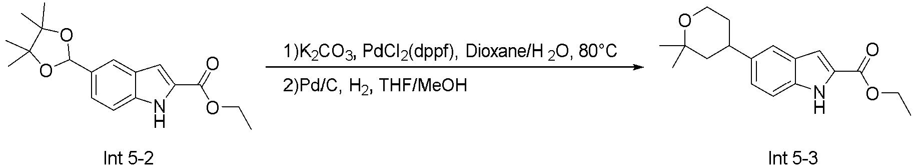

Procedures: Intermediate **Int 5-2** (19.0 g, 60.0 mmol), crude intermediate **Int 5-1** (100 g, 200.0 mmol), K₂CO₃ (17.0 g, 120.0 mmol), and PdCl₂(dppf) (4.4 g, 6.0 mmol) were sequentially added to a mixture of dioxane and water (v/v = 10:1, 500 mL). The system was purged with nitrogen three times and reacted at 80 °C overnight. After cooling to room temperature, the reaction solution was filtered through diatomaceous earth. The filter cake was rinsed with ethyl acetate (100 mL), and the filtrate was combined with water (500 mL). The resultant solution was then extracted with ethyl acetate (300 mL × 2). The organic layers were combined, dried over anhydrous sodium sulfate, and concentrated under reduced pressure. The mixed solution of tetrahydrofuran and methanol (v/v = 5:1, 200 mL) was added to the residue, followed by adding 10% Pd/C (1.0 g). The system was purged with nitrogen three times and then hydrogen three times, and then reacted at room temperature overnight. After filtering through diatomaceous earth, the filtrate was concentrated under reduced pressure. The residue was purified by column chromatography to obtain 11.0 g of the product (**Int 5-3)**, with a yield of 60.9%. Ms: 302.2 (M+H⁺).

### Step 4: Separation of intermediates 5-3-P1 and 5-3-P2

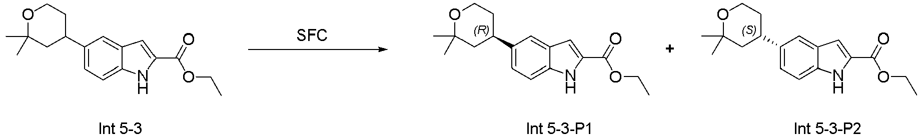

Procedures: Intermediate **Int 5-3** (10.0 g, 33.2 mmol) was separated by SFC (column: DAICEL CHIRALPAK^{®} AD-H (30×250 mm, 5 µm); mobile phase: A = CO2, Co-Solvent B=IPA/CAN, Co-Solvent B%=35%; flow rate: 45 mL; wavelength: 215 nm; temperature: 35°C) to give intermediate **Int 5-3-P1** (3.8 g) and intermediate **Int 5-3-P2** (4.5 g).

### Step 5: Synthesis of intermediate ethyl (S)-1-(cyanomethyl)-5-(2,2-dimethyltetrahydro-2H-pyran-4-yl)-1H-indole-2-carboxylate (Int 5-4)

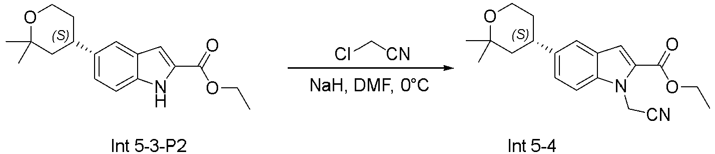

Procedures: Intermediate **Int 5-3-P2** (4 g, 13.3 mmol) was dissolved in *N,N-*dimethylformamide (40 mL), and then stirred for 10 min in an ice bath, to which was then added NaH (479 mg, 19.95 mmol) in batches. After that, the reaction solution was warmed to room temperature and stirred for 2 h. Chloroacetonitrile (5 g, 66.67 mmol) was added to the solution in an ice bath, and the solution was then warmed to room temperature and stirred for another 2 h. After the reaction was complete, 40 mL of aqueous NH₄Cl solution was added, followed by extraction with ethyl acetate (25 mL × 3). The organic layers were combined, washed twice with saturated saline, dried over anhydrous sodium sulfate, and concentrated under reduced pressure. The residue was purified by column chromatography to obtain 3.8 g of product (**Int 5-4**). Yield: 84.4%. Ms: 341.2 (M+H⁺).

### Step 6: Synthesis of intermediate ethyl (S)-1-(1-cyanocyclopropyl)-5-(2,2-dimethyltetrahydro-2H-pyran-4-yl)-1H-indole-2-carboxylate (Int 5-5)

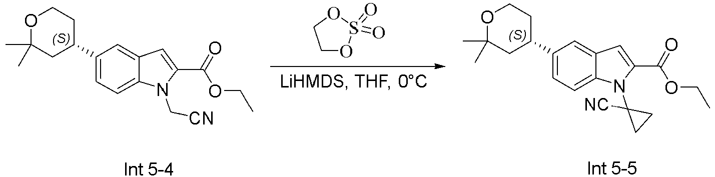

Procedures: Intermediate **Int 5-4** (1.45 g, 4.3 mmol) and ethylene sulfate (1.6 g, 12.8 mmol) were dissolved in tetrahydrofuran (20 mL). After stirring for 10 min in an ice-salt bath, a solution of LiHMDS in tetrahydrofuran (1 M, 22 mL) was gradually added, followed by stirring for 1 h in the ice-salt bath. After completion of the reaction, NH₄Cl aqueous solution (50 mL) was added, and the resultant solution was extracted with ethyl acetate (25 mL × 3). The organic layers were combined, dried over anhydrous sodium sulfate, and concentrated under reduced pressure. The residue was purified by column chromatography to obtain 1.14 g of product (**Int 5-5**), with a yield of 73.1%. Ms: 367.2 (M+H⁺).

### Step 7: Synthesis of intermediate (S)-1-(1-cyanocyclopropyl)-5-(2,2-dimethyltetrahydro-2H-pyran-4-yl)-1H-indole-2-carboxylic acid (Int 5)

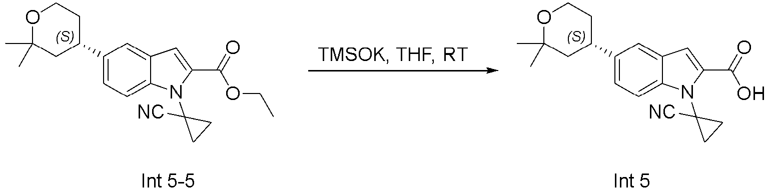

Procedures: Intermediate **Int 5-5** (1.14 g, 3.1 mmol) and potassium trimethylsilanolate (1.6 g, 12.4 mmol) were dissolved in tetrahydrofuran (11 mL) and stirred at room temperature for 4 h. After the reaction was complete, 2N HCl was added to adjust the pH to 1. The resultant solution was then extracted with ethyl acetate (25 mL × 3). The organic layers were combined, dried over anhydrous sodium sulfate, and concentrated under reduced pressure. The residue was purified by HPLC to obtain 840 mg of **Int** 5, with a yield of 83.0%. Ms: 339.1 (M+H⁺).

### 6. Synthesis of intermediate 1-((1S, 2S)-1-cyano-2-methylcyclopropyl)-5-((S)-2,2-dimethyltetrahydro-2H-pyran-4-yl)-1H-indole-2-carboxylic acid (Int 6)

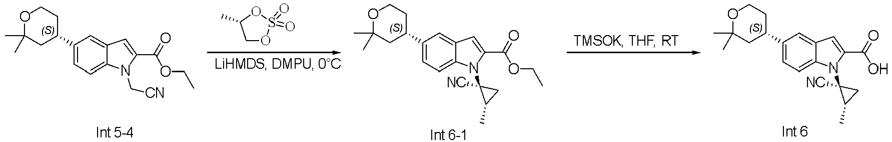

### Step 1: Synthesis of ethyl 1-((1S,2S)-1-cyano-2-methylcyclopropyl)-5-((S)-2,2-dimethyltetrahydro-2H-pyran-4-yl)-1H-indole-2-carboxylate (Int 6-1)

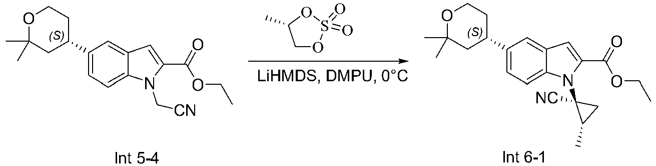

Procedures: Intermediate **Int 5-4** (1.45 g, 4.3 mmol) and (4S)-4-propyl-[1,3-2]dioxothiolane-2,2-oxide (1.8 g, 12.8 mmol) were dissolved in N,N-dimethylpropyleneurea (9 mL). The reaction was stirred for 10 min in an ice-salt bath, followed by the slow addition of a solution of LiHMDS in tetrahydrofuran (1 M, 22 mL). Afterwards, the reaction solution was stirred for 1 h in the ice-salt bath. After completion of the reaction, NH₄Cl aqueous solution (50 mL) was added. The mixture was then extracted with ethyl acetate (25 mL × 3). The organic layers were combined, dried over anhydrous sodium sulfate, and concentrated under reduced pressure. The residue was purified by column chromatography to obtain 1.0 g of product (**Int 6-1**). Yield: 62.0%. Ms: 381.2 (M+H⁺).

### Step 1: Synthesis of intermediate 1-((1S, 2S)-1-cyano-2-methylcyclopropyl)-5-((S)-2,2-dimethyltetrahydro-2H-pyran-4-yl)-1H-indole-2-carboxylic acid (Int 6)

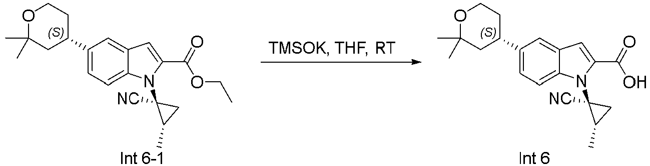

Procedures: Intermediate **Int 6-1** (1.0 g, 2.6 mmol) and potassium trimethylsilanolate (1.3 g, 10.4 mmol) were dissolved in tetrahydrofuran (9 mL) and stirred at room temperature for 4 h. After the reaction was complete, 2N HCl was added to adjust the pH to 1. The resultant solution was then extracted with ethyl acetate (25 mL × 3). The organic layers were combined, dried over anhydrous sodium sulfate, and concentrated under reduced pressure. The residue was purified by HPLC to obtain 880 mg of **Int 6**, with a yield of 95.0%. Ms: 353.1 (M+H⁺).

### 7. Synthesis of intermediate tert-butyl (S)-2-(3-bromo-4-fluorophenyl)-4-methyl-3-(2-oxo-2,3-dihydro-1H-imidazole-1-yl)-2,4,6,7-tetrahydro-5H-pyrazolo[4,3-c]pyridine-5-carboxylate (Int 7)

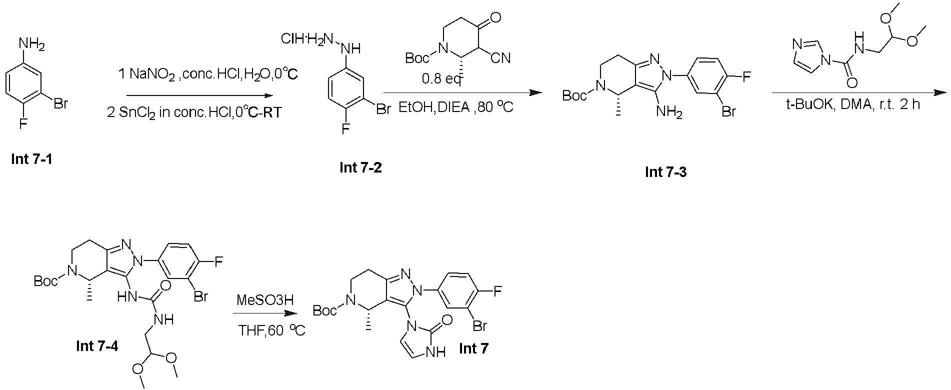

### Step 1: Synthesis of intermediate (3-bromo-4-fluorophenyl)hydrazine hydrochloride (Int 7-2)

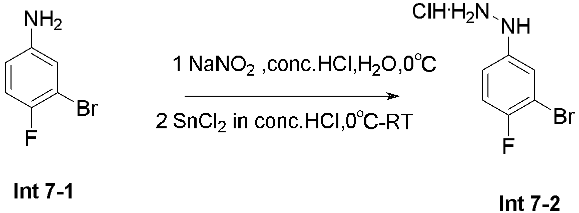

At room temperature, To **Int 7-1** (6.0g, 38mmol), water (70 mL), and concentrated hydrochloric acid (30 mL), was added NaNO₂ aqueous solution (2.4 g, 34.73 mmol, 6 mL) dropwise at 0 °C. After that, the mixture was stirred at 0 °C for 1 h. Subsequently, a solution of SnCl₂ (11.97 g, 63.15 mmol) in concentrated hydrochloric acid (20 mL) was added dropwise at the same temperature. After the addition, the mixture was stirred at room temperature for 2 h. The reaction solution was then filtered, and the filter cake was dried and triturated in water (10 mL). After filtration, a white solid compound **Int 7-2** (6.0 g, 24.85 mmol) was obtained. Yield: 78%. M/S: 205.1 (M+H⁺).

### Step 2: Synthesis of intermediate tert-butyl (S)-3-amino-2-(3-bromo-4-fluorophenyl)-4-methyl-2,4,6,7-tetrahydro-5H-pyrazolo[4,3-c]pyridine-5-carboxylate (Int 7-3)

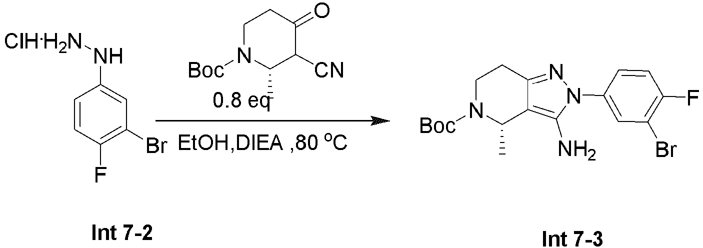

A mixture of intermediate **Int7-2** (7.0 g, 28.99 mmol), tert-butyl (2S)-3-cyano-2-methyl-4-oxopiperidine-1-carboxylate (5.87 g, 24.64 mmol), N,N-diisopropylethylamine (7.49 g, 57.97 mmol), and ethanol (100 mL) was heated to 80 °C and stirred overnight. After the reaction was complete, the mixture was cooled to room temperature, to which was added water (100 mL), and then extracted with ethyl acetate (100 mL × 3 times). The organic layers were combined, and dried over anhydrous sodium sulfate. The solvent was rotary evaporated. The residue was purified by column chromatography to obtain the solid compound **Int 7-3** (11 g, 25.86 mmol) with a yield of 89%. Ms: 425.1 (M+H⁺).

### Step 3: Synthesis of intermediate tert-butyl (S)-2-(3-bromo-4-fluorophenyl)-3-(3-(2,2-dimethoxyethyl)ureido)-4-methyl-2,4,6,7-tetrahydro-5H-pyrazolo[4,3-c]pyridine-5-carboxylate (Int 7-4)

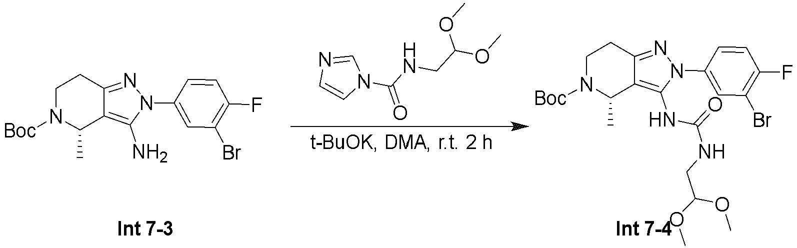

At room temperature, the intermediate **Int7-3** (11.0 g, 25.86 mmol) was dissolved in DMA (150 mL), to which was added N-(2,2-dimethoxyethyl)-1H-imidazole-1-carboxamide (10.3 g, 51.73 mmol). After nitrogen replacement, potassium tert-butoxide (7.26 g, 64.66 mmol) was slowly added dropwise in an ice bath. The reaction was stirred at room temperature for 2 h. TLC was used to monitor the complete reaction of the raw materials. The reaction solution was diluted with 200 mL of water, and then extracted with ethyl acetate (200 mL × 3). The organic phase was washed with 100 mL of saturated saline, dried over sodium sulfate, and filtered. The solution was concentrated under reduced pressure, and the concentrate was purified by silica gel column chromatography to obtain product **Int 7-4** as light yellow solid (9.0 g, 16.17 mmol), with a yield of 62%. M/S: 556.1 (M+H⁺).

### Step 4: Synthesis of compound tert-butyl (S)-2-(3-bromo-4-fluorophenyl)-4-methyl-3-(2-oxo-2,3-dihydro-1H-imidazole-1-yl)-2,4,6,7-tetrahydro-5H-pyrazolo[4,3-c]pyridine-5-carboxylate (Int 7)

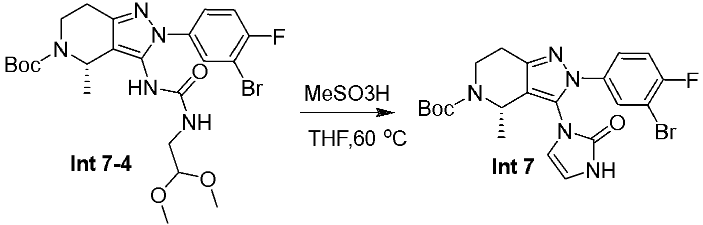

At room temperature, the intermediate **Int 7-4** (9.0 g, 16.17 mmol) and methanesulfonic acid (1.87 g, 19.41 mmol) were dissolved in THF (200 mL), and the reaction was stirred at 60 °C for 1 h. The reaction of raw materials was monitored to be complete by TLC. The reaction solution was diluted with 100 mL of water, and then extracted with ethyl acetate (150 mL × 3). The organic phase was washed with 100 mL of saturated saline, dried over sodium sulfate, filtered, and concentrated under reduced pressure. The concentrate was purified by silica gel column chromatography to obtain product **Int7** as light yellow solid (4.0 g, 0.52 mmol), with a yield of 50%. M/S: 492.1 (M+H⁺).

### Example 1. Synthesis of 3-((1S,2S)-1-(5-((S)-2,2-dimethyltetrahydro-2H-pyran-4-yl)-2-(S)-3-(3-(4-(4-fluoro-1-methyl-1H-indazole-5-yl)-2-oxo-2,3-dihydro-1H-imidazole-1-yl)-2-one (compound R):

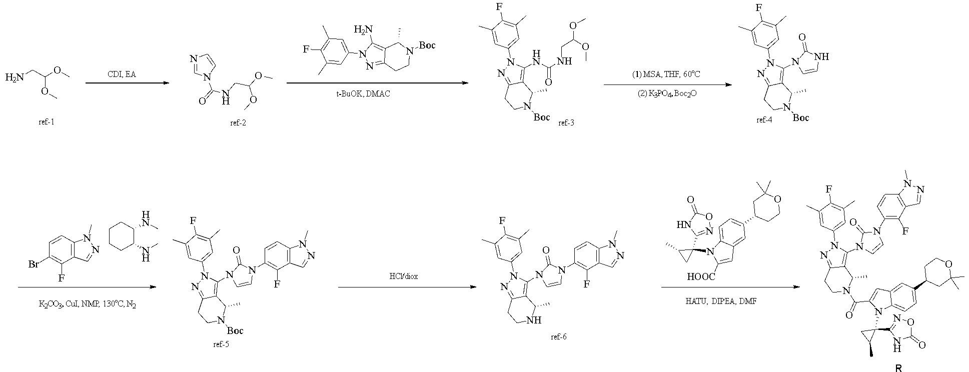

### Step 1: Synthesis of intermediate N-(2,2-dimethoxyethyl)-1H-imidazole-1-formamide (ref-2):

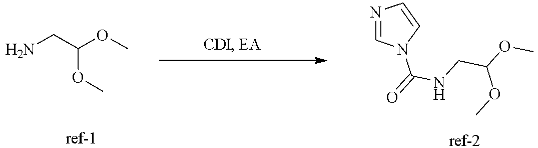

Procedures: CDI (1.55g, 9.5 mmol) was added to a reaction flask, to which was added EA (10 mL). The reaction was stirred for 10 min in an ice bath, and then **ref-1** (1g, 9.5 mmol) was slowly added dropwise. Afterwards, the reaction was stirred at room temperature for 2 h. After the reaction was complete, the reaction solution was diluted with 20 mL of water, and then extracted with ethyl acetate (20 mL x 3). The organic phase was washed with 20 mL of saturated saline, dried over sodium sulfate, filtered, and concentrated under reduced pressure. The concentrate was purified by silica gel column chromatography to obtain the intermediate **ref-2** (1.47 g, yield: 77.8%). Ms: 200 (M+H⁺)

### Step 2: Synthesis of intermediate tert-butyl (S)-3-(3-(2,2-dimethoxyethyl)ureido)-2-(4-fluoro-3,5-dimethylphenyl)-4-methyl-2,4,6,7-tetrahydro-5H-pyrazolo[4,3-c]pyridine-5-carboxylate (ref-3):

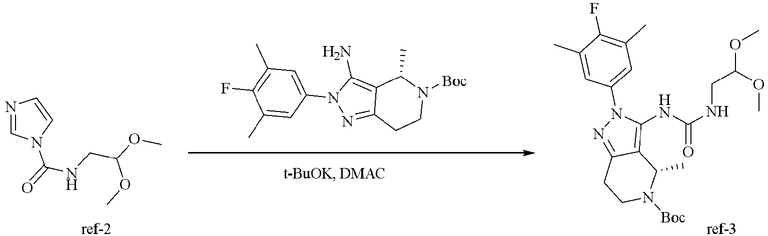

Procedures: At room temperature, the intermediate **ref-2** (200 mg, 1.01 mmol) and tert-butyl (S)-3-amino-2-(4-fluoro-3,5-dimethylphenyl)-4-methyl-2,4,6,7-tetrahydro-5H-pyrazolo[4,3-c]pyridine-5-carboxylate (400 mg, 0.84 mmol) were added to a solution of t-BuOK (282 mg, 2.52 mmol) in N,N-dimethylacetamide (20 mL). The resulting system was stirred at room temperature for 2 h. The reaction solution was diluted with 50 mL of water, and then extracted with ethyl acetate (20 mL x 3). The organic phase was washed with 50 mL of saturated saline, dried over sodium sulfate, filtered, and concentrated under reduced pressure. The concentrate was purified by silica gel column chromatography to obtain the intermediate **ref-3** (450.0 mg, yield: 83.3%). Ms: 506 (M+H⁺)

### Step 3: Synthesis of intermediate tert-butyl (S)-2-(4-fluoro-3,5-dimethylphenyl)-4-methyl-3-(2-oxo-2,3-dihydro-1H-imidazole-1-yl)-2,4,6,7-tetrahydro-5H-pyrazolo[4,3-c]pyridine-5-carboxylate (ref-4):

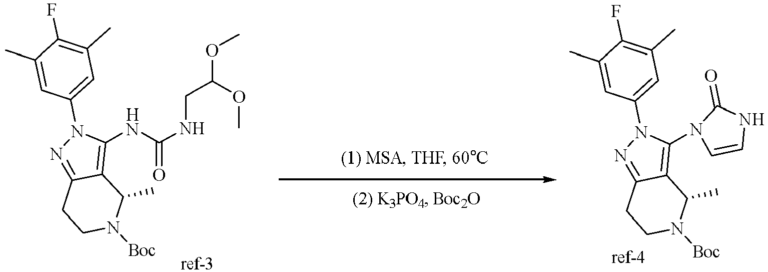

Procedures: Compound **ref-3** (450 mg, 0.89 mmol) was placed in a 50 mL one-neck flask, to which was added tetrahydrofuran (20 mL), followed by adding methanesulfonic acid (41 mg, 0.44 mmol), and then the mixture was stirred at 60 °C for 2 h. After the reaction was complete, the reaction solution was cooled to room temperature, and then K₃PO₄ aqueous solution was added to adjust the pH to 9, followed by adding di-tert-butyl dicarbonate (78 mg, 0.356 mmol). The reaction was stirred for 1 h. 50 mL of water was added to the reaction solution. The resultant solution was extracted with ethyl acetate (20 mL x 3). The organic phases were combined, washed with 50 mL of saturated saline, dried over sodium sulfate, filtered, and concentrated under reduced pressure. The concentrate was purified by silica gel column chromatography to obtain intermediate **ref-4** (340 mg, yield: 86.5%). Ms: 442 (M+H⁺)

### Step 4: Synthesis of intermediate tert-butyl (S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-2,4,6,7-tetrahydro-5H-pyrazolo[4,3-c]pyridine-5-carboxylate (ref-5):

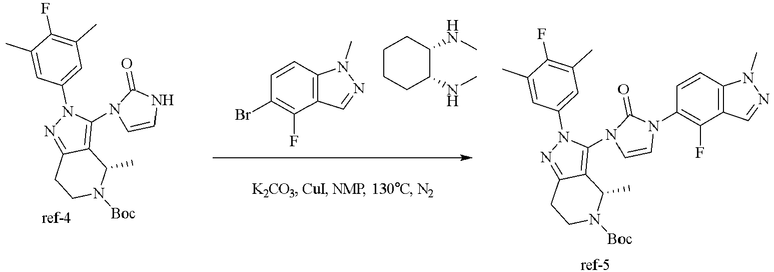

Procedures: To a three-neck flask, compound **ref-4** (100 mg, 0.159 mmol), 5-bromo-4-fluoro-1-methyl-1H-indazole (104 mg, 0.317 mmol), and (1S,2S)-N1,N2-dimethylcyclohexane-1,2-diamine (17 mg, 0.079 mmol) were dissolved in N-methylpyrrolidone (10 mL). Then, CuI (9 mg, 0.032 mmol) and K₂CO₃ (94 mg, 0.476 mmol) were added. The reaction solution was stirred under nitrogen protection at 130 °C for 2 h. Water (20 mL) was added to the reaction solution. The resultant solution was extracted with ethyl acetate (10 mL x 3). The organic phases were combined, washed with 20 mL of saturated saline, dried over sodium sulfate, filtered, and concentrated under reduced pressure. The concentrate was purified by silica gel column chromatography to obtain intermediate **ref-5** (50 mg, yield: 38%). Ms: 590 (M+H⁺)

### Step 5: Synthesis of intermediate (S)-1-(4-fluoro-1-methyl-1H-indazole-5-yl)-3-(2-(4-fluoro-3,5-dimethylphenyl)-4-methyl-4,5,6,7-tetrahydro-2H-pyrazolo[4,3-c]pyridine-3-yl)-1,3-dihydro-2H-imidazole-2-one (compound ref-6):

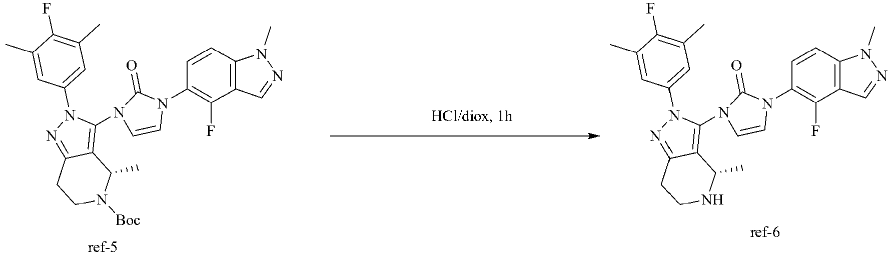

Procedures: Compound **ref-5** (50 mg, 0.085 mmol) was placed in a 25 mL one-neck flask, to which was added 4 M solution of hydrochloric acid in 1,4-dioxane (5 mL), and stirred at room temperature for 1 h. Then, K₂CO₃ aqueous solution was added to adjust the pH to 11. The resultant solution was extracted with ethyl acetate (10 mL x 3). The organic phases were combined, washed with 20 mL of saturated saline, dried over sodium sulfate, filtered, and concentrated under reduced pressure to obtain intermediate **ref-6** (40 mg, yield: 97%). Ms: 490 (M+H⁺)

### Step 6: Synthesis of product 3-((1S,2S)-1-(5-((S)-2,2-dimethyltetrahydro-2H-pyran-4-yl)-2-(S)-3-(3-(4-(4-fluoro-1-methyl-1H-indazole-5-yl)-2-oxo-2,3-dihydro-1H-imidazole-1-yl)-2-one (compound R):

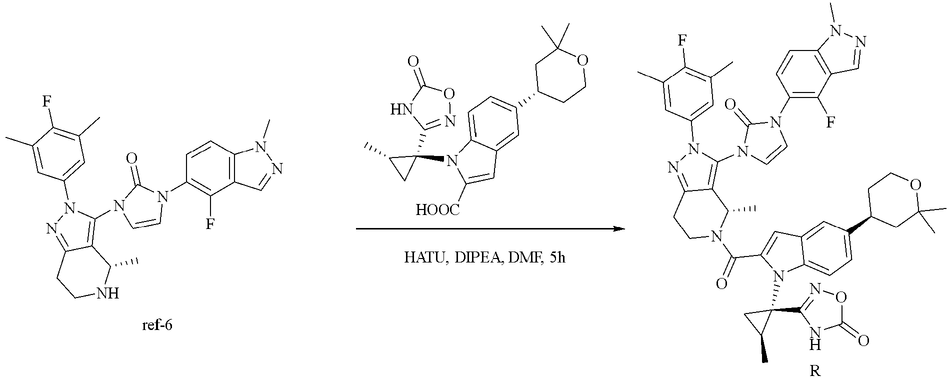

Procedures: Compound **ref-6** (40 mg, 0.12 mmol) and 5-((S)-2,2-dimethyltetrahydro-2H-pyran-4-yl)-1-((1S,2S)-2-methyl-1-(5-oxo-4,5-dihydro-1,2,4-oxadiazole-3-yl)cyclopropyl)-1H-indole-2-carboxylic acid (50 mg, 0.12 mmol) were dissolved in DMF (0.5 mL), to which were added HATU (68 mg, 0.18 mmol) and N,N-diisopropylethylamine (31 mg, 0.24 mmol) respectively, and then stirred at room temperature for 5 h. The reaction solution was quenched by adding 20 mL of water. The resultant solution was extracted with ethyl acetate (10 mL x 3)., The organic phases were combined, washed with 20 mL of saturated saline, dried over sodium sulfate, filtered, and concentrated under reduced pressure. The concentrate was purified by silica gel column chromatography to obtain the target compound (compound R) (13 mg, yield 18%). 883 (M+H+)
¹H NMR (400 MHz, DMSO-d6) δ 11.76 (s, 1H), 8.31 (s, 1H), 7.64 (d, J = 8.9 Hz, 1H), 7.53 (s, 1H), 7.49 (d, J = 7.9 Hz, 1H), 7.39 (d, J = 8.5 Hz, 1H), 7.26 (d, J = 8.5 Hz, 1H), 7.18 (d, J = 6.2 Hz, 2H), 7.09 (s, 1H), 6.95 (s, 2H), 4.10 (d, J = 10.7 Hz, 3H), 3.71 (d, J = 8.4 Hz, 2H), 3.18 (s, 1H), 3.02 (d, J = 12.1 Hz, 1H), 2.88 (d, J = 14.9 Hz, 1H), 2.26 (s, 6H), 1.66 (d, J = 12.2 Hz, 7H), 1.43 (d, J = 6.7 Hz, 2H), 1.28 - 1.17 (m, 13H).

### Example 2. Synthesis of compound 3-((1S,2S)-1-(2-((S)-2-(3-cyclopropyl-4-fluorophenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropyl)-1,2,4-oxadiazole-5(4H)-one (Compound 1)

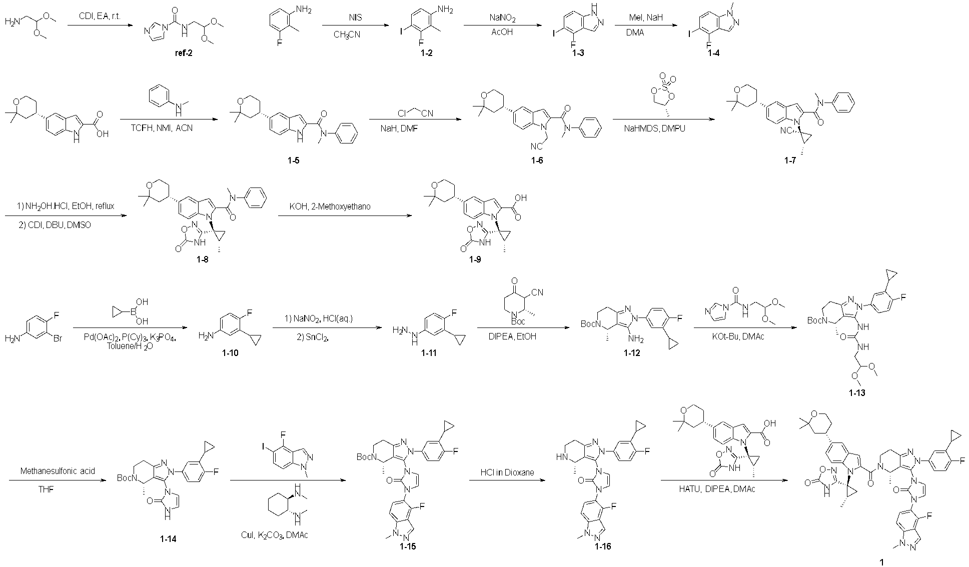

### Step 1: Synthesis of intermediate N-(2,2-dimethoxyethyl)-1H-imidazole-1-formamide (ref-2)

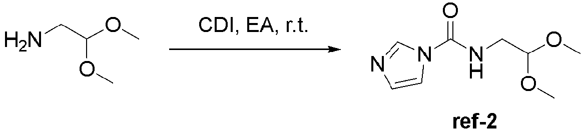

Procedures: *N,N'*-carbonyldiimidazole (3.3 g, 20.0 mmol) was added to ethyl acetate (40 mL), to which was added aminoacetaldehyde dimethyl acetal (2.1 g, 20.0 mmol) dropwise, and then the mixture was allowed to react at room temperature overnight. Ethyl acetate (100 mL) was added. The resultant solution was washed once with water (50 mL) and once with saturated saline (50 mL), dried over anhydrous sodium sulfate, and concentrated under reduced pressure. The residue was purified by column chromatography to obtain 1.5 g of product. Yield: 39.6%. Ms: 200 (M+H⁺).

### Step 2: Synthesis of intermediate 3-fluoro-4-iodo-2-methylaniline (1-2)

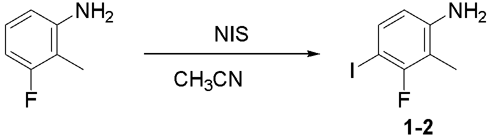

Procedures: 3-fluoro-2-methylaniline (50.0 g, 0.4 mol) was dissolved in 500 mL of acetonitrile, to which was added *N*-iodosuccinimide (99.0 g, 0.44 mol) in batches under the conditions of the temperature below 5°C. The mixture was allowed to react for 2 h at the temperature of below 10 °C. NaOH aqueous solution was added to adjust the pH to 9-10. The resultant solution was extracted with ethyl acetate (500 mL×2). The organic layers were combined, washed once with saturated saline (500 mL), dried over anhydrous sodium sulfate, and concentrated under reduced pressure. The concentrate was purified by column chromatography to obtain 99 g of product, with a yield of 98.7%. Ms: 251.9 (M+H⁺).

### Step 3: Synthesis of intermediate 4-fluoro-5-iodo-1H-indazole (1-3)

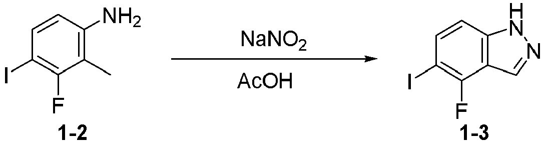

Procedures: Intermediate **1-2** (50 g, 0.2 mol) was dissolved in acetic acid (500 mL), to which was added sodium nitrite (27.6 g, 0.4 mol) in batches, and then the mixture was allowed to react at room temperature for 4 h. The reaction solution was cooled to -10 °C, and under vigorous stirring, NaOH aqueous solution (30%) was added dropwise until pH=7-8. The resultant solution was extracted with ethyl acetate (500 mL×3). The organic layers were combined, washed once with saturated saline (500 mL), dried over anhydrous sodium sulfate, and concentrated under reduced pressure. The concentrate was purified by column chromatography to obtain 26 g of product. Yield: 49.8%. Ms: 262.9 (M+H⁺).

### Step 4: Synthesis of intermediate 4-fluoro-5-iodo-1-methyl-1H-indazole (1-4)

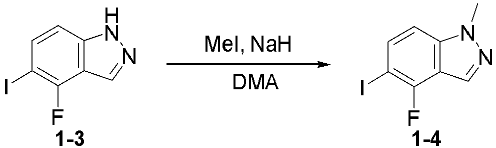

Procedures: NaH (4.8 g, 0.11 mol) was added to *N,N*-dimethylacetamide (160 mL). Under N₂ protection, the reaction solution was cooled to 0 °C in an ice-water bath, to which was added a solution of intermediate **1-3** (26.0 g, 0.1 mol) in *N,N*-dimethylacetamide (100 mL) dropwise. The reaction was stirred at room temperature for 1 h, followed by slowly adding iodomethane (7.5 mL, 0.12 mol) dropwise. The mixture was allowed to react at room temperature for 4 h, and then slowly poured into ice water (1000 mL) for quenching. The resultant solution was extracted with ethyl acetate (500 mL×3). The organic layers were combined, washed with saturated saline (500 mL×2), dried over anhydrous sodium sulfate, and concentrated under reduced pressure. The residue was purified by column chromatography to obtain 19 g of product (intermediate **1-4**). Yield: 69.3%. Ms: 277.0 (M+H⁺).

### Step 5: Synthesis of intermediate (S)-5-(2,2-dimethyltetrahydro-2H-pyran-4-yl)-N-methyl-N-phenyl-1H-indole-2-formamide (1-5)

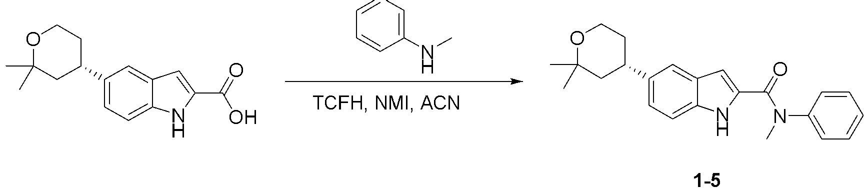

Procedures: (*S*)-5-(2,2-dimethyltetrahydro-2H-pyran-4-yl)-1H-indole-2-carboxylic acid (2.0 g, 7.3 mmol), *N,N,N',N'*-tetramethylchloroformamidinium hexafluorophosphate (4.1 g, 14.6 mmol), and *N*-methylimidazole (1.8 g, 21.9 mmol) were added to acetonitrile (40 mL) in sequence, and then stirred and reacted at room temperature for 0.5 h, followed by adding *N-*methylaniline (1.2 g, 11.0 mmol). The mixture was allowed to react at room temperature for 1 h, and filtered. The filter cake was rinsed with acetonitrile (10 mL), and dried to obtain 2.5 g of product. Yield: 94.2%. Ms: 363.2 (M+H⁺).

### Step 6: Synthesis of intermediate (S)-1-(cyanomethyl)-5-(2,2-dimethyltetrahydro-2H-pyran-4-yl)-N-methyl-N-phenyl-1H-indole-2-formamide (1-6)

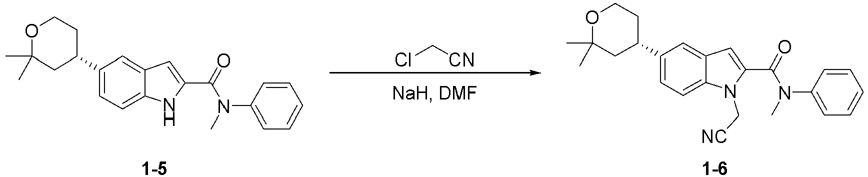

Procedures: NaH (0.42 g, 10.4 mmol) was added to *N,N*-dimethylformamide (20 mL), and then cooled to 0 °C in an ice-water bath under N₂ protection, to which was added a solution of intermediate **1-5** (2.5 g, 6.9 mmol) in *N,N*-dimethylformamide (5 mL) dropwise, and then stirred at room temperature for 1 h, followed by adding chloroacetonitrile (0.79 mg, 10.4 mmol). The mixture was reacted at room temperature for 2 h, and slowly poured into ice-water (50 mL) for quenching. The resultant solution was extracted with ethyl acetate (25 mL×3). The organic layers were combined, washed with saturated saline (50 mL×2), dried over anhydrous sodium sulfate, and concentrated under reduced pressure. The residue was purified by column chromatography to obtain 2.5 g of product. Yield: 90.0%. Ms: 402.1 (M+H⁺).

### Step 7: Synthesis of intermediate 1-((1S,2S)-1-cyano-2-methylcyclopropyl)-5-((S)-2,2-dimethyltetrahydro-2H-pyran-4-yl)-N-methyl-N-phenyl-1H-indole-2-formamide (1-7)

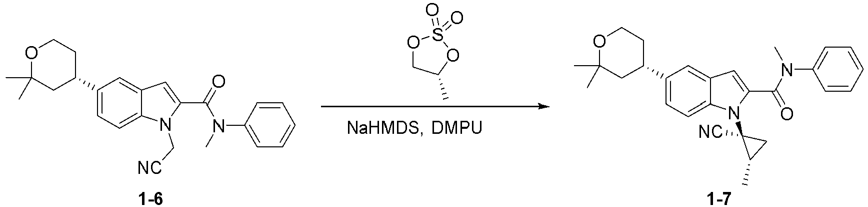

Procedures: Intermediate **1-6** (500 mg, 1.25 mmol) and (4*R*)-4-methyl-1,3,2-dithiolane (430 mg, 3.12 mmol) were subsequently added to *N,N*-dimethylpropyleneurea (5 mL), and then cooled to 0 °C in an ice-water bath under N₂ protection, to which was added sodium bis(trimethylsilyl)amide (2.5 mL, 5.0 mmol) dropwise. The mixture was allowed to react at 0 °C for 4 h, and then NH₄Cl saturated aqueous solution (10 mL) was slowly added to quench the reaction. The resultant solution was extracted with ethyl acetate (10 mL × 2). The organic layers were combined, washed once with saturated saline (10 mL), dried over anhydrous sodium sulfate, and concentrated under reduced pressure. The residue was purified by column chromatography to obtain 400 mg of product. Yield: 72.7%. Ms: 442.2 (M+H⁺).

### Step 8: Synthesis of intermediate 5-((S)-2,2-dimethyltetrahydro-2H-pyran-4-yl)-N-methyl-1-((1S,2S)-2-methyl-1-(5-oxo-4,5-dihydro-1,2,4-oxadiazole-3-yl)cyclopropyl)-N-phenyl-1H-indole-2-formamide (1-8)

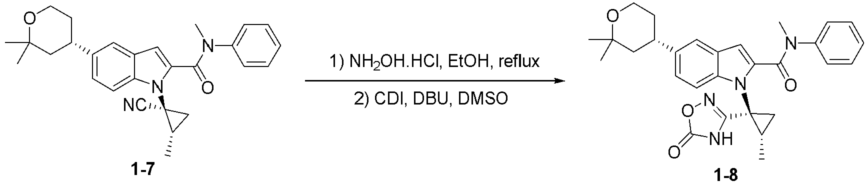

Procedures: Intermediate **1-7** (300 mg, 0.68 mmol), hydroxylamine hydrochloride (235 mg, 3.4 mmol), and K₂CO₃ (516 mg, 3.74 mmol) were sequentially added to ethanol (6 mL) and reacted at 100 °C for 3 h. The reaction solution was then cooled to room temperature, to which was added water (20 mL), and then extracted with ethyl acetate (10 mL × 2). The organic layers were combined, washed once with saturated saline (20 mL), dried over anhydrous sodium sulfate, and concentrated under reduced pressure to obtain a crude product. The crude product was dissolved in dimethylsulfoxide (5 mL), and then *N,N'*-carbonyldiimidazole (162 mg, 1.0 mmol) and 1,8-diazabicyclo[5,4,0]undec-7-ene (213 mg, 1.4 mmol) were sequentially added. The mixture was reacted at 80°C for 3 h, and then cooled to room temperature, followed by adding water (20 mL). The resultant solution was extracted with ethyl acetate (10 mL × 2). The organic layers were combined, washed once with saturated saline (20 mL), dried over anhydrous sodium sulfate, and concentrated under reduced pressure. The residue was purified by column chromatography to obtain 250 mg of product, with a yield of 73.4%. Ms: 501.2 (M+H⁺).

### Step 9: Synthesis of intermediate 5-((S)-2,2-dimethyltetrahydro-2H-pyran-4-yl)-1-((1S,2S)-2-methyl-1-(5-oxo-4,5-dihydro-1,2,4-oxadiazole-3-yl)cyclopropyl)-1H-indole-2-carboxylic acid (1-9)

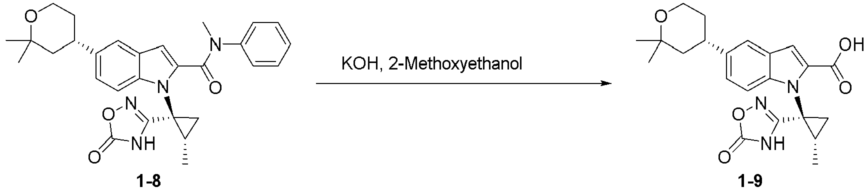

Procedures: Intermediate **1-8** (250 mg, 0.5 mmol) and KOH (280 mg, 5.0 mmol) were added to 2-methoxyethanol (10 mL) in sequence, and then reacted at 125 °C for 1 h. The reaction solution was cooled to room temperature, and then 2N hydrochloric acid was added dropwise to adjust pH to 3, followed by adding water (10 mL). Solid was precipitated and filtered. The filter cake was dried to obtain 150 mg product, yield: 73.0%. Ms: 412.2 (M+H⁺).

### Step 10: Synthesis of intermediate 3-cyclopropyl-4-fluoroaniline (1-10)

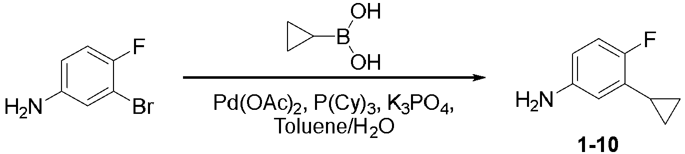

Procedures: 3-bromo-4-fluoroaniline (100 g, 526 mmol), cyclopropylboronic acid (58.8 g, 684 mmol), potassium phosphate (391 g, 1.84 mol), tricyclohexylphosphine (14.8 g, 52.6 mmol), and palladium(II) acetate (5.96 g, 26.3 mmol) were sequentially added to toluene/water (20:1, 2000 mL), and then the system was purged with nitrogen three times. The reaction solution was heated to 100 °C in an oil bath and reacted overnight, followed by cooling to room temperature and filtering. The filter cake was rinsed with ethyl acetate (500 mL). The filtrates were combined, concentrated under reduced pressure, and purified by column chromatography to obtain 70 g of product. Yield: 88%. Ms: 152.2 (M+H⁺).

### Step 11: Synthesis of intermediate (3-cyclopropyl-4-fluorophenyl)hydrazine (1-11)

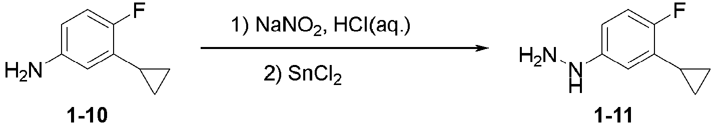

Procedures: Intermediate **1-10** (50 g, 331 mmol) was dissolved in 6N hydrochloric acid (500 mL), and then cooled to 0°C, to which was added a solution of sodium nitrite (34.2 g, 496 mmol) in water (70 mL) dropwise. The mixture was reacted at 0 °C for 0.5 h, followed by addition of a solution of stannous chloride (125 g, 661 mmol) in concentrated hydrochloric acid (150 mL). The reaction solution was naturally warmed and reacted for 3 h. After filtration, the filter cake was triturated in methyl tert-butyl ether (200 mL) and filtered. The filter cake was then dried to obtain 30 g of the product hydrochloride salt, with a yield of 45%. Ms: 167.1 (M+H⁺).

### Step 12: Synthesis of intermediate tert-butyl (S)-3-amino-2-(3-cyclopropyl-4-fluorophenyl)-4-methyl-2,4,6,7-tetrahydro-5H-pyrazolo[4,3-c]pyridine-5-carboxylate (1-12)

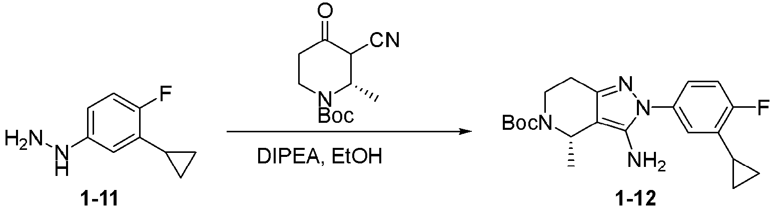

Procedures: Intermediate **1-11** hydrochloride (19 g, 93.7 mmol), tert-butyl (2S)-3-cyano-2-methyl-4-oxopiperidine-1-carboxylate (22.3 g, 93.7 mmol), and N,N-diisopropylethylamine (24.2 g, 187 mmol) were successively added to ethanol (200 mL), and react at 80 °C overnight. The reaction solution was concentrated under reduced pressure, and the residue was purified by column chromatography to obtain 21 g of product, with a yield of 58%. Ms: 387.2 (M+H⁺).

### Step 13: Synthesis of intermediate tert-butyl (S)-2-(3-cyclopropyl-4-fluorophenyl)-3-(3-(2,2-dimethoxyethyl)ureido)-4-methyl-2,4,6,7-tetrahydro-5H-pyrazolo[4,3-c]pyridine-5-carboxylate (1-13)

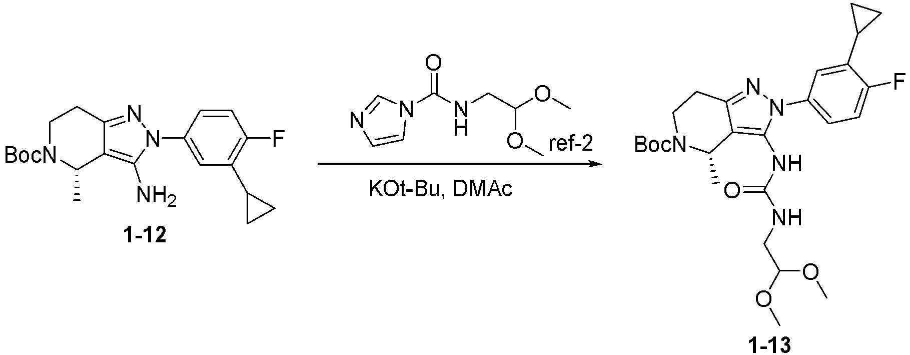

Procedures: Intermediate **1-12** (1.5 g, 3.9 mmol), intermediate **ref-2** (1.6 g, 7.8 mmol), and potassium tert-butoxide (1.3 g, 11.6 mmol) were successively added to *N,N*-dimethylacetamide (15 mL), and reacted at room temperature for 2 h. The reaction solution was diluted with ethyl acetate (150 mL), and then washed once with water (100 mL) and once with saturated saline (100 mL). The organic layer was dried over anhydrous sodium sulfate, and concentrated under reduced pressure. The residue was purified by column chromatography to obtain 1.4 g of product. Yield: 69.7%. Ms: 518.3 (M+H⁺).

### Step 14: Synthesis of intermediate tert-butyl (S)-2-(3-cyclopropyl-4-fluorophenyl)-4-methyl-3-(2-oxo-2,3-dihydro-1H-imidazole-1-yl)-2,4,6,7-tetrahydro-5H-pyrazolo[4,3-c]pyridine-5-carboxylate (1-14)

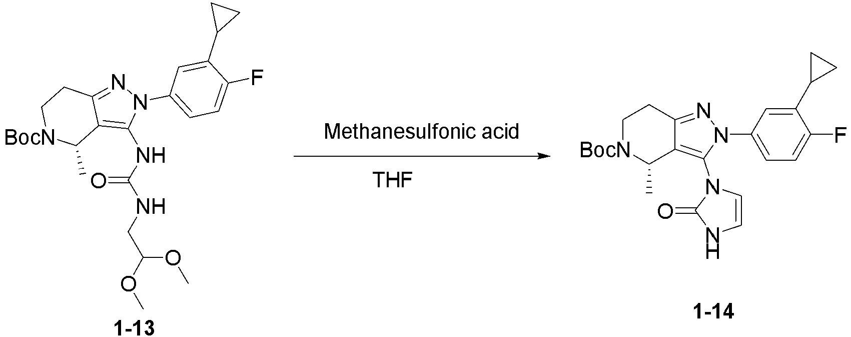

Procedures: Intermediate **1-13** (1.4 g, 2.7 mmol) and methanesulfonic acid (0.26 g, 2.7 mmol) were successively added to tetrahydrofuran (30 mL), and reacted at 60 °C for 2 h. The reaction solution was poured into ice water (50 mL). The resultant solution was extracted with ethyl acetate (50 mL×2). The organic layers were combined, washed once with NaHCO₃ saturated aqueous solution (50 mL), dried over anhydrous sodium sulfate, and concentrated under reduced pressure. The residue was purified by column chromatography to obtain 1.1 g of product. Yield: 89.7%. Ms: 454.2 (M+H⁺).

### Step 15: Synthesis of intermediate tert-butyl (S) -2-(3-cyclopropyl-4-fluorophenyl)-3-(3-(4-fluoro-1-methyl-1H-indazole-5-yl)-2-oxo-2,3-dihydro-1H-imidazole-1-yl)-4-methyl-2,4,6,7-tetrahydro-5H-pyrazolo[4,3-c]pyridine-5-carboxylate (1-15)

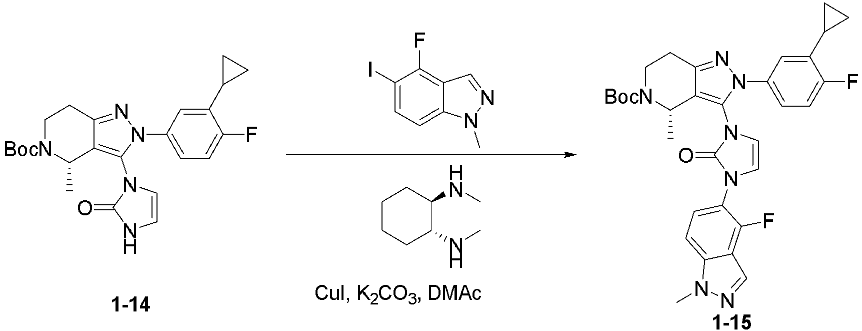

Procedures: Intermediate **1-14** (1.1 g, 2.4 mmol), intermediate **1-4** (1.3 g, 4.8 mmol), (1*S*,2*S*)-(-)-*N,N'*-dimethyl-1,2-cyclohexanediamine (0.35 g, 2.4 mmol), CuI (0.47 g, 2.4 mmol), and K₂CO₃ (1.0 g, 7.3 mmol) were sequentially added to *N,N*-dimethylacetamide (15 mL). The system was purged with nitrogen three times, reacted at 120 °C overnight, cooled to room temperature, and then filtered. The filter cake was triturated in ethyl acetate (50 mL × 2). The filtrates were combined, washed with water (50 mL × 2) and then once with saturated saline (50 mL), dried over anhydrous sodium sulfate, and concentrated under reduced pressure. The residue was purified by column chromatography to obtain 1.0 g of product. Yield: 68.5%. Ms: 602.3 (M+H⁺).

### Step 16: Synthesis of intermediate (S)-1-(2-(3-cyclopropyl-4-fluorophenyl)-4-methyl-4,5,6,7-tetrahydro-2H-pyrazolo[4,3-c]pyridine-3-yl)-3-(4-fluoro-1-methyl-1H-indazole-5-yl)-1,3-dihydro-2H-imidazole-2-one (1-16)

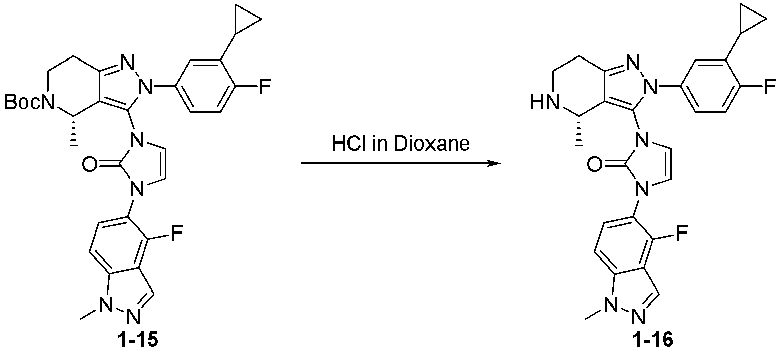

Procedures: Intermediate **1-15** (1.0 g, 1.7 mmol) was added to a solution of HCl in 1,4-dioxane (4 M, 5 mL), and then reacted at room temperature for 2 h. NaHCO₃ saturated aqueous solution was added to adjust the pH to >7. The resultant solution was extracted with ethyl acetate (20 mL × 2). The organic layers were combined, washed with saturated saline (20 mL), dried over anhydrous sodium sulfate, and concentrated under reduced pressure to obtain 750 mg of product. Yield: 90%. Ms: 502.2 (M+H⁺).

### Step 17: Synthesis of compound 3-((1S,2S)-1-(2-((S)-2-(3-cyclopropyl-4-fluorophenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropyl)-1,2,4-oxadiazole-5(4H)-one

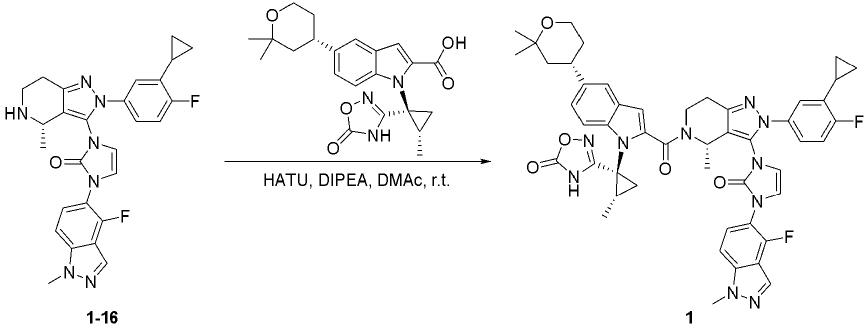

Procedures: Intermediate **1-16** (50 mg, 0.1 mmol), intermediate **1-9** (49 mg, 0.12 mmol), HATU (46 mg, 0.12 mmol), and *N,N*-diisopropylethylamine (26 mg, 0.2 mmol) were sequentially added to N,N-dimethylacetamide (1 mL) and reacted at room temperature for 4 h. The reaction solution was diluted with ethyl acetate (15 mL), and then washed once with water (10 mL) and once with saturated saline (10 mL). The organic layer was dried over anhydrous sodium sulfate, and concentrated under reduced pressure. The residue was purified by column chromatography to obtain 51 mg of product. Yield: 57%. Ms: 894.6 (M+H⁺). ¹H NMR (400 MHz, DMSO-*d*₆) *δ* 11.74 (s, 1H), 8.29 (s, 1H), 7.64 (d, *J =* 8.8 Hz, 1H), 7.53 (s, 1H), 7.51 - 7.46 (m, 1H), 7.44 - 7.33 (m, 2H), 7.32 - 7.21 (m, 2H), 7.18 - 7.09 (m, 2H), 7.04 - 6.92 (m, 2H), 5.69 - 5.61 (m, 1H), 4.15 - 4.00 (m, 4H), 3.75 - 3.64 (m, 2H), 3.39 (d, *J =* 11.0 Hz, 1H), 3.00 - 2.86 (m, 1H), 2.77 - 2.65 (m, 1H), 2.14 - 2.01 (m, 1H), 1.74 - 1.59 (m, 4H), 1.49 (q, *J =* 12.8 Hz, 1H), 1.34 (d, *J =* 6.6 Hz, 3H), 1.28 - 1.13 (m, 9H), 1.04 - 0.92 (m, 5H), 0.81 - 0.61 (m, 2H).

### Example 3. Synthesis of compound 3-((1S,2S)-1-(5-(S)-2,2-dimethyltetrahydro-2H-pyran-4-yl)-2-((S)-3-(3-(4-fluoro-1-methyl-1H-indazole-5-yl)-4-methyl-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-1,2,4-oxadiazole-5(4H)-one (Compound 4)

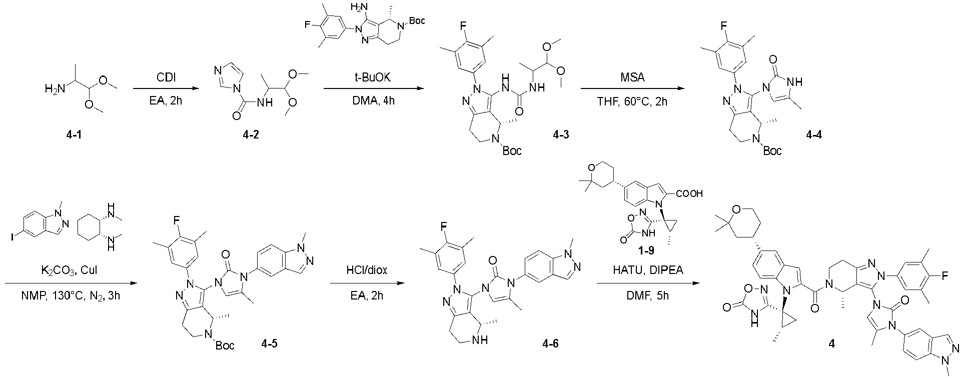

### Step 1: Synthesis of intermediate N-(1,1-dimethoxypropane-2-yl)-1H-imidazole-1-formamide (4-2)

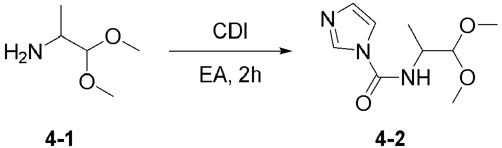

CDI (1.55g, 9.5 mmol) was dissolved in ethyl acetate (10 mL), to which was slowly added compound **4-1** (1 g, 8.4 mmol) dropwise in an ice bath. After that, the reaction was stirred at room temperature for 2 h. After completion of the reaction, the reaction solution was diluted by adding 20 mL of water, and then extracted with ethyl acetate (20 mL×3). The organic phase was washed with 20 mL of saturated saline, dried over sodium sulfate, filtered, and concentrated under reduced pressure. The concentrate was purified by silica gel column chromatography to obtain intermediate **4-2** (1.5 g, yield: 84%). M/S: 214.1 (M+H⁺).

### Step 2: Synthesis of intermediate tert-butyl (4S)-3-(3-(1,1-dimethoxypropane-2-yl)ureido)-2-(4-fluoro-3,5-dimethylphenyl)-4-methyl-2,4,6,7-tetrahydro-5H-pyrazolo[4,3-c]pyridine-5-carboxylate (4-3)

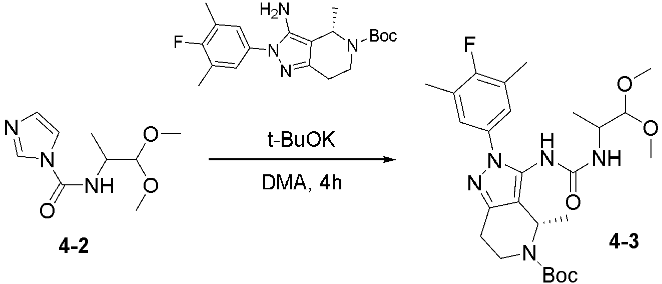

At room temperature, intermediate **4-2** (215 mg, 1.01 mmol), tert-butyl (5)-3-amino-2-(4-fluoro-3,5-dimethylphenyl)-4-methyl-2,4,6,7-tetrahydro-5H-pyrazolo[4,3-c]pyridine-5-carboxylate (400 mg, 0.84 mmol), and t-BuOK (282 mg, 2.52 mmol) were added to a solution of N,N-dimethylacetamide (20 mL). The reaction solution was stirred at room temperature for 4 h, diluted with 50 mL of water, and then extracted with ethyl acetate (20 mL × 3). The organic phase was washed with 50 mL of saturated saline, dried over sodium sulfate, and filtered. The filtrate was concentrated under reduced pressure, and the concentrate was purified by silica gel column chromatography to obtain intermediate **4-3** (450.0 mg, yield: 83.3%). M/S: 520.2 (M+H⁺).

### Step 3: Synthesis of intermediate tert-butyl ((S)-2-(4-fluoro-3,5-dimethylphenyl)-4-methyl-3-(4-methyl-2-oxo-2,3-dihydro-1H-imidazole-1-yl)-2,4,6,7-tetrahydro-5H-pyrazolo[4,3-c]pyridine-5-carboxylate (4-4)

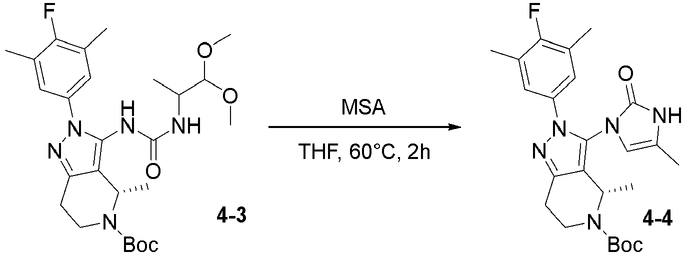

Compound **4-3** (460 mg, 0.89 mmol) was placed in a 50 mL one-neck flask, to which were successively added tetrahydrofuran (20 mL) and methanesulfonic acid (41 mg, 0.44 mmol), and then stirred and reacted at 60 °C for 2 h. After the reaction was complete, the reaction solution was cooled to room temperature, and then 50 mL of water was added. The pH was adjusted to 7 by adding Na₂CO₃ saturated aqueous solution. The resultant solution was then extracted with ethyl acetate (20 mL × 3). The organic phases were combined, washed with 50 mL of saturated saline, dried over sodium sulfate, filtered, and concentrated under reduced pressure. The concentrate was purified by silica gel column chromatography to obtain intermediate **4-4** (340 mg, yield: 75%). Ms: 456.1 (M+H+).

### Step 4: Synthesis of compound tert-butyl (S-2-(4-fluoro-3,5-dimethylphenyl)-4-methyl-3-(4-methyl-3-(1-methyl-1H-indazole-5-yl)-2-oxo-2,3-dihydro-1H-imidazole-1-yl)-2,4,6,7-tetrahydro-5H-pyrazolo[4,3-c]pyridine-5-carboxylate (4-5)

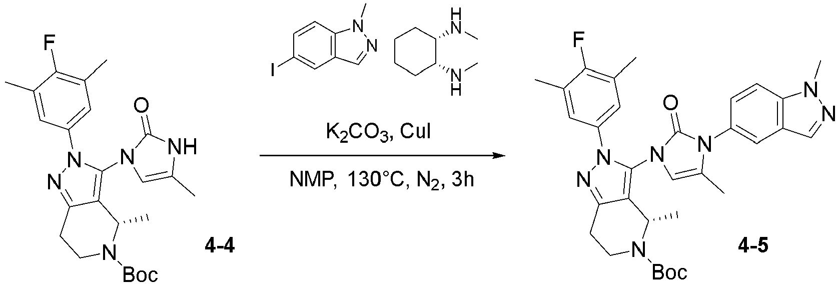

Compound **4-4** (75 mg, 0.16 mmol), 5-iodo-1-methyl-1*H*-indazole (104 mg, 0.317 mmol), and (1S,2S)-*N1,N2*-dimethylcyclohexane-1,2-diamine (17 mg, 0.079 mmol) were dissolved in *N-*methylpyrrolidone (10 mL), to which were then added CuI (9 mg, 0.032 mmol) and K₂CO₃ (94 mg, 0.476 mmol). The reaction solution was stirred at 130 °C under nitrogen protection for 3 h, and then cooled to room temperature, followed by adding 20 mL of water. The resultant solution was extracted with ethyl acetate (10 mL × 3). The organic phases were combined, washed with 20 mL of saturated saline, dried over sodium sulfate, filtered, and concentrated under reduced pressure. The concentrate was purified by silica gel column chromatography to obtain intermediate **4-5** (50 mg, 0.085 mmol), with a yield of 53%. M/S: 586.2 (M+H⁺).

### Step 5: Synthesis of intermediate (S)-1-(2-(4-fluoro-3,5-dimethylphenyl)-4-methyl-4,5,6,7-tetrahydro-2H-pyrazolo[4,3-c]pyridine-3-yl)-4-methyl-3-(1-methyl-1H-indazole-5-yl)-1,3-dihydro-2H-imidazole-2-one (4-6)

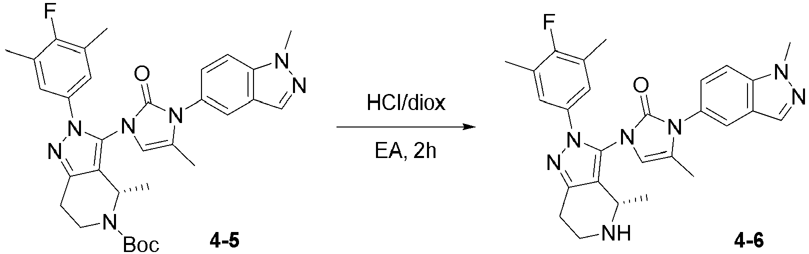

At room temperature, compound **4-5** (50 mg, 0.085 mmol) was placed in a 25 mL one-neck flask, to which was added a solution of HCl in 1,4-dioxane (5 mL, 4N), and then the mixture was stirred at room temperature for 2 h. K₂CO₃ aqueous solution was added to adjust the pH to 11. The resultant solution was extracted with ethyl acetate (10 mL × 3). The organic phases were combined, washed with 20 mL of saturated saline, dried over sodium sulfate, filtered, and concentrated under reduced pressure, to obtain intermediate **4-6** (40 mg, 0.084 mmol), with a yield of 97%. M/S: 485.1 (M+H⁺).

### Step 6. Synthesis of compound 3-((1S,2S)-1-(5-(S)-2,2-dimethyltetrahydro-2H-pyran-4-yl)-2-(S)-3-(3-(4-fluoro-1-methyl-1H-indazole-5-yl)-4-methyl-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-1,2,4-oxadiazole-5(4H)-one (4)

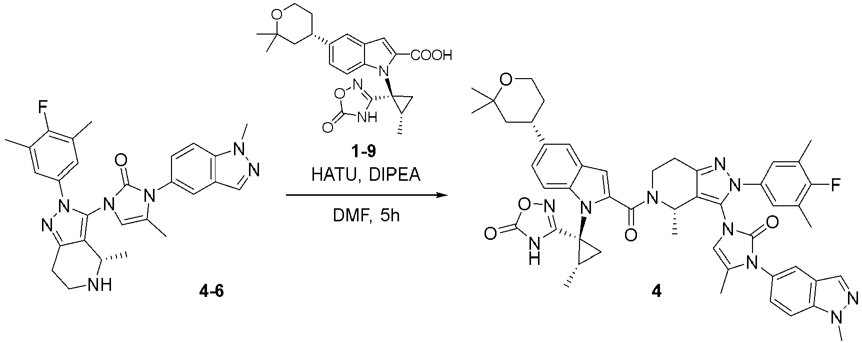

Intermediate **1-9** (33 mg, 0.08 mmol) was dissolved in DMF (10 mL), to which were added HATU (46 mg, 0.12 mmol) and N,N-diisopropylethylamine (31 mg, 0.24 mmol), and then stirred for 10 min, followed by adding compound **4-6** (40 mg, 0.08 mmol). The reaction solution was stirred at room temperature for 5 h. The reaction solution was quenched by adding 20 mL of water, and then extracted with ethyl acetate (10 mL × 3). The organic phases were combined, washed with 20 mL of saturated saline, dried over sodium sulfate, filtered, and concentrated under reduced pressure. The concentrate was purified by silica gel column chromatography to obtain compound **4** (20 mg, 0.023 mmol), with a yield of 28%. M/S: 879 (M+H⁺). ¹H NMR (400 MHz, DMSO-d₆) *δ* 11.76 (s, 1H), 8.31 (s, 1H), 7.64 (d, J = 8.8 Hz, 1H), 7.50 (dd, J = 17.5, 9.6 Hz, 2H), 7.40 (d, J = 8.4 Hz, 1H), 7.26 (d, J = 8.4 Hz, 1H), 7.18 (d, J = 6.4 Hz, 1H), 7.09 (d, J = 3.2 Hz, 1H), 6.95 (s, 2H), 4.11 (s, 3H), 3.71 (d, J = 8.4 Hz, 2H), 3.17 (d, J = 12.4 Hz, 1H), 3.06 - 2.99 (m, 1H), 2.92 - 2.86 (m, 1H), 2.26 (s, 6H), 1.99 (s, 1H), 1.54 (dd, J = 89.6, 9.6 Hz, 13H), 1.30 - 1.14 (m, 11H).

### Example 4. Synthesis of compound 3-((1S,2S)-1-(5-((S)-2,2-dimethyltetrahydro-2H-pyran-4-yl)-2-((4R,7S)-3-(3-(4-fluoro-1-methyl-1H-indazole-5-yl)-2-oxo-2,3-dihydro-1H-imidazole-1-yl)-2-(4-fluoro-3,5-dimethylphenyl)-2,4,5,6,7,8-hexahydro-4,7-bicyclohepta[c]pyrazole-9-carbonyl)-1H-indole-1-yl)-2-methylcyclopropyl)-1,2,4-oxadiazole-5(4H)-one (Compound 18)

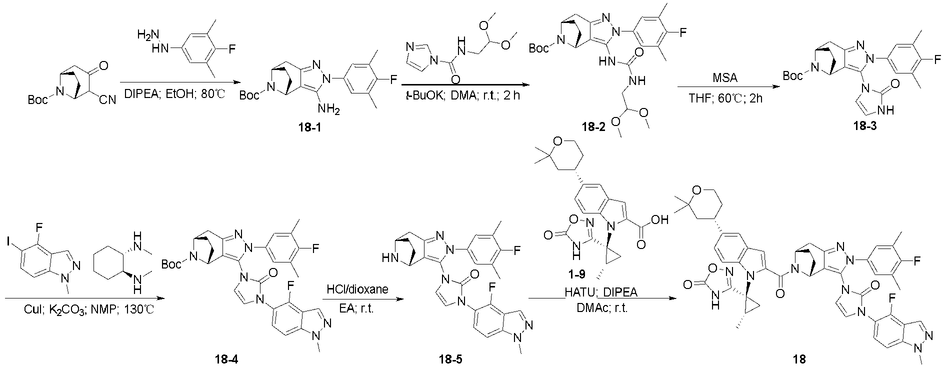

### Step 1. Synthesis of intermediate tert-butyl (4R,7S)-3-amino-2-(4-fluoro-3,5-dimethylphenyl)-2,4,5,6,7,8-hexahydro-4,7-bicyclohepta[c]pyrazole-9-carboxylate (18-1)

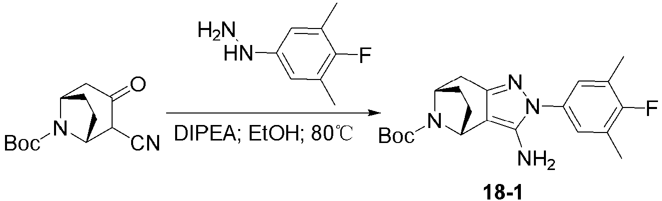

tert-Butyl (*1R,5S*)-2-cyano-3-oxo-8-azabicyclo[3.2.1]octane-8-carboxylate (2.5 g, 10 mmol) and (4-fluoro-3,5-dimethylphenyl)hydrazine hydrochloride (2.29 g, 12 mmol) were dissolved in ethanol (50 mL), to which was added N,N-diisopropylethylamine (2.84 g, 22 mmol), and then reacted and stirred at 80 °C for 12 h. After completion of the reaction, the reaction solution was diluted with 50 mL of water, and then extracted with ethyl acetate (50 mL×3). The organic phase was washed with 30 mL of saturated saline, dried over sodium sulfate, filtered, and concentrated under reduced pressure. The concentrate was purified by silica gel column chromatography, to obtain intermediate **18-1** (2 g, yield: 52%). M/S: 387.2 (M+H⁺).

### Step 2: Synthesis of intermediate tert-butyl (4R,7S)-3-(3-(2,2-dimethoxyethyl)urea)-2-(4-fluoro-3,5-dimethylphenyl)-2,4,5,6,7,8-hexahydro-4,7-bicyclohepta[c]pyrazole-9-carboxylate (18-2)

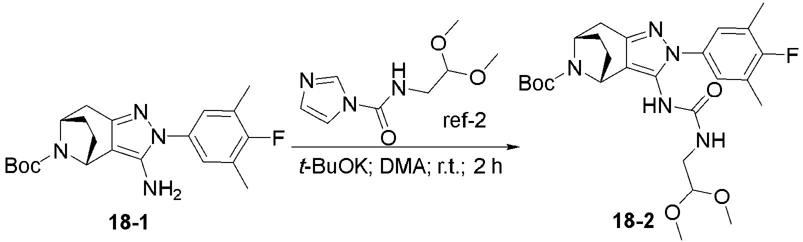

At room temperature, intermediate **18-1** (872 mg, 2.26 mmol), **ref-2** (900 mg, 4.25 mmol), and t-BuOK (633 mg, 5.65 mmol) were added to a solution of *N,N*-dimethylacetamide (20 mL), and then stirred at room temperature for 2 h. The reaction solution was diluted with 50 mL of water, and then extracted with ethyl acetate (20 mL × 3). The organic phase was washed with 50 mL of saturated saline, dried over sodium sulfate, filtered, and concentrated under reduced pressure. The concentrate was purified by silica gel column chromatography to obtain intermediate **18-2** (995 mg, yield: 85%). M/S: 518.3 (M+H⁺).

### Step 3: Synthesis of intermediate tert-butyl (4R,7S)-2-(4-fluoro-3,5-dimethylphenyl)-3-(2-oxo-2,3-dihydro-1H-imidazole-1-yl)-2,4,5,6,7,8-hexahydro-4,7-bicyclohepta[c]pyrazole-9-carboxylate (18-3)

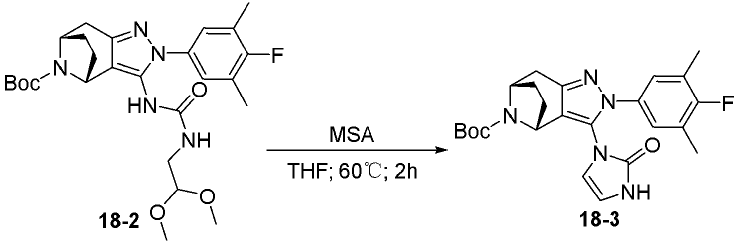

Compound **18-2** (880 mg, 1.70 mmol) was placed in a 50 mL one-neck flask, to which was added tetrahydrofuran (20 mL), followed by adding methanesulfonic acid (254 mg, 2.55 mmol), and then stirred and reacted at 60 °C for 2 h. After completion of the reaction, the reaction solution was cooled to room temperature, and then 50 mL of water was added. The pH was adjusted to 7 with Na₂CO₃ saturated aqueous solution. The resultant solution was extracted with ethyl acetate (30 mL × 3). The organic phases were combined, washed with 50 mL of saturated saline, dried over sodium sulfate, filtered, and concentrated under reduced pressure. The concentrate was purified by silica gel column chromatography to obtain intermediate **18-3** (710 mg, yield: 92%). Ms: 454.1 (M+H⁺).

### Step 4: Synthesis of compound tert-butyl (4R,7S)-3-(3-(4-fluoro-1-methyl-1H-indazole-5-yl)-2-oxo-2,3-dihydro-1H-imidazole-1-yl)-2-(4-fluoro-3,5-dimethylphenyl)-2,4,5,6,7,8-hexahydro-4,7-bicyclohepta[c]pyrazole-9-carboxylate (18-4)

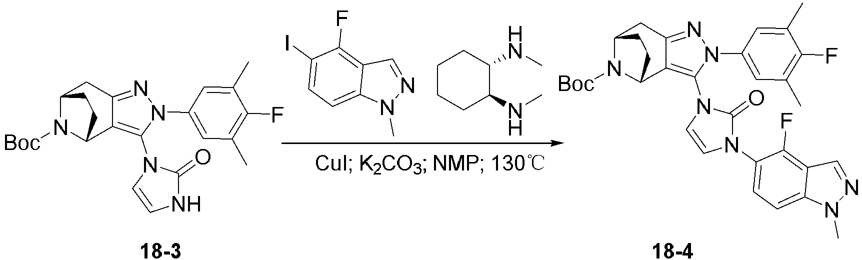

Compound **18-3** (680 mg, 1.50 mmol), 5-iodo-1-methyl-1*H*-indazole (705 mg, 2.56 mmol), and (1*S*,2*S*)-*N1,N2*-dimethylcyclohexane-1,2-diamine (214 mg, 1.50 mmol) were dissolved in *N-*methylpyrrolidone (10 mL), to which was then added CuI (115 mg, 0.6 mmol) and K₂CO₃ (478 mg, 2.26 mmol). The reaction solution was stirred at 130 °C for 2 h, and then cooled to room temperature, followed by adding 20 mL of water. The resultant solution was extracted with ethyl acetate (20 mL × 3). The organic phases were combined, washed with 50 mL of saturated saline, dried over sodium sulfate, filtered, and concentrated under reduced pressure. The concentrate was purified by silica gel column chromatography to obtain intermediate **18-4** (225 mg, 0.38 mmol), with a yield of 25%. M/S: 602.3 (M+H⁺).

### Step 5: Synthesis of 1-(4-fluoro-1-methyl-1H-indazole-5-yl)-3-((4R,7S)-2-(4-fluoro-3,5-dimethylphenyl)-2,4,5,6,7,8-hexahydro-4,7-bicyclohepta[c]pyrazole-3-yl)-1,3-dihydro-2H-imidazole-2-one (18-5)

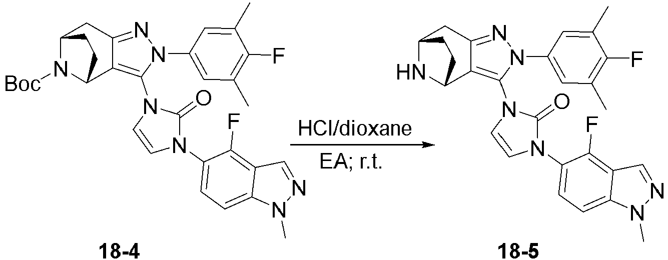

At room temperature, compound **18-4** (225 mg, 0.38 mmol) was placed in a 25 mL one-neck flask, to which was added a solution of HCl in 1,4-dioxane (10 mL, 4N), and then stirred and reacted at room temperature for 1 h. K₂CO₃ aqueous solution was added to adjust the pH to 11, followed by extraction with ethyl acetate (10 mL × 3). The organic phases were combined, washed with 20 mL of saturated saline, dried over sodium sulfate, filtered, and concentrated under reduced pressure to obtain intermediate **18-5** (184 mg, 0.37 mmol), with a yield of 97%. M/S: 502.3 (M+H⁺).

### Step 6: Synthesis of compound 3-((1S,2S)-1-(5-((S)-2,2-dimethyltetrahydro-2H-pyran-4-yl)-2-((4R,7S)-3-(3-(4-fluoro-1-methyl-1H-indazole-5-yl)-2-oxo-2,3-dihydro-1H-imidazole-1-yl)-2-(4-fluoro-3,5-dimethylphenyl)-2,4,5,6,7,8-hexahydro-4,7-bicyclohepta[c]pyrazole-9-carbonyl)-1H-indole-1-yl)-2-methylcyclopropyl)-1,2,4-oxadiazole-5(4H)-one (18)

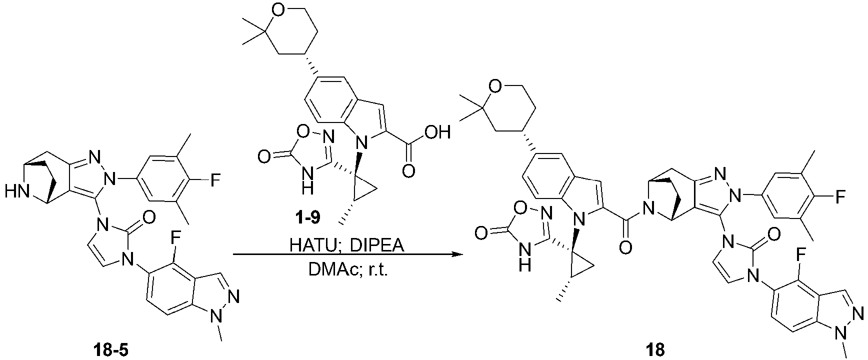

Intermediate **1-9** (40 mg, 0.1 mmol) was dissolved in DMAc (10 mL), to which were added HATU (44 mg, 0.12 mmol) and *N,N*-diisopropylethylamine (20 mg, 0.015 mmol), and then stirred for 10 min, followed by adding compound **18-5** (50 mg, 0.1 mmol). The mixture was stirred and reacted at room temperature for 2 h. The reaction solution was quenched by adding 20 mL of water, and then extracted with ethyl acetate (10 mL × 3). The organic phases were combined, washed with 20 mL of saturated saline, dried over sodium sulfate, filtered, and concentrated under reduced pressure. The concentrate was purified by silica gel column chromatography to obtain compound **18** (44 mg, 0.049 mmol), with a yield of 97%. M/S: 895.4 (M+H⁺).

### Example 5. Synthesis of compound 3-((1S,2S)-1-(2-((4R,7S)-2-(3-cyclopropyl-4-fluorophenyl)-3-(3-(4-fluoro-1-methyl-1H-indazole-5-yl)-2-oxo-2,3-dihydro-1H-imidazole-1-yl)-2,4,5,6,7,8-hexahydro-4,7-bicyclohepta[c]pyrazole-9-carbonyl)-5-((S)-2,2-dimethyltetrahydro-2H-pyran-4-yl)-1H-indole-1-yl)-2-methylcyclopropyl)-1,2,4-oxadiazole-5(4H)-one (19)

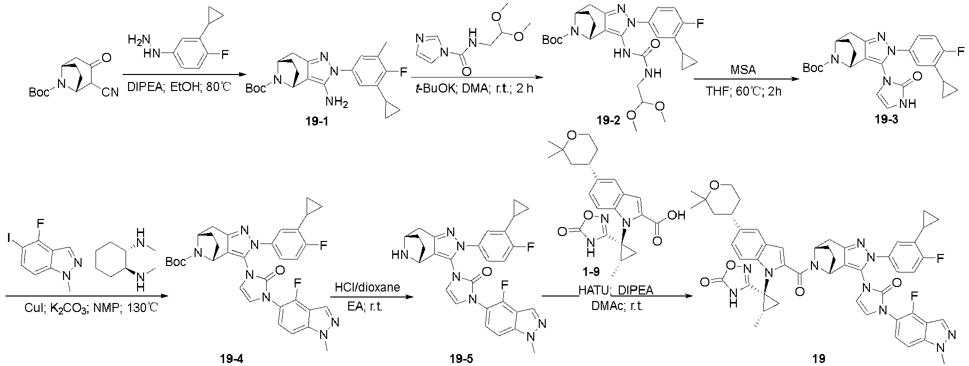

### Step 1. Synthesis of intermediate tert-butyl (4R,7S)-3-amino-2-(3-cyclopropyl-4-fluorophenyl)-2,4,5,6,7,8-hexahydro-4,7-bicyclohepta[c]pyrazole-9-carboxylate (19-1)

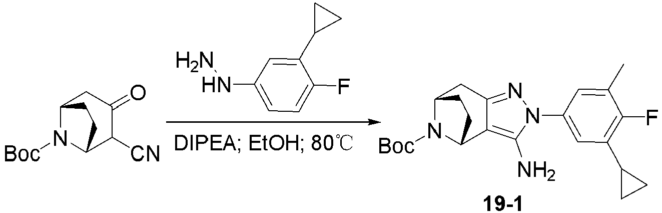

tert-Butyl (1*R*,5*S*)-2-cyano-3-oxo-8-azabicyclo[3.2.1]octane-8-carboxylate (3.5 g, 13.98 mmol) and (3-cyclopropyl-4-fluorophenyl)hydrazine hydrochloride (3.4 g, 16.78 mmol) were dissolved in ethanol (50 mL), to which was added N,N-diisopropylethylamine (3.61 g, 27.97 mmol), and then reacted and stirred at 80 °C for 12 h. After completion of the reaction, the reaction solution was diluted with 50 mL of water, and then extracted with ethyl acetate (50 mL×3). The organic phase was washed with 30 mL of saturated saline, dried over sodium sulfate, filtered, and concentrated under reduced pressure. The concentrate was purified by silica gel column chromatography and subjected to chiral resolution to obtain intermediate 19-1 (1.2 g, yield: 21%). M/S: 403.2 (M+H⁺).

### Step 2: Synthesis of intermediate tert-butyl (4R,7S)-(2-(3-cyclopropyl-4-fluorophenyl)-3-(3-(2,2-dimethoxyethyl)ureido)-2,4,5,6,7,8-hexahydro-4,7-bicyclohepta[c]pyrazole-9-carboxylate (19-2)

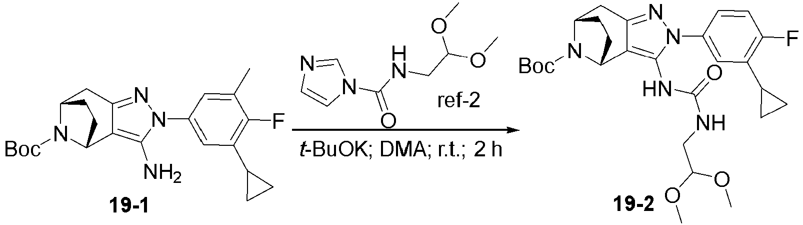

At room temperature, intermediate 19-1 (900 mg, 2.26 mmol), intermediate ref-2 (900 mg, 4.25 mmol), and t-BuOK (633 mg, 5.65 mmol) were added to N,N-dimethylacetamide (20 mL), and then stirred at room temperature for 2 h. The reaction solution was diluted with 50 mL of water, and then extracted with ethyl acetate (20 mL × 3). The organic phase was washed with 50 mL of saturated saline, dried over sodium sulfate, and filtered. The filtrate was concentrated under reduced pressure, and the concentrate was purified by silica gel column chromatography to obtain intermediate 19-2 (1.0 g, yield: 83%). M/S: 530.3 (M+H⁺).

### Step 3: Synthesis of intermediate tert-butyl (4R,7S)-(2-(3-cyclopropyl-4-fluorophenyl)-3-(2-oxo-2,3-dihydro-1H-imidazole-1-yl)-2,4,5,6,7,8-hexahydro-4,7-bicyclohepta[c]pyrazole-9-carboxylate (19-3)

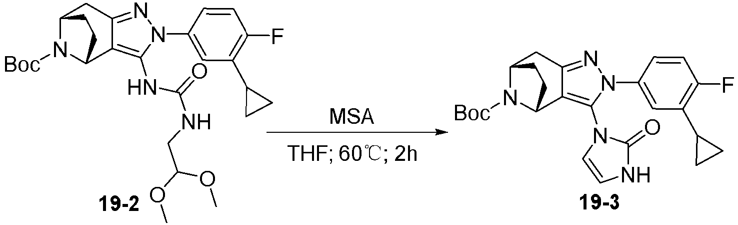

Compound **19-2** (900 mg, 1.70 mmol) was placed in a 50 mL one-neck flask, to which were successively added tetrahydrofuran (20 mL) and methanesulfonic acid (254 mg, 2.55 mmol), and then stirred and reacted at 60 °C for 2 h. After the reaction was complete, the reaction solution was cooled to room temperature, and then 50 mL of water was added. Its pH was adjusted to 7 with Na₂CO₃ saturated aqueous solution. The resultant solution was extracted with ethyl acetate (30 mL × 3). The organic phases were combined, washed with 50 mL of saturated saline, dried over sodium sulfate, filtered, and concentrated under reduced pressure. The concentrate was purified by silica gel column chromatography to obtain intermediate 19-3 (700 mg, yield: 88%). Ms: 466.1 (M+H⁺).

### Step 4: Synthesis of compound tert-butyl (4R,7S)-2-(3-cyclopropyl-4-fluorophenyl)-3(-3-(4-fluoro-1-methyl-1H-indazole-5-yl)-2-oxo-2,3-dihydro-1H-imidazole-1-yl)-2,4,5,6,7,8-hexahydro-4,7-bicyclohepta[c]pyrazole-9-carboxylate (19-4)

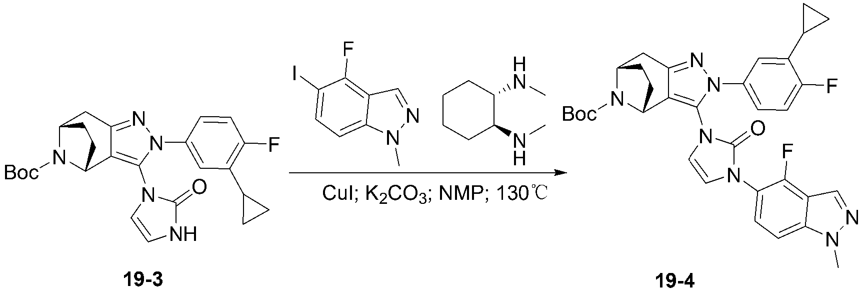

Compound **19-3** (700 mg, 1.50 mmol), 5-iodo-1-methyl-1H-indazole (705 mg, 2.56 mmol), and (1S,2S)-N1,N2-dimethylcyclohexane-1,2-diamine (214 mg, 1.50 mmol) were dissolved in N-methylpyrrolidone (10 mL), to which were then added CuI (115 mg, 0.6 mmol) and K₂CO₃ (478 mg, 2.26 mmol). The reaction solution was stirred at 130 °C for 2 h, and then cooled to room temperature, followed by adding 20 mL of water. The resultant solution was extracted with ethyl acetate (20 mL × 3). The organic phase was combined, washed with 20 mL of saturated saline, dried over sodium sulfate, and filtered. The filtrate was concentrated under reduced pressure, and the concentrate was purified by silica gel column chromatography to obtain intermediate 19-4 (230 mg, 0.38 mmol), with a yield of 25%. M/S: 614.3 (M+H⁺).

### Step 5: Synthesis of intermediate 1-((4R,7S)-(2-(3-cyclopropyl-4-fluorophenyl)-2,4,5,6,7,8-hexahydro-4,7-bicyclohepta[c]pyrazole-3-yl)-3-(4-fluoro-1-methyl-1H-indazole-5-yl)-1,3-dihydro-2H-imidazole-2-one (19-5)

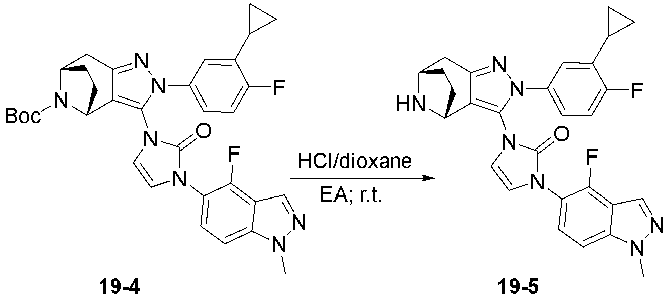

At room temperature, compound 19-4 (230 mg, 0.37 mmol) was placed in a 25 mL one-neck flask, to which was added the solution of HCl in 1,4-dioxane (10 mL, 4N), and then stirred at room temperature for 1 h. K₂CO₃ aqueous solution was added to adjust the pH to 11. The resultant solution was extracted with ethyl acetate (10 mL × 3). The organic phases were combined, washed with 50 mL of saturated saline, dried over sodium sulfate, filtered, and concentrated under reduced pressure, to obtain intermediate 19-5 (180 mg, 0.35 mmol), with a yield of 97%. M/S: 514.3 (M+H⁺).

### Step 6: Synthesis of compound 3-((1S,2S)-1-(2-((4R,7S)-2-(3-cyclopropyl-4-fluorophenyl)-3-(3-(4-fluoro-1-methyl-1H-indazole-5-yl)-2-oxo-2,3-dihydro-1H-imidazole-1-yl)-2,4,5,6,7,8-hexahydro-4,7-bicyclohepta[c]pyrazole-9-carbonyl)-5-((S)-2,2-dimethyltetrahydro-2H-pyran-4-yl)-1H-indole-1-yl)-2-methylcyclopropyl)-1,2,4-oxadiazole-5(4H)-one (19)

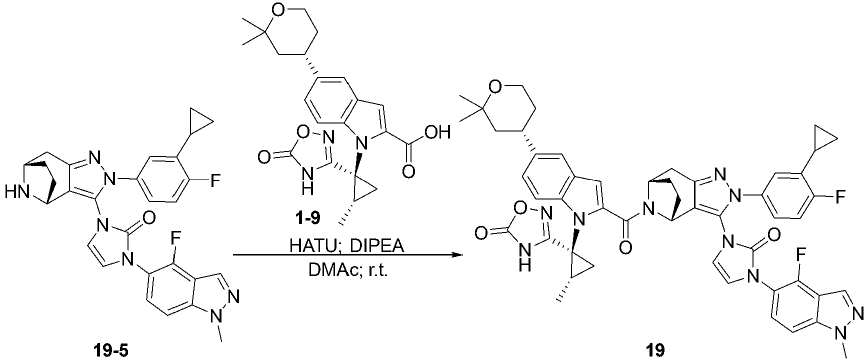

Intermediate 1-9 (38 mg, 0.09 mmol) was dissolved in DMAc (10 mL), to which were added HATU (44 mg, 0.12 mmol) and *N*,*N*-diisopropylethylamine (20 mg, 0.015 mmol), and then stirred for 10 min, followed by adding compound 19-5 (40 mg, 0.077 mmol). The mixture was stirred and reacted at room temperature for 2 h. The reaction solution was quenched by adding 20 mL of water. The resultant solution was extracted with ethyl acetate (10 mL × 3). The organic phase was combined, washed with 20 mL of saturated saline, dried over sodium sulfate, and filtered. The filtrate was concentrated under reduced pressure, and the concentrate was purified by silica gel column chromatography to obtain compound 19 (40 mg, 0.044 mmol), with a yield of 56%. M/S: 907.4 (M+H⁺).

### Example 6. Synthesis of compound 3-((1S,2S)-1-(5-((S)-2,2-dimethyltetrahydro-2H-pyran-4-yl)-2-((4S,7R)-3-(3-(4-fluoro-1-methyl-1H-indazole-5-yl)-2-oxo-2,3-dihydro-1H-imidazole-1-yl)-2-(4-fluoro-3,5-dimethylphenyl)-2,4,5,6,7,8-hexahydro-4,7-bicyclohepta[c]pyrazole-9-carbonyl)-1H-indole-1-yl)-2-methylcyclopropyl)-1,2,4-oxadiazole-5(4H)-one (30)

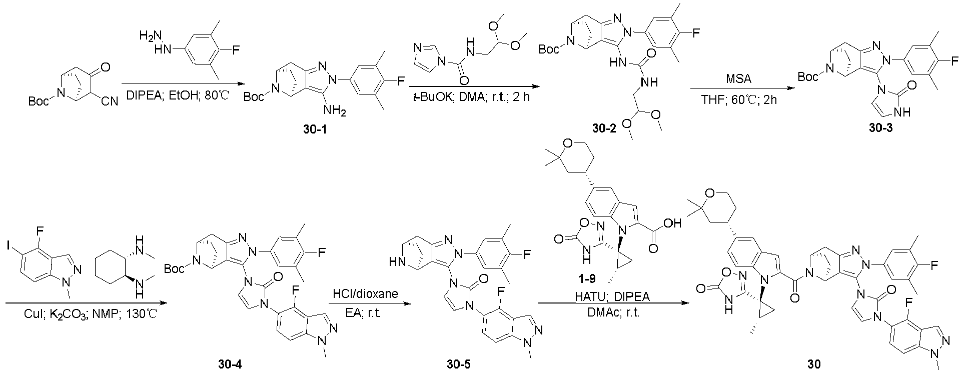

Using a method similar to that in Example 4, tert-butyl (1*R*,5*S*)-2-cyano-3-oxo-8-azabicyclo[3.2.1]octane-8-carboxylate was substituted with tert-butyl (1S,5R)-2-cyano-3-oxo-8-azabicyclo[3.2.1]octane-8-carboxylate, to obtain compound 30, with a yield of 49%. M/S: 895.4 (M+H⁺).

### Example 7. Synthesis of compound 3-((15,25)-1-(2-((4S,7R)-2-(3-cyclopropyl-4-fluorophenyl)-3-(3-(4-fluoro-1-methyl-1H-indazole-5-yl)-2-oxo-2,3-dihydro-1H-imidazole-1-yl)-2,4,5,6,7,8-hexahydro-4,7-bicyclohepta[c]pyrazole-9-carbonyl)-5-((S)-2,2-dimethyltetrahydro-2H-pyran-4-yl)-1H-indole-1-yl)-2-methylcyclopropyl)-1,2,4-oxadiazole-5(4H)-one (31)

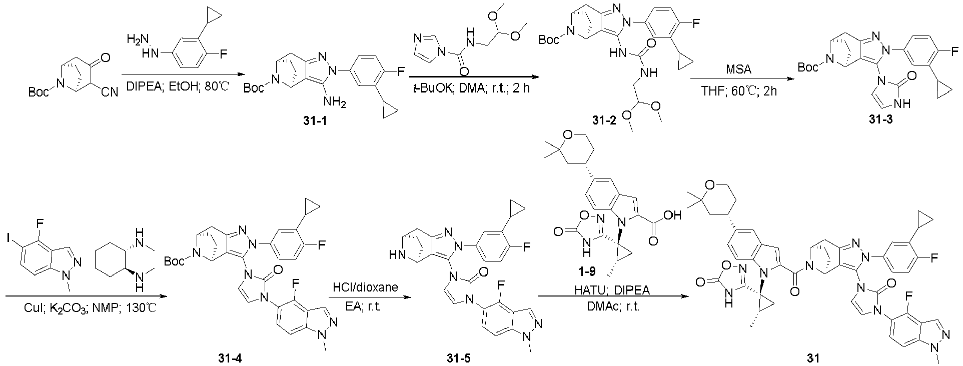

Using a method similar to that in Example 5, tert-butyl (1R,5S)-2-cyano-3-oxo-8-azabicyclo[3.2.1]octane-8-carboxylate was substituted with tert-butyl (1S,5R)-2-cyano-3-oxo-8-azabicyclo[3.2.1]octane-8-carboxylate, to obtain compound 31, with a yield of 56%. M/S: 907.4 (M+H⁺).

### Example 8. Synthesis of compound 3-(1-(7-((S)-2,2-dimethyltetrahydro-2H-pyran-4-yl)-2-((S)-3-(3-(4-fluoro-1-methyl-1H-indole-5-yl)-2-oxo-2,3-dihydro-1H-indole-1-yl)-2-(4-fluoro-3,5-dimethylphenyl)-4-methyl-4,5,6,7-tetrahydro-2H-pyrazolo[4,3-c]pyridine-5-carbonyl)indole-3-yl)cyclopropyl)-1,2,4-oxazole-5(4H)-one (41)

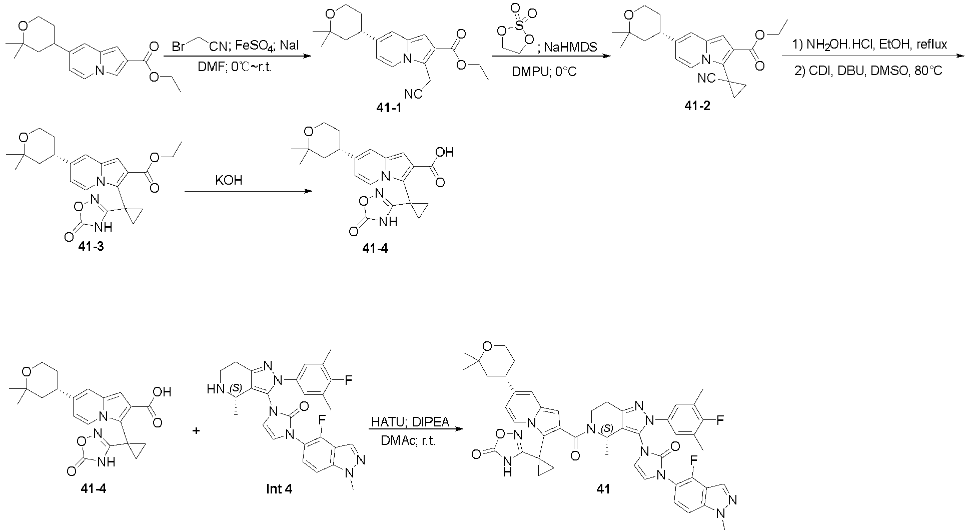

### Step 1: Synthesis of intermediate ethyl (S)-3-(cyanomethyl)-7-(2,2-dimethyltetrahydro-2H-pyran-4-yl)indole-2-carboxylate (41-1)

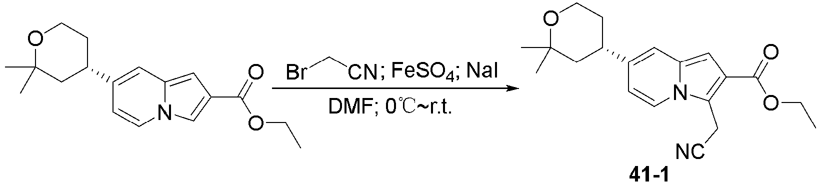

Ethyl (*S*)-7-(2,2-dimethyltetrahydro-2*H*-pyran-4-yl)indole-2-carboxylate (1.1 g, 3.65 mmol), 2-bromoacetonitrile (482 mg, 4.01 mmol), FeSO₄ hydrate (508 mg, 1.82 mmol), and NaI (547 mg, 3.65 mmol) were added to DMF (10 mL), and then cooled to 0 °C in an ice-water bath under N₂ protection, to which was added hydrogen peroxide (1.88 mL, 30%) dropwise. The reaction was stirred at 0 °C for 20 min, and then quenched by slowly pouring into ice water (20 mL). The resultant solution was extracted with ethyl acetate (20 mL × 3). The organic layers were combined, washed with saturated saline (50 mL × 2), dried over anhydrous sodium sulfate, and concentrated under reduced pressure. The residue was purified by column chromatography to obtain 530 mg of product. Yield: 43%. Ms: 341.3 (M+H⁺).

### Step 2: Synthesis of intermediate ethyl (S)-3-(1-cyanocyclopropyl)-7-(2,2-dimethyltetrahydro-2H-pyran-4-yl)indole-2-carboxylate (41-2)

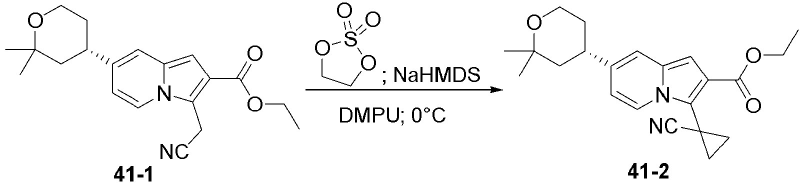

Intermediate **41-1** (425 mg, 1.25 mmol) and 1,3,2-dioxathiolane (387 mg, 3.12 mmol) were sequentially added to N,N-dimethylpropyleneurea (4 mL), and then cooled to 0 °C in an ice-water bath under N₂ protection, to which was added sodium bis(trimethylsilyl)amide (2.5 mL, 5.0 mmol) dropwise. The mixture was allowed to react at 0 °C for 4 h. The reaction was then quenched by slowly adding NH₄Cl saturated aqueous solution (10 mL). The resultant solution was extracted with ethyl acetate (10 mL × 2). The organic layers were combined, washed once with saturated saline (10 mL), dried over anhydrous sodium sulfate, and concentrated under reduced pressure. The residue was purified by column chromatography, to obtain 330 mg of product, with a yield of 75%. Ms: 367.2 (M+H⁺).

### Step 3: Synthesis of intermediate ethyl (5)-7-(2,2-dimethyltetrahydro-2H-pyran-4-yl)-3-(1-(5-oxo-4,5-dihydro-1,2,4-oxadiazole-3-yl)cyclopropyl)indole-2-carboxylate (41-3)

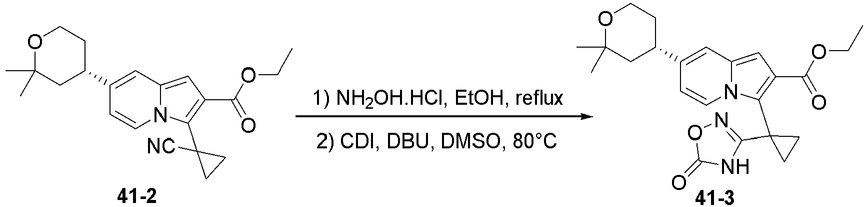

Intermediate **41-2** (249 mg, 0.68 mmol), hydroxylamine hydrochloride (235 mg, 3.4 mmol), and K₂CO₃ (516 mg, 3.74 mmol) were sequentially added to ethanol (6 mL), and then allowed to react under reflux for 3 h. The reaction solution was cooled to room temperature, and then water (20 mL) was added. The resultant solution was extracted with ethyl acetate (10 mL × 2). The organic layers were combined, washed once with saturated saline (20 mL), dried over anhydrous sodium sulfate, and concentrated under reduced pressure to obtain a crude product. The crude product was dissolved in dimethyl sulfoxide (5 mL), and then N,N'-carbonyldiimidazole (162 mg, 1.0 mmol) and 1,8-diazabicyclo[5,4,0]undec-7-ene (213 mg, 1.4 mmol) were sequentially added. The mixture was reacted at 80 °C for 3 h, and then cooled to room temperature, followed by adding water (20 mL). The resultant solution was extracted with ethyl acetate (10 mL × 2). The organic layers were combined, washed once with saturated saline (20 mL), dried over anhydrous sodium sulfate, and concentrated under reduced pressure. The residue was purified by column chromatography to obtain 205 mg of product, with a yield of 73.4%. Ms: 426.2 (M+H⁺).

### Step 4: Synthesis of intermediate (S)-7-(2,2-dimethyltetrahydro-2H-pyran-4-yl)-3-(1-(5-oxo-4,5-dihydro-1,2,4-oxadiazole-3-yl)cyclopropyl)indole-2-carboxylic acid (41-4)

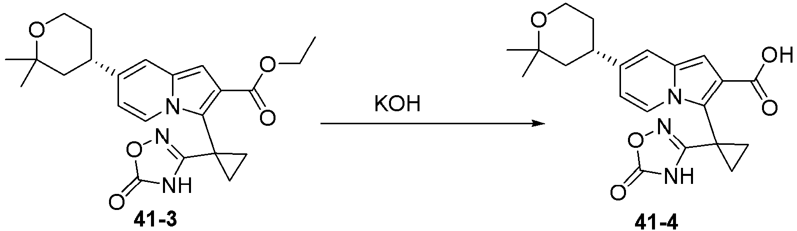

Intermediate **41-3** (212 mg, 0.5 mmol) and KOH (280 mg, 5.0 mmol) were successively added to 2-methoxyethanol (10 mL), and then reacted at 125 °C for 1 h. The reaction solution was cooled to room temperature, and then 2N hydrochloric acid was added dropwise to adjust pH to 3, followed by adding water (10 mL). Solid was precipitated and filtered. The filter cake was dried to obtain 151 mg of product. Yield: 76.0%. Ms: 398.2 (M+H⁺).

### Step 5: Synthesis of compound 3-(1-(7-((S)-2,2-dimethyltetrahydro-2H-pyran-4-yl)-2-((S)-3-(3-(4-fluoro-1-methyl-1H-indole-5-yl)-2-oxo-2,3-dihydro-1H-indole-1-yl)-2-(4-fluoro-3,5-dimethylphenyl)-4-methyl-4,5,6,7-tetrahydro-2H-pyrazolo[4,3-c]pyridine-5-carbonyl)indole-3-yl)cyclopropyl)-1,2,4-oxazole-5(4H)-one (41)

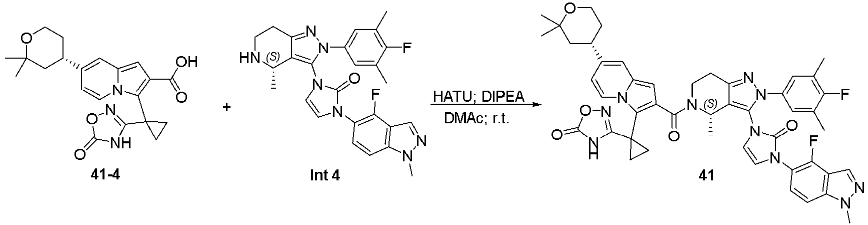

Intermediate **41-4** (49 mg, 0.1 mmol), intermediate Int 4 (49 mg, 0.1 mmol), HATU (46 mg, 0.12 mmol), and N,N-diisopropylethylamine (26 mg, 0.2 mmol) were sequentially added to *N,N-*dimethylacetamide (1 mL) and reacted at room temperature for 4 h. The reaction solution was then diluted with ethyl acetate (15 mL), and then washed once with water (10 mL) and once with saturated saline (10 mL). The organic layer was dried over anhydrous sodium sulfate, and concentrated under reduced pressure. The residue was purified by column chromatography to obtain 42 mg of product. Yield: 47.7%. Ms: 880.6 (M+H⁺).

¹H NMR (400 MHz, DMSO-*d*₆) *δ* 12.02 (s, 1H), 8.30 (s, 1H), 8.16 (d, *J=* 7.3 Hz, 1H), 7.64 (d, *J= 8.9* Hz, 1H), 7.48 - 7.41 (m, 1H), 7.33 - 7.20 (m, 3H), 7.10 (d, *J* = 3.2 Hz, 1H), 7.02 - 6.90 (m, 2H), 6.72 (dd, *J* = 7.3, 1.8 Hz, 1H), 6.42 (s, 1H), 5.69 - 5.61 (m, 1H), 4.15 - 4.00 (m, 4H), 3.75 - 3.64 (m, 2H), 3.39 (d, *J* = 11.0 Hz, 1H), 3.00 - 2.86 (m, 1H), 2.77 - 2.65 (m, 1H), 2.14 - 2.01 (m, 1H), 1.74 - 1.59 (m, 4H), 1.49 (q, *J =* 12.8 Hz, 1H), 1.34 (d, *J=* 6.6 Hz, 3H), 1.28 - 1.13 (m, 9H), 1.04 - 0.92 (m, 2H), 0.87 - 0.80 (m, 1H), 0.71 - 0.58 (m, 2H).

### Example 9. Synthesis of compound 3-(1-(2-((5)-2-(3-cyclopropyl-4-fluorophenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-7-((S)-2,2-dimethyltetrahydro-2H-pyran-4-yl)-indolizine-3-yl)cyclopropyl)-1,2,4-oxadiazole-5(4H)-one (42)

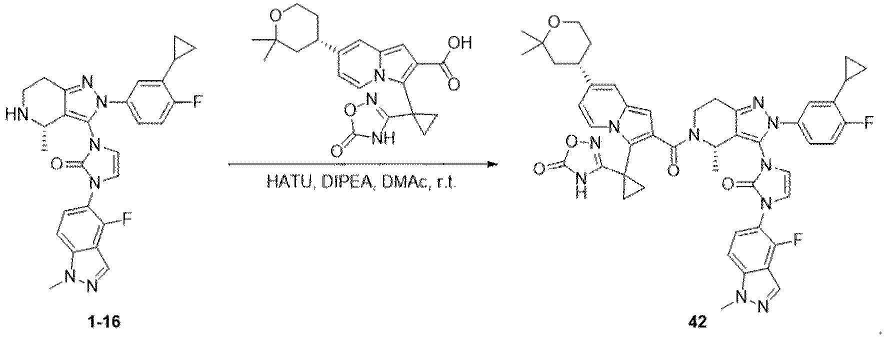

Procedures: Intermediate 1-16 (50 mg, 0.1 mmol), intermediate 41-4 (40 mg, 0.1 mmol), HATU (46 mg, 0.12 mmol), and N,N-diisopropylethylamine (26 mg, 0.2 mmol) were sequentially added to N,N-dimethylacetamide (1 mL) and reacted at room temperature for 4 h. The reaction solution was then diluted with ethyl acetate (15 mL), and then washed once with water (10 mL), and once with saturated saline (10 mL). The organic layer was dried over anhydrous sodium sulfate, concentrated under reduced pressure, and purified by column chromatography to obtain 42 mg of product. Yield: 47.7%. Ms: 880.6 (M+H⁺). ¹H NMR (400 MHz, DMSO-*d*₆) *δ* 12.02 (s, 1H), 8.30 (s, 1H), 8.16 (d, *J= 7.3* Hz, 1H), 7.64 (d, *J=* 8.9 Hz, 1H), 7.48 - 7.41 (m, 1H), 7.33 - 7.20 (m, 3H), 7.10 (d, *J =* 3.2 Hz, 1H), 7.02 - 6.90 (m, 2H), 6.72 (dd, *J* = 7.3, 1.8 Hz, 1H), 6.42 (s, 1H), 5.69 - 5.61 (m, 1H), 4.15 - 4.00 (m, 4H), 3.75 - 3.64 (m, 2H), 3.39 (d, *J =* 11.0 Hz, 1H), 3.00 - 2.86 (m, 1H), 2.77 - 2.65 (m, 1H), 2.14 - 2.01 (m, 1H), 1.74 - 1.59 (m, 4H), 1.49 (q, *J =* 12.8 Hz, 1H), 1.34 (d, *J* = 6.6 Hz, 3H), 1.28 - 1.13 (m, 9H), 1.04 - 0.92 (m, 2H), 0.87 - 0.80 (m, 1H), 0.71 - 0.58 (m, 2H).

### Example 10. Synthesis of compound 3-(1-(7-(S)-2,2-dimethyltetrahydro-2H-pyran-4-yl)-2-((4R,7S)-3-(3-(4-fluoro-1-methyl-1H-indazole-5-yl)-2-oxo-2,3-dihydro-1H-imidazole-1-yl)-2-(4-fluoro-3,5-dimethylphenyl)-2,4,5,6,7,8-hexahydro-4,7-bicyclohepta[c]pyrazole-9-carbonyl)indazole-3-yl)cyclopropyl)-1,2,4-oxazole-5(4H)-one (59)

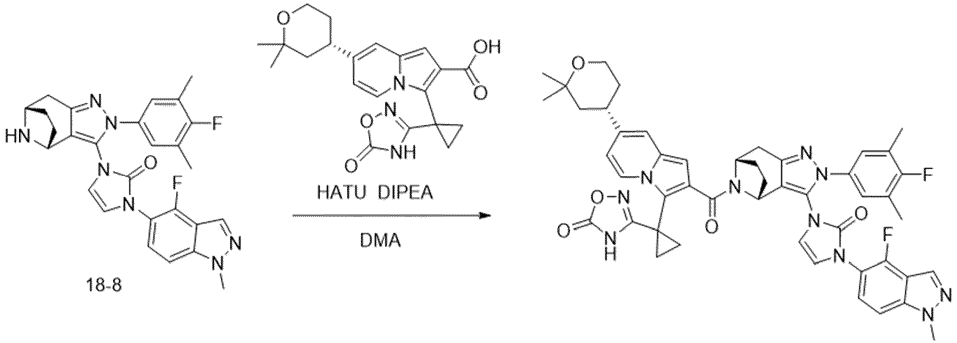

Compounds 18-8 (60 mg, 0.12 mmol) and 41-4 (53 mg, 0.13 mmol) were dissolved in DMA (3 mL), to which were added HATU (69 mg, 0.18 mmol) and DIPEA (31 mg, 0.24 mmol), and then reacted at room temperature for 6 h. The reaction was monitored by TLC, and then quenched by adding water (20 mL). The resultant solution was extracted with EA (10 mL × 3). The EA layers were combined, washed with water (20 mL × 2) and saturated saline (20 mL × 2), dried over anhydrous sodium sulfate, and concentrated. The residue was subjected to column chromatography to obtain 45 mg of white solid, with a yield of 42.7%. M/S: 881 (M+H⁺). ¹H NMR (400 MHz, DMSO-*d*₆) *δ* 12.04 (s, 1H), 8.39 - 8.21 (m, 1H), 8.13 (dd, *J* = 32.7, 7.2 Hz, 1H), 7.68 - 7.45 (m, 1H), 7.35 - 7.11 (m, 3H), 6.99 (dd, *J* = 49.9, 21.3 Hz, 3H), 6.69 (d, *J =* 32.3 Hz, 1H), 6.61 - 6.36 (m, 1H), 5.06 (d, *J =* 35.1 Hz, 1H), 4.10 (s, 3H), 3.79 - 3.35 (m, 4H), 2.97 - 2.57 (m, 2H), 2.42-2.16 (m, 7H), 2.06 (d, *J =* 9.5 Hz, 3H), 1.83 - 1.34 (m, 6H), 1.35 - 1.11 (m, 6H), 1.08 (d, *J* = 6.9 Hz, 2H).

### Example 11. Synthesis of compound 3-(1-(2-((4R,7S)-2-(3-cyclopropyl-4-fluoropheny)-3-(3-(4-fluoro-1-methyl-1H-indazole-5-yl)-2-oxo-2,3-dihydro-1H-imidazole-1-yl)-2,4,5,6,7,8-hexahydro-4,7-bicyclohepta [c]pyrazole-9-carbonyl)-7-((S)-2,2-dimethyltetrahydro-2H-pyran-4-yl)indoline-3-yl)cyclopropyl)-1,2,4-oxadiazole-5(4H)-one (60):

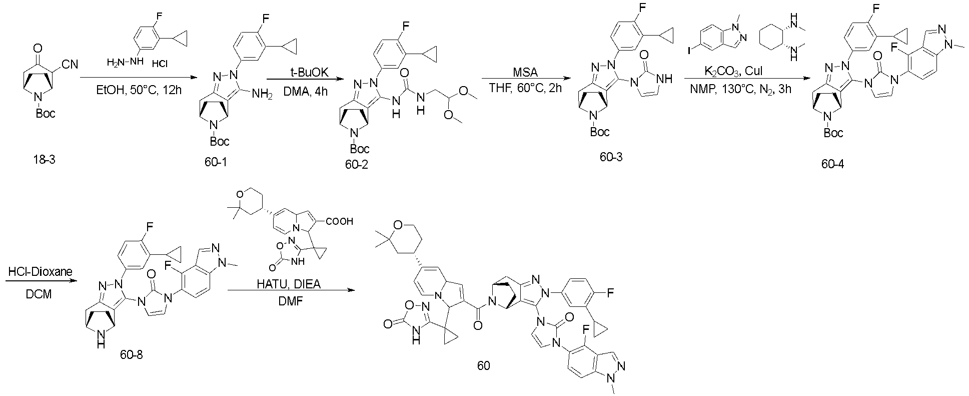

### Step 1: Synthesis of intermediate tert-butyl (4R,7S)-3-amino-2-(3-cyclopropyl-4-fluorophenyl)-2,4,5,6,7,8-hexahydro-4,7-bicyclohepta[c]pyrazole-9-carboxylate:

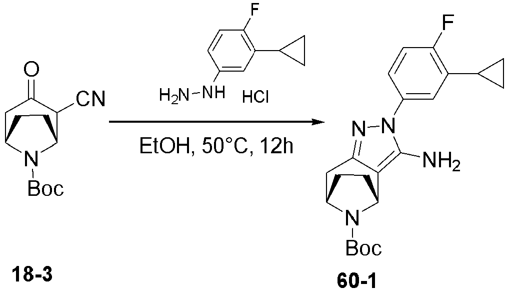

Compound **18-3** (3.2 g, 12.8 mmol) and (3-cyclopropyl-4-fluorophenyl)hydrazine hydrochloride (2.9 g, 14.2 mmol) were dissolved in ethanol (50 mL) and stirred at 50 °C for 12 h. After completion of the reaction, the solution was diluted with 50 mL of water, and then extracted with ethyl acetate (50 mL × 3). The organic phases were combined, washed with 50 mL of saturated saline, dried over sodium sulfate, filtered, and concentrated under reduced pressure. The residue was subjected to column chromatography, to obtain intermediate 60-1 (2.8 g, 5.3 mmol). Yield: 55%. M/S: 399 (M+H⁺).

### Step 2: Synthesis of intermediate tert-butyl (4R,7S)-3-(3-(2,2-dimethoxyethyl)ureido)-2-(3-cyclopropyl-4-fluorophenyl)-2,4,5,6,7,8-hexahydro-4,7-bicyclohepta[c]pyrazole-9-carboxylate (60-2)

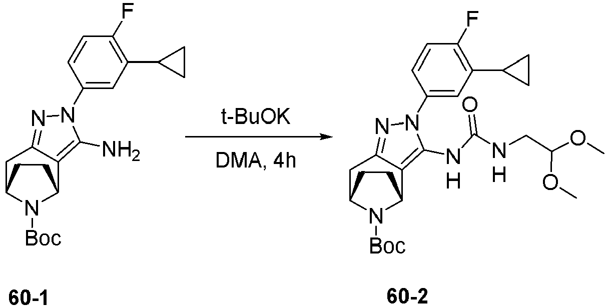

At room temperature, intermediate 60-1 (2.8 g, 7.1 mmol), N-(2,2-dimethoxyethyl)-1H-imidazole-1-formamide (3 g, 14.2 mmol), and potassium tert-butoxide (2.9 g, 21.3 mmol) were dissolved in N,N-dimethylacetamide (30 mL) and stirred at room temperature for 4 h. The reaction solution was diluted with 50 mL of water, and then extracted with ethyl acetate (30 mL × 3). The organic phase was washed with 50 mL of saturated saline, dried over sodium sulfate, and filtered. The filtrate was concentrated under reduced pressure. The residue was subjected to column chromatography, to obtain intermediate 60-2 (2.5 g, 4.7 mmol), with a yield of 67.6%. M/S: 530 (M+H⁺).

### Step 3: Synthesis of intermediate tert-butyl (4R,7S)-2-(3-cyclopropyl-4-fluorophenyl)-3-(2-oxo-2,3-dihydro-1H-imidazole-1-yl)-2,4,5,6,7,8-hexahydro-4,7-bicyclohepta[c]pyrazole-9-carboxylate (60-3)

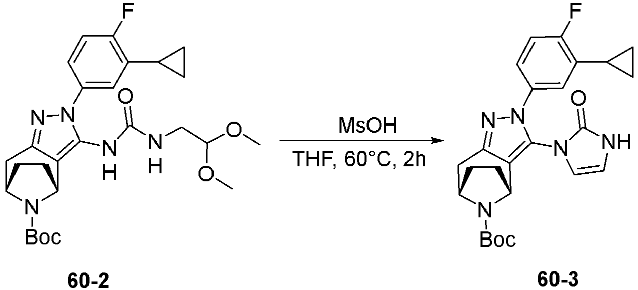

Compound **60-2** (2.5 g, 4.7 mmol) was placed in a 100 mL one-neck flask, to which was added tetrahydrofuran (50 mL), followed by adding methanesulfonic acid (454 mg, 4.7 mmol), and then stirred at 60°C for 2 h. After completion of the reaction, the reaction solution was cooled to room temperature, and then 50 mL of water was added. Na₂CO₃ saturated aqueous solution was added to adjust the pH to 7. The resultant solution was extracted with ethyl acetate (30 mL × 3). The organic phases were combined, washed with 50 mL of saturated saline, dried over sodium sulfate, filtered, and concentrated under reduced pressure. The residue was subjected to column chromatography to obtain intermediate 60-3 (1.9 g, 2.4 mmol), with a yield of 86.4%. Ms: 466 (M+H⁺).

### Step 4: Synthesis of intermediate tert-butyl (4R,7S)-3-(3-(4-fluoro-1-methyl-1H-indazole-5-yl)-2-oxo-2,3-dihydro-1H-imidazole-1-yl)-2-(3-cyclopropyl-4-fluorophenyl)-2,4,5,6,7,8-hexahydro-4,7-bicyclohepta[c]pyrazole-9-carboxylate (60-4)

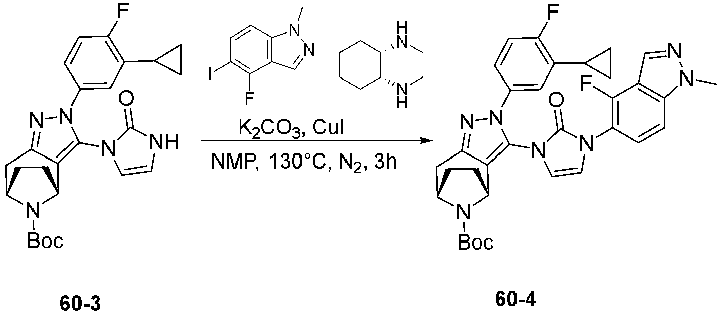

Compound **60-3** (1.9 g, 4.1 mmol), 5-iodo-4-fluoro-1-methyl-1H-indazole (2.2 g, 8.2 mmol), and (1S,2S)-N1,N2-dimethylcyclohexane-1,2-diamine (581 mg, 4.1 mmol) were dissolved in N-methylpyrrolidone (20 mL), to which were then added CuI (779 mg, 4.1 mmol) and K₂CO₃ (1698 mg, 12.3 mmol). The reaction solution was stirred at 130 °C for 3 h under nitrogen protection, and then cooled to room temperature, followed by adding 30 mL of water. The resultant solution was extracted with ethyl acetate (30 mL × 3). The organic phase was combined, washed with 20 mL of saturated saline, dried over sodium sulfate, and filtered. The filtrate was concentrated under reduced pressure, and the concentrate was subjected to column chromatography to obtain intermediate 60-4 (1349 mg, 2.2 mmol), with a yield of 54%. M/S: 614 (M+ H⁺).

### Step 5: Synthesis of intermediate 1-(4-fluoro-1-methyl-1H-indazole-5-yl)-3-((4R,7S)-2-(3-cyclopropyl-4-fluorophenyl)-2,4,5,6,7,8-hexahydro-4,7-bicyclohepta[c]pyrazole-3-yl)-1,3-dihydro-2H-imidazole-2-one (60-5)

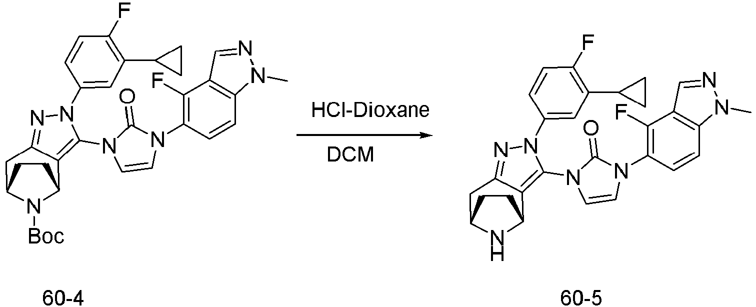

Compound 60-4 (1.35 mg, 2.2 mmol) was dissolved in DCM (4 mL) at room temperature, to which was added a solution of hydrochloric acid in 1,4-dioxane (6 mL, 4N), and then stirred at room temperature for 1 h. The reaction was monitored by TLC, and then NaHCO₃ solution was added to adjust pH to 10. The resultant solution was extracted with ethyl acetate (10 mL × 3). The organic phases were combined, washed with 20 mL of saturated saline, dried over sodium sulfate, and filtered. The filtrate was concentrated under reduced pressure to obtain intermediate 60-5 (1 g, 1.95 mmol), with a yield of 90%. M/S: 514 (M+H⁺).

### Step 6: Synthesis of compound 3-((1S,2S)-1-(5-((S)-2,2-dimethyltetrahydro-2H-pyran-4-yl)-2-((4R,7S)-3-(3-(4-fluoro-1-methyl-1H-indazole-5-yl)-2-oxo-2,3-dihydro-1H-imidazole-1-yl)-2-(3-cyclopropyl-4-fluorophenyl)-2,4,5,6,7,8-hexahydro-4,7-bicyclohepta[c]pyrazole-9-carbonyl)-1H-indole-1-yl)-2-methylcyclopropyl)-1,2,4-oxadiazole-5(4H)-one (60)

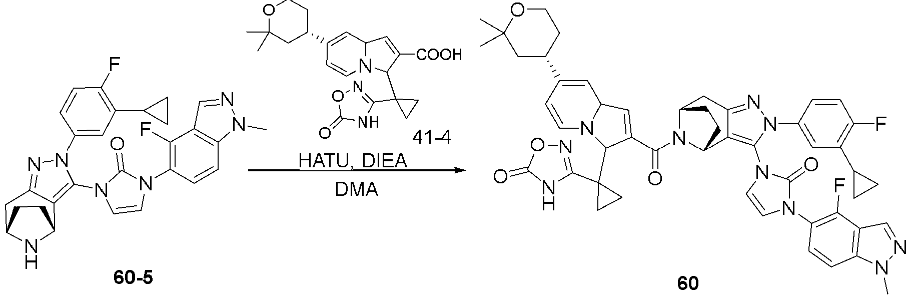

Intermediate 60-5 (62 mg, 0.12 mmol) and intermediate 41-4 (54 mg, 0.13 mmol) were dissolved in DMA (5 mL), to which were added HATU (91 mg, 0.24 mmol) and DIPEA (31 mg, 0.24 mmol), and then stirred at room temperature for 2 h. The reaction was quenched by adding 30 mL of water. The resultant solution was extracted with ethyl acetate (20 mL × 3). The organic phases were combined, washed with water (20 mL × 3) and saturated saline (20 mL × 2), dried over anhydrous sodium sulfate, and concentrated. The residue was purified by column chromatography to obtain compound 60 (40 mg, 0.045 mmol), with a yield of 36.5%. M/S: 907 (M+H⁺). ¹H NMR (400 MHz, DMSO-*d*₆) *δ* 12.02 (s, 1H), 8.36 - 8.22 (m, 1H), 8.10 (dd, *J=* 22.1, 7.3 Hz, 1H), 7.58 (ddd, *J =* 28.5, 19.5, 8.4 Hz, 1H), 7.30 (s, 1H), 7.15 (dt, *J* = 12.6, 6.3 Hz, 2H), 7.10 - 6.83 (m, 3H), 6.79 - 6.57 (m, 1H), 6.57 - 6.29 (m, 1H), 5.06 (d, *J=* 36.8 Hz, 1H), 4.10 (t, *J= 4.0* Hz, 3H), 3.59 (dd, *J =* 92.6, 9.0 Hz, 3H), 3.11 - 2.73 (m, 2H), 2.38-2.16 (m, 7H), 2.17 - 1.89 (m, 3H), 1.73 - 1.35 (m, 5H), 1.29-1.13 (m, 6H), 0.96 (d, *J =* 18.6 Hz, 2H), 0.89 - 0.78 (m, 2H).

### Example 12. Synthesis of compound 3-((1S,2S)-1-(5-((S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-7-methyl-4,5,6,7-tetrahydro-2H-pyrazolo[4,3-c]pyridine-5-carbonyl)-1H-indole-1-yl)-2-methylcyclopropyl)-1,2,4-oxazole-5(4H)-one (118)

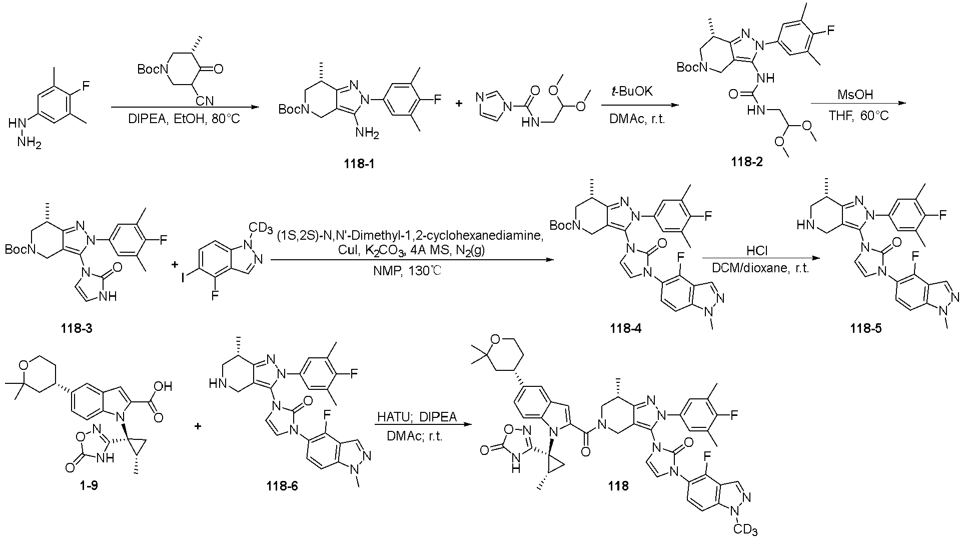

### Step 1: Synthesis of intermediate tert-butyl (S)-3-amino-2-(4-fluoro-3,5-dimethylphenyl)-7-methyl-2,4,6,7-tetrahydro-5H-pyrazolo[4,3-c]pyridine-5-carboxylate (118-1)

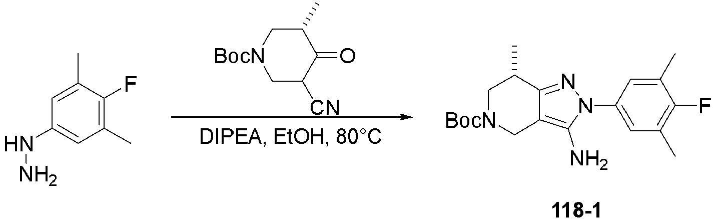

Intermediate tert-butyl (5S)-3-cyano-5-methyl-4-oxopiperidine-1-carboxylate (20 g, 84.0 mmol) was dissolved in ethanol (200 mL), to which were successively added (4-fluoro-3,5-dimethylphenyl)hydrazine hydrochloride (17.5 g, 92.4 mmol) and N,N-diisopropylethylamine (21.7 g, 168.0 mmol). Then, the reaction solution was warmed to 80 °C, reacted overnight, and concentrated under reduced pressure. The residue was purified by column chromatography to obtain 23 g of product, with a yield of 73.2%. Ms: 375.2 (M+H⁺).

### Step 2: Synthesis of intermediate tert-butyl (S)-3-(3-(2,2-dimethoxyethyl)urea)-2-(4-fluoro-3,5-dimethylphenyl)-7-methyl-2,4,6,7-tetrahydro-5H-pyrazolo[4,3-c]pyridine-5-carboxylate (118-2)

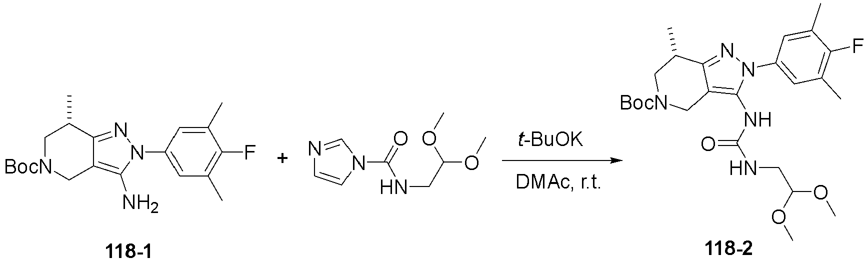

Intermediate **118-1** (2 g, 5.35 mmol), N-(2,2-dimethoxyethyl)-1H-imidazole-1-formamide (1.38 g, 6.42 mmol), and potassium tert-butoxide (0.9 g, 8.03 mmol) were sequentially added to DMAc (10 mL), and then stirred at room temperature. The reaction was monitored by TLC. After completion of the reaction, the reaction solution was cooled to room temperature, dropped into water, and then filtered. The resultant solid was purified by column chromatography to obtain 1.1 g of product. Yield: 40.7%. 506.1 (M+H⁺).

### Step 3: Synthesis of intermediate tert-butyl (S)-2-(4-fluoro-3,5-dimethylphenyl)-7-methyl-3-(2-oxo-2,3-dihydro-1H-imidazole-1-yl)-2,4,6,7-tetrahydro-5H-pyrazolo[4,3-c]pyridine-5-carboxylate (118-3)

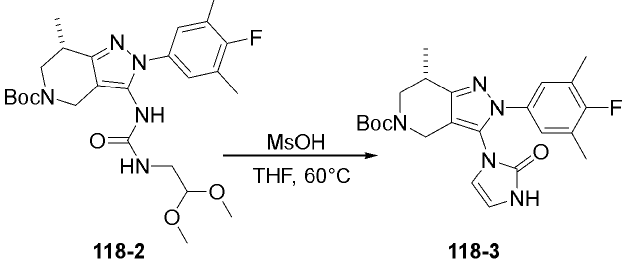

Intermediate **118-2** (1.1 g, 2.17 mmol) was dissolved in tetrahydrofuran (22 mL), to which was added methanesulfonic acid (0.21 g, 2.17 mmol) dropwise, and then stirred at 60 °C. The reaction was monitored by TLC. Water (110 mL) was added to the reaction, and the resultant solution was extracted with ethyl acetate (50 mL × 3). The organic layer was washed once with saturated saline (50 mL), dried over anhydrous sodium sulfate, and concentrated under reduced pressure. The residue was purified by column chromatography to obtain 0.92 g of product, with a yield of 96.1%. Ms: 442.1 (M+H⁺).

### Step 4: Synthesis of intermediate tert-butyl (S)-3-(3-(4-fluoro-1-(methyl-d3)-1H-imidazole-5-yl)-2-oxo-2,3-dihydro-1H-imidazole-1-yl)-2-(4-fluoro-3,5-dimethylphenyl)-7-methyl-2,4,6,7-tetrahydro-5H-pyrazolo[4,3-c]pyridine-5-carboxylate (118-4)

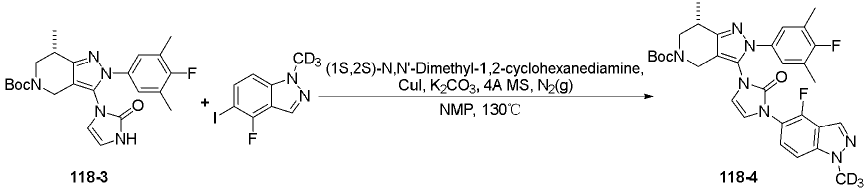

Intermediate **118-3** (0.92 g, 2 mmol), 4-fluoro-5-iodo-1-deuteromethyl-1/-indazole (1.1 g, 4 mmol), ligand (0.28 g, 2 mmol), CuI (0.38 g, 2 mmol), K₂CO₃ (0.82 g, 6 mmol), and molecular sieves (0.92 g) were sequentially added to NMP (10 mL), and under N₂ (g) protection, the mixture was reacted at 130 °C for 12 h, followed by adding ethyl acetate (50 mL) for dilution. The resultant solution was washed once with water (10 mL) and once with saturated saline (60 mL), dried over anhydrous sodium sulfate, and then concentrated under reduced pressure. The residue was purified by column chromatography to obtain 735 mg of product. Yield: 63.7%. Ms: 593.1 (M+H⁺).

### Step 5: Synthesis of intermediate (S)-1-(4-fluoro-1-(methyl-d3)-1H-indazole-5-yl)-3-(2-(4-fluoro-3,5-dimethylphenyl)-4-methyl-4,5,6,7-tetrahydro-2H-pyrazolo[4,3-c]pyridine-3-yl)-1,3-dihydro-2H-imidazole-2-one (118-5)

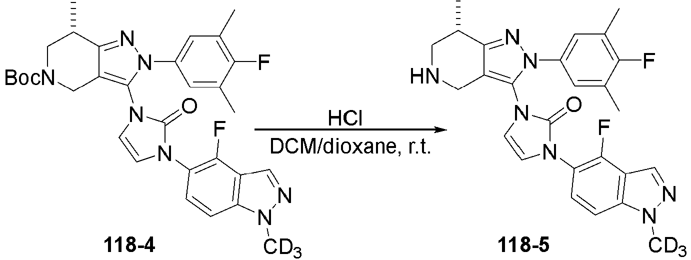

Intermediate **118-4** (680 mg, 1.15 mmol) was added to dichloromethane (7 ml) and a solution of HCl in 1,4-dioxane (4 M, 3 mL), and then reacted at room temperature for 2 h. NaHCO₃ saturated aqueous solution was added to adjust the pH to >7. The resultant solution was extracted with ethyl acetate (20 mL × 2). The organic layers were combined, washed with saturated saline (20 mL), dried over anhydrous sodium sulfate, and concentrated under reduced pressure to obtain 505 mg of product. Yield: 89%. Ms: 493.1 (M+H⁺).

### Step 6: Synthesis of compound 3-(1-(5-((S)-2,2-dimethyltetrahydro-2H-pyran-4-yl)-2-((S)-3-(3-(4-fluoro-1-(methyl-d3)-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)cyclopropyl)-1,2,4-oxazole-5(4H)-one (118)

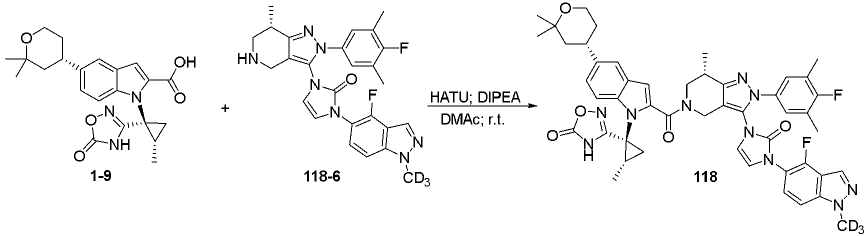

Intermediate 1-9 (40 mg, 0.1 mmol), intermediate **118-6** (50 mg, 0.1 mmol), HATU (46 mg, 0.12 mmol), and N,N-diisopropylethylamine (26 mg, 0.2 mmol) were sequentially added to *N,N-*dimethylacetamide (1 mL) and reacted at room temperature for 4 h. The reaction solution was diluted with ethyl acetate (15 mL), and then washed once with water (10 mL) and once with saturated saline (10 mL). The organic layer was dried over anhydrous sodium sulfate, and concentrated under reduced pressure. The residue was purified by column chromatography to obtain 50 mg of product. Yield: 56.8%. Ms: 886.2 (M+H⁺).

Referring to the synthesis methods in the above examples, the remaining target compounds listed in Table 1 were prepared using appropriate reagents under suitable conditions:

**Table 1. Compounds synthesized in the present invention and their characterization data**

| Compound No. | Structures of compounds | Charaterization data |
|---|---|---|
| 1 | 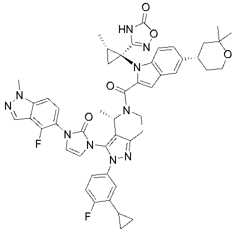 | LCMS (M+1): 895 |
| 2 | 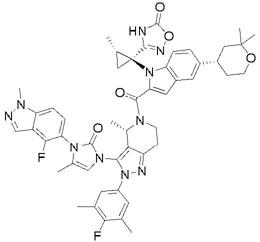 | LCMS (M+1): 897 |
| 3 | 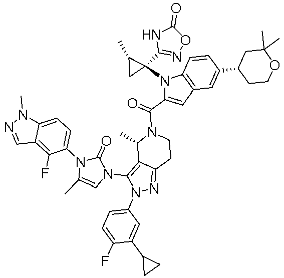 | LCMS (M+1): 909 |
| 4 | 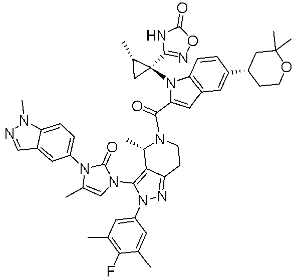 | LCMS (M+1): 879 |
| 5 | 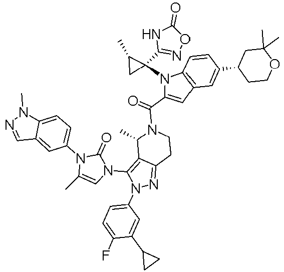 | LCMS (M+1): 891 |
| 6 | 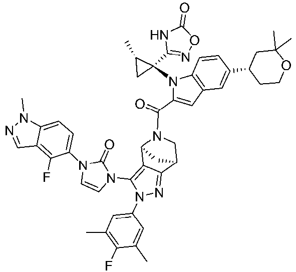 | LCMS (M+1): 881 |
| 7 | 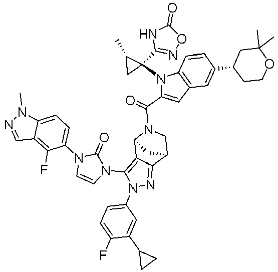 | LCMS (M+1): 893 |
| 8 | 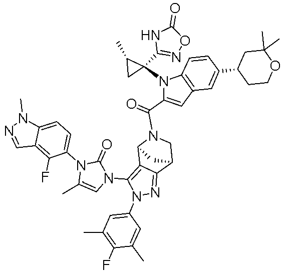 | LCMS (M+1): 895 |
| 9 | 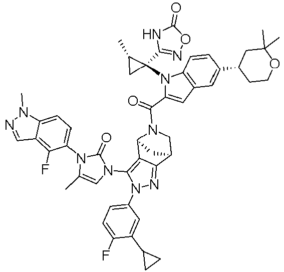 | LCMS (M+1): 907 |
| 10 | 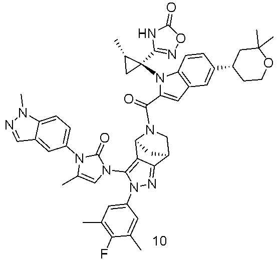 | LCMS (M+1): 877 |
| 11 | 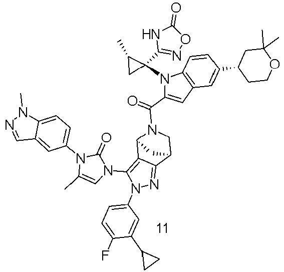 | LCMS (M+1): 889 |
| 12 | 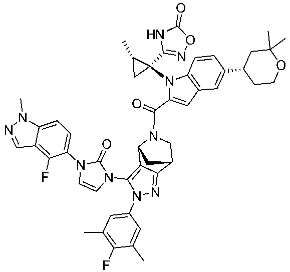 | LCMS (M+1): 881 |
| 13 | 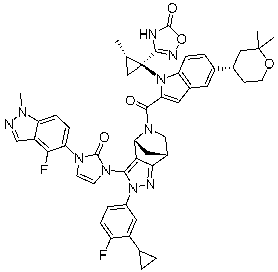 | LCMS (M+1): 893 |
| 14 | 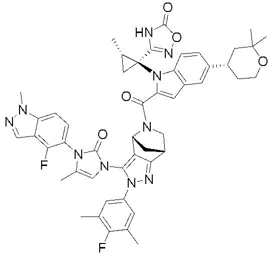 | LCMS (M+1): 895 |
| 15 | 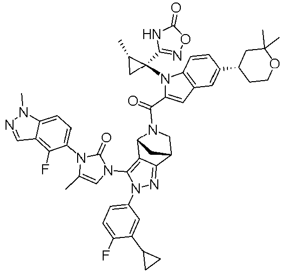 | LCMS (M+1): 907 |
| 16 | 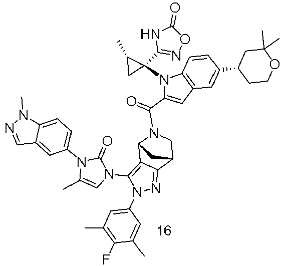 | LCMS (M+1): 877 |
| 17 | 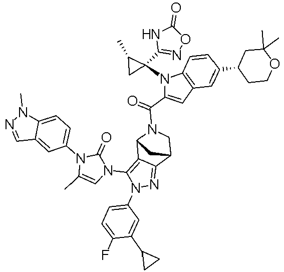 | LCMS (M+1): 890 |
| 18 | 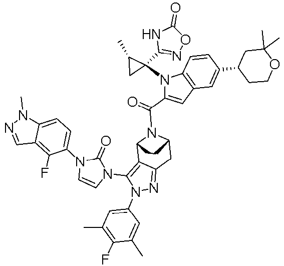 | LCMS (M+1): 895 |
| 19 | 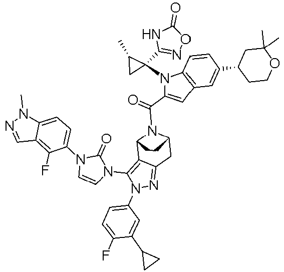 | LCMS (M+1): 907 |
| 20 | 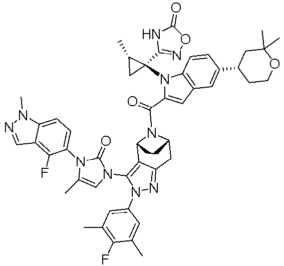 | LCMS (M+1): 909 |
| 21 | 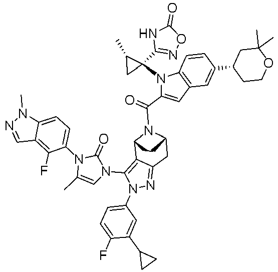 | LCMS (M+1): 920 |
| 22 | 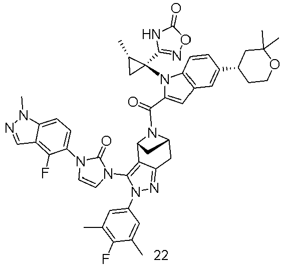 | LCMS (M+1): 881 |
| 23 | 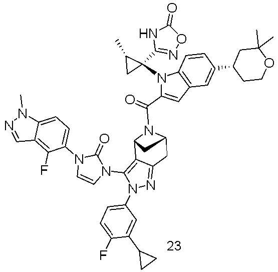 | LCMS (M+1): 893 |
| 24 | 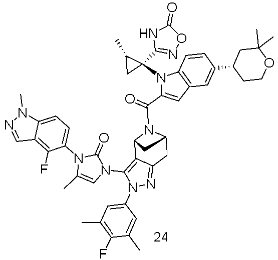 | LCMS (M+1): 895 |
| 25 | 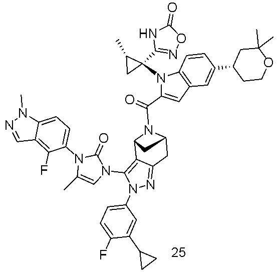 | LCMS (M+1): 907 |
| 26 | 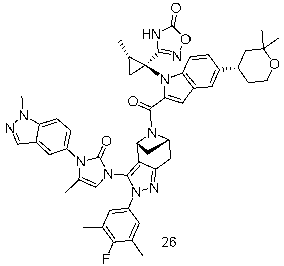 | LCMS (M+1): 877 |
| 27 | 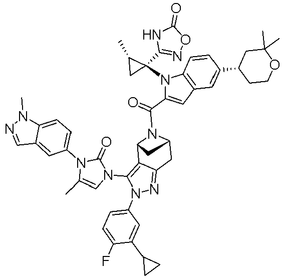 | LCMS (M+1): 889 |
| 30 | 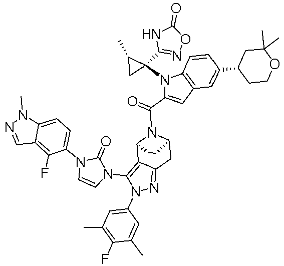 | LCMS (M+1): 895 |
| 31 | 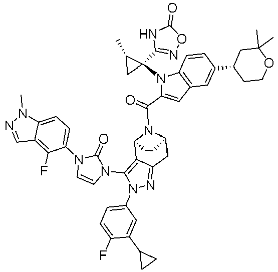 | LCMS (M+1): 907 |
| 32 | 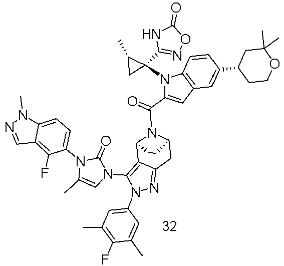 | LCMS (M+1): 909 |
| 33 | 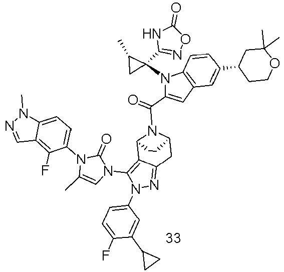 | LCMS (M+1): 921 |
| 34 | 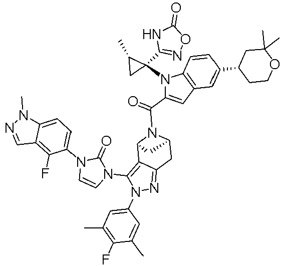 | LCMS (M+1): 881 |
| 35 | 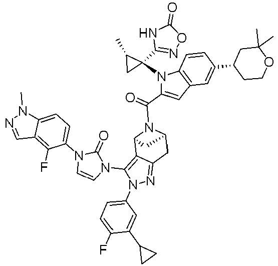 | LCMS (M+1): 893 |
| 36 | 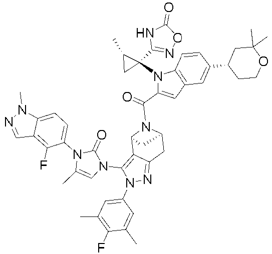 | LCMS (M+1): 895 |
| 37 | 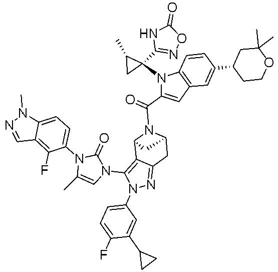 | LCMS (M+1): 907 |
| 38 | 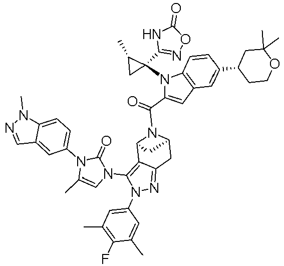 | LCMS (M+1): 877 |
| 39 | 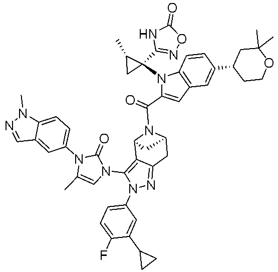 | LCMS (M+1): 889 |
| 41 | 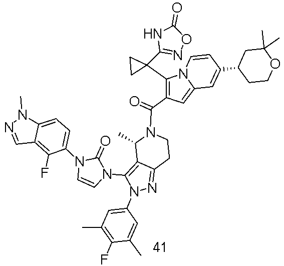 | LCMS (M+1): 869 |
| 42 | 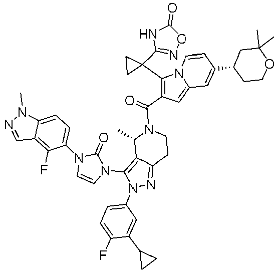 | LCMS (M+1): 881 |
| 43 | 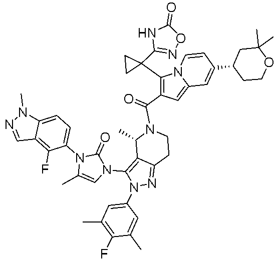 | LCMS (M+1): 883 |
| 44 | 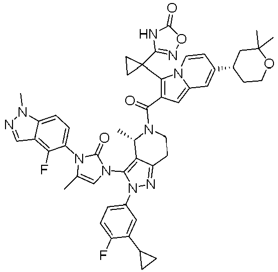 | LCMS (M+1): 895 |
| 45 | 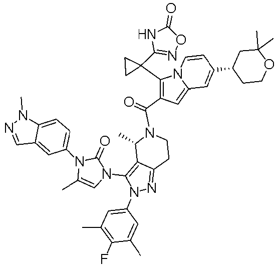 | LCMS (M+1): 865 |
| 46 | 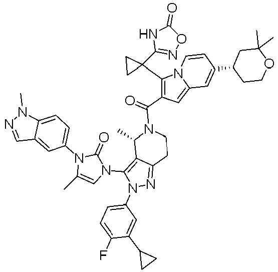 | LCMS (M+1): 876 |
| 47 | 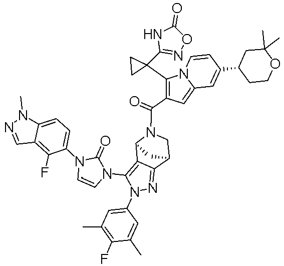 | LCMS (M+1): 867 |
| 48 | 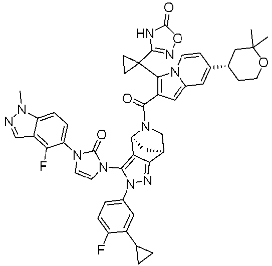 | LCMS (M+1): 879 |
| 49 | 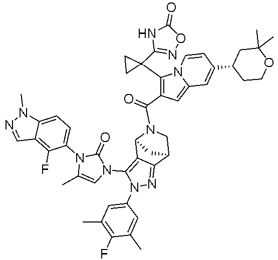 | LCMS (M+1): 881 |
| 50 | 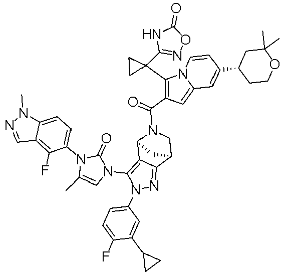 | LCMS (M+1): 893 |
| 51 | 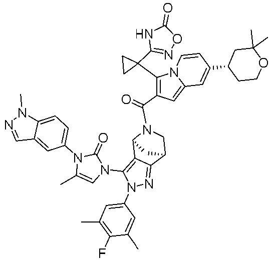 | LCMS (M+1): 863 |
| 52 | 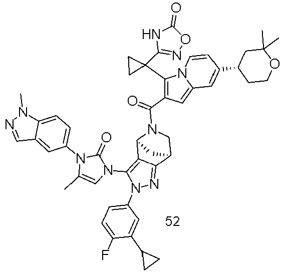 | LCMS (M+1): 875 |
| 53 | 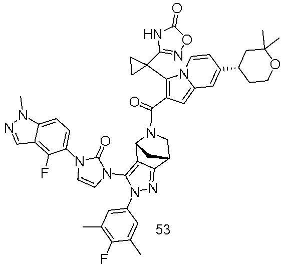 | LCMS (M+1): 867 |
| 54 | 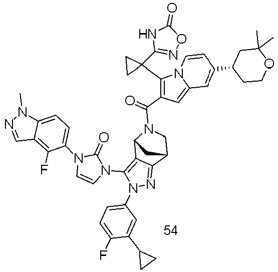 | LCMS (M+1): 879 |
| 55 | 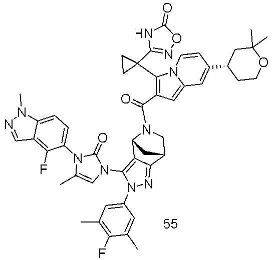 | LCMS (M+1): 881 |
| 56 | 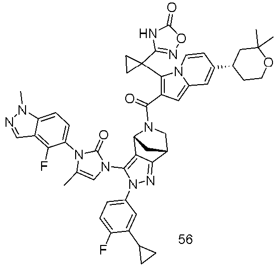 | LCMS (M+1): 893 |
| 57 | 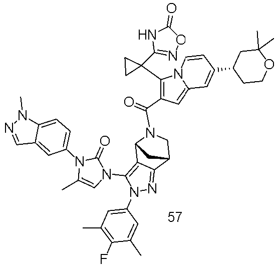 | LCMS (M+1): 863 |
| 58 | 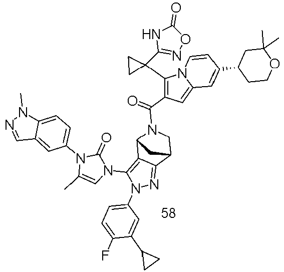 | LCMS (M+1): 875 |
| 59 | 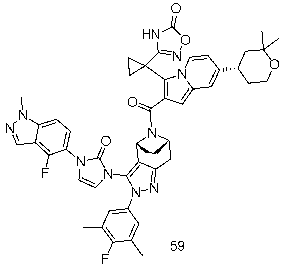 | LCMS (M+1): 881 |
| 60 | 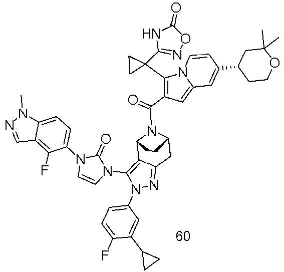 | LCMS (M+1): 893 |
| 61 | 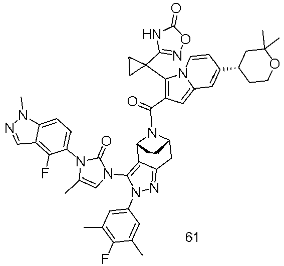 | LCMS (M+1): 895 |
| 62 | 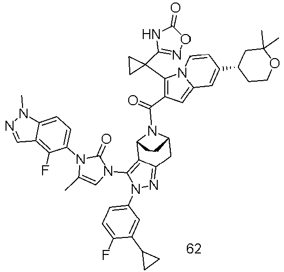 | LCMS (M+1): 907 |
| 63 | 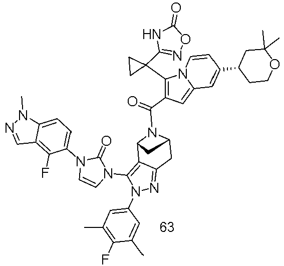 | LCMS (M+1): 867 |
| 64 | 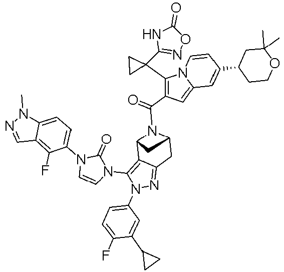 | LCMS (M+1): 879 |
| 65 | 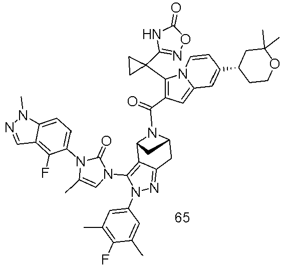 | LCMS (M+1): 881 |
| 66 | 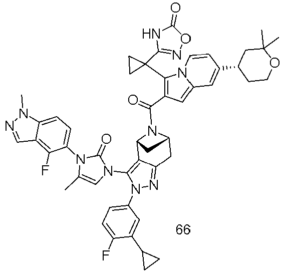 | LCMS (M+1): 893 |
| 67 | 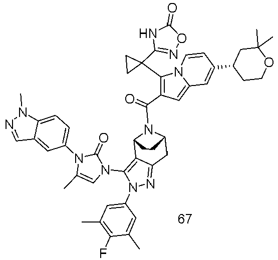 | LCMS (M+1): 877 |
| 68 | 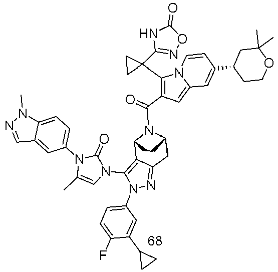 | LCMS (M+1): 889 |
| 69 | 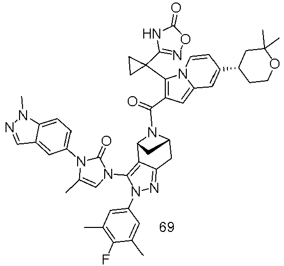 | LCMS (M+1): 863 |
| 70 | 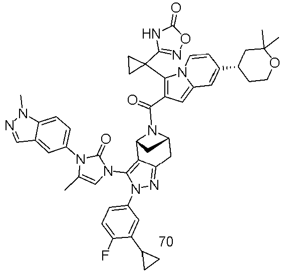 | LCMS (M+1): 875 |
| 71 | 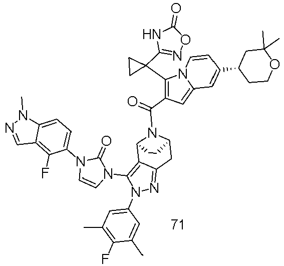 | LCMS (M+1): 881 |
| 72 | 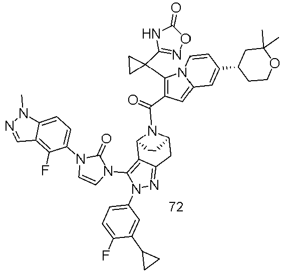 | LCMS (M+1): 893 |
| 73 | 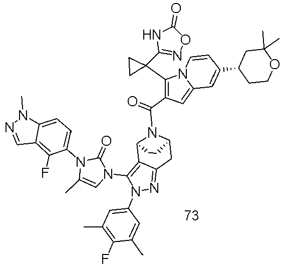 | LCMS (M+1): 895 |
| 74 | 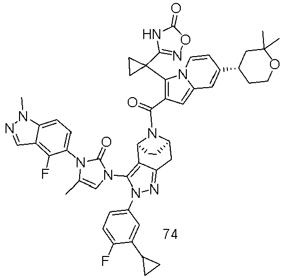 | LCMS (M+1): 907 |
| 75 | 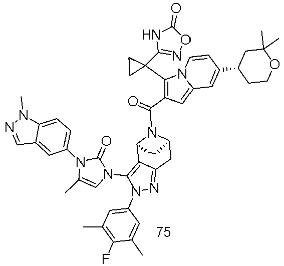 | LCMS (M+1): 877 |
| 76 | 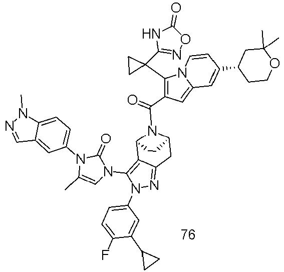 | LCMS (M+1): 889 |
| 81 | 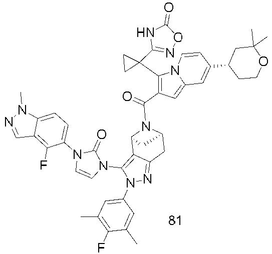 | LCMS (M+1): 867 |
| 82 | 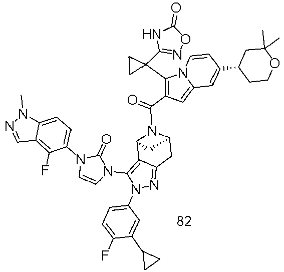 | LCMS (M+1): 879 |
| 83 | 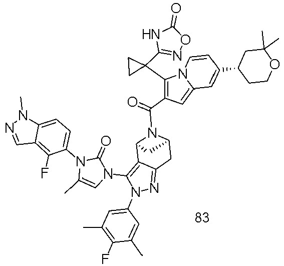 | LCMS (M+1): 881 |
| 84 | 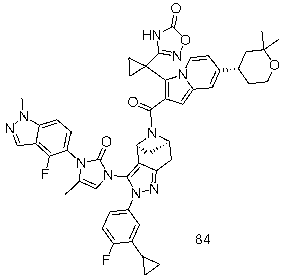 | LCMS (M+1): 893 |
| 85 | 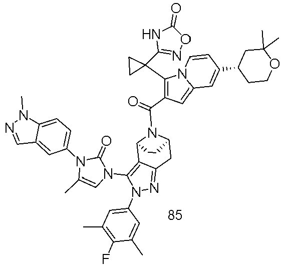 | LCMS (M+1): 877 |
| 86 | 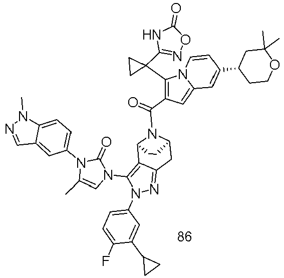 | LCMS (M+1): 889 |
| 87 | 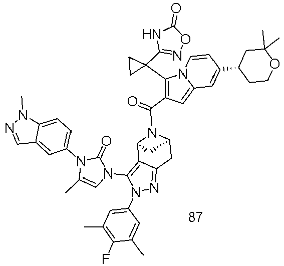 | LCMS (M+1): 869 |
| 88 | 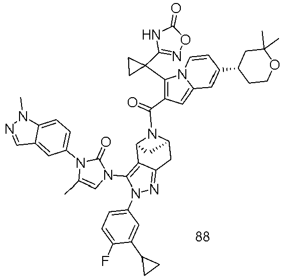 | LCMS (M+1): 875 |
| 89 | 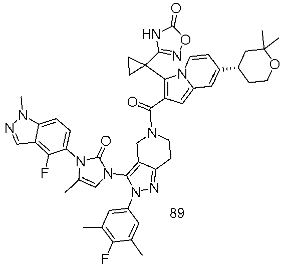 | LCMS (M+1): 869 |
| 90 | 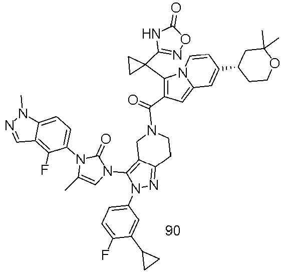 | 881 |
| 91 | 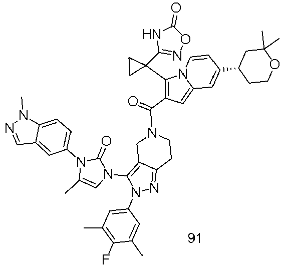 | LCMS (M+1): 851 |
| 92 | 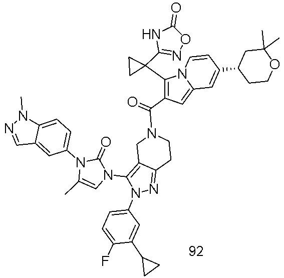 | LCMS (M+1): 863 |
| 93 | 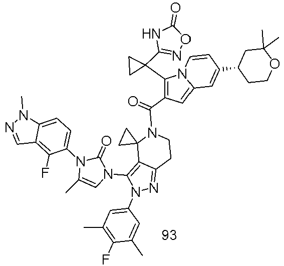 | LCMS (M+1): 895 |
| 94 | 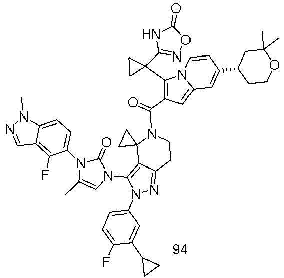 | LCMS (M+1): 907 |
| 95 | 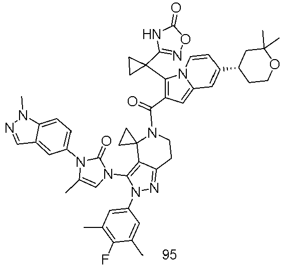 | LCMS (M+1): 877 |
| 96 | 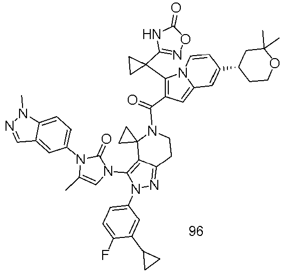 | LCMS (M+1): 889 |
| 97 | 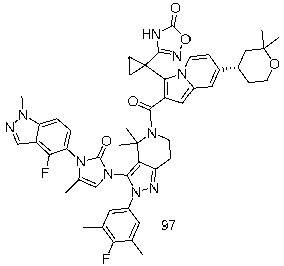 | LCMS (M+1): 897 |
| 98 | 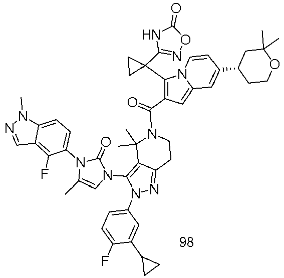 | LCMS (M+1): 909 |
| 99 | 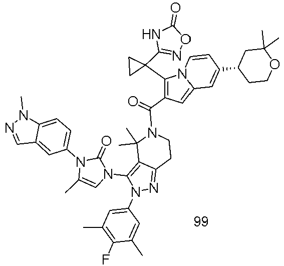 | LCMS (M+1): 879 |
| 100 | 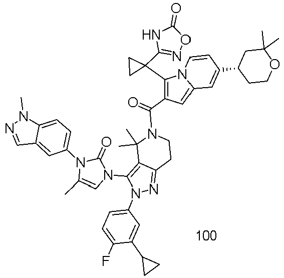 | LCMS (M+1): 891 |
| 101 | 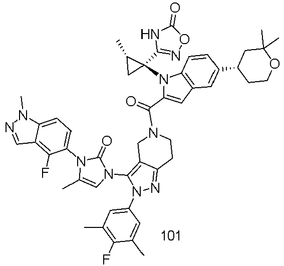 | LCMS (M+1): 883 |
| 102 | 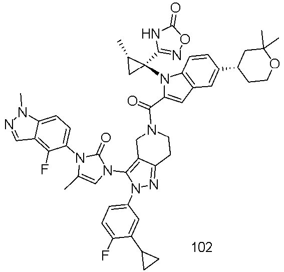 | LCMS (M+1): 895 |
| 103 | 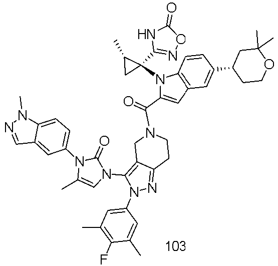 | LCMS (M+1): 865 |
| 104 | 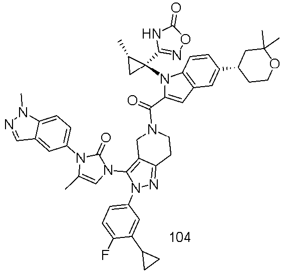 | LCMS (M+1): 877 |
| 105 | 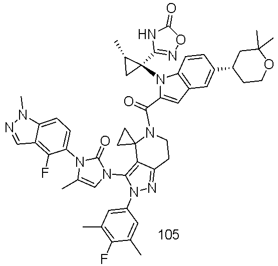 | LCMS (M+1): 909 |
| 106 | 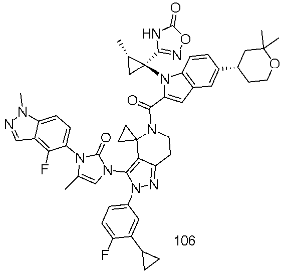 | LCMS (M+1): 921 |
| 107 | 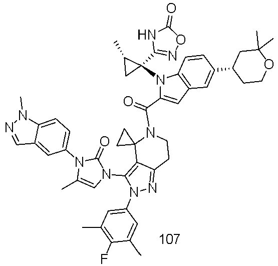 | LCMS (M+1): 891 |
| 108 | 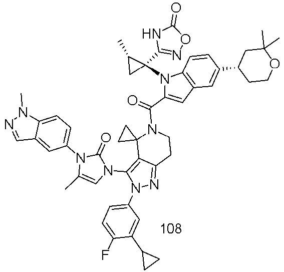 | LCMS (M+1): 903 |
| 109 | 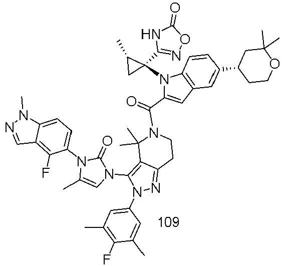 | LCMS (M+1): 911 |
| 110 | 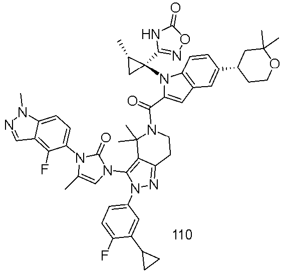 | LCMS (M+1): 922 |
| 111 | 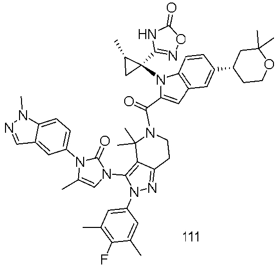 | LCMS (M+1): 893 |
| 112 | 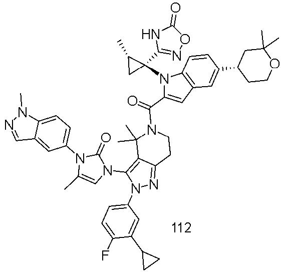 | LCMS (M+1): 905 |
| 113 | 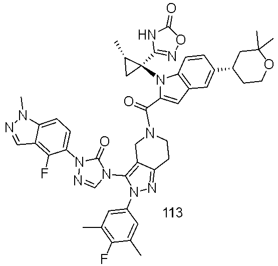 | LCMS (M+1): 870 |
| 114 | 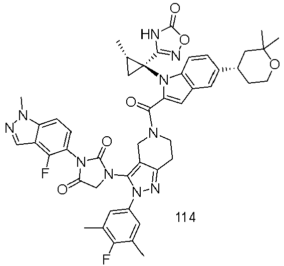 | LCMS (M+1): 885 |
| 115 | 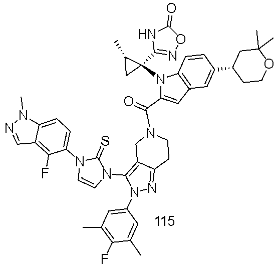 | LCMS (M+1): 885 |
| 116 | 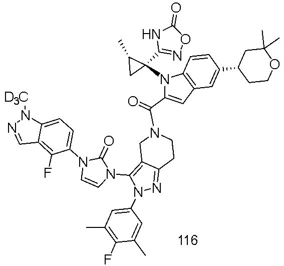 | LCMS (M+1): 872 |
| 117 | 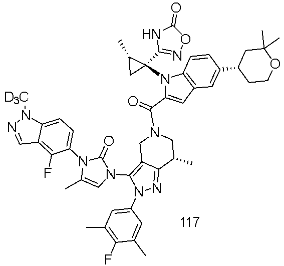 | LCMS (M+1): 890 |
| 118 | 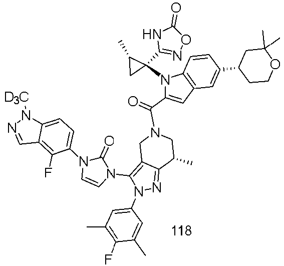 | LCMS (M+1): 886 |
| 119 | 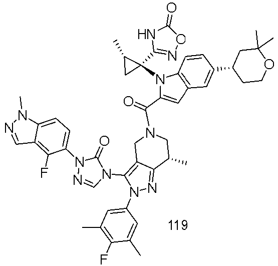 | LCMS (M+1): 884 |
| 120 | 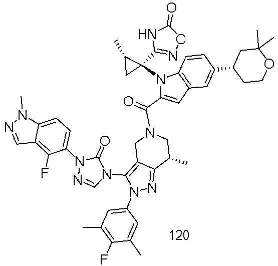 | LCMS (M+1): 884 |
| 121 | 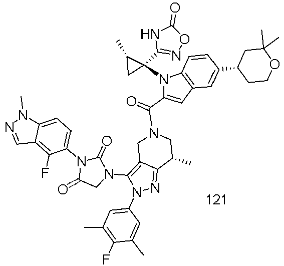 | LCMS (M+1): 899 |
| 122 | 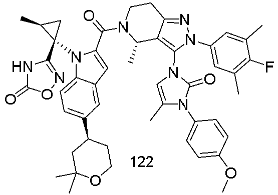 | LCMS (M+1): 855 |
| 123 | 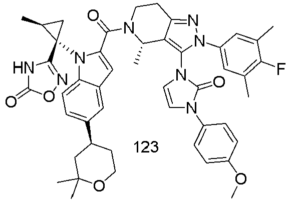 | LCMS (M+1): 841 |
| 125 | 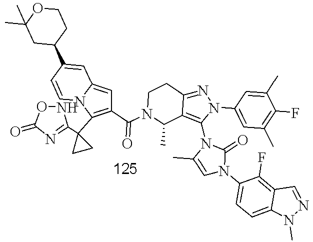 | LCMS (M+1): 883 |
| 126 | 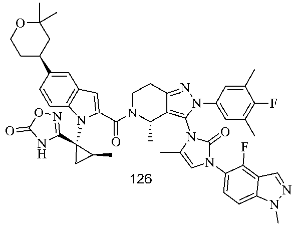 | LCMS (M+1): 897 |
| 127 | 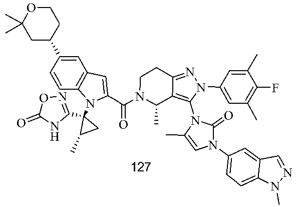 | LCMS (M+1): 879 |
| 128 | 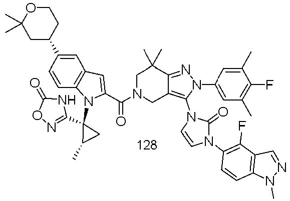 | LCMS (M+1): 897 |
| 129 | 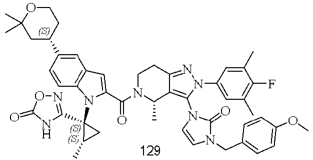 | LCMS (M+1): 855 |
| 130 | 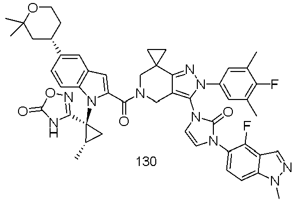 | LCMS (M+1): 895 |
| 131 | 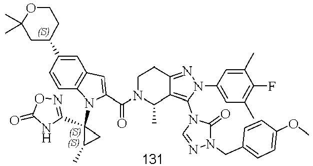 | LCMS (M+1): 856 |
| 132 | 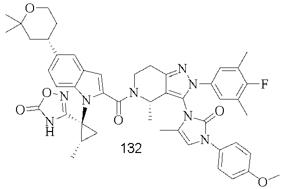 | LCMS (M+1): 855 |
| 133 | 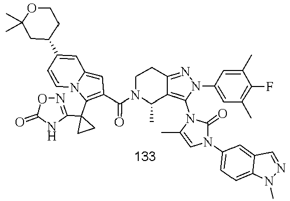 | LCMS (M+1): 865 |
| 134 | 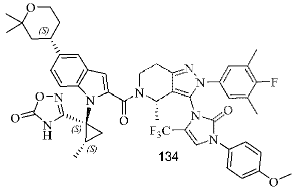 | LCMS (M+1): 909 |
| 135 | 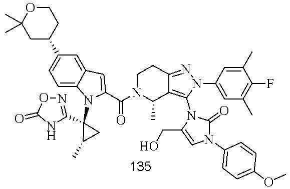 | LCMS (M+1): 871 |
| 136 | 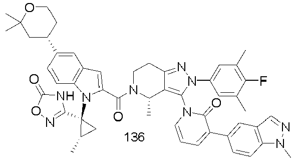 | LCMS (M+1): 876 |
| 137 | 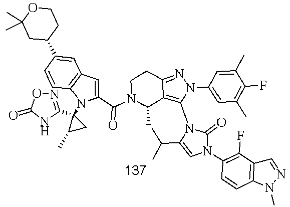 | LCMS (M+1): 925 |
| 138 | 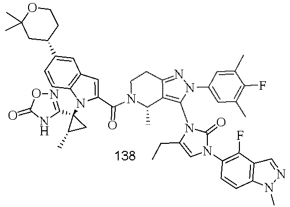 | LCMS (M+1): 911 |
| 139 | 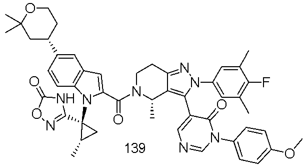 | LCMS (M+1): 853 |
| 140 | 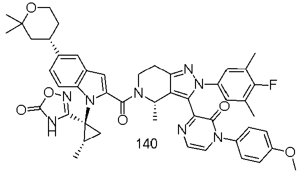 | LCMS (M+1): 853 |
| 141 | 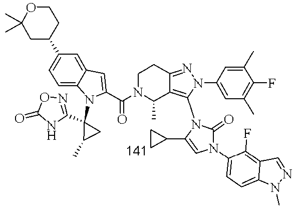 | LCMS (M+1): 923 |
| 142 | 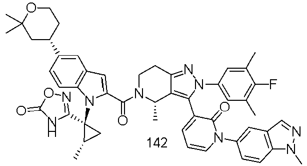 | LCMS (M+1): 876 |
| 143 | 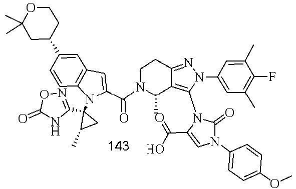 | LCMS (M+1): 885 |

### Example 13. Synthesis of compound 3-((1S,2S)-1-(5-((S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3-((R)-3-hydroxybut-1-yn-1-yl)-5-methylphenyl)-4-methyl-4,5,6,7-tetrahydro-2H-pyrazolo[4,3-c]pyridine-5-carbonyl)-1H-indole-1-yl)-2-methylcyclopropyl)-1,2,4-diazole-5(4H)-one (Compound 214)

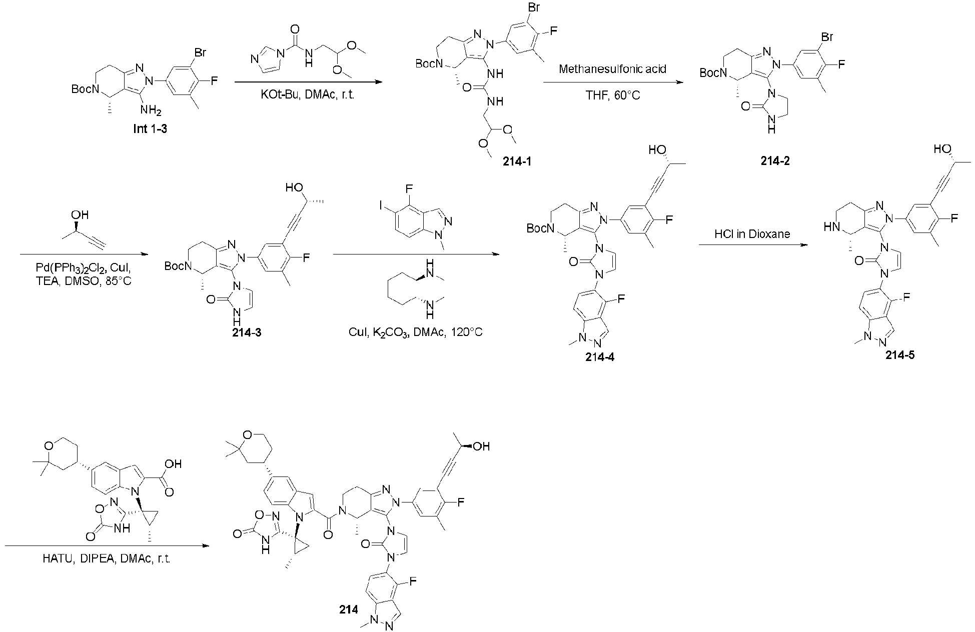

### Step 1: Synthesis of intermediate tert-butyl (S)-2-(3-bromo-4-fluoro-5-methylphenyl)-3-(3-(2,2-dimethoxyethyl)ureido)-4-methyl-2,4,6,7-tetrahydro-5H-pyrazolo[4,3-c]pyridine-5-carboxylate (214-1)

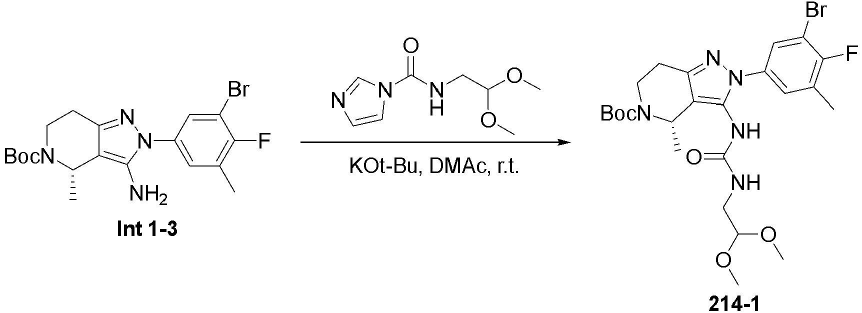

Procedures: Intermediate Int 1-3 (0.92 g, 2.1 mmol), intermediate ref-2 (0.84 g, 4.2 mmol), and potassium tert-butoxide (0.71 g, 6.3 mmol) were successively added to *N,N-*dimethylacetamide (15 mL), and allowed to react at room temperature for 2 h. The reaction solution was diluted with ethyl acetate (50 mL), washed once with water (25 mL) and once with saturated saline (25 mL), dried over anhydrous sodium sulfate, and concentrated under reduced pressure. The residue was purified by column chromatography to obtain 1.1 g of product. Yield: 92.1%. Ms: 570.2/572.2 (M+H⁺).

### Step 2: Synthesis of intermediate tert-butyl (S)-2-(3-bromo-4-fluoro-5-methylphenyl)-4-methyl-3-(2-oxo-2,3-dihydro-1H-imidazole-1-yl)-2,4,6,7-tetrahydro-5H-pyrazolo[4,3-c]pyridine-5-carboxylate (214-2)

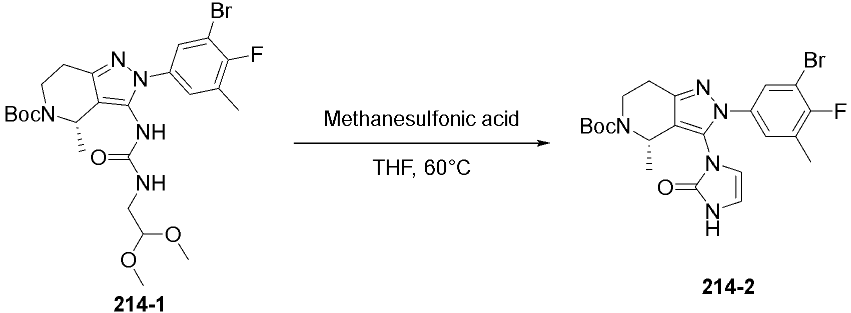

Procedures: Intermediate **214-1** (1.1 g, 1.9 mmol) and methanesulfonic acid (0.37 g, 3.8 mmol) were successively added to tetrahydrofuran (55 mL), and then allowed to react at 60 °C for 2 h. The reaction solution was poured into ice water (50 mL), and extracted with ethyl acetate (100 mL×2). The organic layers were combined, washed once with NaHCO₃ saturated aqueous solution (50 mL), dried over anhydrous sodium sulfate, and concentrated under reduced pressure. The residue was purified by column chromatography to obtain 0.84 g of product. Yield: 86.0%. Ms: 506.1/508.1 (M+H⁺).

### Step 3: Synthesis of intermediate tert-butyl (S)-2-(4-fluoro-3-((R)-3-hydroxybut-1-yn-1-yl)-5-methylphenyl)-4-methyl-3-(2-oxo-2,3-dihydro-1H-imidazole-1-yl)-2,4,6,7-tetrahydro-5H-pyrazolo[4,3-c]pyridine-5-carboxylate (214-3)

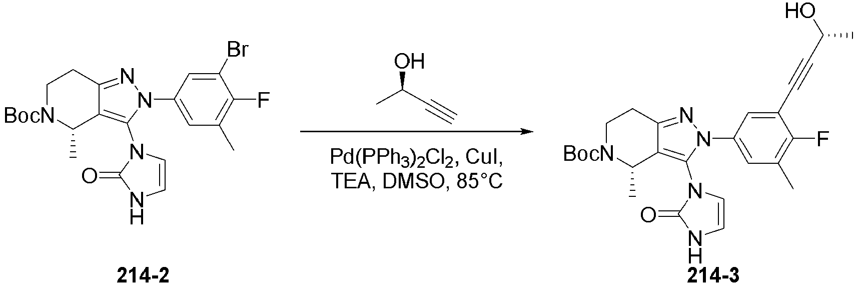

Procedures: Intermediate 214-2 (290 mg, 0.57 mmol), (R)-(+)-3-butyn-2-ol (200 mg, 2.9 mmol), bis(triphenylphosphine)palladium(II) dichloride (80 mg, 0.11 mmol), CuI (22 mg, 0.11 mmol), and triethylamine (580 mg, 5.7 mmol) were sequentially added to dimethyl sulfoxide (6 mL). The system was purged with nitrogen three times, reacted at 85 °C for 4 h, cooled to room temperature, and filtered. The filter cake was triturated in ethyl acetate (20 mL × 2). The filtrates were combined, washed with water (20 mL × 2) and once with saturated saline (20 mL), dried over anhydrous sodium sulfate, and concentrated under reduced pressure. The residue was purified by column chromatography to obtain 205 mg of product. Yield: 72.2%. Ms: 496.2 (M+H⁺).

### Step 4: Synthesis of intermediate tert-butyl (S)-3-(3-(4-fluoro-1-methyl-1H-indazole-5-yl)-2-oxo-2,3-dihydro-1H-imidazole-1-yl)-2-(4-fluoro-3-((R)-3-hydroxybut-1-yn-1-yl)-5-methylphenyl)-4-methyl-2,4,6,7-tetrahydro-5H-pyrazolo[4,3-c]pyridine-5-carboxylate (214-4)

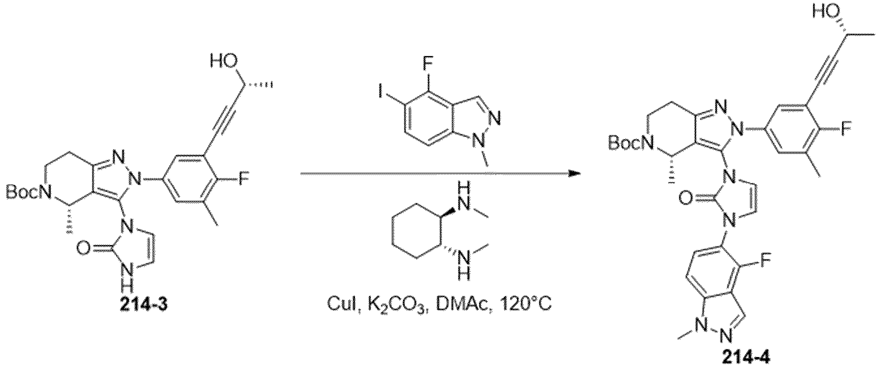

Procedures: Intermediate **214-3** (205 mg, 0.41 mmol), intermediate 1-N-methyl-4-fluoro-5-iodoindazole (137 mg, 0.50 mmol), (1S,2S)-(-)-N,N'-dimethyl-1,2-cyclohexanediamine (59 mg, 0.41 mmol), CuI (79 mg, 0.41 mmol), and K₂CO₃ (86 mg, 0.62 mmol) were sequentially added to N,N-dimethylacetamide (6 mL). The system was purged with nitrogen three times, reacted at 120 °C for 4 h, cooled to room temperature, and filtered. The filter cake was triturated in ethyl acetate (20 mL × 2). The filtrates were combined, washed with water (20 mL × 2) and once with saturated saline (20 mL), dried over anhydrous sodium sulfate, and concentrated under reduced pressure. The residue was purified by column chromatography to obtain 160 mg of product. Yield: 60.1%. Ms: 644.2 (M+H⁺).

### Step 5: Synthesis of intermediate 1-(4-fluoro-1-methyl-1H-indazole-5-yl)-3-((S)-2-(4-fluoro-3-((R)-3-hydroxybut-1-yn-1-yl)-5-methylphenyl)-4-methyl-4,5,6,7-tetrahydro-2H-pyrazolo[4,3-c]pyridine-3-yl)-1,3-dihydro-2H-imidazole-2-one (214-5)

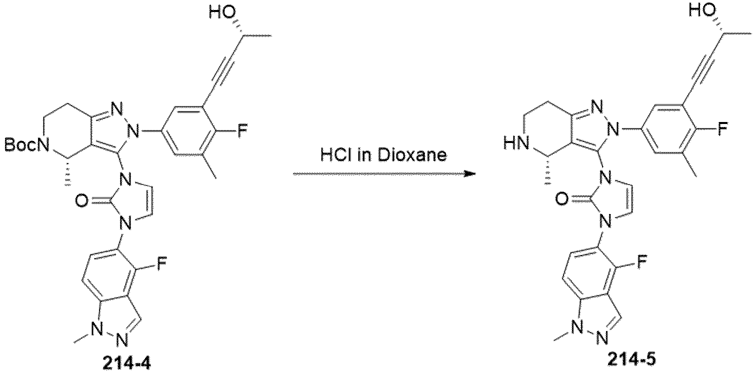

Procedures: Intermediate **214-4** (160 mg, 0.25 mmol) was added to a solution of HCl in 1,4-dioxane (4 M, 5 mL), and then reacted at room temperature for 2 h. The reaction solution was adjusted to pH >7 with NaHCO₃ saturated aqueous solution, and then extracted with ethyl acetate (20 mL×2). The organic layers were combined, washed with saturated saline (20 mL), dried over anhydrous sodium sulfate, and concentrated under reduced pressure to obtain 120 mg of product. Yield: 88.8%. Ms: 544.2 (M+H⁺).

### Step 6: Synthesis of compound 3-((1S,2S)-1-(5-((S)-2,2-dimethyltetrahydro-2H-pyran-4-yl)-2-((S)-3-(3-(4-(4-fluoro-1-methyl-1H-indazole-5-yl)-2-oxo-2,3-dihydro-1H-imidazole-1-yl)-2-(4-fluoro-3-((R)-3-hydroxybut-1-yn-1-yl)-5-methylphenyl)-4-methyl-4,5,6,7-tetrahydro-2H-pyrazolo[4,3-c]pyridine-5-carbonyl)-1H-indole-1-yl)-2-methylcyclopropyl)-1,2,4-oxadiazole-5(4H)-one (Compound 214)

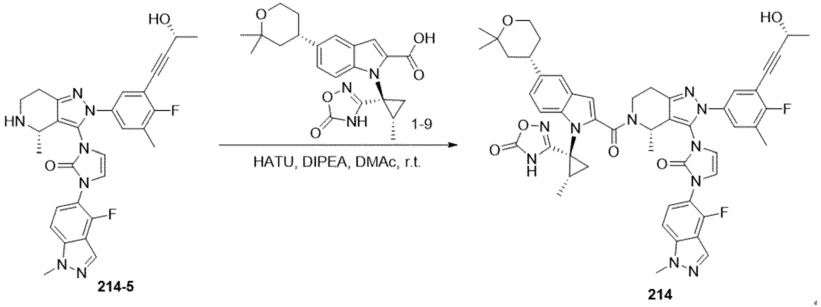

Procedures: Intermediate 214-5 (100 mg, 0.18 mmol), intermediate 1-9 (91 mg, 0.22 mmol), HATU (105 mg, 0.28 mmol), and N,N-diisopropylethylamine (48 mg, 0.37 mmol) were sequentially added to N,N-dimethylacetamide (2 mL), and then allowed to react at room temperature for 4 h. The reaction solution was then diluted with ethyl acetate (15 mL), and then washed once with water (10 mL) and once with saturated saline solution (10 mL). The organic layers were dried over anhydrous sodium sulfate, and concentrated under reduced pressure. The residue was purified by column chromatography to obtain 90 mg of product. Yield: 52.2%. Ms: 937.2 (M+H⁺). ¹H NMR (400 MHz, DMSO-*d*₆) *δ* 11.74 (s, 1H), 8.29 (s, 1H), 7.64 (d, *J* = 8.8 Hz, 1H), 7.53 (s, 1H), 7.51 - 7.45 (m, 1H), 7.40 (d, *J=* 8.1 Hz, 2H), 7.30 - 7.23 (m, 2H), 7.12 (d, *J* = 3.1 Hz, 1H), 6.99 (s, 1H), 6.95 (s, 1H), 5.63 - 5.55 (m, 1H), 4.70 - 4.59 (m, 1H), 4.44 - 4.34 (m, 1H), 4.11 (s, 3H), 3.76 - 3.65 (m, 2H), 3.09 - 2.97 (m, 2H), 2.96 - 2.85 (m, 1H), 2.29 (s, 3H), 1.82 - 1.58 (m, 3H), 1.54 - 1.36 (m, 4H), 1.30 - 1.21 (m, 6H), 1.17 (d, *J* = 6.5 Hz, 6H), 1.09 - 0.98 (m, 1H), 0.88 - 0.79 (m, 4H).

### Example 14. Synthesis of compound 3-((1S,2S)-1-(5-((S)-2,2-dimethyltetrahydro-2H-pyran-4-yl)-2-((S)-3-(3-(4-fluoro-1-methyl-1H-indole-5-yl)-2-oxo-2,3-dihydro-1H-imidazoline-1-yl)-2-(4-fluoro-3-(3-hydroxyll-3-methylbut-1-enyl)phenyl)-4-methyl-4,5,6,7-tetrahydro-2H-pyrazolo[4,3-c]pyridine-5-formyl)-1H-indole-1-yl)-2-methylcyclopropyl)-1,2,4-oxadiazepine-5(4H)-one (Compound 203)

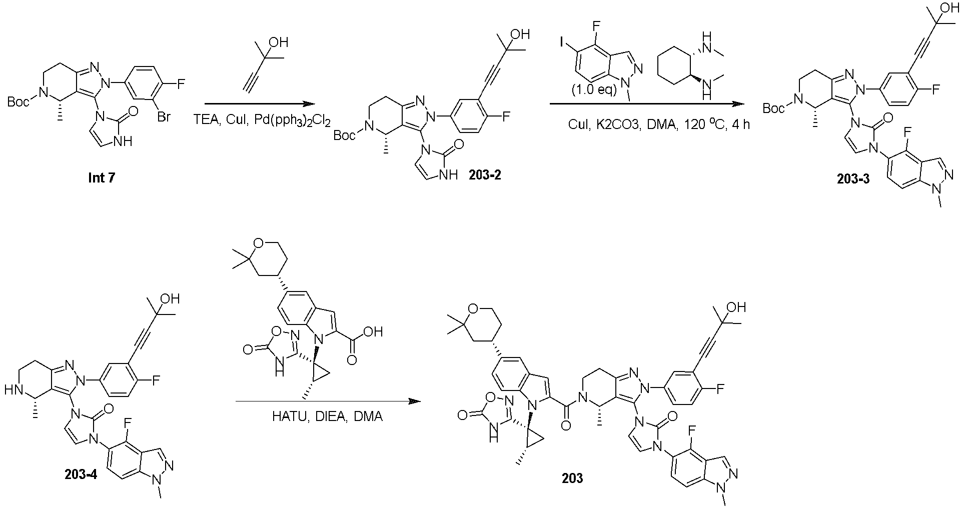

### Step 1: Synthesis of intermediate tert-butyl (S)-2-(4-fluoro-3-(3-hydroxyl-3-methylbut-1-yn-1-yl)phenyl)-4-methyl-3-(2-oxo-2,3-dihydro-1H-imidazole-1-yl)-2,4,6,7-tetrahydro-5H-pyrazolo[4,3-c]pyridine-5-carboxylate (Compound 203-2)

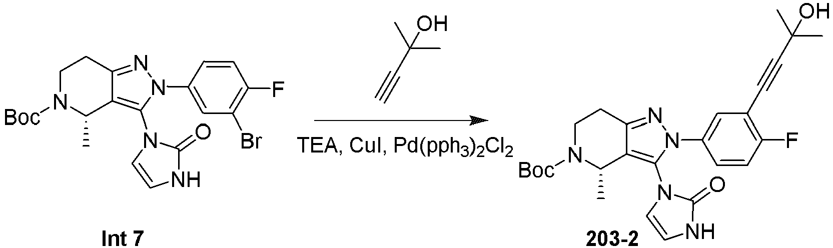

At room temperature, intermediate Int 7 (0.3 g, 0.61 mmol), 2-methylbut-3-yn-2-ol (307.53 mg, 3.66 mmol), triethylamine (246.64 mg, 2.44 mmol), CuI (23.21 mg, 121.86 µmol), and bis(triphenylphosphine)palladium dichloride (85.54 mg, 121.86 µmol) were dissolved in DMSO (10 mL), and then stirred and reacted under nitrogen protection at 85 °C for 12 h. The reaction of the raw material was monitored by TLC to ensure its disappearance. The reaction solution was diluted with 10 mL of water, and extracted with ethyl acetate (10 mL × 3). The organic phase was washed with 10 mL of saturated saline, dried over sodium sulfate, and filtered. The filtrate was concentrated under reduced pressure, and the concentrate was purified by silica gel column chromatography to obtain the light yellow solid product, intermediate **203-2** (0.22 g, 415 µmol), with a yield of 68%. M/S: 496.2 (M+H⁺).

### Step 2: Synthesis of intermediate tert-butyl ((S)-3-(3-(4-fluoro-1-methyl-1H-indazole-5-yl)-2-oxo-2,3-dihydro-1H-imidazole-1-yl)-2-(4-fluoro-3-(3-hydroxyl-3-methylbut-1-yn-1-yl)phenyl)-4-methyl-2,4,6,7-tetrahydro-5H-pyrazolo[4,3-c]pyridine-5-carboxylate (Intermediate 203-3)

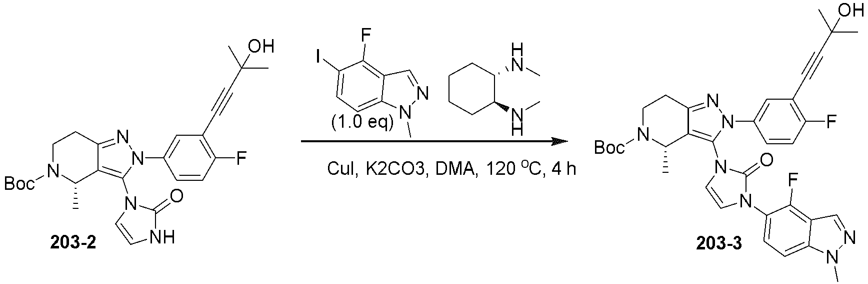

Intermediate **203-2** (120 mg, 201.79 µmol), 4-fluoro-5-iodo-1-methyl-1H-indazole (114.41 mg, 403.59 µmol), CuI (15.37 mg, 80.72 µmol), (1S,2S)-N1,N2-dimethylcyclohexane-1,2-diamine (28.70 mg, 201.79 µmol), and K₂CO₃ (41.83 mg, 302.69 µmol) were added to anhydrous N,N-dimethylacetamide (5 mL), and then allowed to react for 4 h under nitrogen protection with stirring at 120 °C. The reaction of raw materials was monitored by TLC to ensure completeness. The reaction solution was diluted with 10 mL of water, and then extracted with ethyl acetate (10 mL × 3). The organic phase was washed with 10 mL of saturated saline, dried over sodium sulfate, and filtered. The filtrate was concentrated under reduced pressure. The concentrate was purified by silica gel column chromatography to obtain intermediate **203-3** (40.0 mg, 62.14 µmol), with a yield of 25%. M/S: 644.3 (M+H⁺).

### Step 3: Synthesis of intermediate 1-(4-fluoro-1-methyl-1H-indazole-5-yl)-3-((S)-2-(4-fluoro-3-((R)-3-hydroxybut-1-yn-1-yl)phenyl)-4-methyl-4,5,6,7-tetrahydro-2H-pyrazolo[4,3-c]pyridine-3-yl)-1,3-dihydro-2H-imidazole-2-one (Compound 203-4)

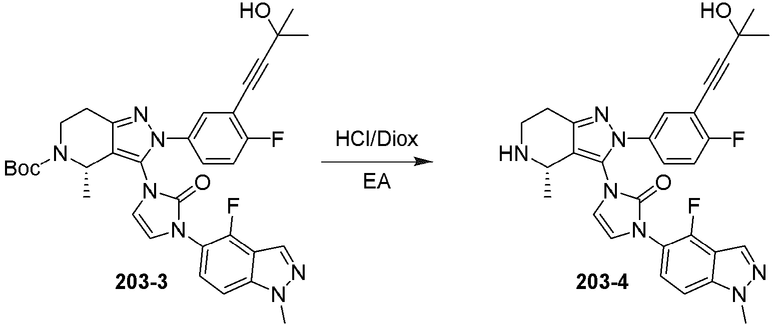

Intermediate **203-3** (40.0 mg, 62.14 µmol) was dissolved in ethyl acetate (2 mL), to which was added a solution of HCl in dioxane (2 mL). The reaction was stirred at room temperature for 2 h. The reaction of the raw material was monitored by TLC to ensure completeness. The reaction solution was concentrated under reduced pressure to obtain light yellow solid product, intermediate **203-4** (25.0 mg, 45.99 µmol), with a yield of 74%. M/S: 544.2 (M+H⁺).

### Step 4: Synthesis of compound 3-((1S,2S)-1-(5-((S)-2,2-dimethyltetrahydro-2H-pyran-4-yl)-2-((S)-3-(3-(4-fluoro-1-methyl-1H-indole-5-yl)-2-oxo-2,3-dihydro-1H-imidazoline-1-yl)-2-(4-fluoro-3-(3-hydroxyl-3-methylbut-1-enyl)phenyl)-4-methyl-4,5,6,7-tetrahydro-2H-pyrazolo[4,3-c]pyridine-5-formyl)-1H-indole-1-yl)-2-methylcyclopropyl)-1,2,4-oxadiazepine-5(4H)-one (203)

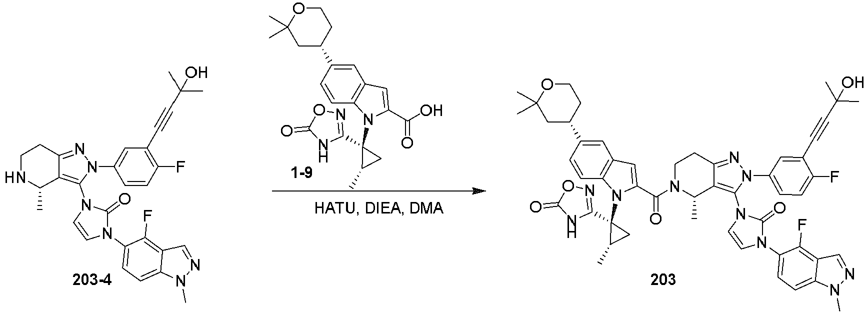

At room temperature, intermediate **203-4** (20.0 mg, 36.79 µmol), intermediate **1-9** (Example 2) (23.31 mg, 56.66 µmol), and DIEA (9.51 mg, 73.59 µmol) were dissolved in DMF (1.0 mL), to which was added HATU (20.99 mg, 55.19 µmol), and then stirred at room temperature for 2 h. The reaction of raw material was monitored to be complete by TLC. The reaction solution was diluted with 5.0 mL of water, and extracted with ethyl acetate (5.0 mL × 3). The organic phase was washed with 5.0 mL of saturated saline, dried over sodium sulfate, and filtered. The filtrate was concentrated under reduced pressure, and the concentrate was purified by silica gel column chromatography to obtain product **203** as light yellow solid (10.0 mg, 10.67 µmol), with a yield of 29%. M/S: 937.4 (M+H⁺).

### Example 15. Synthesis of compound 3-[(1S,2S)-1-(5-((S)-2,2-dimethyltetrahydro-2H-pyran-4-yl)-2-((S)-3-(3-(4-fluoro-1-methyl-1H-indole-5-yl)-2-oxo-2,3-dihydro-1H-imidazoline-1-yl)-2-(4-fluoro-3-(R)-3-hydroxybut-1-ene-1-yl)phenyl)-4-methyl-4,5,6,7-tetrahydro-2H-pyrazolo[4,3-c]pyridine-5-formyl)-1H-indole-1-yl)-2-methylcyclopropyl]-1,2,4-oxadiazepine-5(4H)-one (204)

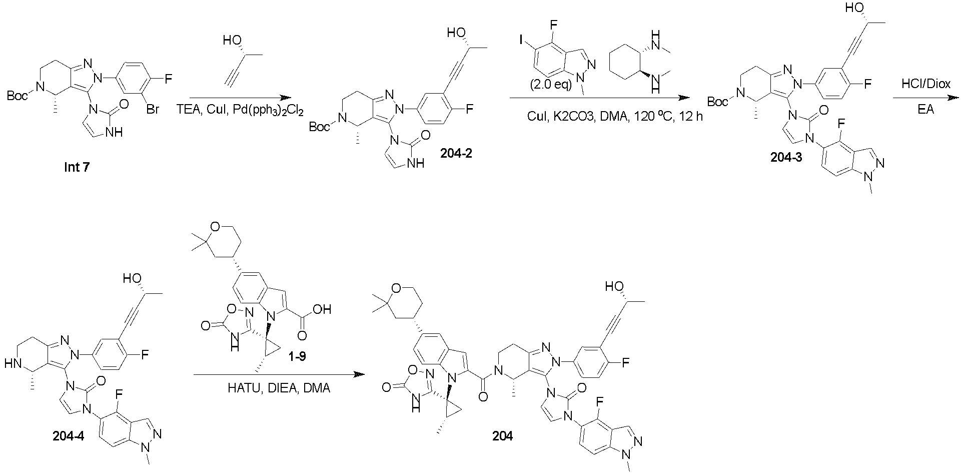

The synthesis of compound **204** was carried out using a method similar to that of **203,** substituting 2-methylbut-3-yn-2-ol with (R)-but-3-yn-2-ol, with a yield of 28%. M/S: 923.4 (M+H⁺).

### Example 16. Synthesis of compound 3-((1S,2S)-1-(5-(S)-2,2-dimethyltetrahydro-2H-pyran-4-yl)-2-((S)-2-(3-ethynyl-4-fluorophenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-1H-indole-1-yl)-2-methylcyclopropyl)-1,2,4-oxadiazole-5(4H)-one (206)

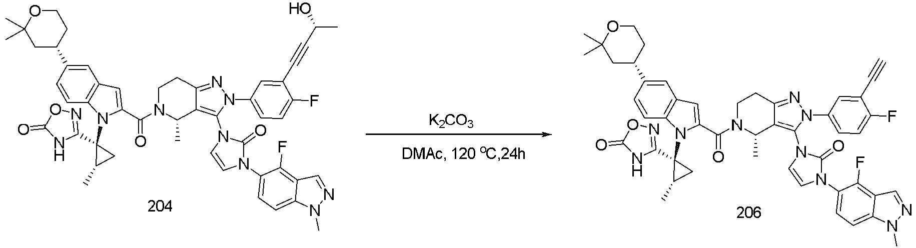

At room temperature, compound **204** (20.0 mg, 21.67 µmol) and K₂CO₃ (29.95 mg, 216.69 µmol) were dissolved in DMAc (1.0 mL), and the reaction was stirred at 120 °C for 24 h. The reaction of raw materials was monitored to be complete by TLC. The reaction solution was diluted with 5.0 mL of water, and extracted with ethyl acetate (5.0 mL × 3). The organic phase was washed with 5.0 mL of saturated saline, dried over sodium sulfate, and filtered. The filtrate was concentrated under reduced pressure. The concentrate was purified by silica gel column chromatography to obtain product **206** as light yellow solid (10.0 mg, 11.38 µmol), with a yield of 52%. M/S: 879.3 (M+H⁺).

### Example 17. Synthesis of compound 3-((1S,2S)-1-(5-(S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3-methyl-5-vinylphenyl)-4-methyl-4,5,6,7-tetrahydro-2H-pyrazolo[4,3-c]pyridine-5-carbonyl)-1H-indole-1-yl)-2-methylcyclopropyl)-1,2,4-oxadiazole-5(4H)-one (210)

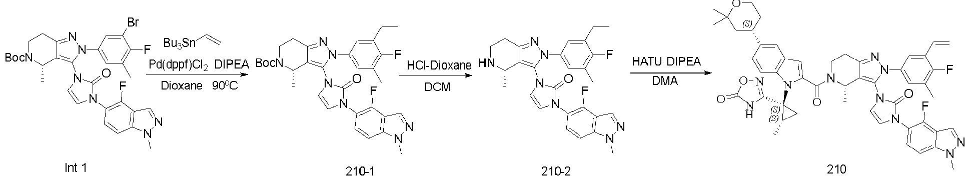

### Step 1: Synthesis of intermediate tert-butyl (S)-3-(3-(4-fluoro-1-methyl-1H-indazole-5-yl)-2-oxo-2,3-dihydro-1H-imidazole-1-yl)-2-(4-fluoro-3-methyl-5-vinylphenyl)-4-methyl-2,4,6,7-tetrahydro-5H-pyrazolo[4,3-c]pyridine-5-carboxylate (210-1)

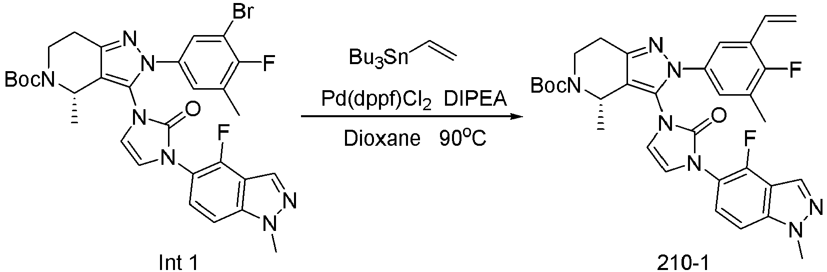

Intermediate **Int 1** (6.7 g, 10.2 mmol), tributylvinyltin (7.5 g, 20.5 mmol), Pd(dppf)Cl₂ (1.5 g, 2.1 mmol), and DIPEA (2.7 g, 20.5 mmol) were dissolved in dioxane (67mL), and under N₂ protection, the mixture was reacted at 90 °C for 12 h. The reaction was monitored by TLC. The reaction solution was cooled to room temperature, and then the reaction was quenched by adding water (150mL), followed by adding EA (100mL). The resultant solution was filtered through diatomaceous earth. The filter cake was rinsed with EA (50 mL x 2). The layers were separated, followed by extracting with EA (50 mL x 2). The EA layers were combined, washed with saturated saline (50mL x 2), dried over anhydrous sodium sulfate, and filtered. The filtrate was concentrated, and the residue was subjected to column chromatography, to obtain 5.9 g of white solid **(210-1),** with a yield of 95.3%, M/S: 602 (M+H⁺).

### Step 2: Synthesis of intermediate (S) -1-(4-fluoro-1-methyl-1H-indazole-5-yl)-3-(2-(4-fluoro-3-methyl-5-vinylphenyl)-4-methyl-4,5,6,7-tetrahydro-2H-pyrazolo[4,3-c]pyridine-3-yl)-1,3-dihydro-2H-imidazole-2-one (210-2)

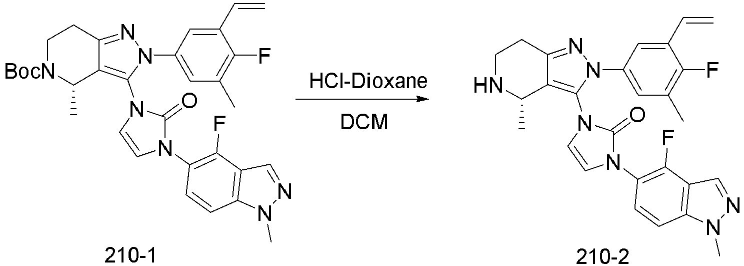

Intermediate **210-1** (5.9g, 9.8mmol) was dissolved in DCM (18 mL), to which was added HCl-Dioxane (27 mL, 4 N) dropwise in an ice bath, and then the reaction was naturally warmed and reacted for 3 h. The reaction was monitored by TLC, and then quenched by adding water (50 mL). NaHCO₃ saturated solution was added to adjust the pH to 8-9. The resultant solution was extracted with DCM (100mL x 3). DCM layers were combined, washed with saturated saline (100 mL x 2), dried over anhydrous sodium sulfate, filtered with suction, and concentrated, to obtain 4.7 g of white solid **(210-2),** with a yield of 95.5%, M/S: 502 (M+H⁺).

### Step 3: Synthesis of compound 3-((1S,2S)-1-(5-(S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3-methyl-5-vinylphenyl)-4-methyl-4,5,6,7-tetrahydro-2H-pyrazolo[4,3-c]pyridine-5-carbonyl)-1H-indole-1-yl)-2-methylcyclopropyl)-1,2,4-oxadiazole-5(4H)-one (210)

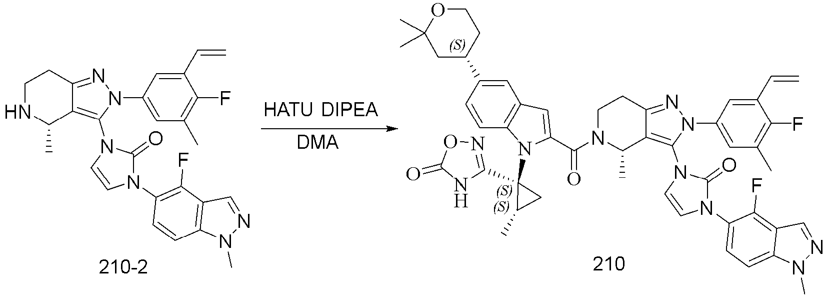

Intermediate **210-2** (4.7 g, 9.4 mmol), intermediate **1-9** (5 g, 12.2 mmol), and HATU (7.1 g, 18.7 mmol) were dissolved in DMA (15mL), to which was added DIPEA (2.4 g, 18.7 mmol) dropwise, and then reacted at room temperature for 8 h. The reaction was monitored by TLC. After completion of the reaction, water (50 mL) was added for quenching. The resultant solution was extracted with EA (50 mL x 3). The EA layers were combined, washed with water (50 mL x 3) and saturated saline (50mL x 2), dried over anhydrous sodium sulfate, filtered with suction, and concentrated. The residue was purified by column chromatography to obtain 6 g of white solid **(210),** with a yield of 71.5%, M/S: 895 (M+H⁺).

¹HNMR (400 MHz, DMSO-*d*₆) *δ* 11.76 (s, 1H), 8.30 (s, 1H), 7.63 (d, *J* = 8.9 Hz, 1H), 7.57 - 7.47 (m, 2H), 7.43 (dd, *J* = 16.7, 8.2 Hz, 2H), 7.38 - 7.30 (m, 1H), 7.27 (d, *J* = 8.6 Hz, 1H), 7.15 - 6.93 (m, 2H), 6.87 (dd, *J* = 17.6, 11.3 Hz, 1H), 5.84 (d, *J* = 17.6 Hz, 1H), 5.54 (dd, *J* = 48.7, 9.1 Hz, 2H), 4.40 (d, *J* = 12.8 Hz, 1H), 4.11 (s, 3H), 3.78 - 3.58 (m, 3H), 3.28 - 2.68 (m, 5H), 2.28 (d, *J* = 12.3 Hz, 3H), 1.83 - 1.40 (m, 9H), 1.27 (s, 3H), 1.11 (s, 6H).

### Example 18. Synthesis of compound 3-((1S,2S)-1-(2-((S)-2-(3-(3,3-dimethylbut-1-en-1-yl)-4-fluoro-5-methylphenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropyl)-1,2,4-oxadiazole-5(4H)-one (213)

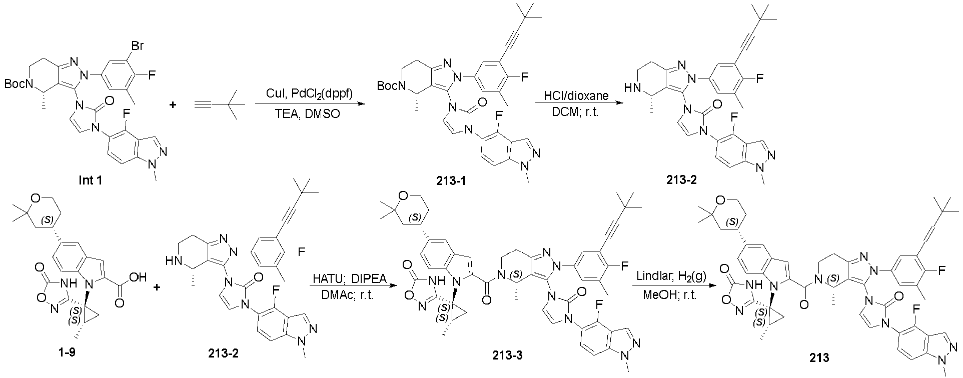

### Step 1: Synthesis of intermediate tert-butyl (S)-2-(3-(3,3-dimethylbut-1-yn-1-yl)-4-fluoro-5-methylphenyl)-3-(3-(4-fluoro-1-methyl-1H-indazole-5-yl)-2-oxo-2,3-dihydro-1H-imidazole-1-yl)-4-methyl-2,4,6,7-tetrahydro-5H-pyrazolo[4,3-c]pyridine-5-carboxylate (213-1)

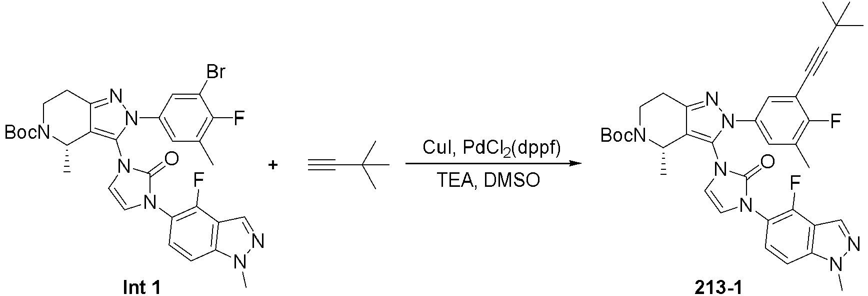

**Int 1** (653 mg, 1 mmol), tert-butyl acetylene (164 mg, 2 mmol), CuI (190 mg, 1 mmol), Pd(dppf)Cl₂ (73 mg, 0.1 mmol), and triethylamine (202 mg, 2 mmol) were successively added to DMSO (7 mL), and then stirred at 90 °C for 4 h. After cooling to room temperature, the reaction was subjected to column chromatography to obtain intermediate compound **213-1** (282 mg, 0.43 mmol), with a yield of 43%. M/S: 656.3 (M+H⁺).

### Step 2: Synthesis of intermediate (S)-1-(2-(3-(3,3-dimethylbut-1-yn-1-yl)-4-fluoro-5-methylphenyl)-4-methyl-4,5,6,7-tetrahydro-2H-pyrazolo[4,3-c]pyridine-3-yl)-3-(4-fluoro-1-methyl-1H-indazole-5-yl)-1,3-dihydro-2H-imidazole-2-one (213-2)

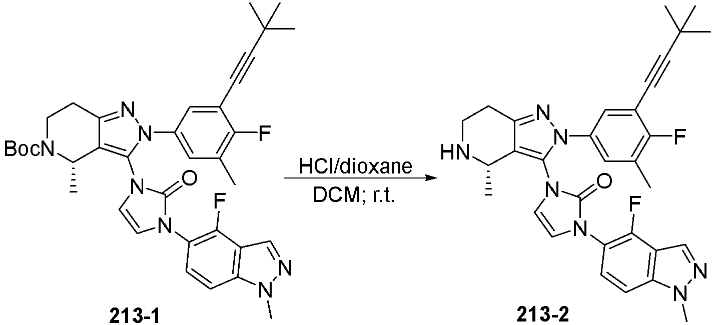

Intermediate **213-1** (282 mg, 0.43 mmol) was dissolved in DCM (3 mL), to which was added a solution of HCl in 1,4-dioxane (4 M, 1 mL), and then the mixture was allowed to react at room temperature for 2 h. The pH was adjusted to >7 by adding NaHCO₃ saturated aqueous solution. The resultant solution was extracted with ethyl acetate (20 mL × 2). The organic layers were combined, washed with saturated saline (20 mL), dried over anhydrous sodium sulfate, and concentrated under reduced pressure to obtain the product (211 mg, 0.38 mmol), with a yield of 88.8%. Ms: 556.2 (M+H⁺).

### Step 3: Synthesis of intermediate 3-((1S,2S)-1-(2-((S)-2-(3-(3,3-dimethylbut-1-yn-1-yl)-4-fluoro-5-methylphenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropyl)-1,2,4-oxadiazole-5(4H)-one (213-3)

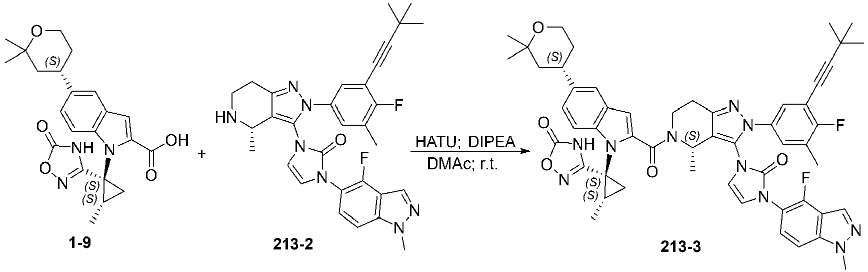

Compounds **1-9** (Example 2) (49 mg, 0.1 mmol), **213-2** (55 mg, 0.1 mmol), HATU (76 mg, 0.2 mmol), and DIPEA (26 mg, 0.2 mmol) were added to DMAc (0.5 mL), and then allowed to react at room temperature for 8 h. The reaction was monitored by TLC. After completion of the reaction, water (5 mL) was added for quenching. The resultant solution was extracted with EA (5 mL × 3). The EA layers were combined, washed with water (5 mL x 3) and saturated saline (5 mL x 2), dried over anhydrous sodium sulfate, filtered with suction, and concentrated. The residue was subjected to column chromatography, to obtain **213-3** (68 mg, 0.072 mml), with a yield of 71.5%. M/S: 949.1 (M+H⁺).

### Step 4: Synthesis of compound 3-((1S,2S)-1-(2-((S)-2-(3-(3,3-dimethylbut-1-yn-1-yl)-4-fluoro-5-methylphenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropyl)-1,2,4-oxadiazole-5(4H)-one (213)

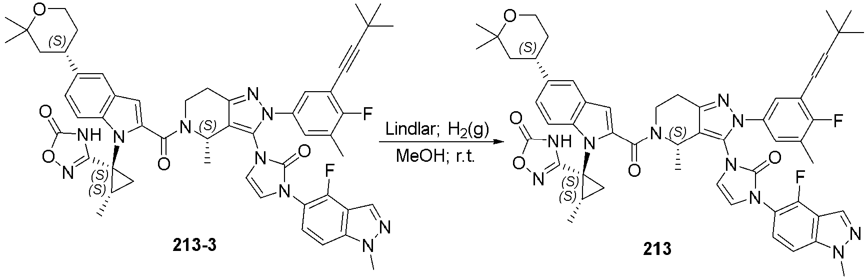

**213-3** (68 mg, 0.072 mmol) was added to methanol (1 mL), to which was added Lindlar catalyst (3.4 mg, 5%), and then stirred under hydrogen atmosphere. The reaction was monitored by TLC. After completion of the reaction, the solution was filtered, followed by column chromatography to obtain compound **213** (34 mg, 0.036 mmol), with a yield of 50%, M/S: 951.1 (M+H⁺).

### Example 19. Synthesis of compound 3-((1S,2S)-1-(5-((S)-2,2-dimethyltetrahydro-2H-pyran-4-yl)-2-((S)-2-(3-ethynyl-4-fluoro-5-methylphenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-1H-indole-1-yl)-2-methylcyclopropyl)-1,2,4-oxadiazole-5(4H)-one (215)

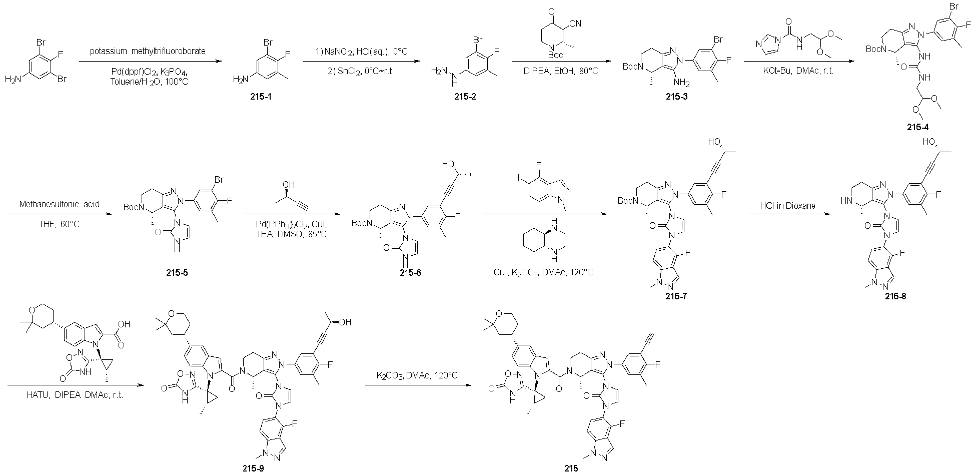

### Step 1. Synthesis of intermediate 3-bromo-4-fluoro-5-methylaniline (215-1)

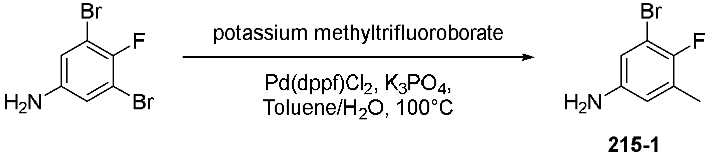

Procedures: 3,5-dibromo-4-fluoroaniline (10.8 g, 40.0 mmol), potassium methyltrifluoroborate (9.8 g, 80.0 mmol), 1,1'-bis(diphenylphosphino)ferrocene palladium(II) dichloride (2.9 g, 4.0 mmol), and potassium phosphate (25.4 g, 120.0 mmol) were successively added to a mixture of toluene and water (10:1, 150 mL). The system was purged with nitrogen three times. The reaction solution was heated to 100 °C in an oil bath, allowed to react overnight, and then cooled to room temperature, followed by filtering. The filter cake was rinsed with ethyl acetate (50 mL). The filtrates were combined, and then concentrated under reduced pressure. The residue was purified by column chromatography to obtain 4.1 g of product. Yield: 50.0%. Ms: 204.0/206.0 (M+H⁺).

### Step 2. Synthesis of intermediate (3-bromo-4-fluoro-5-methylphenyl)hydrazine (215-2)

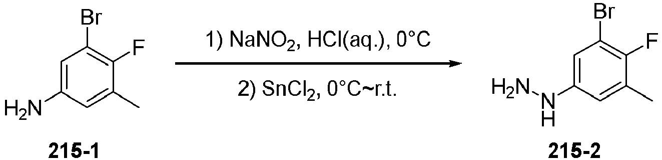

Procedures: Intermediate **215-1** (4.1 g, 20.1 mmol) was dissolved in 6N hydrochloric acid (40 mL), and then cooled to 0°C, to which was added an aqueous solution (4 mL) of sodium nitrite (2.1 g, 30.1 mmol) dropwise. The mixture was allowed to react at 0 °C for 0.5 h, and then a solution (10 mL) of stannous chloride (7.6 g, 40.1 mmol) in concentrated hydrochloric acid was added dropwise. The reaction solution was naturally warmed and reacted for 3 h. After filtration, the filter cake was triturated in methyl tert-butyl ether (50 mL) and then filtered. The filter cake was dried to obtain 2.2 g of the product hydrochloride salt, with a yield of 50.0%. Ms: 219.0/221.0 (M+H⁺).

### Step 3. Synthesis of intermediate tert-butyl (S)-3-amino-2-(3-bromo-4-fluoro-5-methylphenyl)-4-methyl-2,4,6,7-tetrahydro-5H-pyrazolo[4,3-c]pyridine-5-carboxylate (215-3)

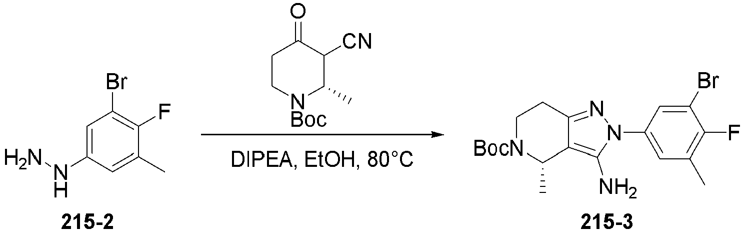

Procedures: Intermediate **215-2** hydrochloride (1.6 g, 6.3 mmol), tert-butyl (2S)-3-cyano-2-methyl-4-oxopiperidine-1-carboxylate (1.5 g, 6.3 mmol), and N,N-diisopropylethylamine (1.6 g, 12.6 mmol) were sequentially added to ethanol (20 mL), and then reacted at 80°C overnight. The reaction solution was concentrated under reduced pressure. The residue was purified by column chromatography to obtain 0.92 g of product. Yield: 33.4%. Ms: 439.1/441.1 (M+H⁺).

### Step 4. Synthesis of intermediate tert-butyl (S)-2-(3-bromo-4-fluoro-5-methylphenyl)-3-(3-(2,2-dimethoxyethyl)ureido)-4-methyl-2,4,6,7-tetrahydro-5H-pyrazolo[4,3-c]pyridine-5-carboxylate (215-4)

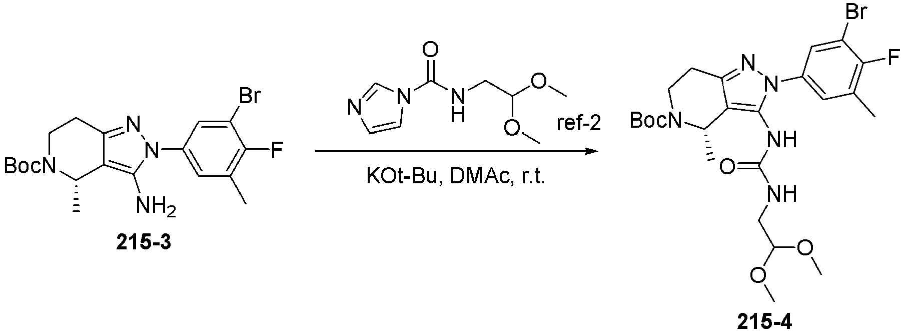

Procedures: Intermediate **215-3** (0.92 g, 2.1 mmol), intermediate **ref-2** (0.84 g, 4.2 mmol), and potassium tert-butoxide (0.71 g, 6.3 mmol) were sequentially added to *N,N-*dimethylacetamide (15 mL), and then reacted at room temperature for 2 h. The reaction solution was diluted with ethyl acetate (50 mL), and then washed once with water (25 mL) and once with saturated saline (25 mL). The organic layer was dried over anhydrous sodium sulfate, and concentrated under reduced pressure. The residue was purified by column chromatography to obtain 1.1 g of product. Yield: 92.1%. Ms: 570.2/572.2 (M+H⁺).

### Step 5. Synthesis of intermediate tert-butyl (S)-2-(3-bromo-4-fluoro-5-methylphenyl)-4-methyl-3-(2-oxo-2,3-dihydro-1H-imidazole-1-yl)-2,4,6,7-tetrahydro-5H-pyrazolo[4,3-c]pyridine-5-carboxylate (215-5)

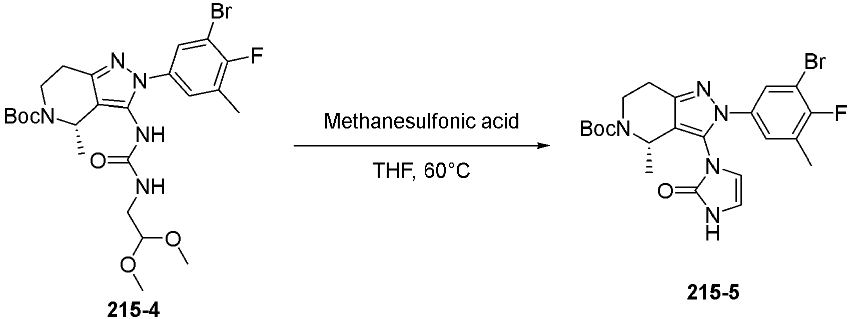

Procedures: Intermediate **215-4** (1.1 g, 1.9 mmol) and methanesulfonic acid (0.37 g, 3.8 mmol) were sequentially added to tetrahydrofuran (55 mL), and then allowed to react at 60 °C for 2 h. The reaction solution was poured into ice water (50 mL), and then extracted with ethyl acetate (100 mL×2). The organic layers were combined, washed once with NaHCO₃ saturated aqueous solution (50 mL), dried over anhydrous sodium sulfate, and concentrated under reduced pressure. The residue was purified by column chromatography to obtain 0.84 g of product. Yield: 86.0%. Ms: 506.1/508.1 (M+H⁺).

### Step 6. Synthesis of intermediate tert-butyl (S)-2-(4-fluoro-3-((R)-3-hydroxybut-1-yn-1-yl)-5-methylphenyl)-4-methyl-3-(2-oxo-2,3-dihydro-1H-imidazole-1-yl)-2,4,6,7-tetrahydro-5H-pyrazolo[4,3-c]pyridine-5-carboxylate (215-6)

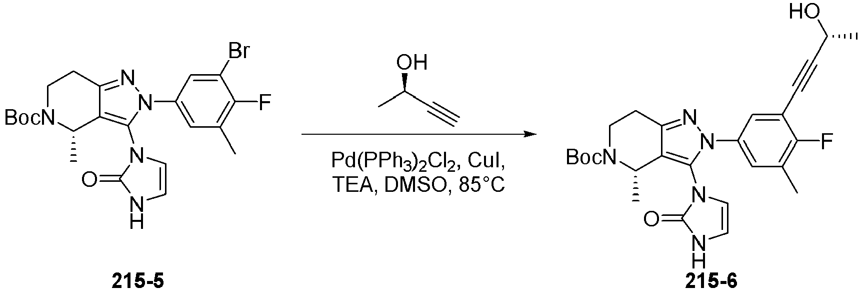

Procedures: Intermediate **215-5** (290 mg, 0.57 mmol), (R)-(+)-3-butyn-2-ol (200 mg, 2.9 mmol), bis(triphenylphosphine)palladium(II) dichloride (80 mg, 0.11 mmol), CuI (22 mg, 0.11 mmol), and triethylamine (580 mg, 5.7 mmol) were sequentially added to dimethylsulfoxide (6 mL). The system was purged with nitrogen three times, reacted at 85 °C for 4 h, cooled to room temperature, and filtered. The filter cake was triturated in ethyl acetate (20 mL × 2). The filtrates were combined, washed with water (20 mL × 2) and once with saturated saline (20 mL), dried over anhydrous sodium sulfate, and concentrated under reduced pressure. The residue was purified by column chromatography to obtain 205 mg of product. Yield: 72.2%. Ms: 496.2 (M+H⁺).

### Step 7. Synthesis of intermediate tert-butyl (S)-3-(3-(4-fluoro-1-methyl-1H-indazole-5-yl)-2-oxo-2,3-dihydro-1H-imidazole-1-yl)-2-(4-fluoro-3-((R)-3-hydroxybut-1-yn-1-yl)-5-methylphenyl)-4-methyl-2,4,6,7-tetrahydro-5H-pyrazolo[4,3-c]pyridine-5-carboxylate (215-7)

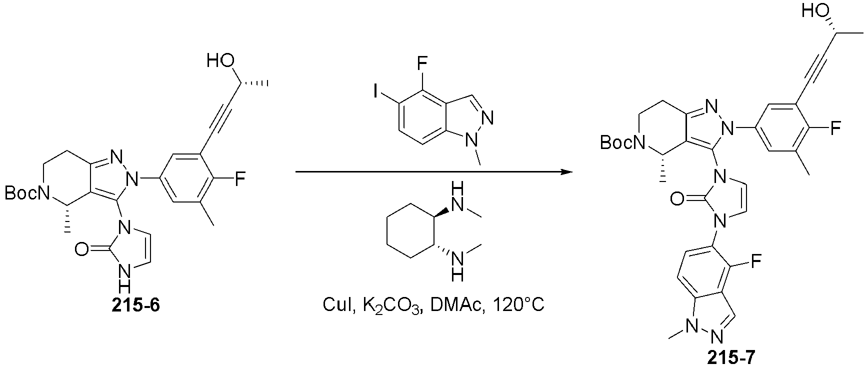

Procedures: Intermediate **215-6** (205 mg, 0.41 mmol), intermediate **1-4** (137 mg, 0.50 mmol), (1S,2S)-(-)-*N*,*N*'-dimethyl-1,2-cyclohexanediamine (59 mg, 0.41 mmol), CuI (79 mg, 0.41 mmol), and K₂CO₃ (86 mg, 0.62 mmol) were sequentially added to *N*,*N*-dimethylacetamide (6 mL). The system was purged with nitrogen three times, reacted at 120 °C for 4 h, cooled to room temperature, and filtered. The filter cake was triturated in ethyl acetate (20 mL × 2). The filtrates were combined, washed with water (20 mL × 2) and once with saturated saline (20 mL), dried over anhydrous sodium sulfate, and concentrated under reduced pressure. The residue was purified by column chromatography to obtain 160 mg of product. Yield: 60.1%. Ms: 644.2 (M+H⁺).

### Step 8. Synthesis of intermediate tert-butyl 1-(4-fluoro-1-methyl-1H-indazole-5-yl)-3-((S)-2-(4-fluoro-3-((R)-3-hydroxybut-1-yn-1-yl)-5-methylphenyl)-4-methyl-4,5,6,7-tetrahydro-2H-pyrazolo[4,3-c]pyridine-3-yl)-1,3-dihydro-2H-imidazole-2-one (215-8)

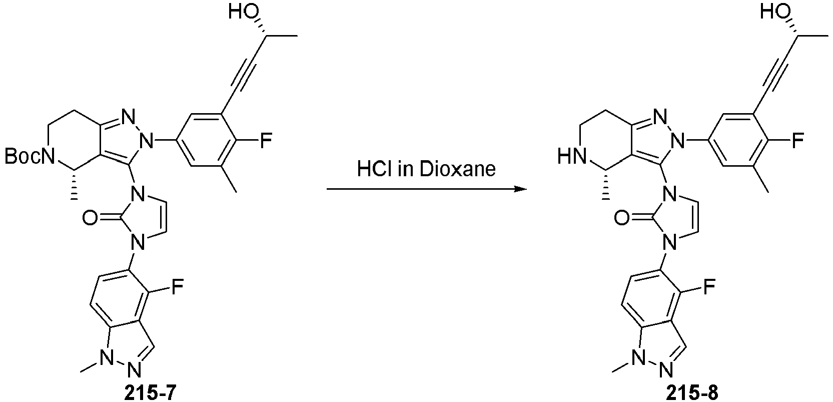

Procedures: Intermediate **215-7** (160 mg, 0.25 mmol) was added to a solution of HCl in 1,4-dioxane (4 M, 5 mL), and then reacted at room temperature for 2 h. The reaction solution was adjusted to pH >7 with NaHCO₃ saturated aqueous solution, and then extracted with ethyl acetate (20 mL × 2). The organic layers were combined, washed with saturated saline (20 mL), dried over anhydrous sodium sulfate, and concentrated under reduced pressure, to obtain 120 mg of product. Yield: 88.8%. Ms: 544.2 (M+H⁺).

### Step 9. Synthesis of intermediate 3-((1S,2S)-1-(5-((S)-2,2-dimethyltetrahydro-2H-pyran-4-yl)-2-((S)-3-(3-(4-(4-fluoro-1-methyl-1H-indazole-5-yl)-2-oxo-2,3-dihydro-1H-imidazole-1-yl)-2-(4-fluoro-3-((R)-3-hydroxybut-1-yn-1-yl)-5-methylphenyl)-4-methyl-4,5,6,7-tetrahydro-2H-pyrazolo[4,3-c]pyridine-5-carbonyl)-1H-indole-1-yl)-2-methylcyclopropyl)-1,2,4-oxadiazole-5(4H)-one (215-9)

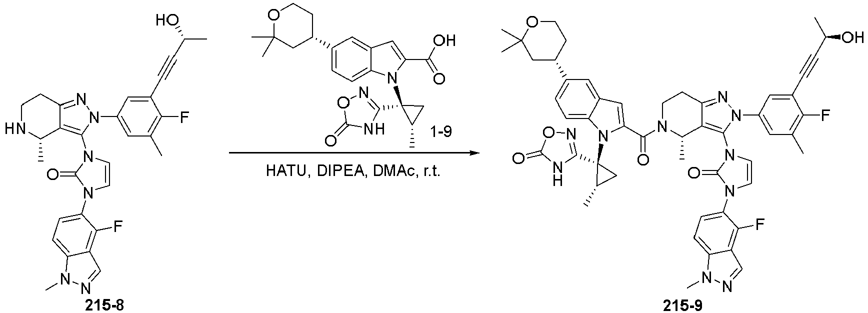

Procedures: Intermediate **215-8** (100 mg, 0.18 mmol), intermediate **1-9** (91 mg, 0.22 mmol), HATU (105 mg, 0.28 mmol), and *N,N*-diisopropylethylamine (48 mg, 0.37 mmol) were sequentially added to *N,N*-dimethylacetamide (2 mL), and then reacted at room temperature for 4 h. The reaction solution was diluted with ethyl acetate (15 mL), and then washed once with water (10 mL) and once with saturated saline (10 mL). The organic layer was dried over anhydrous sodium sulfate, and concentrated under reduced pressure. The residue was purified by column chromatography to obtain 90 mg of product, with a yield of 52.2%. Ms: 937.2 (M+H⁺). ¹H NMR (400 MHz, DMSO-*d*₆) *δ* 11.74 (s, 1H), 8.29 (s, 1H), 7.64 (d, *J* = 8.8 Hz, 1H), 7.53 (s, 1H), 7.51 - 7.46 (m, 1H), 7.44 - 7.33 (m, 2H), 7.32 - 7.21 (m, 2H), 7.12 (d, *J* = 3.2 Hz, 1H), 7.04 - 6.92 (m, 2H), 5.66 - 5.52 (m, 2H), 4.71 - 4.57 (m, 1H), 4.47 - 4.33 (m, 1H), 4.17 - 4.03 (m, 4H), 3.74 - 3.66 (m, 3H), 3.10 - 2.97 (m, 1H), 2.96 - 2.83 (m, 1H), 2.37 - 2.20 (m, 4H), 1.76 - 1.48 (m, 5H), 1.46 - 1.35 (m, 5H), 1.34 - 1.21 (m, 5H), 1.20 - 1.14 (m, 6H).

### Step 10. Synthesis of compound 3-((1S,2S)-1-(5-((S)-2,2-dimethyltetrahydro-2H-pyran-4-yl)-2-((S)-2-(3-ethynyl-4-fluoro-5-methylphenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-1H-indole-1-yl)-2-methylcyclopropyl)-1,2,4-oxadiazole-5(4H)-one (215)

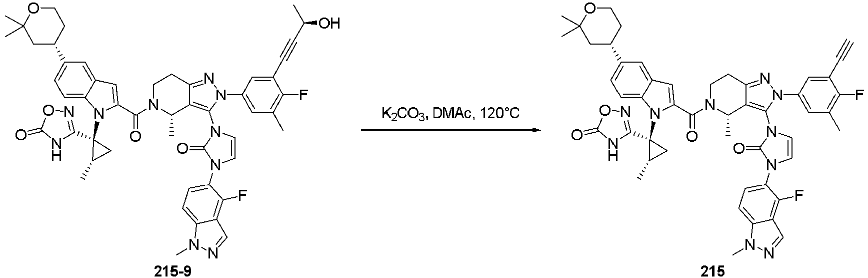

Procedures: Intermediate **215-9** (35 mg, 0.04 mmol) and K₂CO₃ (103 mg, 0.75 mmol) were sequentially added to *N,N*-dimethylacetamide (1 mL), and reacted at 120 °C for 24 h. The reaction solution was cooled to room temperature, diluted with ethyl acetate (10 mL), washed with water (5 mL × 2) and once with saturated saline solution (5 mL), dried over anhydrous sodium sulfate, and concentrated under reduced pressure. The residue was purified by column chromatography to obtain 15 mg of product. Yield: 45.0%. Ms: 893.0 (M+H⁺). ¹H NMR (400 MHz, DMSO-*d*₆) *δ* 11.74 (s, 1H), 8.29 (d, *J* = 5.3 Hz, 1H), 7.64 (d, *J* = 8.8 Hz, 1H), 7.53 (s, 1H), 7.50 - 7.33 (m, 3H), 7.31 - 7.21 (m, 2H), 7.12 (d, *J* = 3.1 Hz, 1H), 7.02 (s, 1H), 6.95 (s, 1H), 5.74 - 5.60 (m, 1H), 4.59 (s, 1H), 4.17 - 3.99 (m, 4H), 3.78 - 3.62 (m, 2H), 3.10 - 2.96 (m, 1H), 2.96 - 2.85 (m, 1H), 2.34 - 2.22 (m, 4H), 1.84 - 1.37 (m, 7H), 1.36 - 1.21 (m, 7H), 1.20 - 1.14 (m, 6H).

### Example 20. Synthesis of compound 3-((1S,2S)-1-(5-(S)-2,2-dimethyltetrahydro-2H-pyran-4-yl)-2-((S)-3-(3-(4-fluoro-1-methyl-1H-indazole-5-yl)-5-methyl-2-oxo-2,3-dihydro-1H-imidazole-1-yl)-2-(4-fluoro-3,5-divinylphenyl)-4-methyl-4,5,6,7-tetrahydro-2H-pyrazolo[4,3-c]pyridine-5-carbonyl)-1H-indole-1-yl)-2-methylcyclopropyl)-1,2,4-triazine-5(4H)-one (232)

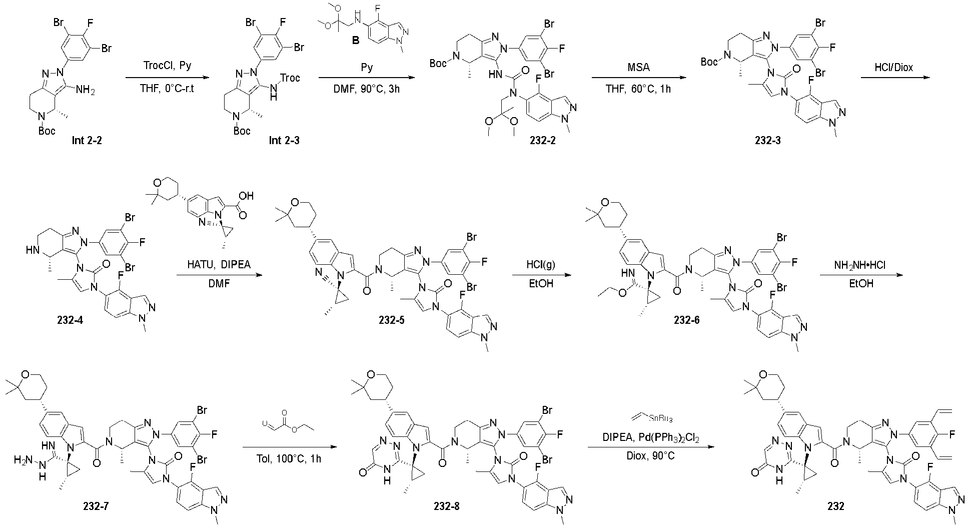

### Step 1: Synthesis of intermediate tert-butyl (S)-2-(3,5-dibromo-4-fluorophenyl)-4-methyl-3-((2,2,2-trichloroethoxycarbonyl)amino)-2,4,6,7-tetrahydro-5H-pyrazolo[4,3-c]pyridine-5-carboxylate (Int 2-3)

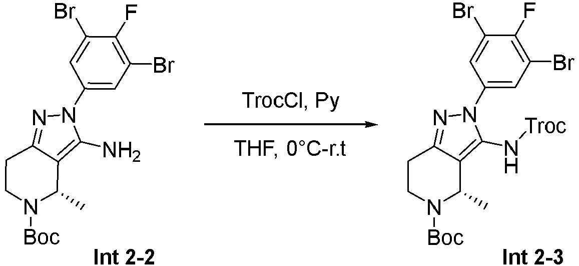

At room temperature, intermediate **Int 2-2** (8 g, 15.9 mmol) was dissolved in THF (160 mL), to which was added pyridine (2.5 g, 79.1 mmol). The system was purged with nitrogen, and then chloroformic acid-2,2,2-trichloroethyl ester (1.6 g, 7.44 mmol) was slowly added dropwise in an ice bath. The reaction solution was stirred at room temperature overnight, and then adjusted to pH 12 with NaOH saturated aqueous solution, followed by stirring for half an hour. TLC was used to monitor the reaction until disappearance of the raw materials. The reaction solution was diluted with 50 mL of water, and then extracted with ethyl acetate (100 mL × 3). The organic phase was washed with 50 mL of saturated saline, dried over sodium sulfate, and filtered. The filtrate was concentrated under reduced pressure. The residue was purified by silica gel column chromatography to obtain 9.5 g of product as light yellow solid, with a yield of 88%. M/S: 677 (M+H⁺).

### Step 2: Synthesis of intermediate tert-butyl (S)-2-(3,5-dibromo-4-fluorophenyl)-3-(3-(2,2-dimethoxypropyl)-3-(4-fluoro-1-methyl-1H-indazole-5-yl)ureido)-4-methyl-2,4,6,7-tetrahydro-5H-pyrazolo[4,3-c]pyridine-5-carboxylate (232-2)

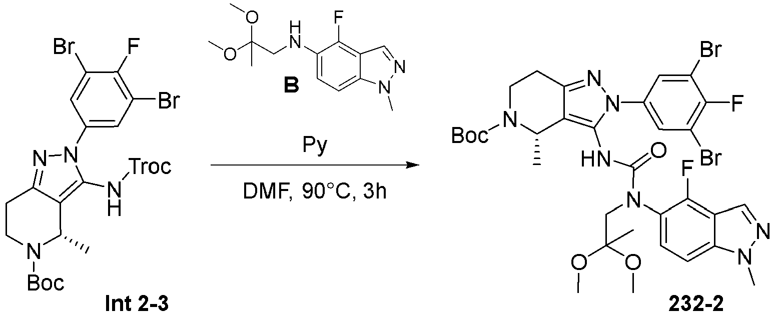

Intermediate **Int 2-3** (9.5 g, 14 mmol), intermediate **B** (7.5 g, 28 mmol), and pyridine (33.2 g, 419 mmol) were dissolved in DMF (100 mL), and then stirred at 90 °C for 3 h. The reaction of raw materials was monitored to be complete by TLC. The reaction solution was diluted with 100 mL of water and extracted with ethyl acetate (100 mL × 3). The organic phases were combined, washed with 50 mL of saturated saline, dried over sodium sulfate, and concentrated under reduced pressure. The residue was purified by column chromatography, to obtain 10 g of yellow solid compound, with a yield of 91%. M/S: 796 (M+H⁺).

### Step 3: Synthesis of intermediate tert-butyl (S)-2-(3,5-dibromo-4-fluorophenyl)-3-(3-(4-fluoro-1-methyl-1H-indazole-5-yl)-5-methyl-2-oxo-2,3-dihydro-1H-imidazole-1-yl)-4-methyl-2,4,6,7-tetrahydro-5H-pyrazolo[4,3-c]pyridine-5-carboxylate (232-3)

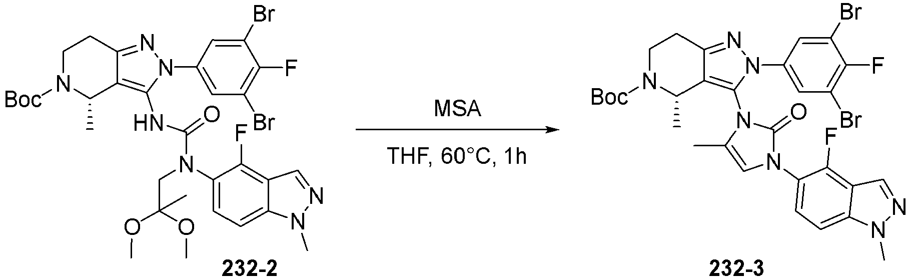

At room temperature, intermediate **232-2** (10 g, 12.8 mmol) and methanesulfonic acid (12.3 g, 128 mmol) were dissolved in THF (200 mL), and then stirred at 60 °C for 1 h. The reaction of raw materials was monitored to be complete by TLC. The reaction solution was diluted with 100 mL of water, and then extracted with ethyl acetate (150 mL × 3). The organic phase was washed with 100 mL of saturated saline, dried over sodium sulfate, and filtered. The filtrate was concentrated under reduced pressure, and the concentrate was purified by silica gel chromatography to obtain 8.6 g of product as light yellow solid, with a yield of 90%. M/S: 732 (M+H⁺).

### Step 4: Synthesis of intermediate (S)-3-(2-(3,5-dibromo-4-fluorophenyl)-4-methyl-4,5,6,7-tetrahydro-2H-pyrazolo[4,3-c]pyridine-3-yl)-1-(4-fluoro-1-methyl-1H-indazole-5-yl)-4-methyl-1,3-dihydro-2H-imidazole-2-one (232-4)

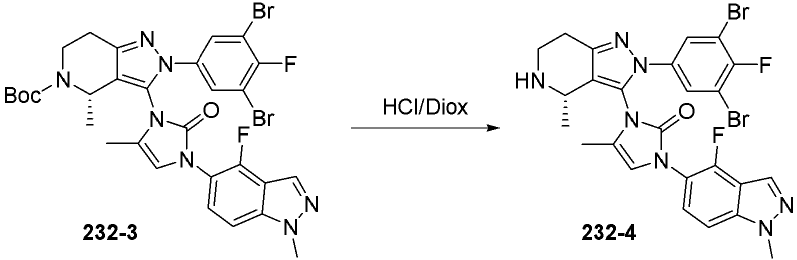

At room temperature, compound **232-3** (8.6 g, 11.7 mmol) was placed in a 250 mL one-neck flask, to which was added a solution of hydrochloric acid in 1,4-dioxane (50 mL, 4N), and the mixture was stirred at room temperature for 1 h. K₂CO₃ aqueous solution was added to adjust the pH to 11. The resultant solution was extracted with ethyl acetate (10 mL × 3). The organic phases were combined, washed with 20 mL of saturated saline, dried over sodium sulfate, filtered, and concentrated under reduced pressure, to obtain 6.9 g of yellow solid product, with a yield of 94%. M/S: 632 (M+H⁺).

### Step 5: Synthesis of intermediate ((1S,2S)-1-(2-((S)-2-(3,5-dibromo-4-fluorophenyl)-3-(3-(4-fluoro-1-methyl-1H-indazole-5-yl)-5-methyl-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropane-1-nitrile (232-5)

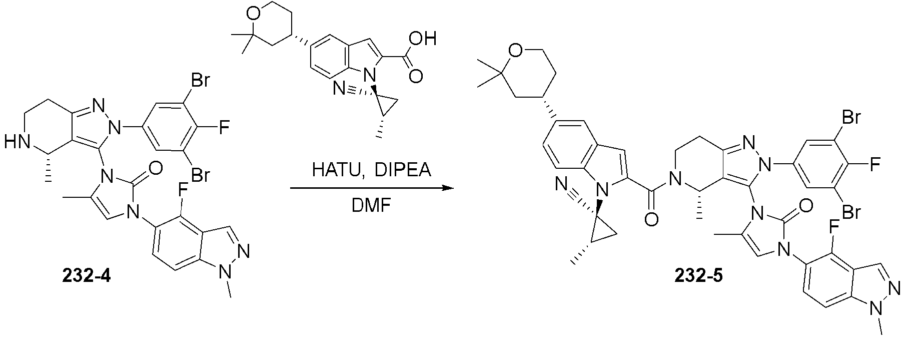

At room temperature, 1-((1S,2S)-1-cyano-2-methylcyclopropyl)-5-((S)-2,2-dimethyltetrahydro-2H-pyran-4-yl)-1H-indole-2-carboxylic acid (160 mg, 0.46 mmol) was dissolved in DMF (20 mL), to which were added HATU (250 mg, 0.68 mmol) and DIPEA (175 mg, 1.4 mmol), and the mixture was stirred and reacted for 20 min. Then, intermediate **232-4** (290 mg, 0.46 mmol) was added, and the reaction was stirred and reacted at room temperature for 2 h. The raw material was monitored to be completely reacted by TLC. The reaction solution was diluted with 50 mL of water, and extracted with ethyl acetate (50 mL × 3). The organic phase was washed with 50 mL of saturated saline, dried over sodium sulfate, and filtered. The filtrate was concentrated under reduced pressure, and the concentrate was purified by silica gel column chromatography to obtain 300 mg of product as light yellow solid, with a yield of 68%. M/S: 966 (M+H⁺).

### Step 6: Synthesis of intermediate (1S,2S)-1-(2-((S)-2-(3,5-dibromo-4-fluorophenyl)-3-(3-(4-fluoro-1-methyl-1H-indazole-5-yl)-5-methyl-2-oxo-2,3-dihydro-1-imidazole-1-yl)-4-methyl-4,5,6,7-tetrahydro--2H-pyrazolo[4,3-c]pyridine-5-carbonyl)-5-((S)-2,2-dimethyltetrahydro-2H-pyran-4-yl)-1H-indole-1-yl)-2-methylcyclopropane-1-carbimide ester (232-6)

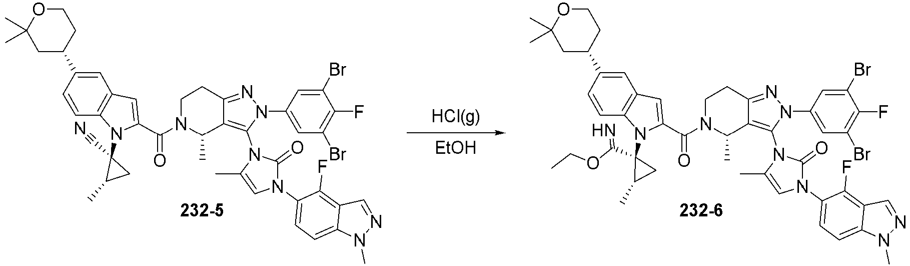

Intermediate **232-5** (300 mg, 0.31 mmol) was dissolved in anhydrous ethanol (30 mL), to which was introduced hydrochloric acid gas at 10 °C, and then stirred and reacted for 6 h. The reaction of the raw material was monitored by TLC until it was complete. The reaction solution was adjusted to pH ≈ 7 with NaHCO₃ aqueous solution, then extracted with ethyl acetate (50 mL × 3). The organic phases were combined, dried over anhydrous sodium sulfate, filtered, and concentrate under reduced pressure, to obtain 290 mg of crude product, with a yield of 79%. M/S: 1012 (M+H⁺).

### Step 7: Synthesis of intermediate (1S,2S)-1-(2-((S)-2-(3,5-dibromo-4-fluorophenyl)-3-(3-(4-fluoro-1-methyl-1H-indazole-5-yl)-5-methyl-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropane-1-carboxylic hydrazide (232-7)

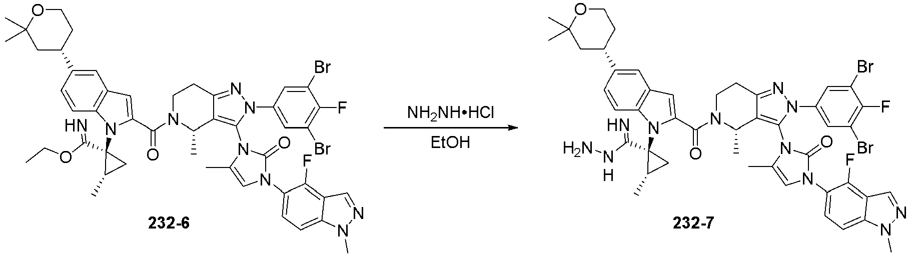

Intermediate **232-6** (290 mg, 0.29 mmol) was dissolved in ethanol (10 mL), to which were added hydrazine hydrochloride (200 mg, 2.9 mmol) and NaHCO₃ saturated solution (2 mL) in an ice bath, and the mixture was stirred at room temperature for 2 h. TLC monitoring indicated complete reaction of the starting materials. The reaction solution was diluted with 50 mL of water and extracted with ethyl acetate (50 mL × 3). The organic phases were combined, dried over anhydrous sodium sulfate, filtered, and concentrated under reduced pressure. The concentrate was purified by silica gel column chromatography to obtain 170 mg of product as light yellow solid, with a yield of 65%. M/S: 998 (M+H⁺).

### Step 8: Synthesis of intermediate 3-((1S,2S)-1-(2-(S)-2-(3,5-dibromo-4-fluorophenyl)-3-(3-(4-fluoro-1-methyl-1H-indazole-5-yl)-5-methyl-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropyl)-1,2,4-triazine-5(4H)-one (232-8)

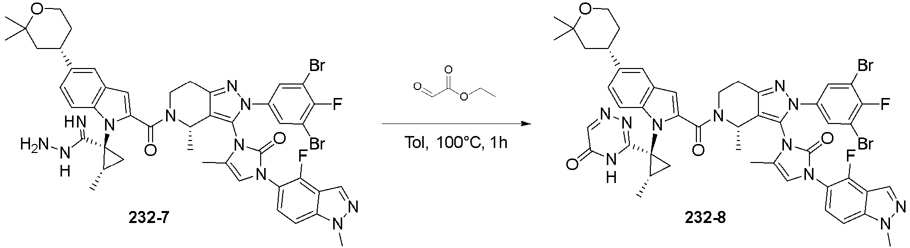

Intermediate **232-7** (170 mg, 0.19 mmol) was dissolved in toluene (10 mL), to which was added ethyl glyoxylate (52 mg, 0.5 mmol), and then stirred and reacted at 100 °C for 1 h. The reaction of raw materials was monitored by TLC until complete. The reaction solution was cooled to room temperature, diluted with 50 mL of water, and extracted with ethyl acetate (50 mL×3). The organic phases were combined, dried over anhydrous sodium sulfate, filtered, and concentrated under reduced pressure. The concentrate was purified by silica gel column chromatography to obtain 100 mg of product as light yellow solid, with a yield of 51%. M/S: 1036 (M+H⁺).

### Step 9: Synthesis of compound 3-((1S,2S)-1-(2-(S)-2-(3,5-divinyl-4-fluorophenyl)-3-(3-(4-fluoro-1-methyl-1H-indazole-5-yl)-5-methyl-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropyl)-1,2,4-triazine-5(4H)-one (232)

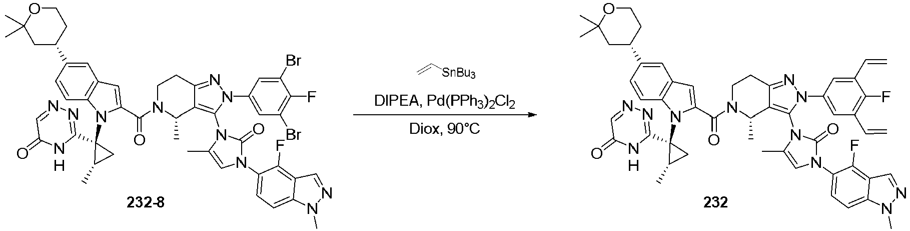

Intermediate **232-8** (170 mg, 0.19 mmol), tributylvinyltin (190 mg, 0.6 mmol), DIPEA (78 mg, 0.6 mmol), and Pd(PPh3)₂Cl₂ (30 mg, 0.04 mmol) were dissolved in 1,4-dioxane (3 mL). The system was purged with nitrogen three times, and then the mixture was stirred at 90 °C overnight. TLC monitoring indicated complete reaction of the starting materials. The reaction solution was cooled to room temperature, diluted with 50 mL of water, and extracted with ethyl acetate (50 mL × 3). The organic phases were combined, dried over anhydrous sodium sulfate, filtered, and concentrated under reduced pressure. The concentrate was purified by silica gel chromatography to obtain 50 mg of product as light yellow solid, with a yield of 28%. M/S: 932 (M+H⁺).

¹H NMR (400 MHz, DMSO-*d*₆) *δ* 8.31 (s, 1H), 8.14 (d, *J* = 2.0 Hz, 1H), 7.65 (d, *J* = 9.2 Hz, 1H), 7.56 (d, *J* = 6.1 Hz, 1H), 7.50 (d, *J* = 7.2 Hz, 1H), 7.41 (s, 1H), 7.20 (s, 1H), 6.99 - 6.72 (m, 4H), 5.88 (d, *J* = 17.6 Hz, 2H), 5.52 (d, *J* = 11.2 Hz, 2H), 4.12 (s, 3H), 3.74 - 3.65 (m, 2H), 3.01 (d, *J* = 12.8 Hz, 2H), 2.67 (d, *J* = 1.6 Hz, 1H), 2.00 (d, *J* = 7.6 Hz, 1H), 1.80 - 1.06 (m, 26H).

### Example 21. Synthesis of compound 3-(1-(7-((S)-2,2-dimethyltetrahydro-2H-pyran-4-yl)-2-((S)-2-(3-ethynyl-4-fluoro-5-methylphenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)indolizine-3-yl)cyclopropyl)-1,2,4-oxadiazole-5(4H)-one (322)

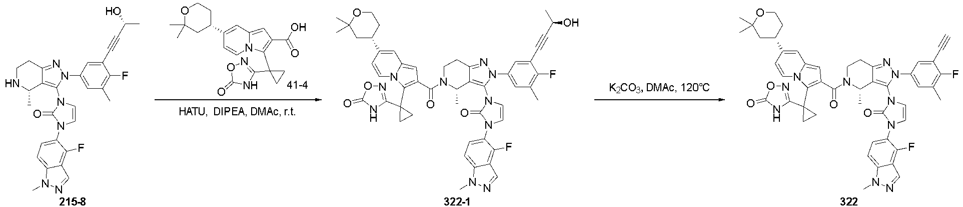

### Step 1. Synthesis of intermediate 3-(1-(7-((S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3-((R)-3-hydroxyl-1-butyne-1-yl)-5-methylphenyl)-4-methyl-4,5,6,7-tetrahydro-2H-pyrazolo[4,3-c]pyridine-5-carbonyl)indolizine-3-yl)cyclopropyl)-1,2,4-oxadiazole-5(4H)-one (322-1)

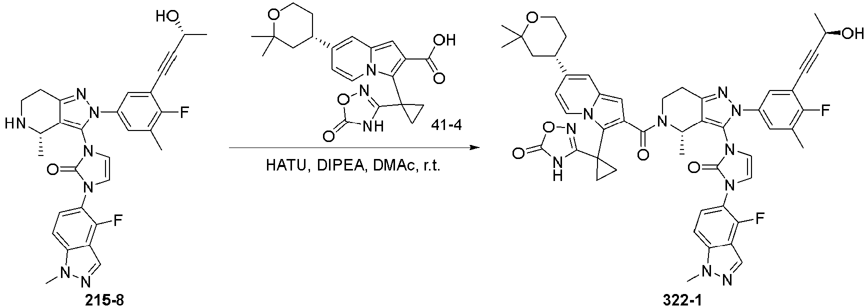

Procedures: Intermediate **215-8** (100 mg, 0.18 mmol), intermediate **41-4** (88 mg, 0.22 mmol), HATU (105 mg, 0.28 mmol), and *N,N*-diisopropylethylamine (48 mg, 0.37 mmol) were sequentially added to *N,N*-dimethylacetamide (2 mL) and reacted at room temperature for 4 h. The reaction solution was then diluted with ethyl acetate (15 mL), and then washed once with water (10 mL) and once with saturated saline (10 mL). The organic layer was dried over anhydrous sodium sulfate, concentrated under reduced pressure, and purified by column chromatography to obtain 90 mg of product. Yield: 53.0%. Ms: 923.2 (M+H⁺).

### Step 2. Synthesis of compound -3-(1-(7-((S)-2,2-dimethyltetrahydro-2H-pyran-4-yl)-2-((S)-2-(3-ethynyl-4-fluoro-5-methylphenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)indolizine-3-yl)cyclopropyl)-1,2,4-oxadiazole-5(4H)-one (322)

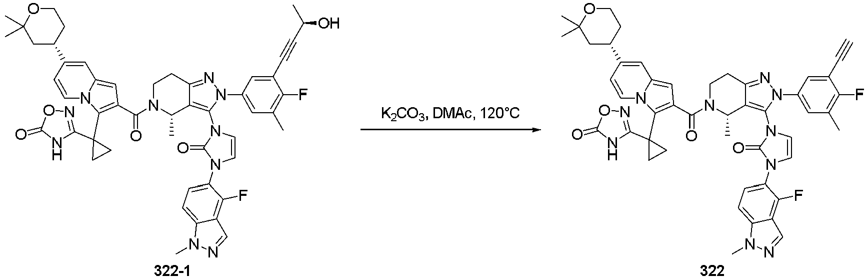

Procedures: Intermediate **322-1** (50 mg, 0.05 mmol) and K₂CO₃ (149 mg, 1.08 mmol) were successively added to *N,N*-dimethylacetamide (1 mL), and then reacted at 120 °C for 24 h. The reaction solution was cooled to room temperature, diluted with ethyl acetate (10 mL), washed with water (5 mL×2) and once with saturated saline (5 mL), dried over anhydrous sodium sulfate, and concentrated under reduced pressure. The residue was purified by column chromatography to obtain 20 mg of product. Yield: 42.0%. Ms: 879.3 (M+H⁺). ¹H NMR (400 MHz, DMSO-*d*₆) *δ* 12.03 (s, 1H), 8.30 (s, 1H), 8.16 (d, *J* = 7.2 Hz, 1H), 7.64 (d, *J* = 8.7 Hz, 1H), 7.50 - 7.40 (m, 1H), 7.38 - 7.25 (m, 4H), 7.15 - 6.99 (m, 1H), 6.72 (d, *J* = 7.3 Hz, 1H), 6.43 (s, 1H), 5.74 - 5.60 (m, 1H), 4.59 (s, 1H), 4.17 - 3.99 (m, 4H), 3.78 - 3.62 (m, 2H), 2.99 - 2.86 (m, 1H), 2.83 - 2.65 (m, 1H), 2.27 (s, 3H), 1.77 - 1.41 (m, 7H), 1.38 - 1.10 (m, 10H), 0.89 - 0.77 (m, 2H).

### Example 22. Synthesis of compound 3-((1S,2S)-1-(5-((S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-divinylphenyl)-4-methyl-4,5,6,7-tetrahydro-2H-pyrazolo[4,3-c]pyridine-5-carbonyl)-1H-indole-1-yl)-2-methylcyclopropyl)-1,2,4-oxadiazole-5(4H)-one (302)

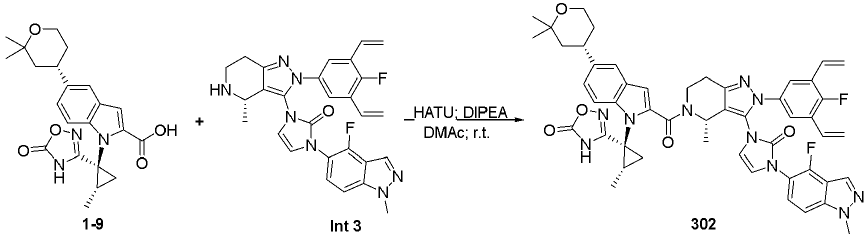

Procedures: Compound **1-9** (41 mg, 0.1 mmol) and **Int 3** (51 mg, 0.1 mmol) were dissolved in DMAc (0.5 mL), to which were added HATU (57 mg, 0.15 mmol) and N,N-diisopropylethylamine (26 mg, 0.2 mmol) respectively, and then stirred at room temperature for 5 h. The reaction solution was added with 5 mL of water for quenching, and extracted with ethyl acetate (5 mL x 3). The organic phases were combined, washed with 15 mL of saturated saline, dried over sodium sulfate, filtered, and concentrated under reduced pressure. The concentrate was purified by silica gel column chromatography to obtain the target compound (42 mg, yield 47%). Ms: 907.1 (M+H⁺)

1H NMR (400 MHz, DMSO-*d*₆) *δ* 8.31 (s, 1H), 8.14 (d, *J* = 2.0 Hz, 1H), 7.65 (d, *J* = 9.2 Hz, 1H), 7.56 (d, *J* = 6.1 Hz, 1H), 7.50 (d, *J* = 7.2 Hz, 1H), 7.41 (s, 1H), 7.20 (s, 1H), 6.99 - 6.72 (m, 4H), 5.88 (d, *J* = 17.6 Hz, 2H), 5.52 (d, *J* = 11.2 Hz, 2H), 4.12 (s, 3H), 3.74 - 3.65 (m, 2H), 3.01 (d, *J* = 12.8 Hz, 2H), 2.67 (d, *J* = 1.6 Hz, 1H), 2.00 (d, *J* = 7.6 Hz, 1H), 1.80 - 1.06 (m, 26H).

Using methods similar to those in Examples 13-22 above, the compounds listed in Table 2 could be obtained by using appropriate reactants under suitable conditions.

**Table 2. The compounds synthesized in the present invention and their characterization data**

| Compound No. | Structure | Analytical data |
|---|---|---|
| 201 | 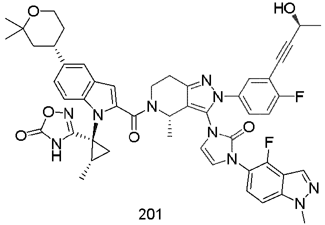 | LCMS: 923 (M+1) |
| | | ¹H NMR (400 MHz, DMSO-d6) *δ* 8.29 (s, 1H), 7.64 (d, *J* = 8.8 Hz, 1H), 7.54 (s, 1H), 7.49 (d, *J* = 7.0 Hz, 5H), 7.40 (d, *J* = 8.8 Hz, 1H), 7.26 (d, J = 8.5 Hz, 1H), 7.12 (s, 1H), 7.02 (s, 1H), 6.95 (s, 1H), 5.59 (d, *J* = 7.5 Hz, 2H), 4.66 (d, *J* = 7.1 Hz, 2H), 4.39 (d, J = 13.7 Hz, 1H), 4.11 (s, 5H), 3.71 (d, *J* = 8.3 Hz, 3H), 3.03 (s, 1H), 2.96 - 2.84 (m, 1H), 1.66 (d, *J* = 11.9 Hz, 3H), 1.47 - 1.35 (m, 4H), 1.27 (s, 4H). |
| 202 | 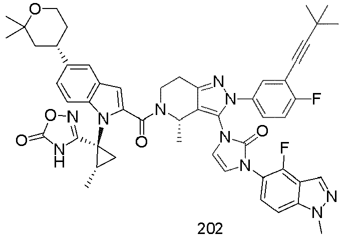 | LCMS: 935 (M+1) |
| | | ¹H NMR (400 MHz, DMSO-d6) *δ* 11.76 (s, 1H), 8.27 (s, 1H), 7.64 (d, *J* = 8.9 Hz, 1H), 7.55 (d, *J* = 9.0 Hz, 1H), 7.46 (t, *J* = 7.4 Hz, 3H), 7.40 (d, *J* = 9.0 Hz, 3H), 7.26 (d, *J* = 8.5 Hz, 2H), 7.13 (d, *J* = 3.1 Hz, 1H), 6.98 (d, *J* = 26.5 Hz, 2H), 5.59 (d, *J* = 7.1 Hz, 1H), 4.39 (d, *J* = 13.1 Hz, 1H), 4.12 (s, 5H), 3.76 - 3.59 (m, 4H), 3.11 - 2.84 (m, 3H), 1.66 (d, *J* = 11.8 Hz, 6H), 1.42 (dd, *J* = 9.8, 6.5 Hz, 3H), 1.30 (s, 10H), 1.27 (s, 8H). |
| 203 | 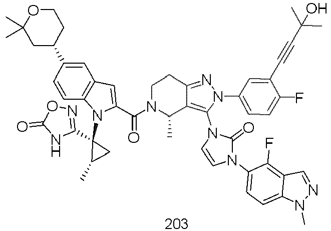 | LCMS: 937 (M+1) |
| | | ¹H NMR (400 MHz, DMSO-d6) δ 8.28 (s, 1H), 7.63 (d, *J* = 8.8 Hz, 1H), 7.55 - 7.32 (m, 4H), 7.26 (s, 1H), 7.15 - 6.88 (m, 2H), 4.11 (s, 2H), 3.71 (d, *J* = 8.5 Hz, 2H), 2.06 - 1.90 (m, 1H), 1.61 (d,*J* = 50.5 Hz, 2H), 1.47 (d, *J* = 6.9 Hz, 7H), 1.25 (d, *J* = 15.2 Hz, 12H), 1.18 (s, 6H). |
| 204 | 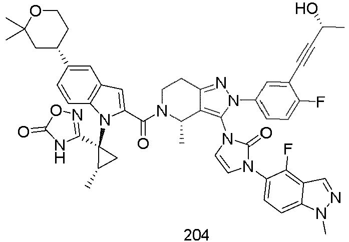 | LCMS: 923 (M+1) |
| | | ¹H NMR (400 MHz, DMSO-d6) δ 11.76 (s, 1H), 8.26 (d, *J* = 19.8 Hz, 1H), 7.74 - 7.31 (m, 4H), 7.26 (d, *J* = 8.5 Hz, 1H), 7.16 - 6.87 (m, 2H), 4.72 - 4.57 (m, 1H), 4.10 (d, *J* = 9.7 Hz, 3H), 3.71 (d, *J* = 8.5 Hz, 2H), 3.09 - 2.84 (m, 1H), 2.00 (d, *J* = 8.9 Hz, 1H), 1.75 - 1.35 (m, 10H), 1.25 (d, *J* = 15.3 Hz, 6H), 1.17 (d, *J* = 5.9 Hz, 5H). |
| 205 | 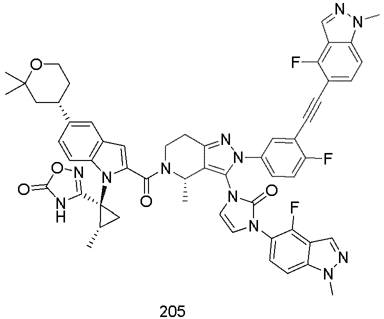 | LCMS: 1027 (M+1) |
| | | ¹H NMR (400 MHz, DMSO-d6) δ 11.75 (s, 1H), 8.26 (d, *J* = 6.9 Hz, 1H), 8.17 (s, 1H), 7.56 (tdt, *J* = 23.7, 15.8, 7.3 Hz, 9H), 7.40 (d, *J* = 8.6 Hz, 1H), 7.26 (d, *J* = 8.5 Hz, 1H), 7.14 (d, *J* = 3.1 Hz, 1H), 7.00 (d, *J=* 31.3 Hz, 2H), 5.62 (d, *J* = 6.9 Hz, 1H), 5.28 (d, *J* = 21.0 Hz, 0H), 4.41 (d, *J* = 13.3 Hz, 1H), 4.09 (d, *J* = 13.4 Hz, 6H), 3.75 - 3.60 (m, 3H), 1.63 (td, *J* = 37.9, 37.0, 8.8 Hz, 9H), 1.45 (d, *J* = 6.6 Hz, 2H), 1.26 (d, *J* = 4.2 Hz, 4H), 1.17 (d, *J* = 3.3 Hz, 6H). |
| 206 | 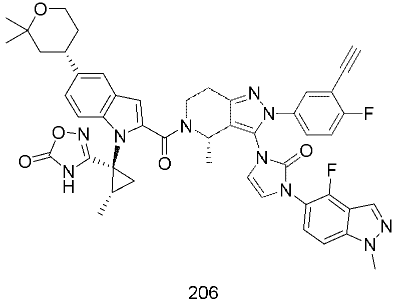 | LCMS: 879 (M+1) |
| | | ¹H NMR (400 MHz, DMSO-d6) δ 8.30 (s, 1H), 7.64 (d, J = 8.9 Hz, 1H), 7.58 - 7.44 (m, 4H), 7.40 (d, J = 8.6 Hz, 1H), 7.26 (d, J = 8.6 Hz, 2H), 7.13 (s, 1H), 7.04 (s, 1H), 6.95 (s, 1H), 5.59 (d, J = 7.0 Hz, 1H), 4.66 (d, J = 11.9 Hz, 2H), 4.39 (d, J = 14.0 Hz, 1H), 4.10 (d, J = 11.6 Hz, 5H), 3.71 (d, J = 8.6 Hz, 3H), 3.08 (s, 1H), 1.76 - 1.48 (m, 9H), 1.43 (d, J = 6.1 Hz, 3H), 1.27 (d, J = 5.9 Hz, 5H), 1.23 (s, 4H), 1.17 (d, J = 5.8 Hz, 8H), 1.11 (s, 2H). |
| 207 | 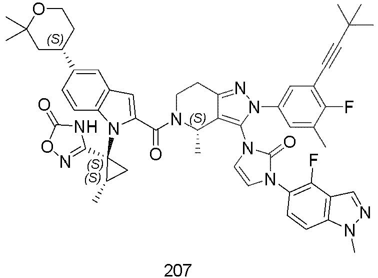 | LCMS: 949 (M+1) |
| | | ¹H NMR (400 MHz, DMSO-d6) δ 11.75 (s, 1H), 8.26 (s, 1H), 7.64 (d, J = 8.8 Hz, 1H), 7.53 (s, 1H), 7.47 (t, J = 8.0 Hz, 1H), 7.38 (dd, J = 16.7, 6.4 Hz, 3H), 7.26 (d, J = 8.5 Hz, 1H), 7.21 (s, 1H), 7.13 (d, J = 3.1 Hz, 1H), 6.99 (s, 1H), 6.95 (s, 1H), 5.58 (d, J = 6.9 Hz, 1H), 4.39 (d, J = 13.6 Hz, 1H), 4.11 (s, 4H), 3.77 - 3.55 (m, 3H), 3.25 - 3.11 (m, 1H), 3.03 (t, J = 12.4 Hz, 1H), 2.90 (dd, J = 19.1, 9.0 Hz, 1H), 2.26 (d, J = 12.6 Hz, 4H), 1.83 - 1.47 (m, 9H), 1.43 (d, J = 6.7 Hz, 3H), 1.28 (d, J = 9.4 Hz, 7H), 1.17 (d, J = 7.0 Hz, 6H). |
| 208 | 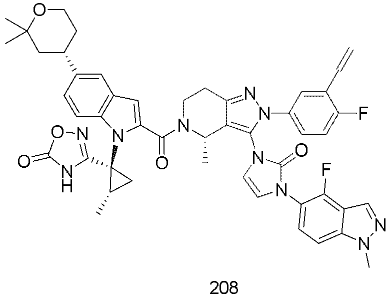 | LCMS: 881 (M+1) |
| | | ¹H NMR (400 MHz, DMSO-d6) δ 8.29 (s, 1H), 7.71 - 7.66 (m, 1H), 7.63 (d, J = 8.8 Hz, 1H), 7.54 (s, 1H), 7.42 (dd, J = 12.7, 8.5 Hz, 5H), 7.26 (d, J = 8.6 Hz, 1H), 7.12 (s, 1H), 7.05 (s, 1H), 6.97 - 6.82 (m, 2H), 5.88 (d, J = 17.6 Hz, 1H), 5.59 (d, J = 6.6 Hz, 1H), 5.51 (d, J = 11.2 Hz, 1H), 4.39 (d, J = 13.9 Hz, 1H), 4.09 (d, J = 11.4 Hz, 5H), 3.71 (d, J = 8.5 Hz, 4H), 3.20 (s, 0H), 3.06 (d, J = 16.9 Hz, 1H), 2.94 (s, 2H), 1.75 - 1.50 (m, 7H), 1.44 (d, J = 6.7 Hz, 3H), 1.27 (d, J = 6.1 Hz, 5H), 1.23 (s, 2H), 1.18 (d, J = 5.5 Hz, 7H). |
| 209 | 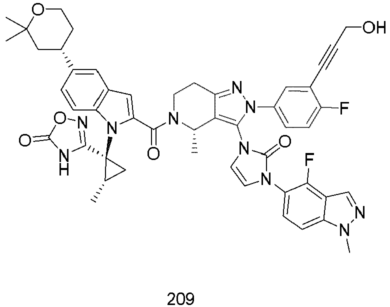 | LCMS: 909 (M+1) |
| | | ¹H NMR (400 MHz, DMSO-d6) δ 11.74 (s, 1H), 8.30 (s, 1H), 7.64 (d, J = 8.8 Hz, 1H), 7.58 - 7.44 (m, 4H), 7.40 (d, J = 8.6 Hz, 1H), 7.26 (d, J = 8.4 Hz, 1H), 7.14 - 6.89 (m, 2H), 5.59 (d, J = 7.4 Hz, 1H), 5.49 (t, J = 6.0 Hz, 1H), 4.37 (d, J = 6.1 Hz, 2H), 4.11 (s, 2H), 3.71 (d, J = 8.6 Hz, 2H), 3.10 - 2.86 (m, 1H), 2.03 - 1.94 (m, 0H), 1.58 (dt, J = 50.2, 12.1 Hz, 4H), 1.43 (d, J = 6.5 Hz, 2H), 1.25 (d, J = 15.4 Hz, 6H), 1.18 (s, 4H). |
| 210 | 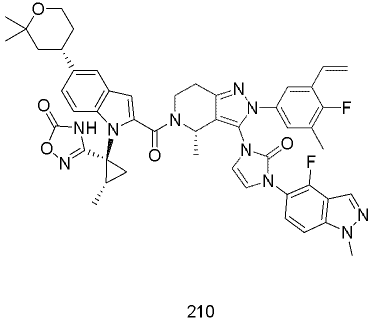 | LCMS: 895 (M+1) |
| | | ¹H NMR (400 MHz, DMSO-d6) δ 14.13 (s, 1H), 8.30 (s, 1H), 7.70 (s, 1H), 7.63 (d, J = 8.9 Hz, 1H), 7.54 (s, 1H), 7.49 (dd, J = 6.3, 2.6 Hz, 1H), 7.47 - 7.41 (m, 1H), 7.39 (d, J = 8.6 Hz, 1H), 7.34 (dd, J = 6.3, 2.7 Hz, 1H), 7.26 (dd, J = 8.5, 1.7 Hz, 1H), 7.08 (dd, J = 33.2, 3.6 Hz, 2H), 6.92 - 6.82 (m, 1H), 5.85 (d, J = 17.6 Hz, 1H), 5.76 (s, 1H), 5.56 (dd, J = 59.0, 9.0 Hz, 2H), 4.47 (d, J = 13.0 Hz, 1H), 4.11 (s, 3H), 3.73 - 3.68 (m, 3H), 3.25 (dd, J = 28.7, 5.3 Hz, 2H), 3.09 - 2.88 (m, 3H), 2.30 (d, J = 2.1 Hz, 3H), 2.05 - 1.93 (m, 2H), 1.84 (h, J = 6.5 Hz, 1H), 1.64 (t, J = 8.0 Hz, 3H), 1.58 - 1.41 (m, 5H), 1.15(s, 6H). |
| 211 | 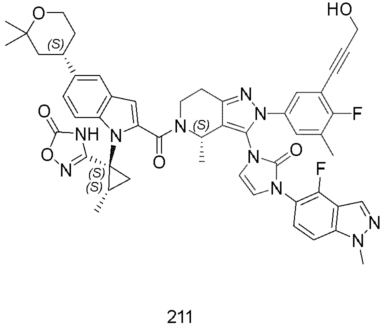 | LCMS: 923 (M+1) |
| 212 | 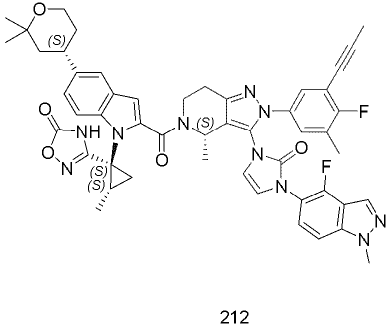 | LCMS: 907 (M+1) |
| | | ¹H NMR (400 MHz, DMSO-d6) δ 11.75 (s, 1H), 8.26 (s, 1H), 7.64 (d, J = 8.8 Hz, 1H), 7.53 (s, 1H), 7.47 (t, J = 8.0 Hz, 1H), 7.38 (dd, J = 16.7, 6.4 Hz, 3H), 7.26 (d, J = 8.5 Hz, 1H), 7.21 (s, 1H), 7.13 (d, J = 3.1 Hz, 1H), 6.99 (s, 1H), 6.95 (s, 1H), 5.58 (d, J = 6.9 Hz, 1H), 4.39 (d, J = 13.6 Hz, 1H), 4.11 (s, 4H), 3.77 - 3.55 (m, 3H), 3.25 - 3.11 (m, 1H), 3.03 (t, J = 12.4 Hz, 1H), 2.90 (dd, J = 19.1, 9.0 Hz, 1H), 2.26 (d, J = 12.6 Hz, 4H), 1.83 - 1.47 (m, 3H), 1.43 (d, J = 6.7 Hz, 3H), 1.28 (d, J = 9.4 Hz, 7H), 1.17 (d, J = 7.0 Hz, 6H). |
| 213 | 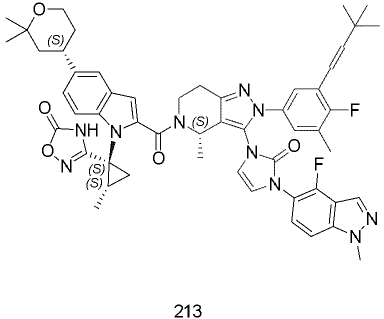 | LCMS: 951 (M+1) |
| | | ¹H NMR (400 MHz, DMSO-d6) δ 11.75 (s, 1H), 8.26 (s, 1H), 7.64 (d, J = 8.8 Hz, 1H), 7.53 (s, 1H), 7.47 (t, J = 8.0 Hz, 1H), 7.38 (dd, J = 16.7, 6.4 Hz, 3H), 7.26 (d, J = 8.5 Hz, 1H), 7.21 (s, 1H), 7.13 (d, J = 3.1 Hz, 1H), 6.99 (s, 1H), 6.95 (s, 1H), 6.16 (d, J = 12.5 Hz, 1H), 5.76 (d, J = 12.5 Hz, 1H), 5.50 (d, J = 6.9 Hz, 1H), 4.39 (d, J = 13.6 Hz, 1H), 4.11 (s, 4H), 3.77 - 3.55 (m, 3H), 3.25 - 3.11 (m, 1H), 3.03 (t, J = 12.4 Hz, 1H), 2.90 (dd, J = 19.1, 9.0 Hz, 1H), 2.26 (d, J = 12.6 Hz, 4H), 1.83 - 1.47 (m, 9H), 1.43 (d, J = 6.7 Hz, 3H), 1.28 (d, J = 9.4 Hz, 7H), 1.17 (d, J = 7.0 Hz, 6H). |
| 214 | 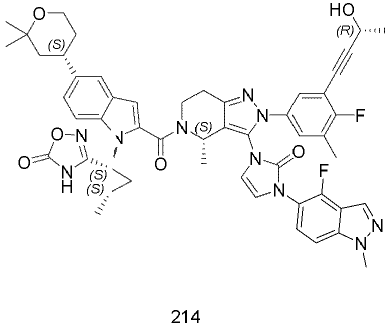 | LCMS: 937 (M+1) |
| | | ¹H NMR (400 MHz, DMSO-d6) δ 11.74 (s, 1H), 8.29 (s, 1H), 7.64 (d, J = 8.8 Hz, 1H), 7.53 (s, 1H), 7.51 - 7.46 (m, 1H), 7.44 - 7.33 (m, 2H), 7.32 - 7.21 (m, 2H), 7.12 (d, J = 3.2 Hz, 1H), 7.04 - 6.92 (m, 2H), 5.66 - 5.52 (m, 2H), 4.71 - 4.57 (m, 1H), 4.47 - 4.33 (m, 1H), 4.17 - 4.03 (m, 4H), 3.74 - 3.66 (m, 3H), 3.10 - 2.97 (m, 1H), 2.96 - 2.83 (m, 1H), 2.37 - 2.20 (m, 4H), 1.76 - 1.48 (m, 5H), 1.46 - 1.35 (m, 5H), 1.34 - 1.21 (m, 5H), 1.20 - 1.14 (m, 6H). |
| 215 | 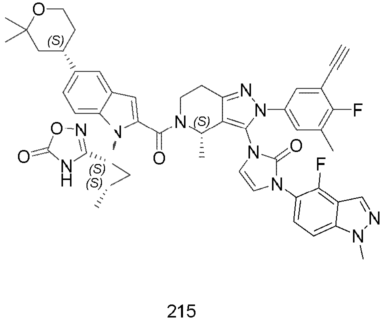 | LCMS: 893 (M+1) |
| | | ¹H NMR (400 MHz, DMSO-d6) δ 11.74 (s, 1H), 8.29 (d, J = 5.3 Hz, 1H), 7.64 (d, J = 8.8 Hz, 1H), 7.53 (s, 1H), 7.50 - 7.33 (m, 3H), 7.31 - 7.21 (m, 2H), 7.12 (d, J = 3.1 Hz, 1H), 7.02 (s, 1H), 6.95 (s, 1H), 5.74 - 5.60 (m, 1H), 4.59 (s, 1H), 4.17 - 3.99 (m, 4H), 3.78 - 3.62 (m, 2H), 3.10 - 2.96 (m, 1H), 2.96 - 2.85 (m, 1H), 2.34 - 2.22 (m, 4H), 1.84 - 1.37 (m, 7H), 1.36 - 1.21 (m, 7H), 1.17 (d, J = 6.0 Hz, 6H). |
| 216 | 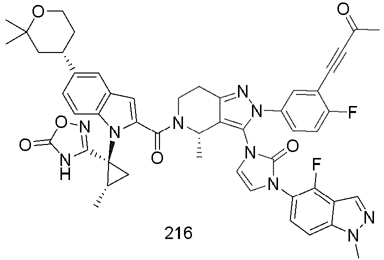 | LCMS: 921 (M+1) |
| | | ¹H NMR (400 MHz, DMSO-d6) δ 11.74 (s, 1H), 8.27 (s, 1H), 7.67 (dd, J = 27.9, 8.6 Hz, 3H), 7.56 - 7.45 (m, 1H), 7.40 (d, J = 8.5 Hz, 1H), 7.26 (d, J = 8.5 Hz, 1H), 7.16 - 6.89 (m, 2H), 5.60 (s, 1H), 4.40 (d, J = 13.5 Hz, 1H), 4.11 (s, 2H), 3.71 (d, J = 8.6 Hz, 3H), 3.12 - 2.84 (m, 1H), 2.48 (s, 2H), 1.76 - 1.50 (m, 4H), 1.43 (d, J = 6.5 Hz, 2H), 1.25 (d, J = 15.5 Hz, 7H), 1.17 (d, J = 5.9 Hz, 5H). |
| 217 | 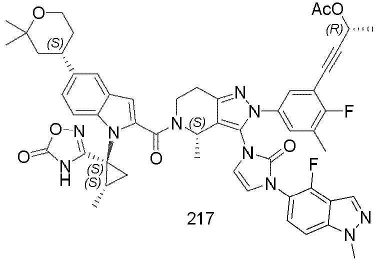 | LCMS: 979 (M+1) |
| | | ¹H NMR (400 MHz, DMSO-d6) δ 11.74 (s, 1H), 8.29 (s, 1H), 7.64 (d, J = 8.9 Hz, 1H), 7.53 (s, 1H), 7.50 - 7.37 (m, 3H), 7.32 - 7.23 (m, 2H), 7.12 (d, J = 3.1 Hz, 1H), 7.00 (s, 1H), 6.95 (s, 1H), 5.68 - 5.55 (m, 2H), 4.44 - 4.34 (m, 1H), 4.15 - 4.03 (m, 4H), 3.75 - 3.66 (m, 2H), 3.08 - 2.97 (m, 1H), 2.95 - 2.85 (m, 1H), 2.34 - 2.22 (m, 4H), 2.04 (s, 3H), 1.75 - 1.37 (m, 9H), 1.36 - 1.11 (m, 13H). |
| 218 | 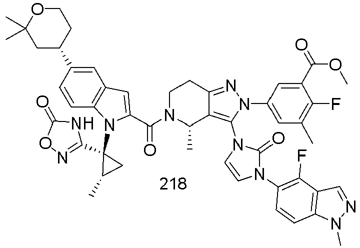 | LCMS: 927 (M+1) |
| 219 | 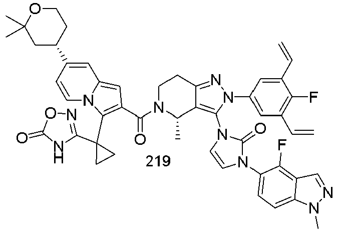 | LCMS: 893 (M+1) |
| 220 | 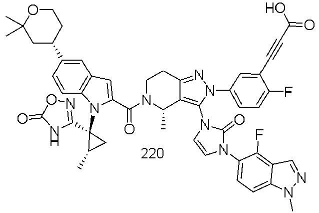 | LCMS: 923 (M+1) |
| 221 | 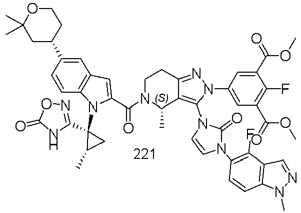 | LCMS: 971 (M+1) |
| 222 | 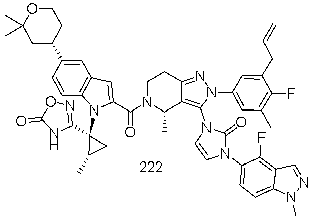 | LCMS: 909 (M+1) |
| 223 | 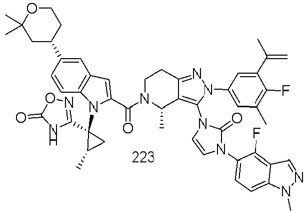 | LCMS: 909 (M+1) |
| 224 | 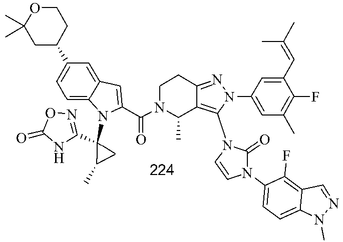 | LCMS: 923 (M+1) |
| 225 | 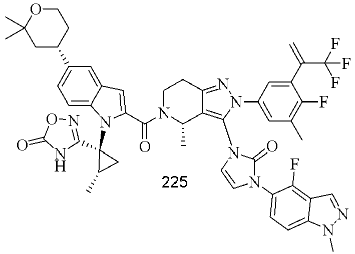 | LCMS: 963 (M+1) |
| 226 | 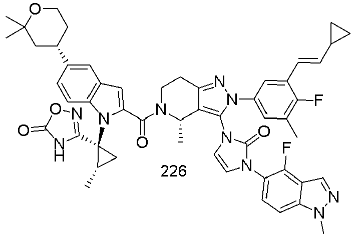 | LCMS: 935 (M+1) |
| 227 | 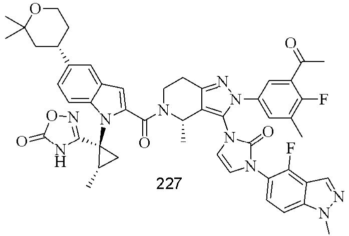 | LCMS: 911 (M+1) |
| 228 | 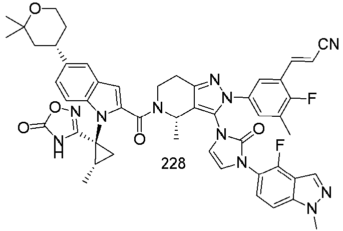 | LCMS: 920 (M+1) |
| 229 | 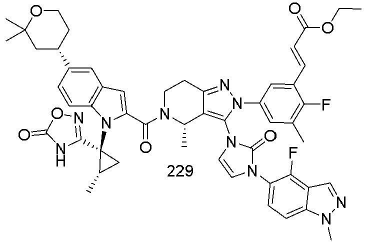 | LCMS: 967 (M+1) |
| 230 | 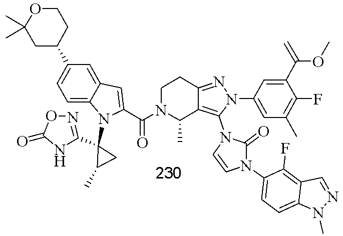 | LCMS: 925 (M+1) |
| 232 | 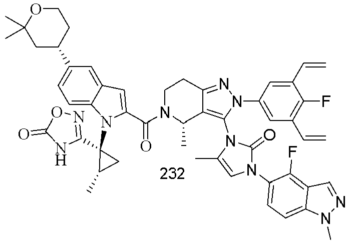 | LCMS: 921 (M+1) |
| 301 | 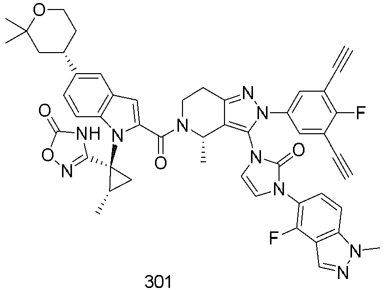 | LCMS: 903 (M+1) |
| 302 | 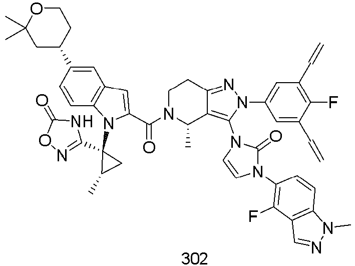 | LCMS: 907.1 (M+1) |
| | | ¹H NMR (400 MHz, DMSO-d6) δ 8.31 (s, 1H), 8.14 (d, J = 2.0 Hz, 1H), 7.65 (d, J = 9.2 Hz, 1H), 7.56 (d, J = 6.1 Hz, 1H), 7.50 (d, J = 7.2 Hz, 1H), 7.41 (s, 1H), 7.20 (s, 1H), 6.99 - 6.72 (m, 4H), 5.88 (d, J = 17.6 Hz, 2H), 5.52 (d, J = 11.2 Hz, 2H), 4.12 (s, 3H), 3.74 - 3.65 (m, 2H), 3.01 (d, J = 12.8 Hz, 2H), 2.67 (d, J = 1.6 Hz, 1H), 2.00 (d, J = 7.6 Hz, 1H), 1.80 - 1.06 (m, 26H). |
| 303 | 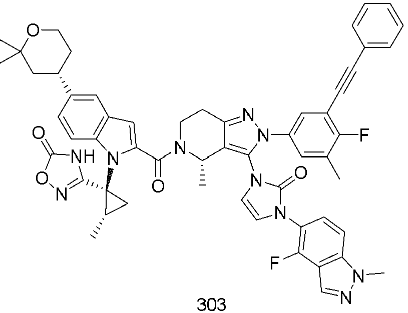 | LCMS: 969 (M+1) |
| 304 | 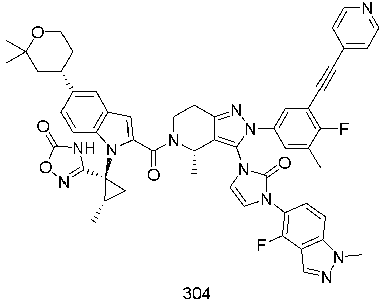 | LCMS: 970 (M+1) |
| 305 | 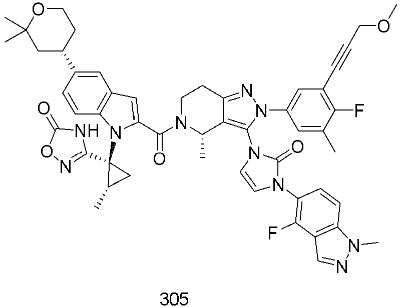 | LCMS: 937 (M+1) |
| 306 | 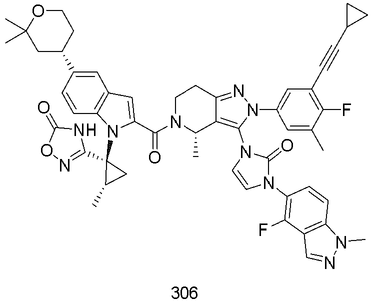 | LCMS: 933 (M+1) |
| 307 | 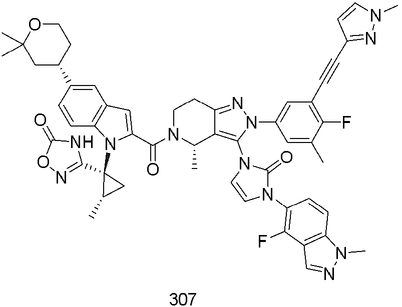 | LCMS: 973 (M+1) |
| 308 | 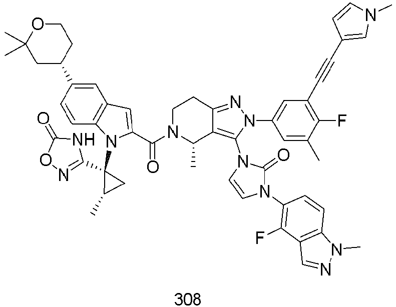 | LCMS: 972 (M+1) |
| 309 | 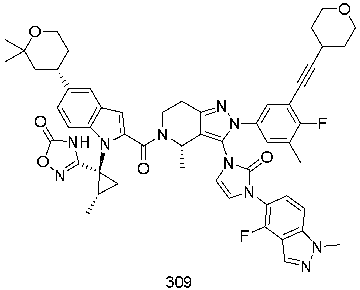 | LCMS: 977 (M+1) |
| 310 | 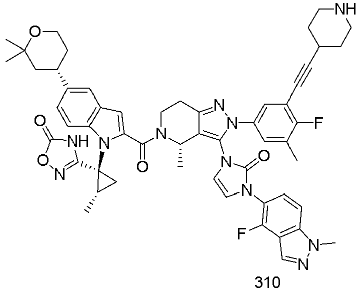 | LCMS: 976 (M+1) |
| 311 | 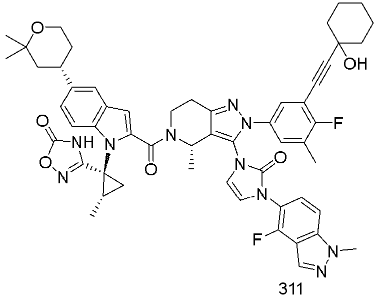 | LCMS: 991 (M+1) |
| 312 | 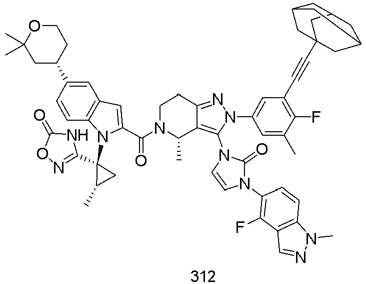 | LCMS: 1027 (M+1) |
| 313 | 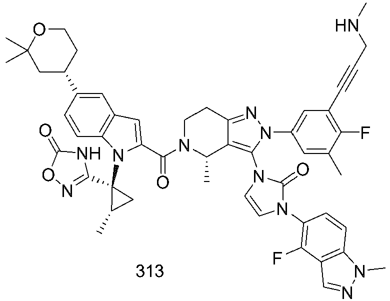 | LCMS: 936 (M+1) |
| | | ¹H NMR (400 MHz, DMSO-d6) δ 11.74 (s, 1H), 8.31 (s, 1H), 7.64 (d, J = 8.8 Hz, 1H), 7.50 (dd, J = 17.6, 9.6 Hz, 3H), 7.40 (d, J = 8.4 Hz, 1H), 7.26 (d, J = 8.4 Hz, 1H), 7.18 (d, J = 6.4 Hz, 2H), 7.09 (d, J = 3.2 Hz, 1H), 6.95 (s, 2H), 4.11 (s, 3H), 3.71 (d, J = 8.4 Hz, 2H), 3.17 (d, J = 12.4 Hz, 1H), 3.06 - 2.99 (m, 3H), 2.92 - 2.86 (m, 1H), 2.26 (s, 6H), 1.99 (s, 1H), 1.54 (dd, J = 89.6, 9.3 Hz, 10H), 1.30 - 1.14 (m, 11H). |
| 314 | 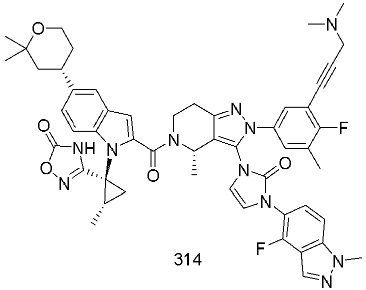 | LCMS: 950 (M+1) |
| 315 | 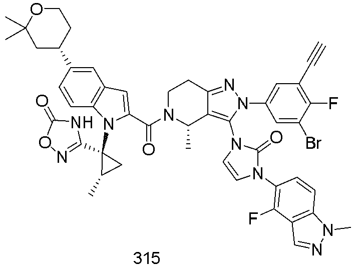 | LCMS: 957 (M+1) |
| 316 | 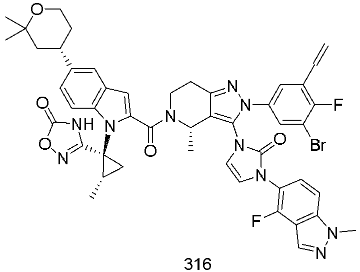 | LCMS: 959 (M+1) |
| 317 | 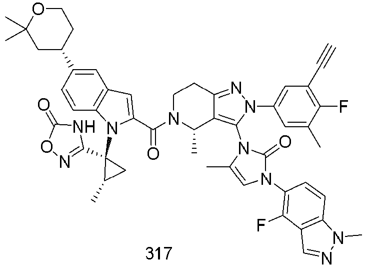 | LCMS: 907 (M+1) |
| 318 | 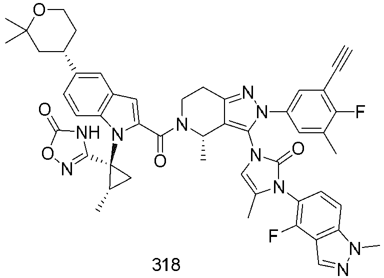 | LCMS: 907 (M+1) |
| 319 | 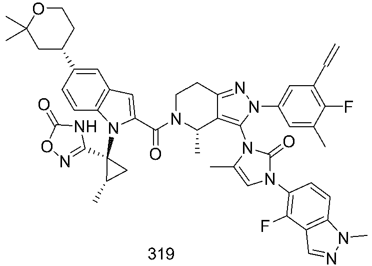 | LCMS: 909 (M+1) |
| 320 | 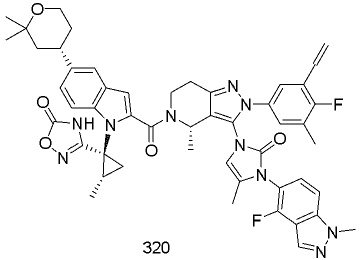 | LCMS: 909 (M+1) |
| 321 | 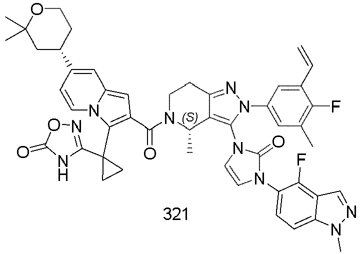 | LCMS: 881 (M+1) |
| 322 | 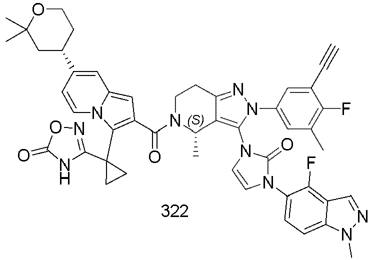 | LCMS: 879 (M+1) |
| | | ¹H NMR (400 MHz, DMSO-d6) δ 12.03 (s, 1H), 8.30 (s, 1H), 8.16 (d, J = 7.2 Hz, 1H), 7.64 (d, J = 8.7 Hz, 1H), 7.50 - 7.40 (m, 1H), 7.38 - 7.25 (m, 4H), 7.15 - 6.99 (m, 1H), 6.72 (d, J = 7.3 Hz, 1H), 6.43 (s, 1H), 5.74 - 5.60 (m, 1H), 4.59 (s, 1H), 4.17 - 3.99 (m, 4H), 3.78 - 3.62 (m, 2H), 2.99 - 2.86 (m, 1H), 2.83 - 2.65 (m, 1H), 2.27 (s, 3H), 1.77 - 1.41 (m, 7H), 1.38 - 1.10 (m, 10H), 0.89 - 0.77 (m, 2H). |
| 323 | 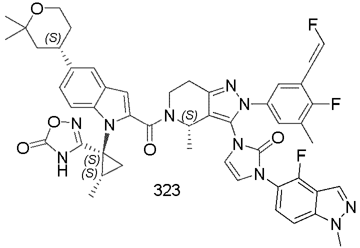 | LCMS: 913 (M+1) |
| 324 | 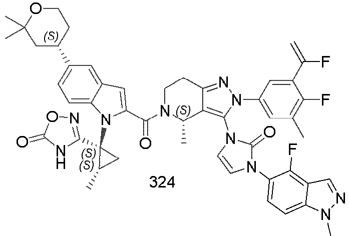 | LCMS: 913 (M+1) |

### Example 23. Synthesis of compound 3-((2S)-1-(5-((S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-5-oxo-4,5-dihydro-1,2,4-triazine-6-nitrile (compound 419)

**ethyl 3-[(2S)-1-(5-((S)-2,2-dimethyltetrahydro-2H-pyran-4-yl)-2-((S)-3-(3-(4-fluoro-1-methyl-1H-indole-5-yl)-2-oxo-2,3-dihydro-1H-imidazoline-1-yl)-2-(4-fluoro-3,5-dimethylphenyl)-4-methyl-4,5,6,7-tetrahydro-2H-pyrazolo[4,3-c]pyridine-5-carboxyl)-1H-indole-1-yl)-2-methylcyclopropyl]-5-oxo-4,5-dihydro-1,2,4-triazine-6-carboxylate (compound 430)**
**compound 3-((2S)-1-(5-((S)-2,2-dimethyltetrahydro-2H-pyran-4-yl)-2-((S)-3-(3-(4-fluoro-1-methyl-1H-indole-5-yl)-2-oxo-2,3-dihydro-1H-imidazoline-1-yl)-2-(4-fluoro-3,5-dimethylphenyl)-4-methyl-4,5,6,7-tetrahydro-2H-pyrazolo[4,3-c]pyridine-5-acyl)-1H-indole-1-yl)-2-methylcyclopropyl)-5-oxo-4,5-dihydro-1,2,4-triazine-6-carboxylic acid (compound 429)**
**compound 3-((2S)-1-(5-((S)-2,2-dimethyltetrahydro-2H-pyran-4-yl)-2-((S)-3-(3-(4-fluoro-1-methyl-1H-indole-5-yl)-2-oxo-2,3-dihydro-1H-imidazoline-1-yl)-2-(4-fluoro-3,5-dimethylphenyl)-4-methyl-4,5,6,7-tetrahydro-2H-pyrazolo[4,3-c]pyridine-5-acyl)-1H-indole-1-yl)-2-methylcyclopropyl)-5-oxo-4,5-dihydro-1,2,4-triazine-6-formamide (compound 432)**

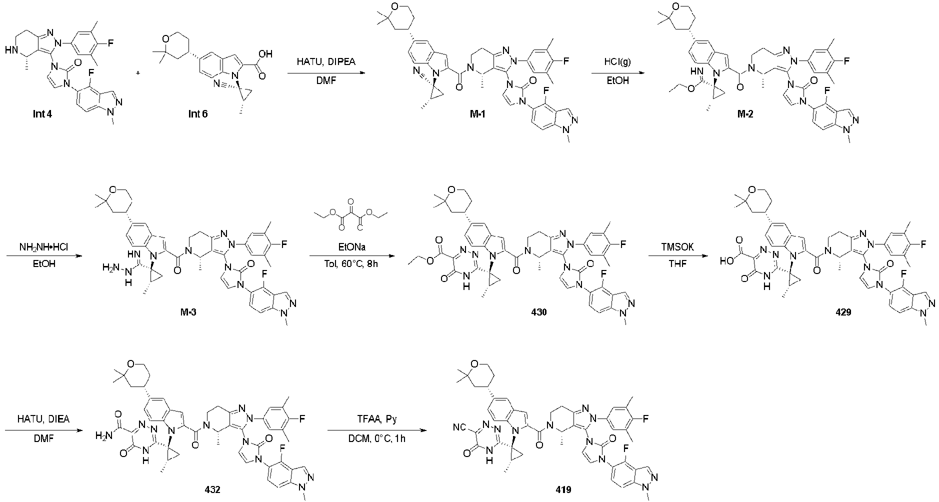

### Step 1: Synthesis of (2S)-1-(5-((S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropane-1-nitrile (intermediate M-1)

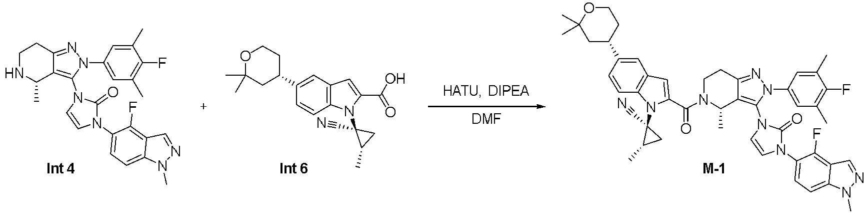

At room temperature, **Int 6** (1.6 g, 4.55 mmol) was dissolved in DMF (20 mL), to which were added HATU (2.59 g, 6.82 mmol) and DIPEA(1.76 g, 13.65 mmol). The mixture was stirred and reacted for 20 min, and then **Int 4** (2.23 g, 4.55 mmol) was added. The reaction was stirred and reacted at room temperature for 2 h. TLC monitoring indicated the completion of the raw material reaction. The reaction solution was diluted with 50 mL of water, and then extracted with ethyl acetate (50 mL × 3). The organic phase was washed with 50 mL of saturated saline, dried over sodium sulfate, and filtered. The filtrate was concentrated under reduced pressure, and the concentrate was purified by silica gel column chromatography to obtain light yellow solid product, intermediate **M-1** (3.0 g, 3.65 mmol), with a yield of 80.2%. M/S: 824 (M+H⁺).

### Step 2: Synthesis of intermediate (2S)-1-(5-((S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropane-1-carbimide ester (intermediate M-2)

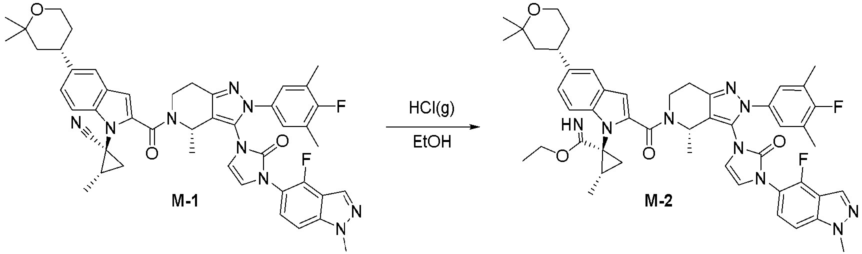

Intermediate **M-1** (3.0 g, 3.65 mmol) was dissolved in anhydrous ethanol (30 mL), to which was introduced hydrochloric acid gas at 10 °C, and then stirred and reacted for 6 h. TLC monitoring indicated completion of the raw material reaction. The reaction solution was adjusted to pH ≈ 7 with NaHCO₃ aqueous solution, and then extracted with ethyl acetate (50 mL × 3). The organic phases were combined, dried over anhydrous sodium sulfate, filtered, and concentrated under reduced pressure, to obtain crude intermediate **M-2** (2.5 g, 2.88 mmol), with a yield of 78.9%. M/S: 870 (M+H⁺).

### Step 3: Synthesis of intermediate (2S)-1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropane-1-carboxamidoxime (intermediate M-3)

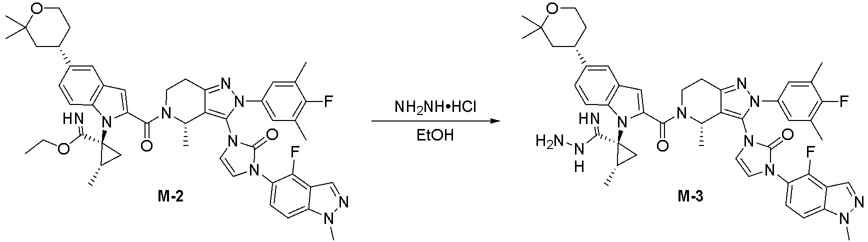

Intermediate **M-2** (2.5 g, 2.88 mmol) was dissolved in ethanol (30 mL), to which was added hydrazine hydrochloride (1.8 g, 28.8 mmol) and NaHCO₃ saturated solution (2 mL) in an ice bath, and then stirred and reacted at room temperature for 2 h. TLC monitoring indicated the reaction of the raw materials was complete. The reaction solution was diluted with 50 mL of water, and extracted with ethyl acetate (50 mL × 3). The organic phases were combined, dried over anhydrous sodium sulfate, filtered, and concentrated under reduced pressure. The concentrate was purified by silica gel column chromatography to obtain light yellow solid product, intermediate **M-3** (1.5 g, 1.75 mmol), with a yield of 60.9%. M/S: 856 (M+H⁺).

### Step 4: Synthesis of compound ethyl 3-[(2S)-1-(5-((S)-2,2-dimethyltetrahydro-2H-pyran-4-yl)-2-((S)-3-(3-(4-fluoro-1-methyl-1H-indole-5-yl)-2-oxo-2,3-dihydro-1H-imidazoline-1-yl)-2-(4-fluoro-3,5-dimethylphenyl)-4-methyl-4,5,6,7-tetrahydro-2H-pyrazolo[4,3-c]pyridine-5-carboxyl)-1H-indole-1-yl)-2-methylcyclopropyl]-5-oxo-4,5-dihydro-1,2,4-triazine-6-carboxylate (compound 430)

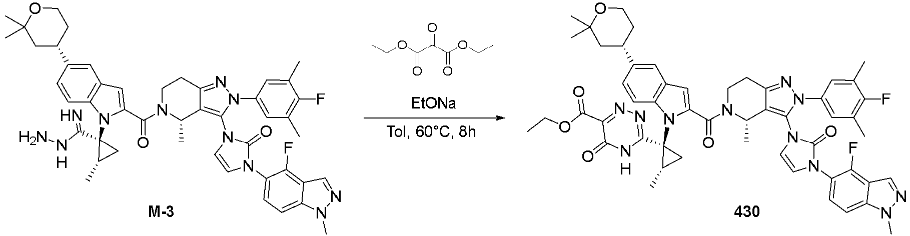

Intermediate **M-3** (700 mg, 0.82 mmol) was dissolved in toluene (10 mL), to which were added sodium ethoxide (111 mg, 1.63 mmol) and diethyl ketomalonate (1.1 g, 0.21 mmol), and then stirred and reacted at 60 °C for 8 h. TLC monitoring indicated the reaction of raw materials was complete. The reaction solution was cooled to room temperature, diluted with 50 mL of water, and extracted with ethyl acetate (50 mL×3). The organic phases were combined, dried over anhydrous sodium sulfate, filtered, and concentrated under reduced pressure. The concentrate was purified by silica gel column chromatography to obtain light yellow solid product, compound **430** (500 mg, 0.52 mmol), with a yield of 63%. M/S: 966 (M+H⁺).

¹HNMR (400 MHz, DMSO-*d*₆) *δ* 8.31 (s, 1H), 7.63 (d, *J* = 8.8 Hz, 1H), 7.54 (s, 1H), 7.48 (d, *J* = 7.6 Hz, 1H), 7.36 (d, *J* = 8.4 Hz, 1H), 7.25 (d, *J* = 8.8 Hz, 1H), 7.18 (d, *J* = 6.6 Hz, 2H), 7.08 (d, *J* = 3.2 Hz, 1H), 7.03 (s, 1H), 6.95 (s, 2H), 4.28 (d, *J* = 7.2 Hz, 2H), 4.11 (s, 3H), 3.70 (d, *J* = 8.8 Hz, 2H), 3.17 (s, 1H), 3.03 (s, 1H), 2.92 (d, *J* = 16.4 Hz, 1H), 2.26 (d, *J* = 2.2 Hz, 6H), 2.02 (s, 1H), 1.69 - 1.38 (m, 10H), 1.31 - 1.21 (m, 9H), 1.19 (m, 5H).

### Step 5: Synthesis of compound 3-((2S)-1-(5-((S)-2,2-dimethyltetrahydro-2H-pyran-4-yl)-2-((S)-3-(3-(4-fluoro-1-methyl-1H-indole-5-yl)-2-oxo-2,3-dihydro-1H-imidazoline-1-yl)-2-(4-fluoro-3,5-dimethylphenyl)-4-methyl-4,5,6,7-tetrahydro-2H-pyrazolo[4,3-c]pyridine-5-acyl)-1H-indole-1-yl)-2-methylcyclopropyl)-5-oxo-4,5-dihydro-1,2,4-triazine-6-carboxylic acid (compound 429)

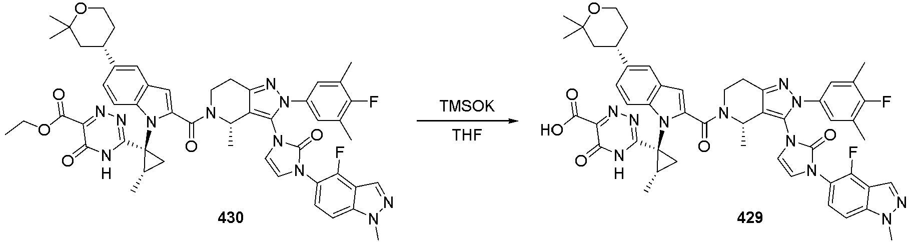

Compound **430** (300 mg, 0.31 mmol) was dissolved in tetrahydrofuran (6 mL) at room temperature, followed by adding potassium trimethylsilanolate (199 mg, 1.55 mmol). The mixture was stirred at room temperature for 1 h, and TLC monitoring indicated the reaction of the starting material was complete. The reaction solution was adjusted to pH ≈ 3 with hydrochloric acid (2N) solution, and then extracted with ethyl acetate (50 mL × 3). The organic phases were combined, dried over anhydrous sodium sulfate, filtered, and concentrated under reduced pressure. The concentrate was purified by silica gel column chromatography to obtain a light yellow solid product, compound **429** (250 mg, 0.27 mmol), with a yield of 87%. M/S: 938 (M+H⁺).

¹HNMR (400 MHz, DMSO-*d*₆) *δ* 8.31 (s, 1H), 7.63 (d, *J* = 8.8 Hz, 1H), 7.54 (s, 1H), 7.48 (d, *J* = 7.6 Hz, 2H), 7.36 (d, *J* = 8.4 Hz, 1H), 7.25 (d, *J* = 8.8 Hz, 1H), 7.18 (d, *J* = 6.6 Hz, 2H), 7.08 (d, *J* = 3.2 Hz, 1H), 7.03 (s, 1H), 6.95 (s, 2H), 4.11 (s, 3H), 3.70 (d, *J* = 8.8 Hz, 2H), 3.17 (s, 1H), 3.03 (s, 1H), 2.92 (d, *J* = 16.4 Hz, 1H), 2.26 (d, *J* = 2.2 Hz, 6H), 2.02 (s, 1H), 1.69 - 1.38 (m, 7H), 1.31 - 1.21 (m, 9H), 1.19 (m, 5H).

### Step 6: Synthesis of compound 3-((2S)-1-(5-((S)-2,2-dimethyltetrahydro-2H-pyran-4-yl)-2-((S)-3-(3-(4-fluoro-1-methyl-1H-indole-5-yl)-2-oxo-2,3-dihydro-1H-imidazoline-1-yl)-2-(4-fluoro-3,5-dimethylphenyl)-4-methyl-4,5,6,7-tetrahydro-2H-pyrazolo[4,3-c]pyridine-5-acyl)-1H-indole-1-yl)-2-methylcyclopropyl)-5-oxo-4,5-dihydro-1,2,4-triazine-6-formamide (compound 432)

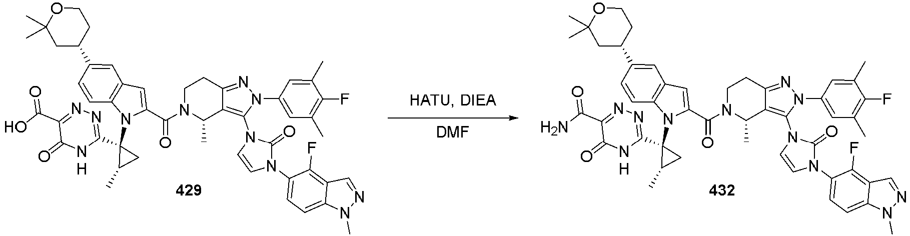

At room temperature, compound **429** (200 mg, 0.21 mmol) was dissolved in DMF (4 mL), to which were added HATU (200 mg, 0.32 mmol) and DIEA (82 mg, 0.63 mmol), and then stirred for 20 min, followed by adding ammonium chloride (34 mg, 0.63 mmol). The mixture was stirred and reacted at room temperature for 2 h. The completion of the raw material reaction was monitored by TLC. The reaction solution was diluted with 20 mL of water, and extracted with ethyl acetate (30 mL × 3). The organic phase was washed with 50 mL of saturated saline, dried over sodium sulfate, and filtered. The filtrate was concentrated under reduced pressure, and the concentrate was purified by silica gel column chromatography to obtain light yellow solid product, compound **432** (160 mg, 0.17 mmol), with a yield of 80.9%. M/S: 937 (M+H⁺).

¹HNMR (400 MHz, DMSO-*d*₆) *δ* 8.31 (s, 1H), 7.63 (d, *J* = 8.8 Hz, 1H), 7.54 (s, 1H), 7.48 (d, *J* = 7.6 Hz, 2H), 7.36 (d, *J* = 8.4 Hz, 1H), 7.25 (d, *J* = 8.8 Hz, 2H), 7.18 (d, *J* = 6.6 Hz, 2H), 7.08 (d, *J* = 3.2 Hz, 1H), 7.03 (s, 1H), 6.95 (s, 2H), 4.11 (s, 3H), 3.70 (d, *J* = 8.8 Hz, 2H), 3.17 (s, 1H), 3.03 (s, 1H), 2.92 (d, *J* = 16.4 Hz, 1H), 2.26 (d, *J* = 2.2 Hz, 6H), 2.02 (s, 1H), 1.69 - 1.38 (m, 7H), 1.31 - 1.21 (m, 9H), 1.19 (m, 5H).

### Step 7: Synthesis of compound 3-((2S)-1-(5-((S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-5-oxo-4,5-dihydro-1,2,4-triazine-6-nitrile (compound 419)

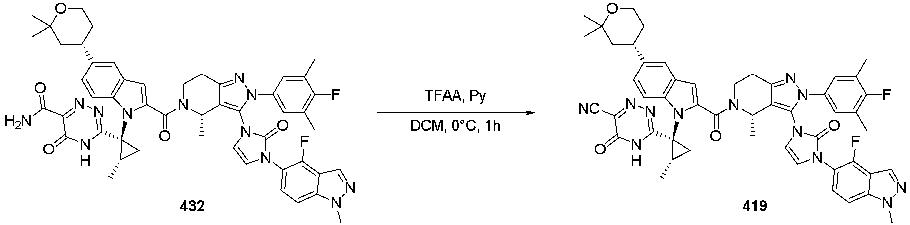

Compound **432** (60 mg, 0.06 mmol) and pyridine (19 mg, 0.24 mmol) were sequentially added to dichloromethane (2 mL), to which was added trifluoroacetic anhydride (38 mg, 0.18 mmol) at 0°C. The mixture was stirred and reacted at 0 °C for 1 hour, and the completion of the starting material reaction was monitored by TLC. The reaction solution was diluted with 10 mL of water, and extracted with dichloromethane (20 mL × 3). The organic phases were combined, dried over anhydrous sodium sulfate, filtered, and concentrated under reduced pressure. The concentrate was purified by silica gel column chromatography to obtain light yellow solid product, compound **419** (30 mg, 0.03 mmol), with a yield of 54.4%. M/S:919 (M+H⁺).

¹HNMR (400 MHz, DMSO-*d*₆) *δ* 8.31 (s, 1H), 7.63 (d, *J* = 8.8 Hz, 1H), 7.54 (s, 1H), 7.48 (d, *J* = 7.6 Hz, 1H), 7.36 (d, *J* = 8.4 Hz, 1H), 7.25 (d, *J* = 8.8 Hz, 1H), 7.18 (d, *J* = 6.6 Hz, 2H), 7.08 (d, *J* = 3.2 Hz, 1H), 7.03 (s, 1H), 6.95 (s, 2H), 4.11 (s, 3H), 3.70 (d, *J* = 8.8 Hz, 2H), 3.17 (s, 1H), 3.03 (s, 1H), 2.92 (d, *J* = 16.4 Hz, 1H), 2.26 (d, *J* = 2.2 Hz, 6H), 2.02 (s, 1H), 1.69 - 1.38 (m, 7H), 1.31 - 1.21 (m, 9H), 1.19 (m, 5H).

### Example 24. Synthesis of compound 6-((dimethylamino)methyl)-3-((2S)-1-(5-((S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-1,2,4-triazine-5(4H)-one (compound 433)

### 3-((2S)-1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-5-oxo-4,5-dihydro-1,2,4-triazine-6-aldehyde (compound 434)

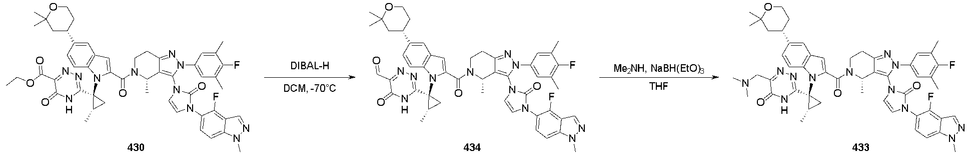

### Step 1: Synthesis of compound (3-((2S)-1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-5-oxo-4,5-dihydro-1,2,4-triazine-6-aldehyde (compound 434)

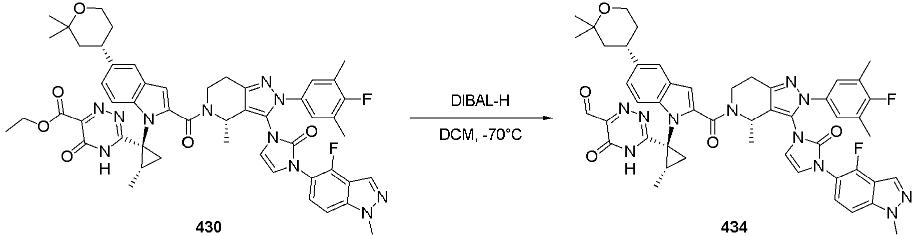

Compound **430** (100 mg, 0.10 mmol) was dissolved in dichloromethane (20 mL), and then the system was purged with nitrogen three times, to which was slowly added DIBAL-H (1.5N, 0.08 mL, 0.12 mmol) dropwise at -70°C. After that, the reaction was stirred and reacted at -70 °C for 1 h. The reaction of the starting material was monitored by TLC to be complete. The reaction solution was quenched with NaOH saturated solution (0.2 mL) at 0 °C, and then extracted with ethyl acetate (50 mL × 3). The organic phase was washed with 50 mL of saturated saline, dried over sodium sulfate, and filtered. The filtrate was concentrated under reduced pressure, and the concentrate was purified by silica gel column chromatography to obtain product **434** as light yellow solid (60 mg, 0.06 mmol), with a yield of 65.1%. M/S: 922 (M+H⁺).

### Step 2: Synthesis of compound 6-((dimethylamino)methyl)-3-((2S)-1-(5-((S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-1,2,4-triazine-5(4H)-one (compound 433)

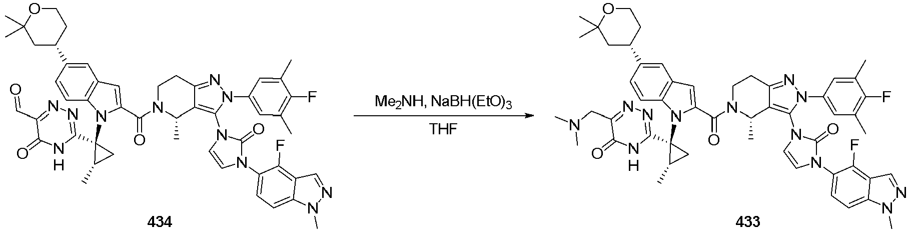

Compound **434** (60 mg, 0.06 mmol) was dissolved in anhydrous tetrahydrofuran (30 mL), and then the system was purged with nitrogen three times, to which was slowly added a solution of dimethylamine in tetrahydrofuran (2N, 0.2 mL, 0.36 mmol) dropwise at 0°C. After that, the reaction was stirred and reacted at room temperature for 2 h. When the reaction solution was cooled to 0°C, sodium triacetoxyborohydride (25 mg, 0.12 mmol) was added, and the reaction was stirred at room temperature for 1 h. TLC was used to monitor the completion of the raw material reaction. When the reaction was cooled to room temperature, the reaction solution was diluted with 20 mL of water, and extracted with ethyl acetate (20 mL × 3). The organic phases were combined, dried over anhydrous sodium sulfate, filtered, and concentrated under reduced pressure. The concentrate was purified by silica gel column chromatography to obtain product as light yellow solid (compound **433)** (30 mg, 0.03 mmol), with a yield of 52.6%. M/S: 952 (M+H⁺).

¹HNMR (400 MHz, DMSO-d₆) *δ* 8.31 (s, 1H), 7.63 (d, *J* = 8.8 Hz, 1H), 7.54 (s, 1H), 7.48 (d, *J* = 7.6 Hz, 1H), 7.36 (d, *J* = 8.4 Hz, 1H), 7.25 (d, *J* = 8.8 Hz, 1H), 7.18 (d, *J* = 6.6 Hz, 2H), 7.08 (d, *J* = 3.2 Hz, 1H), 7.03 (s, 1H), 6.95 (s, 2H), 4.11 (s, 3H), 3.70 (d, *J* = 8.8 Hz, 2H), 3.17 (s, 1H), 3.03 (s, 1H), 2.92 (d, *J* = 16.4 Hz, 1H), 2.26 (d, *J* = 2.2 Hz, 8H), 2.02 (s, 1H), 1.69 - 1.38 (m, 13H), 1.31 - 1.21 (m, 9H), 1.19 (m, 5H).

### Example 25. Synthesis of compound 3-((1S,2S)-1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-1,2,4-triazine-5(4H)-one (401)

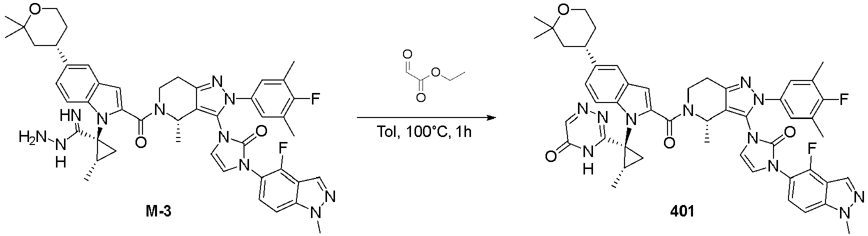

Intermediate **M-3** (70 mg, 0.08 mmol) was dissolved in toluene (10 mL), to which was added ethyl glyoxylate (26 mg, 0.25 mmol), and then stirred and reacted at 100 °C for 1 h. TLC monitoring indicated the reaction of raw material was complete. The reaction solution was cooled to room temperature, dilute with 50 mL of water, and extracted with ethyl acetate (50 mL×3). The organic phases were combined, dried over anhydrous sodium sulfate, filtered, and concentrated under reduced pressure. The concentrate was purified by silica gel column chromatography to obtain 40 mg of product as light yellow solid, with a yield of 51%. M/S: 894 (M+H⁺).

¹HNMR (400 MHz, DMSO-d₆) *δ* 13.20 (d, *J* = 2.0 Hz, 1H), 8.31 (d, *J* = 0.8 Hz, 1H), 7.70 (s, 1H), 7.64 (d, *J* = 8.8 Hz, 1H), 7.58 - 7.44 (m, 2H), 7.38 (d, *J* = 8.4 Hz, 1H), 7.26 (dd, *J* = 8.8, 1.6 Hz, 1H), 7.19 (d, *J* = 6.4 Hz, 2H), 7.08 (d, *J* = 3.2 Hz, 1H), 7.03 (s, 1H), 6.96 (s, 1H). 4.11 (s, 3H), 3.70 (d, *J* = 8.8 Hz, 2H), 3.17 (s, 1H), 3.03 (s, 1H), 2.92 (d, J = 16.4 Hz, 1H), 2.26 (d, J = 2.4 Hz, 6H), 2.02 (s, 1H), 1.69 - 1.38 (m, 7H), 1.31 - 1.21 (m, 9H), 1.19 (m, 5H).

### Example 26. Synthesis of compound 3-(1S,2S)-1-5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3-methyl-5-vinylphenyl)-4-methyl-4,5,6,7-tetrahydro-2H-pyrazolo[4,3-c]pyridine-5-carbonyl)-1H-indole-1-yl)-2-methylcyclopropyl)-1,2,4-triazine-5(4H)-one (405)

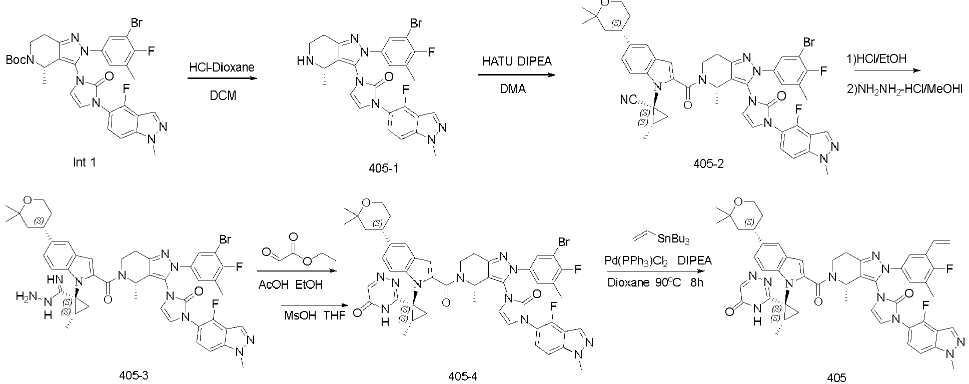

### Step 1: Synthesis of intermediate (S)-1-(2-(3-bromo-4-fluoro-5-methylphenyl)-4-methyl-4,5,6,7-tetrahydro-2H-pyrazolo[4,3-c]pyridine-3-yl)-3-(4-fluoro-1-methyl-1H-indazole-5-yl)-1,3-dihydro-2H-imidazole-2-one (405-1)

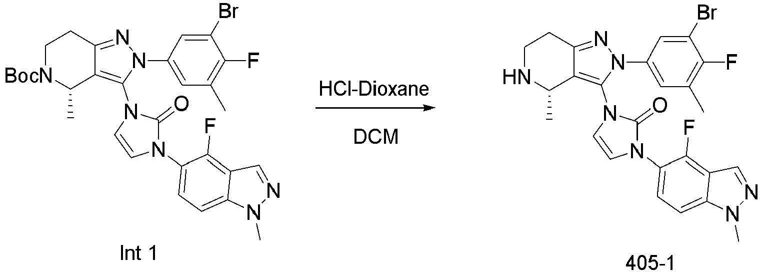

Intermediate **Int 1** (1.5 g, 2.3 mmol) was dissolved in DCM (5 mL), to which was added HCl-Dioxane (7.5 mL, 4 N) dropwise in an ice bath. The reaction was naturally warmed and reacted for 3 h. The reaction was monitored by TLC. Water (20 mL) was added to quench the reaction. Then, the reaction solution was adjusted pH (8-9) with NaHCO₃ saturated solution, and extracted with DCM (50 mL x 3). The DCM layers were combined, washed with saturated saline (30 mL x 2), dried over anhydrous sodium sulfate, filtered with suction, and concentrated, to obtain 1.2 g of white solid **(405-1),** with a yield of 94.5%, M/S: 554 (M+H⁺).

### Step 2: Synthesis of intermediate (1S,2S)-1-(2-((S)-2-(3-bromo-4-fluoro-5-methylphenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropane-1-nitrile (405-2)

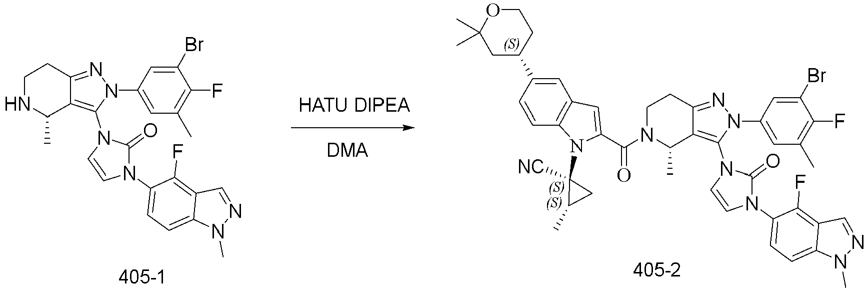

Intermediate **405-1** (1.2 g, 2.16 mmol), intermediate **1-9** (1.16 g, 2.82 mmol), and HATU (1.65 g, 4.33 mmol) were dissolved in DMA (5 mL), to which was added DIPEA (0.56 g, 4.33 mmol) dropwise, and then reacted at room temperature for 8 h. The reaction was monitored by TLC, and upon completion, the reaction was quenched by adding water (20 mL). The resultant solution was extracted with EA (30 mL x 3). The EA layers were combined, washed with water (30 mL x 3) and saturated saline (20mL x 2), dried over anhydrous sodium sulfate, filtered with suction, and concentrated. The concentrate was purified by column chromatography, to obtain 1.67 g of white solid **(405-2),** with a yield of 86.8%, M/S: 888 (M+H⁺).

### Step 3: Synthesis of intermediate (1S,2S)-1-((2Z,5E)-1-(S)-2-(3-bromo-4-fluoro-5-methylphenyl)-3-(3-(4-fluoro-1-methyl-1H-indazole-5-yl)-2-oxo-2,3-dihydro-1H-imidazole-1-yl)-4-methyl-2,4,6,7-tetrahydro-5H-pyrazolo[4,3-c]pyridine-5-yl)-6-((S)-2,2-dimethyltetrahydro-2H-pyran-4-yl)-1-oxoocta-2,5,7-trien-2-yl)amino)-2-methylcyclopropane-1-carboxylic hydrazide (405-3)

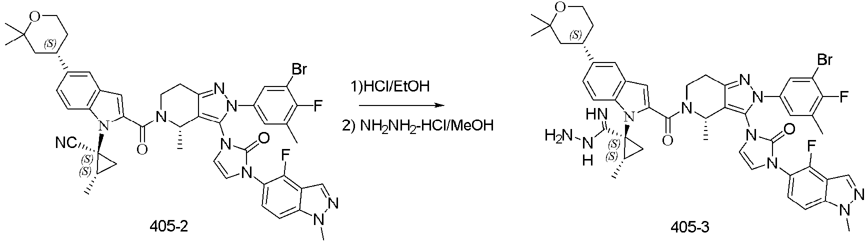

Intermediate **405-2** (1.67 g, 1.88 mmol) was dissolved in EtOH (25 mL), to which was introduced HCl for 6 h. The reaction was monitored by TLC. The reaction solution was concentrated to dryness, and then co-evaporated with DCM three times, followed by adding MeOH (25 mL) to dissolve completely. The solution was adjusted to pH 8-9 with DIPEA, and then hydrazine hydrochloride (3.87 g, 56.4 mmol) was added. The pH was adjusted to be >8. The reaction was allowed to react at room temperature for 4 h, which was monitored by TLC. After completion of the reaction, the reaction was quenched by adding water (50 mL). The reaction solution was extracted with DCM (50 mL x 3). The DCM layers were combined, washed with saturated saline (30 mL x 2), dried over anhydrous sodium sulfate, filtered under suction, and concentrated. The residue was subjected to column chromatograph, to obtain 1.12 g of white solid **(405-3),** with a yield of 64.7%, M/S: 920 (M+H⁺).

### Step 4: Synthesis of intermediate 3-((1S,2S)-1-(2-(S)-2-(3-bromo-4-fluoro-5-methylphenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropyl)-1,2,4-triazine-5(4H)-one (405-4)

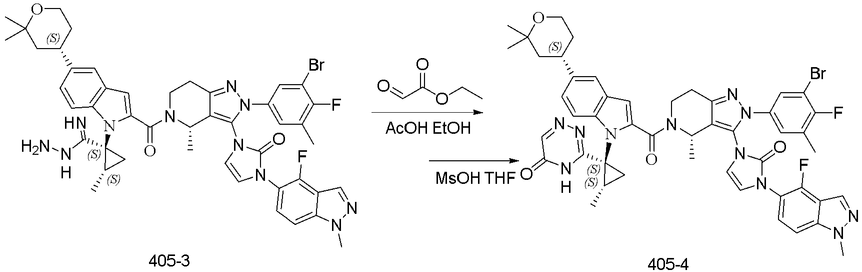

Intermediate **405-3** (200 mg, 0.22 mmol), ethyl glyoxylate (110 mg, 1.1 mmol), and glacial acetic acid (53 mg, 0.88 mmol) were dissolved in EtOH (5 mL), and under N₂ protection, the mixture was allowed to react at 90 °C for 4 h. The reaction was monitored by TLC, and upon completion, it was quenched by adding water (20 mL). The resultant solution was extracted with DCM (20 mL x 3). The DCM layers were combined, washed with saturated saline (30 mL x 2), dried over anhydrous sodium sulfate, filtered under suction, and concentrated. The residue was subjected to column chromatograph to obtain 150 mg of white solid, which was dissolved in THF (3 mL), followed by adding MsOH (150 mg, 1.56 mmol). The reaction was stirred at 60 °C for 30 min. LC-MS was used to monitor the reaction. After completion, the reaction was quenched by adding water (20 mL). The pH was adjusted to 6-7 with NaHCO₃ solution. The resultant solution was extracted with DCM (20 mL x 3). The DCM layers were combined, washed with saturated saline (20 mL x 2), dried over anhydrous sodium sulfate, filtered under suction, and concentrated. The residue was subjected to column chromatography to obtain 130 mg of white solid **(405-4).** Yield: 62.4%, M/S: 958 (M+H⁺).

### Step 5: Synthesis of compound 3-(1S,2S)-1-5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3-methyl-5-vinylphenyl)-4-methyl-4,5,6,7-tetrahydro-2H-pyrazolo[4,3-c]pyridine-5-carbonyl)-1H-indole-1-yl)-2-methylcyclopropyl)-1,2,4-triazine-5(4H)-one (405)

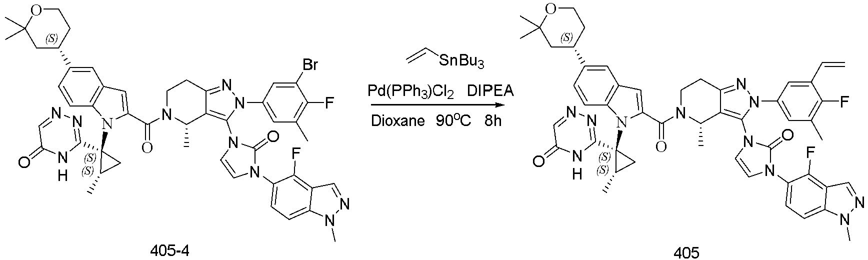

Intermediate **405-4** (130 g, 0.136 mmol), tributylvinyltin (86 mg, 0.272 mmol), Pd(dppf)Cl₂ (19 mg, 0.027 mmol), and DIPEA (35 mg, 0.272 mmol) were dissolved in dioxane (5 mL), and under N₂ protection, the mixture was allowed to react at 90 °C for 8 h. TLC monitoring indicated completion of the reaction. The reaction solution was cooled to room temperature, and then quenched by adding water (10 mL), followed by adding EA (10 mL). The resultant solution was filtered through diatomaceous earth. The filter cake was rinsed with EA (10 mL x 2), and then the layers were separated, followed by extracting with EA (10 mL x 2). The EA layers were combined, washed with saturated saline (10 mL x 2), dried over anhydrous sodium sulfate, filtered with suction, and concentrated. The residue was subjected to column chromatography to obtain 90 mg of white solid **(405),** with a yield of 73.3%, M/S: 906 (M+H⁺).

¹HNMR (400 MHz, DMSO-*d*₆) *δ* 14.12 (s, 1H), 8.29 (d, *J* = 0.9 Hz, 1H), 7.70 (s, 1H), 7.63 (d, *J* = 8.9 Hz, 1H), 7.49 (dd, *J* = 6.3, 2.7 Hz, 1H), 7.47 - 7.40 (m, 1H), 7.38 (d, *J* = 8.5 Hz, 1H), 7.33 (dd, *J* = 6.4, 2.6 Hz, 1H), 7.26 (dd, *J* = 8.7, 1.7 Hz, 1H), 7.12 (d, *J* = 3.2 Hz, 1H), 7.03 (d, *J* = 5.0 Hz, 2H), 6.92 - 6.82 (m, 1H), 5.84 (d, *J* = 17.6 Hz, 1H), 5.63 (q, *J* = 6.5 Hz, 1H), 5.51 - 5.45 (m, 1H), 4.51 - 4.35 (m, 1H), 4.10 (s, 3H), 3.70 (d, *J* = 9.6 Hz, 3H), 3.06 - 2.89 (m, 2H), 2.30 (d, *J* = 2.1 Hz, 3H), 2.00 (dd, *J* = 8.0, 5.5 Hz, 1H), 1.83 (dt, *J* = 14.5, 7.5 Hz, 1H), 1.65 (d, *J* = 10.8 Hz, 3H), 1.46 (dd, *J* = 6.7, 3.7 Hz, 4H), 1.27 - 1.22 (m, 6H), 1.17 (s, 3H).

### Example 27. Synthesis of compound 3-((1S,2S)-1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-6-methyl-1,2,4-triazine-5(4H)-one (413)

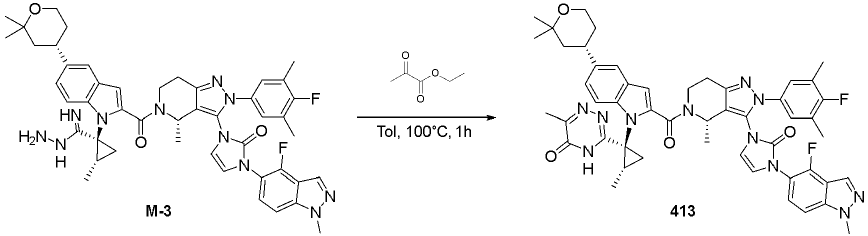

Intermediate **M-3** (70 mg, 0.08 mmol) was dissolved in toluene (10 mL), to which was added ethyl pyruvate (29 mg, 0.25 mmol), and then stirred and reacted at 100 °C for 1 h. TLC monitoring indicated completion of the raw material reaction. The reaction solution was cooled to room temperature, diluted with 50 mL of water, and extracted with ethyl acetate (50 mL×3). The organic phases were combined, dried over anhydrous sodium sulfate, filtered, and concentrated under reduced pressure. The concentrate was purified by silica gel column chromatography to obtain 40 mg of product as light yellow solid, with a yield of 50%. M/S: 908 (M+H⁺).

¹HNMR (400 MHz, DMSO-d₆) *δ* 8.31 (s, 1H), 7.63 (d, *J* = 8.8 Hz, 1H), 7.54 (s, 1H), 7.48 (d, *J* = 7.6 Hz, 1H), 7.36 (d, *J* = 8.4 Hz, 1H), 7.25 (d, *J* = 8.8 Hz, 1H), 7.18 (d, *J* = 6.8 Hz, 2H), 7.08 (d, *J* = 3.2 Hz, 1H), 7.03 (s, 1H), 6.95 (s, 2H), 4.11 (s, 3H), 3.70 (d, *J* = 8.8 Hz, 2H), 3.17 (s, 1H), 3.03 (s, 1H), 2.92 (d, *J*= 16.4 Hz, 1H), 2.26-2.18 (m, 9H), 2.02 (s, 1H), 1.69 - 1.38 (m, 7H), 1.31 - 1.21 (m, 9H), 1.19 (m, 5H).

### Example 28. Synthesis of compound 2-((1S,2S)-1-(5-(S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)pyrimidine-4(3H)-one (420)

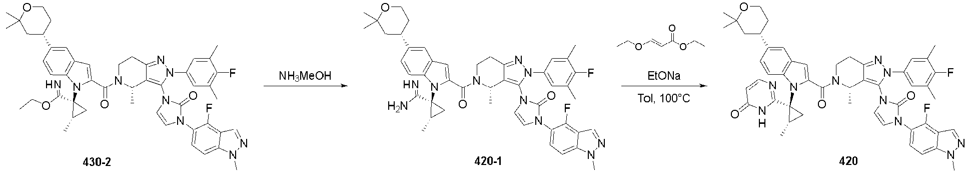

### Step 1: Synthesis of intermediate (1S,2S)-1-(5-(S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropane-1-carboximidamide (420-1)

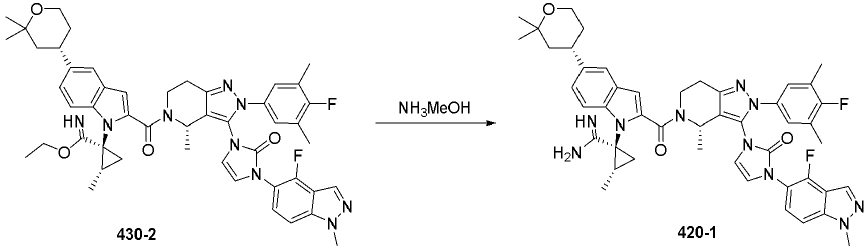

Intermediate **430-2** (2 g, 2.3 mmol) was dissolved in a solution of amine in methanol (10N, 20 mL), and then stirred at room temperature for 2 h. TLC monitoring indicated the reaction of the raw material was complete. The reaction solution was diluted with 50 mL of water, and extracted with ethyl acetate (50 mL × 3). The organic phases were combined, dried over anhydrous sodium sulfate, filtered, and concentrated under reduced pressure. The concentrate was purified by silica gel column chromatography, to obtain 1 g of product as light yellow solid, with a yield of 52%. M/S: 841 (M+H⁺).

### Step 2: Synthesis of compound 2-((1S,2S)-1-(5-(S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2 -(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)pyrimidine-4(3H)-one (420)

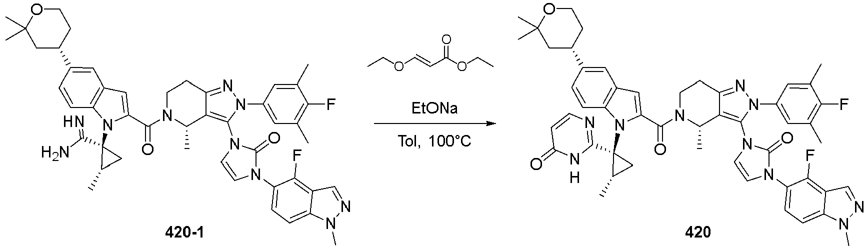

Intermediate **420-1** (100 mg, 0.12 mmol) was dissolved in toluene (2 mL), to which were added sodium ethoxide (9 mg, 0.24 mmol) and (E)-3-ethoxyethyl acrylate (52 mg, 0.36 mmol), and then stirred and reacted at 100 °C for 3 h. TLC monitoring indicated completion of the raw material reaction. The reaction solution was cooled to room temperature, diluted with 50 mL of water, and extracted with ethyl acetate (50 mL×3). The organic phases were combined, dried over anhydrous sodium sulfate, filtered, and concentrated under reduced pressure. The concentrate was purified by silica gel column chromatography to obtain 30 mg of product as light yellow solid, with a yield of 28%. M/S: 893 (M+H⁺).

¹HNMR (400 MHz, DMSO-d₆) *δ* 13.20 (d, *J* = 2.0 Hz, 1H), δ 8.31 (d, *J* = 0.8 Hz, 1H), 8.00 (d, *J* = 6.4 Hz, 1H), 7.64 (dd, *J* = 8.8, 1.0 Hz, 1H), 7.55 - 7.41 (m, 3H), 7.25 (dd, *J* = 8.4, 1.6 Hz, 2H), 7.19 (d, *J* = 6.4 Hz, 2H), 7.08 (d, *J* = 3.2 Hz, 1H), 7.01 (s, 1H), 6.98 (d, *J* = 5.2 Hz, 1H)., 4.11 (s, 3H), 3.70 (d, *J* = 8.8 Hz, 2H), 3.17 (s, 1H), 3.03 (s, 1H), 2.92 (d, *J* = 16.4 Hz, 1H), 2.26 (d, *J* = 2.4 Hz, 6H), 2.02 (s, 1H), 1.69 - 1.38 (m, 7H), 1.31 - 1.21 (m, 9H), 1.19 (m, 5H).

### Example 29. Synthesis of compound 3-(1-(5-((S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)cyclopropyl)-1,2,4-triazine-5(4H)-one (426)

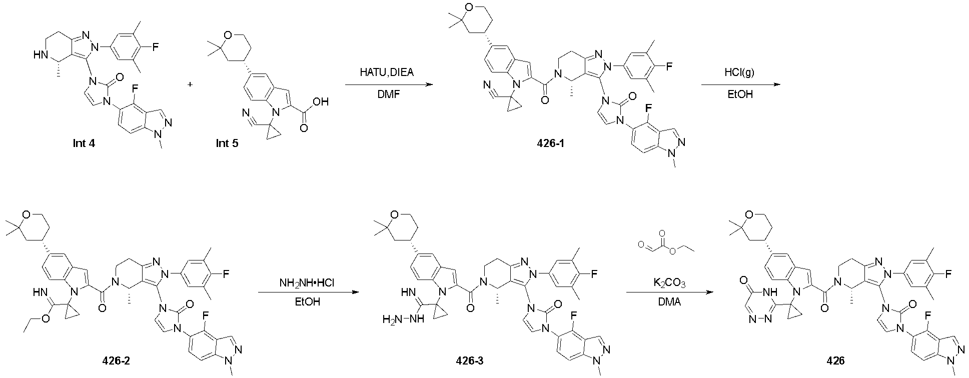

### Step 1: Synthesis of intermediate 1-(5-((S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)cyclopropane-1-carbonitrile (426-1)

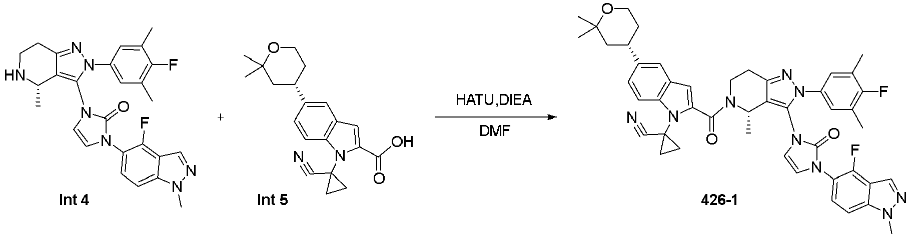

Intermediate **Int.5** (2 g, 5.91 mmol) was dissolved in DMF (20 mL), to which were added DIEA (1.53 g, 11.82 mmol) and HATU (3.37 g, 8.87 mmol), and then stirred at room temperature for 20 min, followed by adding intermediate Int 4 (3.18 g, 6.50 mmol). The reaction was stirred at room temperature for 2 h. TLC monitoring indicated the reaction of raw material was complete. Water (50 mL) was added for working up. The resultant solution was extracted with EA (50 mL × 3). The organic phase was dried over anhydrous sodium sulfate, and then concentrated to dry, followed by column chromatography to obtain 3.8 g of light yellow solid, with a yield of 79.4%. M/S: 810 (M+H⁺).

### Step 2: Synthesis of intermediate 1-(5-((S)-2,2-dimethyltetrahydro-2H-pyran-4-yl)-2-(S)-3-(3-(4-fluoro-1-methyl-1H-imidazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)cyclopropane-1-carboxylate (426-2)

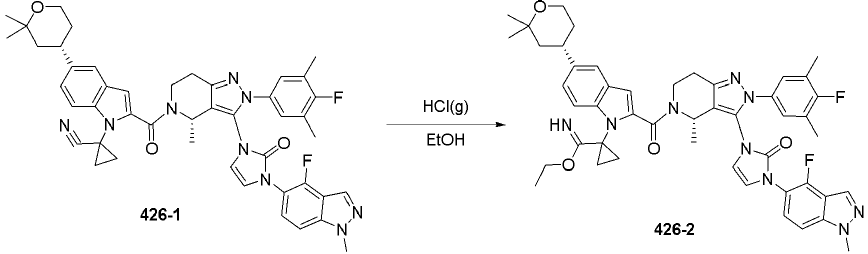

Intermediate **426-1** (3.8 g, 4.69 mmol) was dissolved in anhydrous ethanol (40 mL), to which was introduced hydrochloric acid gas, and then reacted at 10 °C for 6 h. TLC monitoring indicated the reaction of the raw material was complete. During working up, NaHCO₃ aqueous solution was added to adjust the pH to 7-8. The resultant solution was extracted with EA (50 mL × 3). The organic phase was dried over anhydrous sodium sulfate, and concentrated to obtain 3.2 g of crude product. Yield: 84%. M/S: 856 (M+H⁺).

### Step 3: Synthesis of intermediate 1-(5-((S)-2,2-dimethyltetrahydro-2H-pyran-4-yl)-2-(S)-3-(3-(4-fluoro-1-methyl-1H-imidazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)cyclopropane-1-formic hydrazide (426-3)

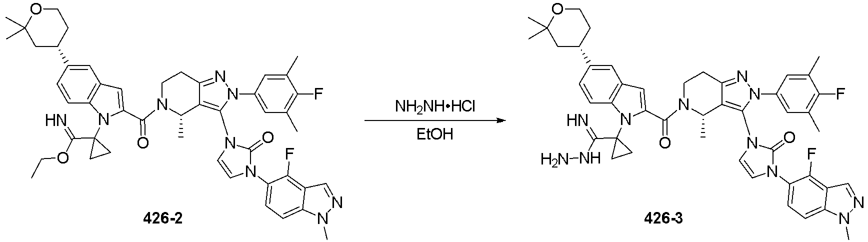

Intermediate **426-2** (1.0 g, 1.17 mmol) was dissolved in ethanol (10 mL), to which were added hydrazine hydrochloride (0.4 g, 5.85 mmol) and NaHCO₃ saturated solution, and then stirred at room temperature for 2 h. TLC monitoring indicated completion of the raw material reaction. Water (20 mL) was added for working up. The resultant solution was extracted with EA (30 mL × 3). The organic phase was dried over anhydrous sodium sulfate, and then concentrated to dry, followed by column chromatography to obtain 500 mg of light yellow solid, with a yield of 50.4%. M/S: 842 (M+H⁺).

### Step 4: Synthesis of 3-(1-(5-((S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)cyclopropyl)-1,2,4-triazine-5(4H)-one (426)

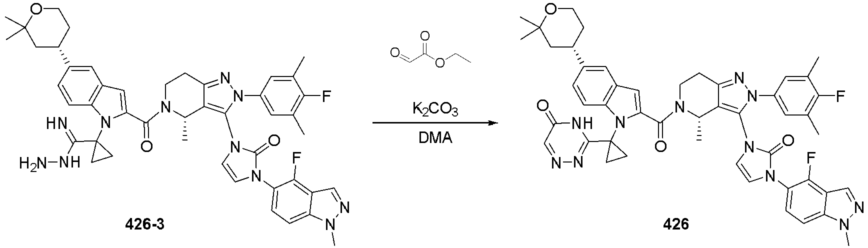

Intermediate **426-3** (60 mg, 0.07 mmol) was dissolved in DMA (1 mL), to which were added K₂CO₃ (39 mg, 0.28 mmol) and ethyl glyoxylate (22 mg, 0.21 mmol), and then stirred at 120 °C for 12 h. The completion of the raw material reaction was monitored by TLC. Afterwards, water (10 mL) was added for post-treatment, and the resultant solution was extracted with EA (10 mL × 3). The organic phase was dried over anhydrous sodium sulfate and concentrated to dryness, followed by column chromatography, to obtain 25 mg of white solid, with a yield of 40.0%. M/S: 880 (M+H⁺).

¹H NMR (400 MHz, DMSO-d₆) *δ* 11.78 (s, 1H), 8.31 (s, 1H), 7.66 (d, *J=* 8.9 Hz, 1H), 7.53 (s, 1H), 7.47 (d, *J= 7.9* Hz, 1H), 7.39 (d, *J=* 8.5 Hz, 1H), 7.26 (d, *J =* 8.5 Hz, 1H), 7.16 (d, *J =* 6.2 Hz, 2H), 7.09 (s, 1H), 6.97 (s, 2H), 4.12 (d, *J=* 10.7 Hz, 3H), 3.73 (d, *J=* 8.4 Hz, 2H), 3.16 (s, 1H), 3.02 (d, *J=* 12.1 Hz, 1H), 2.86 (d, *J =* 14.9 Hz, 1H), 2.28 (s, 3H), 1.66 (d, *J =* 12.2 Hz, 7H), 1.45 (d, *J =* 6.7 Hz, 2H), 1.28 - 1.17 (m, 14H).

### Example 30. Synthesis of compound 3-(1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)cyclopropyl)-5-oxo-4,5-dihydro-1,2,4-triazine-6-carboxylic acid (428)

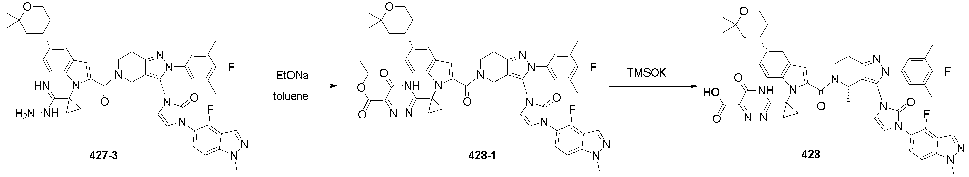

### Step 1: Synthesis of intermediate ethyl 3-(1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)cyclopropyl)-5-oxo-4,5-dihydro-1,2,4-triazine-6-carboxylate (428-1)

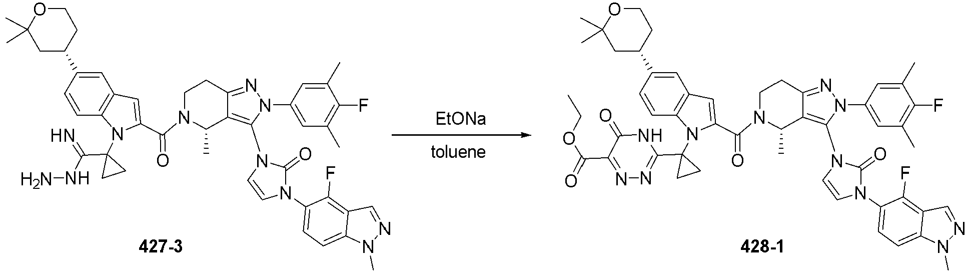

Intermediate **427-3** (168 mg, 0.2 mmol) was dissolved in toluene (3 mL), to which were added sodium ethoxide (68 mg, 1.0 mmol) and diethyl ketomalonate (172 mg, 1.0 mmol), and then stirred and reacted at 60 °C for 8 h. TLC monitoring indicated the reaction of raw material was complete. The reaction solution was cooled to room temperature, diluted with 10 mL of water, and extracted with ethyl acetate (5 mL×3). The organic phases were combined, dried over anhydrous sodium sulfate, filtered, and concentrated under reduced pressure. The concentrate was purified by column chromatography to obtain 90 mg of product, with a yield of 47.3%. M/S: 952 (M+H⁺).

### Step 2: Synthesis of 3-(1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)cyclopropyl)-5-oxo-4,5-dihydro-1,2,4-triazine-6-carboxylic acid (428)

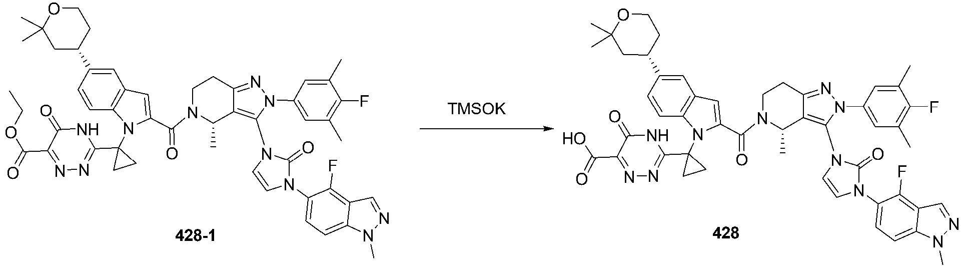

Intermediate **428-1** (57 mg, 0.06 mmol) was dissolved in tetrahydrofuran (2 mL) at room temperature, followed by addition of potassium trimethylsilanolate (38 mg, 0.3 mmol). The mixture was stirred at room temperature for 1 h, and TLC monitoring indicated the completion of the starting material reaction. The reaction solution was adjusted to pH ≈ 3 with hydrochloric acid (2N) solution and then extracted with ethyl acetate (5 mL × 3). The organic phases were combined, dried over anhydrous sodium sulfate, filtered, and concentrated under reduced pressure. The concentrate was purified by column chromatography to obtain 50 mg of product, with a yield of 90.2%. M/S: 924 (M+H⁺).

¹HNMR (400 MHz, DMSO-d6) *δ* 8.31 (s, 1H), 7.63 (d, *J=* 8.8 Hz, 1H), 7.54 (s, 1H), 7.48 (d, *J= 7.6* Hz, 2H), 7.36 (d, *J =* 8.4 Hz, 1H), 7.25 (d, *J =* 8.8 Hz, 1H), 7.18 (d, *J =* 6.6 Hz, 2H), 7.08 (d, *J= 3.2* Hz, 1H), 7.03 (s, 1H), 6.95 (s, 2H), 4.11 (s, 3H), 3.70 (d, J= 8.8 Hz, 2H), 3.17 (s, 1H), 3.03 (s, 1H), 2.92 (d, *J =* 16.4 Hz, 1H), 2.26 (d, *J =* 2.2 Hz, 6H), 2.02 (s, 1H), 1.69 - 1.38 (m, 7H), 1.31 - 1.21 (m, 7H), 1.19 (m, 5H).

### Example 31. Synthesis of compound 3-(1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)cyclopropyl)-5-oxo-4,5-dihydro-1,2,4-triazine-6-formamide (437)

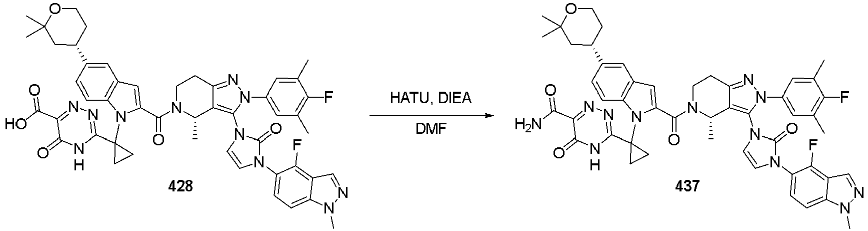

At room temperature, intermediate 428 (28 mg, 0.03 mmol) was dissolved in DMF (1 mL), to which were added HATU (17 mg, 0.05 mmol) and DIEA (19 mg, 0.15 mmol). The mixture was stirred and reacted for 20 min, and then ammonium chloride (8 mg, 0.15 mmol) was added. The reaction was stirred at room temperature for 2 h. TLC monitoring confirmed complete reaction of the starting material. The reaction solution was diluted with 4 mL of water, and extracted with ethyl acetate (3 mL × 3). The organic phase was washed with 10 mL of saturated saline, dried over anhydrous sodium sulfate, and filtered. After concentration under reduced pressure, the residue was purified by column chromatography to obtain 20 mg of product, with a yield of 72.2%. M/S: 923 (M+H⁺).

¹HNMR (400 MHz, DMSO-d₆) *δ* 8.31 (s, 1H), 7.63 (d, *J =* 8.8 Hz, 1H), 7.54 (s, 1H), 7.48 (d, *J= 7.6* Hz, 2H), 7.36 (d, *J =* 8.4 Hz, 1H), 7.25 (d, *J =* 8.8 Hz, 1H), 7.18 (d, *J =* 6.6 Hz, 2H), 7.08 (d, *J* = 3.2 Hz, 1H), 7.03 (s, 1H), 6.95 (s, 2H), 4.11 (s, 3H), 3.70 (d, *J =* 8.8 Hz, 2H), 3.17 (s, 1H), 3.03 (s, 1H), 2.92 (d, *J* = 16.4 Hz, 1H), 2.26 (d, *J =* 2.2 Hz, 6H), 2.02 (s, 1H), 1.69 - 1.38 (m, 7H), 1.31 - 1.21 (m, 7H), 1.19 (m, 5H).

### Example 32. Synthesis of compound 6-((dimethylamino)methyl)-3-((2S)-1-(5-((S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-1,2,4-triazine-5(4H)-one (442)

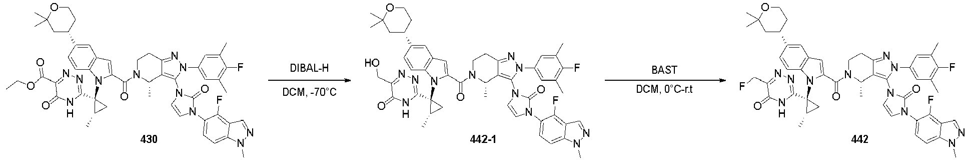

### Step 1: Synthesis of 3-((2S)-1-(5-(S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-6-(hydroxymethyl)-1,2,4-triazine-5(4H)-one (442-1)

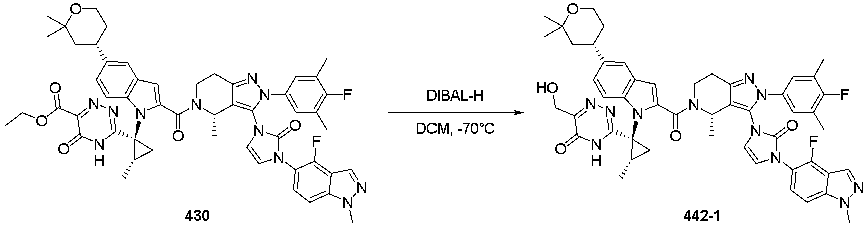

Intermediate 430 (100 mg, 0.10 mmol) was dissolved in dichloromethane (20 mL), and then the system was purged with nitrogen for 3 times, to which was then slowly added DIBAL-H (1.5N, 0.08 mL, 0.12 mmol) dropwise at -70 °C. After that, the reaction was stirred and reacted at -70 °C for 1 h. TLC monitoring indicated the reaction of the raw material was complete. The reaction solution was quenched with NaOH saturated solution (0.2 mL) at 0°C. The resultant solution was extracted with ethyl acetate (50 mL×3). The organic phase was washed with 50 mL of saturated saline, dried over sodium sulfate, filtered, and concentrated under reduced pressure. The concentrate was purified by silica gel column chromatography to obtain 60 mg of product as light yellow solid, with a yield of 65.1%. M/S: 924 (M+H⁺).

### Step 2: Synthesis of 3-((2S)-1-(5-(S)-2,2-dimethyltetrahydro-2H-pyran-4-yl)-2-(S)-3-(3-(4-fluoro-1-methyl-1H-indazole-5-yl)-2-oxo-2,3-dihydro-1H-imidazole-1-yl)-2-(4-fluoro-3,5-dimethylphenyl)-4-methyl-4,5,6,7-tetrahydro-2H-pyrazolo[4,3-c]pyridine-5-carbonyl)-1H-indole-1-yl)-2-methylcyclopropyl)-6-(fluoromethyl)-1,2,4-triazine-5(4H)-one (442)

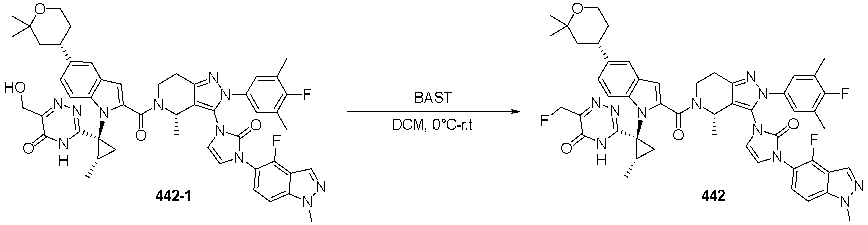

Intermediate 442-1 (60 mg, 0.06 mmol) was dissolved in anhydrous dichloromethane (30 mL), and then the system was purged with nitrogen three times, to which was slowly added bis(2-methoxyethyl)aminosulfur trifluoride (18 mg, 0.08 mmol) dropwise at 0 °C. After that, the reaction solution was stirred at room temperature for 1 h, and then cooled 0 °C. The reaction was quenched by adding methanol. The reaction solution was diluted with 20 mL of water, and extracted with ethyl acetate (20 mL × 3). The organic phase was combined, dried over anhydrous sodium sulfate, filtered, and concentrated under reduced pressure. The concentrate was purified by silica gel column chromatography to obtain 30 mg of product as light yellow solid, with a yield of 52.6%. M/S: 926 (M+H⁺).

¹HNMR (400 MHz, DMSO-d₆) *δ* 13.20 (d, *J =* 2.0 Hz, 1H), 8.31 (d, *J =* 0.8 Hz, 1H), 7.70 (s, 1H), 7.64 (d, *J =* 8.8 Hz, 1H), 7.58 - 7.44 (m, 1H), 7.38 (d, *J =* 8.4 Hz, 1H), 7.26 (dd, *J = 8.8,* 1.6 Hz, 1H), 7.19 (d, *J=* 6.4 Hz, 2H), 7.08 (d, *J=* 3.2 Hz, 1H), 7.03 (s, 1H), 6.96 (s, 1H). 4.11 (s, 3H), 4.09 (d, *J =* 2.4 Hz, 2H), 3.70 (d, *J =* 8.8 Hz, 2H), 3.17 (s, 1H), 3.03 (s, 1H), 2.92 (d, *J =* 16.4 Hz, 1H), 2.26 (d, *J=* 2.4 Hz, 6H), 2.02 (s, 1H), 1.69 - 1.38 (m, 7H), 1.31 - 1.21 (m, 9H), 1.19 (m, 5H).

### Example 33. Synthesis of compound 3-(1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-divinylphenyl)-4-methyl-4,5,6,7-tetrahydro-2H-pyrazolo[4,3-c]pyridine-5-carbonyl)-1H-indole-1-yl)cyclopropyl)-1,2,4-triazine-5(4H)-one (447)

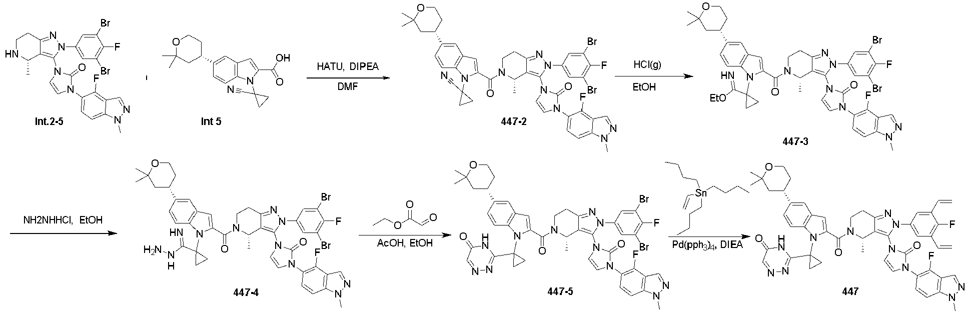

### Step 1: Synthesis of intermediate 1-(2-(S)-2-(3,5-dibromo-4-fluorophenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)cyclopropane-1-nitrile (intermediate 447-2)

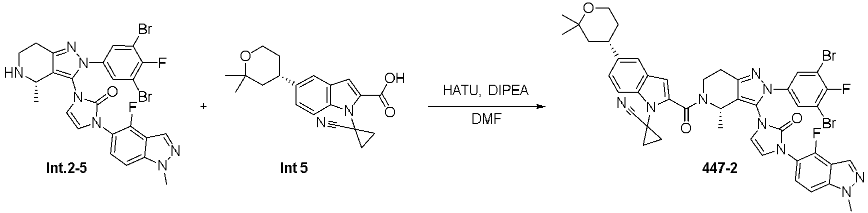

At room temperature, intermediate Int 5 (700 mg, 1.13 mmol), **Int.2-5** (347 mg, 1.03 mmol), and DIPEA (0.265 g, 2.06 mmol) were dissolved in DMF (10 mL), to which was added HATU (586 mg, 1.54 mmol), and then stirred and reacted at room temperature for 2 h. The reaction of the raw materials was monitored by TLC to be complete. The reaction solution was diluted with 10 mL of water, and extracted with ethyl acetate (50 mL × 3). The organic phase was washed with 50 mL of saturated saline solution, dried over sodium sulfate, and filtered. The filtrate was concentrated under reduced pressure, and the concentrate was purified by silica gel chromatography to obtain light yellow solid product, intermediate 447-2 (0.9 g, 0.95 mmol), with a yield of 93%. M/S: 938.2 (M+H⁺).

### Step 2: Synthesis of 1-(2-(S)-2-(3,5-dibromo-4-fluorophenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)cyclopropane-1-carbimide ethyl ester (intermediate 447-3)

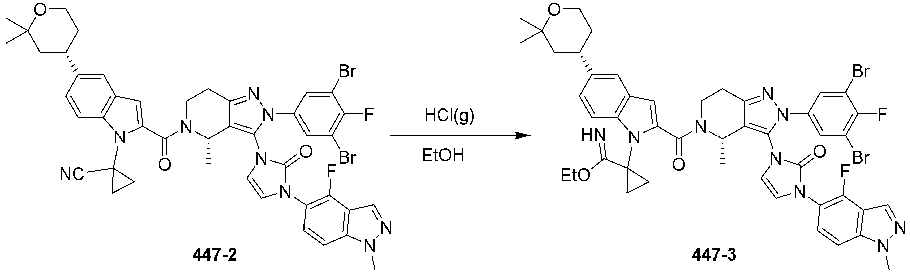

Intermediate **447-2** (0.9 g, 0.95 mmol) was dissolved in anhydrous ethanol (10 mL), to which was introduced hydrochloric acid gas at 10 °C, and then stirred and reacted for 6 h. TLC monitoring indicated the reaction of the raw material was complete. The reaction solution was directly concentrated to complete dryness to obtain crude intermediate 447-3 (0.8 g, 0.81 mmol), with a yield of 84%. M/S: 984.2 (M+H⁺).

### Step 3: Synthesis of 1-(2-(S)-2-(3,5-dibromo-4-fluorophenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)cyclopropane-1-formylhydrazine (intermediate 447-4)

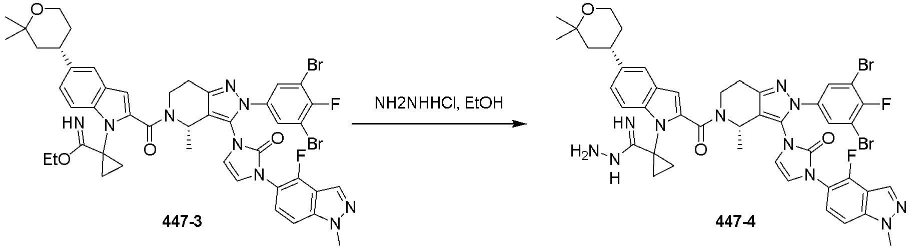

Intermediate **447-3** (0.7 g, 0.71 mmol) was dissolved in ethanol (10 mL), and then hydrazine hydrochloride (0.8 g, 10.65 mmol) and TEA (1.44 g, 14.20 mmol) were added in an ice bath. The mixture was stirred and reacted at room temperature for 2 h. TLC monitoring indicated complete reaction of the starting materials. The reaction solution was diluted with 50 mL of water and extracted with ethyl acetate (50 mL × 3). The organic phases were combined, dried over anhydrous sodium sulfate, filtered, and concentrated under reduced pressure. The concentrate was purified by silica gel column chromatography to obtain light yellow solid product, intermediate **447-4** (0.5 g, 0.5 mmol), with a yield of 72%. M/S: 970.2 (M+H⁺).

### Step 4: Synthesis of compound 3-(1-(2-(S)-2-(3,5-dibromo-4-fluorophenyl)-3-(3-(4-fluoro-1-methyl-1H-indazole-5-yl)-2-oxo-2,3-dihydro-1H-imidazole-1-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)cyclopropyl)-1,2,4-triazine-5(4H)-one (447-5)

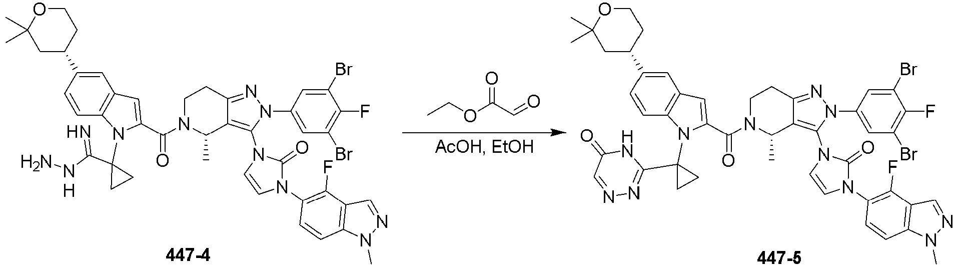

Intermediate 4 (500 mg, 0.51 mmol) was dissolved in ethanol (10 mL), followed by addition of acetic acid (92 mg, 1.54 mmol) and ethyl 2-oxoacetate (136 mg, 0.66 mmol). The mixture was stirred and reacted at 90 °C for 3 h, and TLC monitoring indicated completion of the starting material reaction. After cooling to room temperature, the reaction solution was diluted with 10 mL of water and extracted with ethyl acetate (10 mL × 3). The organic phases were combined, dried over anhydrous sodium sulfate, filtered, and concentrated under reduced pressure. The concentrate was purified by silica gel column chromatography to obtain light yellow solid product, compound 447-5 (300 mg, 0.29 mmol), with a yield of 57%. M/S: 1008.2 (M+H⁺).

### Step 5: Synthesis of compound 3-(1-(5-(S)-2,2-dimethyltetrahydro-2H-pyran-4-yl)-2-((S)-3-(3-(4-fluoro-1-methyl-1H-indazole-5-yl)-2-oxo-2,3-dihydro-1H-imidazole-1-yl)-2-(4-fluoro-3,5-divinylphenyl)-4-methyl-4,5,6,7-tetrahydro-2H-pyrazolo[4,3-c]pyridine-5-carbonyl)-1H-indole-1-yl)cyclopropyl)-1,2,4-triazine-5(4H)-one (447)

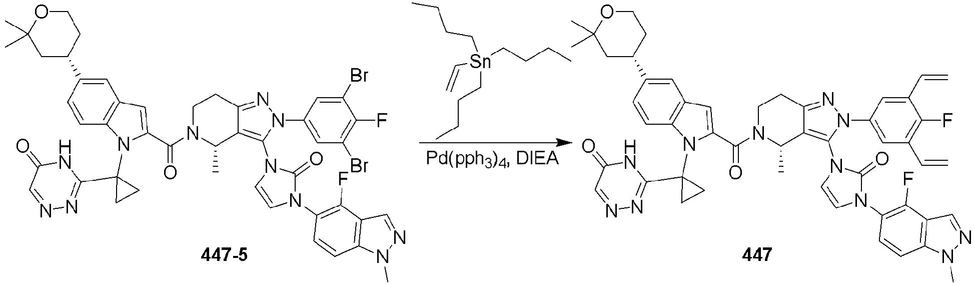

At room temperature, compound **447-5** (80 mg, 79.23 µmol), tributylvinyltin (125.62 mg, 396.15 µmol), DIEA (20.48 mg, 158.46 µmol), and tetrakis(triphenylphosphine)palladium (27.81 mg, 39.15 µmol) were dissolved in 1,4-dioxane (2.0 mL), and then stirred under nitrogen protection at 80 °C for 12 h. Completion of the raw material reaction was monitored by TLC. The reaction solution was stirred with KF solution (2.0 mL) for 1 h, and extracted with ethyl acetate (5 mL × 3). The organic phases were combined, dried over anhydrous sodium sulfate, filtered, and concentrated under reduced pressure. The concentrate was purified by HPLC to obtain product compound 447 as white solid (19 mg, 21.02 µmol), with a yield of 26%. M/S: 904.4 (M+H⁺). ¹HNMR (400 MHz, DMSO-d₆) *δ* 8.31 (s, 1H), 7.63 (d, *J =* 8.8 Hz, 1H), 7.54 (s, 1H), 7.48 (d, *J= 7.6* Hz, 2H), 7.36 (d, *J =* 8.4 Hz, 1H), 7.25 (d, *J= 8.8* Hz, 1H), 7.18 (d, *J =* 6.6 Hz, 2H), 7.08 (d, *J =* 3.2 Hz, 1H), 7.03 (s, 1H), 6.95 (s, 2H), 4.11 (s, 3H), 3.70 (d, *J =* 8.8 Hz, 2H), 3.17 (s, 1H), 3.03 (s, 1H), 2.92 (d, *J =* 16.4 Hz, 1H), 2.26 (d, *J =* 2.2 Hz, 6H), 2.02 (s, 1H), 1.69 - 1.38 (m, 7H), 1.31 - 1.21 (m, 9H), 1.19 (m, 5H).

The compounds listed in Table 3 and Table 4 could be synthesized by referring to the synthesis methods described in the above examples, using corresponding reagents under suitable conditions.

**Table 3. Compounds synthesized in the present invention and characterization data**

| Compound No. | Structure | Analytical data |
|---|---|---|
| 401 | 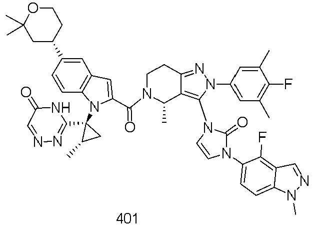 | LCMS (M+1): 894 |
| | | ¹HNMR (400 MHz, DMSO-d₆) *δ* 13.20 (d, J = 2.0 Hz, 1H), δ 8.31 (d, J = 0.8 Hz, 1H), 7.70 (s, 1H), 7.64 (d, J = 8.8 Hz, 1H), 7.58 - 7.44 (m, 2H), 7.38 (d, J = 8.4 Hz, 1H), 7.26 (dd, J = 8.8, 1.6 Hz, 1H), 7.19 (d, J = 6.4 Hz, 2H), 7.08 (d, J = 3.2 Hz, 1H), 7.03 (s, 1H), 6.96 (s, 1H). 4.11 (s, 3H), 3.70 (d, J = 8.8 Hz, 2H), 3.17 (s, 1H), 3.03 (s, 1H), 2.92 (d, J = 16.4 Hz, 1H), 2.26 (d, J = 2.4 Hz, 6H), 2.02 (s, 1H), 1.69 - 1.38 (m, 7H), 1.31 - 1.21 (m, 9H), 1.19 (m, 5H). |
| 402 | 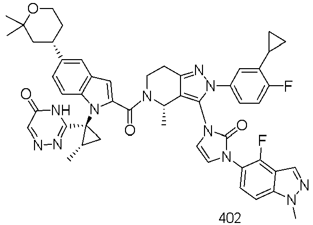 | LCMS (M+1): 906 |
| 403 | 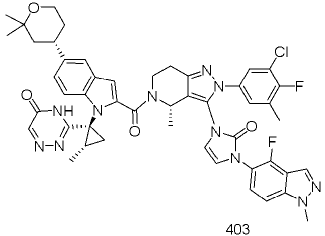 | LCMS (M+1): 914 |
| 404 | 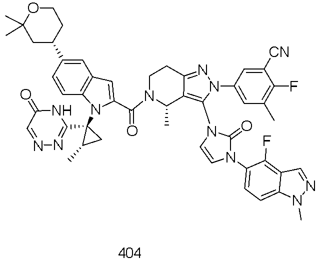 | LCMS (M+1): 905 |
| 405 | 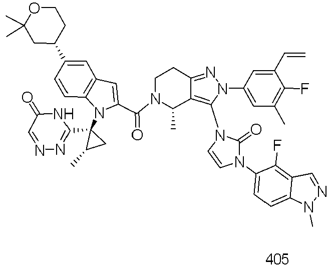 | LCMS (M+1): 906 |
| | | ¹H NMR (400 MHz, DMSO-d₆) *δ* 14.13 (s, 1H), 8.30 (s, 1H), 7.70 (s, 1H), 7.63 (d, J = 8.9 Hz, 1H), 7.54 (s, 1H), 7.49 (dd, J = 6.3, 2.6 Hz, 1H), 7.47 - 7.41 (m, 1H), 7.39 (d, J = 8.6 Hz, 1H), 7.34 (dd, J = 6.3, 2.7 Hz, 1H), 7.26 (dd, J = 8.5, 1.7 Hz, 1H), 7.08 (dd, J = 33.2, 3.6 Hz, 2H), 6.92 - 6.82 (m, 1H), 5.85 (d, J = 17.6 Hz, 1H), 5.76 (s, 1H), 5.56 (dd, J = 59.0, 9.0 Hz, 2H), 4.47 (d, J = 13.0 Hz, 1H), 4.11 (s, 3H), 3.73 - 3.68 (m, 3H), 3.25 (dd, J = 28.7, 5.3 Hz, 2H), 3.09 - 2.88 (m, 3H), 2.30 (d, J = 2.1 Hz, 3H), 2.05 - 1.93 (m, 2H), 1.84 (h, J = 6.5 Hz, 1H), 1.64 (t, J = 8.0 Hz, 3H), 1.58 - 1.41 (m, 5H), 1.15(s, 6H). |
| 406 | 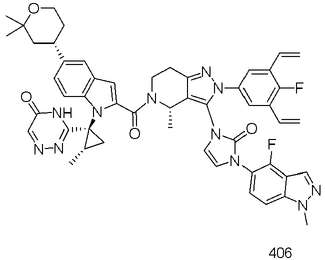 | LCMS (M+1): 918 |
| | | ¹H NMR (400 MHz, DMSO-d₆) *δ* 14.11 (s, 0H), 8.29 (s, 0H), 7.77 - 7.50 (m, 2H), 7.41 (d, J = 9.3 Hz, 1H), 7.29 - 6.81 (m, 2H), 5.90 (d, J = 17.5 Hz, 1H), 5.71 - 5.21 (m, 2H), 4.47 (s, 0H), 4.09 (d, J = 12.0 Hz, 2H), 3.70 (d, J = 8.8 Hz, 2H), 3.08 - 2.78 (m, 1H), 2.00 (d, J = 7.8 Hz, 1H), 1.73 - 1.38 (m, 3H), 1.25 (d, J = 12.7 Hz, 4H), 1.20 - 1.08 (m, 7H). |
| 407 | 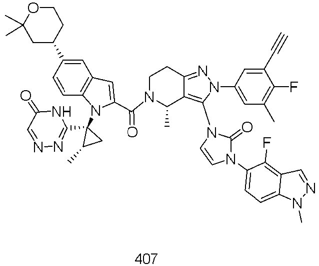 | LCMS (M+1): 904 |
| 408 | 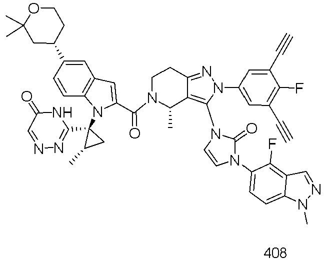 | LCMS (M+1): 914 |
| 409 | 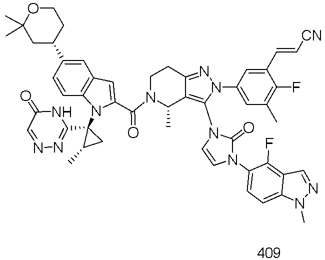 | LCMS (M+1): 931 |
| 410 | 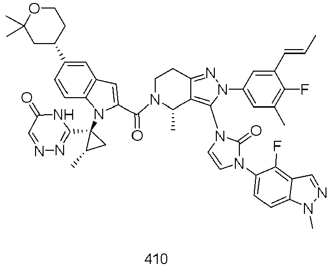 | LCMS (M+1): 920 |
| 411 | 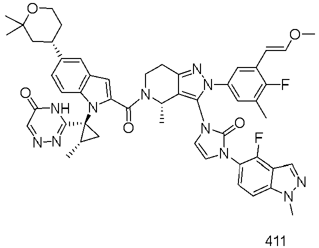 | LCMS (M+1): 936 |
| 412 | 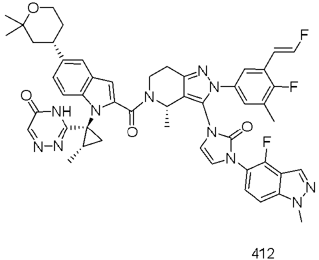 | LCMS (M+1): 924 |
| 413 | 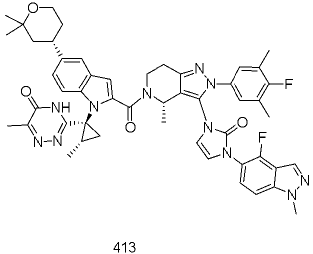 | LCMS (M+1): 908 |
| | | ¹HNMR (400 MHz, DMSO-d₆) *δ* 8.31 (s, 1H), 7.63 (d, J = 8.8 Hz, 1H), 7.54 (s, 1H), 7.48 (d, J = 7.6 Hz, 1H), 7.36 (d, J = 8.4 Hz, 1H), 7.25 (d, J = 8.8 Hz, 1H), 7.18 (d, J = 6.8 Hz, 2H), 7.08 (d, J = 3.2 Hz, 1H), 7.03 (s, 1H), 6.95 (s, 2H), 4.11 (s, 3H), 3.70 (d, J = 8.8 Hz, 2H), 3.17 (s, 1H), 3.03 (s, 1H), 2.92 (d, J = 16.4 Hz, 1H), 2.26-2.18 (m, 9H), 2.02 (s, 1H), 1.69 - 1.38 (m, 7H), 1.31 - 1.21 (m, 9H), 1.19 (m, 5H). |
| 414 | 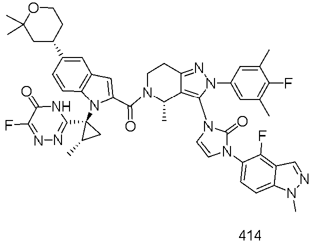 | LCMS (M+1): 912 |
| 415 | 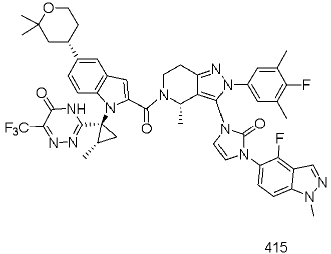 | LCMS (M+1): 962 |
| | | ¹HNMR (400 MHz, DMSO-d₆) *δ* 14.45 (s, 1H), 8.30 (s, 1H), 7.63 (d, J = 8.8 Hz, 1H), 7.55 (s, 1H), 7.47 (s, 1H), 7.38 (d, J = 8.4 Hz, 1H), 7.25 (d, J = 8.4 Hz, 1H), 7.18 (d, J = 6.0 Hz, 2H), 7.05 (d, J = 13.6 Hz, 1H), 6.94 (s, 1H), 6.52 (s, 1H), 4.11 (s, 3H), 3.70 (d, J = 8.8 Hz, 2H), 3.17 (s, 1H), 3.03 (s, 1H), 2.92 (d, J = 16.4 Hz, 1H), 2.26 (d, J = 2.4 Hz, 6H), 2.02 (s, 1H), 1.69 - 1.38 (m, 7H), 1.31 - 1.21 (m, 9H), 1.19 (m, 5H). |
| 416 | 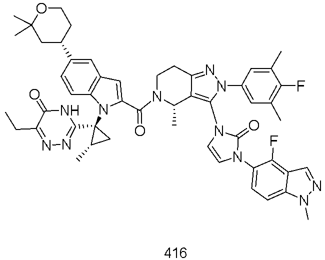 | LCMS (M+1): 894 |
| 417 | 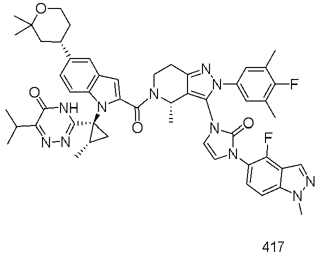 | LCMS (M+1): 894 |
| 418 | 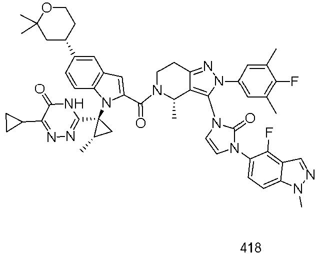 | LCMS (M+1): 894 |
| 419 | 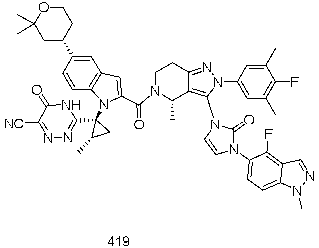 | LCMS (M+1): 894 |
| | | ¹HNMR (400 MHz, DMSO-d₆) *δ* 8.31 (s, 1H), 7.63 (d, J = 8.8 Hz, 1H), 7.54 (s, 1H), 7.48 (d, J = 7.6 Hz, 1H), 7.36 (d, J = 8.4 Hz, 1H), 7.25 (d, J = 8.8 Hz, 1H), 7.18 (d, J = 6.6 Hz, 2H), 7.08 (d, J = 3.2 Hz, 1H), 7.03 (s, 1H), 6.95 (s, 2H), 4.11 (s, 3H), 3.70 (d, J = 8.8 Hz, 2H), 3.17 (s, 1H), 3.03 (s, 1H), 2.92 (d, J = 16.4 Hz, 1H), 2.26 (d, J = 2.2 Hz, 6H), 2.02 (s, 1H), 1.69 - 1.38 (m, 7H), 1.31 - 1.21 (m, 9H), 1.19 (m, 5H). |
| 420 | 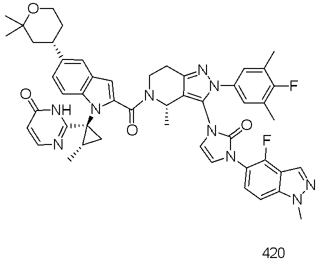 | LCMS (M+1): 894 |
| | | ¹HNMR (400 MHz, DMSO-d₆) *δ* 13.20 (d, J = 2.0 Hz, 1H), δ 8.31 (d, J = 0.8 Hz, 1H), 8.00 (d, J = 6.4 Hz, 1H), 7.64 (dd, J = 8.8, 1.0 Hz, 1H), 7.55 - 7.41 (m, 3H), 7.25 (dd, J = 8.4, 1.6 Hz, 2H), 7.19 (d, J = 6.4 Hz, 2H), 7.08 (d, J = 3.2 Hz, 1H), 7.01 (s, 1H), 6.98 (d, J = 5.2 Hz, 1H)., 4.11 (s, 3H), 3.70 (d, J = 8.8 Hz, 2H), 3.17 (s, 1H), 3.03 (s, 1H), 2.92 (d, J = 16.4 Hz, 1H), 2.26 (d, J = 2.4 Hz, 6H), 2.02 (s, 1H), 1.69 - 1.38 (m, 7H), 1.31 - 1.21 (m, 9H), 1.19 (m, 5H). |
| 421 | 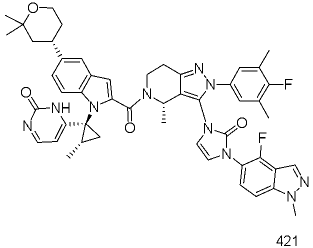 | LCMS (M+1): 894 |
| 422 | 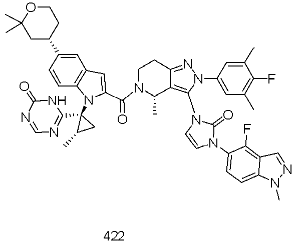 | LCMS (M+1): 894 |
| 423 | 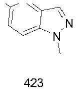 | LCMS (M+1): 894 |
| 424 | | LCMS (M+1): 894 |
| 425 | | LCMS (M+1): 894 |
| 426 | 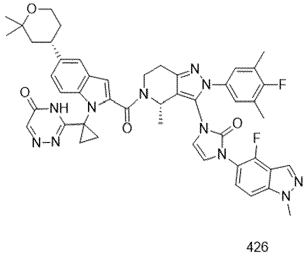 | ¹H NMR (400 MHz, DMSO-d₆) *δ* 11.78 (s, 1H), 8.31 (s, 1H), 7.66 (d, J = 8.9 Hz, 1H), 7.53 (s, 1H), 7.47 (d, J = 7.9 Hz, 1H), 7.39 (d, J = 8.5 Hz, 1H), 7.26 (d, J = 8.5 Hz, 1H), 7.16 (d, J = 6.2 Hz, 2H), 7.09 (s, 1H), 6.97 (s, 2H), 4.12 (d, J = 10.7 Hz, 3H), 3.73 (d, J = 8.4 Hz, 2H), 3.16 (s, 1H), 3.02 (d, J = 12.1 Hz, 1H), 2.86 (d, J = 14.9 Hz, 1H), 2.28 (s, 3H), 1.66 (d, J = 12.2 Hz, 7H), 1.45 (d, J = 6.7 Hz, 2H), 1.28 - 1.17 (m, 14H). |
| 427 | | LCMS (M+1): 894 |
| | | ¹HNMR (400 MHz, DMSO-d₆) *δ* 13.20 (d, J = 2.0 Hz, 1H), δ 8.31 (d, J = 0.8 Hz, 1H), 7.70 (s, 1H), 7.64 (d, J = 8.8 Hz, 1H), 7.58 - 7.44 (m, 2H), 7.38 (d, J = 8.4 Hz, 1H), 7.26 (dd, J = 8.8, 1.6 Hz, 1H), 7.19 (d, J = 6.4 Hz, 2H), 7.08 (d, J = 3.2 Hz, 1H), 7.03 (s, 1H), 6.96 (s, 1H). 4.11 (s, 3H), 3.70 (d, J = 8.8 Hz, 2H), 3.17 (s, 1H), 3.03 (s, 1H), 2.92 (d, J = 16.4 Hz, 1H), 2.26 (d, J = 2.4 Hz, 6H), 2.02 (s, 1H), 1.69 - 1.38 (m, 7H), 1.31 - 1.21 (m, 7H), 1.19 (m, 5H). |

**Table 4. Compounds synthesized in the present invention and characterization data**

| No. | Structure | Analytical data |
|---|---|---|
| 428 | 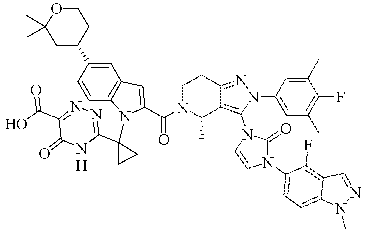 | LCMS (M+1): 924 |
| | | ¹HNMR (400 MHz, DMSO-d₆) *δ* 8.31 (s, 1H), 7.63 (d, J = 8.8 Hz, 1H), 7.54 (s, 1H), 7.48 (d, J = 7.6 Hz, 2H), 7.36 (d, J = 8.4 Hz, 1H), 7.25 (d, J = 8.8 Hz, 1H), 7.18 (d, J = 6.6 Hz, 2H), 7.08 (d, J = 3.2 Hz, 1H), 7.03 (s, 1H), 6.95 (s, 2H), 4.11 (s, 3H), 3.70 (d, J = 8.8 Hz, 2H), 3.17 (s, 1H), 3.03 (s, 1H), 2.92 (d, J = 16.4 Hz, 1H), 2.26 (d, J = 2.2 Hz, 6H), 2.02 (s, 1H), 1.69 - 1.38 (m, 7H), 1.31 - 1.21 (m, 7H), 1.19 (m, 5H). |
| 429 | 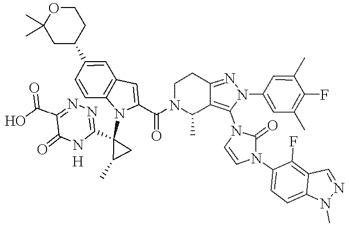 | LCMS (M+1): 938 |
| 430 | 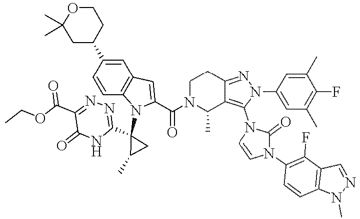 | LCMS (M+1): 966 |
| | | ¹HNMR (400 MHz, DMSO-d₆) δ 8.31 (s, 1H), 7.63 (d, J = 8.8 Hz, 1H), 7.54 (s, 1H), 7.48 (d, J = 7.6 Hz, 1H), 7.36 (d, J = 8.4 Hz, 1H), 7.25 (d, J = 8.8 Hz, 1H), 7.18 (d, J = 6.6 Hz, 2H), 7.08 (d, J = 3.2 Hz, 1H), 7.03 (s, 1H), 6.95 (s, 2H), 4.28 (d, J = 7.2 Hz, 2H), 4.11 (s, 3H), 3.70 (d, *J =* 8.8 Hz, 2H), 3.17 (s, 1H), 3.03 (s, 1H), 2.92 (d, J = 16.4 Hz, 1H), 2.26 (d, J = 2.2 Hz, 6H), 2.02 (s, 1H), 1.69 - 1.38 (m, 10H), 1.31 - 1.21 (m, 9H), 1.19 (m, 5H). |
| 431 | 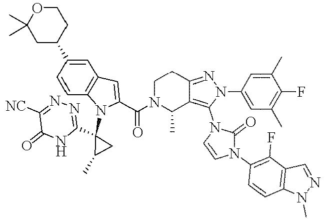 | LCMS (M+1): 919 |
| 432 | 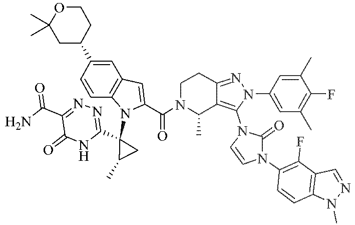 | LCMS (M+1): 937 |
| | | ¹HNMR (400 MHz, DMSO-d₆) *δ* 8.31 (s, 1H), 7.63 (d, J = 8.8 Hz, 1H), 7.54 (s, 1H), 7.48 (d, J = 7.6 Hz, 1H), 7.36 (d, J = 8.4 Hz, 1H), 7.25 (d, J = 8.8 Hz, 1H), 7.18 (d, J = 6.6 Hz, 2H), 7.08 (d, J = 3.2 Hz, 1H), 7.03 (s, 1H), 6.95 (s, 2H), 4.11 (s, 3H), 3.70 (d, J = 8.8 Hz, 2H), 3.17 (s, 1H), 3.03 (s, 1H), 2.92 (d, J = 16.4 Hz, 1H), 2.26 (d, J = 2.2 Hz, 6H), 2.02 (s, 1H), 1.69 - 1.38 (m, 7H), 1.31 - 1.21 (m, 9H), 1.19 (m, 5H). |
| 433 | 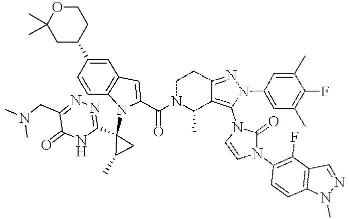 | LCMS (M+1): 951 |
| | | ¹HNMR (400 MHz, DMSO-d₆) δ 8.31 (s, 1H), 7.63 (d, J = 8.8 Hz, 1H), 7.54 (s, 1H), 7.48 (d, J = 7.6 Hz, 1H), 7.36 (d, J = 8.4 Hz, 1H), 7.25 (d, J = 8.8 Hz, 1H), 7.18 (d, J = 6.6 Hz, 2H), 7.08 (d, J = 3.2 Hz, 1H), 7.03 (s, 1H), 6.95 (s, 2H), 4.11 (s, 3H), 3.70 (d, J = 8.8 Hz, 2H), 3.17 (s, 1H), 3.03 (s, 1H), 2.92 (d, *J =* 16.4 Hz, 1H), 2.26 (d, J = 2.2 Hz, 8H), 2.02 (s, 1H), 1.69 - 1.38 (m, 13H), 1.31 - 1.21 (m, 9H), 1.19 (m, 5H). |
| 434 | 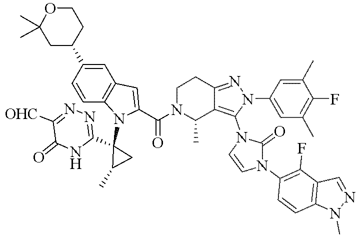 | LCMS (M+1): 922 |
| 435 | 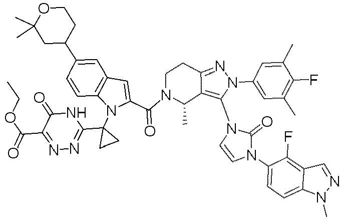 | LCMS (M+1): 952 |
| 437 | 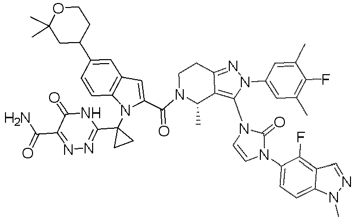 | LCMS (M+1): 951 |
| | | ¹HNMR (400 MHz, DMSO-d₆) *δ* 13.66 (s, 1H), 8.30 (s, 1H), 7.62 (d, J = 8.8 Hz, 1H), 7.56 (s, 1H), 7.46 (d, J = 8.4 Hz, 2H), 7.27-7.20 (m, 1H), 7.18 (d, J = 6.5 Hz, 3H), 7.05 (d, J = 3.1 Hz, 1H), 6.97 (s, 1H), 6.90 (d, J = 13.3 Hz, 1H), 5.45 (d, J = 6.8 Hz, 1H), 4.49 (d, J = 12.6 Hz, 1H), 4.10 (s, 4H), 3.70 (d, J = 8.8 Hz, 2H), 3.17 (s, 1H), 3.03 (s, 1H), 2.92 (d, J = 16.4 Hz, 1H), 2.26 (d, J = 2.2 Hz, 6H), 2.02 (s, 1H), 1.69-1.38 (m, 7H), 1.31-1.21 (m, 7H), 1.19 (m, 5H). |
| 438 | 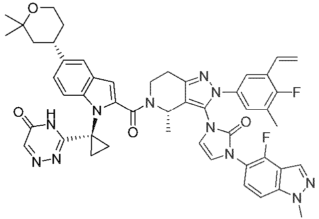 | LCMS (M+1): 892 |
| | | ¹H NMR (400 MHz, DMSO-d₆) *δ* 13.34 (s, 1H), 8.27 (d, J = 16.4 Hz, 1H), 7.70 - 7.59 (m, 1H), 7.56 (s, 1H), 7.42 (d, J = 8.2 Hz, 2H), 7.35 - 7.04 (m, 3H), 7.01 - 6.80 (m, 1H), 5.47 (d, J = 11.1 Hz, 1H), 4.48 (d, *J =* 13.8 Hz, 1H), 4.10 (s, 2H), 3.72 (d, J = 8.7 Hz, 2H), 3.12 - 2.57 (m, 2H), 2.28 (d, J = 7.4 Hz, 4H), 1.93 (d, J = 46.9 Hz, 1H), 1.76 - 1.47 (m, 3H), 1.36 (d, J = 6.6 Hz, 2H), 1.28 (s, 3H), 1.23 (s, 1H), 1.19 (s, 3H). |
| 439 | 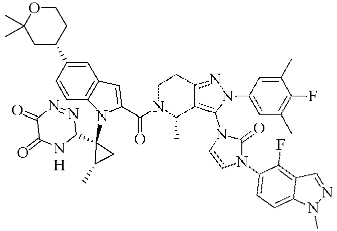 | LCMS (M+1): 910 |
| | | ¹HNMR (400 MHz, DMSO-d₆) *δ* 13.00 (s, 1H), 8.31 (s, 1H), 7.64 (d, J = 8.8 Hz, 1H), 7.59 (d, J = 9.2 Hz, 1H), 7.50 (d, J = 8.0 Hz, 2H), 7.28 (d, J = 8.8 Hz, 2H), 7.19 (d, J = 6.0 Hz, 1H), 7.08 (d, J = 3.1 Hz, 1H), 6.98 (d, J = 5.2 Hz, 1H), 6.52 (s, 1H), 4.11 (s, 3H), 3.70 (d, J = 8.8 Hz, 3H), 3.17 (s, 1H), 3.03 (s, 1H), 2.92 (d, J = 16.4 Hz, 1H), 2.26 (d, J = 2.4 Hz, 6H), 2.02 (s, 1H), 1.69 - 1.38 (m, 7H), 1.31 - 1.21 (m, 9H), 1.19 (m, 5H). |
| 440 | 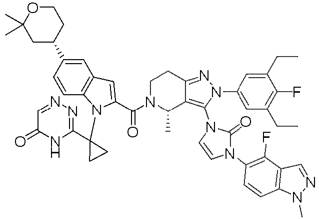 | LCMS (M+1): 908 |
| 441 | 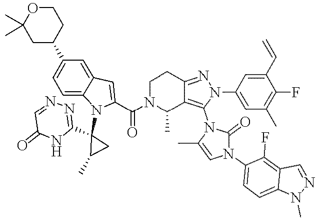 | LCMS (M+1): 920 |
| | | ¹H NMR (400 MHz, DMSO-d₆) *δ* 14.11 (s, 1H), 8.29 (s, 1H), 7.70 (s, 1H), 7.63 (d, J *=* 8.8 Hz, 1H), 7.53 (d, J = 8.8 Hz, 2H), 7.40 (d, J = 7.6 Hz, 1H), 7.37 (s, 1H), 7.29 - 7.22 (m, 2H), 7.06 (s, 1H), 6.83 (s, 1H), 5.82 (d, *J =* 17.6 Hz, 1H), 5.47 (d, J = 11.2 Hz, 1H), 4.11 (s, 3H), 3.69 (s, 2H), 2.98 (t, J *=* 18.8 Hz, 3H), 2.29 (s, 3H), 1.99 (d, J = 6.4 Hz, 1H), 1.80 (s, 3H), 1.67 (d, J = 18.0 Hz, 4H), 1.49 (d, J = 7.2 Hz, 6H), 1.28 - 1.16 (m, 12H). |
| 442 | 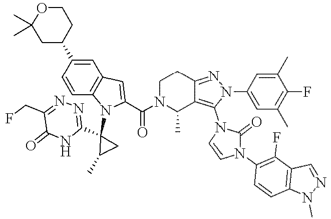 | LCMS (M+1): 926 |
| | | ¹HNMR (400 MHz, DMSO-d₆) *δ* 13.20 (d, J = 2.0 Hz, 1H), δ 8.31 (d, J = 0.8 Hz, 1H), 7.70 (s, 1H), 7.64 (d, J = 8.8 Hz, 1H), 7.58 - 7.44 (m, 1H), 7.38 (d, J = 8.4 Hz, 1H), 7.26 (dd, J = 8.8, 1.6 Hz, 1H), 7.19 (d, J = 6.4 Hz, 2H), 7.08 (d, J = 3.2 Hz, 1H), 7.03 (s, 1H), 6.96 (s, 1H). 4.11 (s, 3H), 4.09 (d, J = 2.4 Hz, 2H), 3.70 (d, J = 8.8 Hz, 2H), 3.17 (s, 1H), 3.03 (s, 1H), 2.92 (d, J = 16.4 Hz, 1H), 2.26 (d, J = 2.4 Hz, 6H), 2.02 (s, 1H), 1.69 - 1.38 (m, 7H), 1.31 - 1.21 (m, 9H), 1.19 (m, 5H). |
| 443 | 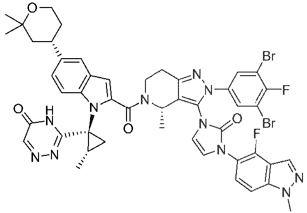 | LCMS (M+1): 1022 |
| | | ¹H NMR (400 MHz, DMSO-d₆) *δ* 14.09 (s, 0H), 8.30 (s, 1H), 7.76 (d, J = 5.4 Hz, 1H), 7.73 - 7.61 (m, 1H), 7.56 - 7.43 (m, 1H), 7.32 (dd, J = 48.6, 8.6 Hz, 1H), 7.22 - 6.97 (m, 2H), 5.67 (dd, J = 18.9, 6.8 Hz, 0H), 4.47 (d, J = 14.5 Hz, 1H), 4.11 (s, 2H), 3.70 (d, J = 9.0 Hz, 2H), 3.12 - 2.84 (m, 1H), 2.06 - 1.93 (m, 1H), 1.64 (d, J = 11.2 Hz, 2H), 1.58 - 1.40 (m, 3H), 1.26 (dd, J = 14.7, 11.7 Hz, 7H), 1.15 (d, J *=* 18.2 Hz, 3H). |
| 444 isomer 1 | 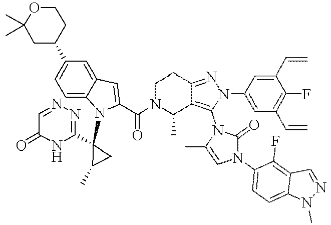 | LCMS (M+1): 932 |
| | | 1H NMR (400 MHz, DMSO-d6) *δ* 14.09 (s, 1H), 8.14 (d, J = 2.0 Hz, 1H), 7.65 (d, J = 9.2 Hz, 1H), 7.56 (d, J = 6.0 Hz, 1H), 7.50 (d, J = 7.2 Hz, 1H), 7.41 (s, 1H), 7.20 (s, 1H), 6.99 - 6.72 (m, 5H), 5.88 (d, J = 17.6 Hz, 2H), 5.52 (d, J = 11.2 Hz, 2H), 4.12 (s, 3H), 3.74 - 3.65 (m, 2H), 3.01 (d, J = 12.8 Hz, 2H), 2.67 (d, J = 1.6 Hz, 1H), 2.00 (d, J = 7.6 Hz, 1H), 1.80 - 1.06 (m, 26H). |
| 445 isomer 2 | 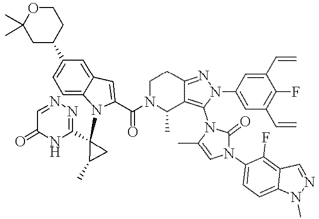 | LCMS (M+1): 932 |
| 446 | 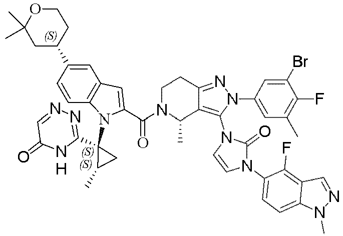 | LCMS (M+1): 958 |
| | | ¹H NMR (400 MHz, DMSO-d₆) *δ* 14.11 (s, 1H), 8.30 (s, 1H), 7.70 (s, 1H), 7.64 (d, J *=* 8.9 Hz, 1H), 7.54 (d, J = 4.0 Hz, 1H), 7.48 (d, J = 7.7 Hz, 1H), 7.43 - 7.36 (m, 2H), 7.26 (dd, J = 8.6, 1.9 Hz, 1H), 7.14 (d, J = 3.2 Hz, 1H), 7.06 - 7.01 (m, 2H), 5.63 (q, J = 6.6 Hz, 1H), 4.46 (d, J *=* 13.3 Hz, 1H), 4.11 (s, 3H), 3.73 - 3.68 (m, 3H), 3.06 - 2.89 (m, 3H), 2.34 (d, J = 2.2 Hz, 3H), 2.03 - 1.95 (m, 2H), 1.84 (q, J = 7.7 Hz, 1H), 1.64 (d, J = 9.4 Hz, 3H), 1.45 (dd, J = 8.3, 3.3 Hz, 4H), 1.27-1.24 (m, 6H), 1.17 (s, 3H). |
| 447 | 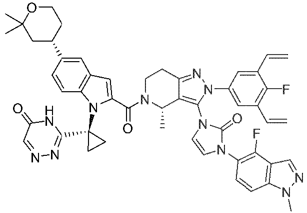 | LCMS (M+1): 904 |
| | | ¹H NMR (400 MHz, DMSO-d₆) *δ* 13.34 (s, 1H), 8.28 (s, 1H), 7.73 - 7.49 (m, 3H), 7.47 - 7.34 (m, 2H), 7.23 (dd, J = 8.7, 1.7 Hz, 1H), 7.17 - 7.01 (m, 1H), 6.99 - 6.82 (m, 2H), 5.91 (dd, J = 17.6, 11.3 Hz, 1H), 5.53 (dt, J = 23.3, 11.1 Hz, 2H), 4.49 (d, J = 13.3 Hz, 0H), 4.10 (s, 2H), 3.72 (d, J = 8.0 Hz, 1H), 3.57 (dd, J = 29.6, 16.9 Hz, 1H), 3.24 - 2.83 (m, 2H), 2.07 - 1.80 (m, 1H), 1.74 - 1.46 (m, 2H), 1.41 - 1.32 (m, 2H), 1.31 - 1.21 (m, 7H), 1.19 (s, 3H). |
| 448 | 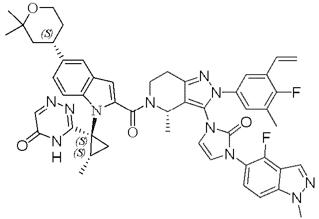 | LCMS (M+1): 906 |
| | | ¹H NMR (400 MHz, DMSO-d₆) *δ* 14.12 (s, 1H), 8.29 (d, J = 0.9 Hz, 1H), 7.70 (s, 1H), 7.63 (d, J = 8.9 Hz, 1H), 7.49 (dd, J = 6.3, 2.7 Hz, 1H), 7.47 - 7.40 (m, 1H), 7.38 (d, J = 8.5 Hz, 1H), 7.33 (dd, J = 6.4, 2.6 Hz, 1H), 7.26 (dd, J = 8.7, 1.7 Hz, 1H), 7.12 (d, J = 3.2 Hz, 1H), 7.03 (d, J = 5.0 Hz, 2H), 6.92 - 6.82 (m, 1H), 5.84 (d, J = 17.6 Hz, 1H), 5.63 (q, J = 6.5 Hz, 1H), 5.51 - 5.45 (m, 1H), 4.51 - 4.35 (m, 1H), 4.10 (s, 3H), 3.70 (d, J = 9.6 Hz, 3H), 3.06 - 2.89 (m, 2H), 2.30 (d, J = 2.1 Hz, 3H), 2.00 (dd, J = 8.0, 5.5 Hz, 1H), 1.83 (dt, J = 14.5, 7.5 Hz, 1H), 1.65 (d, J = 10.8 Hz, 3H), 1.46 (dd, J = 6.7, 3.7 Hz, 4H), 1.27 - 1.22 (m, 6H), 1.17 (s, 3H). |
| 449 | 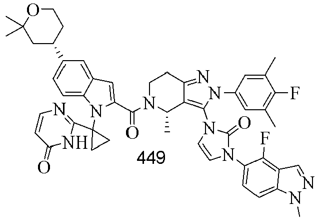 | LCMS (M+1): 879 |
| 450 | 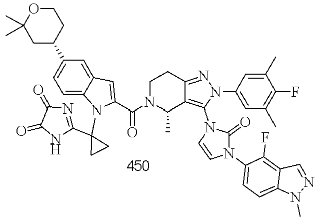 | LCMS (M+1): 881 |
| 451 | 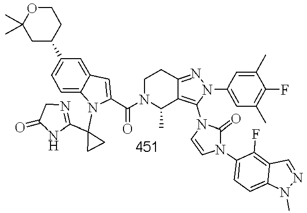 | LCMS (M+1): 867 |
| 452 | 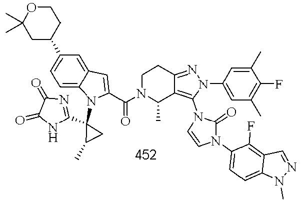 | LCMS (M+1): 895 |
| 453 | 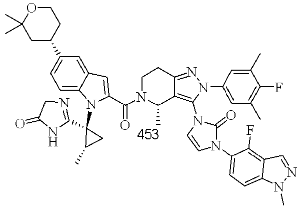 | LCMS (M+1): 881 |
| 454 | 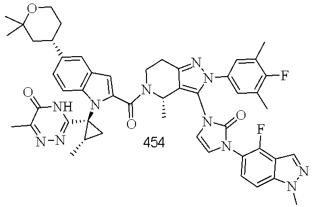 | LCMS (M+1): 908 |
| 455 | 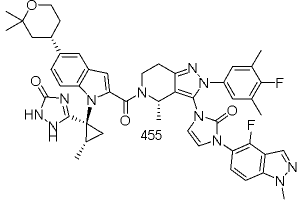 | LCMS (M+1): 882 |
| 456 | 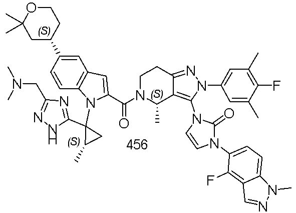 | LCMS (M+1): 923 |
| 457 | 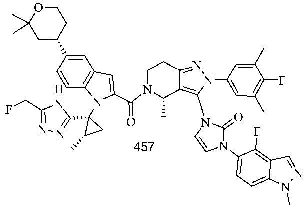 | LCMS (M+1): 898 |
| 458 | 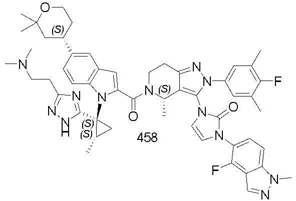 | LCMS (M+1): 937 |
| 459 | 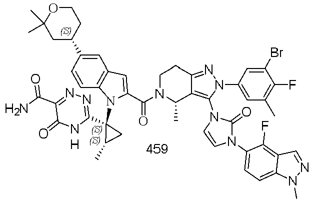 | LCMS (M+1): 1001 |
| 460 | 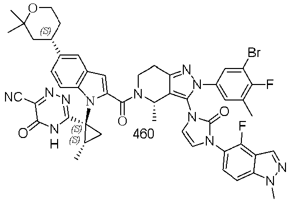 | LCMS (M+1): 983 |
| 461 | 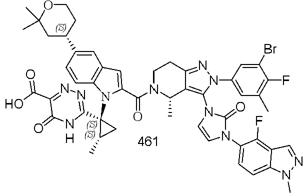 | LCMS (M+1): 1002 |
| 462 | 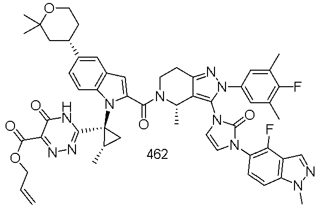 | LCMS (M+1): 978 |
| 463 | 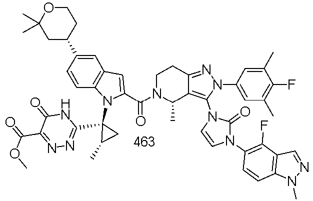 | LCMS (M+1): 952 |
| 464 | 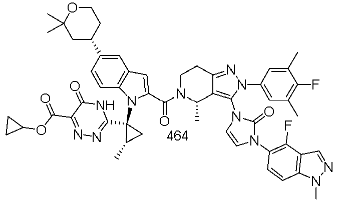 | LCMS (M+1): 978 |

### Example 34. Preparation method I of compound calcium salt:

NaOH (93mg, 1.2 eq) and water (1.23g, 0.7 W) were added to a glass reaction flask, and then stirred to dissolve, to which were successively added 95% ethanol (8.8 mL, 5V) and compound 302 (1.75 g), followed by stirring for dissolving. Then, an aqueous solution of calcium acetate (prepared by dissolving calcium acetate (213 mg, 0.68 eq) in water (1.05 g, 0.6W)) was added, followed by adding water (1 mL, 0.57 V) and a small amount of seed crystal. Water (9.5 mL, 5.4 V) was slowly added. The solution was stirred at room temperature for 4-6 h, filtered, and dried under reduced pressure at 45 °C to obtain 1.70 g of the calcium salt of compound 302.

The preparation process of calcium salts such as compound **R, 210,** and **401** was the same as that of calcium salt 302.

### Example 35. Preparation method II of compound calcium salt:

Compound **430** (1.75 g) and 95% ethanol (15 mL, 8.6V) were added to a glass reaction flask, and then stirred to dissolve, to which was added calcium acetate aqueous solution (prepared by dissolving calcium acetate (195 mg, 0.68 eq) in water (1.05 g, 0.6 W)), followed by slowly adding water (22 mL, 12.6V). The solution was stirred at room temperature for 16-20 h, filtered, and dried under reduced pressure at 45 °C to obtain 1.54 g of the calcium salt of compound 430.

The beneficial effects of the compounds according to the present invention were demonstrated by the following specific experimental examples.

### Experimental example 1: GLP-1R agonistic activity detection (Intracellular cAMP concentration detection)

The agonistic activity of test compounds on GLP-1R was assessed using HEK 293 cells stably expressing GLP-1R. Frozen cells were rapidly thawed in a 37 °C water bath and resuspended in HBSS buffer (Invitrogen, #14025). The cells were collected by centrifugation at 1,000 rpm for 5 min and resuspended in assay buffer. The cell count was determined using a Vi-cell cell counter (Beckman), and the cell density was adjusted to 1 × 10⁵ cells/mL. The cell suspension was seeded at 10 µL/well into white 384-well cell plates (PerkinElmer, #6007290). Test compounds (starting working concentration of 10 µM, 4-fold dilution, 10 concentration gradients, with duplicate wells) were added and incubated at 37°C with 5% CO₂ for 30 min. The intracellular cAMP concentration was measured using a cAMP detection kit (Cisbio, #62AM4PEJ) and an EnVision multi-mode microplate reader (PerkinElmer). The formula for calculating the agonistic activity of the test compound was as follows: Relative activity (%) = [(cAMP concentration_{test compound} - cAMP concentration_{negative control}) / (cAMP concentration_{positive control} - cAMP concentration_{negative control})] * 100%. The concentration of the test compound that produces 50% agonistic activity is the half-effective concentration EC₅₀.

The results are presented in Table 5.

### Experimental example 2: β-arrestin recruitment experiment

The effect of test compounds on **β-arrestin** 2 recruitment was assessed using GLP1R/β-Arrestin/CHO reporter cells (COBIOER, #CBP71420). The reporter cell line was seeded at 20,000 cells per well in a 96-well cell culture plate (LABSELECT, #11515) using complete medium (COBIOER, #CBP71420M). After overnight incubation, the complete medium was replaced with Opti-MEM medium (Thermo Fisher, #31985062), and test compounds (starting working concentration of 10 µM, 4-fold dilution, 9 concentration gradients, with duplicate wells) were added and incubated at 37°C with 5% CO₂ for 30 min, with a total volume of 100 µL. After the incubation, 25 µL of Nano-Glo detection reagent (Promega, N2012) was added to each well, followed by shaking at 300 rpm for 3 min at room temperature. The chemiluminescence value was then read using a multi-function microplate reader (FLUOstar Omega). The formula for calculating the effect of the test compound on **β-arrestin** 2 recruitment is as follows: Relative activity (%) = [(chemiluminescence value_{test compound} - chemiluminescence value_{negative control}) / (chemiluminescence value_{positive control} - chemiluminescence value_{negative control})] * 100%. The concentration of the test compound that produces 50% recruitment activity is the half-maximal effective concentration (EC₅₀).

The results are presented in Table 5.

### Experimental example 3: Method for detecting downstream transcriptional activity of GLP-1R

The effect of test compounds on the downstream transcriptional activity of human GLP-1R was assessed using the HEK 293 GLP-1R/Cre-Luc luciferase reporter gene cell line (COBIOER, #CBP71117). The reporter cell line was seeded at 20,000 cells per well in a 96-well cell culture plate (LABSELECT, #11515) with complete medium (DMEM/HIGH GLUCOSE +10% FBS+1% P/S +400 µg/mL G418, wherein DMEM/HIGH GLUCOSE (HyClone, SH30243.01), FBS (Biological Industries, 04-001-1ACS), P/S (Gibco, 15140-122), G418 (Gibco, 10131-035)). After overnight incubation, the complete medium was replaced with Opti-MEM medium (Thermo Fisher, #31985062), and test compounds (initial working concentration of 100 nM, 4-fold dilution, 9 concentration gradients, with duplicate wells) were added for 6 hours of incubation at 37 °C with 5% CO₂, in a total volume of 100 µL. After the incubation was completed, 100 µL of Bright-Glo assay reagent (Promega, E2620) was added to each well, shaken at 300 rpm for 3 min at room temperature, and then read the chemiluminescence value using a multi-function microplate reader (FLUOstar Omega). The formula for calculating the effect of the test compound on transcriptional activity is as follows: Relative activity (%) = [(chemiluminescence value of test compound - chemiluminescence value of negative control) / (chemiluminescence value of positive control - chemiluminescence value of negative control)] * 100%.

The results are presented in Table 5.

**Table 5. Experimental results of GLP-1R activation, β-arrestin 2 recruitment, and downstream transcriptional activity of GLP-1R**

| Compound No. | GLP-1R EC50(nM) | **β-arrestin 2** EC 50(nM) | GLP-1R/Cre-Luc EC 50 (nM) |
|---|---|---|---|
| R | 7.244 | >10000 | 0.26 |
| 2 | >10000 | >10000 | |
| 18 | 253.4 | >10000 | |
| 19 | NA | >10000 | >1000 |
| 30 | 9.911 | >10000 | |
| 31 | 73.37 | >10000 | 29.27 |
| 41 | 1.46 | >10000 | |
| 42 | 6.2 | >10000 | 7.8 |
| 59 | NA | >10000 | |
| 60 | NA | >10000 | >1000 |
| 71 | 92.48 | >10000 | |
| 72 | NA | >10000 | >1000 |
| 118 | 10.66 | >10000 | |
| 122 | 36.44 | >10000 | |
| 123 | 2.425 | >10000 | |
| 125 | 15.2 | >10000 | |
| 126 | 9.34 | >10000 | 2.59 |
| 127 | 7.416 | >10000 | 1.71 |
| 128 | 19.62 | >10000 | 8.15 |
| 129 | 71.7 | >10000 | 8.94 |
| 130 | 6.25 | >10000 | 1.25 |
| 131 | NA | | >100 |
| 132 | 26.17 | >10000 | 5.32 |
| 133 | 10.52 | >10000 | 42.22 |
| 134 | NA | >10000 | 67.98 |
| 135 | NA | >10000 | >100 |
| 136 | 26.63 | >10000 | 0.22 |
| 137 | 123.5 | >10000 | 4.87 |
| 138 | 59.2 | >10000 | 4.01 |
| 139 | 83.38 | >10000 | 3.92 |
| 140 | NA | >10000 | >100 |
| 141 | 27 | >10000 | 2.48 |
| 201 | 16.02 | >10000 | 4.788 |
| 202 | 141.7 | >10000 | 11.68 |
| 203 | 49.96 | >10000 | 8.278 |
| 204 | 32.43 | >10000 | 7.892 |
| 205 | NA | >10000 | 34.4 |
| 206 | 27.82 | >10000 | 1.086 |
| 207 | 96.15 | >10000 | 13.44 |
| 208 | 33.77 | >10000 | 0.38 |
| 209 | 200 | >10000 | 2.64 |
| 210 | 3.443 | >10000 | 0.068 |
| 211 | 28.89 | >10000 | 0.014 |
| 212 | 18.55 | >10000 | 0.15 |
| 213 | 410.6 | >10000 | 1.38 |
| 214 | 11.88 | >10000 | 0.8 |
| 215 | 4.254 | >10000 | 0.04 |
| 216 | NA | >10000 | 83.66 |
| 217 | NA | >10000 | 0.66 |
| 218 | 26.68 | >10000 | 3.69 |
| 219 | 20.77 | | |
| 220 | NA | | |
| 221 | 77.32 | | |
| 222 | 23.21 | >10000 | 0.041 |
| 223 | 20.5 | >10000 | 0.097 |
| 224 | 42.26 | >10000 | 0.262 |
| 225 | 59.55 | >10000 | 0.24 |
| 226 | 46.27 | >10000 | 0.88 |
| 227 | 4.24 | >10000 | 2.41 |
| 228 | 5.242 | >10000 | 0.17 |
| 229 | 31.55 | >10000 | 0.31 |
| 230 | 20.83 | >10000 | 3.13 |
| 232 | 26.68 | >10000 | 0.66 |
| 301 | 14.19 | >10000 | 0.104 |
| 302 | 4.83 | >10000 | 6.8 |
| 313 | 25.5 | >10000 | 6.55 |
| 322 | 6.645 | | |
| 324 | 231.2 | >10000 | 13.82 |
| 401 | 9.075 | >10000 | 0.11 |
| 406 | 4.27 | >10000 | 0.3 |
| 413 | 176.43 | >10000 | 1.95 |
| 415 | 118.49 | >10000 | 0.73 |
| 419 | 3.14 | >10000 | 0.24 |
| 420 | 78.06 | >10000 | 3.96 |
| 427 | 7.05 | >10000 | 1.91 |
| 428 | 89.43 | >10000 | 1.76 |
| 429 | 87.74 | >10000 | 1.98 |
| 430 | 3.2 | >10000 | 0.11 |
| 433 | 3.14 | >10000 | 0.57 |
| 437 | | >10000 | 1.82 |
| 438 | 14.08 | >10000 | 1.12 |
| 439 | 31.86 | >10000 | 0.43 |
| 440 | 22.23 | >10000 | 0.76 |
| 441 | 32.74 | >10000 | 2.78 |
| 442 | | >10000 | 2.68 |
| 443 | 6.2 | >10000 | 0.22 |
| 444 | | >10000 | 13.82 |
| 445 | | >10000 | 69.59 |
| 446 | 5.45 | >10000 | 0.32 |
| 447 | | >10000 | 0.47 |
| 449 | 90.74 | >10000 | 8.15 |
| 450 | 758.6 | >10000 | >100 |
| 451 | 52.99 | >10000 | 12.54 |
| 452 | 2434 | >10000 | >100 |
| 453 | 84.47 | | |
| 454 | 322.81 | >10000 | 8.07 |
| 455 | 50.96 | >10000 | 2.47 |
| 456 | 26.14 | >10000 | 2.06 |
| 457 | | >10000 | 2.05 |
| 458 | | >10000 | 88.31 |
| 459 | 3.342 | >10000 | 0.051 |
| 460 | 8.557 | >10000 | 0.19 |
| 461 | 24.18 | >10000 | 0.88 |
| 462 | 5.007 | >10000 | 0.21 |
| 463 | 4.26 | >10000 | 0.4 |
| 464 | 1.94 | >10000 | 0.085 |

The data indicated that some compounds of the present invention exhibited comparable or superior agonistic activity and selectivity towards GLP-1R compared to the reference compounds.

### Experimental example 4: Liver microsome stability experiment

(1) 100 µL of 2 mg/mL liver microsomal working solution (adding 50 µL of 0.8 mg/mL liver microsomal working solution for the positive control) was transfered to a 96-well plate, to which was added 196 µL of 6 mM MgCl₂ working solution (adding 98 µL of MgCl₂ working solution for the positive control), followed by adding 4 µL of the test compound working solution (adding 2 µL of working solution for the positive control). The solution was mixed well to obtain the mixed solution of the incubation system, in duplicate;
(2) The incubation system was pre-incubated in a 37 °C water bath shaker for 5 min;
(3) Control group (without NADPH): 120 µL of mixed solution from the incubation system was added with 40 µL of PB, and two parallel samples were prepared. The solution was vortexed for 30 seconds and mixed well. Then, the solution was placed in a 37 °C water bath shaker for incubation, and timing began. At 0, 30, and 60 min, respectively, 20 µL of reaction solution was taken out from the system and transferred to a 96-well deep plate, followed by adding 275 µL of stop solution;
(4) Sample group (with NADPH): 120 µL of mixed solution from the incubation system was added with 40 µL of 4 mM NADPH solution, and two replicates were prepared. The solution was vortexed to mix well, and then placed in a 37°C water bath shaker for incubation. Timing began, and at 0, 5, 15, 30, 45, and 60 min, 20 µL of reaction solution from the system was collected and transferred to a 96-well deep plate, followed by adding 275 µL of stop solution;
(5) Positive group (including NADPH): 120 µL of mixed solution from the incubation system was added with 40 µL of 4 mM NADPH solution, and two parallel samples were prepared. The solution was vortexed to mix evenly, and then placed in a 37°C water bath shaker for incubation. Timing began, and at 0 and 60 min, respectively, 20 µL of reaction solution from the system was collected and transferred to a 96-well plate, followed by adding 275 µL of stop solution;
(6) After vortexing all samples for 2 min, the plate was centrifuged at 3800 rpm for 15 min at 4 °C;
(7) 140 µL of supernatant was collected, to which was added 70 µL of ultrapure water, and then vortexed to mix well, that was subjected to LC/MS/MS analysis.

Use the following first-order kinetic formula to calculate the half-life (t_{1/2}) and clearance (CL) ${C}_{t} = {C}_{0} * {e}^{- kt}$ ${t}_{1 / 2} = ln 2 / k = 0.693 / k$ $CL = {V}_{d} * k$ ${V}_{d} = 1 / Protein content in liver microsomes$ 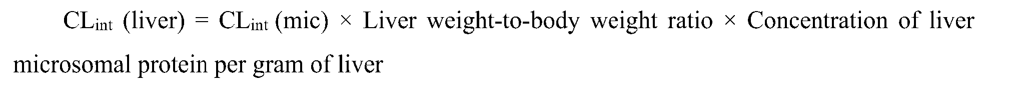 CLint(liver) = CLint (mic) × Liver weight-to-body weight ratio × Concentration of liver microsomal protein per gram of livermicrosomal protein per gram of liver

The experimental results are presented in Table 6:

**Table 6. Results of liver microsomal stability experiment**

| Compound No. | **LM T_{1/2} (min)** | | | |
|---|---|---|---|---|
| | Human | Mice | Rat | Monkey |
| R | >120 | >120 | >120 | 72.2 |
| 210 | >120 | >120 | >120 | 74.5 |
| 430 | >120 | >120 | >120 | >120 |
| 302 | >120 | >120 | >120 | 117 |
| 401 | 70.7 | 70.7 | >120 | 79.7 |

Compared to the reference compound, the compound of the present invention exhibited superior liver microsomal stability

### Experimental example 5: Pharmacokinetic experiment

Six healthy adult male SD rats, ICR mice, or cynomolgus monkeys were subjected to intravenous injection and gavage administration, with three animals in each group for each administration way. The intravenous injection group was allowed to eat and drink freely, while the gavage group was fasted overnight (>12 hours) but allowed to drink water. Animals were allowed to eat 4 hours after administration. At different time points after administration, blood was collected intravenously and anticoagulated with EDTA-K2 (the blood samples were placed in an ice bath after collection). The blood was then centrifuged at 4°C and 6000 g for 5 minutes to separate plasma, which was stored at -70 °C for subsequent testing. The concentration of compounds in plasma was determined using LC-MS/MS.

The compound concentration data in plasma at all time points were used to calculate the main pharmacokinetic parameters using the Winnolin 8.3 non-compartmental model.

The area under the plasma concentration-time curve (AUC) value: it was calculated using the trapezoidal rule; AUC_{inf} = AUCₐₗₗ + Cₜ/ke, where Ct is the plasma concentration at the last measurable time point and ke is the elimination rate constant; $Elimination half - life time {t}_{1 / 2} = 0.693 / ke ;$ $Clearance rate \left(CL\right) = D / {AUC}_{inf} \left(D represents the administered dose\right) ;$ 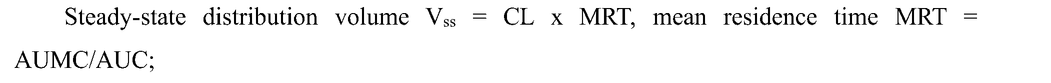 Steady-state distribution volume Vss = CL x MRT, mean residence time MRT = AUMC/AUC; $Absolute bioavailability F = \left({AUC}_{i . g .}\times {D}_{i . v .}\right) / \left({AUC}_{i . v .}\times {D}_{i . g .}\right) \times 100 %$

**Table 7. Pharmacokinetic data of rats**

| | | Reference compound R | 210 | 215 | 302 |
|---|---|---|---|---|---|
| iv (1 mpk) | AUCₐₗₗ (ng*h/mL) | 4579 | 10837 | 4948 | 28426 |
| | t_{1/2} (h) | 8.09 | 13.7 | 6.22 | 15.8 |
| | CL (mL/min/kg) | 3.69 | 1.65 | 3.42 | 0.651 |
| | Vss (mL/kg) | 1.91 | 1.65 | 1.62 | 0.817 |
| ig (3 mpk) | AUCₐₗₗ (ng*h/mL) | 6001 | 13978 | 3889 | 45174 |
| | t_{1/2} (h) | 8.91 | 13.4 | 6.68 | **13.9** |
| | F (%) | 43.7 | 43.9 | 26.2 | 55 |

The reference compound R is LY3502970, with the structure:

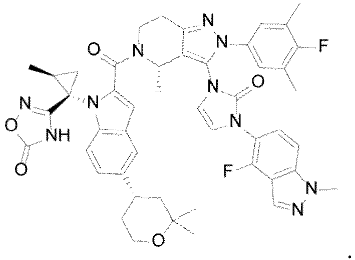

**Table 8. Pharmacokinetic data of cynomolgus monkeys**

| | | Reference compound R (Ca salt) | 430 (Ca salt) |
|---|---|---|---|
| iv | AUCₐₗₗ (ng*h/mL) | 408 | 382 |
| (0.15 mpk) | t_{1/2} (h) | 4.48 | 2.98 |
| | CL (mL/min/kg) | 0.37 | 0.41 |
| | Vss (mL/kg) | 1.46 | 1.18 |
| ig (3 mpk) | AUCₐₗₗ (ng*h/mL) | 1422 | 2740 |
| | t_{1/2} (h) | 5.18 | 5.75 |
| | F (%) | 17.4 | 35.7 |

The above results indicated that the compound of the present invention exhibited good pharmacokinetics.

### Experimental example 6: Solubility experiment

30 µL of 10 mM stock solution of the test compound and the control drug was added to the corresponding position of the 96-well plate in the specified order. 970 µL of FaSSGF, FaSSIF, or PBS pH 7.4 was respectively added to the corresponding vials of the sample plate. The experiment was conducted in duplicate. A stirrer was added to each vial, which was then covered with a polytetrafluoroethylene/silicone organic resin stopper. Then, the sample plate was placed into the Eppendorf Thermomixer Comfort and shaken at 1100 rpm at 25 °C for 2 h. After 2 hours, the stopper was removed, and a magnet was used to remove the stirrer. Then, the sample was transferred from the sample plate to the filter plate. The sample was filtered by using a vacuum pump to generate negative pressure. 5 µL of the filtrate was transferred to a new sample plate, to which were then added 5 µL of DMSO and 490 µL of acetonitrile-water (V:V=1:1). 200 µL of the diluent was transferred to a new 96-well plate for LC-MS/MS analysis. The dilution ratio may be adjusted based on the solubility of the analyte or the strength of its liquid-mass response signal. The sample plate was placed into the sample tray of the autosampler, to evaluate the sample by liquid-mass analysis. All calculations were performed using Microsoft Excel.

The analysis and quantification of the sample filtrate were accomplished by using liquid chromatography-mass spectrometry (LC-MS) to identify and quantify the peaks of known concentrations of standard samples.

**Table 9. Solubility data**

| Compound No. | Solubility (uM) |
|---|---|
| | FaSSGF/FaSSIF/PBS h 7.4 |
| R | <0.056/ **0.66**/ <0.055 |
| 210 | <0.016/ **32.88** / *<0.015* |
| 302 | <0.032/ **245.21** / <0.032 |
| 401 | <0.0069/ **65.02** / <0.0066 |
| 430 | <0.24/ **285.1** / <0.24 |

As shown in Table 9, the compound of the present invention had excellent solubility and even more excellent pharmaceutical properties.

The present invention provided a new class of small molecule compounds. The small molecule compounds of the present invention could serve as GLP-1 receptor agonists for the manufacture of medicaments for preventing and/or treating diseases such as diabetes, obesity, fatty liver, and polycystic ovary syndrome. Compared to LY3502970, the compounds of the present invention exhibited superior pharmaceutical properties, specifically manifested in better pharmacokinetic (PK) properties, improved oral bioavailability, reduced toxicity, enhanced permeability, and improved efficacy. Therefore, the compounds of the present invention held broad application prospects.

## Claims

1. Compounds of Formula I, their pharmaceutically acceptable salts, or their stereoisomers: 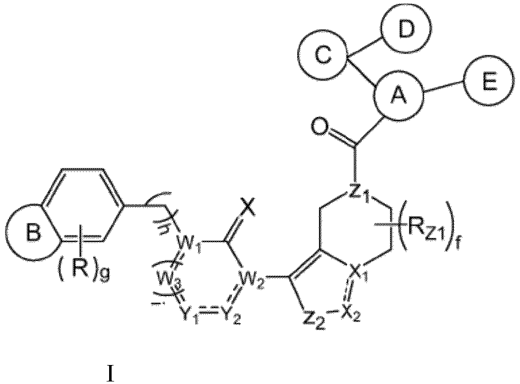 wherein,
Ring A is selected from aryl, heteroaryl, fused cycloalkyl, and heterofused cycloalkyl groups;
The E ring is selected from substituted or unsubstituted 3-10 membered cycloalkyl groups and substituted or unsubstituted 3-10 membered heterocycloalkyl groups;
The C ring is selected from substituted or unsubstituted 3-10 membered cycloalkyl groups and substituted or unsubstituted 3-10 membered heterocycloalkyl groups;
The D ring is selected from substituted or unsubstituted aryl groups and substituted or unsubstituted heteroaryl groups;
Z₁ is selected from N and CH;
f is the number of substituents R_{Z1}, an integer selected from 0 to 6;
R_{Z1} are each independently selected from hydrogen, substituted or unsubstituted C₁∼C₈ alkyl, substituted or unsubstituted C₁∼C₈ alkoxy, halogen; or two adjacent R_{Z1} are connected to form a 3-10 membered cycloalkyl, a 3-10 membered heterocycloalkyl; or two non-adjacent R_{Z1} are connected to form 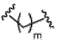 where m is 1, 2 or 3;
X₁ is selected from N or C; X₂ is selected from N or CH₂; and there is one and only one N in X₁ and X₂; the dashed line between X₁ and X₂ is none or a bond depending on the selection of X₁ and X₂;
Z₂ is selected from NR_{z2} and CHR_{z2};
R_{z2} is selected from substituted or unsubstituted aryl groups and substituted or unsubstituted heteroaryl groups;
X is selected from O and S;
i is selected from 0 or 1;
W₁ and W₂ are each independently selected from N or C; W₃ is selected from CH or N; and at least one of W₁, W₂, and W₃ is N;
Y₁ and Y₂ are each independently selected from C=O, C=S, N or CR₁₄; R₁₄ are each independently selected from hydrogen, substituted or unsubstituted C₁ ~ C₈ alkyl, substituted or unsubstituted C₁~C₈ alkoxy, halogen, hydroxyl, carboxyl, substituted or unsubstituted 3- to 10-membered cycloalkyl;
The dashed lines between W₁, W₂, W₃, Y₁, and Y₂ are either none or keys depending on the selection of W₁, W₂, W₃, Y₁, and Y₂.
h is selected from integers from 0 to 3;
g is the number of substituents R, which is an integer selected from 0 to 3;
R is independently selected from hydrogen, substituted or unsubstituted C₁∼C₈ alkyl, substituted or unsubstituted C₁~C₈ alkoxy, and halogen;
Ring B is selected from unsubstituted, substituted or unsubstituted aryl groups, and substituted or unsubstituted heteroaryl groups;
The substituents of the alkyl, alkenyl, alkynyl, alkoxy, aldehyde, amino, ester, and amide groups are selected from the following groups: deuterium, halogen, hydroxyl, cyano, C₁∼C₈alkyl, halogenated C₁~C₈alkyl, C₁~C₈alkoxy, halogenated C₁~C₈alkoxy, substituted or unsubstituted 3- to 10-membered cycloalkyl, substituted or unsubstituted 3- to 10-membered heterocycloalkyl, substituted or unsubstituted aryl, substituted or unsubstituted heteroaryl, - C(O)R₁', -NR₂'R₃';
The substituents of the cycloalkyl, heterocycloalkyl, aryl, and heteroaryl groups are selected from the following groups: substituted or unsubstituted C₁-C₈ alkyl, halogen, cyano, hydroxyl, substituted or unsubstituted C₁-C₈ alkoxy, substituted or unsubstituted C₂-C₈ alkenyl, substituted or unsubstituted C₂-C₈ alkynyl, substituted or unsubstituted 3- to 10-membered cycloalkyl, substituted or unsubstituted, substituted 3- to 10-membered heterocyclic alkyl group, substituted or unsubstituted C₁ to C₈ ester group, substituted or unsubstituted C₁ to C₈ amide group, amino group, carboxyl group, substituted or unsubstituted C₁ to C₈ aldehyde group, substituted or unsubstituted C₁ to C₈ amino group, =O; or the substituents on the same carbon atom of the cycloalkyl, heterocyclic alkyl, aryl, or heteroaryl group form =O or =S;
R₁', R₂', and R₃' are independently selected from hydrogen and C₁-C₈alkyl groups, respectively;
The compound is not:

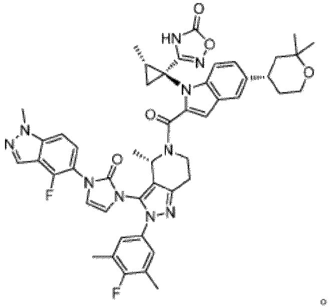

2. The compound according to claim 1, its pharmaceutically acceptable salt, or its stereoisomer, is **characterized in that**: the compound is as shown in Formula I-1: 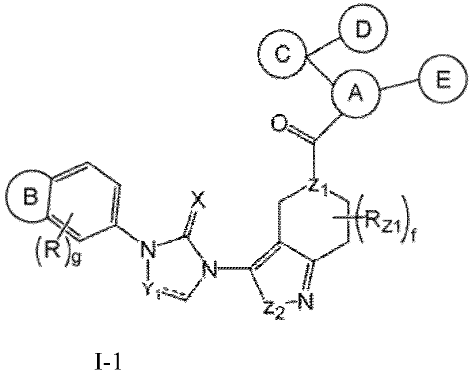 wherein,
Ring A is selected from aryl, heteroaryl, fused cycloalkyl, and heterofused cycloalkyl groups;
The E ring is selected from substituted or unsubstituted 3-8 membered saturated cycloalkyl groups, substituted or unsubstituted 3-8 membered saturated heterocycloalkyl groups; each substituent is independently selected from C₁~C₈ alkyl, C₁~C₈ alkoxy, halo-C₁~C₈ alkyl, halo-C₁~C₈ alkoxy, and halogen;
The C ring is selected from substituted or unsubstituted 3-8 membered saturated cycloalkyl groups, substituted or unsubstituted 3-8 membered saturated heterocycloalkyl groups; each substituent is independently selected from C₁~C₈alkyl, C₁~C₈alkoxy, halo-C₁~C₈alkyl, halo-C₁~C₈alkoxy, and halogens;
The D ring is selected from aryl and heteroaryl groups;
Z₁ is selected from N and CH;
f is an integer selected from 0 to 6;
R_{Z1} are each independently selected from hydrogen, C₁~C₈ alkyl, C₁~C₈ alkoxy, halo-C₁~C₈ alkyl, halo-C₁~C₈ alkoxy, halogen; or two adjacent R_{Z1} are connected to form a 3-8 membered saturated cycloalkyl, a 3-8 membered saturated heterocycloalkyl; or two non-adjacent R_{Z1} are connected to form 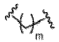 where m is 1, 2 or 3;
Z₂ is selected from NR_{z2} and CHR_{z2};
R_{z2} is selected from substituted or unsubstituted aryl groups and substituted or unsubstituted heteroaryl groups; each of the substituents is independently selected from C₁~C₈ alkyl, C₁~C₈ alkoxy, halo-C₁~C₈ alkyl, halo-C₁~C₈ alkoxy, halogen, 3-8 member saturated cycloalkyl, and 3-8 member saturated heterocycloalkyl.
X is selected from O and S;
- is selected from - or =;
provided that - is -, Y1 represents C=O or C=S;
provided that = is =, Y₁ represents N or CR₁₄; Y₁ is selected from N or CR₁₄; R₁₄ is selected from hydrogen, C₁~C₈alkyl, C₁~C₈alkoxy, halo-C₁~C₈alkyl, halo-C₁~C₈alkoxy, halogen, hydroxyl;
g is an integer selected from 0 to 3;
R is independently selected from hydrogen, C₁~C₈alkyl, C₁~C₈alkoxy, halo-C₁~C₈alkyl, halo-C₁~C₈alkoxy, and halogen;
Ring B is selected from substituted or unsubstituted aryl groups, substituted or unsubstituted heteroaryl groups; each substituent is independently selected from C₁~C₈alkyl, deuterated C₁~ C₈alkyl, halosubstituted C₁ halosubstituted C₁~C₈alkoxy, and halogen;
The compound is not:

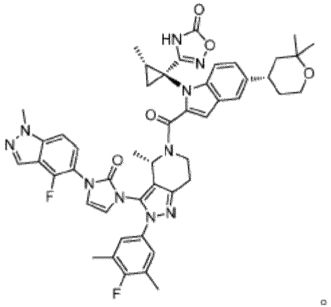

3. The compound according to claim 2, its pharmaceutically acceptable salt, or its stereoisomer, is **characterized in that**: the structure of the compound is as shown in Formula II-1: 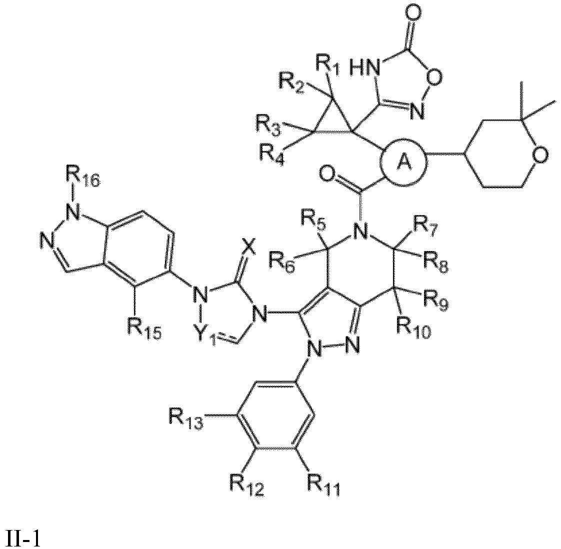 wherein,
A is 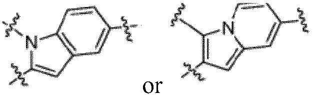
R₁ is selected from hydrogen, C₁~C₅ alkyl groups, and halogens;
R₂ is selected from hydrogen, C₁~C₅ alkyl, and halogen;
R₃ is selected from hydrogen, C₁~C₅ alkyl groups, and halogens;
R₄ is selected from hydrogen, C₁~C₅ alkyl groups, and halogens;
R₅ is selected from hydrogen, C_{1~}C₅ alkyl groups, and halogens;
R₆ is selected from hydrogen, C_{1~}C₅ alkyl groups, and halogens;
R₇ is selected from hydrogen, C_{1~}C₅ alkyl groups, and halogens;
R₈ is selected from hydrogen, C_{1~}C₅ alkyl groups, and halogens;
R₉ is selected from hydrogen, C_{1~}C₅ alkyl groups, and halogens;
R₁₀ is selected from hydrogen, C_{1~}C₅ alkyl groups, and halogens;
Alternatively, R₅ and R₆ can be linked to form a 3-6 member saturated cycloalkyl group;
Alternatively, R₅ and R₉ can be connected to form 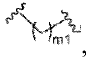 where m1 is 1, 2, or 3;
Alternatively, R₅ and R₇ can be connected to form 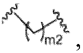 where m2 is 1, 2, or 3;
R₁₁ is selected from hydrogen, C₁~C₅ alkyl, halogen, and 3-6 membered saturated cycloalkyl;
R₁₂ is selected from hydrogen, C₁~C₅ alkyl, halogen, and 3-6 membered saturated cycloalkyl;
R₁₃ is selected from hydrogen, C₁~C₅ alkyl, halogen, and 3-6 membered saturated cycloalkyl;
X is selected from O and S;
- is selected from - or =;
provided that - is -, Y1 represents C=O or C=S;
provided that - is =, Y₁ is N or CR₁₄; R₁₄ is selected from hydrogen, C_{1~}C₅ alkyl, halogen,
hydroxyl;
R₁₅ is selected from hydrogen, C₁~C₅ alkyl groups, and halogens;
R₁₆ is selected from C_{1~}C₅ alkyl and deuterated C₁~C₅ alkyl.

4. The compound according to claim 3, its pharmaceutically acceptable salt, or its stereoisomer, is **characterized in that**: the structure of the compound is as shown in Formula III-1: 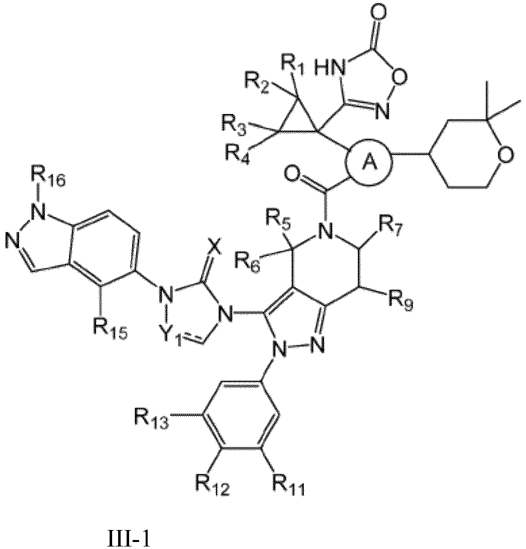 wherein,
A is 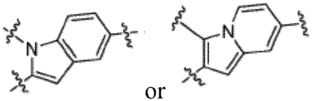
R₁ is selected from hydrogen, C₁~C₃ alkyl groups, and halogens;
R₂ is selected from hydrogen, C₁~C₃ alkyl groups, and halogens;
R₃ is selected from hydrogen, C₁~C₃ alkyl groups, and halogens;
R₄ is selected from hydrogen, C₁~C₃ alkyl, and halogen;
R₅ is selected from hydrogen, C₁~C₃ alkyl, and halogen;
R₆ is selected from hydrogen, C₁~C₃ alkyl groups, and halogens;
R₇ is selected from hydrogen, C₁~C₃ alkyl groups, and halogens;
R₉ is selected from hydrogen, C₁~C₃ alkyl groups, and halogens;
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
- is selected from - or =;
provided that - is -, Y1 represents C=O or C=S;
provided that 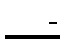 is =, Y₁ is N or CR₁₄; R₁₄ is selected from hydrogen, C₁~C₃ alkyl, halogen, hydroxyl;
R₁₅ is selected from hydrogen, C₁~C₃ alkyl groups, and halogens;
R₁₆ is selected from C₁~C₃ alkyl and deuterated C₁~C₃ alkyl.

5. The compound according to claim 3, its pharmaceutically acceptable salt, or its stereoisomer, is **characterized in that**: the structure of the compound is as shown in formula III-1-1, III-2-1, III-3-1, III-4-1, III-5-1, III-6-1, III-7-1, III-8-1, III-9-1, or III-10-1. 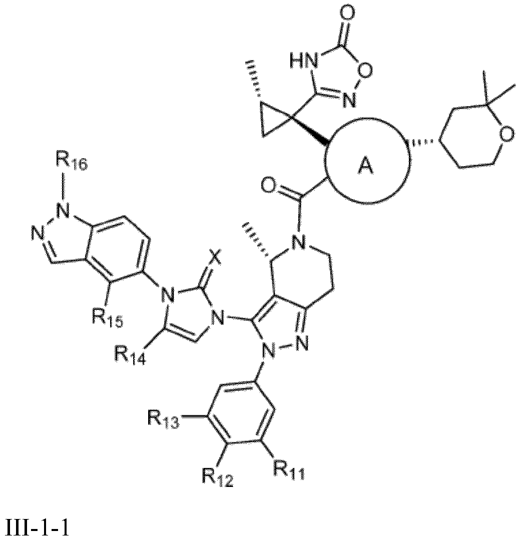 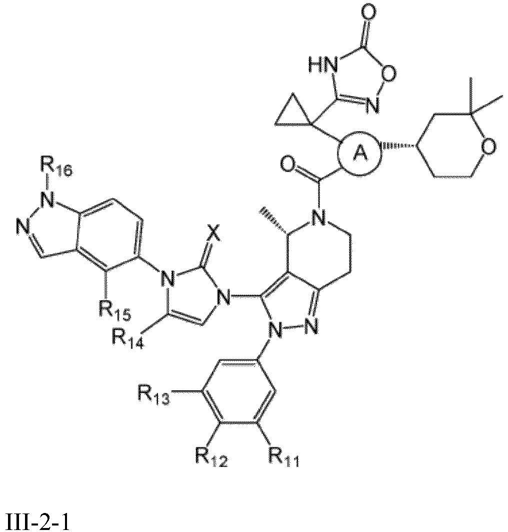 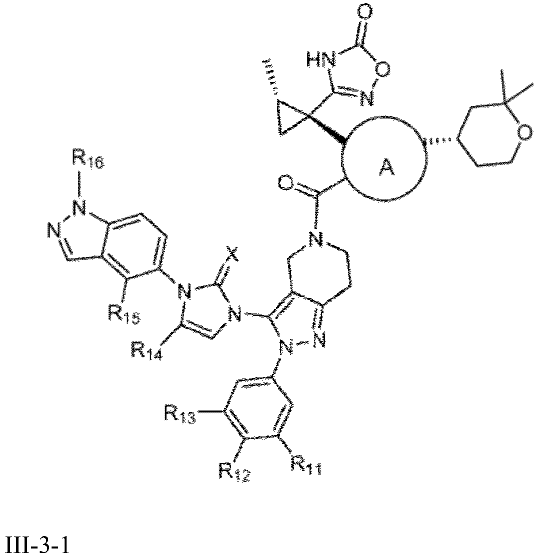 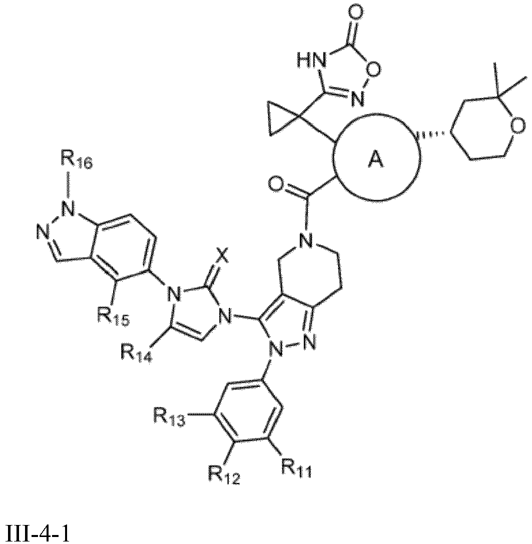 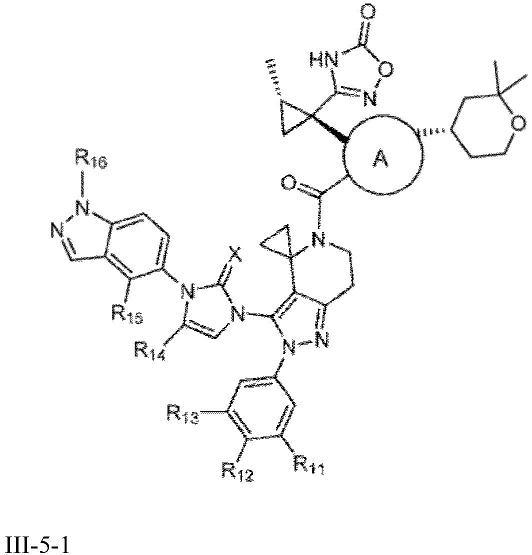 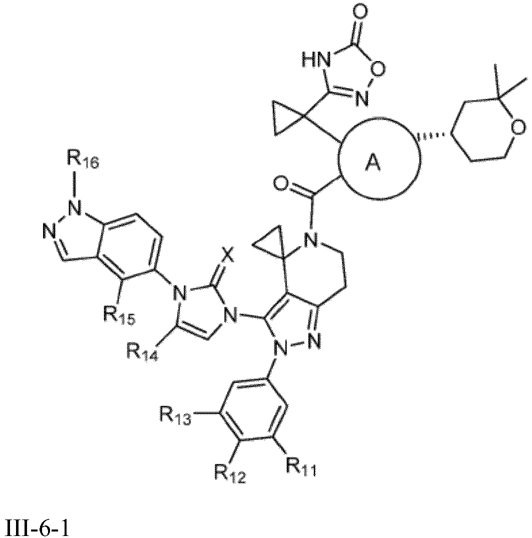 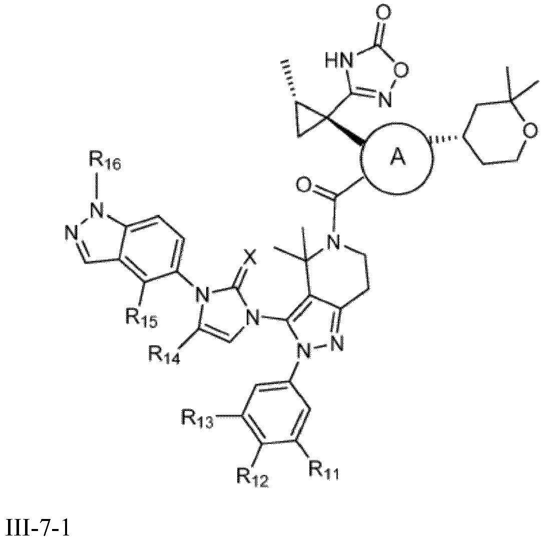 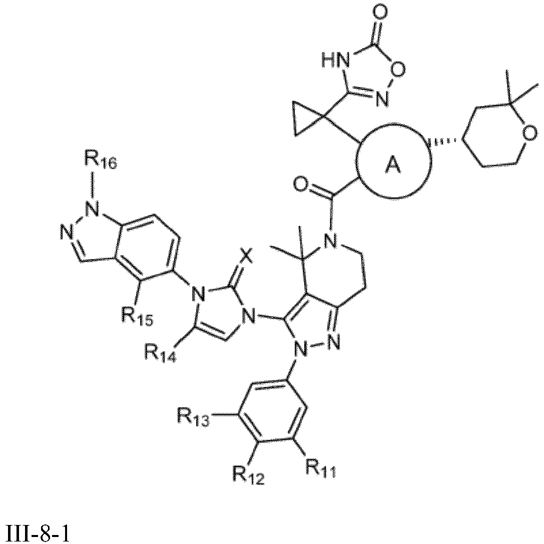 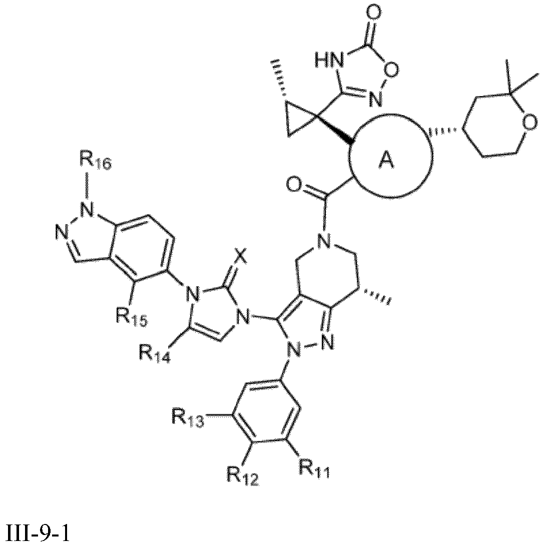 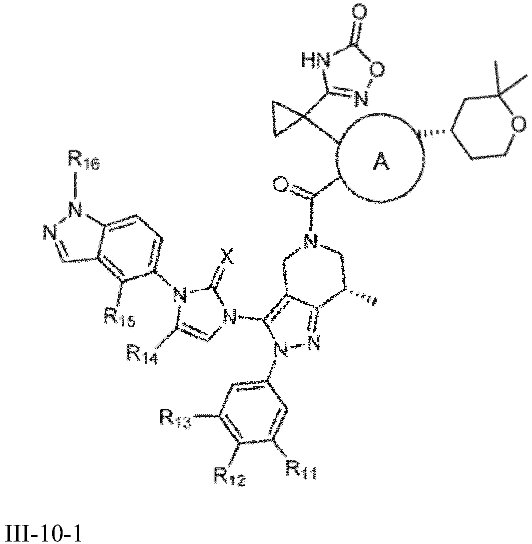 wherein,
A is 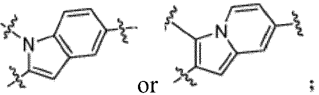
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
R₁₄ is selected from hydrogen, C₁~C₃ alkyl, halogen, and hydroxyl;
R₁₅ is selected from hydrogen, C₁~C₃ alkyl groups, and halogens;
R16 is selected from C₁~C₃ alkyl and deuterated C₁~C₃ alkyl.

6. The compound according to claim 2, its pharmaceutically acceptable salt, or its stereoisomer, is **characterized in that**: the structure of the compound is as shown in Formula III-11-1, Formula III-12-1, and Formula III-13-1. 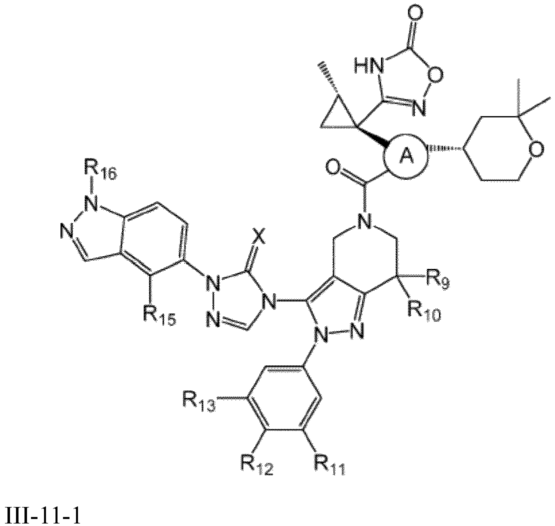 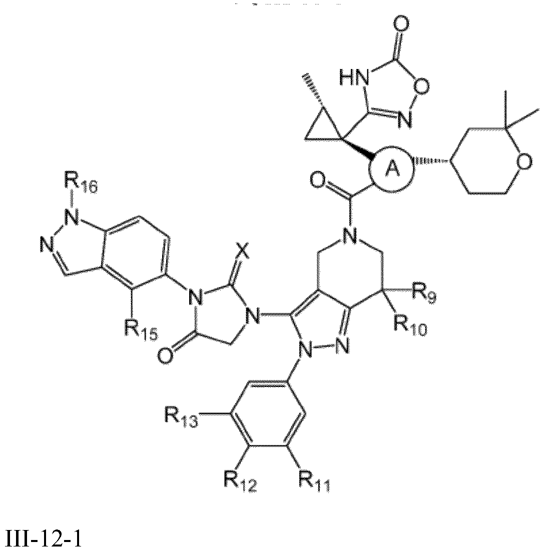 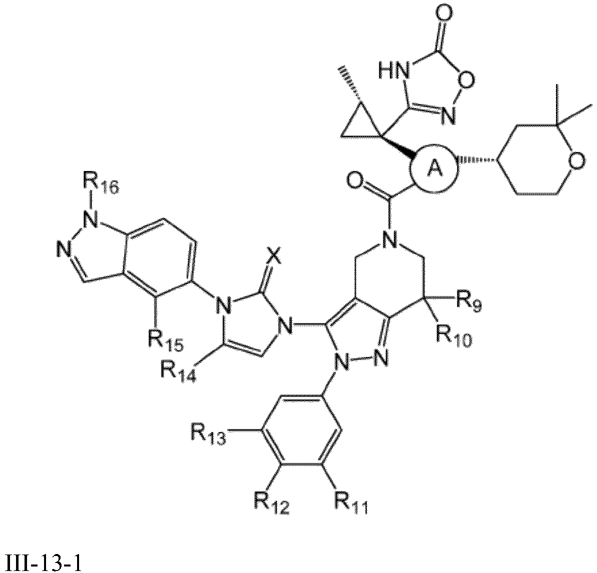 wherein,
A is 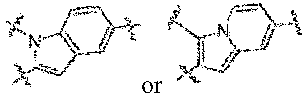
R₉ is selected from hydrogen, C₁~C₃ alkyl groups, and halogens;
R₁₀ is selected from hydrogen, C₁~C₃ alkyl groups, and halogens;
Alternatively, R₉ and R₁₀ can be connected to form a 3-6 member saturated cycloalkyl group;
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
R₁₄ is selected from hydrogen, C₁~C₃ alkyl, halogen, and hydroxyl;
R₁₅ is selected from hydrogen, C₁~C₅ alkyl groups, and halogens;
R₁₆ is selected from C_{1~}C₅ alkyl and deuterated C₁~C₅ alkyl.

7. The compound according to claim 3, its pharmaceutically acceptable salt, or its stereoisomer, is **characterized in that**: the structure of the compound is as shown in Formula IV-1 or Formula IV'-1: 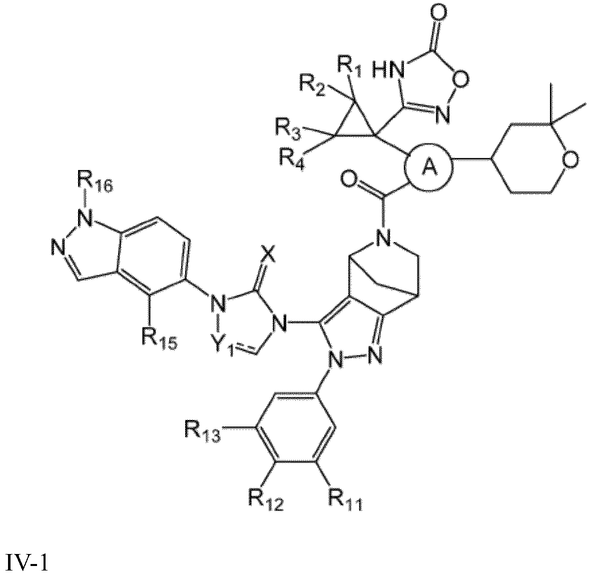 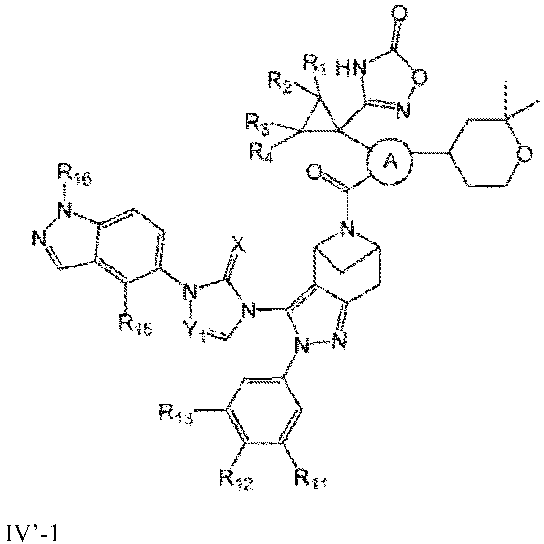 wherein,
A is 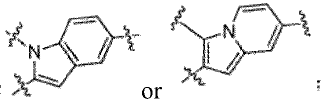
R₁ is selected from hydrogen, C₁~C₃ alkyl groups, and halogens;
R₂ is selected from hydrogen, C₁~C₃ alkyl groups, and halogens;
R₃ is selected from hydrogen, C₁~C₃ alkyl, halogen;
R₄ is selected from hydrogen, C₁~C₃ alkyl, halogen;
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
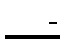 is selected from - or =;
provided that - is -, Y1 represents C=O or C=S;
provided that - is =, Y₁ is N or CR₁₄; R₁₄ is selected from hydrogen, C₁~C₃ alkyl, halogen, hydroxyl;
R₁₅ is selected from hydrogen, C₁~C₃ alkyl groups, and halogens;
R₁₆ is selected from C₁~C₃ alkyl and deuterated C₁~C₃ alkyl.

8. The compound according to claim 7, its pharmaceutically acceptable salt, or its stereoisomer, is **characterized in that**: the structure of the compound is as shown in formula IV-1-1, IV-2-1, IV-3-1, IV-4-1, IV-5-1, IV-6-1, IV-7-1, or IV-8-1. 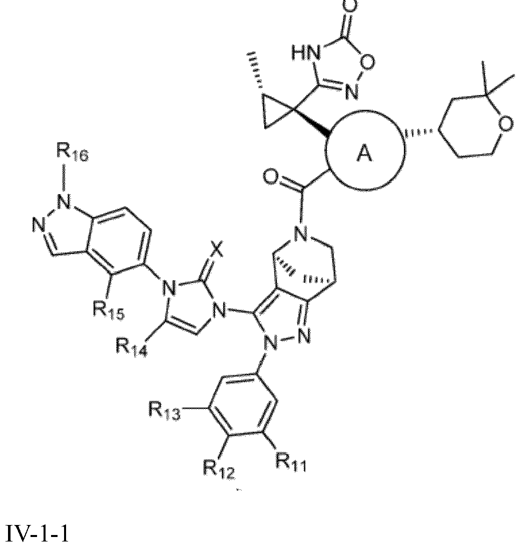 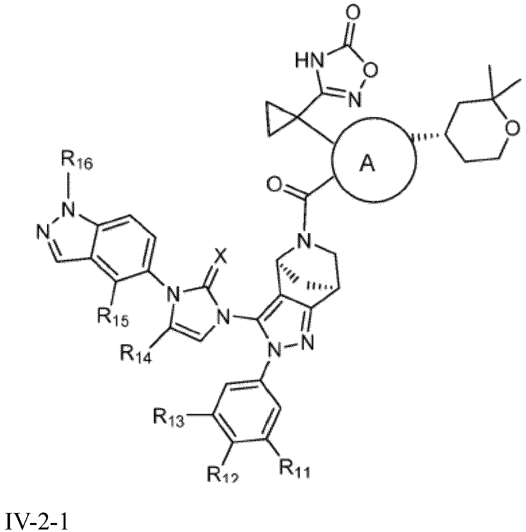 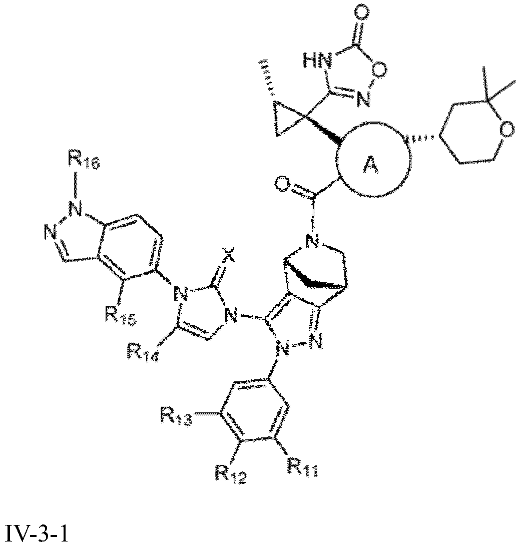 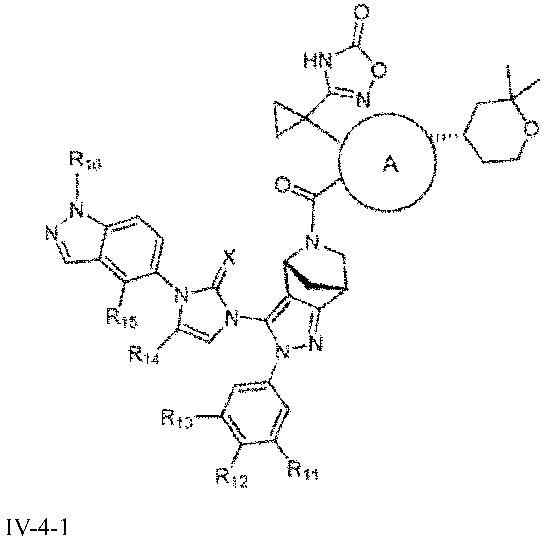 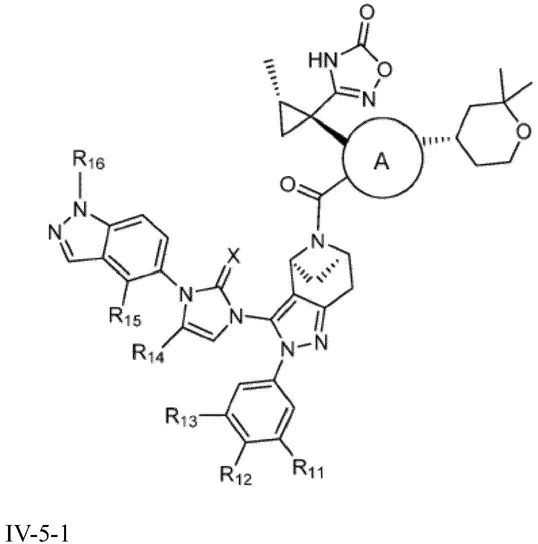 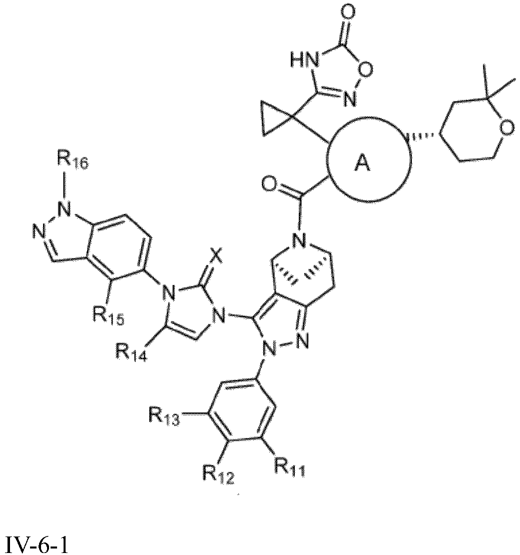 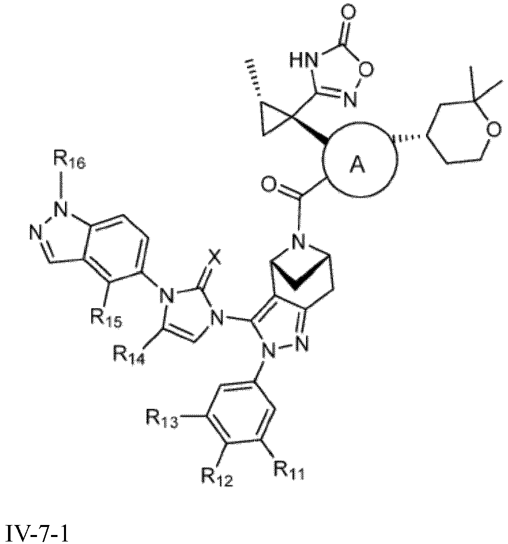 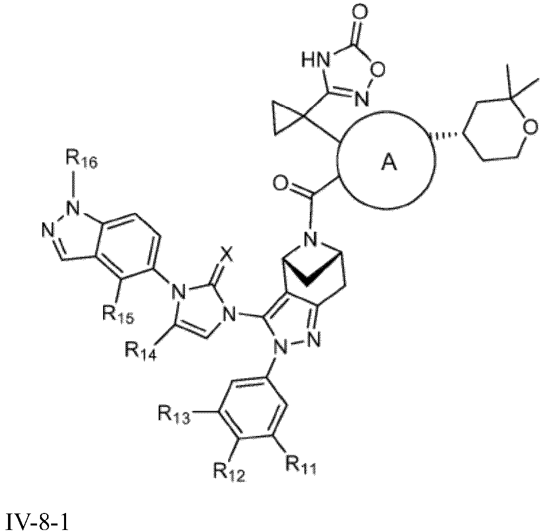 wherein,
A is 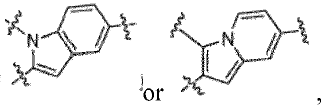
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
R₁₄ is selected from hydrogen, C₁~C₃ alkyl, halogen, and hydroxyl;
R₁₅ is selected from hydrogen, C₁~C₃ alkyl groups, and halogens;
R₁₆ is selected from C₁~C₃ alkyl groups and deuterated C₁~C₃ alkyl groups.

9. The compound according to claim 3, its pharmaceutically acceptable salt, or its stereoisomer, is **characterized in that**: the structure of the compound is as shown in Formula V-1: 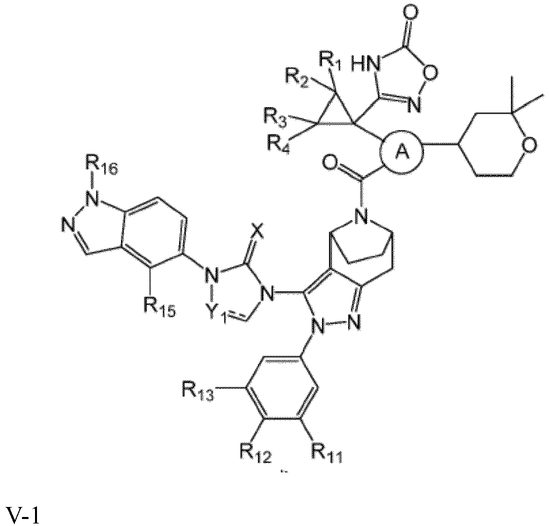 wherein,
A is 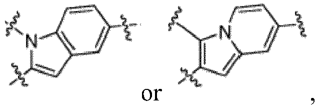
R₁ is selected from hydrogen, C₁ ~C₃ alkyl groups, and halogens;
R₂ is selected from hydrogen, C₁~C₃ alkyl groups, and halogens;
R₃ is selected from hydrogen, C₁~C₃ alkyl groups, and halogens;
R₄ is selected from hydrogen, C₁~C₃ alkyl groups, and halogens;
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
- is selected from - or =;
provided that - is -, Y1 represents C=O or C=S;
provided that - is =, Y1 is N or CR₁₄; R₁₄ is selected from hydrogen, C₁~C₃ alkyl, halogen,
hydroxyl;
R₁₅ is selected from hydrogen, C₁~C₃ alkyl groups, and halogens;
R₁₆ is selected from C₁~C₃ alkyl and deuterated C₁~C₃ alkyl.

10. The compound according to claim 9, its pharmaceutically acceptable salt, or its stereoisomer, is **characterized in that**: the structure of the compound is as shown in formula V-1-1, V-2-1, V-3-1, or V-4-1. 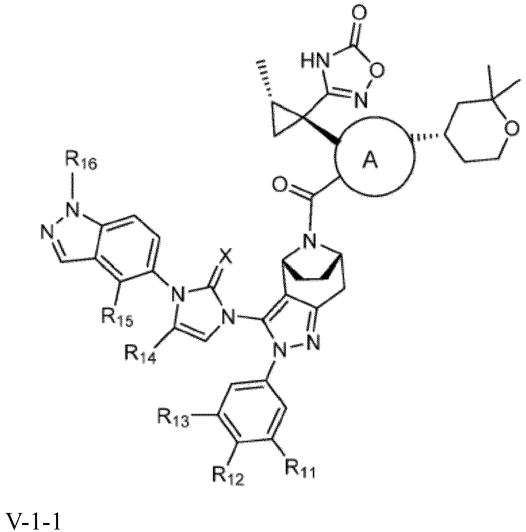 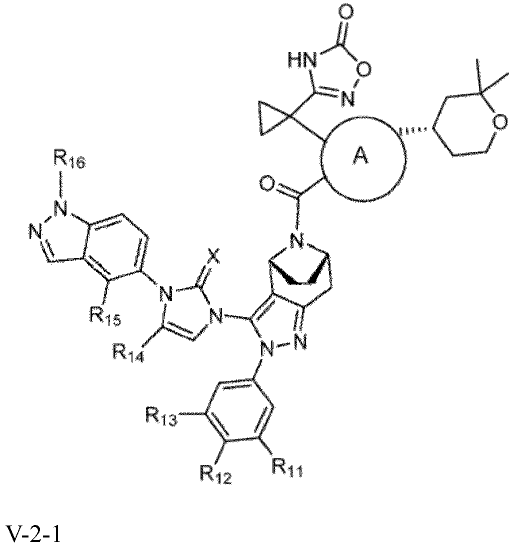 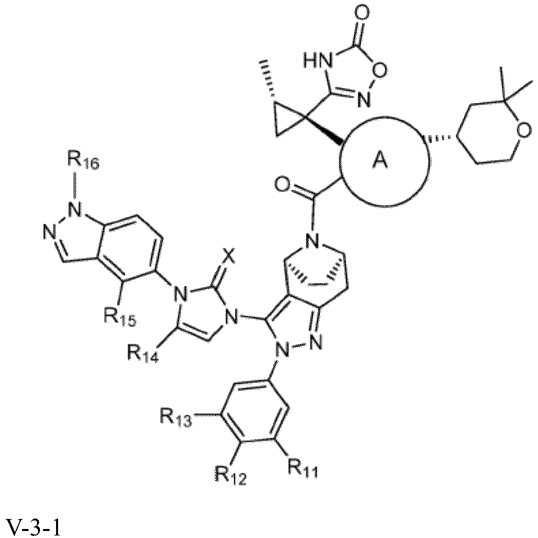 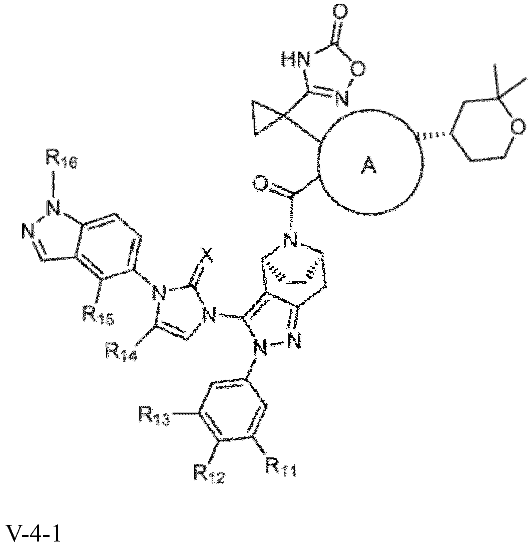 in,
A is 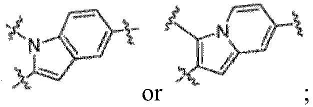
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
R₁₄ is selected from hydrogen, C₁~C₃ alkyl, halogen, and hydroxyl;
R₁₅ is selected from hydrogen, C₁~C₃ alkyl groups, and halogens;
R₁₆ is selected from C₁~C₃ alkyl and deuterated C₁~C₃ alkyl.

11. The compound according to claim 1, its pharmaceutically acceptable salt, or its stereoisomer, is **characterized in that**: the structure of the compound is as shown in Formula I-2, Formula I-3, Formula 1-4, or Formula I-5: 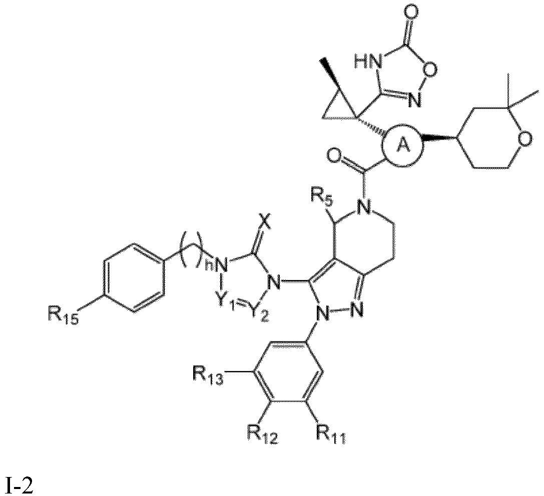 wherein,
A is 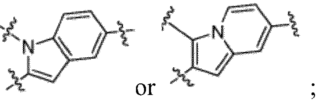
R₅ is selected from hydrogen, C₁~C₃ alkyl groups, and halogens;
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
Y₁ and Y₂ are each independently selected from N or CR₁₄; R₁₄ is selected from hydrogen, C₁~
C₃ alkyl, halo-substituted C₁~C₃ alkyl, hydroxy-substituted C₁~C₃ alkyl, carboxyl, halogen, and hydroxyl;
h is 0 or 1;
R₁₅ is selected from hydrogen, C₁~C₃ alkyl, C₁~C₃ alkoxy, and halogen; 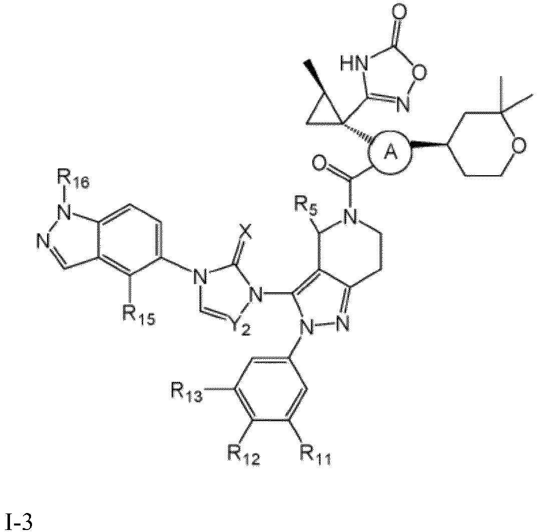
wherein,
A is 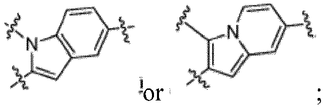
R₅ is selected from hydrogen, C₁~C₃ alkyl groups, and halogens;
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
Y₂ is selected from N or CR₁₄; R₁₄ is selected from hydrogen, C₁~C₃ alkyl, halo-substituted C₁ ~C₃ alkyl, hydroxy-substituted C₁~C₃ alkyl, carboxyl, halogen, hydroxyl, 3-6 member saturated cycloalkyl;
R₁₅ is selected from hydrogen, C₁~C₃ alkyl, and halogen;
R₁₆ is selected from C₁~C₃ alkyl and deuterated C₁~C₃ alkyl; 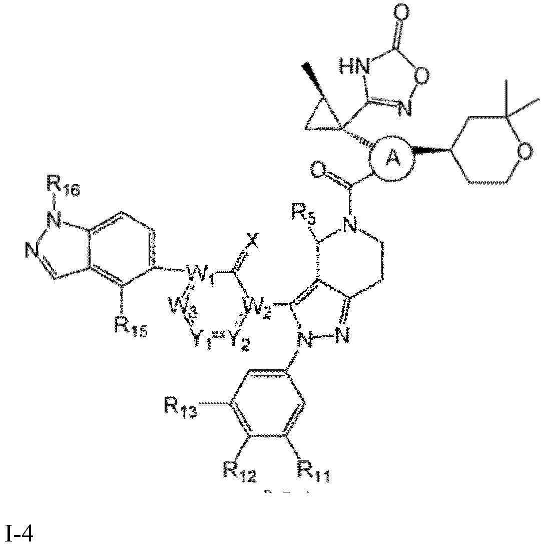
wherein,
A is 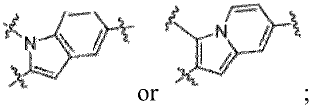
R₅ is selected from hydrogen, C₁~C₃ alkyl, halogen;
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
W₁ and W₂ are each independently selected from N or C; W₃ is selected from CH or N; and at least one of W₁, W₂, and W₃ is N;
Y₁ and Y₂ are each independently selected from N or CR₁₄; R₁₄ is selected from hydrogen, C₁~ C₃ alkyl, halo-substituted C₁~C₃ alkyl, hydroxy-substituted C₁~C₃ alkyl, carboxyl, halogen, hydroxyl, 3-6 member saturated cycloalkyl;
The dashed lines between W₁, W₂, W₃, Y₁, and Y₂ are either none or keys depending on the selection of W₁, W₂, W₃, Y₁, and Y₂.
R₁₅ is selected from hydrogen, C₁~C₃ alkyl, and halogen;
R₁₆ is selected from C₁~C₃ alkyl and deuterated C₁~C₃ alkyl; 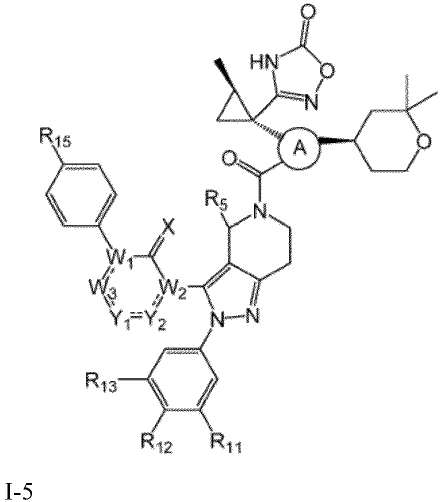
wherein,
A is 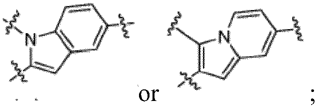
R₅ is selected from hydrogen, C₁~C₃ alkyl, and halogen;
R₁₁ is selected from hydrogen, C₁~C₃ alkyl, halogen, and 3-6 membered saturated cycloalkyl;
R₁₂ is selected from hydrogen, C₁~C₃ alkyl, halogen, and 3-6 membered saturated cycloalkyl;
R₁₃ is selected from hydrogen, C₁~C₃ alkyl, halogen, and 3-6 membered saturated cycloalkyl;
X is selected from O and S;
W₁ and W₂ are each independently selected from N or C; W₃ is selected from CH or N; and at least one of W₁, W₂, and W₃ is N;
Y₁ and Y₂ are each independently selected from N or CR₁₄; R₁₄ is selected from hydrogen, C₁~ C₃ alkyl, halo-substituted C₁~C₃ alkyl, hydroxy-substituted C₁~C₃ alkyl, carboxyl, halogen, hydroxyl, and 3-6 member saturated cycloalkyl;
The dashed lines between W₁, W₂, W₃, Y₁, and Y₂ are either none or keys depending on the selection of W₁, W₂, W₃, Y₁, and Y₂.
R₁₅ is selected from hydrogen, C₁~C₃ alkyl groups, and halogens.

12. The compound according to claim 1, its pharmaceutically acceptable salt, or its stereoisomer, is **characterized in that**: the compound is shown in formulas I-6: 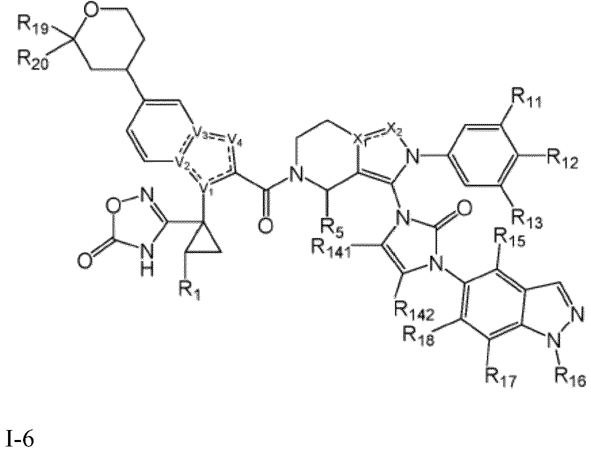 wherein,
R₁₂ is selected from hydrogen, substituted or unsubstituted C₁~C₆ alkyl, halogen;
R₁₁ and R₁₃ are each independently selected from hydrogen, substituted or unsubstituted C₁~C₆ alkyl, substituted or unsubstituted C₂~C₆ alkenyl, substituted or unsubstituted C₂~C₆ alkynyl, and halogen; and at least one of R₁₁ and R₁₃ is selected from substituted or unsubstituted C₂~C₆ alkenyl, substituted or unsubstituted C₂~C₆ alkynyl;
R₁₄₁ and R₁₄₂ are independently selected from hydrogen, substituted or unsubstituted C₁~C₆ alkyl, substituted or unsubstituted C₁~C₆ alkoxy, and halogen, respectively;
R₁, R₅, R₁₅, R₁₆, R₁₇, R₁₈, R₁₉, and R₂₀ are each independently selected from hydrogen, substituted or unsubstituted C₁ to C₆ alkyl groups, and halogens;
X₁ is selected from N or C; X₂ is selected from N or CH₂; and there is exactly one N in X₁ and
X₂; the dashed line between X₁ and X₂ is none or a key depending on the selection of X₁ and X₂;
V₁, V₂, and V₃ are each independently selected from N or C; V₄ is selected from N or CH; the dashed line connecting V₁, V₂, V₃, and V₄ is either empty or a key depending on the selection of V₁, V₂, V₃, and V₄.
The substituents of the alkyl, alkenyl, and alkynyl groups are selected from the following groups: halogen, hydroxyl, C₁~C₆ alkyl, C₁~C₆ alkoxy, substituted or unsubstituted 3- to 10-membered cycloalkyl, substituted or unsubstituted 3- to 10-membered heterocycloalkyl, substituted or unsubstituted 6- to 10-membered aryl, substituted or unsubstituted 5- to 10-membered heteroaryl, -C(O)R₁', -NR₂'R₃';
The substituents of the alkoxy group are selected from the following groups: C₁~C₆ alkyl, halo-C₁~C₆ alkyl, halogen, -C(O)R₁';
The substituents of the cycloalkyl, heterocycloalkyl, aryl, and heteroaryl groups are selected from the following groups: C₁~C₆ alkyl, halogenated C₁~C₆ alkyl, halogen, and hydroxyl;
R₁', R₂', and R₃' are each independently selected from hydrogen and C₁ to C₆ alkyl groups.

13. The compound according to claim 12, its pharmaceutically acceptable salt, or its stereoisomer, is **characterized in that**: the structure of the compound is as shown in Formula II-6: 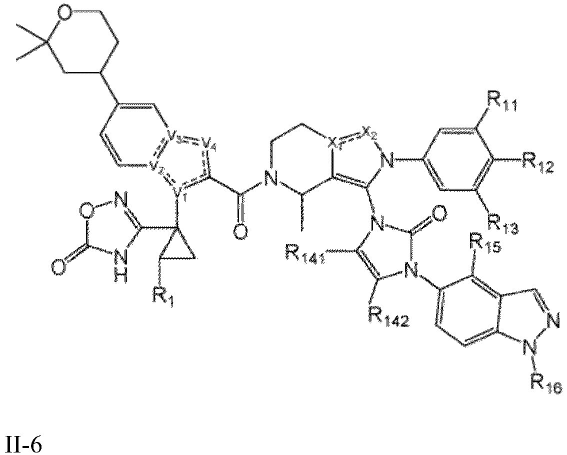 wherein,
R₁₂ is selected from hydrogen, substituted or unsubstituted C₁~C₆ alkyl, halogen;
R₁₁ and R₁₃ are each independently selected from hydrogen, substituted or unsubstituted C₁~ C₆alkyl, substituted or unsubstituted C₂~C₆alkenyl, substituted or unsubstituted C₂~C₆ynyl, and halogen; and at least one of R₁₁ and R₁₃ is selected from substituted or unsubstituted C₂~ C₆alkenyl, substituted or unsubstituted C₂~C₆ynyl;
R₁₄₁ and R₁₄₂ are independently selected from hydrogen, substituted or unsubstituted C₁~C₆ alkyl, substituted or unsubstituted C₁~C₆ alkoxy, and halogen, respectively;
R₁, R₁₅, and R₁₆ are each independently selected from hydrogen, substituted or unsubstituted C₁ ~C₆ alkyl groups, and halogens;
X₁ is selected from N or C; X₂ is selected from N or CH₂; and there is one and only one N in X₁ and X₂; the dashed line between X₁ and X₂ is none or a key depending on the selection of X₁ and X₂;
V₁, V₂, and V₃ are each independently selected from N or C; V₄ is selected from N or CH; the dashed line connecting V₁, V₂, V₃, and V₄ is either empty or a key, depending on the selection of V₁, V₂, V₃, and V₄.
The substituents of the alkyl, alkenyl, and alkynyl groups are selected from the following groups: halogen, hydroxyl, C₁~C₆ alkyl, C₁~C₆ alkoxy, substituted or unsubstituted 3- to 10-membered cycloalkyl, substituted or unsubstituted 3- to 10-membered heterocycloalkyl, substituted or unsubstituted 6- to 10-membered aryl, substituted or unsubstituted 5- to 10-membered heteroaryl, -C(O)R₁', -NR₂'R₃';
The substituents of the alkoxy group are selected from the following groups: C₁~C₆ alkyl, halo-C₁~C₆ alkyl, halogen, -C(O)R₁';
The substituents of the cycloalkyl, heterocycloalkyl, aryl, and heteroaryl groups are selected from the following groups: C₁~C₆ alkyl, halogenated C₁~C₆ alkyl, halogen, and hydroxyl;
R₁', R₂', and R₃' are each independently selected from hydrogen and C₁ to C₆ alkyl groups.

14. The compound according to claim 13, its pharmaceutically acceptable salt, or its stereoisomer, is **characterized in that**: the structure of the compound is as shown in Formula III-6: 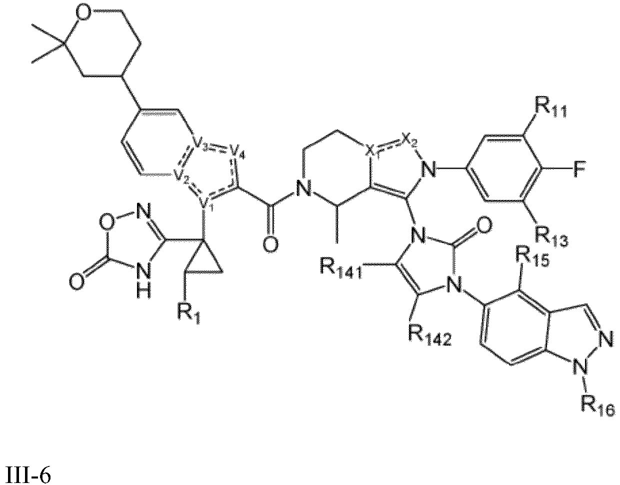 wherein,
R₁₁ and R₁₃ are each independently selected from hydrogen, substituted or unsubstituted C₁~C₆ alkyl, substituted or unsubstituted C₂~C₆ alkenyl, substituted or unsubstituted C₂~C₆ ynyl, and halogen; and at least one of R₁₁ and R₁₃ is selected from substituted or unsubstituted C₂~C₆ alkenyl, substituted or unsubstituted C₂~C₆ ynyl.
R₁₄₁ and R₁₄₂ are independently selected from hydrogen, substituted or unsubstituted C₁~C₆ alkyl, substituted or unsubstituted C₁~C₆ alkoxy, and halogen, respectively;
R₁, R₁₅, and R₁₆ are each independently selected from hydrogen, substituted or unsubstituted C₁ to C₆ alkyl groups, and halogens;
X₁ is selected from N or C; X₂ is selected from N or CH₂; and there is exactly one N in X₁ and X₂; the dashed line between X₁ and X₂ is none or a key depending on the selection of X₁ and X₂; V₁, V₂, and V₃ are each independently selected from N or C; V₄ is selected from N or CH; the dashed line connecting V₁, V₂, V₃, and V₄ is either empty or a key depending on the selection of V₁, V₂, V₃, and V₄.
The substituents of the alkyl, alkenyl, and alkynyl groups are selected from the following groups: halogen, hydroxyl, C₁~C₆alkyl, C₁~C₆alkoxy, substituted or unsubstituted 3- to 10-membered cycloalkyl, substituted or unsubstituted 3- to 10-membered heterocycloalkyl, substituted or unsubstituted 6- to 10-membered aryl, substituted or unsubstituted 5- to 10-membered heteroaryl, -C(O)R₁', -NR₂'R₃';
The substituents of the alkoxy group are selected from the following groups: C₁~C₆alkyl, halo-C₁~C₆alkyl, halogen, -C(O)R₁';
The substituents of the cycloalkyl, heterocycloalkyl, aryl, and heteroaryl groups are selected from the following groups: C₁~C₆alkyl, halogenated C₁~C₆alkyl, halogen, and hydroxyl;
R₁', R₂', and R₃' are each independently selected from hydrogen and C₁ to C₆ alkyl groups.

15. The compound according to claim 14, its pharmaceutically acceptable salt, or its stereoisomer, is **characterized in that**: the structure of the compound is as shown in Formula IV-6: 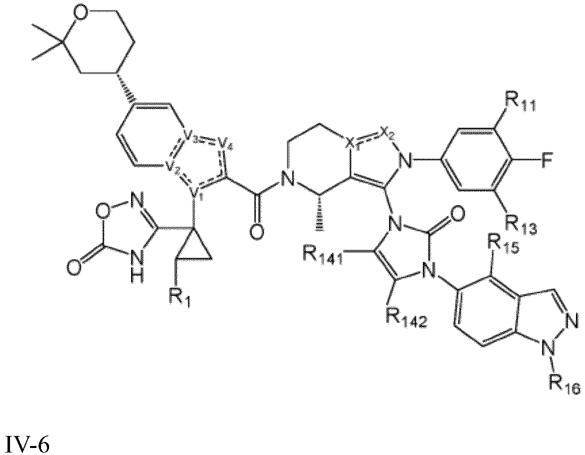 wherein,
R₁₁ and R₁₃ are each independently selected from hydrogen, substituted or unsubstituted C₁~ C₆alkyl, substituted or unsubstituted C₂~C₆alkenyl, substituted or unsubstituted C₂~C₆alkynyl, and halogen; and at least one of R₁₁ and R₁₃ is selected from substituted or unsubstituted C₂~ C₆alkenyl, substituted or unsubstituted C₂~C₆alkynyl;
R₁₄₁ and R₁₄₂ are independently selected from hydrogen, substituted or unsubstituted C₁~C₆ alkyl, substituted or unsubstituted C₁~C₆ alkoxy, and halogen, respectively;
R₁, R₁₅, and R₁₆ are each independently selected from hydrogen, substituted or unsubstituted C₁ ~C₆ alkyl groups, and halogens;
X₁ is selected from N or C; X₂ is selected from N or CH₂; and there is one and only one N in X₁ and X₂; the dashed line between X₁ and X₂ is none or a key depending on the selection of X₁ and X₂;
V₁, V₂, and V₃ are each independently selected from N or C; V₄ is selected from N or CH; the dashed line connecting V₁, V₂, V₃, and V₄ is either empty or a key depending on the selection of V₁, V₂, V₃, and V₄.
The substituents of the alkyl, alkenyl, and alkynyl groups are selected from the following groups: halogen, hydroxyl, C₁~C₆ alkyl, C₁~C₆ alkoxy, substituted or unsubstituted 3- to 10-membered cycloalkyl, substituted or unsubstituted 3- to 10-membered heterocycloalkyl, substituted or unsubstituted 6- to 10-membered aryl, substituted or unsubstituted 5- to 10-membered heteroaryl, -C(O)R₁', -NR₂'R₃';
The substituents of the alkoxy group are selected from the following groups: C₁~C₆ alkyl, halo-C₁~C₆ alkyl, halogen, -C(O)R₁';
The substituents of the cycloalkyl, heterocycloalkyl, aryl, and heteroaryl groups are selected from the following groups: C₁~C₆alkyl, halogenated C₁~C₆alkyl, halogen, and hydroxyl;
R₁', R₂', and R₃' are each independently selected from hydrogen and C₁~C₆ alkyl groups.

16. The compound according to claim 15, its pharmaceutically acceptable salt, or its stereoisomer, is **characterized in that**: the structure of the compound is as shown in formula Va-6: 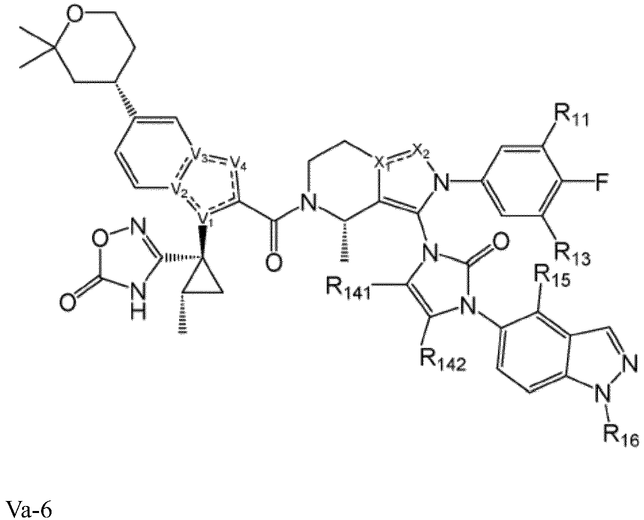 wherein,
R₁₁ and R₁₃ are each independently selected from hydrogen, substituted or unsubstituted C₁~ C₆alkyl, substituted or unsubstituted C₂~C₆alkenyl, substituted or unsubstituted C₂~C₆alkynyl, and halogen; and at least one of R₁₁ and R₁₃ is selected from substituted or unsubstituted C₂~ C₆alkenyl, substituted or unsubstituted C₂~C₆alkynyl;
R₁₄₁ and R₁₄₂ are independently selected from hydrogen, substituted or unsubstituted C₁~C₆ alkyl, substituted or unsubstituted C₁~C₆ alkoxy, and halogen, respectively;
R₁₅ and R₁₆ are independently selected from hydrogen, substituted or unsubstituted C₁~C₆ alkyl, and halogen, respectively;
X₁ is selected from N or C; X₂ is selected from N or CH₂; and there is exactly one N in X₁ and X₂; the dashed line between X₁ and X₂ is none or a bond depending on the selection of X₁ and X₂;
V₁, V₂, and V₃ are each independently selected from N or C; V₄ is selected from N or CH; the dashed line connecting V₁, V₂, V₃, and V₄ is either empty or a key, depending on the selection of V₁, V₂, V₃, and V₄.
The substituents of the alkyl, alkenyl, and alkynyl groups are selected from the following groups: halogen, hydroxyl, C₁~C₆alkyl, C₁~C₆alkoxy, substituted or unsubstituted 3-10 membered cycloalkyl, substituted or unsubstituted 3-10 membered heterocycloalkyl, substituted or unsubstituted 6-10 membered aryl, substituted or unsubstituted 5-10 membered heteroaryl, - C(O)R₁', -NR₂'R₃';
The substituents of the alkoxy group are selected from the following groups: C₁~C₆ alkyl, halo-C₁~C₆ alkyl, halogen, -C(O)R₁';
The substituents of the cycloalkyl, heterocycloalkyl, aryl, and heteroaryl groups are selected from the following groups: C₁~C₆ alkyl, halogenated C₁~C₆ alkyl, halogen, and hydroxyl;
R₁', R₂', and R₃' are each independently selected from hydrogen and C₁ to C₆ alkyl groups.

17. The compound according to claim 15, its pharmaceutically acceptable salt, or its stereoisomer, is **characterized in that**: the structure of the compound is as shown in formula Vb-6: 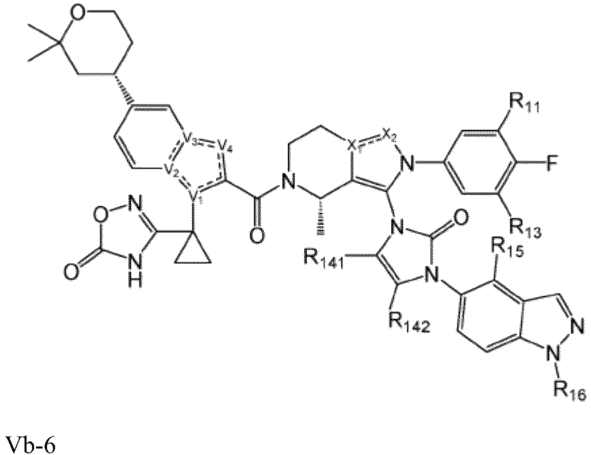 wherein,
R₁₁ and R₁₃ are each independently selected from hydrogen, substituted or unsubstituted C₁~C₆ alkyl, substituted or unsubstituted C₂~C₆ alkenyl, substituted or unsubstituted C₂~C₆ ynyl, and halogen; and at least one of R₁₁ and R₁₃ is selected from substituted or unsubstituted C₂~C₆ alkenyl, substituted or unsubstituted C₂~C₆ ynyl.
R₁₄₁ and R₁₄₂ are independently selected from hydrogen, substituted or unsubstituted C₁~C₆ alkyl, substituted or unsubstituted C₁~C₆ alkoxy, and halogen, respectively;
R₁₅ and R₁₆ are independently selected from hydrogen, substituted or unsubstituted C₁ to C₆ alkyl groups, and halogens, respectively;
X₁ is selected from N or C; X₂ is selected from N or CH₂; and there is exactly one N in X₁ and X₂; the dashed line between X₁ and X₂ is none or a key depending on the selection of X₁ and X₂; V₁, V₂, and V₃ are each independently selected from N or C; V₄ is selected from N or CH; the dashed line connecting V₁, V₂, V₃, and V₄ is either empty or a key, depending on the selection of V₁, V₂, V₃, and V₄.
The substituents of the alkyl, alkenyl, and alkynyl groups are selected from the following groups: halogen, hydroxyl, C₁~C₆alkyl, C₁~C₆alkoxy, substituted or unsubstituted 3- to 10-membered cycloalkyl, substituted or unsubstituted 3- to 10-membered heterocycloalkyl, substituted or unsubstituted 6- to 10-membered aryl, substituted or unsubstituted 5- to 10-membered heteroaryl, -C(O)R₁', -NR₂'R₃';
The substituents of the alkoxy group are selected from the following groups: C₁~C₆ alkyl, halo-C₁~C₆ alkyl, halogen, -C(O)R₁';
The substituents of the cycloalkyl, heterocycloalkyl, aryl, and heteroaryl groups are selected from the following groups: C₁~C₆alkyl, halogenated C₁~C₆alkyl, halogen, and hydroxyl;
R₁', R₂', and R₃' are independently selected from hydrogen and C₁ to C₆ alkyl groups, respectively.

18. The compound, its pharmaceutically acceptable salt, or its stereoisomer according to any one of claims 12 to 17, is **characterized in that**:
R₁₁ and R₁₃ are each independently selected from hydrogen, substituted or unsubstituted C₁~C₆ alkyl, substituted or unsubstituted C₂~C₆ alkenyl, substituted or unsubstituted C₂~C₆ alkynyl,
and halogen; and at least one of R₁₁ and R₁₃ is selected from substituted or unsubstituted C₂~C₆ alkenyl, substituted or unsubstituted C₂~C₆ alkynyl;
The substituents of the alkenyl and alkynyl groups are selected from the following groups: halogen, hydroxyl, C₁~C₆ alkyl, C₁~C₆ alkoxy, substituted or unsubstituted 3- to 10-membered cycloalkyl, substituted or unsubstituted tetrahydropyranyl, substituted or unsubstituted piperidinyl, substituted or unsubstituted pyridinyl, substituted or unsubstituted phenyl, substituted or unsubstituted pyrazolyl, substituted or unsubstituted pyrroleyl, substituted or unsubstituted indazoleyl, substituted or unsubstituted indolyl, -C(O)R₁', -NR₂'R₃';
The substituents of the alkyl group are selected from the following groups: halogen, hydroxyl, C₁-C₆ alkyl, and C₁-C₆ alkoxy;
R₁', R₂', and R₃' are independently selected from hydrogen and C₁~C₆ alkyl groups, respectively;
The substituents of the cycloalkyl, tetrahydropyranyl, piperidinyl, pyridinyl, phenyl, pyrazolyl, pyrroleyl, indazoleyl, and indoleyl groups are selected from the following groups: C₁~C₆ alkyl, halogenated C₁~C₆ alkyl, halogen, and hydroxyl.

19. The compound according to any one of claims 12 to 17, its pharmaceutically acceptable salt, or its stereoisomer, is **characterized in that**: 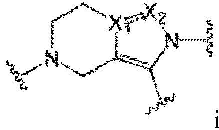 is

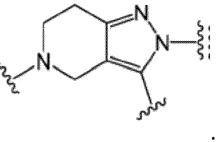

20. The compound according to any one of claims 12 to 17, its pharmaceutically acceptable salt, or its stereoisomer, is **characterized in that**: 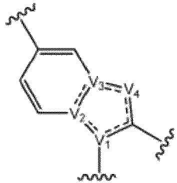 is

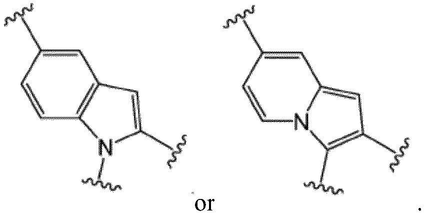

21. The compound, its pharmaceutically acceptable salt, or its stereoisomer according to any one of claims 12 to 17, is **characterized in that** the structure of the compound is as shown in formula VIa-6: 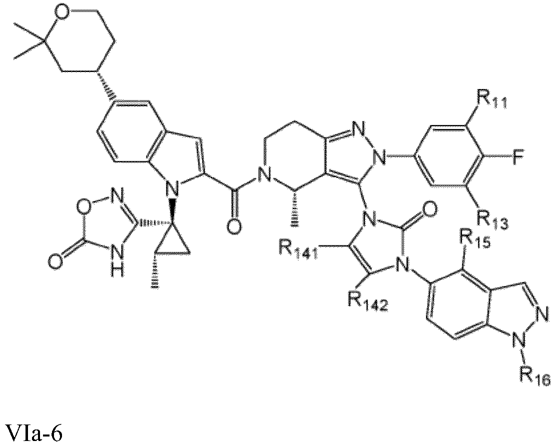 wherein,
R₁₁ and R₁₃ are each independently selected from hydrogen, substituted or unsubstituted C₁~ C₆alkyl, substituted or unsubstituted C₂~C₆alkenyl, substituted or unsubstituted C₂~C₆alkynyl, and halogen; and at least one of R₁₁ and R₁₃ is selected from substituted or unsubstituted C₂~ C₆alkenyl, substituted or unsubstituted C₂~C₆alkynyl;
The substituents of the alkenyl and alkynyl groups are selected from the following groups: halogen, hydroxyl, C₁~C₆ alkyl, C₁~C₆ alkoxy, substituted or unsubstituted 3- to 10-membered cycloalkyl, substituted or unsubstituted tetrahydropyranyl, substituted or unsubstituted piperidinyl, substituted or unsubstituted pyridinyl, substituted or unsubstituted phenyl, substituted or unsubstituted pyrazolyl, substituted or unsubstituted pyrroleyl, substituted or unsubstituted indazoleyl, substituted or unsubstituted indolyl, -C(O)R₁', -NR₂'R₃';
R₁₄₁ and R₁₄₂ are independently selected from hydrogen, substituted or unsubstituted C₁~C₆ alkyl, substituted or unsubstituted C₁~C₆ alkoxy, and halogen, respectively;
R₁₅ and R₁₆ are independently selected from hydrogen, substituted or unsubstituted C₁ to C₆ alkyl, and halogen, respectively;
The substituents of the cycloalkyl, tetrahydropyranyl, piperidinyl, pyridinyl, phenyl, pyrazolyl, pyrroleyl, indazoleyl, and indoleyl groups are selected from the following groups: C₁~C₆alkyl, halogenated C₁~C₆alkyl, halogen, and hydroxyl;
The substituents of the alkoxy group are selected from the following groups: C₁~C₆ alkyl, halo-C₁~C₆ alkyl, halogen, -C(O)R₁';
The substituents of the alkyl group are selected from the following groups: halogen, hydroxyl, C₁ ~C₆alkyl, C₁~C₆alkoxy;
R₁', R₂', and R₃' are each independently selected from hydrogen and C₁ to C₆ alkyl groups,
respectively.

22. The compound according to claim 21, its pharmaceutically acceptable salt, or its stereoisomer, is **characterized in that**: the structure of the compound is as shown in Formula VIa-6-1: 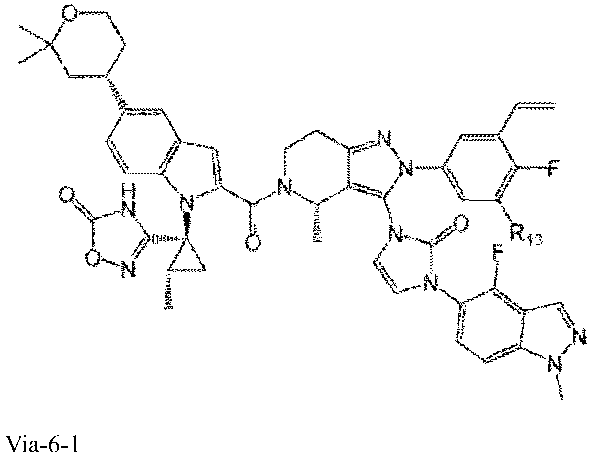 wherein,
R₁₃ is independently selected from hydrogen, C₁~C₆alkyl, C₂~C₆alkenyl, C₂~C₆alkynyl, and halogen.

23. The compound, its pharmaceutically acceptable salt, or its stereoisomer according to any one of claims 12 to 17, is **characterized in that** the structure of the compound is as shown in formula VIb-6: 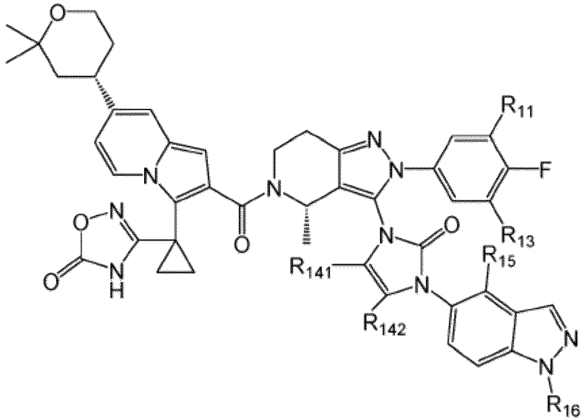 wherein,
R₁₁ and R₁₃ are each independently selected from hydrogen, substituted or unsubstituted C₁~C₆ alkyl, substituted or unsubstituted C₂~C₆ alkenyl, substituted or unsubstituted C₂~C₆ alkynyl, and halogen; and at least one of R₁₁ and R₁₃ is selected from substituted or unsubstituted C₂~C₆ alkenyl, substituted or unsubstituted C₂~C₆ alkynyl;
The substituents of the alkenyl and alkynyl groups are selected from the following groups: halogen, hydroxyl, C₁~C₆alkyl, C₁~C₆alkoxy, substituted or unsubstituted 3- to 10-membered cycloalkyl, substituted or unsubstituted tetrahydropyranyl, substituted or unsubstituted piperidinyl, substituted or unsubstituted pyridinyl, substituted or unsubstituted phenyl, substituted or unsubstituted pyrazolyl, substituted or unsubstituted pyrroleyl, substituted or unsubstituted indazoleyl, substituted or unsubstituted indolyl, -C(O)R₁', -NR₂'R₃';
R₁₄₁ and R₁₄₂ are independently selected from hydrogen, substituted or unsubstituted C₁~C₆ alkyl, substituted or unsubstituted C₁~C₆ alkoxy, and halogen, respectively;
R₁₅ and R₁₆ are independently selected from hydrogen, substituted or unsubstituted C₁ to C₆ alkyl groups, and halogens, respectively;
The substituents of the cycloalkyl, tetrahydropyranyl, piperidinyl, pyridinyl, phenyl, pyrazolyl, pyrroleyl, indazoleyl, and indoleyl groups are selected from the following groups: C₁~C₆ alkyl, halogenated C₁~C₆ alkyl, halogen, and hydroxyl groups;
The substituents of the alkoxy group are selected from the following groups: C₁~C₆ alkyl, halo-C₁~C₆ alkyl, halogen, -C(O)R₁';
The substituents of the alkyl group are selected from the following groups: halogen, hydroxyl, C₁ ~C₆alkyl, C₁~C₆alkoxy;
R₁', R₂', and R₃' are each independently selected from hydrogen and C₁ to C₆ alkyl groups, respectively.

24. The compound according to claim 1, its pharmaceutically acceptable salt, or its stereoisomer, is **characterized in that**: the compound is shown in formulas I-7: 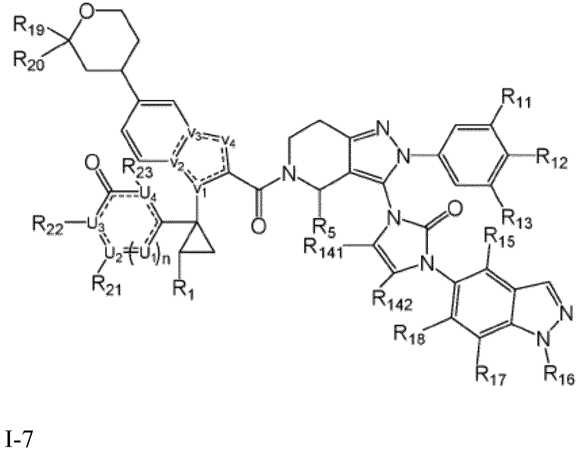 wherein,
R₁₁, R₁₂, and R₁₃ are each independently selected from hydrogen, substituted or unsubstituted C₁ ~C₆ alkyl, substituted or unsubstituted C₁~C₆ alkoxy, substituted or unsubstituted C₂~C₆ alkenyl, substituted or unsubstituted C₂~C₆ alkynyl, halogen, cyano, substituted or unsubstituted 3- to 10-membered cycloalkyl, substituted or unsubstituted C₁~C₆ ester, and substituted or unsubstituted C₁~C₆ amide;
R₁₄₁ and R₁₄₂ are independently selected from hydrogen, substituted or unsubstituted C₁~C₆ alkyl, substituted or unsubstituted C₁~C₆ alkoxy, and halogen, respectively;
R₁₅, R₁₆, R₁₇, and R₁₈ are each independently selected from hydrogen, substituted or unsubstituted C₁ to C₆ alkyl groups, and halogens;
R₁, R₅, R₁₉, and R₂₀ are each independently selected from hydrogen, substituted or unsubstituted C₁ to C₆ alkyl groups, and halogens;
V₁, V₂, and V₃ are each independently selected from N or C; V₄ is selected from N or CH; the dashed line connecting V₁, V₂, V₃, and V₄ is either empty or a key depending on the selection of V₁, V₂, V₃, and V₄.
n is 0 or 1;
U₁ is selected from N or CH; U₂, U₃, and U₄ are independently selected from N, C, and O, respectively; R₂₁, R₂₂, and R₂₃ are independently selected from unsubstituted, hydrogencontaining, hydroxyl-containing, substituted or unsubstituted C₁~C₆ alkyl groups, substituted or unsubstituted C₁~C₆ alkoxy groups, halogens, substituted or unsubstituted 3-10 membered cycloalkyl groups, cyano groups, amino groups, carboxyl groups, substituted or unsubstituted C₁ ~C₆ ester groups, etc. The substituted or unsubstituted C₁~C₆ amide group, the substituted or unsubstituted C₁~C₆ aldehyde group, the substituted or unsubstituted C₁~C₆ amino group, O=;
the dashed lines connecting U₁, U₂, U₃, U₄ are either absent or have no bond, depending on the selection of U₁, U₂, U₃, U₄, R₂₁, R₂₂, R₂₃.
The substituents of the alkyl, alkenyl, alkynyl, alkoxy, aldehyde, amino, ester, and amide groups are selected from the following groups: halogen, hydroxyl, cyano, C₁~C₆alkyl, halo-C₁~ C₆alkyl, C₁~C₆alkoxy, substituted or unsubstituted 3-10 membered cycloalkyl, substituted or unsubstituted 3-10 membered heterocycloalkyl, substituted or unsubstituted 6-10 membered aryl, substituted or unsubstituted 5-10 membered heteroaryl, -C(O)R₁', -NR₂'R₃';
The substituents of the cycloalkyl, heterocycloalkyl, aryl, and heteroaryl groups are selected from the following groups: C₁~C₆ alkyl, halogenated C₁~C₆ alkyl, halogen, and hydroxyl;
R₁', R₂', and R₃' are each independently selected from hydrogen and C₁ to C₆ alkyl groups, respectively.

25. The compound according to claim 24, its pharmaceutically acceptable salt, or its stereoisomer, is **characterized in that**: the structure of the compound is as shown in formula II-A-7 or formula II-B-7: 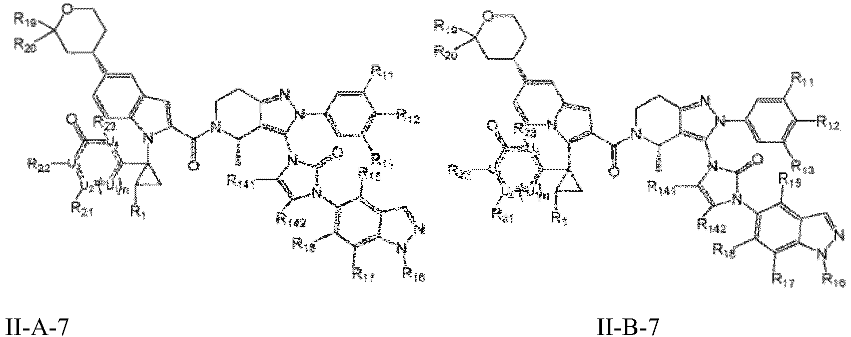 wherein,
R₁₁, R₁₂, and R₁₃ are each independently selected from hydrogen, substituted or unsubstituted C₁ ~C₆ alkyl, substituted or unsubstituted C₁~C₆ alkoxy, substituted or unsubstituted C₂~C₆ alkenyl, substituted or unsubstituted C₂~C₆ alkynyl, halogen, cyano, substituted or unsubstituted 3- to 10-membered cycloalkyl, substituted or unsubstituted C₁~C₆ ester, and substituted or unsubstituted C₁~C₆ amide;
R₁₄₁ and R₁₄₂ are independently selected from hydrogen, substituted or unsubstituted C₁~C₆ alkyl, substituted or unsubstituted C₁~C₆ alkoxy, and halogen, respectively;
R₁₅, R₁₆, R₁₇, and R₁₈ are each independently selected from hydrogen, substituted or unsubstituted C₁ to C₆ alkyl groups, and halogens;
R₁, R₁₉, and R₂₀ are independently selected from hydrogen, substituted or unsubstituted C₁~C₆ alkyl groups, and halogens, respectively;
n is 0 or 1;
U₁ is selected from N or CH; U₂, U₃, and U₄ are independently selected from N, C, and O, respectively; R₂₁, R₂₂, and R₂₃ are independently selected from unsubstituted, hydrogencontaining, hydroxyl-containing, substituted or unsubstituted C₁~C₆ alkyl groups, substituted or unsubstituted C₁~C₆ alkoxy groups, halogens, substituted or unsubstituted 3-10 membered cycloalkyl groups, cyano groups, amino groups, carboxyl groups, substituted or unsubstituted C₁ ~C₆ ester groups, etc. The substituted or unsubstituted C₁~C₆ amide groups, substituted or unsubstituted C₁~C₆ aldehyde groups, substituted or unsubstituted C₁~C₆ amino groups, O=; the dashed lines connecting U₁, U₂, U₃, U₄ are either absent or non-existent depending on the selection of U₁, U₂, U₃, U₄, R₂₁, R₂₂, R₂₃;
The substituents of the alkyl, alkenyl, alkynyl, alkoxy, aldehyde, amino, ester, and amide groups are selected from the following groups: halogen, hydroxyl, cyano, C₁~C₆alkyl, halo-C₁~ C₆alkyl, C₁~C₆alkoxy, substituted or unsubstituted 3-10 membered cycloalkyl, substituted or unsubstituted 3-10 membered heterocycloalkyl, substituted or unsubstituted 6-10 membered aryl, substituted or unsubstituted 5-10 membered heteroaryl, -C(O)R₁', -NR₂'R₃';
The substituents of the cycloalkyl, heterocycloalkyl, aryl, and heteroaryl groups are selected from the following groups: C₁~C₆ alkyl, halogenated C₁~C₆ alkyl, halogen, and hydroxyl;
R₁', R₂', and R₃' are independently selected from hydrogen and C₁ to C₆ alkyl groups, respectively.

26. The compound according to claim 25, its pharmaceutically acceptable salt, or its stereoisomer, is **characterized in that**: the structure of the compound is as shown in Formula III-A-7 or Formula III-B-7. 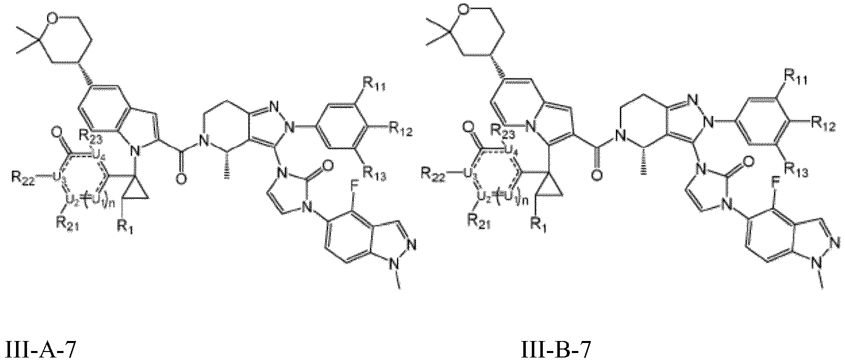 wherein,
R₁₁, R₁₂, and R₁₃ are each independently selected from hydrogen, substituted or unsubstituted C₁ ~C₆ alkyl, substituted or unsubstituted C₁~C₆ alkoxy, substituted or unsubstituted C₂~C₆ alkenyl, substituted or unsubstituted C₂~C₆ alkynyl, halogen, cyano, substituted or unsubstituted 3- to 10-membered cycloalkyl, substituted or unsubstituted C₁~C₆ ester, and substituted or unsubstituted C₁~C₆ amide;
R₁ is selected from hydrogen, substituted or unsubstituted C₁~C₆ alkyl, halogen;
n is 0 or 1;
U₁ is selected from N or CH; U₂, U₃, and U₄ are independently selected from N, C, and O,
respectively; R₂₁, R₂₂, and R₂₃ are independently selected from unsubstituted, hydrogencontaining, hydroxyl-containing, substituted or unsubstituted C₁~C₆ alkyl groups, substituted or unsubstituted C₁~C₆ alkoxy groups, halogens, substituted or unsubstituted 3-10 membered cycloalkyl groups, cyano groups, amino groups, carboxyl groups, substituted or unsubstituted C₁ ~C₆ ester groups, etc. The substituted or unsubstituted C₁~C₆ amide group, the substituted or unsubstituted C₁~C₆ aldehyde group, the substituted or unsubstituted C₁~C₆ amino group, O=;
the dashed lines connecting U₁, U₂, U₃, U₄ are either absent or non-existent depending on the selection of U₁, U₂, U₃, U₄, R₂₁, R₂₂, R₂₃.
The substituents of the alkyl, alkenyl, alkynyl, alkoxy, aldehyde, amino, ester, and amide groups are selected from the following groups: halogen, hydroxyl, cyano, C₁~C₆ alkyl, halo-C₁~C₆ alkyl, C₁~C₆ alkoxy, substituted or unsubstituted 3- to 10-membered cycloalkyl, substituted or unsubstituted 3- to 10-membered heterocycloalkyl, substituted or unsubstituted 6- to 10-membered aryl, substituted or unsubstituted 5- to 10-membered heteroaryl, -C(O)R₁', -NR₂'R₃'; The substituents of the cycloalkyl, heterocycloalkyl, aryl, and heteroaryl groups are selected from the following groups: C₁~C₆ alkyl, halogenated C₁~C₆ alkyl, halogen, and hydroxyl;
R₁', R₂', and R₃' are independently selected from hydrogen and C₁ to C₆ alkyl groups, respectively.

27. The compound according to any one of claims 24 to 26, its pharmaceutically acceptable salt, or its stereoisomer, is **characterized in that**: 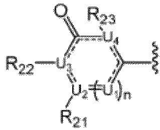 is selected from the group consisting of 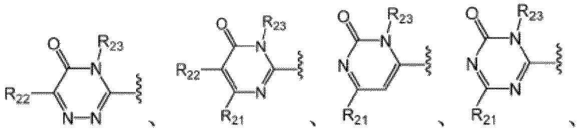 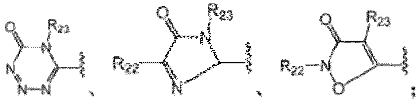
R₂₁, R₂₂, and R₂₃ are each independently selected from hydrogen, hydroxyl, substituted or unsubstituted C₁~C₆ alkyl, substituted or unsubstituted C₁~C₆ alkoxy, halogen, substituted or unsubstituted 3-10 membered cycloalkyl, cyano, amino, carboxyl, substituted or unsubstituted C₁ ~C₆ ester, substituted or unsubstituted C₁~C₆ amide, substituted or unsubstituted C₁~C₆ aldehyde, substituted or unsubstituted C₁~C₆ amino, O=;
The substituents of the alkyl, alkoxy, aldehyde, amino, ester, and amide groups are selected from the following groups: halogen, hydroxyl, cyano, C₁~C₆alkyl, halo-C₁~C₆alkyl, C₁~C₆alkoxy; The substituents of the cycloalkyl group are selected from the following groups: C₁~C₆ alkyl, halogenated C₁~C₆ alkyl, halogen, hydroxyl.

28. The compound according to claim 27, its pharmaceutically acceptable salt, or its stereoisomer, is 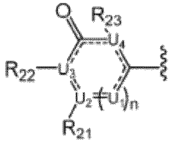 **characterized in that**:
is selected from the group consisting of 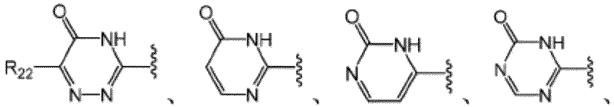 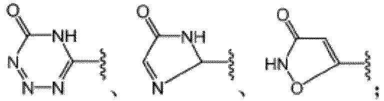
R₂₂ is selected from hydrogen, hydroxyl, C₁~C₃alkyl, C₁~C₃alkoxy, methyl group substituted with 1-3 fluorine groups, halogen, 3-6 membered cycloalkyl, cyano, amino, carboxyl, C₃ester, C₁amide, formaldehyde, C₃amine, and O=.

29. The compound according to claim 27, its pharmaceutically acceptable salt, or its stereoisomer, is **characterized in that**: the structure of the compound is as shown in Formula IV-7: 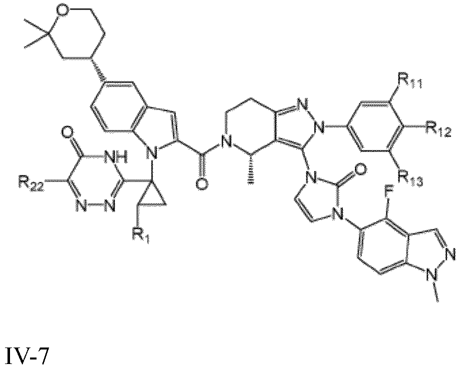 wherein,
R₁₁, R₁₂, and R₁₃ are each independently selected from hydrogen, substituted or unsubstituted C₁ ~C₆ alkyl, substituted or unsubstituted C₁~C₆ alkoxy, substituted or unsubstituted C₂~C₆ alkenyl, substituted or unsubstituted C₂~C₆ alkynyl, halogen, cyano, substituted or unsubstituted 3- to 10-membered cycloalkyl, substituted or unsubstituted C₁~C₆ ester, and substituted or unsubstituted C₁~C₆ amide;
R₁ is selected from hydrogen, substituted or unsubstituted C₁~C₆ alkyl, halogen;
R₂₂ is selected from hydrogen, hydroxyl, substituted or unsubstituted C₁~C₆ alkyl, substituted or unsubstituted C₁~C₆ alkoxy, halogen, substituted or unsubstituted 3-10 membered cycloalkyl, cyano, amino, carboxyl, substituted or unsubstituted C₁~C₆ ester, substituted or unsubstituted C₁~C₆ amide, substituted or unsubstituted C₁~C₆ aldehyde, substituted or unsubstituted C₁~ C₆ amino, O=;
The substituents of the alkyl, alkenyl, alkynyl, alkoxy, aldehyde, amino, ester, and amide groups are selected from the following groups: halogen, hydroxyl, cyano, C₁~C₆alkyl, halo-C₁~ C₆alkyl, C₁~C₆alkoxy;
The substituents of the cycloalkyl group are selected from the following groups: C₁~C₆ alkyl, halogenated C₁~C₆ alkyl, halogen, hydroxyl;
Preferably, R₂₂ is selected from hydrogen, hydroxyl, C₁~C₃alkyl, C₁~C₃alkoxy, methyl group substituted with 1-3 fluorine groups, halogen, 3-6 membered cycloalkyl, cyano, amino, carboxyl, C₃ester, C₁amide, formaldehyde, C₃amine, and O=.

30. The compound according to claim 29, its pharmaceutically acceptable salt, or its stereoisomer, is **characterized in that**: the structure of the compound is as shown in Formula V-7: 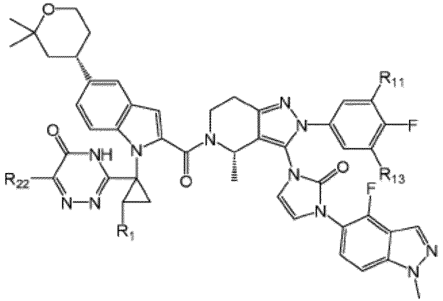 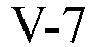 wherein,
R₁₁ and R₁₃ are independently selected from hydrogen, substituted or unsubstituted C₁~C₆ alkyl, substituted or unsubstituted C₁~C₆ alkoxy, substituted or unsubstituted C₂~C₆ alkenyl, substituted or unsubstituted C₂~C₆ alkynyl, halogen, cyano, substituted or unsubstituted 3-10 membered cycloalkyl, substituted or unsubstituted C₁~C₆ ester, and substituted or unsubstituted C₁~C₆ amide;
R₁ is selected from hydrogen, substituted or unsubstituted C₁~C₆ alkyl, halogen;
R₂₂ is selected from hydrogen, hydroxyl, substituted or unsubstituted C₁~C₆ alkyl, substituted or unsubstituted C₁~C₆ alkoxy, halogen, substituted or unsubstituted 3-10 membered cycloalkyl, cyano, amino, carboxyl, substituted or unsubstituted C₁~C₆ ester, substituted or unsubstituted C₁~C₆ amide, substituted or unsubstituted C₁~C₆ aldehyde, substituted or unsubstituted C₁~ C₆ amino, O=;
The substituents of the alkyl, alkenyl, alkynyl, alkoxy, aldehyde, amino, ester, and amide groups are selected from the following groups: halogen, hydroxyl, cyano, C₁~C₆alkyl, halo-C₁~ C₆alkyl, C₁~C₆alkoxy;
The substituents of the cycloalkyl group are selected from the following groups: C₁~C₆ alkyl, halogenated C₁~C₆ alkyl, halogen, hydroxyl;
Preferably, R₂₂ is selected from hydrogen, hydroxyl, C₁~C₃alkyl, C₁~C₃alkoxy, methyl group substituted with 1-3 fluorine groups, halogen, 3-6 membered cycloalkyl, cyano, amino, carboxyl, C₃ester, C₁amide, formaldehyde, C₃amine, and O=.

31. The compound according to claim 30, its pharmaceutically acceptable salt, or its stereoisomer, is **characterized in that**: the structure of the compound is as shown in Formula V-7-1: 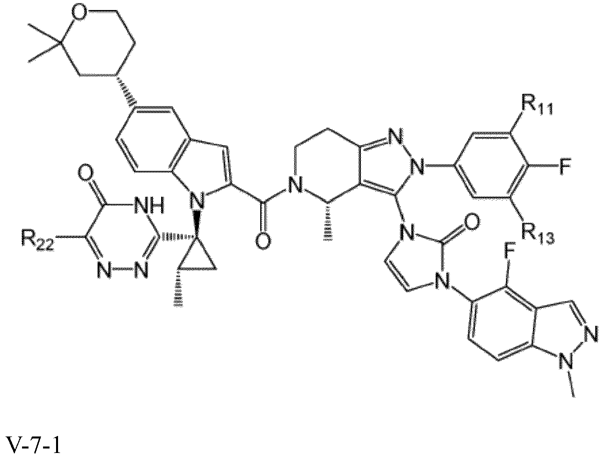 wherein,
R₁₁ and R₁₃ are independently selected from C₁ to C₆ alkyl groups;
R₂₂ is selected from hydrogen, 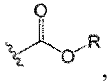 R is selected from C₁alkyl, C₂alkyl, C₃alkyl, C₄alkyl, C₅alkyl, C₆alkyl.

32. The compound according to claim 1, its pharmaceutically acceptable salt, or its stereoisomer, is **characterized in that**: the compound is one of the following compounds with the following structure:

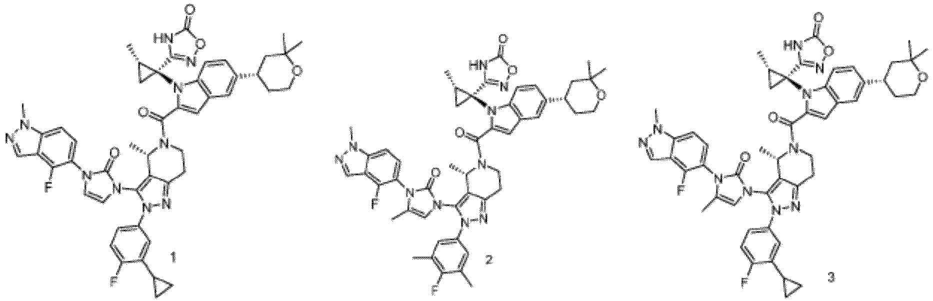

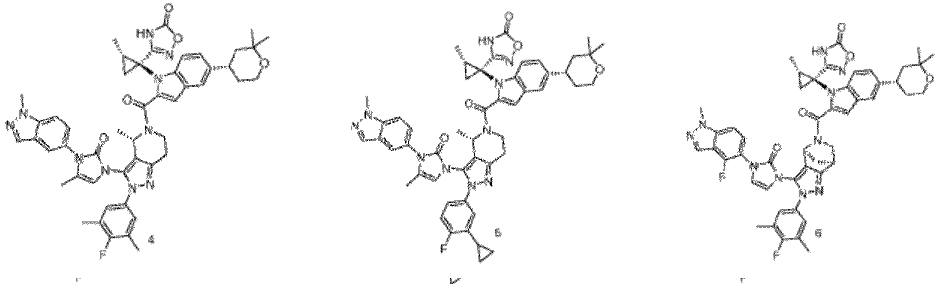

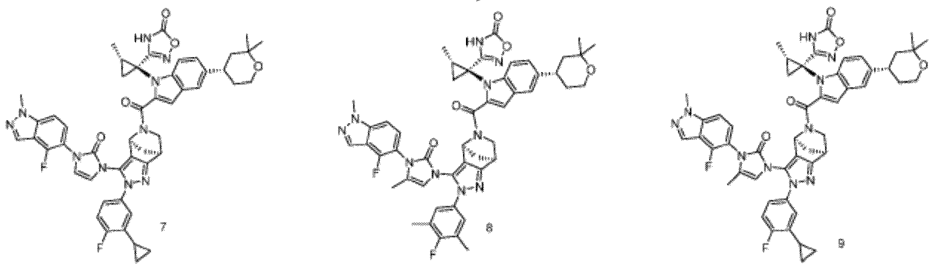

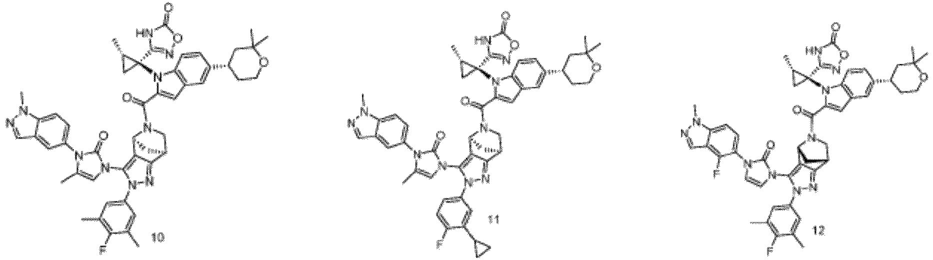

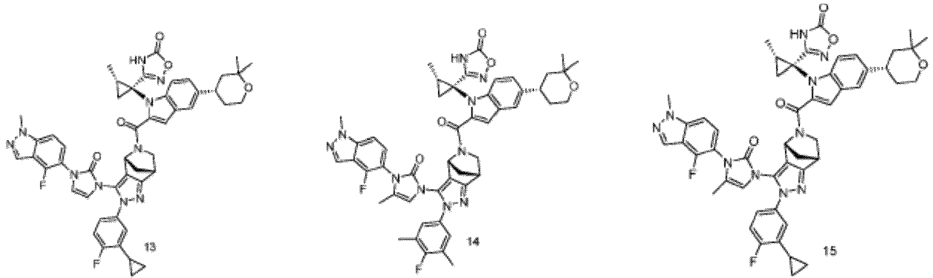

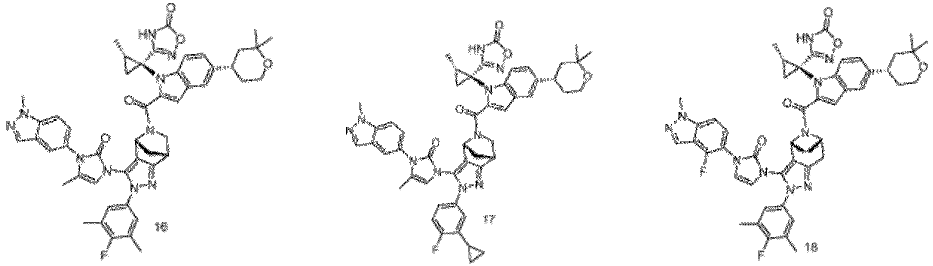

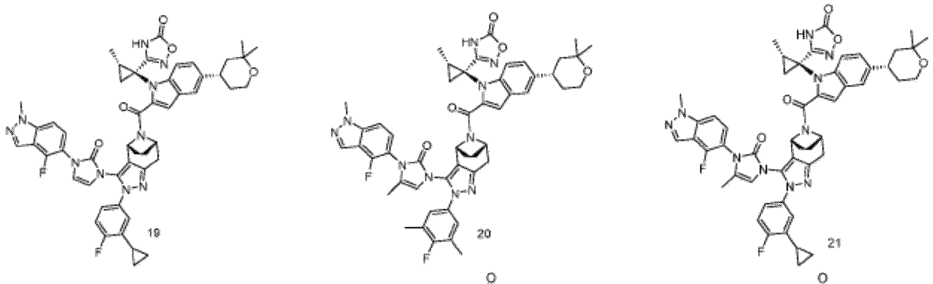

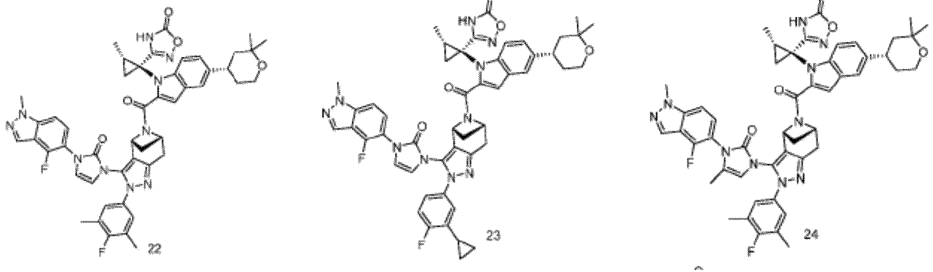

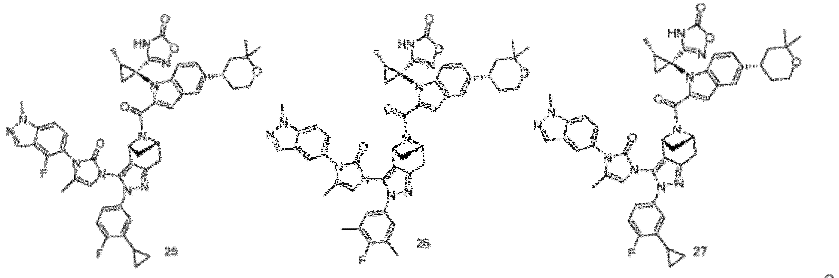

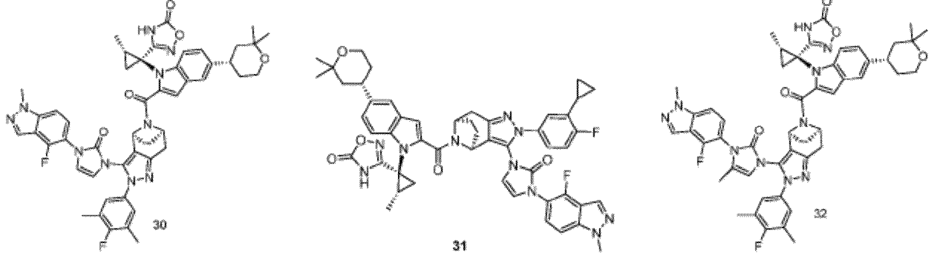

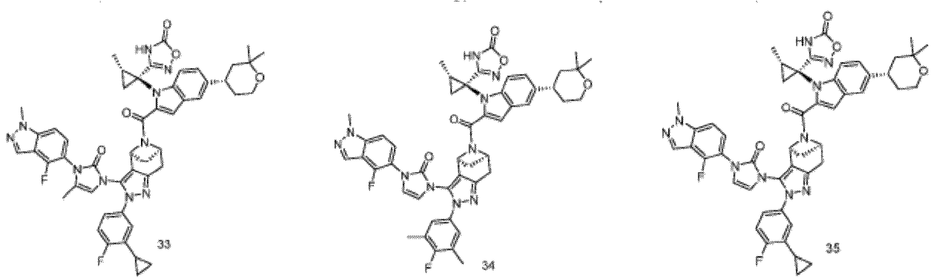

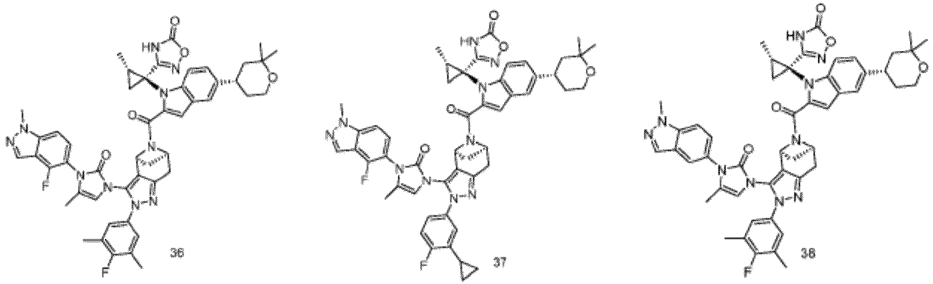

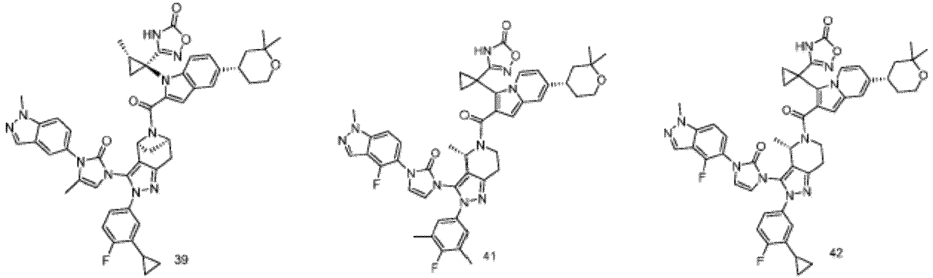

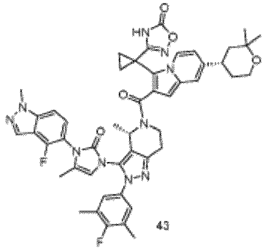

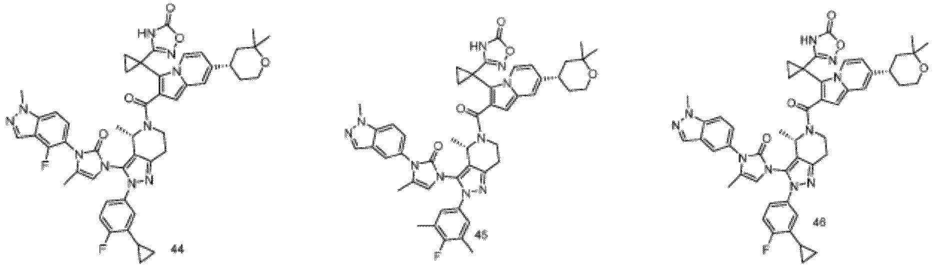

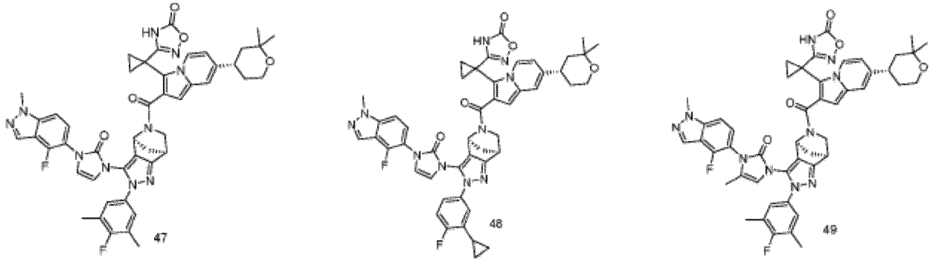

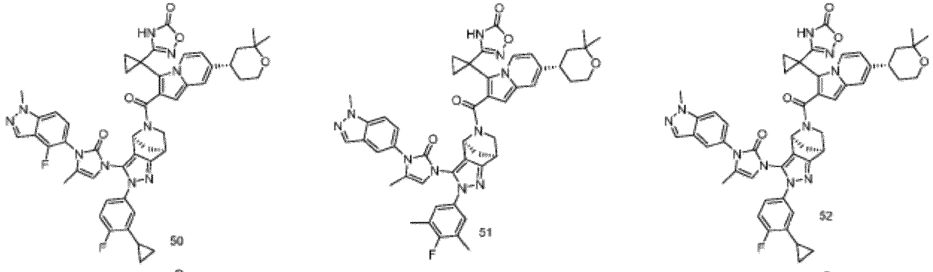

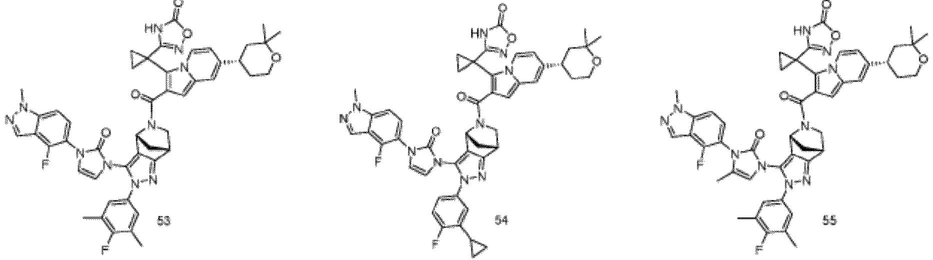

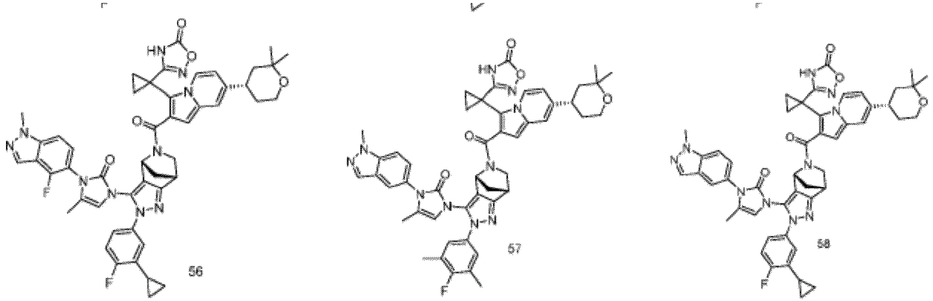

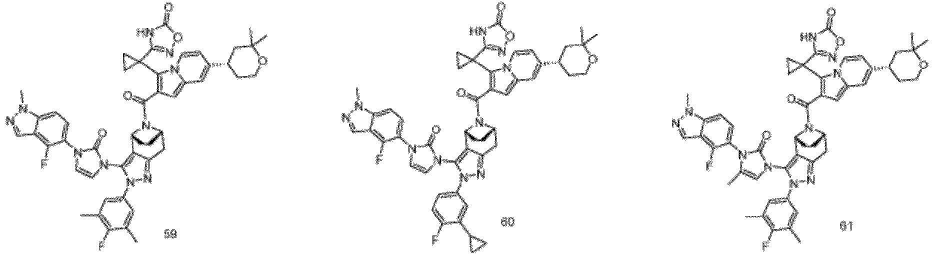

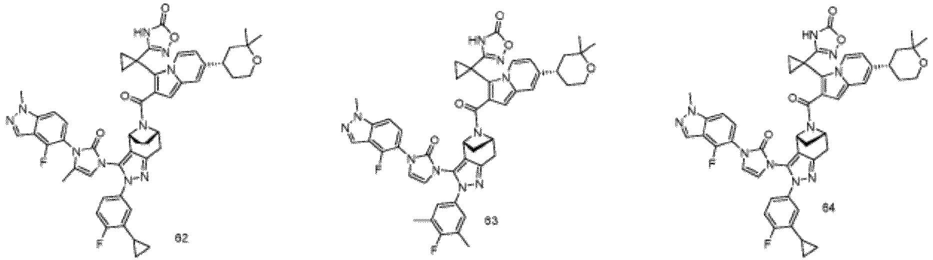

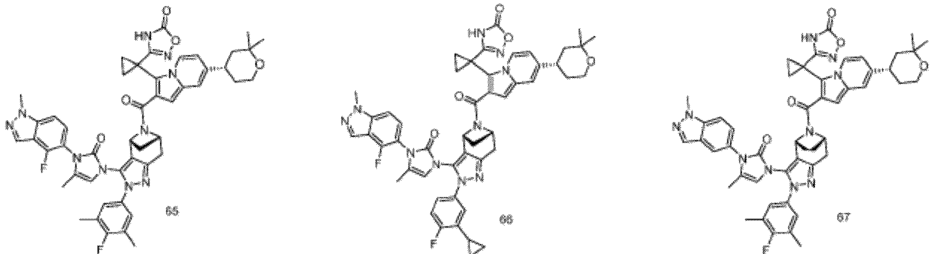

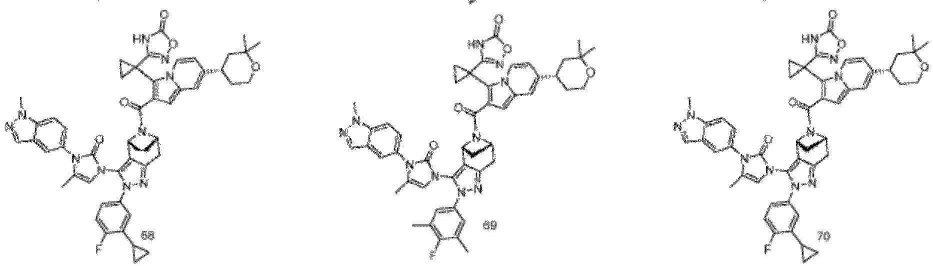

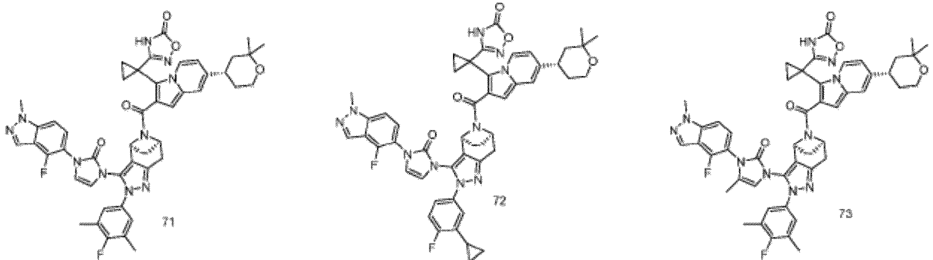

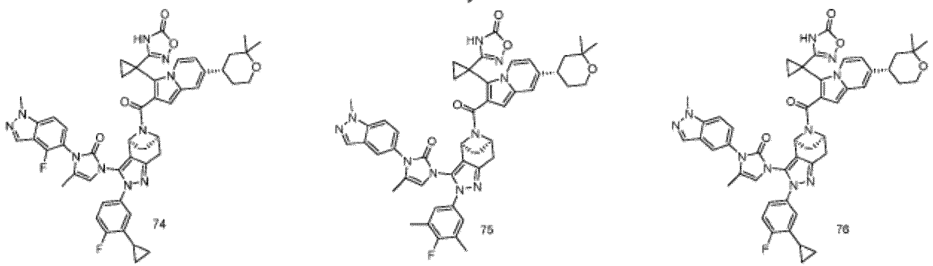

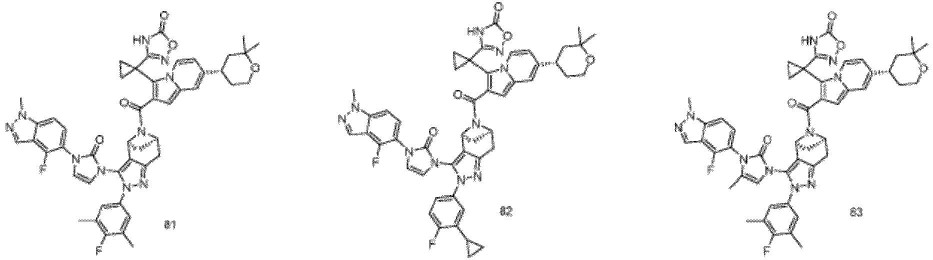

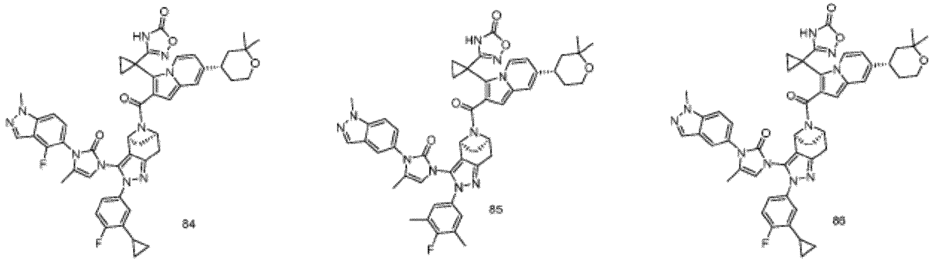

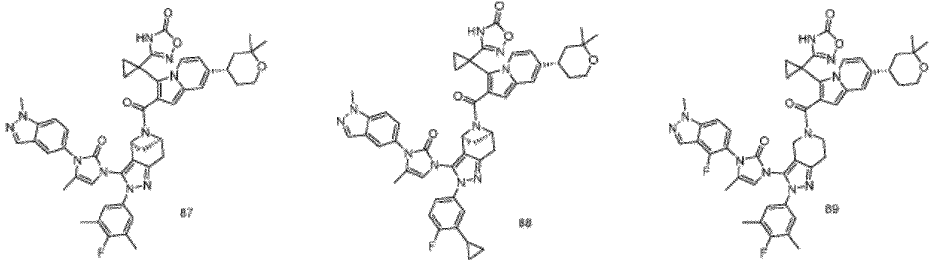

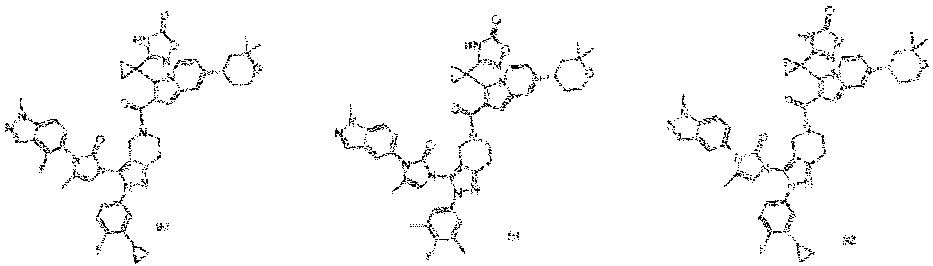

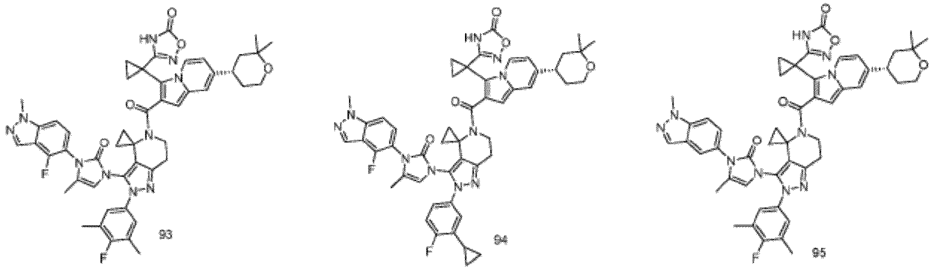

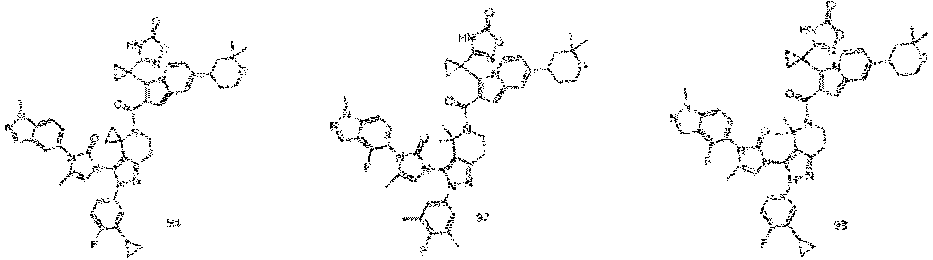

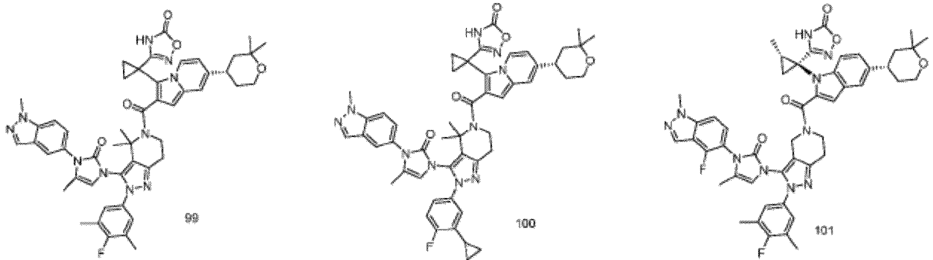

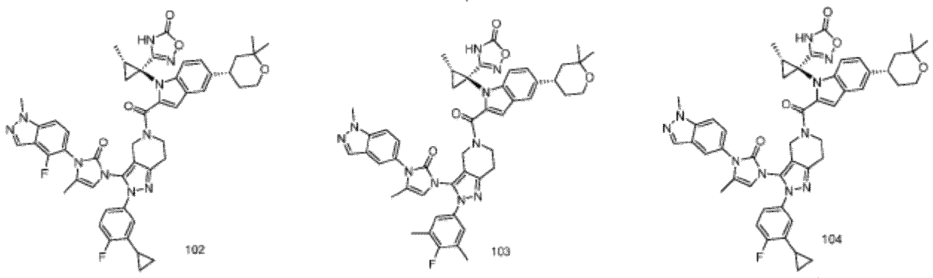

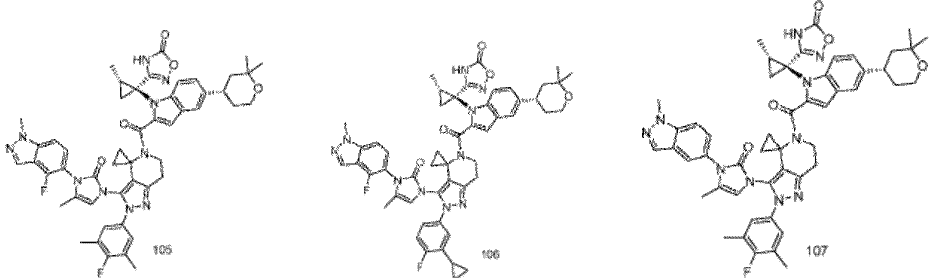

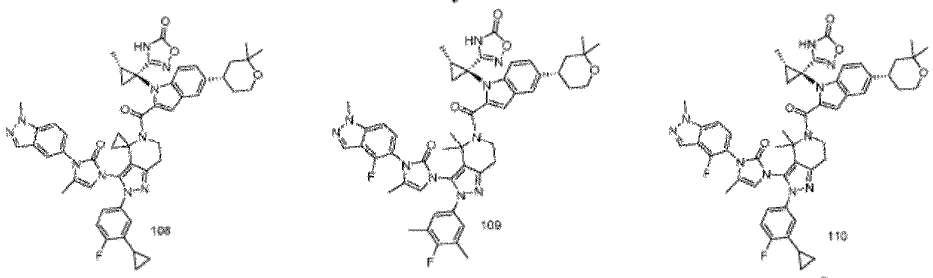

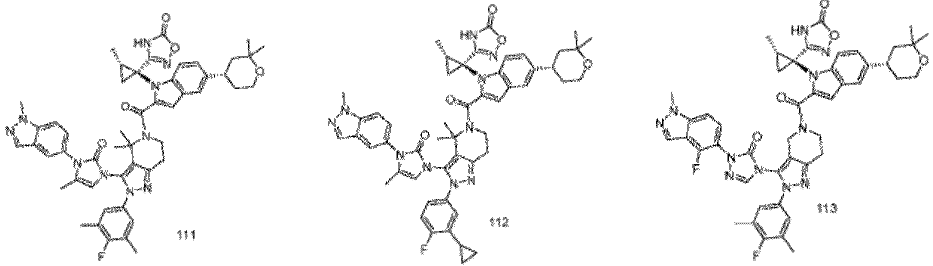

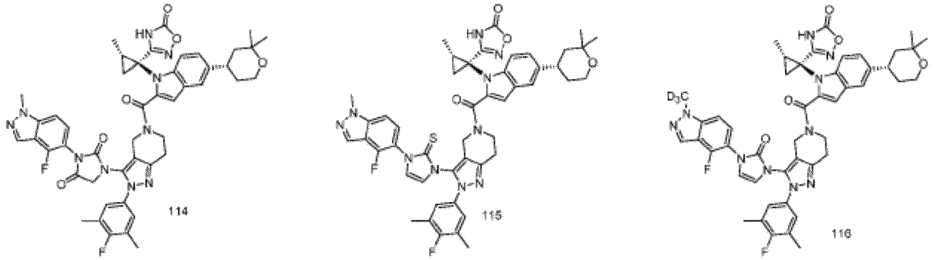

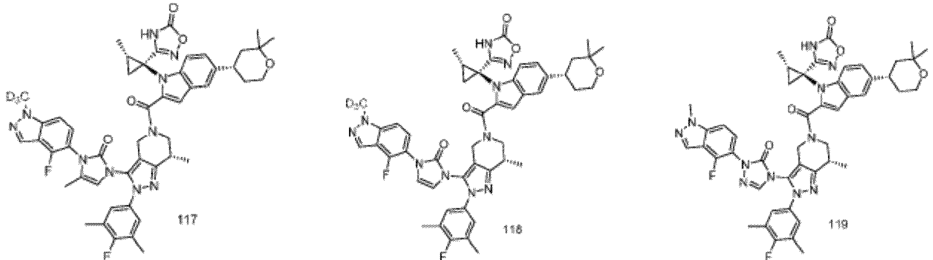

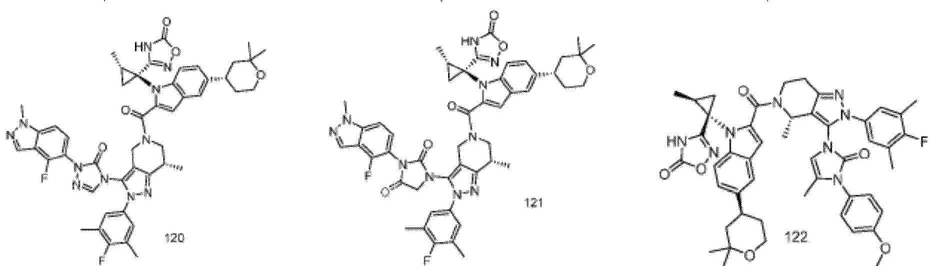

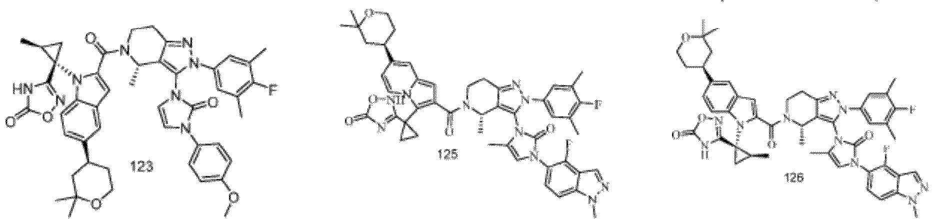

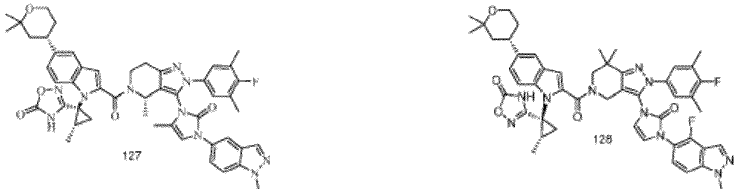

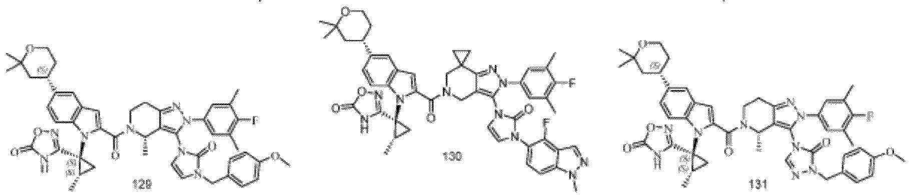

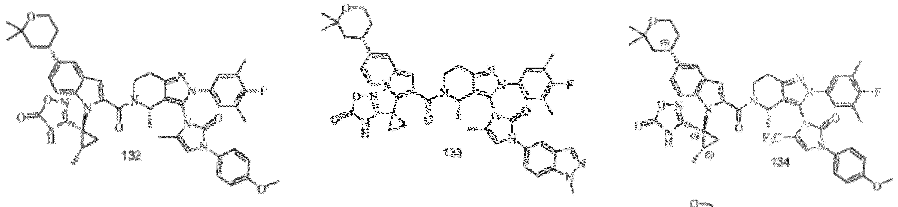

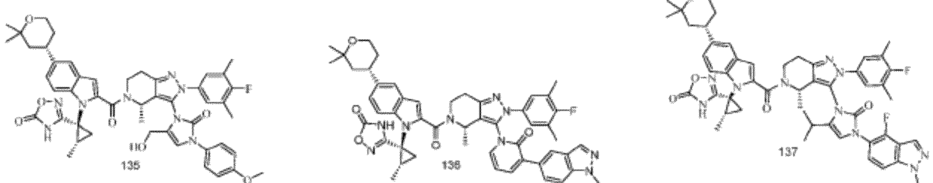

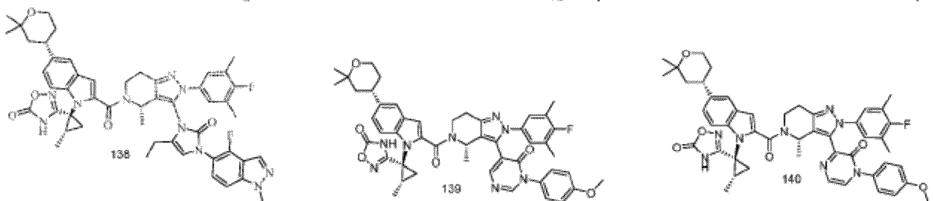

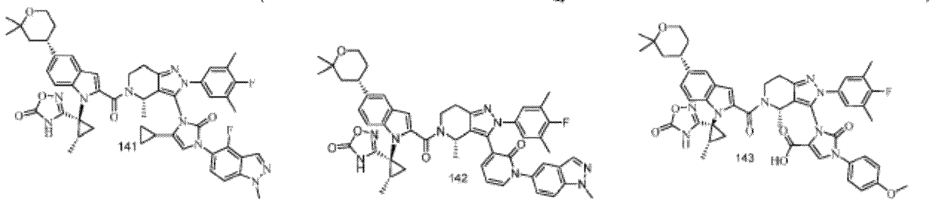

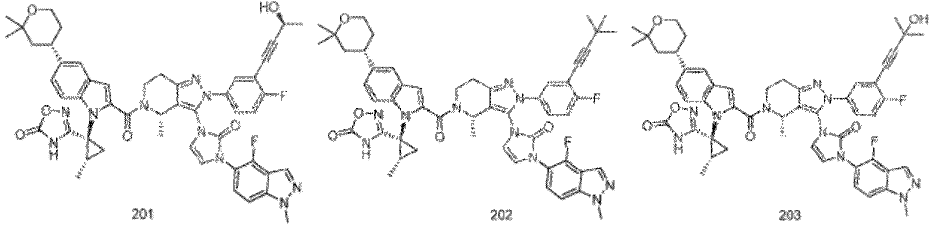

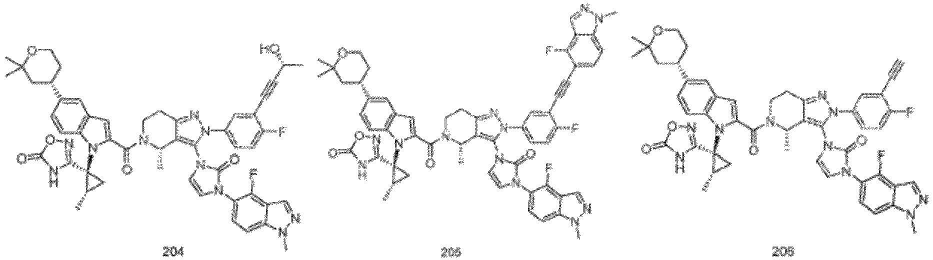

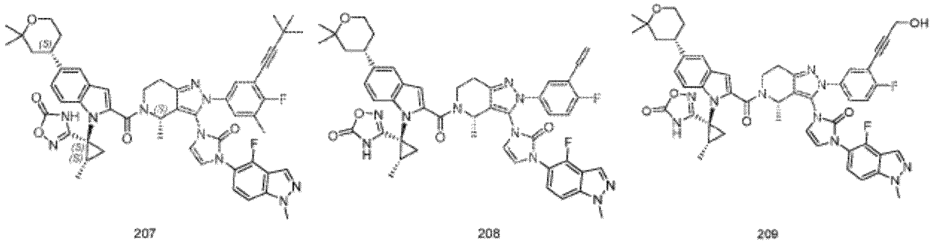

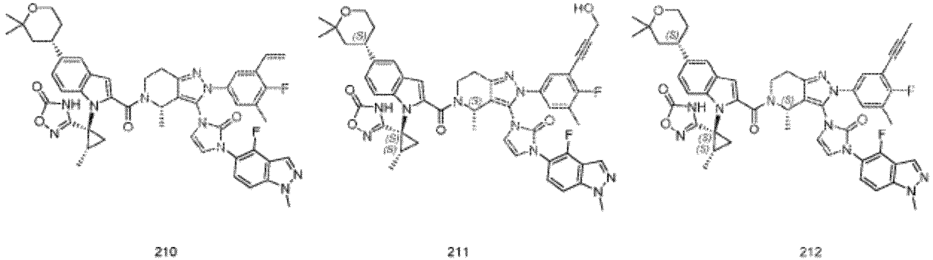

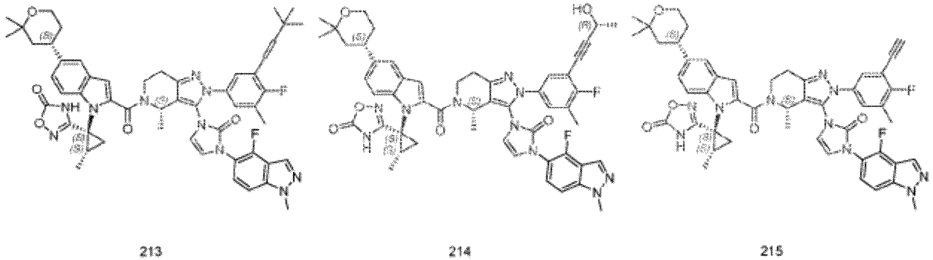

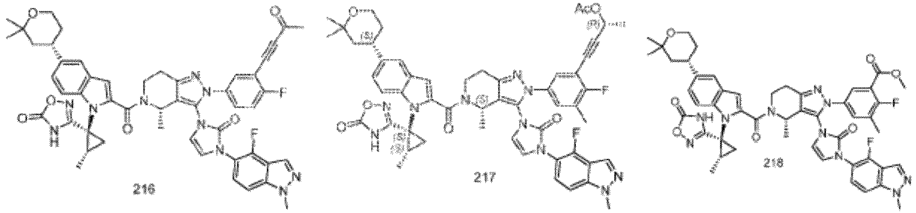

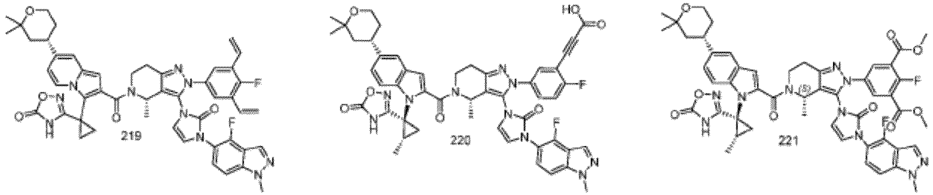

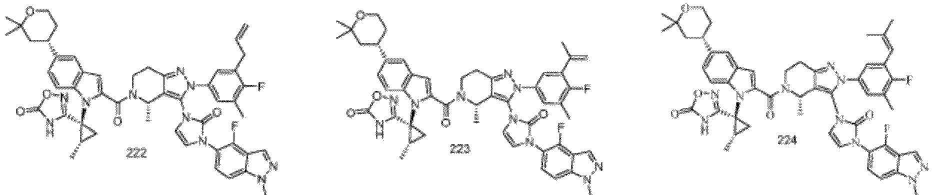

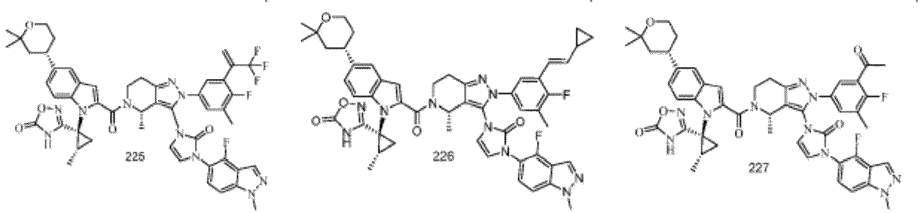

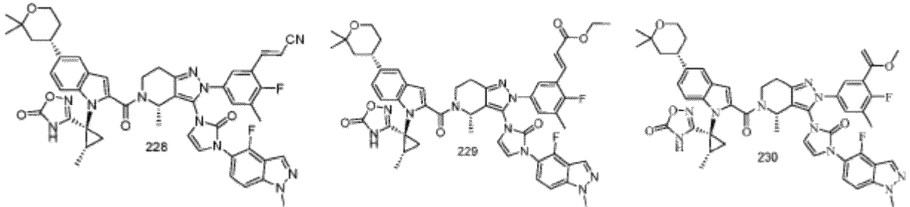

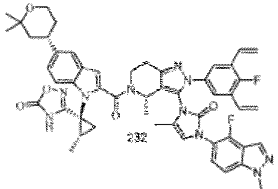

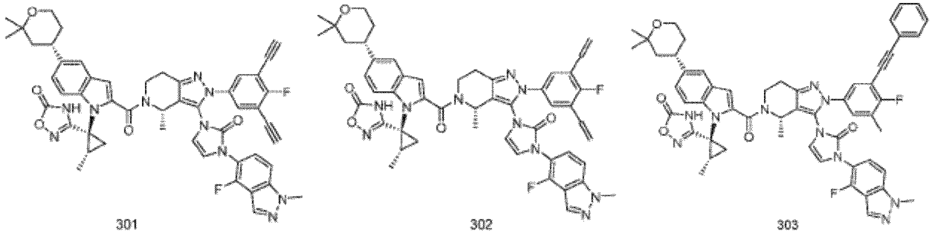

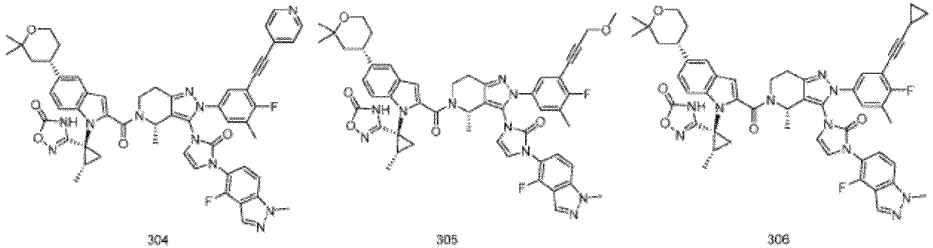

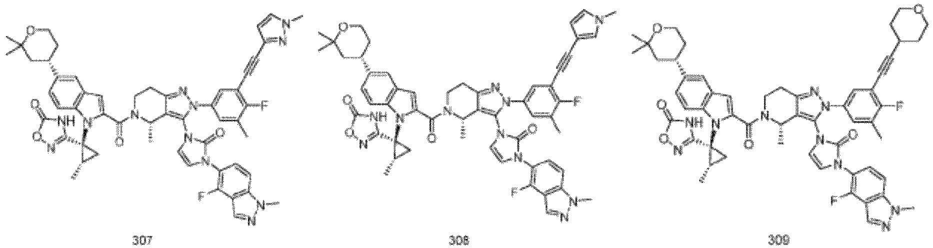

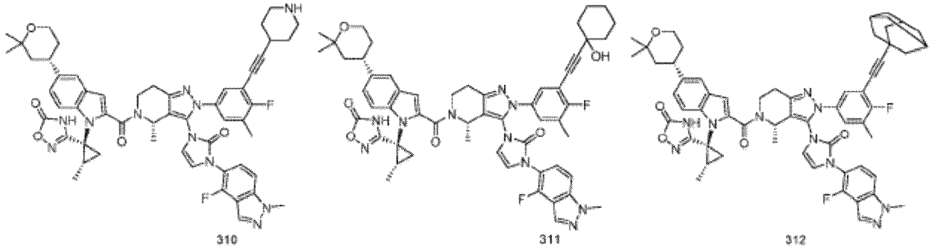

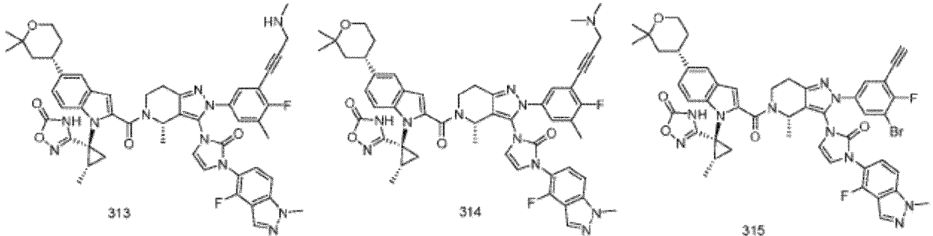

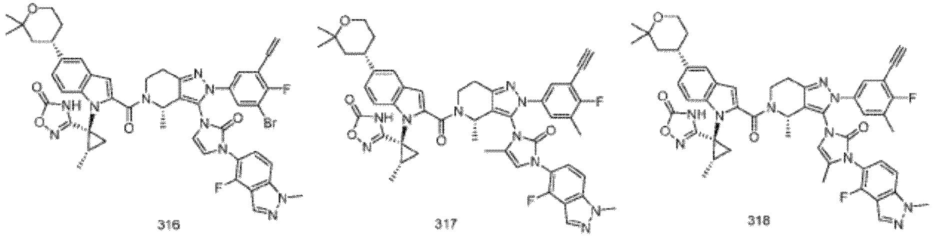

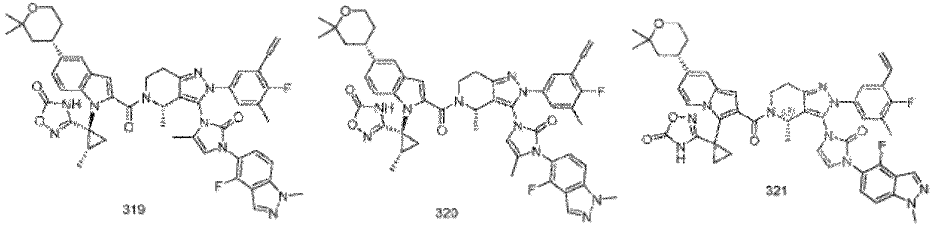

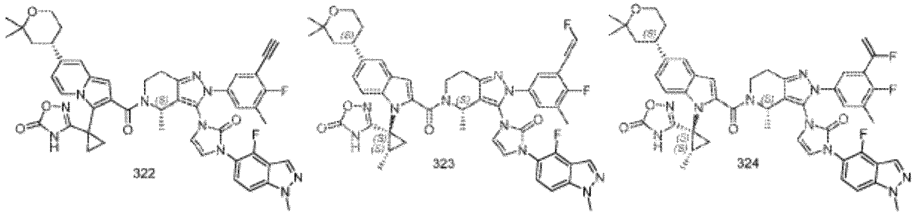

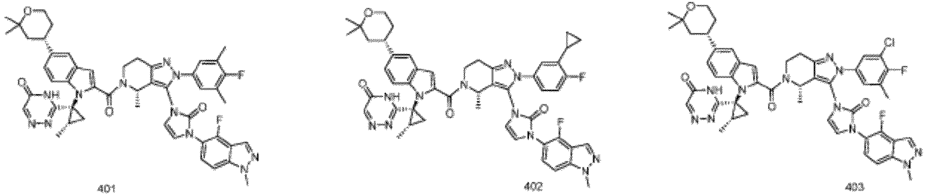

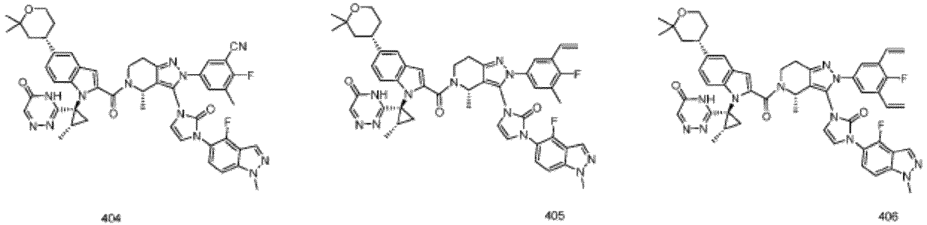

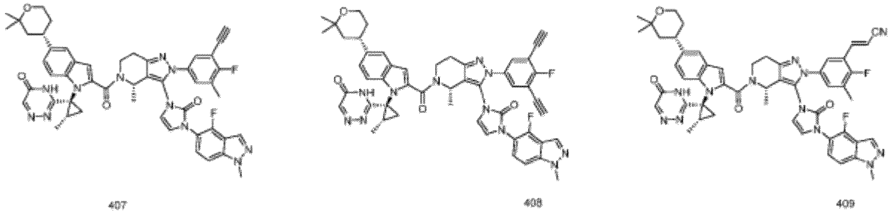

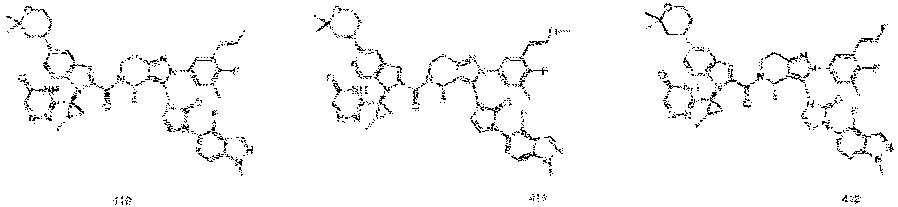

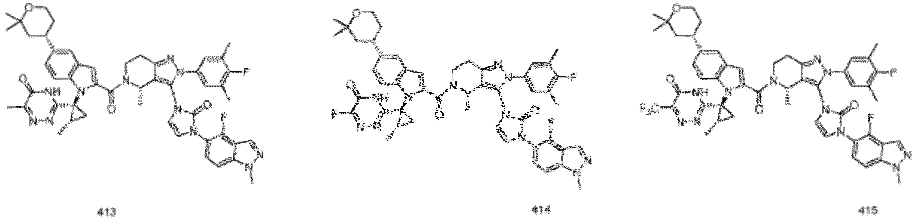

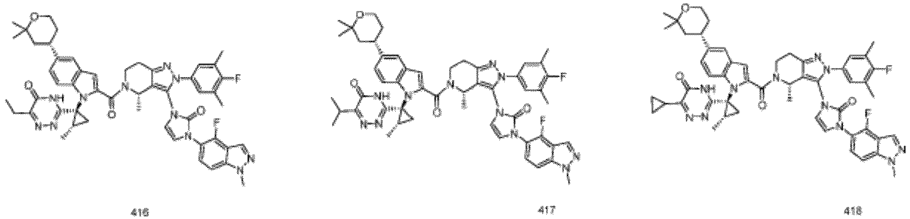

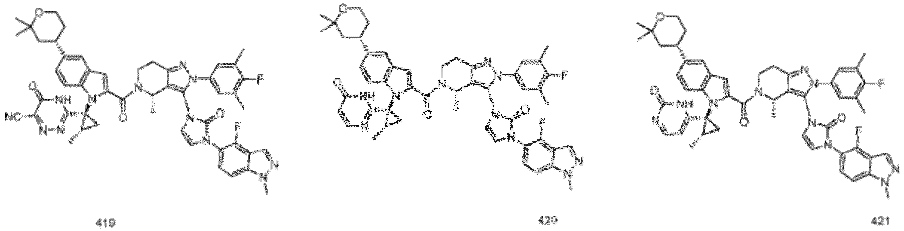

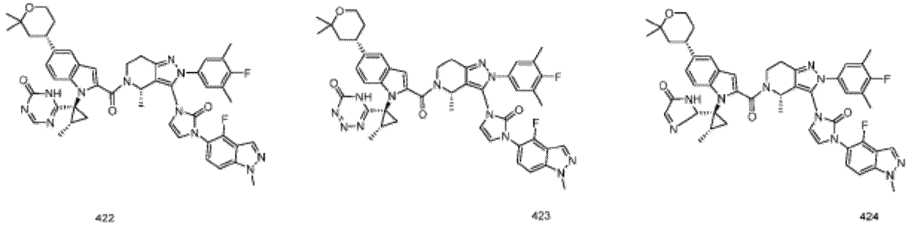

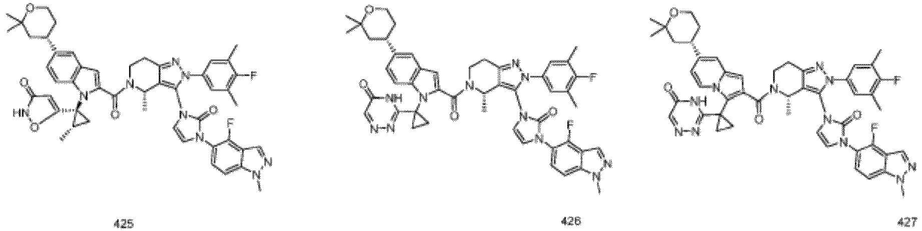

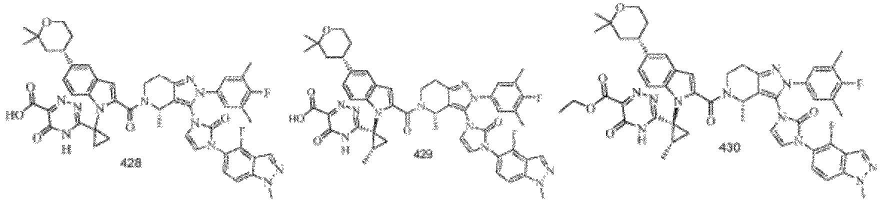

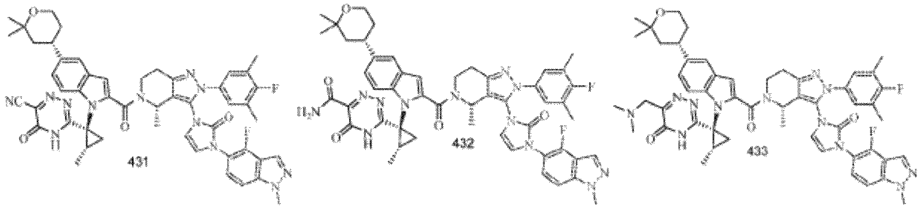

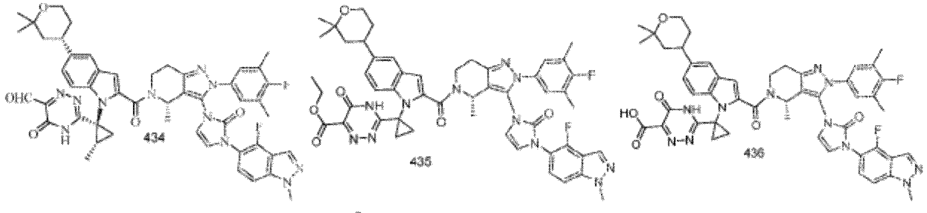

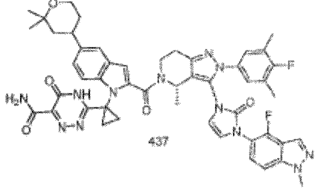

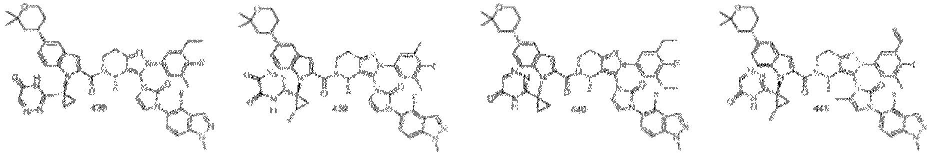

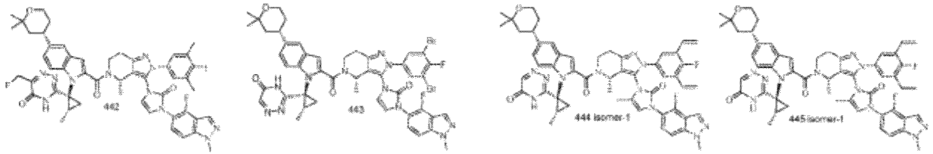

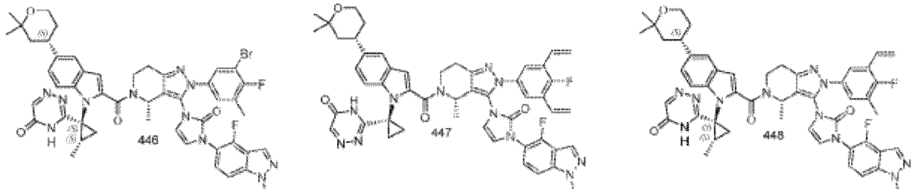

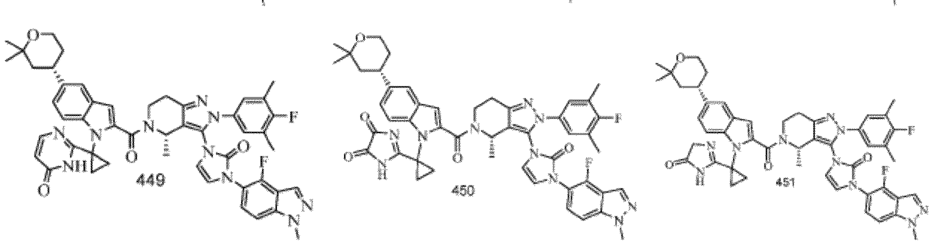

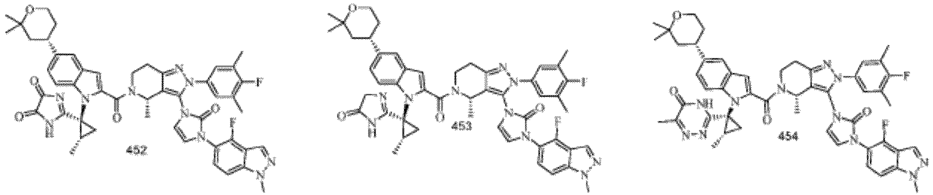

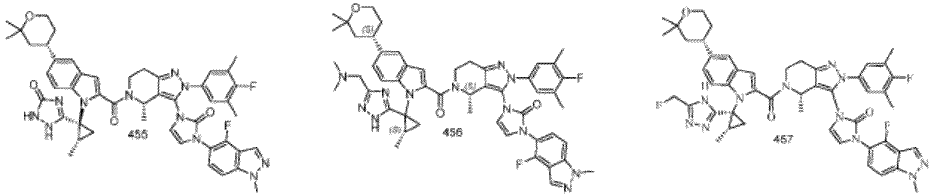

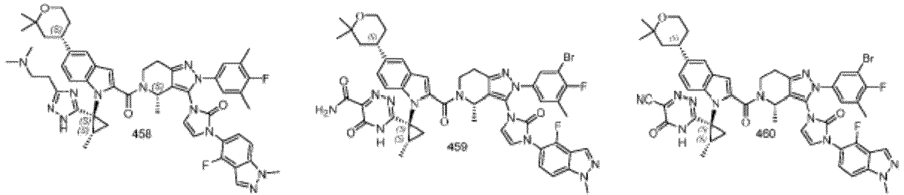

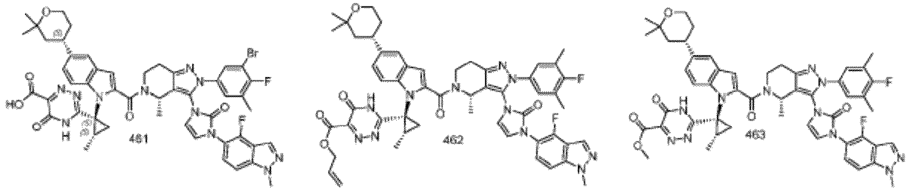

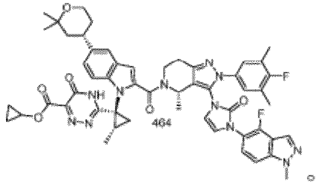

33. Use of the compound of any one of claims 1 to 32, its pharmaceutically acceptable salt or its stereoisomer in the preparation of a GLP-1 receptor agonist.

34. Use of the compound of any one of claims 1 to 32, its pharmaceutically acceptable salt or its stereoisomer in the preparation of a medicament for the prevention and/or treatment of diseases related to the GLP-1 receptor.

35. The use according to claim 34 is **characterized in that**: the diseases associated with the GLP-1 receptor are diabetes, obesity, fatty liver, and polycystic ovary syndrome;
Preferably, the diabetes is type 2 diabetes.

36. A drug, **characterized in that** it is a formulation prepared by adding pharmaceutically acceptable excipients or auxiliary ingredients to a compound as described in any one of claims 1 to 32, a pharmaceutically acceptable salt thereof or a stereoisomer thereof as the active ingredient.
